(19) Europäisches Patentamt
European Patent Office
Office européen des brevets

(11) EP 4 592 283 A1

(12) **EUROPEAN PATENT APPLICATION**
published in accordance with Art. 153(4) EPC

(43) Date of publication:
**30.07.2025 Bulletin 2025/31**

(21) Application number: **23868694.3**

(22) Date of filing: **04.09.2023**

(51) International Patent Classification (IPC):
***C07D 401/12*** (2006.01) ***C07D 401/14*** (2006.01)
***C07D 417/04*** (2006.01) ***C07D 417/12*** (2006.01)
***C07D 277/44*** (2006.01) ***C07D 413/14*** (2006.01)
***C07D 405/14*** (2006.01) ***A61K 31/506*** (2006.01)
***A61P 35/00*** (2006.01) ***A61P 37/00*** (2006.01)
***A61P 29/00*** (2006.01) ***A61P 31/00*** (2006.01)

(52) Cooperative Patent Classification (CPC):
**A61K 31/506; A61P 29/00; A61P 31/00; A61P 35/00; A61P 37/00; C07D 277/44; C07D 401/12; C07D 401/14; C07D 405/14; C07D 413/14; C07D 417/04; C07D 417/12**

(86) International application number:
**PCT/RU2023/050208**

(87) International publication number:
**WO 2024/063670 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.09.2022 RU 2022124945**
**01.09.2023 RU 2023122794**

(71) Applicant: **Joint Stock Company "Biocad"**
**Saint Petersburg 198515 (RU)**

(72) Inventors:
- **GORBUNOVA, Svetlana Leonidovna**
  **Saint Petersburg, 197198 (RU)**
- **MIKHAYLOV, Leonid Evgenevich**
  **Saint Petersburg, 191028 (RU)**
- **IAKOVLEV, Danila Alekseevich**
  **g.Berdsk, 633010 (RU)**
- **CHESTNOVA, Anna Yurievna**
  **Saint Petersburg, 198264 (RU)**
- **LENSHMIDT, Liliana Vyacheslavovna**
  **Saint Petersburg, 198332 (RU)**
- **VIKENTEVA, Yulia Arturovna**
  **Saint Petersburg, 199406 (RU)**
- **EFIMENKO, Nikita Igorevich**
  **g.Miass, 456302 (RU)**
- **GOLUBEV, Artem Alekseevich**
  **Chagodoshchenskii r-n, p.Sazonovo, 162430 (RU)**
- **CHEREMUSHKIN, Andrei Ivanovich**
  **Saint Petersburg, 196158 (RU)**
- **LUKASHENKO, Anton Vladimirovich**
  **g.Samara, 443091 (RU)**
- **SMOLNIKOV, Sergei Alexandrovich**
  **g.Izhevsk, 42605 (RU)**
- **KISELEV, Maksim Aleksandrovich**
  **g.Perm, 614105 (RU)**
- **SHARKOV, Dmitrii Evgenievich**
  **Saint Petersburg, 198517 (RU)**
- **SHPAKOVA, Elena Alexandrovna**
  **Saint Petersburg, 198505 (RU)**
- **VORONKOVA, Maria Sergeevna**
  **Saint Petersburg, 198262 (RU)**
- **MOROZOV, Dmitry Valentinovich**
  **Saint Petersburg, Admiraltejskij r-n,190000 (RU)**

(74) Representative: **Meissner Bolte Partnerschaft mbB**
**Patentanwälte Rechtsanwälte**
**Postfach 86 06 24**
**81633 München (DE)**

# (54) CYCLIN-DEPENDENT KINASE 7 INHIBITORS

(57) The present invention relates to novel compounds of formula I:

EP 4 592 283 A1

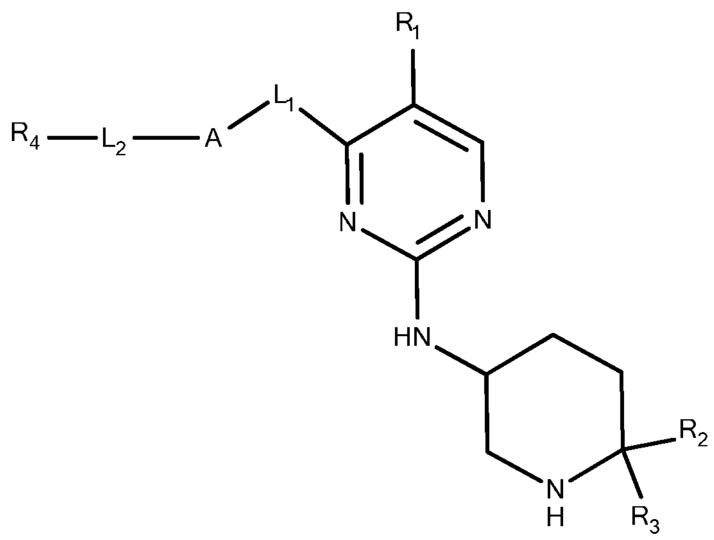

**I**,

or a pharmaceutically acceptable salt, solvate or stereoisomer thereof, which have CDK7 inhibitor properties, and also to a pharmaceutical composition comprising the present compounds, to methods of treating diseases or disorders and the use of the present compounds as pharmaceutical products for treating diseases or disorders.

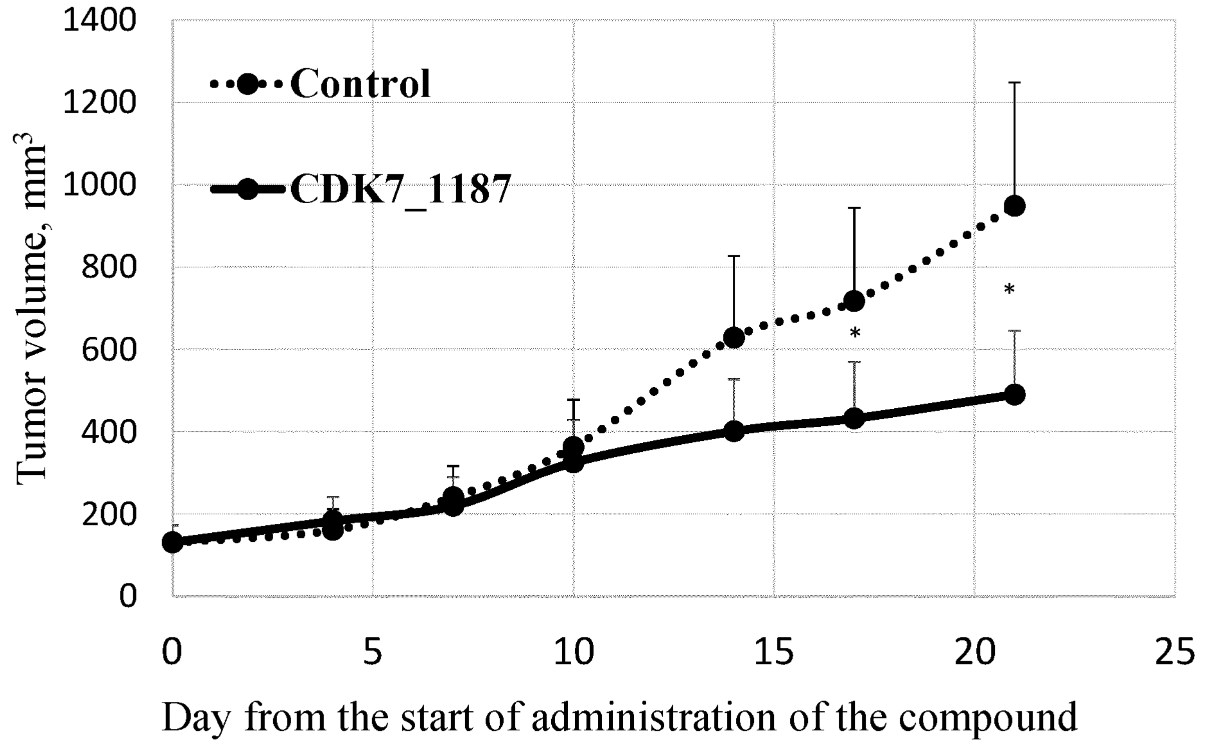


Fig.1

## Description

### Field of the invention

The present invention relates to novel inhibitors of cyclin-dependent protein kinases 7 (CDK7), to pharmaceutically acceptable salts, solvates or stereoisomers thereof, to pharmaceutical compositions comprising the present compounds, to methods for treating diseases or disorders and use of the present compounds as pharmaceutical products for treating diseases or disorders.

### Background of the invention

Cyclin-dependent kinase 7 (CDK7) is an enzyme ubiquitously expressed in diverse tissues of the body; it is a member of the cyclin-dependent protein kinase (CDK) family. CDK7, along with cyclin H and MAT1, forms the CDK-activating complex, which regulates progression of cell cycle phases via phosphorylation of other CDKs. CDK7 is a mandatory component of the transcription factor, TFIIH. The latter is involved in transcription initiation and DNA repair processes. Thus, CDK7 plays a crucial role in the regulation of the cell cycle and gene transcription (Ganuza et al. Genetic inactivation of Cdk7 leads to cell cycle arrest and induces premature aging due to adult stem cell exhaustion. EMBO J. 2012;31(11):2498-510; Larochelle et al. Cyclin-dependent kinase control of the initiation-to-elongation switch of RNA polymerase II. Nat Struct Mol Biol. 2012;19(11):1108-15).

Uncontrolled proliferation and changes in transcription processes are properties characteristic of malignant tumor cells. Excessive activation of CDK7 has been observed in many cancer types, and correlates with an aggressive course of the disease and a poor prognosis to a patient, such as, for example, stomach cancer, squamous cell carcinoma of the esophagus, ER+ and triple negative breast cancer, hepatocellular carcinoma (Liu et al. Cyclin-dependent kinase 7 (CDK7) expression in human hepatocellular carcinoma: association with HCC progression, prognosis and cell proliferative capacity. Transl Cancer Res. 2018;7(3):472-479; Wang et al. Upregulation of CDK7 in gastric cancer cell promotes tumor cell proliferation and predicts poor prognosis. Exp Mol Pathol. 2016;100(3):514-21; Patel et al. Expression of CDK7, Cyclin H, and MAT1 Is Elevated in Breast Cancer and Is Prognostic in Estrogen Receptor-Positive Breast Cancer. Clin Cancer Res. 2016;22(23):5929-5938; Li et al. Therapeutic Rationale to Target Highly Expressed CDK7 Conferring Poor Outcomes in Triple-Negative Breast Cancer. Cancer Res. 2017;77(14):3834-3845; Zhang et al. Low expression of cyclinH and cyclin-dependent kinase 7 can decrease the proliferation of human esophageal squamous cell carcinoma. Dig Dis Sci. 2013;58(7):2028-37). Inhibition of CDK7 can have an antitumor effect by means of reduced rate of cell proliferation, as well as by affecting the transcription of genes involved in the malignant transformation of cells, such as cells of ovarian cancer, T-cell acute lymphoblastic leukemia, neuroblastoma, glioma, small cell lung cancer and others (Chipumuro et al. CDK7 inhibition suppresses super-enhancer-linked oncogenic transcription in MYCN-driven cancer. Cell. 2014;159(5):1126-1139, Kwiatkowski et al. Targeting transcription regulation in cancer with a covalent CDK7 inhibitor. Nature. 2014;511(7511):616-20, Greenall at al. Cyclin-dependent kinase 7 is a therapeutic target in high-grade glioma. Oncogenesis. 2017;6(5):e336, Christensen et al. Targeting transcriptional addictions in small cell lung cancer with a covalent CDK7 inhibitor. Cancer Cell. 2014 Dec 8;26(6):909-922). Furthermore, protein inhibition potentiates genome instability and reduces PD-L1 expression; it triggers anti-tumor immune responses (Zhang et al. CDK7 Inhibition Potentiates Genome Instability Triggering Anti-tumor Immunity in Small Cell Lung Cancer. Cancer Cell 2020;37(1):37-54.e9, Wang et al. CDK7 inhibitor THZ1 enhances antiPD-1 therapy efficacy via the p38$\alpha$/MYC/PD-L1 signaling in non-small cell lung cancer. J Hematol Oncol. 2020;13(1):99).

Thus, there is a need to create novel compounds that preferably target CDK7 and can provide for a better risk-benefit ratio in the therapy of diseases associated with increased CDK7 activity.

### Description of the invention

The terms used in the description of this invention appear below.

Optionally substituted in one, two, three, four or several positions means the specified group can be substituted by a radical or any combination of radicals in one, two, three, four or from one to six positions.

An "annelated compound" or "condensed compound" is a polycyclic compound that has two neighboring atoms in common. Examples of condensed compounds include, but are not limited to, naphthalene, quinoline, tetrahydroquinoline, isoquinoline, quinoxaline, 1,2,3,4-tetrahydroquinoxaline, 1,2-dihydroquinoline, indole, dihydroindole, 1H-pyrrolo[2,3-b]pyridine, 1H,6H,7H-pyrrolo[2,3-c]pyridine 1H,4H,5H,6H,7H-pyrrolo[2,3-c]pyridine, 1H,4H,5H,6H,7H,8H-pyrrolo[2,3-c]azepine, 1H,6H,7H-pyrrolo[2,3-c]pyridine, 1H,4H,7H,8H-pyrrolo[2,3-c]azepine, 1H,6H,7H,8H-pyrrolo[2,3-c]azepine, 1H,8H-pyrrolo[2,3-c]azepine, 7H,8H-pyrrolo[2,3-c]azepine, 1H,4H,5H,6H,7H,8H,9H-pyrrolo[2,3-c]azocine, 1H,4H,5H,8H,9H-pyrrolo[2,3-c]azocine, 1H,4H,7H,8H,9H-pyrrolo[2,3-c]azocine, 1H,6H,7H,8H,9H-pyrrolo[2,3-c]azocine, 1H,8H,9H-pyrrolo[2,3-c]azocine, 9H-pyrrolo[2,3-c]azocine, 1H,4H,5H,6H-pyrrolo[2,3-c]pyrrole, 1H-1,3-benzodiazole, azaspiro[3.5]nonan, azaspiro[2.5]octane, 1H,2H,3H,4H,5H,6H-pyrrolo[2,3-f][1,4]oxazepine, 3,4,6,13-tetraazatricyclo[8.3.0.0$^{2}$,$^{6}$]trideka-1(10),2,4,11- tetraen-11-yl, 1,4-diazatricyclo[7.1.1.0$^{3}$,$^{7}$]]undeca-3(7),5-dienyl.

"Alkyl" means an aliphatic straight chain or branched chain hydrocarbon group having from 1 to 12 carbon atoms, more preferably from 1 to 6 carbon atoms. Branched chain means alkyl chain having one or more "lower alkyl" substituents. Examples of alkyl groups include, but are not limited to, methyl, ethyl, n-propyl, iso-propyl, n-butyl, isobutyl, sec-butyl, tert-butyl, n-pentyl, 2-pentyl, 3-pentyl, neopentyl, n-hexyl. Alkyl may have substituents which may be same or different structure.

"Cycloalkyl" means a fully saturated carbocyclic ring that contains from 3 to 10 carbon ring atoms. Cycloalkyl may have substituents which may be same or different structure. Cycloalkyl may be annelated with aril, heteroaryl, heterocyclyl. Examples of cycloalkyl groups include, but are not limited to, monocyclic groups, such as cyclopropyl, cyclobutyl, cyclopentyl, cyclohexyl, cycloheptyl, cyclooctyl, cyclononyl or cyclodecyl, bicyclic groups, such as bicycloheptyl or bicyclooctyl.

"Alkenyl" means a straight chain or branched chain hydrocarbon group having from 2 to 12 carbon atoms, more preferably from 2 to 6 carbon atoms that contains one or more carbon-carbon double bound. Alkenyl may have substituents which may be same or different structure.

"Aryl" means an aromatic monocyclic or polycyclic system having from 6 to 14 carbon atoms, more preferably from 6 to 10 carbon atoms. Examples of aryl groups include, but are not limited to, phenyl, phenylene, benzenetriyl, indanyl, naphthyl, naphthylene, naphthalenetriyl and anthrylene. Aryl may have substituents which may be same or different structure. Aryl can be annelated with a heterocycle, cycloalkyl, or heteroaryl.

"Alkyloxy", "Alkoxy" or "alkyloxy group" means an alkyl-O- group, wherein alkyl is defined in this section. Examples of alkoxy groups include, but are not limited to, methoxy, ethoxy, n-propoxy, iso-propoxy, n-butoxy, tert-butoxy, iso-butoxy.

"Amino group" means R'R"N-group.

"Aminocarbonyl" means -C(=O)NR‴R⁗ group.

Examples of R', R", R‴, R⁗ include, but are not limited to, substituents selected from the group comprising hydrogen, alkyl, alkenyl, alkynyl, cycloalkyl, aryl, heterocyclyl, heteroaryl, heteroalkyl or R' and R" together with the carbon atom they are attached to, can form 4-7-membered heterocyclyl or heteroaryl.

"Alkylsulfonyl" (-S(O)$_2$-($C_1$-$C_6$)alkyl) means "alkyl" or "cycloalkyl", as defined above, linked to the corresponding fragment of a molecule via a sulfonyl group, -$SO_2$-. Examples of alkylsulfonyls include, but are not limited to, methylsulfonyl, ethylsulfonyl, propylsulfonyl, cyclopropylsulfonyl, etc.

"Alkylsulfonamide" (-NH-S(O)$_2$-($C_1$-$C_6$)alkyl) means "alkyl" or "cycloalkyl", as defined above, linked to the corresponding fragment of a molecule via a sulfonamide group, -NH-$SO_2$-. Examples of alkylsulfonamides include, but are not limited to, methylsulfonamide, ethyl sulfonamide, propylsulfonamide, cyclopropylsulfonamide, etc.

"Dialkylphosphine oxide" or "dialkylphosphoryl" (-P(O)(($C_1$-$C_6$)alkyl)$_2$) means "alkyl" or "cycloalkyl" as defined above, linked to the corresponding fragment of the molecule via a phosphoryl group. Examples of dialkylphosphine oxide include, but are not limited to, dimethylphosphine oxide, diethylphosphine oxide, methylethylphosphine oxide, dipropylphosphine oxide, dicyclopropylphosphine oxide, etc.

The term "oxo" as used herein relates to the radical =O.

"Lower alkyl" means a straight chain or branched chain alkyl having from 1 to 4 carbon atoms.

"Halo" or "Halogen" (Hal) means fluoro, chloro, bromo and iodo.

"Heterocycle", "heterocyclyl", "heterocyclic ring" means a monocyclic or polycyclic system having from 3 to 11 carbon atoms, of which one or more carbon atoms are substituted by a heteroatom, such as nitrogen, oxygen, sulfur. Heterocycle may have one or more substituents which may be same or different structure. Nitrogen and sulfur atoms of heterocycle could be oxidized to N-oxide, S-oxide or S-dioxide. Heterocycle may be saturated, partially saturated or unsaturated. A heterocyclyl may be represented by a bridged-ring compound with one, two or three -$CH_2$-groups; or it may be annelated with a 4-5-membered heteroaryl, wherein one, two or three carbon atoms are substituted by nitrogen atoms. Heterocycle may be annelated with cycloalkyl, aryl or heteroaryl. Examples of heterocycles include, but are not limited to, azetidine, pyrrolidine, piperidine, 2,8-diazaspiro[4.5]decane, piperazine, morpholine, diazepan, azepan, azabicycloheptan, oxane, 1H,4H,5H,6H,7H,8H,9H-pyrrolo[2,3-c]azocine, 1H,4H,5H,6H-pyrrolo[2,3-c]pyrrole, 1,2,3,4-tetrahydroquinoxaline, tetrahydroquinoline, 1,2-dihydroquinoline, dihydroindole, azaspiro[3.5]nonan, azaspiro[2.5]octane, 5H,6H,7H,8H,9H-[1,2,4]triazolo[4,3-a]azepino, 5H,6H,7H-[1,2,4]triazolo[4,3-a]azepino, 1-azabicyclo[4.1.1]octano, 1-azabicyclo[4.1.1]oct-3-enylo, etc.

"Heteroaryl", "heteroarylyl", "heteroaryl ring" means an aromatic monocyclic or polycyclic system having from 5 to 11 carbon atoms, preferably from 5 to 10, of which one or more carbon atoms are substituted by a heteroatom, such as nitrogen, sulfur or oxygen. Nitrogen atom of heterocycle could be oxidized to N-oxide. Heteroaryl may have one or more substituents which may be same or different structure. Heteroaryl may be annelated with cycloalkyl, aryl or heterocycle. Examples of heteroaryls include, but are not limited to, 1H-pyrrolo[2,3-b]pyridine, 7H-pyrrolo[2,3-d]pyrimidine, pyrrole, furan, pyridine, pyrazine, pyrimidine, pyridazine, isooxazole, isothiazole, tetrazole, oxazole, 1,2,4-oxadiazole, thiazole,

pyrazole, furazan, 1,2,4-triazole, 1,2,3-triazole, 1,2,4-thiadiazole, 2H-1,2,3,4-tetrazole quinoxaline, imidazo[1,2-a]pyridine, indole, benzimidazole, quinoline, imidazole, thienopyridine, quinazoline, naphthyridine, thienopyrimidine, imidazopyridine, quinoline, isoquinoline, quinoxaline, indole, 1H -pyrrolo[2,3-b]pyridine, 1H,6H,7H-pyrrolo[2,3-c]pyridine 1H,4H,5H,6H,7H-pyrrolo[2,3-c]pyridine, 1H,4H,5H,6H,7H,8H-pyrrolo[2,3-c]azepine, 1H,6H,7H-pyrrolo[2,3-c]pyridine, 1H,4H,7H,8H-pyrrolo[2,3-c]azepine, 1H,6H,7H,8H-pyrrolo[2,3-c]azepine, 1H,8H-pyrrolo[2,3-c]azepine, 7H,8H-pyrrolo [2,3-c]azepine, 1H,4H,5H,6H,7H,8H,9H-pyrrolo[2,3-c]azocine, 1H,4H,5H,8H,9H-pyrrolo[2,3-c]azocine, 1H,4H,7H,8H,9H-pyrrolo[2,3-c]azocine, 1H,6H,7H,8H,9H-pyrrolo[2,3-c]azocine, 1H,8H,9H-pyrrolo[2,3-c]azocine, 9H-pyrrolo[2,3-c]azocine, 1H,4H,5H,6H-pyrrolo[2,3-c]pyrrole, 1H-1,3-benzodiazole, 1H,2H,3H,4H,5H,6H-pyrrolo[2,3-f][1,4]oxazepine, 3,4,6,13-tetraazatricyclo[8.3.0.$0^{2,6}$]trideka-1(10),2,4,11- tetraenyl, 1,4-diazatricyclo[7.1.1.$0^{3,7}$]undeca-3(7),5-dienyl, and others.

"Substituent" means a chemical radical attached to a scaffold (fragment).

"Solvate" is a molecular aggregate that consists of the compound of the present invention, including its pharmaceutically acceptable salts, with one or more solvent molecules. The solvent molecules are molecules of common pharmaceutical solvents, known to be safe for recipients, e.g. water, ethanol, ethylene glycol, and the like. Other solvents, such as MeOH, methyl-tert-butyl ether, ethyl acetate, methyl acetate, (S)-propylene glycol, (R)-propylene glycol, 1,4-butanediol, and the like, can be used as intermediate solvates for obtaining more desirable solvates.

The term "hydrate" refers to a complex, wherein the solvent molecule is water.

Solvates and/or hydrates preferably exist in crystalline form.

The terms "bond", "chemical bond", or "single bond" refer to a chemical bond of two atoms or two moieties (i.e. groups, fragments) when the atoms joined by the bond are considered to be part of larger substructure.

A chemical bond in the compounds of the present invention shown as " ═ " means a double bond, wherein the configuration of substituents corresponds to the formula.

A chemical bond in the compounds of the present invention shown as "  " means a double bond which may have both *cis*- and *trans*-configurations.

The term "stereoisomers" refers to compounds that have identical chemical composition and the same structure, but differ in the spatial arrangement of atoms or groups. Stereoisomers may include geometric isomers, enantiomers, diastereomers.

The term "standard treatment" includes extraction, processing with solutions of acids, alkalis and/or salts to neutralize or otherwise alter pH and/or remove interferents; filtration through Celite, silica gel and other materials; addition of an inert carrier (celite, silica gel); removal of volatile components and solvents in vacuo.

The term "excipient" is used herein to describe any ingredient other than the compound(s) of the invention.

"Pharmaceutical composition" means a composition comprising a compound of the invention and at least one excipient. The excipient may be selected from a group consisting of pharmaceutically acceptable and pharmacologically compatible fillers, solvents, diluents, carriers, auxiliary, distributing and sensing agents, delivery agents, such as preservatives, stabilizers, fillers, disintegrators, moisteners, emulsifiers, suspending agents, thickeners, sweeteners, flavouring agents, aromatizing agents, antibacterial agents, fungicides, lubricants, and prolonged delivery controllers, the choice and proportions of which depend on the nature and route of administration and dosage. Examples of suspending agents are ethoxylated isostearyl alcohol, polyoxyethene, sorbitol and sorbitol ether, microcrystalline cellulose, aluminum metahydroxide, bentonite, agar-agar and tragacant and mixtures thereof as well. Protection against action of microorganisms can be provided by various antibacterial and antifungal agents, such as, for example, parabens, chlorobutanole, sorbic acid, and similar compounds. The composition may also contain isotonic agents, for example, sugars, sodium chloride, and similar compounds. Prolonged action of the composition may be achieved by agents slowing down absorption of active ingredient, for example, aluminum monostearate and gelatine. Examples of suitable carriers, solvents, diluents and delivery agents are water, ethanol, polyalcohols and mixtures thereof, plant oils (such as olive oil) and organic esters (such as ethyl oleate) for injections. Examples of fillers are lactose, milk sugar, sodium citrate, calcium carbonate, calcium phosphate, and the like. Examples of disintegrators and distributors are starch, alginic acid and its salts, silicates, and the like. Examples of lubricants are magnesium stearate, sodium lauryl sulfate, talc, and polyethylene glycol of high molecular weight as well. The pharmaceutical composition for peroral, sublingual, transdermal, intramuscular, intravenous, subcutaneous, local or rectal administration of active ingredient, alone or in combination with another active ingredient may be administered to animals and human in a standard administration form, in a mixture with traditional pharmaceutical carriers. Suitable standard administration forms include peroral forms such as tablets, gelatin capsules, pills, powders, granules, chewing gums and peroral solutions or suspensions; sublingual and transbuccal administration forms; aerosols; implants; local, transdermal, subcutaneous, intramuscular, intravenous, intranasal or intraocular administration forms and rectal administration forms.

"Pharmaceutically acceptable salt" means relatively nontoxic both organic and inorganic salts of acids and bases disclosed in the present invention. These salts may be prepared in situ in the processes of synthesis, isolation or purification of compounds or they may be prepared specially. In particular, salts of bases may be prepared specially from

purified base of the disclosed compound and suitable organic or inorganic acid. Examples of salts prepared in this manner are hydrochlorides, hydrobromides, sulfates, bisulfates, phosphates, nitrates, acetates, oxalates, valeriates, oleates, palmitates, stearates, laurates, borates, benzoates, lactates, tosilates, citrates, maleates, fumarates, succinates, tartrates, mesilates, malonates, salicylates, propionates, ethane sulphonates, benzene sulfonates, sulfamates and the like (Detailed description of such salts properties is given in: Berge S.M., et al., "Pharmaceutical Salts" J. Pharm. Sci. 1977, 66: 1-19). Salts of disclosed acids may be prepared by reaction of a purified acid with suitable base; furthermore, metal salts and amine salts may be synthesized as well. Metal salts are salts of sodium, potassium, calcium, barium, zinc, magnesium, lithium and aluminum; sodium and potassium salts are most preferred. Suitable inorganic bases from which metal salts may be prepared are: sodium hydroxide, carbonate, bicarbonate and hydride; potassium hydroxide and bicarbonate, lithium hydroxide, calcium hydroxide, magnesium hydroxide, zinc hydroxide. Organic bases from which salts of disclosed acids may be prepared are amines and amino acids, the basicity of which is sufficient enough to produce a stable salt, and which are suitable for use in medical purposes (in particular, they must have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using tetraalkylammonium hydroxides, such as holine, tetramethylammonium, tetraethylammonium, and the like. Basic aminoacids, i.e. lysine, ornithine and arginine, may be used as aminoacids.

"Medicinal product (drug product, medicament)" is a compound (or a mixture of compounds as a pharmaceutical composition) in the form of tablets, granules, capsules, injections, ointments and other ready forms intended for restoration, improvement or modification of physiological functions in humans and animals, as well as for treatment and prophylaxis of diseases, for diagnostics, anesthesia, contraception, cosmetology and others.

"Treat," "treatment," and "therapy" refer to a method of mitigating or eliminating a biological disorder and/or at least one of its accompanying symptoms. The term "to alleviate" a disease, disorder or condition means reducing the severity and/or occurrence frequency of the symptoms of the disease, disorder, or condition. Further, references herein to "treatment" include references to curative, palliative and prophylactic treatment.

"Prophylaxis", "prophylactic therapy" refers to a set of measures aimed at preventing the onset, eliminating risk factors, or early detecting a disease or disorder, its progression, worsening of the course, exacerbation, relapse, complications or other consequences.

In one aspect, the subject of prophylaxis or treatment, or patient, is a mammal, preferably a human subject. Said subject may be either male or female, of any age.

The term "disorder" or "disease" means any condition, the manifestations of which may benefit or subject's prognosis with that condition may benefit from treatment according to the present invention. The definition of the term includes chronic and acute disorders or diseases including those pathological conditions that predispose a mammal to the disorder in question. Non-limiting examples of diseases to be treated include an oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjogren's syndrome, systemic lupus erythematosus, sarcoidosis, ulcerative colitis, multiple sclerosis, myasthenia gravis and uveitis;

an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis,

and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);

an infectious inflammatory disease or non-infectious inflammatory disease selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

"Therapeutically effective amount" refers to that amount of the therapeutic agent being administered in the course of treatment which will relieve the severity or eliminate the symptoms of the disease being treated.

As used in the present description and claims that follow, unless otherwise dictated by the context, the words "have", "include," and "comprise" or variations thereof such as "has", "having," "includes", "including", "comprises," or "comprising," will be understood to imply the inclusion of a stated integer or group of integers but not the exclusion of any other integer or group of integers.

**Description of drawings.**

**Figure 1** illustrates the dynamics of tumor growth of NCI-H69 xenografts in the control group and in the group receiving compound **CDK7_1187** at a dose of 6 mg/kg QD for 21 days. Data are shown as arithmetic means of tumor volumes ± standard error of the mean.

Note: *- p<0.05; statistically significant difference from the control group (Mann-Whitney test).

**Detailed description of the invention**

In one embodiment, the present invention relates to a compound of Formula **I**:

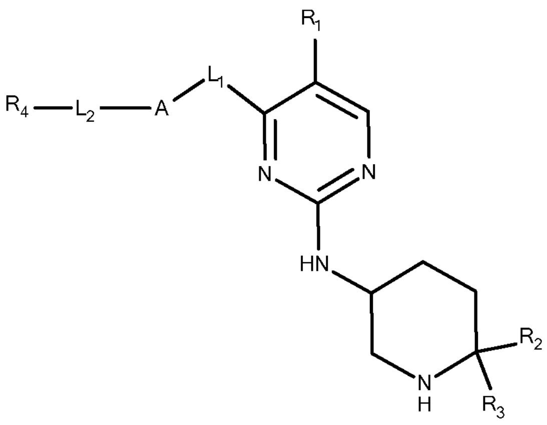

**I**

or a pharmaceutically acceptable salt, solvate or stereoisomer thereof,

$R_1$ is -C(Hal)$_3$, -CH(Hal)$_2$, -CH$_2$Hal, -Hal, -H, -NO$_2$, -(C$_1$-C$_6$)alkyl unsubstituted or substituted by one or several atoms of Hal, -O-(C$_1$-C$_6$)alkyl unsubstituted or substituted by one or several atoms of Hal, -S-(C$_1$-C$_6$)alkyl unsubstituted or substituted by one or several atoms of Hal;

$R_2$, $R_3$ are each independently H, -(C$_1$-C$_6$)alkyl;

or $R_2$, $R_3$ together form -($C_3$-$C_6$)cycloalkyl unsubstituted or substituted by one or several -($C_1$-$C_6$)alkyls;
$L_1$, $L_2$ are each independently a chemical bond, -$NR_5$-, -O-, -S-, -S(O)-, -S(O)$_2$-, -C(O)-, -C(O)O-, -C(S)-, -C(=NH)-, -($CR_{6a}R_{6b}$)$_{1-3}$-, -P(O)($CH_3$)-, -O-($CH_2$)$_{1-3}$-, -($CH_2$)$_{1-3}$-O-, -$NR_{5a}$-($CH_2$)$_{1-3}$-,-($CH_2$)$_{1-3}$-$NR_{5a}$-, -C(O)-($CH_2$)$_{1-3}$-, -($CH_2$)$_{1-3}$-C(O)-, -C(O)-NH- or -NH-C(O)-;
A is 6-10 membered aryl unsubstituted or substituted by one or several $R_7$; 6-10 membered aryl unsubstituted or substituted by one or several $R_{7a}$, fused with -($C_3$-$C_6$)cycloalkyl unsubstituted or substituted by one or several $R_{8a}$; 6-10 membered aryl unsubstituted or substituted by one or several $R_{7b}$, fused with 4-10 membered heterocyclyl with 1,2,3 or 4 heteroatoms selected from N, S or O, unsubstituted or substituted by one or several $R_{8b}$; 6-10 membered aryl unsubstituted or substituted by one or several $R_{7c}$, fused with 5-6 membered heteroaryl with 1,2,3 or 4 heteroatoms selected from N,O or S, unsubstituted or substituted by one or several $R_{8c}$; 5-6 membered heteroaryl with 1,2,3 or 4 atoms N, unsubstituted or substituted by one or several $R_{7d}$; 5-6 membered heteroaryl with 1,2,3 or 4 heteroatoms selected from N,O or S, unsubstituted or substituted by one or several $R_{7e}$, fused with ($C_3$-$C_6$)cycloalkyl unsubstituted or substituted by one or several $R_{8e}$; 5-6 membered heteroaryl with 1,2,3 or 4 heteroatoms selected from N,O or S, unsubstituted or substituted by one or several $R_{7f}$, fused with 4-10 membered heterocyclyl with 1,2,3 or 4 heteroatoms selected from N, S or O, unsubstituted or substituted by one or several $R_{8f}$; 5-6 membered heterocyclyl with 1,2,3 or 4 heteroatoms selected from N, S or O, unsubstituted or substituted by one or several $R_{7g}$; 5-6 membered heterocyclyl with 1,2,3 or 4 heteroatoms selected from N, S or O, unsubstituted or substituted by one or several $R_{7h}$, fused with 4-10 membered heterocyclyl with 1,2,3 or 4 heteroatoms selected from N, S or O, unsubstituted or substituted by one or several $R_{8h}$;
$R_4$ is -H -Hal, (=O), -OH, -C(O)OH, -$NR_{4a}R_{4b}$, -P(O)($C_1$-$C_6$ alkyl)$_2$, -S(O)$_2$NH$_2$, -S(O)$_2$$C_1$-$C_6$ alkyl, -($C_1$-$C_6$)alkyl unsubstituted or substituted by one or several $R_{4c}$; -O-$C_1$-$C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; -($C_3$-$C_6$)cycloalkyl unsubstituted or substituted by one or several $R_{4g}$; -$C_2$-$C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$; 5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;
$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH-NH$_2$, -Hal, -CN, -$C_1$-$C_6$ alkyl unsubstituted or substituted by one or several radicals selected from -Hal, -OH, -NH$_2$, -CN, phenyl; -O-$C_1$-$C_6$ alkyl unsubstituted or substituted by one or several radicals selected from -Hal, -OH, -NH$_2$, -CN, phenyl; -$C_3$-$C_6$ cycloalkyl unsubstituted or substituted by one or several radicals selected from -Hal, -OH, -NH$_2$, -CN, phenyl, -$C_1$-$C_6$ alkyl;-S(O)$_2$$C_1$-$C_6$ alkyl, -S(O)$_2$NH$_2$, -NH($C_1$-$C_6$ alkyl), -N($C_1$-$C_6$ alkyl)$_2$, -P(O)($C_1$-$C_6$ alkyl)$_2$, morpholinyl, thiazolyl, phenyl,
$R_5$, $R_{5a}$ are each independently -H, $C_1$-$C_6$ alkyl,
$R_{6a}$, $R_{6b}$ are each independently -H, $C_1$-$C_6$ alkyl, -Hal,
or $R_{6a}$ and $R_{6b}$ together with the C atom they are attached to form $C_3$-$C_6$ cycloalkyl unsubstituted or unsubstituted by several -($C_1$-$C_6$)alkyls;
$R_7$, $R_{7a}$, $R_{7b}$, $R_{7c}$, $R_{7d}$, $R_{7e}$ $R_{7f}$ $R_{7g}$ $R_{7h}$ are each independently -Hal, -OH, -NH$_2$, -CN, (=O), -($C_1$-$C_6$)alkyl unsubstituted or substituted by one or several -Hal; -O ($C_1$-$C_6$)alkyl unsubstituted or substituted by one or several -Hal;
$R_{8a}$, $R_{8b}$, $R_{8c}$, $R_{8d}$, $R_{8e}$ $R_{8f}$ $R_{8h}$ are each independently -Hal, -OH, -NH$_2$, -CN, (=O), -($C_1$-$C_6$)alkyl unsubstituted or substituted by one or several -Hal;
Hal is an atom of F, Cl, Br, I.

In one embodiment, the present invention relates to a compound of Formula I:

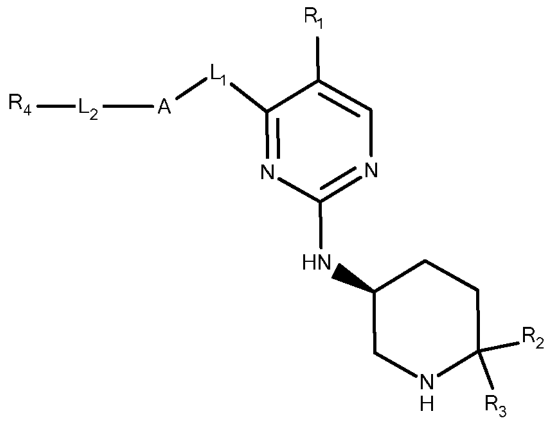

I.a

or a pharmaceutically acceptable salt, solvate or stereoisomer thereof,

$R_1$ is -C(Hal)$_3$, -CH(Hal)$_2$, -CH$_2$Hal, -Hal, -H, -NO$_2$, -($C_1$-$C_6$)alkyl unsubstituted or substituted by one or several atoms of Hal, -O-($C_1$-$C_6$)alkyl unsubstituted or substituted by one or several atoms of Hal, -S-($C_1$-$C_6$)alkyl unsubstituted or substituted by one or several atoms of Hal;

$R_2$, $R_3$ are each independently H, -($C_1$-$C_6$)alkyl;

or $R_2$, $R_3$ together form -($C_3$-$C_6$)cycloalkyl unsubstituted or substituted by one or several -($C_1$-$C_6$)alkyls;

$L_1$, $L_2$ are each independently a chemical bond, -NR$_5$-, -O-, -S-, -S(O)-, -S(O)$_2$-, -C(O)-, -C(O)O-, -C(S)-, -C(=NH)-, -(CR$_{6a}$R$_{6b}$)$_{1-3}$-, -P(O)(CH$_3$)-, -O-(CH$_2$)$_{1-3}$-, -(CH$_2$)$_{1-3}$-O-, -NR$_{5a}$-(CH$_2$)$_{1-3}$-,-(CH$_2$)$_{1-3}$-NR$_{5a}$-, -C(O)-(CH$_2$)$_{1-3}$-, -(CH$_2$)$_{1-3}$-C(O)-, -C(O)-NH- or -NH-C(O)-;

A is 6-10 membered aryl unsubstituted or substituted by one or several $R_7$; 6-10 membered aryl unsubstituted or substituted by one or several $R_{7a}$, fused with -($C_3$-$C_6$)cycloalkyl unsubstituted or substituted by one or several $R_{8a}$; 6-10 membered aryl unsubstituted or substituted by one or several $R_{7b}$, fused with 4-10 membered heterocyclyl with 1,2,3 or 4 heteroatoms selected from N, S or O, unsubstituted or substituted by one or several $R_{8b}$; 6-10 membered aryl unsubstituted or substituted by one or several $R_{7c}$, fused with 5-6 membered heteroaryl with 1,2,3 or 4 heteroatoms selected from N,O or S, unsubstituted or substituted by one or several $R_{8c}$; 5-6 membered heteroaryl with 1,2,3 or 4 atoms N, unsubstituted or substituted by one or several $R_{7d}$; 5-6 membered heteroaryl with 1,2,3 or 4 heteroatoms selected from N,O or S, unsubstituted or substituted by one or several $R_{7e}$, fused with ($C_3$-$C_6$)cycloalkyl unsubstituted or substituted by one or several $R_{8e}$; 5-6 membered heteroaryl with 1,2,3 or 4 heteroatoms selected from N,O or S, unsubstituted or substituted by one or several $R_{7f}$, fused with 4-10 membered heterocyclyl with 1,2,3 or 4 heteroatoms selected from N, S or O, unsubstituted or substituted by one or several $R_{8f}$; 5-6 membered heterocyclyl with 1,2,3 or 4 heteroatoms selected from N, S or O, unsubstituted or substituted by one or several $R_{7g}$; 5-6 membered heterocyclyl with 1,2,3 or 4 heteroatoms selected from N, S or O, unsubstituted or substituted by one or several $R_{7h}$, fused with 4-10 membered heterocyclyl with 1,2,3 or 4 heteroatoms selected from N, S or O, unsubstituted or substituted by one or several $R_{8h}$;

$R_4$ is -H -Hal, (=O), -OH, -C(O)OH, -NR$_{4a}$R$_{4b}$, -P(O)($C_1$-$C_6$ alkyl)$_2$, -S(O)$_2$NH$_2$, -S(O)$_2$$C_1$-$C_6$ alkyl, -($C_1$-$C_6$)alkyl unsubstituted or substituted by one or several $R_{4c}$; -O-$C_1$-$C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; -($C_3$-$C_6$)cycloalkyl unsubstituted or substituted by one or several $R_{4g}$; -$C_2$-$C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$; 5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH-NH$_2$, -Hal, -CN, -$C_1$-$C_6$ alkyl unsubstituted or substituted by one or several radicals selected from -Hal, -OH, -NH$_2$, -CN, phenyl; -O-$C_1$-$C_6$ alkyl unsubstituted or substituted by one or several radicals selected from -Hal, -OH, -NH$_2$, -CN, phenyl; -$C_3$-$C_6$ cycloalkyl unsubstituted or substituted by one or several radicals selected from -Hal, -OH, -NH$_2$, -CN, phenyl, -$C_1$-$C_6$ alkyl; - S(O)$_2$$C_1$-$C_6$ alkyl, -S(O)$_2$NH$_2$, -NH($C_1$-$C_6$ alkyl), -N($C_1$-$C_6$ alkyl)$_2$, -P(O)($C_1$-$C_6$ alkyl)$_2$, morpholinyl, thiazolyl, phenyl,

$R_5$, $R_{5a}$ are each independently -H, $C_1$-$C_6$ alkyl,

$R_{6a}$, $R_{6b}$ are each independently -H, $C_1$-$C_6$ alkyl, -Hal,

or $R_{6a}$ and $R_{6b}$ together with the C atom they are attached to form $C_3$-$C_6$ cycloalkyl unsubstituted or unsubstituted by

several -($C_1$-$C_6$)alkyls;
$R_7$, $R_{7a}$, $R_{7b}$, $R_{7c}$, $R_{7d}$, $R_{7e}$ $R_{7f}$ $R_{7g}$ $R_{7h}$ are each independently -Hal, -OH, -$NH_2$, -CN, (=O), -($C_1$-$C_6$)alkyl unsubstituted or substituted by one or several -Hal; -O ($C_1$-$C_6$)alkyl unsubstituted or substituted by one or several -Hal;
$R_{8a}$, $R_{8b}$, $R_{8c}$, $R_{8a}$, $R_{8e}$ $R_{8f}$ $R_{8h}$ are each independently -Hal, -OH, -$NH_2$, -CN, (=O), -($C_1$-$C_6$)alkyl unsubstituted or substituted by one or several -Hal;
Hal is an atom of F, Cl, Br, I.

In one embodiment, the present invention relates to a compound of Formula **I.b**:

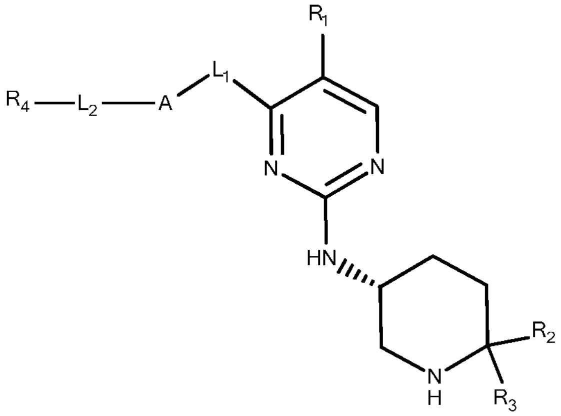

**I.b**

or a pharmaceutically acceptable salt, solvate or stereoisomer thereof,
$R_1$ is -C(Hal)$_3$, -CH(Hal)$_2$, -$CH_2$Hal, -Hal, -H, -$NO_2$, -($C_1$-$C_6$)alkyl unsubstituted or substituted by one or several atoms of Hal, -O-($C_1$-$C_6$)alkyl unsubstituted or substituted by one or several atoms of Hal, -S-($C_1$-$C_6$)alkyl unsubstituted or substituted by one or several atoms of Hal;
$R_2$, $R_3$ are each independently H, -($C_1$-$C_6$)alkyl;
or $R_2$, $R_3$ together form -($C_3$-$C_6$)cycloalkyl unsubstituted or substituted by one or several -($C_1$-$C_6$)alkyls;
$L_1$, $L_2$ are each independently a chemical bond, -$NR_5$-, -O-, -S-, -S(O)-, -S(O)$_2$-, -C(O)-, -C(O)O-, -C(S)-, -C(=NH)-, -($CR_{6a}R_{6b}$)$_{1-3}$-, -P(O)($CH_3$)-, -O-($CH_2$)$_{1-3}$-, -($CH_2$)$_{1-3}$-O-, -$NR_{5a}$-($CH_2$)$_{1-3}$-,-($CH_2$)$_{1-3}$-$NR_{5a}$-, -C(O)-($CH_2$)$_{1-3}$-, -($CH_2$)$_{1-3}$-C(O)-, -C(O)-NH- or -NH-C(O)-;
A is 6-10 membered aryl unsubstituted or substituted by one or several $R_7$; 6-10 membered aryl unsubstituted or substituted by one or several $R_{7a}$, fused with -($C_3$-$C_6$)cycloalkyl unsubstituted or substituted by one or several $R_{8a}$; 6-10 membered aryl unsubstituted or substituted by one or several $R_{7b}$, fused with 4-10 membered heterocyclyl with 1,2,3 or 4 heteroatoms selected from N, S or O, unsubstituted or substituted by one or several $R_{8b}$; 6-10 membered aryl unsubstituted or substituted by one or several $R_{7c}$, fused with 5-6 membered heteroaryl with 1,2,3 or 4 heteroatoms selected from N,O or S, unsubstituted or substituted by one or several $R_{8c}$; 5-6 membered heteroaryl with 1,2,3 or 4 atoms N, unsubstituted or substituted by one or several $R_{7d}$; 5-6 membered heteroaryl with 1,2,3 or 4 heteroatoms selected from N,O or S, unsubstituted or substituted by one or several $R_{7e}$, fused with ($C_3$-$C_6$)cycloalkyl unsubstituted or substituted by one or several $R_{8e}$; 5-6 membered heteroaryl with 1,2,3 or 4 heteroatoms selected from N,O or S, unsubstituted or substituted by one or several $R_{7f}$, fused with 4-10 membered heterocyclyl with 1,2,3 or 4 heteroatoms selected from N, S or O, unsubstituted or substituted by one or several $R_{8f}$; 5-6 membered heterocyclyl with 1,2,3 or 4 heteroatoms selected from N, S or O, unsubstituted or substituted by one or several $R_{7g}$; 5-6 membered heterocyclyl with 1,2,3 or 4 heteroatoms selected from N, S or O, unsubstituted or substituted by one or several $R_{7h}$, fused with 4-10 membered heterocyclyl with 1,2,3 or 4 heteroatoms selected from N, S or O, unsubstituted or substituted by one or several $R_{8h}$;
$R_4$ is -H -Hal, (=O), -OH, -C(O)OH, -$NR_{4a}R_{4b}$, -P(O)($C_1$-$C_6$ alkyl)$_2$, -S(O)$_2$NH$_2$, -S(O)$_2$C$_1$-C$_6$ alkyl, -($C_1$-$C_6$)alkyl unsubstituted or substituted by one or several $R_{4c}$; -O-$C_1$-$C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; -($C_3$-$C_6$)cycloalkyl unsubstituted or substituted by one or several $R_{4g}$; -$C_2$-$C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$; 5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH-$NH_2$, -Hal, -CN, -$C_1$-$C_6$ alkyl unsubstituted or substituted by one or several radicals selected from -Hal, -OH, -$NH_2$, -CN, phenyl; -O-$C_1$-$C_6$ alkyl unsubstituted or substituted by one or several radicals selected from -Hal, -OH, -$NH_2$, -CN, phenyl; -$C_3$-$C_6$ cycloalkyl unsubstituted or substituted by one or several radicals selected from -Hal, -OH, -$NH_2$, -CN, phenyl, -$C_1$-$C_6$ alkyl; - $S(O)_2C_1$-$C_6$ alkyl, -$S(O)_2NH_2$, -NH($C_1$-$C_6$ alkyl), -N($C_1$-$C_6$ alkyl)$_2$, -P(O)($C_1$-$C_6$ alkyl)$_2$, morpholinyl, thiazolyl, phenyl,

$R_5$, $R_{5a}$ are each independently -H, $C_1$-$C_6$ alkyl,

$R_{6a}$, $R_{6b}$ are each independently -H, $C_1$-$C_6$ alkyl, -Hal,

or $R_{6a}$ and $R_{6b}$ together with the C atom they are attached to form $C_3$-$C_6$ cycloalkyl unsubstituted or unsubstituted by several -($C_1$-$C_6$)alkyls;

$R_7$, $R_{7a}$, $R_{7b}$, $R_{7c}$, $R_{7d}$, $R_{7e}$ $R_{7f}$ $R_{7g}$ $R_{7h}$ are each independently -Hal, -OH, -$NH_2$, -CN, (=O), -($C_1$-$C_6$)alkyl unsubstituted or substituted by one or several -Hal; -O ($C_1$-$C_6$)alkyl unsubstituted or substituted by one or several -Hal;

$R_{8a}$, $R_{8b}$, $R_{8c}$, $R_{8d}$, $R_{8e}$ $R_{8f}$ $R_{8h}$ are each independently -Hal, -OH, -$NH_2$, -CN, (=O), -($C_1$-$C_6$)alkyl unsubstituted or substituted by one or several -Hal;

Hal is an atom of F, Cl, Br, I.

In one embodiment, the present invention relates to the S-isomer of the compound of formula **I.**

In one embodiment, the present invention relates to a compound, wherein -A-$L_2$-$R_4$ is:

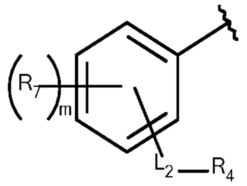
,
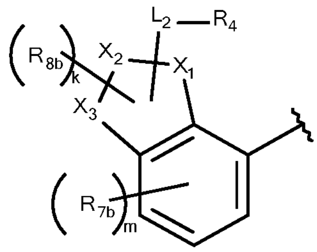
,
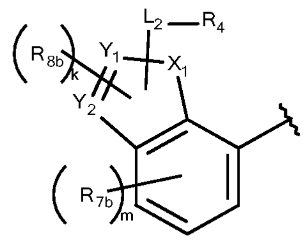
,
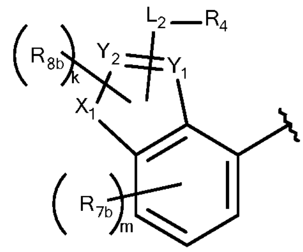
,

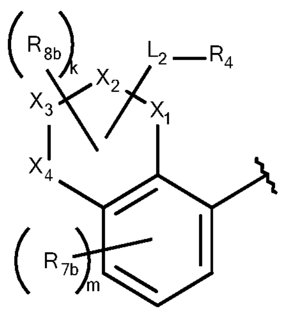
,
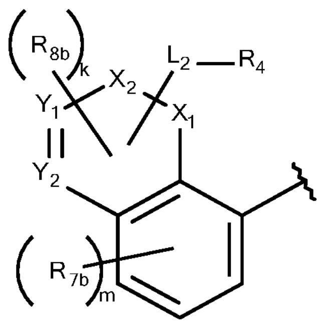
,
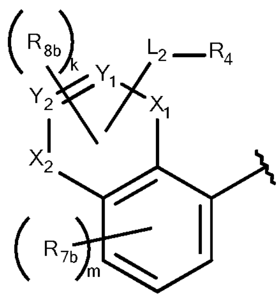
,
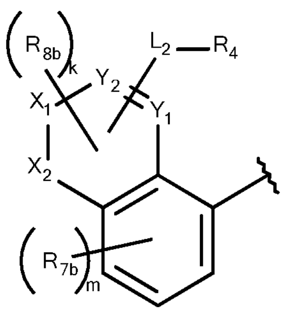
,

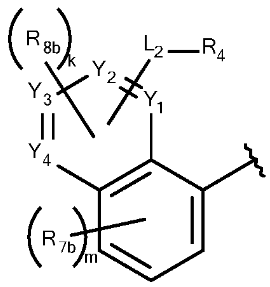
,
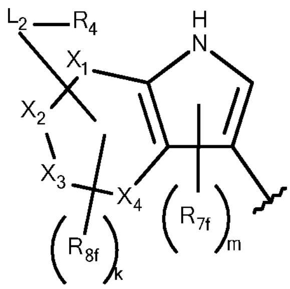
,
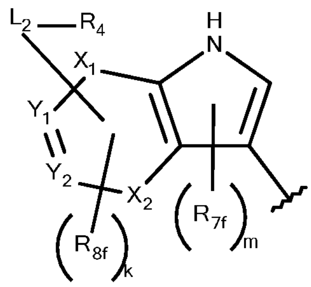
,
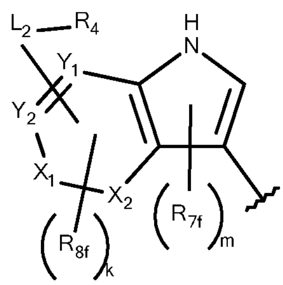
,

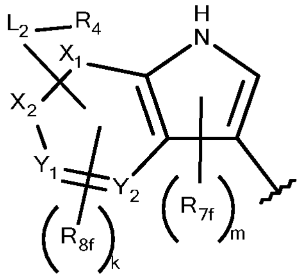 , 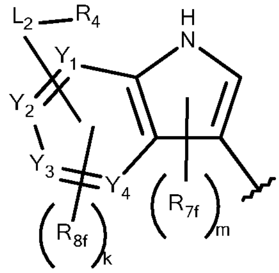 , 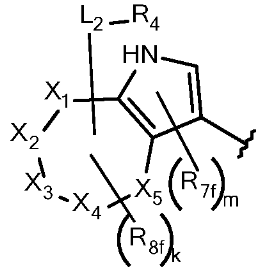 , 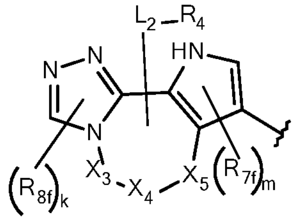 ,

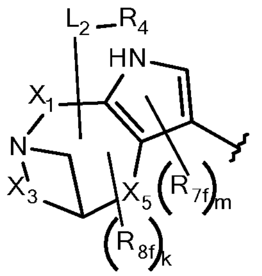 , 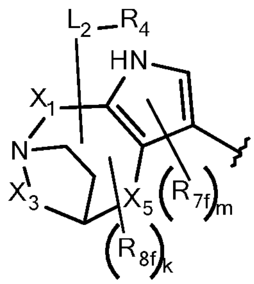 , 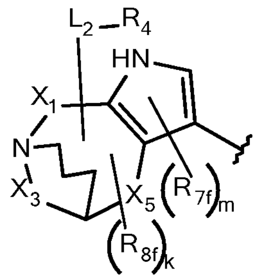 , 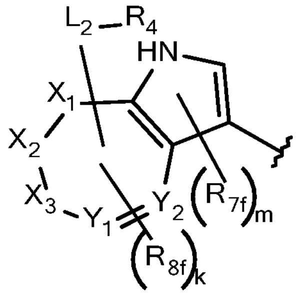 ,

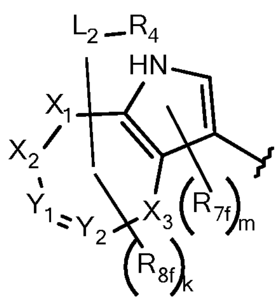 , 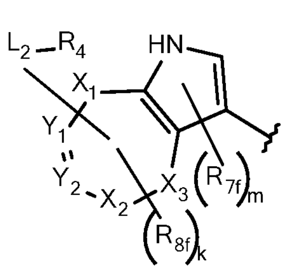 , 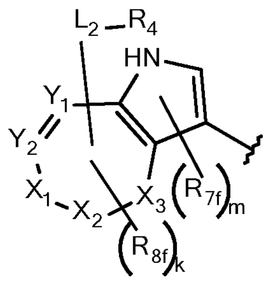 , 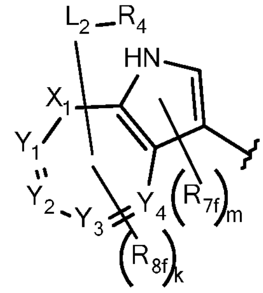 ,

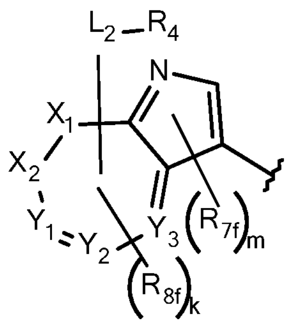 , 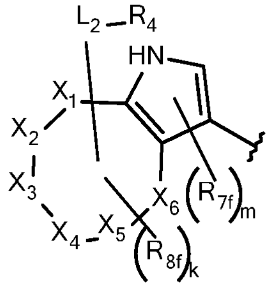 , 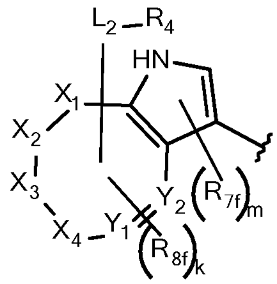 , 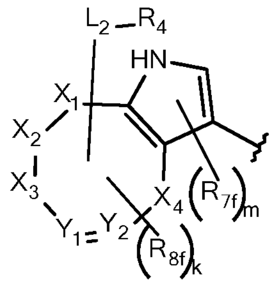 ,

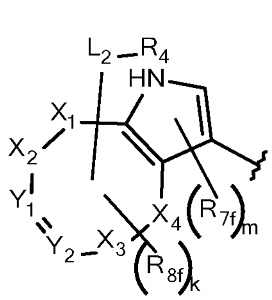 , 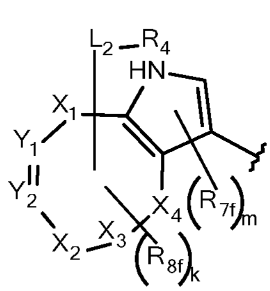 , 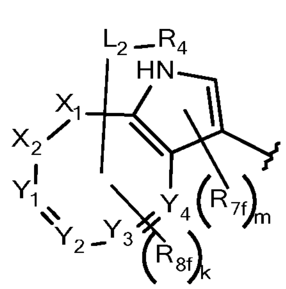 , 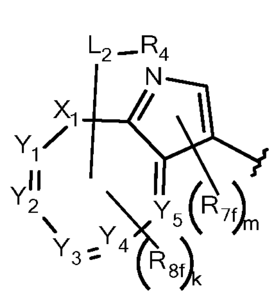 ,

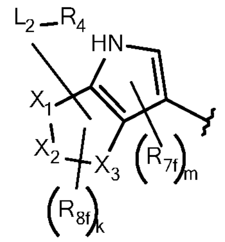, 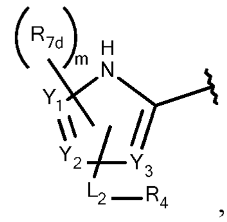, 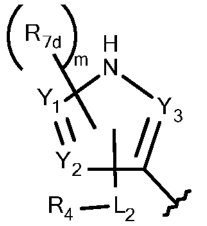, 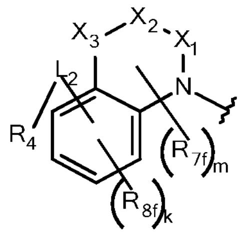, 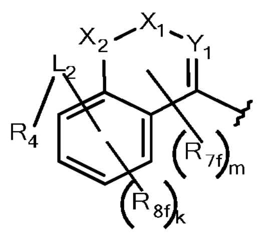,

,

wherein k,m are each independently 0, 1,2 or 3,

$X_1$, $X_2$, $X_3$, $X_4$, $X_5$, $X_6$ are each independently an atom of C, an atom of N, CH, $CH_2$, NH, S or O; $Y_1$, $Y_2$, $Y_3$, $Y_4$, $Y_5$ are each independently an atom of C, an atom of N or CH;

$L_2$ is a chemical bond, $-NR_5-$, -O-, -S-, -S(O)-, $-S(O)_2-$, -C(O)-, -C(O)O-, -C(S)-, -C(=NH)-, - $(CR_{6a}R_{6b})_{1-3}-$, -P(O)$(CH_3)$-, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;

$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH-$NH_2$, -Hal, -CN, $-C_1-C_6$ alkyl unsubstituted or substituted by one or several radicals selected from -Hal, -OH, $-NH_2$, -CN, phenyl; $-O-C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl, $-C_1-C_6$ alkyl;$-S(O)_2C_1-C_6$ alkyl, $-S(O)_2NH_2$, $-NH(C_1-C_6\ alkyl)$, $-N(C_1-C_6\ alkyl)_2$, $-P(O)(C_1-C_6\ alkyl)_2$, morpholinyl, thiazolyl, phenyl,

$R_5$, $R_{5a}$ are each independently -H, $C_1-C_6$ alkyl,

$R_{6a}$, $R_{6b}$ are each independently -H, $C_1-C_6$ alkyl, -Hal,

or $R_{6a}$ and $R_{6b}$ together with the C atom they are attached to form $C_3-C_6$ cycloalkyl unsubstituted or unsubstituted by several $-(C_1-C_6)$alkyls;

$R_7$, $R_{7b}$, $R_{7d}$, $R_{7f}$, are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;

$R_{8b}$, $R_{8d}$, $R_{8f}$ are each independently -Hal, -OH, $-NH_2$, -CN, (=O), $-(C_1-C_6)$alkyl unsubstituted or substituted by one or several -Hal;

Hal is an atom of F, Cl, Br, I.

In one embodiment, the present invention relates to a compound, wherein $-A-L_2-R_4$ is:

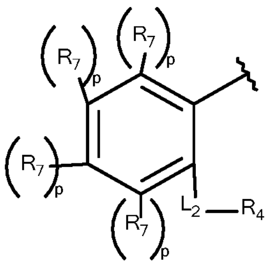, 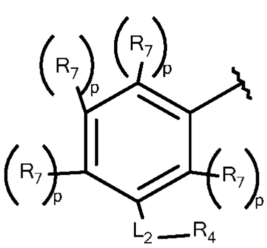, 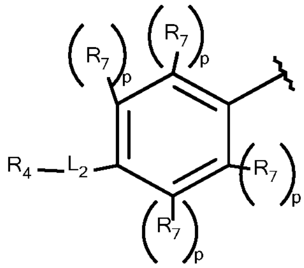, 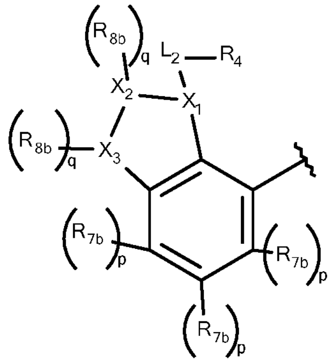,

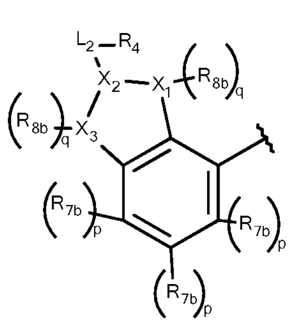
,
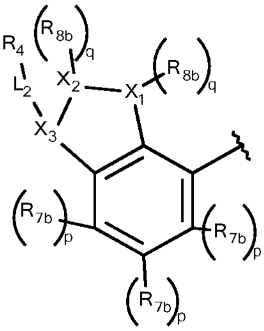
,
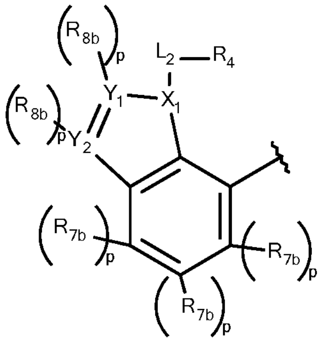
,
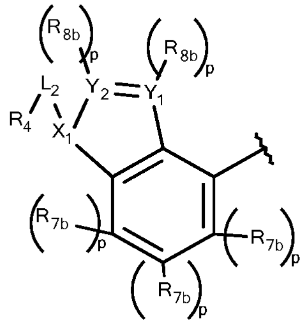
,
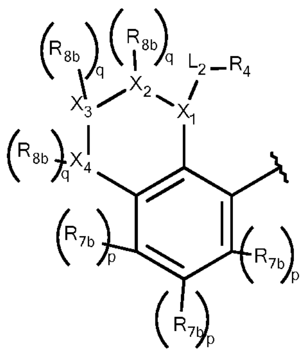
,
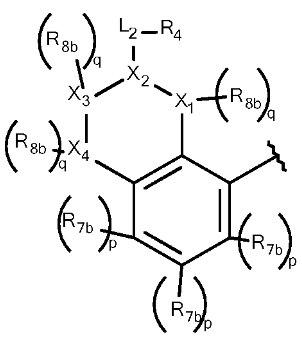
,
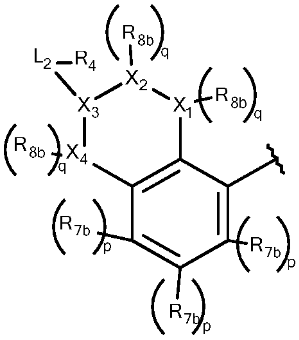
,
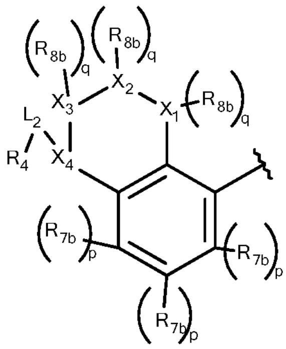
,
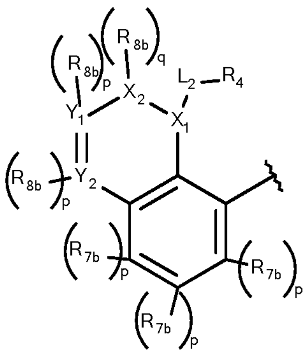
,
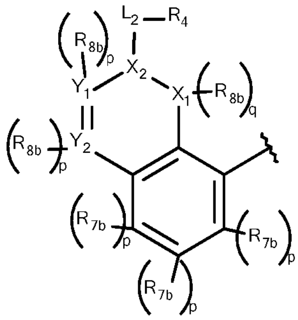
,
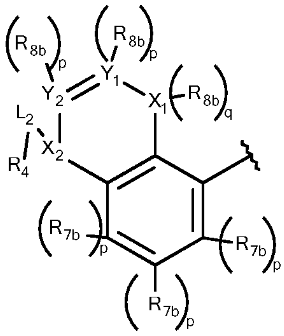
,
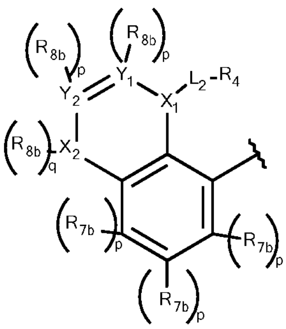
,
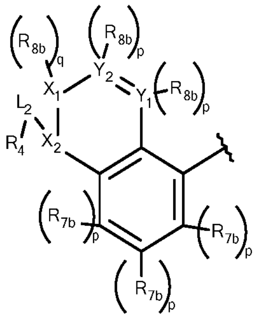
,
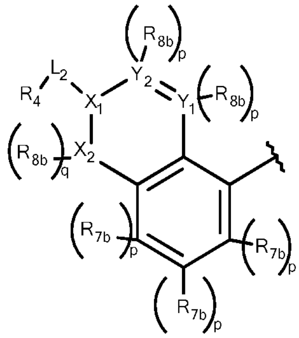
,
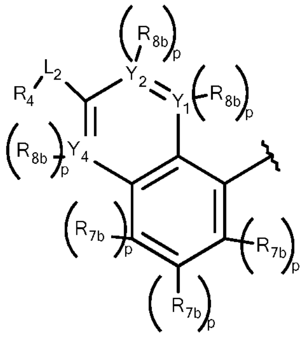
,
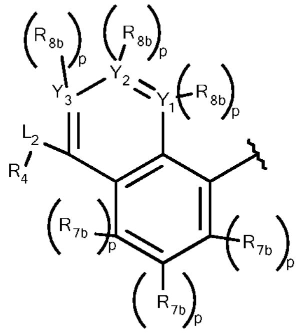
,

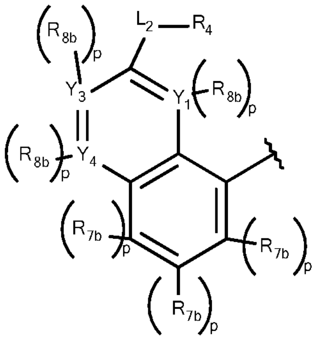,
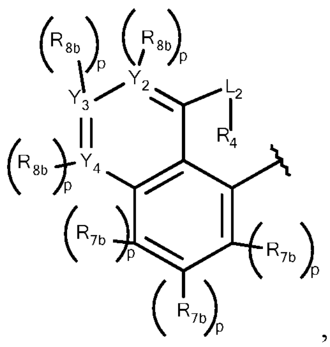,
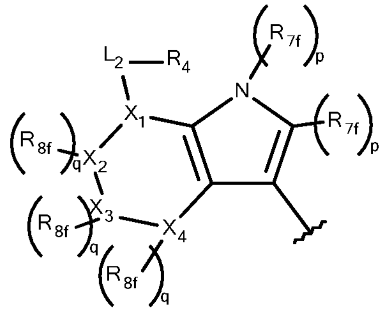,
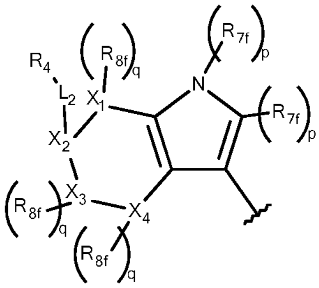,
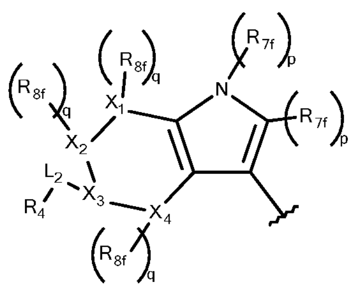,
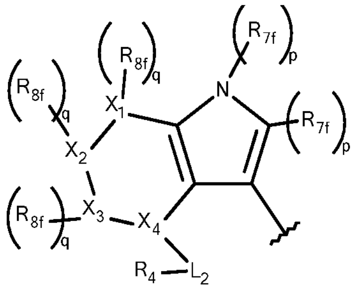,
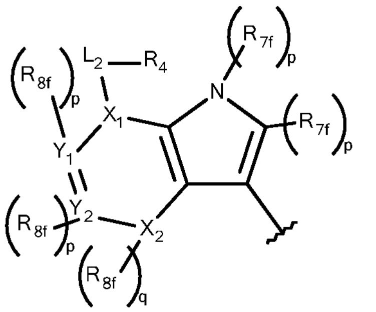,
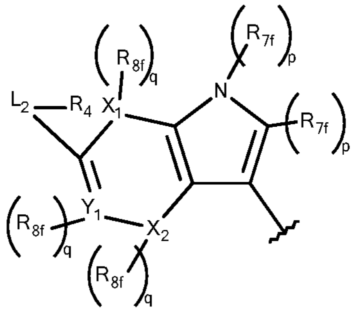,
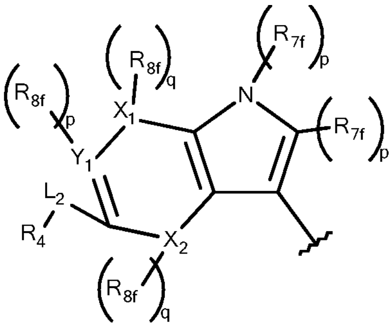,
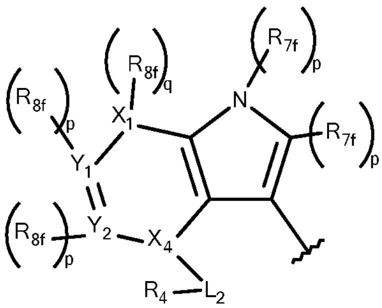,
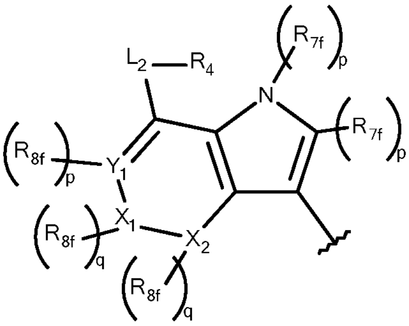,
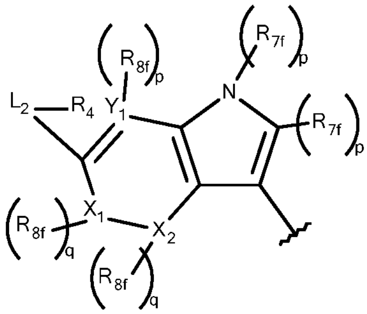,

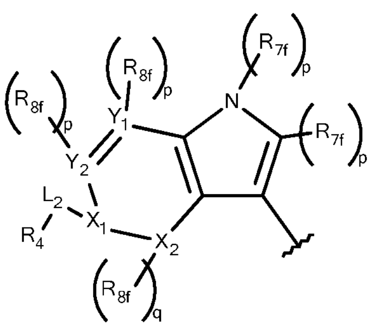 , 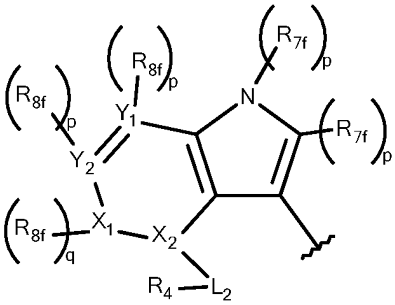 , 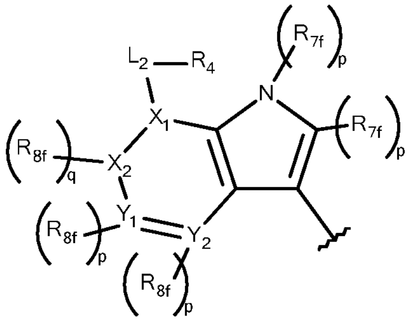 ,

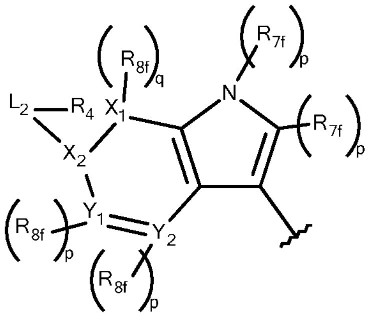 , 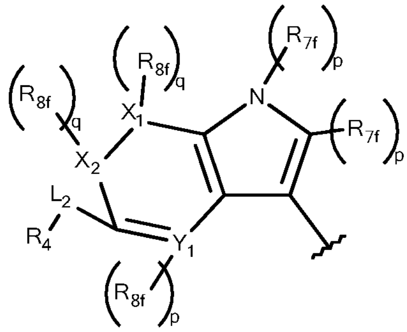 , 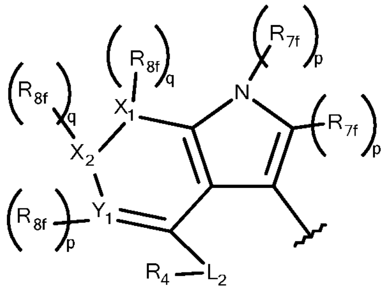 ,

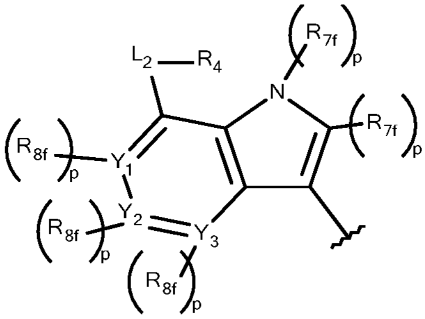 , 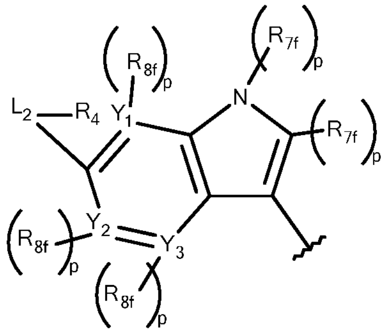 , 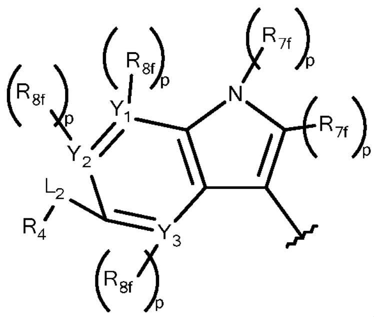 ,

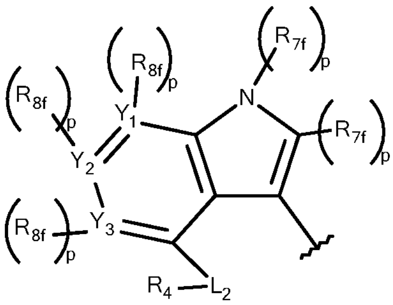 , 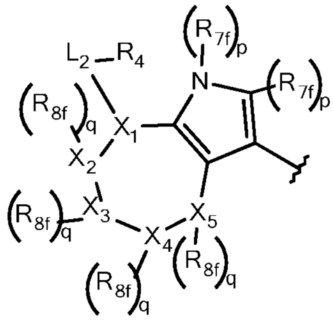 , 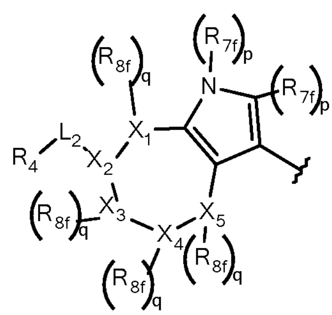 ,

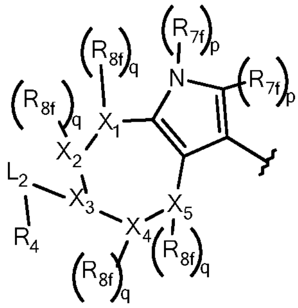,
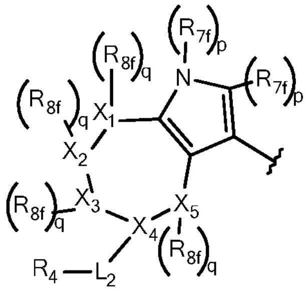,
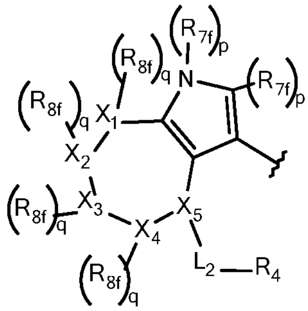,
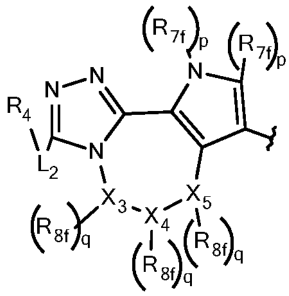,
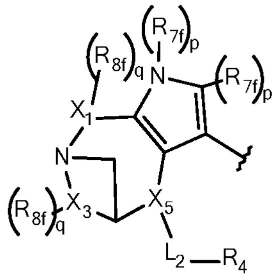,
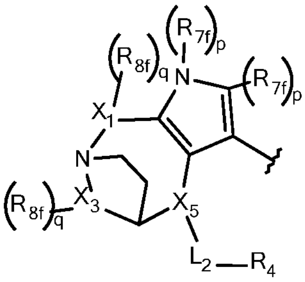,
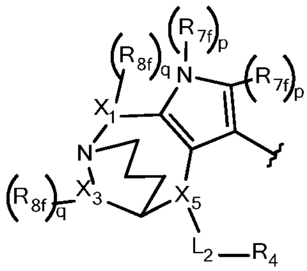,
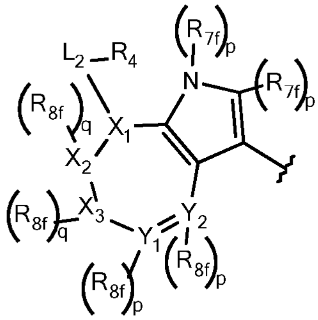,
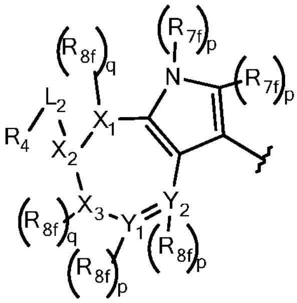,
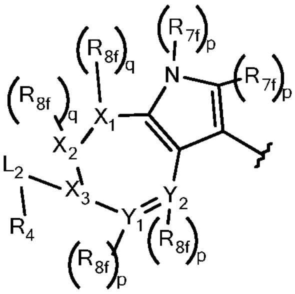,
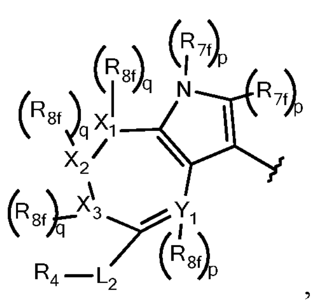,
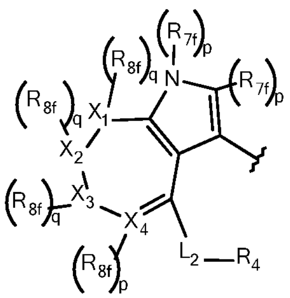,
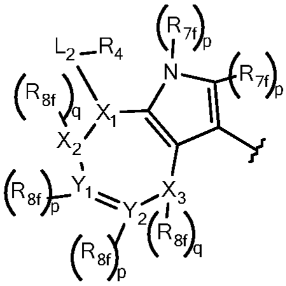,
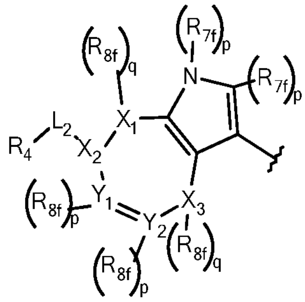,
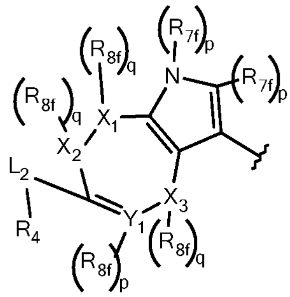,
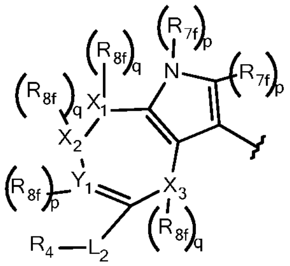,
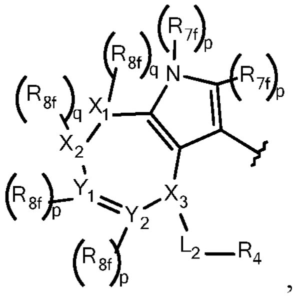,
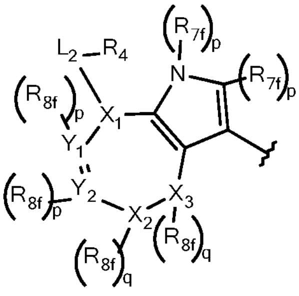,
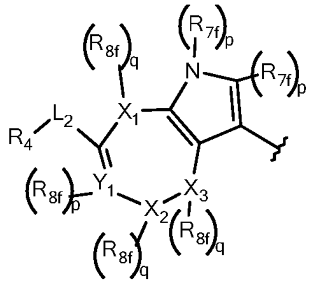,

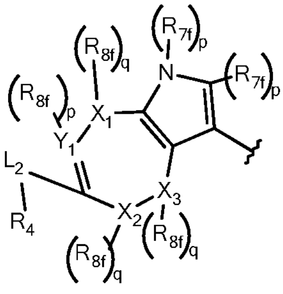,
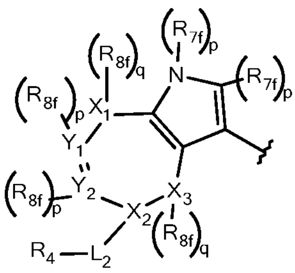,
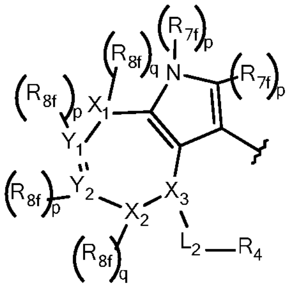,
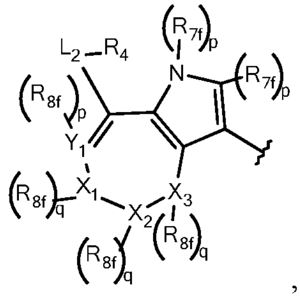,

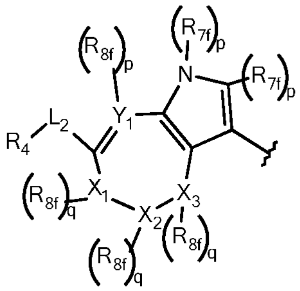,
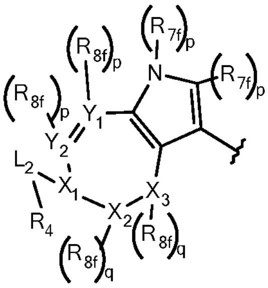,
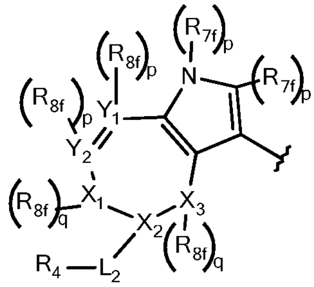,

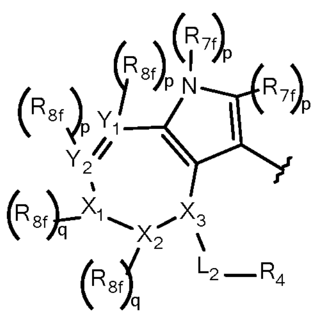,
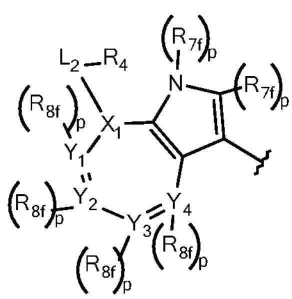,
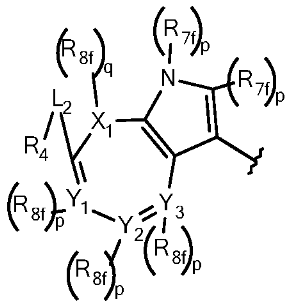,
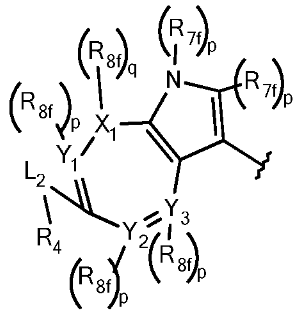

,
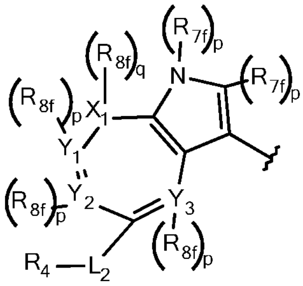,
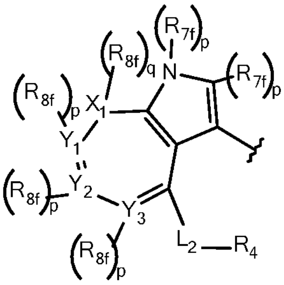,
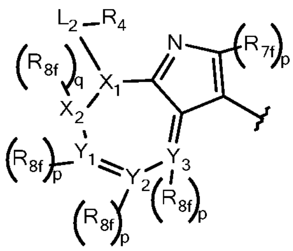,

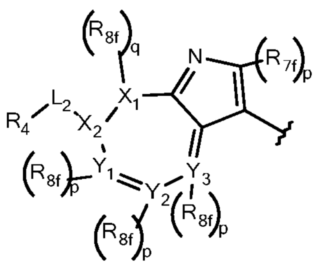,
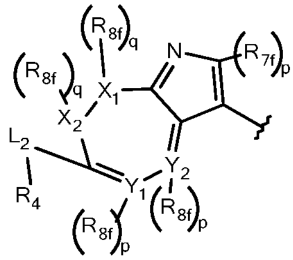,
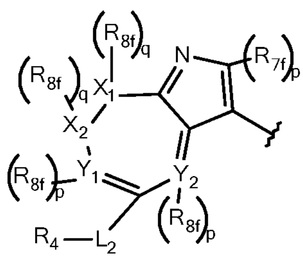,
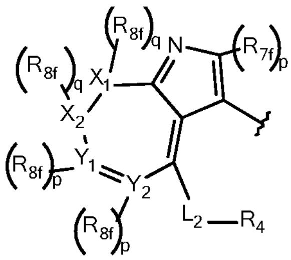

, 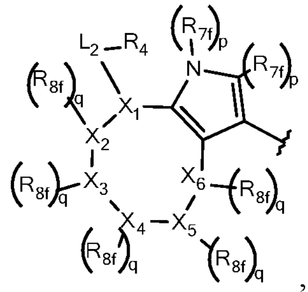 , 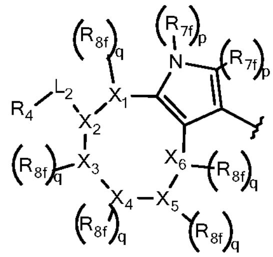 , 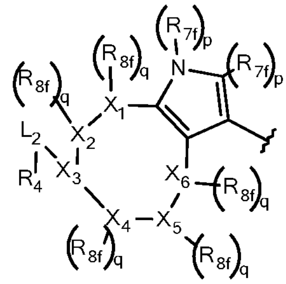 , 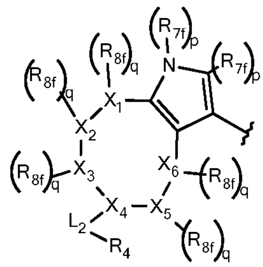 ,

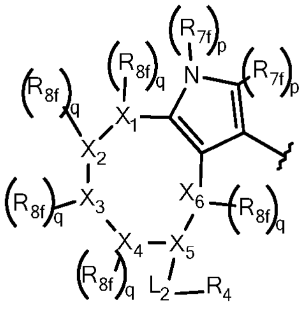 , 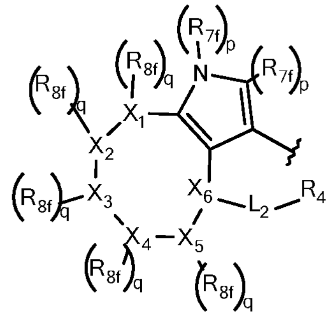 , 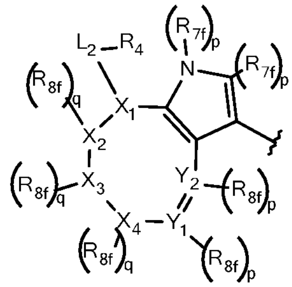 ,

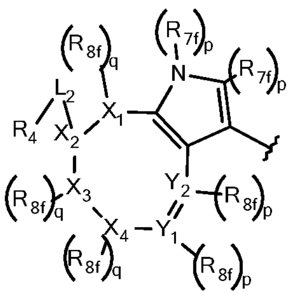 , 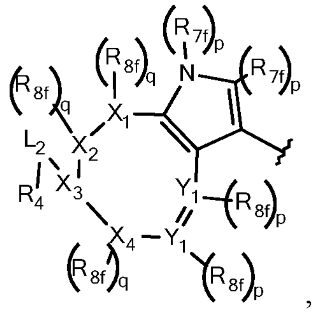 , 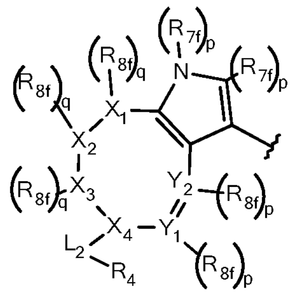 , 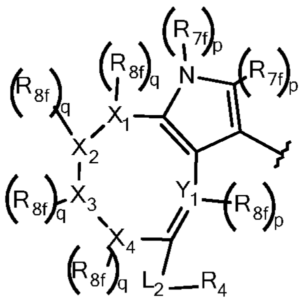 ,

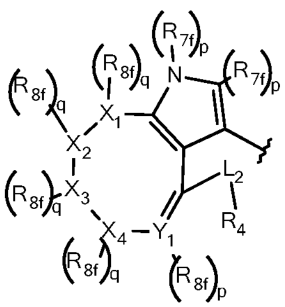 , 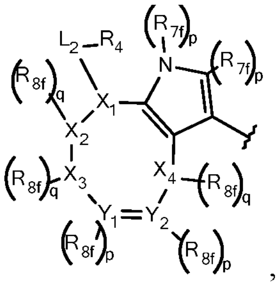 , 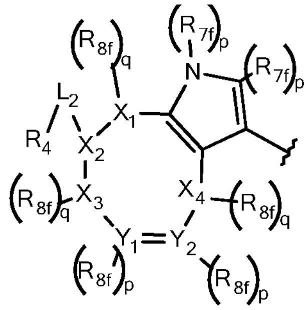 , 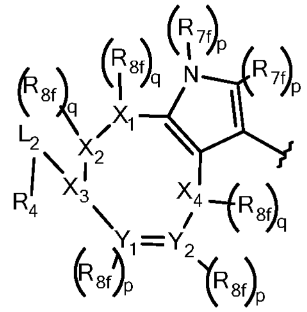 ,

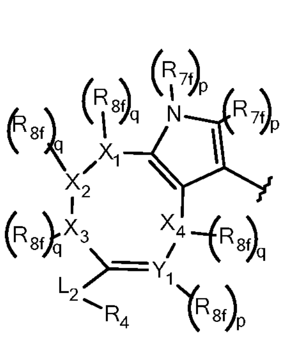 , 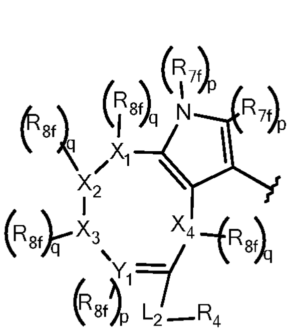 , 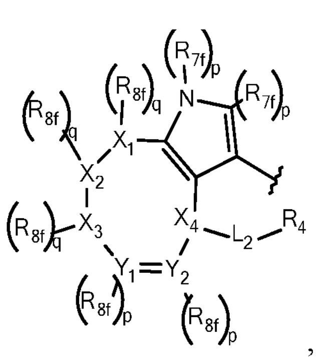 , 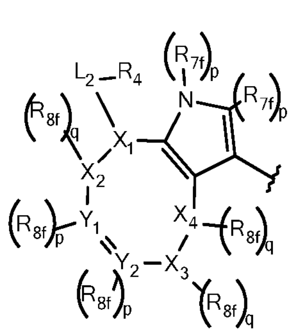 ,

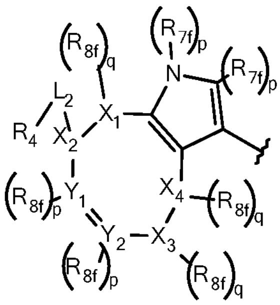,
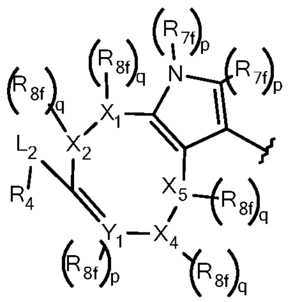,
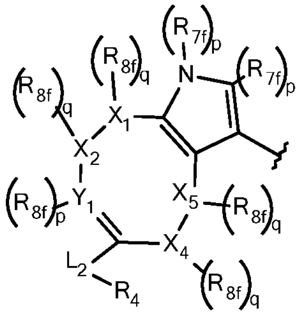,
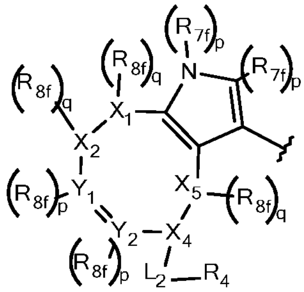,
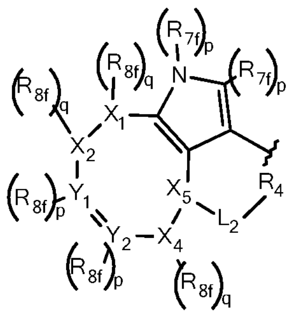,
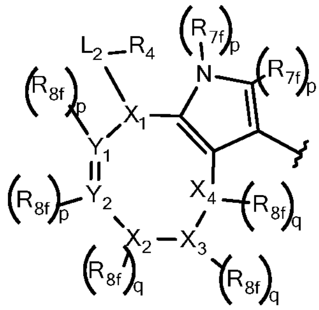,
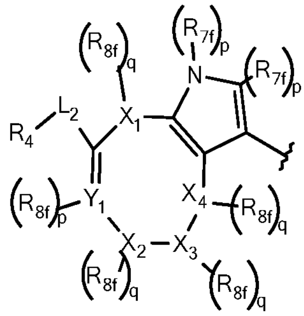,
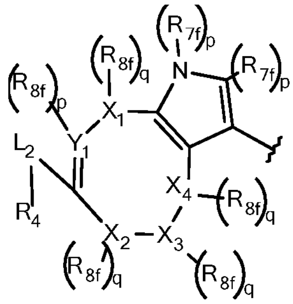
, 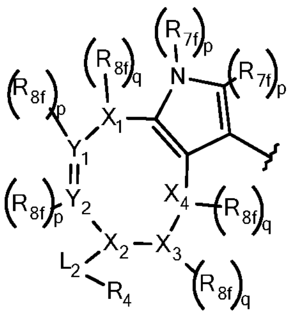
, 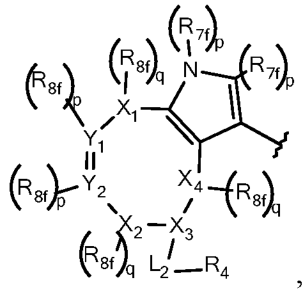
, 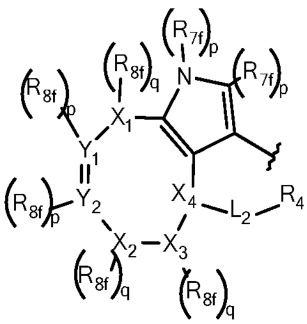
, 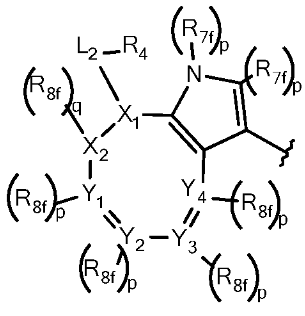
, 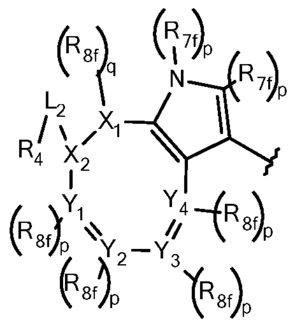
, 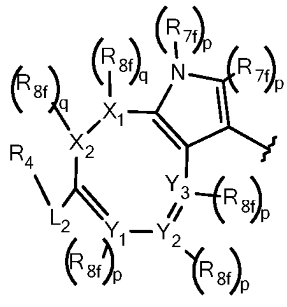
, 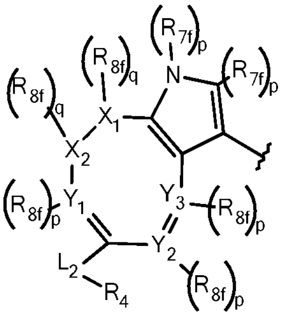
, 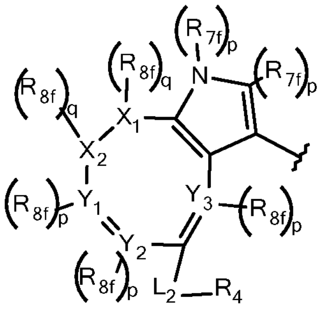
, 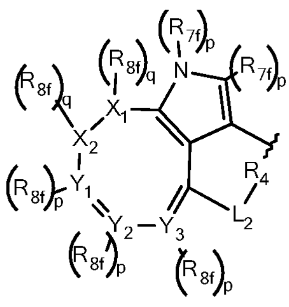
, 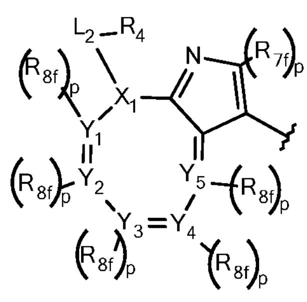
, 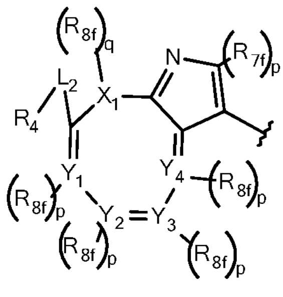
,

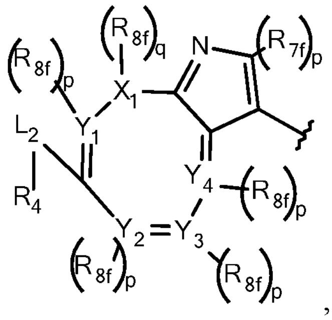,
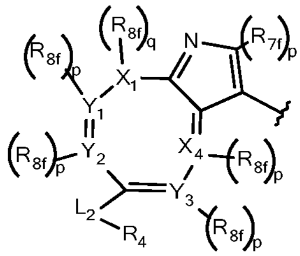,
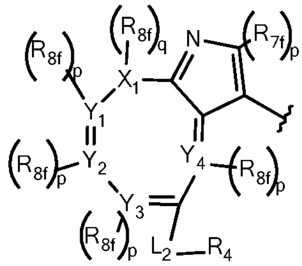,

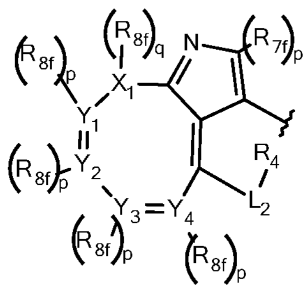,
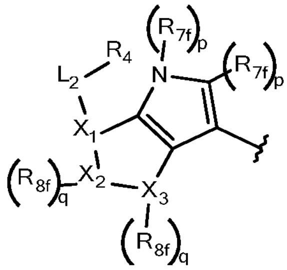,
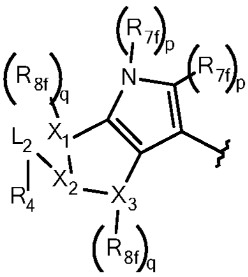,
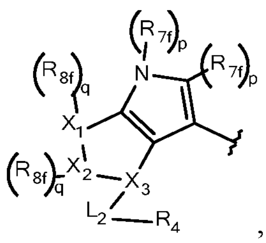,

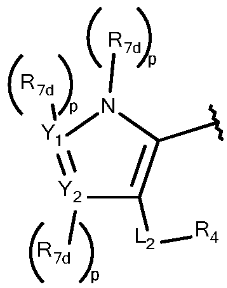,
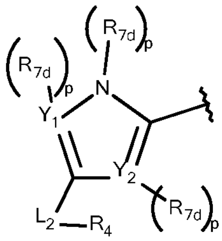,
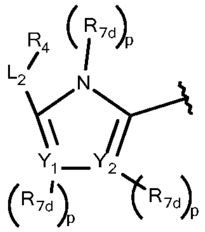,
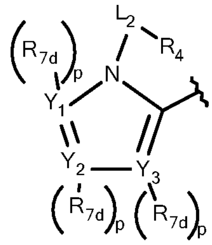,
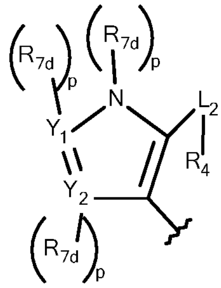,

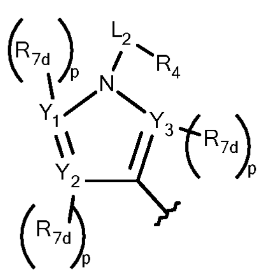,
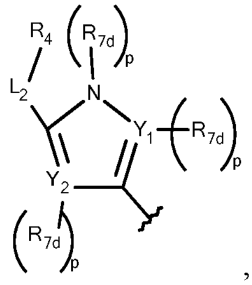,
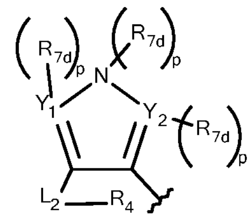,
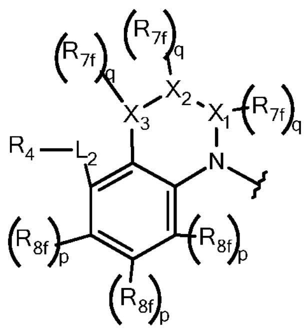,

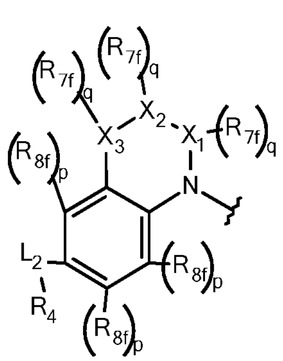,
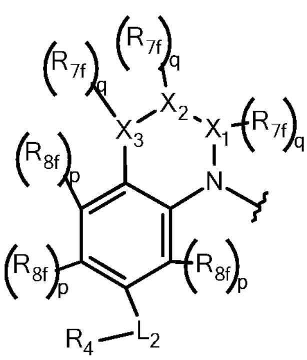,
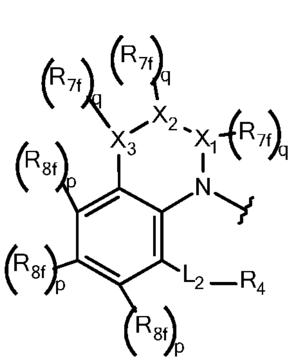,
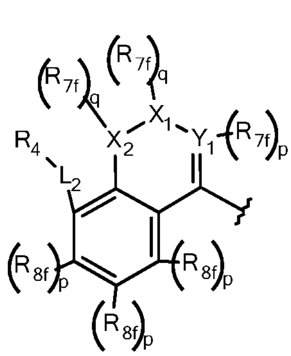,

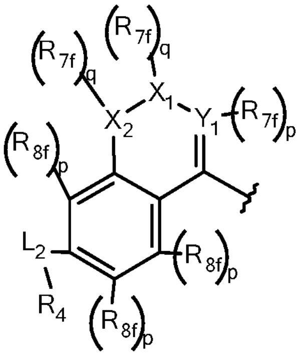
,
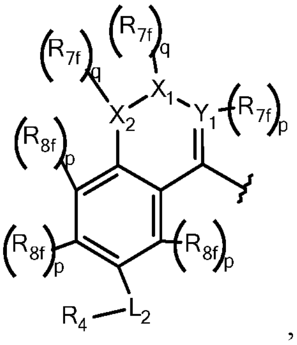
,
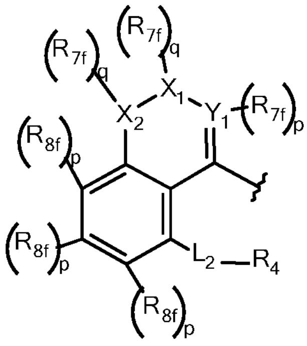
,

where p is each independently 0 or 1 and q is each independently 0,1 or 2, wherein the total number of substituents in each cycle is no more than 3;

$X_1$, $X_2$, $X_3$, $X_4$, $X_5$, $X_6$ are each independently an atom of C, an atom of N, CH, $CH_2$, NH, S or O; $Y_1$, $Y_2$, $Y_3$, $Y_4$, $Y_5$ are each independently an atom of C, an atom of N or CH;

$L_2$ is a chemical bond, $-NR_5-$, -O-, -S-, -S(O)-, $-S(O)_2-$, -C(O)-, -C(O)O-, -C(S)-, -C(=NH)-,$-(CR_{6a}R_{6b})_{1-3}-$, $-P(O)(CH_3)-$, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;

$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH-$NH_2$, -Hal, -CN, $-C_1-C_6$ alkyl unsubstituted or substituted by one or several radicals selected from -Hal, -OH, $-NH_2$, -CN, phenyl; $-O-C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl, $-C_1-C_6$ alkyl;$-S(O)_2C_1-C_6$ alkyl, $-S(O)_2NH_2$, $-NH(C_1-C_6\ alkyl)$, $-N(C_1-C_6\ alkyl)_2$, $-P(O)(C_1-C_6\ alkyl)_2$, morpholinyl, thiazolyl, phenyl,

$R_5$, $R_{5a}$ are each independently -H, $C_1-C_6$ alkyl,

$R_{6a}$, $R_{6b}$ are each independently -H, $C_1-C_6$ alkyl, -Hal,

or $R_{6a}$ and $R_{6b}$ together with the C atom they are attached to form $C_3-C_6$ cycloalkyl unsubstituted or unsubstituted by several $-(C_1-C_6)$alkyls;

$R_7$, $R_{7b}$, $R_{7d}$, $R_{7f}$, are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;

$R_{8b}$, $R_{8d}$, $R_{8f}$ are each independently -Hal, -OH, $-NH_2$, -CN, (=O), $-(C_1-C_6)$alkyl unsubstituted or substituted by one or several -Hal;

Hal is an atom of F, Cl, Br, I.

In one embodiment, the present invention relates to a compound, wherein $-A-L_2-R_4$ is

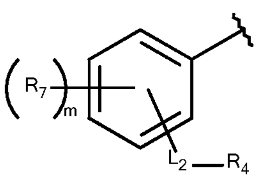
,
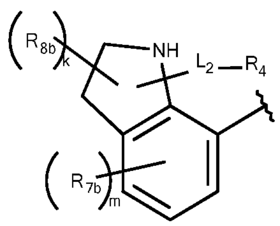
,
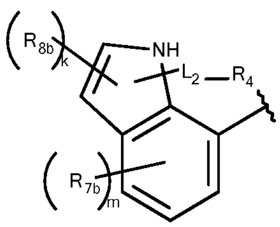
,
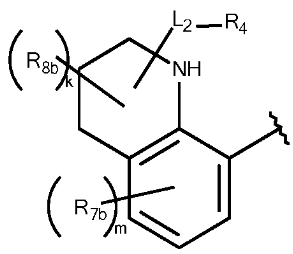
,

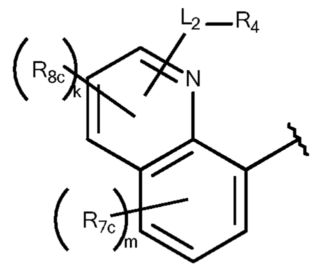

,

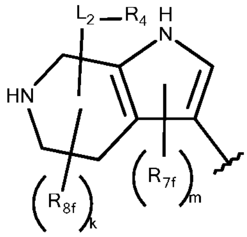

,

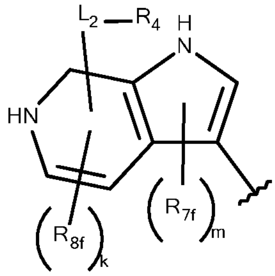

,

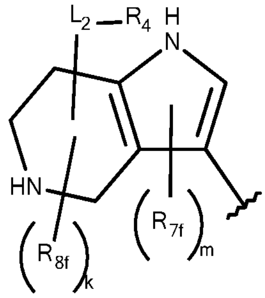

,

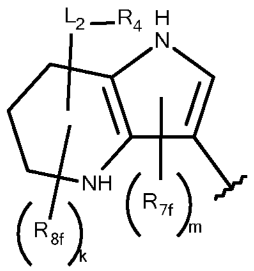

,

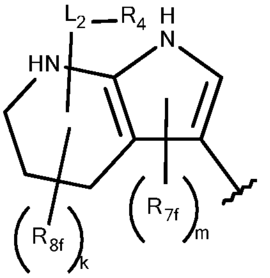

,

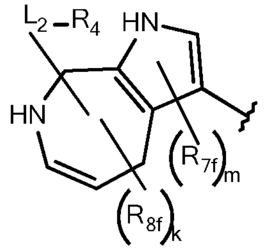

,

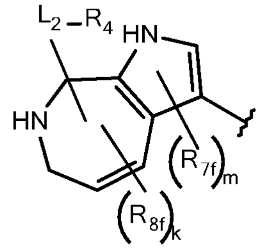

,

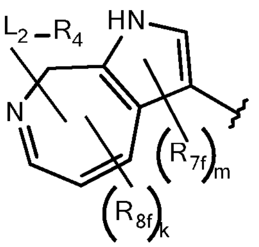

,

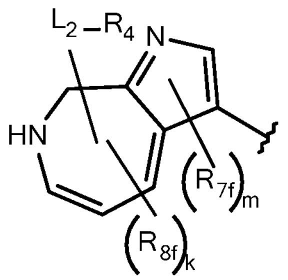

,

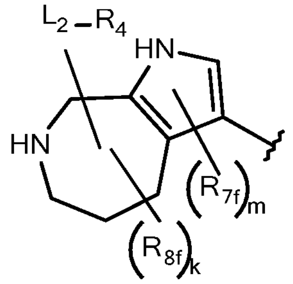

,

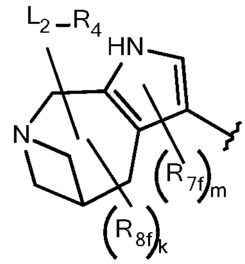

,

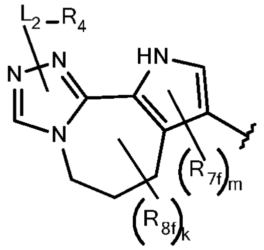

,

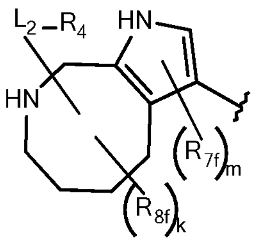

,

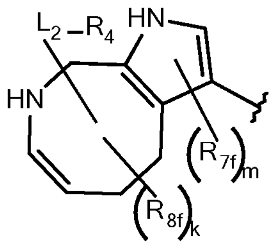

,

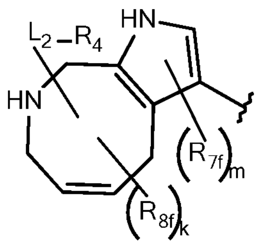

,

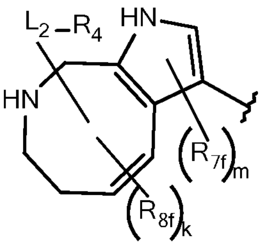

,

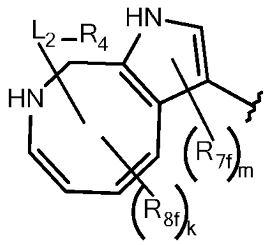

,

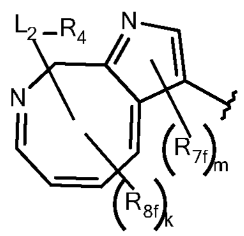

,

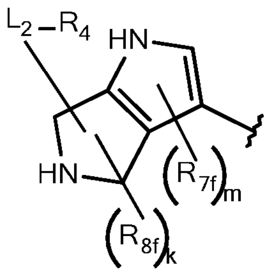

,

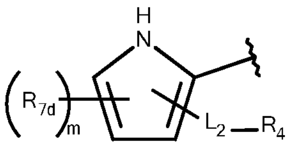,
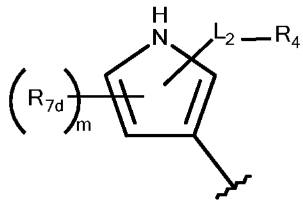,
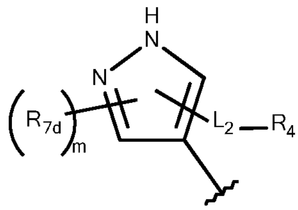,
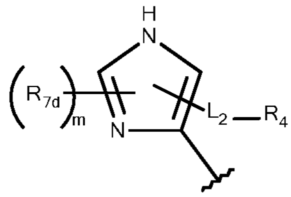,

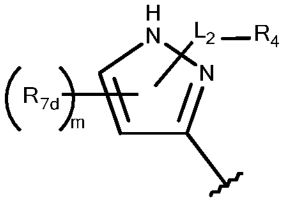,
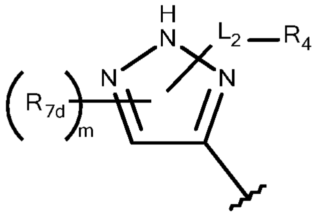,
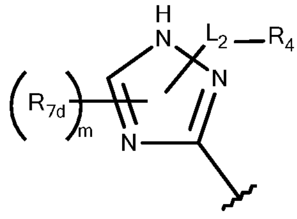,
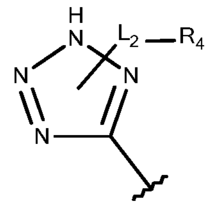,

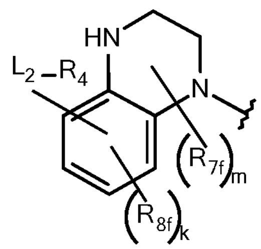,
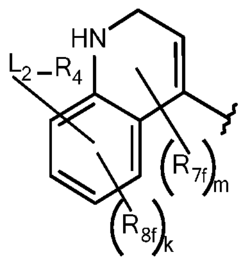,
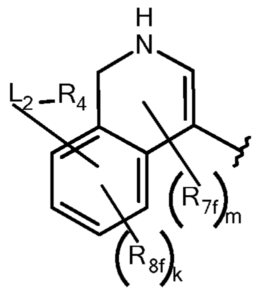,
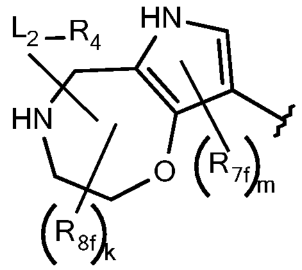,

wherein k,m are each independently 0, 1,2 or 3,

$L_2$ is a chemical bond, $-NR_5-$, -O-, -S-, -S(O)-, $-S(O)_2-$, -C(O)-, -C(O)O-, -C(S)-, -C(=NH)-, $-(CR_{6a}R_{6b})_{1-3}-$, $-P(O)(CH_3)-$, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;

$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), $-OH-NH_2$, -Hal, -CN, $-C_1-C_6$ alkyl unsubstituted or substituted by one or several radicals selected from -Hal, -OH, $-NH_2$, -CN, phenyl; $-O-C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl, $-C_1-C_6$ alkyl; $-S(O)_2C_1-C_6$ alkyl, $-S(O)_2NH_2$, $-NH(C_1-C_6\ alkyl)$, $-N(C_1-C_6\ alkyl)_2$, $-P(O)(C_1-C_6\ alkyl)_2$, morpholinyl, thiazolyl, phenyl,

$R_5$, $R_{5a}$ are each independently -H, $C_1-C_6$ alkyl,

$R_{6a}$, $R_{6b}$ are each independently -H, $C_1-C_6$ alkyl, -Hal,

or $R_{6a}$ and $R_{6b}$ together with the C atom they are attached to form $C_3-C_6$ cycloalkyl unsubstituted or unsubstituted by several $-(C_1-C_6)$alkyls;

$R_7$, $R_{7b}$, $R_{7d}$, $R_{7f}$, are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;

$R_{8b}$, $R_{8d}$, $R_{8f}$ are each independently -Hal, -OH, $-NH_2$, -CN, (=O), $-(C_1-C_6)$alkyl unsubstituted or substituted by one or several -Hal;

Hal is an atom of F, Cl, Br, I.

In one embodiment, the present invention relates to a compound, wherein $-A-L_2-R_4$ is

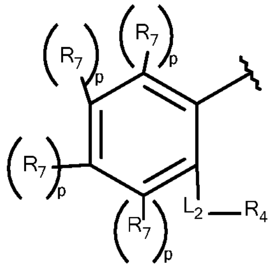,
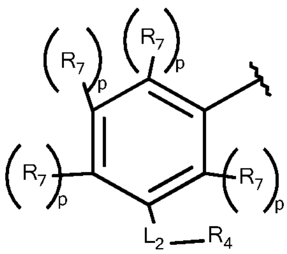,
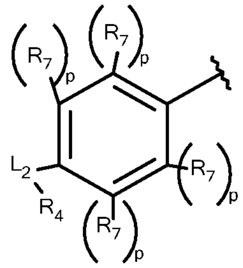,
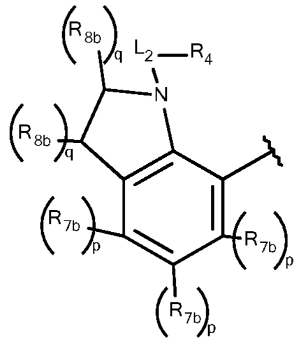,
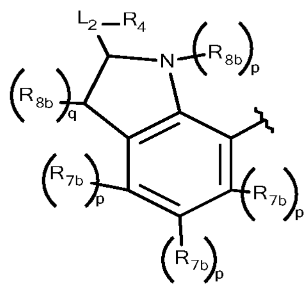,
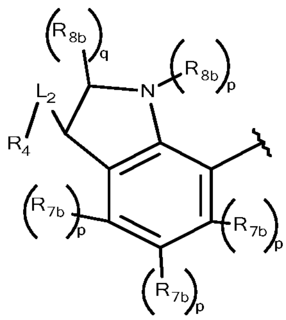,
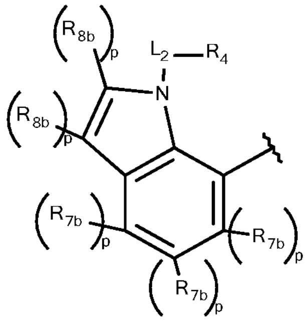,
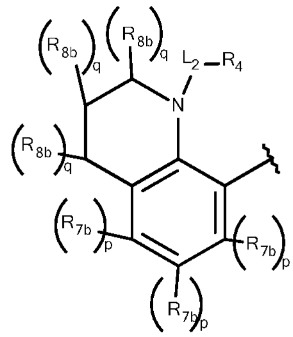,
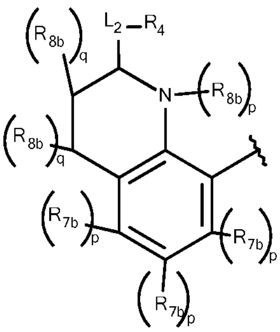,
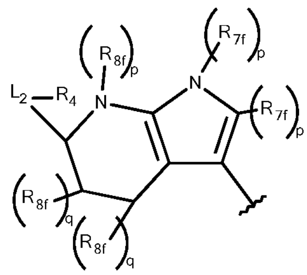,
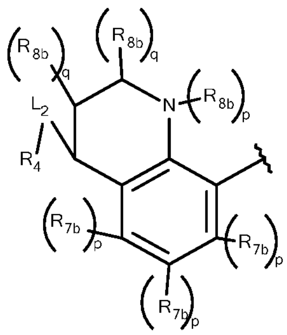,
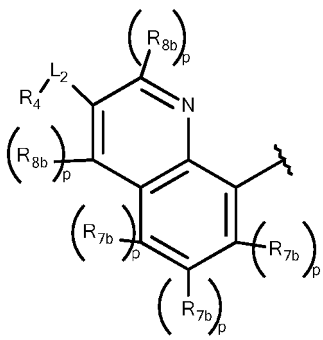,
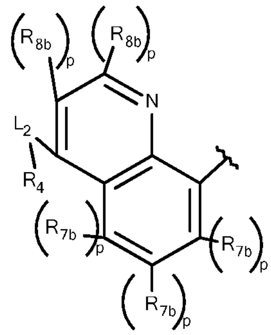,
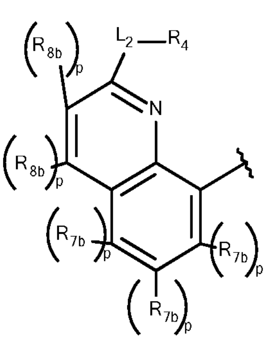,
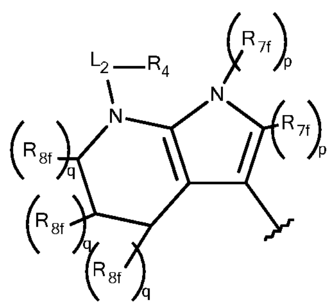

,

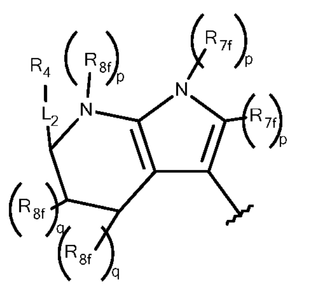

,

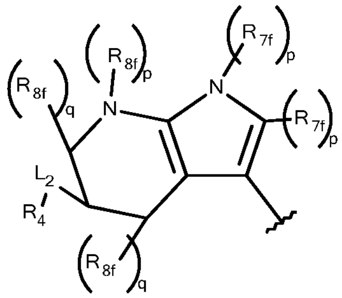

,

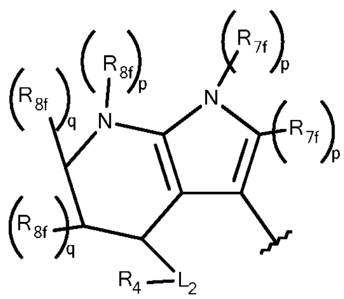

,

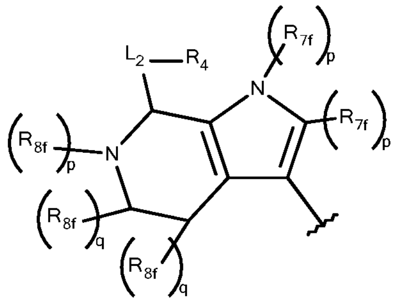

,

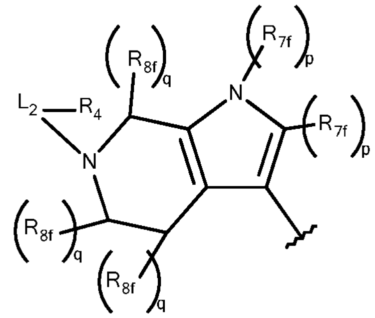

,

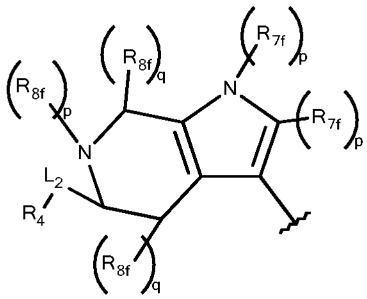

,

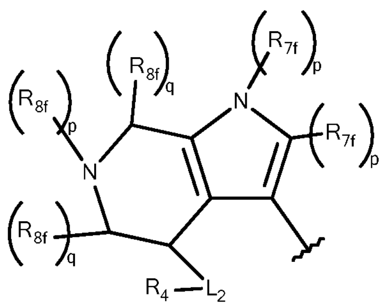

,

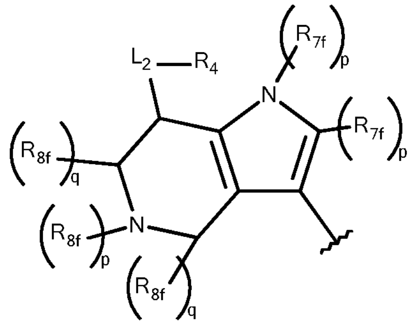

,

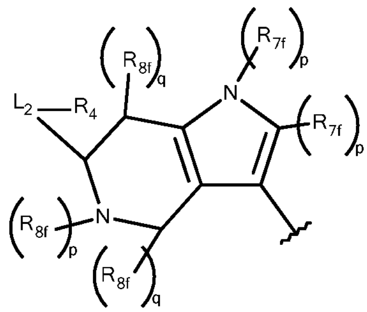

,

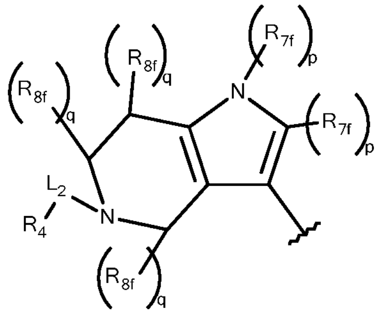

,

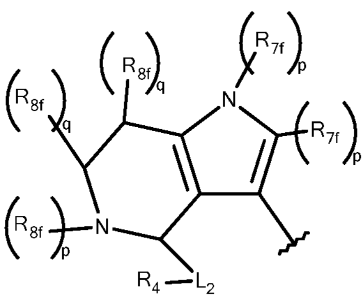

,

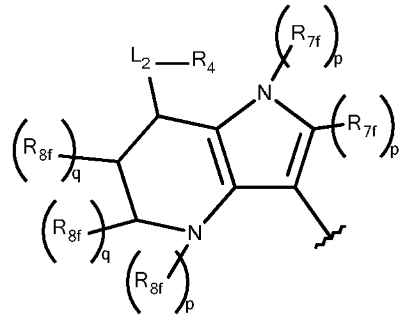

,

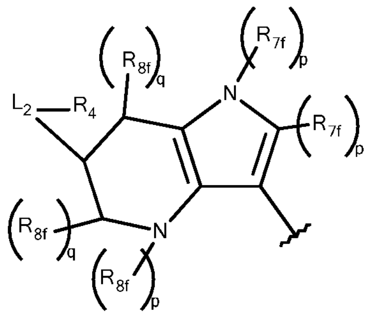

,

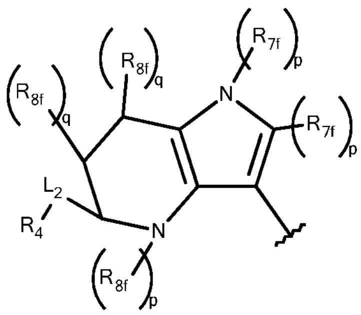

,

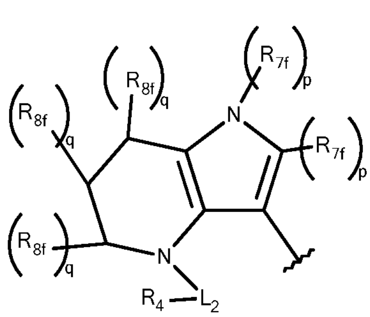

,

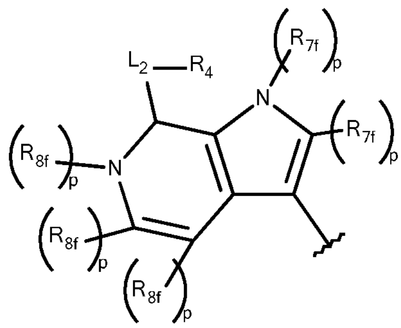
,
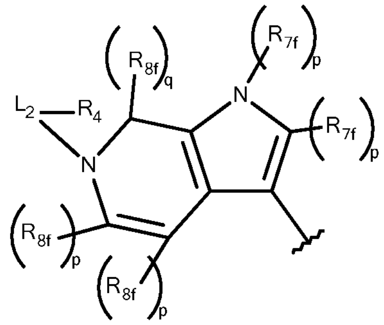
,
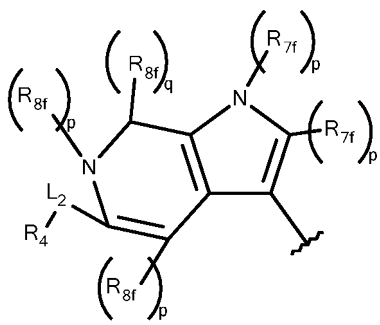
,

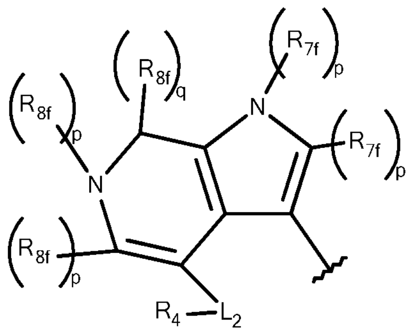
,
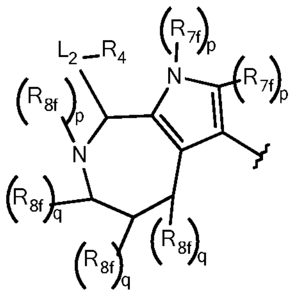
,
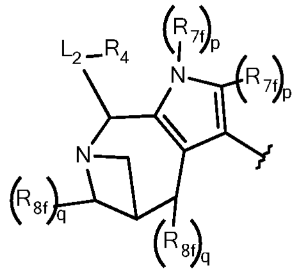
,

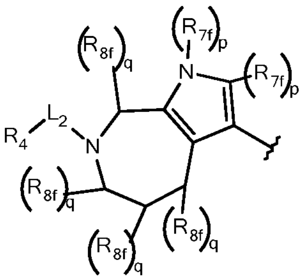
,
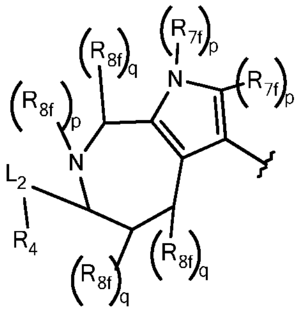
,
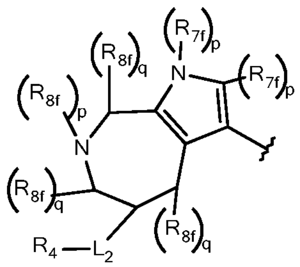
,
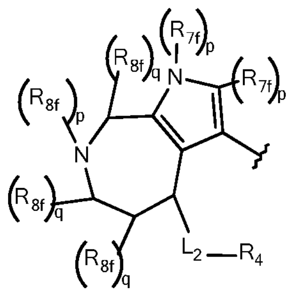

,
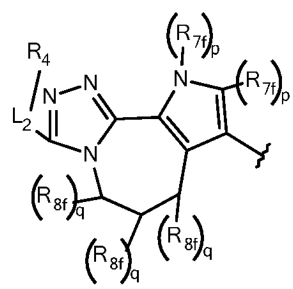
,
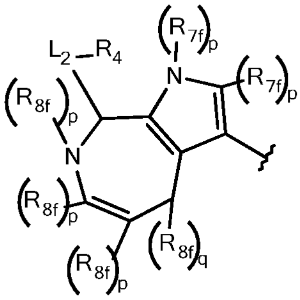
,
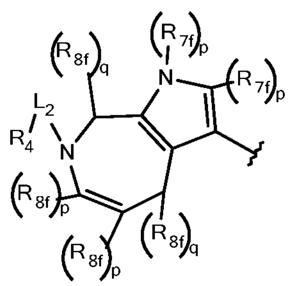
,
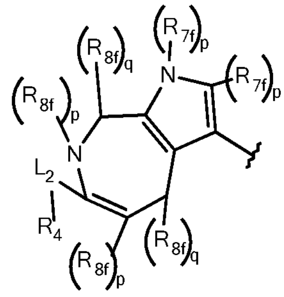

,
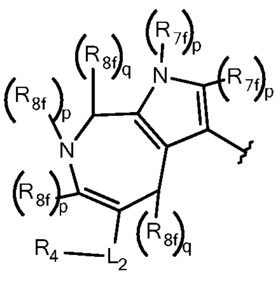
,
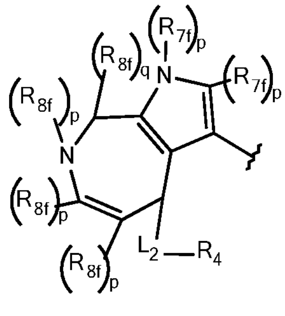
,
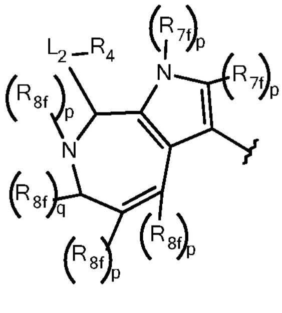
,
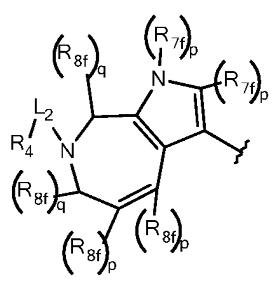
,

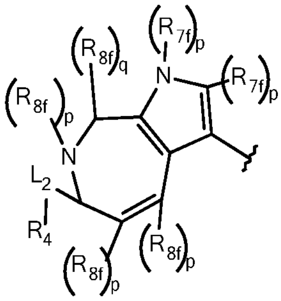,
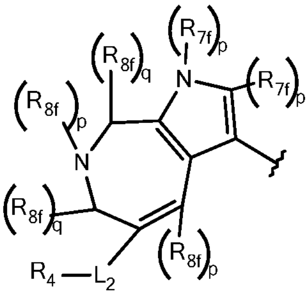,
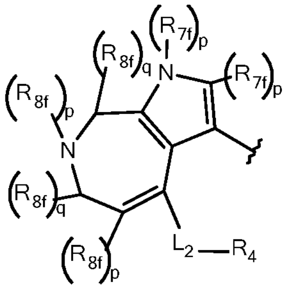,
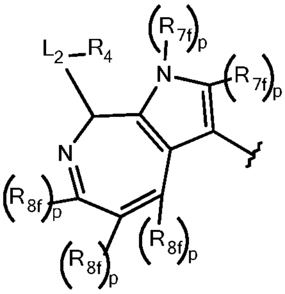,

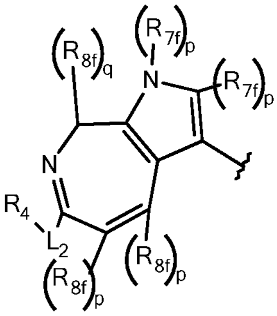,
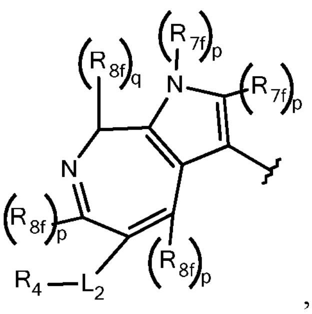,
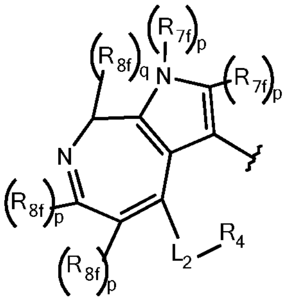,
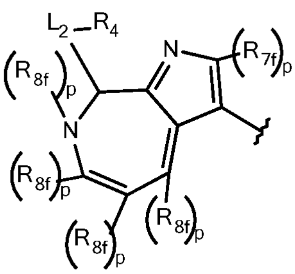,

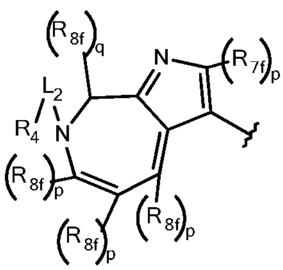,
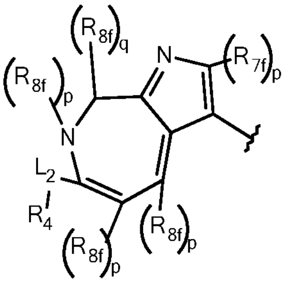,
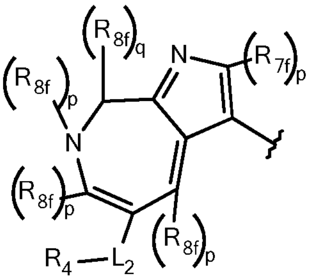,
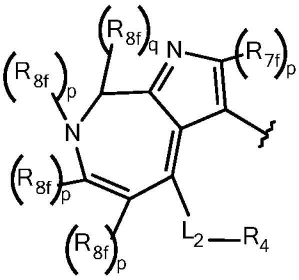,

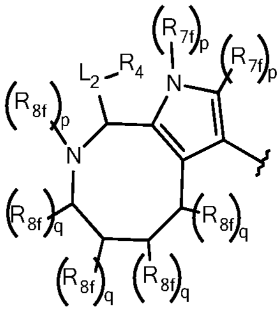,
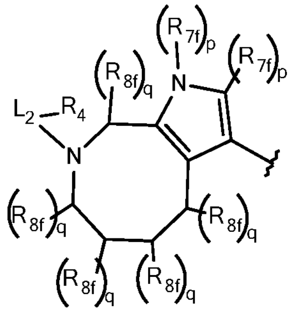,
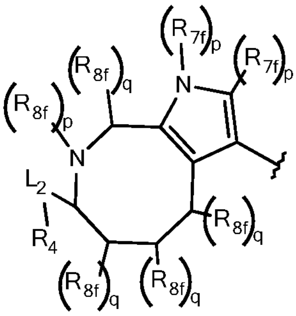,
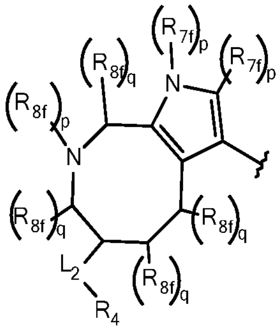,

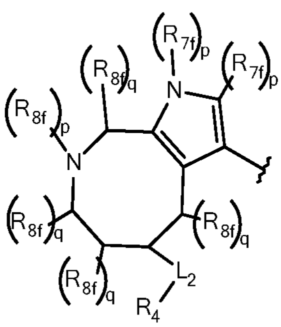,
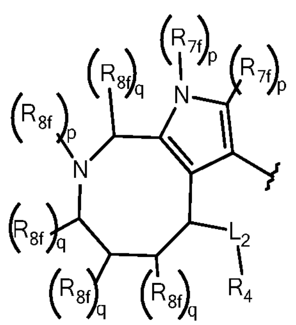,
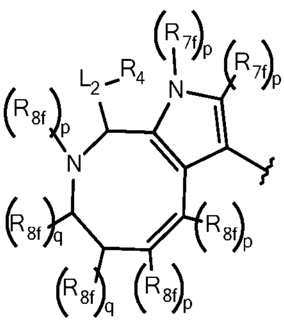,
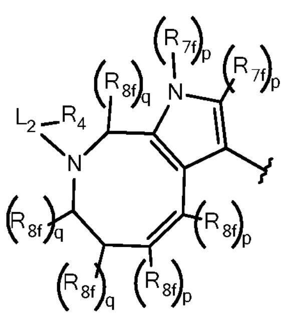,

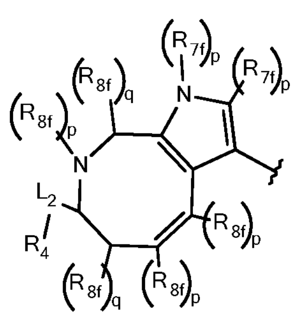,
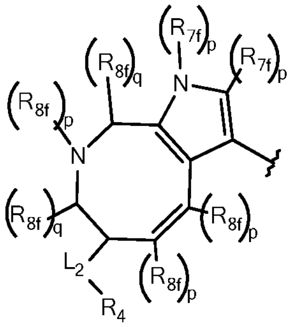,
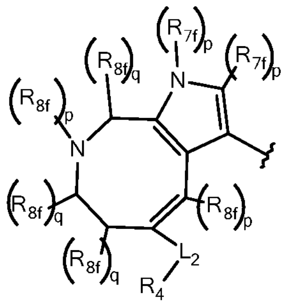,
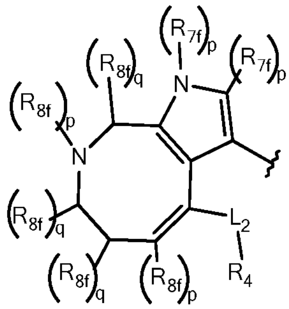,

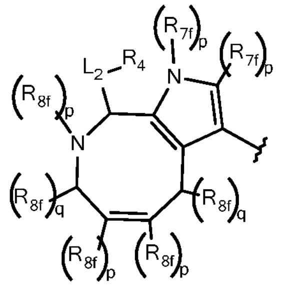,
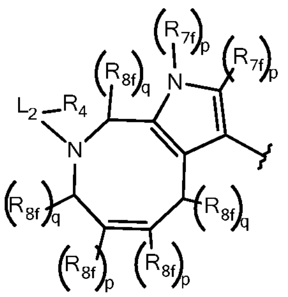,
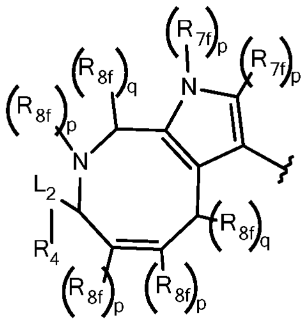,
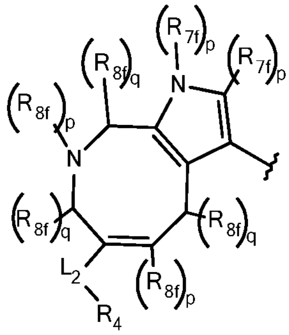,

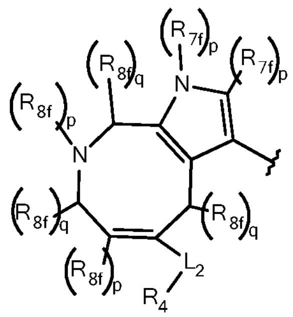,
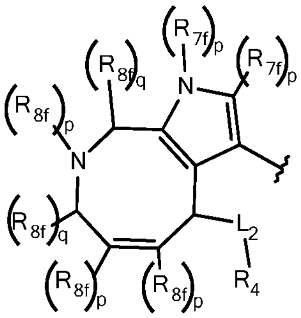,
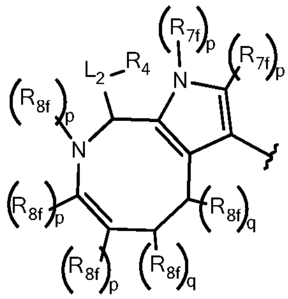,
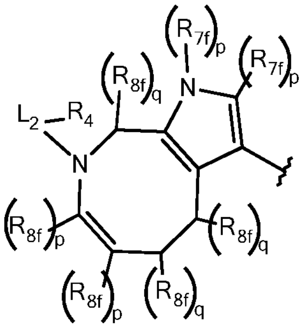,

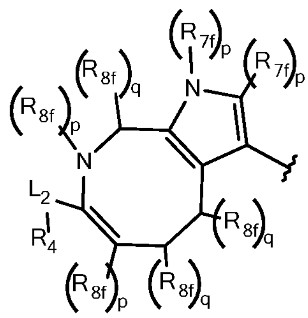,
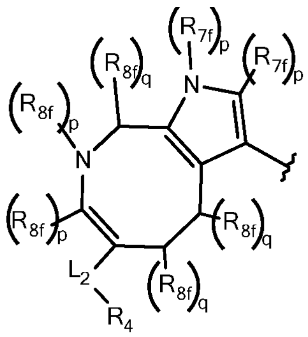,
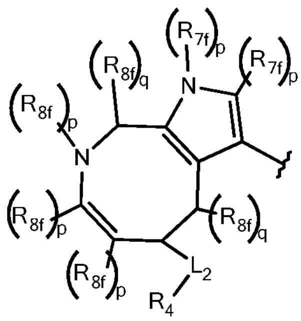,

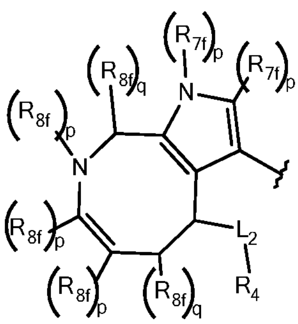, 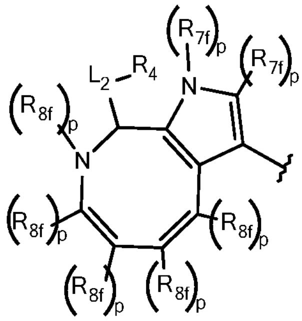, 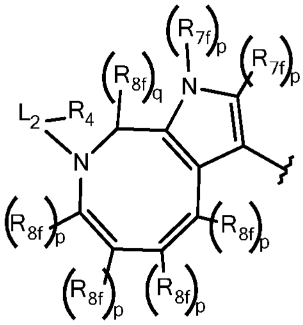, 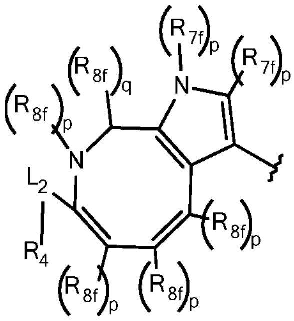

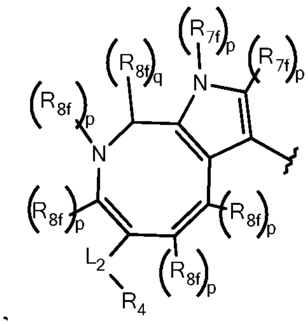, 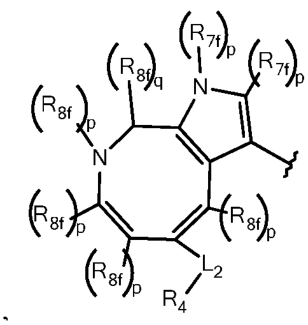, 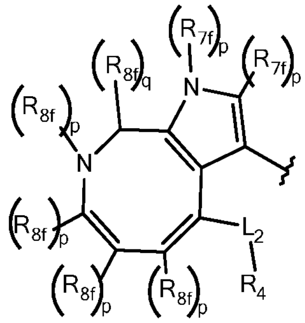, 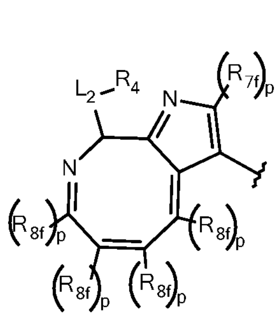, 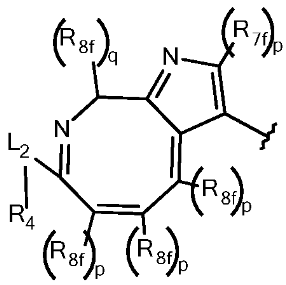

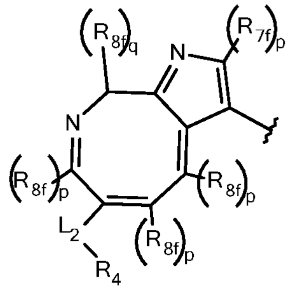, 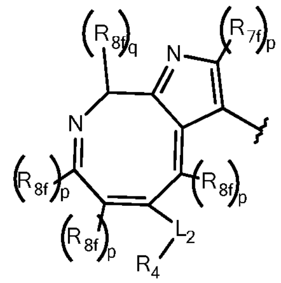, 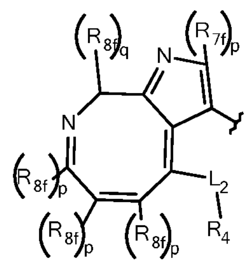, 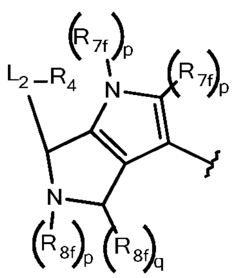

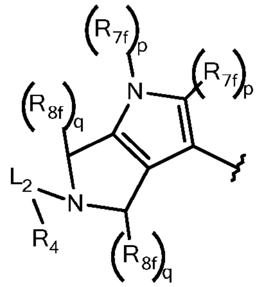, 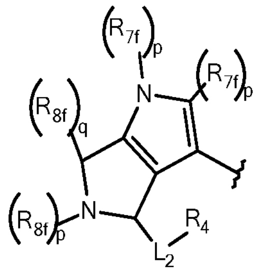, 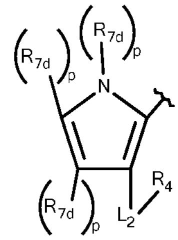, 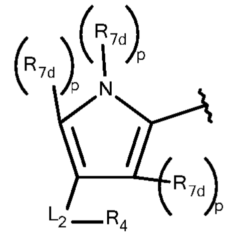, 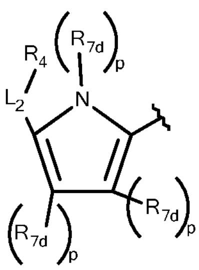, 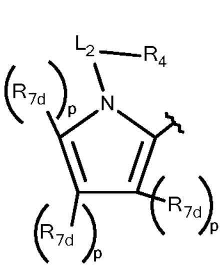, 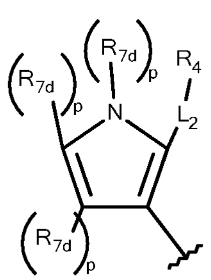, 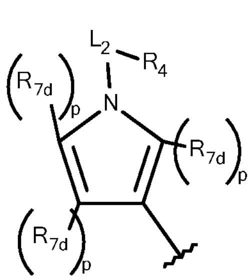, 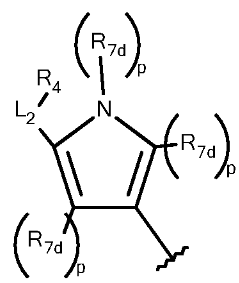

, 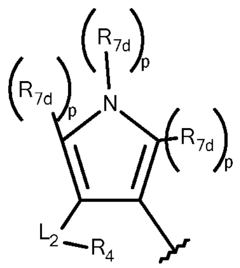 , 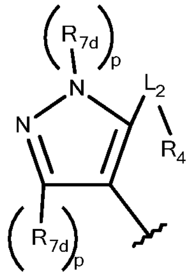 , 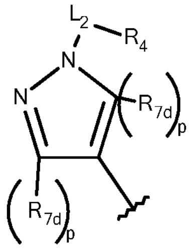 , 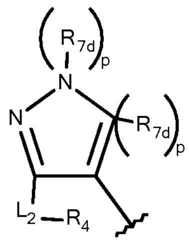 , 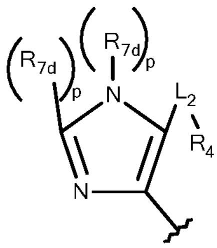 ,

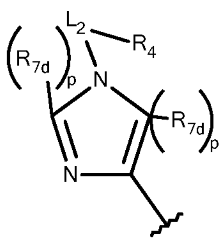 , 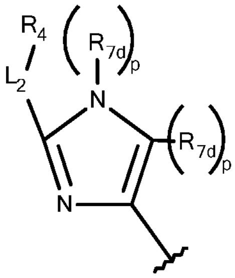 , 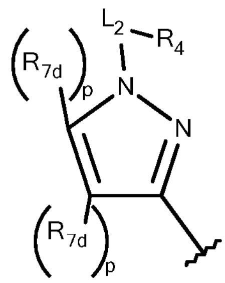 , 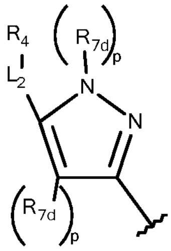 , 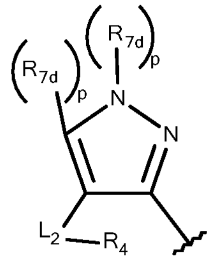 ,

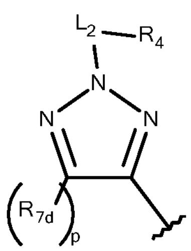 , 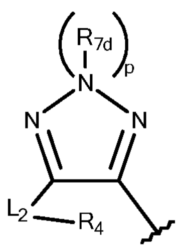 , 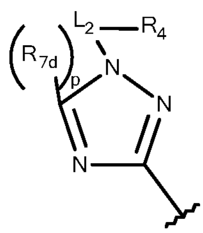 , 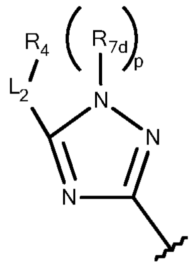 , 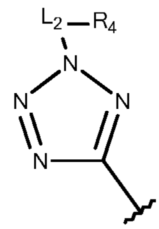 ,

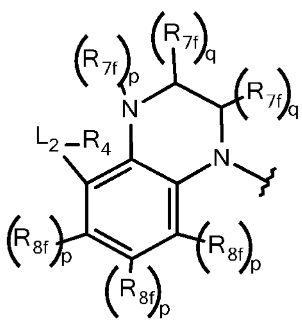 , 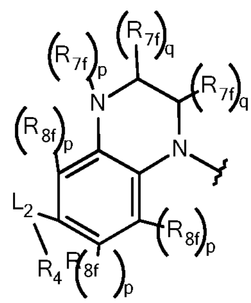 , 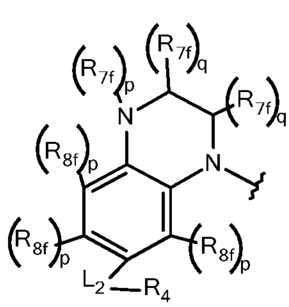 , 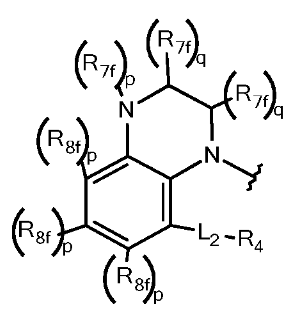 ,

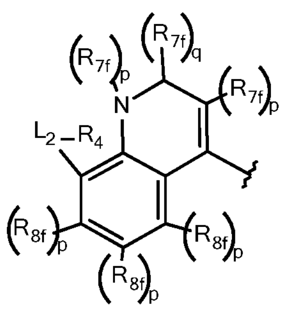 , 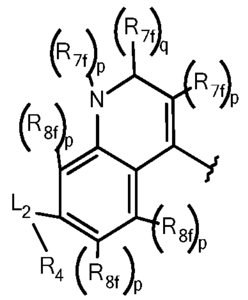 , 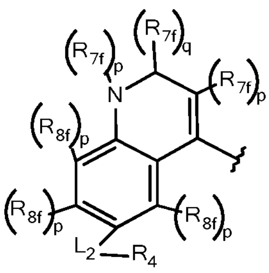 , 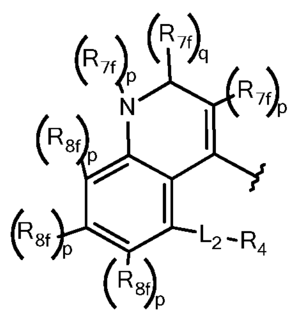 ,

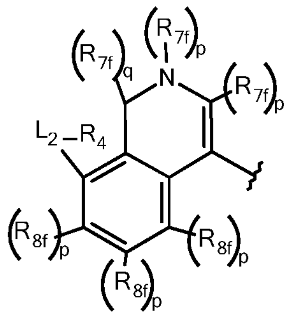 , 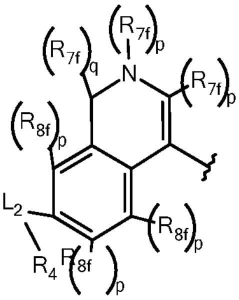 , 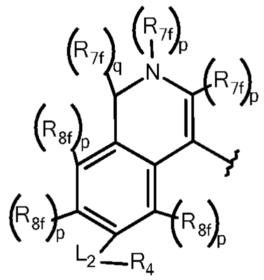 , 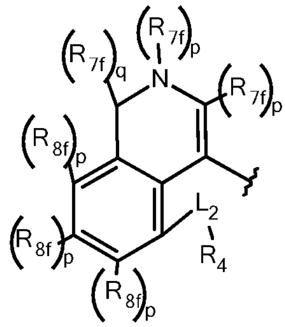 ,

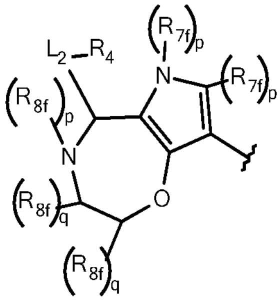 , 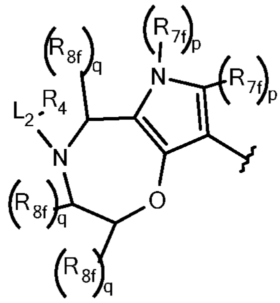 , 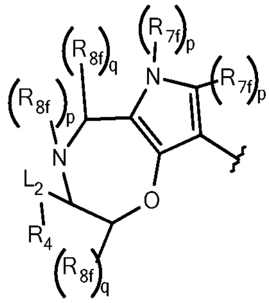 , 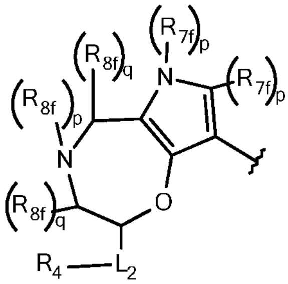 ,

where p is each independently 0 or 1 and q is each independently 0,1 or 2, wherein the total number of substituents in each cycle is no more than 3;

$L_2$ is a chemical bond, $-NR_5-$, -O-, -S-, -S(O)-, $-S(O)_2-$, -C(O)-, -C(O)O-, -C(S)-, -C(=NH)-, - $(CR_{6a}R_{6b})_{1-3}-$, -P(O)$(CH_3)-$, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;

$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH-$NH_2$, -Hal, -CN, $-C_1-C_6$ alkyl unsubstituted or substituted by one or several radicals selected from -Hal, -OH, $-NH_2$, -CN, phenyl; -O-$C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl, $-C_1-C_6$ alkyl;$-S(O)_2C_1-C_6$ alkyl, $-S(O)_2NH_2$, $-NH(C_1-C_6\ alkyl)$, $-N(C_1-C_6\ alkyl)_2$, $-P(O)(C_1-C_6\ alkyl)_2$, morpholinyl, thiazolyl, phenyl,

$R_5$, $R_{5a}$ are each independently -H, $C_1-C_6$ alkyl,

$R_{6a}$, $R_{6b}$ are each independently -H, $C_1-C_6$ alkyl, -Hal,

or $R_{6a}$ and $R_{6b}$ together with the C atom they are attached to form $C_3-C_6$ cycloalkyl unsubstituted or unsubstituted by several $-(C_1-C_6)$alkyls;

$R_7$, $R_{7b}$, $R_{7d}$, $R_{7f}$, are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;

$R_{8b}$, $R_{8d}$, $R_{8f}$ are each independently -Hal, -OH, $-NH_2$, -CN, (=O), $-(C_1-C_6)$alkyl unsubstituted or substituted by one or several -Hal;

Hal is an atom of F, Cl, Br, I.

In one embodiment, the present invention relates to a compound, wherein $L_1$, $L_2$ are each independently a chemical bond, -C(O)-, $-CH_2-$, $-CH(CH_3)-$, $-C(CH_3)_2-$, $-C(CH_2)_2-$, $-CF_2-$, -S-,-S(O)-, $-S(O)_2-$, -O-, -NH-, $-NCH_3-$, -P(O)$(CH_3)-$, -C(O)O-, -C(O)-NH- or -NH-C(O)-..

In one embodiment, the present invention relates to a compound, wherein $R_1$ is -H, $-CF_3$,$-CCl_3$, $-CHF_2$, $-CH_2F$, $-CHCl_2$, $-CH_2Cl$, $-NO_2$.

In one embodiment, the present invention relates to a compound, wherein $R_2$, $R_3$ are each independently -H, methyl, ethyl, propyl, or $R_2$ and $R_3$ together with the C atom they are attached to form cyclopropyl, cyclobutyl or cyclopentyl.

In one embodiment, the present invention relates to a compound, wherein $R_4$ is -H, (=O),-OH, -F, -Cl, -Br, -P(O)$(C_1-C_6$ alkyl$)_2$; $-S(O)_2NH_2$; $-S(O)_2-(C_1-C_6)$alkyl; $-(C_1-C_6)$alkyl unsubstituted or substituted by one or several (=O), -Hal, -CN, $-S(O)_2-(C_1-C_6)$alkyl; $-O-(C_1-C_6)$alkyl unsubstituted or substituted by one or several (=O), -CN, -Hal; $-(C_3-C_6)$cycloalkyl unsubstituted or substituted by one or several (=O), -OH, -Hal, -CN; phenyl unsubstituted or substituted by one or several -Hal, -CN, morpholine, $-P(O)(CH_3)_2$; 5-6 membered heterocyclyl with 1,2 or 3 heteroatoms selected from N or O unsubstituted or substituted by $-(C_1-C_6)$alkyl, (=O), -Hal, -CN; 5-6 membered heteroaryl with 1,2,3 or 4 heteroatoms selected from N,O or S, unsubstituted or substituted by (=O), $-(C_1-C_6)$alkyl, $-(C_3-C_6)$cycloalkyl, -Hal, -CN; 5-6 membered heteroaryl with 1 or 2 atoms N, unsubstituted or substituted by one or several (=O), $-(C_1-C_6)$alkyl, fused with phenyl; $-NR_{4a}R_{4b}$,

$R_{4a}$, $R_{4b}$ are each independently -H, (=O), morpholinyl, -F, $-S(O)_2CH_3$, -CN, -one, cyclopropyl, thiazolyl, $-C_1-C_3$ alkyl unsubstituted or substituted with phenyl, $-P(O)(CH_3)_2$.

In one embodiment, the present invention relates to a compound, wherein $R_4$ is -H, -OH, (=O), $-P(O)(CH_3)_2$, $-CH_3$, $-CH_2CH_3$, tert-butyl, $-CH_2CF_3$, $- CH_2CH_2S(O)_2CH_3$, $-CF_3$, -Cl, $-OCF_3$, $-S(O)_2NH_2$, $-S(O)_2CH_3$, phenyl unsubstituted or substituted by one or several -Hal, -CN; methylpiperazinyl, piperazinyl, imidazolyl, thiazolyl, methylpyrazolyl, pyrazolyl, pyridinyl, pyrimidinyl, morpholine, oxanyl, oxolanyl, cyclohexyl, cyclopentyl, cyclopropyl, oxazolyl, methyloxazolyl, dimethyloxazolyl, dihydropyridinyl, methyl dihydropyridinyl, $-NR_{4a}R_{4b}$,

$R_{4a}$, $R_{4b}$ are each independently -H, (=O), morpholinyl, -F, $-S(O)_2CH_3$, -CN, -one, cyclopropyl, thiazolyl, $-C_1-C_3$ alkyl unsubstituted or substituted with phenyl, $-P(O)(CH_3)_2$.

In one embodiment, the present invention relates to a compound, wherein $R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O) $-CH_3$, morpholinyl, -F, $-S(O)_2CH_3$, -CN, -OH, cyclopropyl, thiazolyl, $-C_1-C_3$ alkyl unsubstituted or substituted with phenyl, $-P(O)(CH_3)_2$.

In one embodiment, the present invention relates to a compound, wherein $R_5$ is --H, $-CH_3$.

In one embodiment, the present invention relates to a compound, wherein $R_{6a}$ and $R_{6b}$ are each independently -H, -F, $-CH_3$, or $R_{6a}$ and $R_{6b}$ together with the C atom they are attached to are cyclopropyl.

In one embodiment, the present invention relates to a compound, wherein $R_7$, $R_{7a}$, $R_{7b}$, $R_{7c}$, $R_{7d}$, $R_{7e}$ $R_{7f}$ $R_{7g}$ $R_{7h}$ are each independently (=O), -Hal, $-CH_3$; $-OCH_3$; $-CHal_3$; $-OCHal_3$.

In one embodiment, the present invention relates to a compound, wherein $R_{8a}$, $R_{8b}$, $R_{8c}$, $R_{8a}$, $R_{8e}$ $R_{8f}$ $R_{8h}$ are each independently (=O), -Hal, $-CH_3$; $-OCH_3$; $-CHal_3$; $-OCHal_3$.

Compounds, described in the present invention, may be obtained as, and/or used as, pharmaceutically acceptable salts. The type of pharmaceutical acceptable salts, include, but are not limited to: acid salts formed by reacting the free base form of the compound with a pharmaceutically acceptable inorganic acid such as hydrochloric acid, hydrobromic acid, sulfuric acid, nitric acid, phosphoric acid, metaphosphoric acid, and the like; or with an organic acid such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanedisulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-3-hydroxy-2-ene-1-carboxylic acid, 3-phenylpropionic acid, trimethylacetic acid, tert-butylacetic acid, lauryl sulfuric acid, gluconic acid, glutamic acid, hydroxynaphthoic acid, salicylic acid, stearic acid, muconic acid, and the like.

The corresponding counterions of the pharmaceutically acceptable salts may be analyzed and identified using various methods including, but not limited to, ion exchange chromatography, ion chromatography, capillary electrophoresis, inductively coupled plasma, atomic absorption spectroscopy, mass spectrometry, or any combination thereof.

The salts are recovered by using at least one of the following techniques: filtration, precipitation with a non-solvent followed by filtration, evaporation of the solvent, or, in the case of aqueous solutions, lyophilization. It should be understood that a reference to a pharmaceutically acceptable salt includes the solvent addition forms or crystal forms thereof, particularly solvates or polymorphs. Solvates contain either stoichiometric or non-stoichiometric amounts of a solvent, and may be formed during the process of crystallization with pharmaceutically acceptable solvents such as water, ethanol, and the like. Hydrates are formed when the solvent is water, or alcoholates are formed when the solvent is alcohol. Solvates of compounds described in the present patent can be conveniently prepared or formed during the processes described in the present invention. In addition, the compounds provided in the present invention can exist in unsolvated as well as solvated forms. In general, the solvated forms are considered equivalent to the unsolvated forms for the purposes of the compounds and methods provided in the present invention.

Compounds described in the present invention may be provided in various forms, including but not limited to, amorphous forms, milled forms and nano-particulate forms. In addition, compounds described in the present invention

include crystalline forms, also known as polymorphs. Polymorphs include different crystal packing arrangements of the same elemental composition of a compound. Polymorphs typically have different X-ray diffraction patterns, infrared spectra, melting points, different density, hardness, crystal shape, optical and electrical properties, stability, and solubility. Various factors such as the recrystallization solvent, rate of crystallization, and storage temperature may cause one crystal form to dominate.

The screening and characterization of the pharmaceutically acceptable salts, polymorphs and/or solvates may be accomplished using a variety of techniques including, but not limited to, thermal analysis, x-ray diffraction, spectroscopy, vapor sorption, and microscopy. Thermal analysis methods address to analysis of thermo chemical degradation or thermo physical processes including, but not limited to, polymorphic transitions, and such methods are used to analyze the relationships between polymorphic forms, to determine weight loss, to find the glass transition temperature, or for excipient compatibility studies. Such methods include, but are not limited to, differential scanning calorimetry (DSC), modulated differential scanning calorimetry (MDCS), thermogravimetric analysis (TGA), thermogravi-metric and infrared analysis (TG/IR). Crystallographic methods include, but are not limited to, single crystal and powder diffractometers and synchrotron sources. The various spectroscopic techniques used include, but are not limited to, Raman (combinational scattering), FTIR, UVIS, and NMR (liquid and solid state). The various microscopy techniques include, but are not limited to, polarized light microscopy, scanning electron microscopy (SEM) with energy dispersive x-ray analysis (EDX), environmental scanning electron microscopy with EDX (in gas or water vapor atmosphere), IR microscopy, and Raman microscopy.

In another embodiment, the present invention relates to compounds selected from the group including:

| Code Name | Structure | Code Name | Structure |
|---|---|---|---|
| **CDK7_461**<br>4-(2-aminobenzoyl) -N-[(3S)-6,6-dimethylpiperi din-3-yl]-5-(trifluoromethy l)pyrimidin-2-amine | 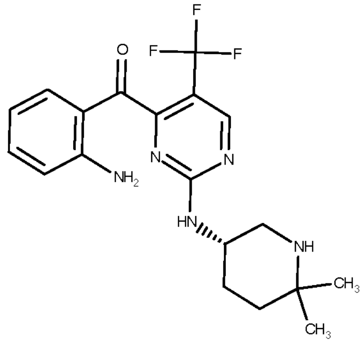 | **CDK7_1000**<br>4-[5-(2-methyl-2H-1,2,3,4-tetrazol-5-yl)-1H-pyrrol-3-yl]-N-[(3S)-piperidin-3-yl]-5-(trifluoromethyl)p yrimidin-2-amine | 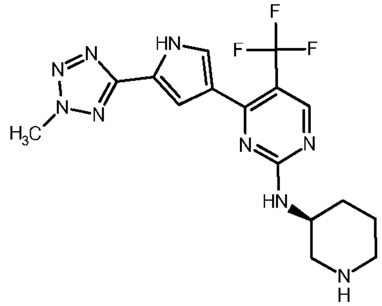 |
| **CDK7_461_a**<br>4-(2-aminobenzoyl) -N-(6,6-dimethylpiperi din-3-yl)-5-(trifluoromethy l)pyrimidin-2-amine | 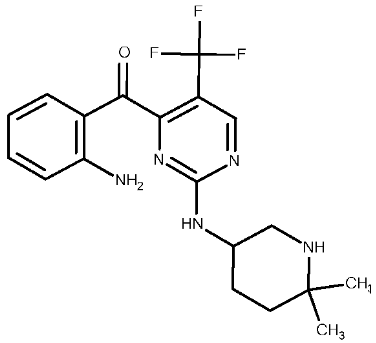 | **CDK7_1000_a**<br>4-[5-(2-methyl-2H-1,2,3,4-tetrazol-5-yl)-1H-pyrrol-3-yl]-N-(piperidin-3-yl)-5-(trifluoromethyl)p yrimidin-2-amine | 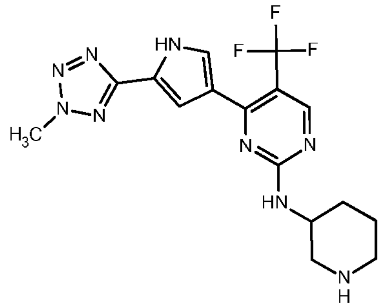 |
| **CDK7_549**<br>4-(2-aminobenzoyl) -N-[(3S)-piperidin-3-yl]-5-(trifluoromethy l)pyrimidin-2-amine | 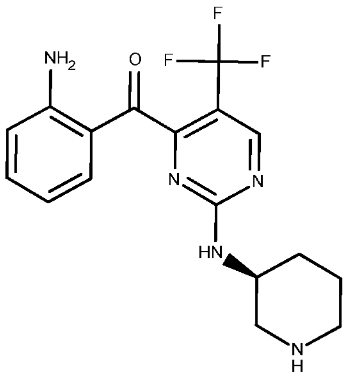 | **CDK7_1060**<br>4-[5-(5-methyl-1,2,4-oxadiazol-3-yl)-1H-pyrrol-3-yl]-N-[(3S)-piperidin-3-yl]-5-(trifluoromethyl)p yrimidin-2-amine | 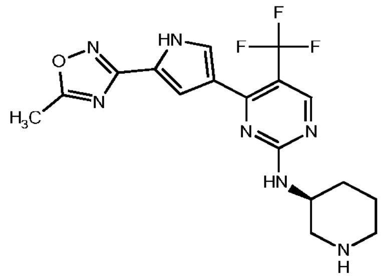 |

(continued)

| Code Name | Structure | Code Name | Structure |
|---|---|---|---|
| **CDK7_549_a**<br>4-(2-aminobenzoyl) -N-(piperidin-3-yl)-5-(trifluoromethy l)pyrimidin-2-amine | 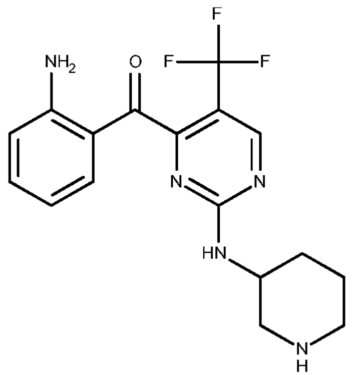 | **CDK7_1060_a**<br>4-[5-(5-methyl-1,2,4-oxadiazol-3-yl)-1H-pyrrol-3-yl]-N-(piperidin-3-yl)-5-(trifluoromethyl)pyrimidin-2-amine | 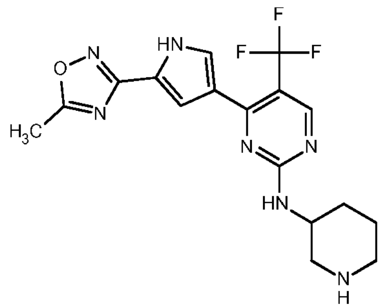 |
| **CDK7_813**<br>4-benzoyl-N-[(3S)-piperidin-3-yl]-5-(trifluoromethy l)pyrimidin-2-amine | 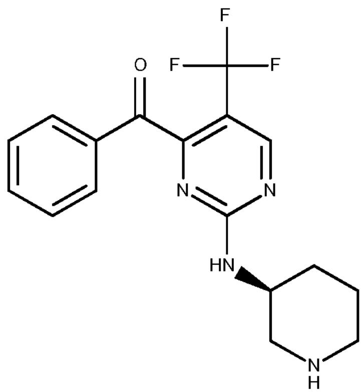 | **CDK7_1102**<br>4-[5-(5-methyl-1,2,4-oxadiazol-3-yl)-1H-pyrrol-3-yl]-N-(piperidin-3-yl)-5-(trifluoromethyl)pyrimidin-2-amine | 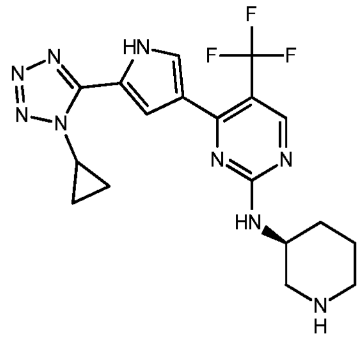 |

(continued)

| Code Name | Structure | Code Name | Structure |
|---|---|---|---|
| **CDK7_813_a** 4-benzoyl-N-(piperidin-3-yl)-5-(trifluoromethy l)pyrimidin-2-amine | 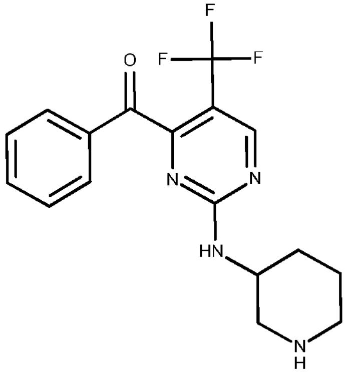 | **CDK7_1102_a** 4-[5-(1-cyclopropyl-1H-1,2,3,4-tetrazol-5-yl)-1H-pyrrol-3-yl]-N-[(3S)-piperidin-3-yl]-5-(trifluoromethyl)p yrimidin-2-amine | 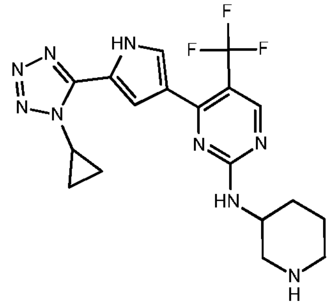 |
| **CDK7_540** 4-[(2-aminophenyl) methyl]-N-[(3S)-piperidin-3-yl]-5-(trifluoromethy l)pyrimi-din-2-amine | 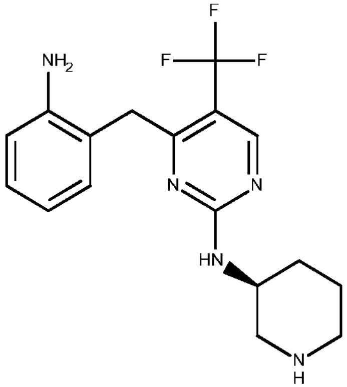 | **CDK7_1103** 4-[5-(1-cyclopropyl-1H-1,2,3,4-tetrazol-5-yl)-1H-pyrrol-3-yl]-N-(piperidin-3-yl)-5-(trifluoromethyl)p yrimidin-2-amine | 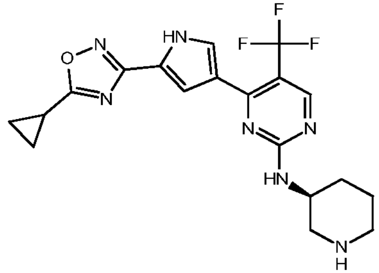 |

(continued)

| Code Name | Structure | Code Name | Structure |
|---|---|---|---|
| **CDK7_540_a** 4-[(2-aminophenyl) methyl]-N-[(3S)-piperidin-3-yl]-5-(trifluoromethy l)pyrimidin-2-amine | 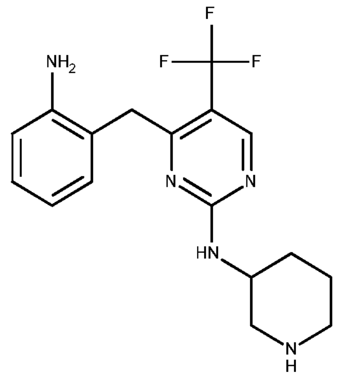 | **CDK7_1103_a** 4-[5-(5-cyclopropyl-1,2,4-oxadiazol-3-yl)-1H-pyrrol-3-yl]-N-(piperidin-3-yl)-5-(trifluoromethyl)p yrimidin-2-amine | 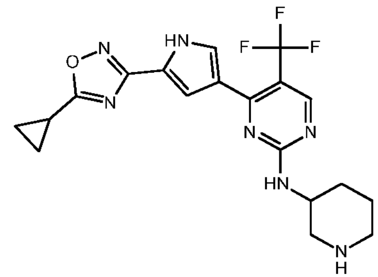 |
| **CDK7_541** 4-[1-(2-aminophenyl)e thyl]-N-[(3S)-piperidin-3-yl]-5-(trifluoromethy l)pyrimidin-2-amine | 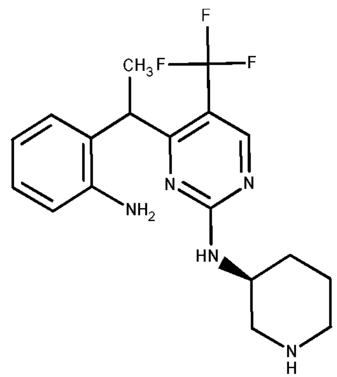 | **CDK7_1124** 4-[5-(1-methyl-1H-1,3-benzodiazol-2-yl)-1H-pyrrol-3-yl]-N-[(3S)-piperidin-3-yl]-5-(trifluoromethyl)p yrimidin-2-amine | 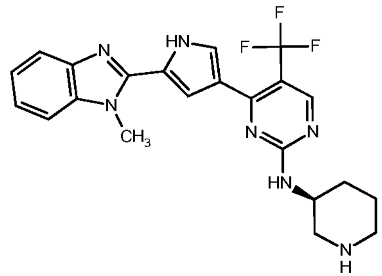 |
| **CDK7_541_a** 4-[1-(2-aminophenyl)e thyl]-N-(piperidin-3-yl)-5-(trifluoromethy l)pyrimidin-2-amine | 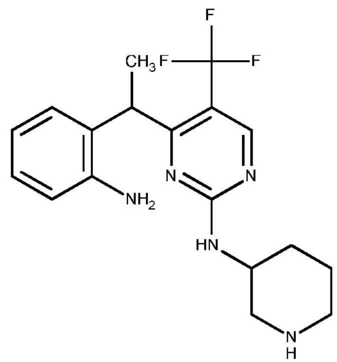 | **CDK7_1124a** 4-[5-(1-methyl-1H-1,3-benzodiazol-2-yl)-1H-pyrrol-3-yl]-N-(piperidin-3-yl)-5-(trifluoromethyl)p yrimidin-2-amine | 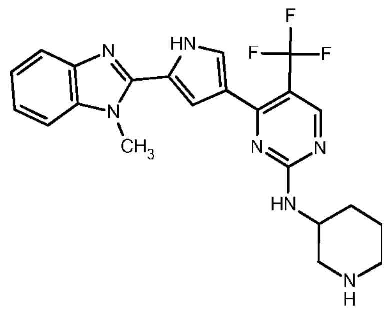 |

(continued)

| Code Name | Structure | Code Name | Structure |
|---|---|---|---|
| **CDK7_541_b** 4-[(1S)-1-(2-aminophenyl)e thyl]-N-[(3S)-piperidin-3-yl]-5-(trifluoromethy l)pyrimidin-2-amine | 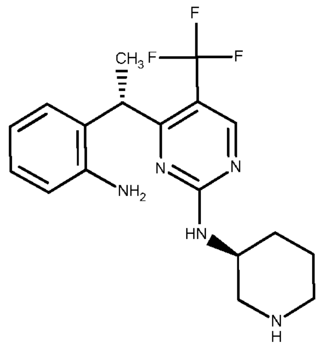 | **CDK7_1020** ethyl 4-(2-{[(3S)-piperidin-3-yl]amino}-5-(trifluoromethyl)p yrimidin-4-yl)-1H-pyrrole-2-carboxylate | 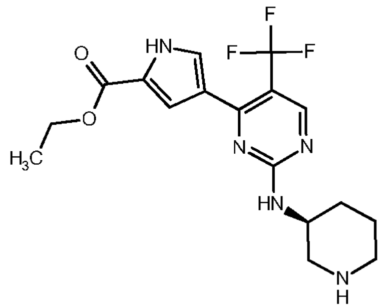 |
| **CDK7_541_c** 4-[(1R)-1-(2-aminophenyl)e thyl]-N-[(3S)-piperidin-3-yl]-5-(trifluoromethy l)pyrimidin-2-amine | 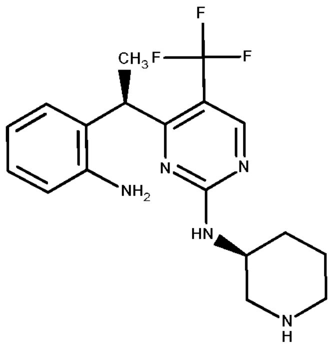 | **CDK7_1020_a** ethyl 4-{2-[(piperidin-3-yl)amino]-5-(trifluoromethyl)p yrimidin-4-yl }-1H-pyrrole-2-carboxy-late | 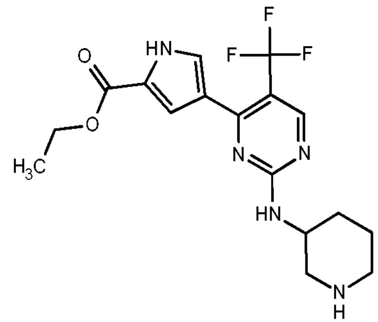 |
| **CDK7_542** 4-[2-(2-aminophenyl)p ropan-2-yl]-N-[(3S)-piperidin-3-yl]-5-(trifluoromethy l)pyrimidin-2-amine | 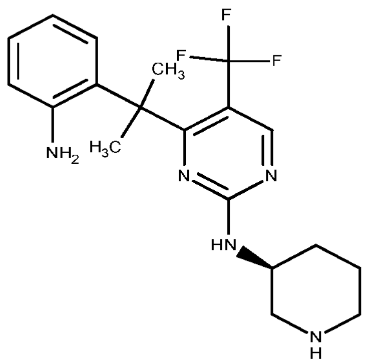 | **CDK7_1017** N,N, 1-trimethyl-4-(2-{[(3 S)-piperidin-3-yl] amino}-5-(trifluoromethyl)p yrimidin-4-yl)-1H-pyrrole-2-carboxamide | 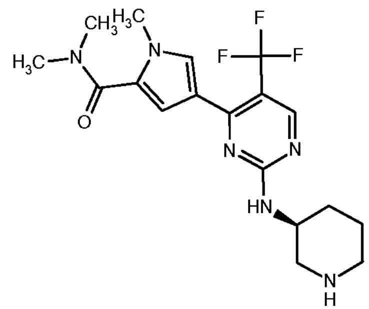 |

(continued)

| Code Name | Structure | Code Name | Structure |
|---|---|---|---|
| **CDK7_542_a** 4-[2-(2-aminophenyl)p ropan-2-yl]-N-(piperidin-3-yl)-5-(trifluoromethy l)pyrimidin-2-amine | 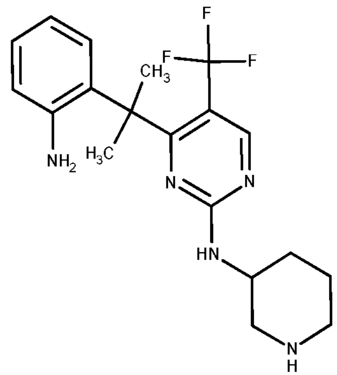 | **CDK7_1017_a** N,N,1-trimethyl-4-(2-{[piperidin-3-yl]amino}-5-(trifluoromethyl)p yrimidin-4-yl)-1H-pyrrole-2-carboxamide | 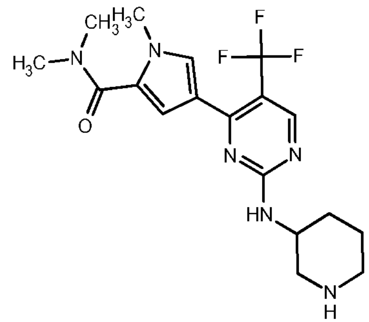 |
| **CDK7_543** 4-[1-(2-aminophenyl)c yclopropyl]-N-[(3S)-piperidin-3-yl]-5-(trifluoromethy l)pyrimidin-2-amine | 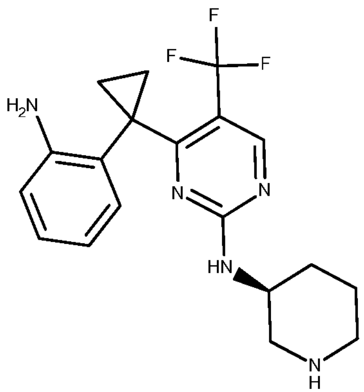 | **CDK7_1061** N-[(3S)-piperidin-3-yl]-4-[5-(pyridin-2-yl)-1H-pyrrol-3-yl]-5-(trifluoromethyl)p yrimidin-2-amine | 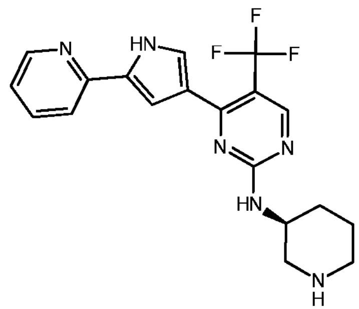 |

(continued)

| Code Name | Structure | Code Name | Structure |
|---|---|---|---|
| **CDK7_543_a**<br>4-[1-(2-aminophenyl)c yclopropyl]-N-(piperidin-3-yl)-5-(trifluoromethy l)pyrimidin-2-amine | 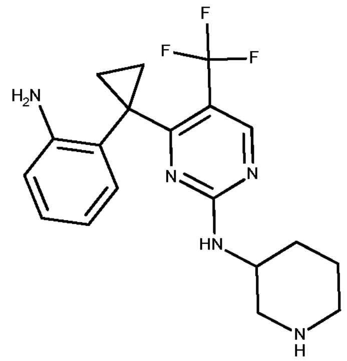 | **CDK7_1061_a**<br>N-(piperidin-3-yl)-4-[5-(pyridin-2-yl)-1H-pyrrol-3-yl]-5-(trifluoromethyl)p yrimidin-2-amine | 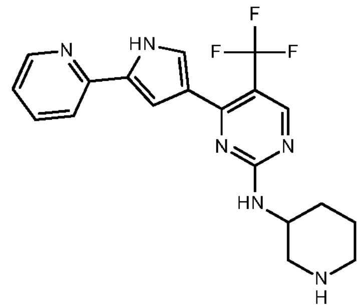 |
| **CDK7_544**<br>4-[(2-aminophenyl)d ifluoromethyl]-N-[(3S)-piperidin-3-yl]-5-(trifluoromethy l)pyrimidin-2-amine | 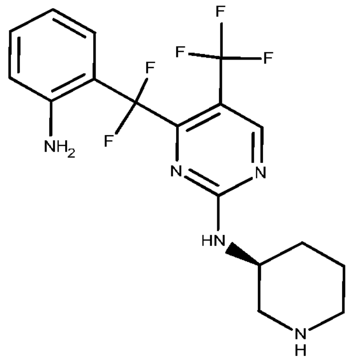 | **CDK7_1062**<br>N-[(3S)-piperidin-3-yl]-4-[5-(pyrimidin-2-yl)-1H-pyrrol-3-yl]-5-(trifluoromethyl)p yrimidin-2-amine | 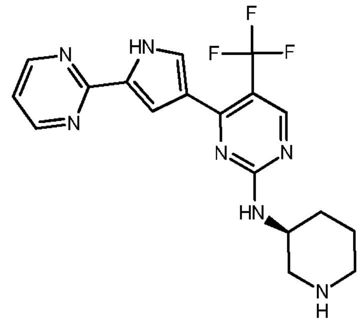 |
| **CDK7_544_a**<br>4-[(2-aminophenyl)d ifluoromethyl]-N-(piperidin-3-yl)-5-(trifluoromethy l)pyrimidin-2-amine | 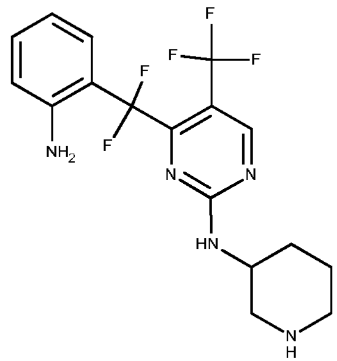 | **CDK7_1062_a**<br>N-(piperidin-3-yl)-4-[5-(pyrimidin-2-yl)-1H-pyrrol-3-yl]-5-(trifluoromethyl)p yrimidin-2-amine | 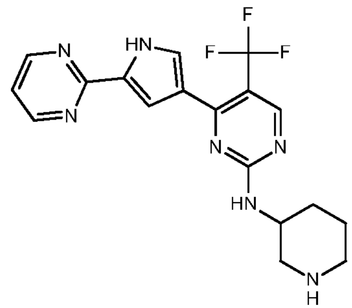 |

(continued)

| Code Name | Structure | Code Name | Structure |
|---|---|---|---|
| **CDK7_545**<br>4-[(2-aminophenyl)s ulfanyl]-N-[(3S)-piperidin-3-yl]-5-(trifluoromethy l)pyrimidin-2-amine | 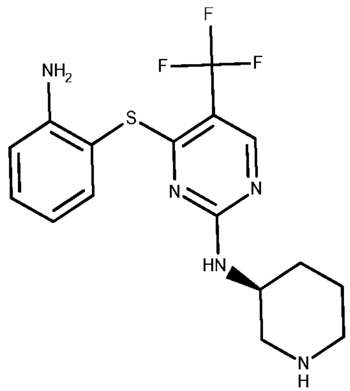 | **CDK7_1057**<br>4-{5-[2-(dimethylphospho ryl)phenyl]-1H-pyrrol-3-yl}-N-[(3S)-piperidin-3-yl]-5-(trifluoromethyl)p yrimidin-2-amine | 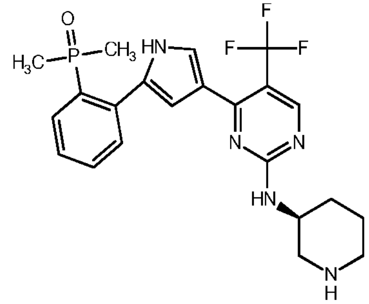 |
| **CDK7_545_a**<br>4-[(2-aminophenyl)s ulfanyl]-N-(piperidin-3-yl)-5-(trifluoromethy l)pyrimi-din-2-amine | 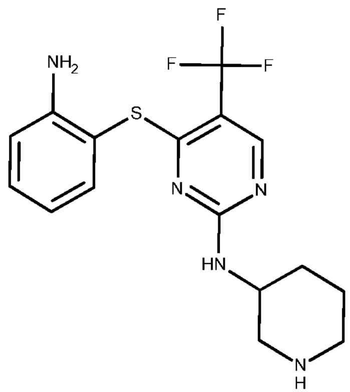 | **CDK7_1057_a**<br>4-{5-[2-(dimethylphospho ryl)phenyl]-1H-pyrrol-3-yl}-N-(piperidin-3-yl)-5-(trifluoromethyl)p yrimidin-2-amine | 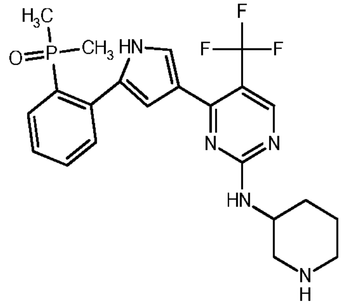 |

(continued)

| Code Name | Structure | Code Name | Structure |
|---|---|---|---|
| **CDK7_546**<br>4-(2-aminobenzenes ulfi-nyl)-N-[(3S)-piperidin-3-yl]-5-(trifluoromethy l)pyrimidin-2-amine | 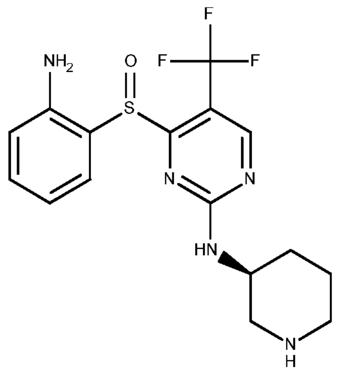 | **CDK7_955**<br>7-(1-methyl-1H-pyrazol-4-yl)-3-(2-{[(3S)-piperidin-3-yl]amino}-5-(trifluoromethyl)p yrimidin-4-yl)-1H,4H,5H,6H,7H, 8H-pyrrolo[2,3-c] azepin-8-one | 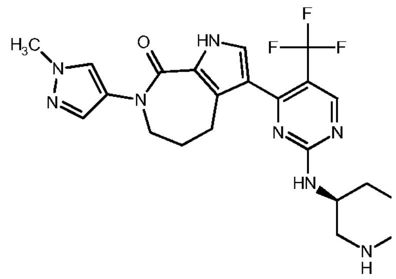 |
| **CDK7_546_a**<br>4-(2-aminobenzenes ulfinyl)-N-(piperidin-3-yl)-5-(trifluoromethy l)pyrimi-din-2-amine | 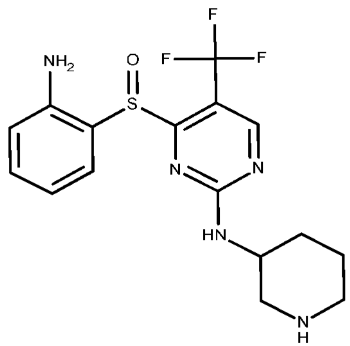 | **CDK7_955_a**<br>7-(1-methyl-1H-pyrazol-4-yl)-3-{2-[(piperidin-3-yl)amino]-5-(trifluoromethyl)p yrimidin-4-yl}-1H,4H,5H,6H,7H, 8H-pyrrolo[2,3-c]aze-pin-8-one | 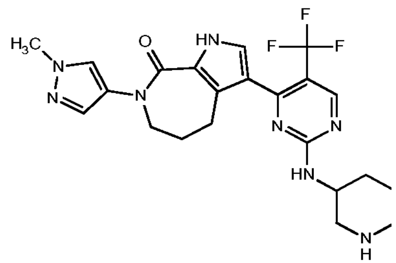 |
| **CDK7_546_b**<br>4-[(R)-2-aminobenzenes ulfinyl]-N-[(3S)-piperidin-3-yl]-5-(trifluoromethy l)pyrimidin-2-amine | 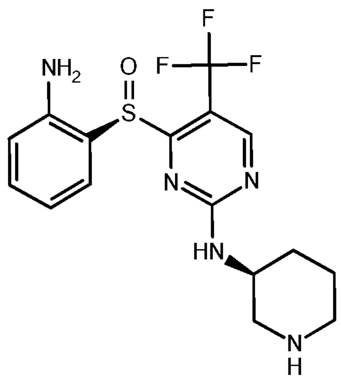 | **CDK7_1122**<br>3-(2-{[(3S)-6,6-dimethylpiperidin -3-yl]amino}-5-(trifluoromethyl)p yrimidin-4-yl)-7-(1-methyl-1H-pyrazol-4-yl)-1H,4H,5H,6H,7H, 8H-pyrrolo[2,3-c]azepin-8-one | 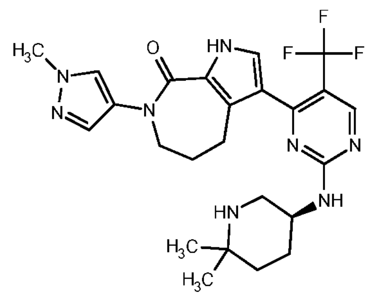 |

(continued)

| Code Name | Structure | Code Name | Structure |
|---|---|---|---|
| **CDK7_546_c** 4-[(S)-2-aminobenzenes ulfinyl]-N-[(3S)-piperidin-3-yl]-5-(trifluoromethy l)pyrimidin-2-amine | 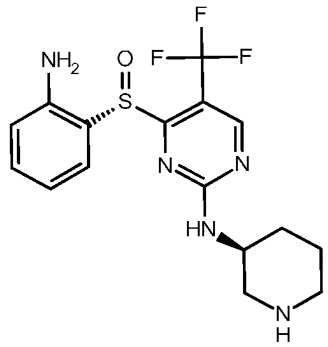 | **CDK7_1122_a** 3-{2-[(6,6-dimethylpiperidin -3-yl)amino]-5-(trifluoromethyl)p yrimidin-4-yl}-7-(1-methyl-1H-pyrazol-4-yl)-1H,4H,5H,6H,7H, 8H-pyrrolo [2,3-c]azepin-8-one | 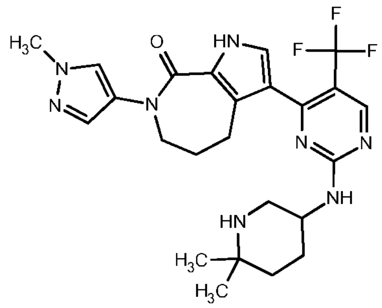 |
| **CDK7_547** 4-(2-aminobenzenes ulfonyl)-N-[(3S)-piperidin-3-yl]-5-(trifluoromethy l)pyrimidin-2-amine | 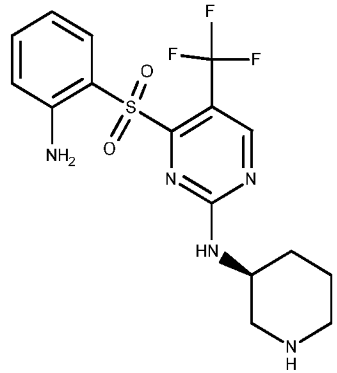 | **CDK7_1123** 3-(2-{[(3S)-6,6-dimethylpiperidin -3-yl]amino}-5-(trifluoromethyl)p yrimidin-4-yl)-6-(1-methyl-1H-pyrazol-4-yl)-1H,6H,7H-pyrrolo [2,3-c]pyridin-7-one | 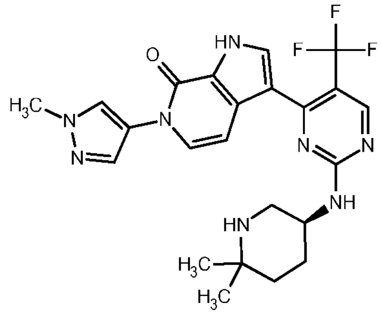 |
| **CDK7_547_a** 4-(2-aminobenzenes ulfonyl)-N-(piperidin-3-yl)-5-(trifluoromethy l)pyrimidin-2-amine | 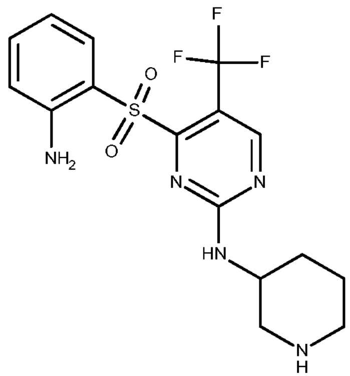 | **CDK7_1123_a** 3-{2-[(6,6-dimethylpiperidin -3-yl)amino]-5-(trifluoromethyl)p yrimidin-4-yl}-6-(1-methyl-1H-pyrazol-4-yl)-1H,6H,7H-pyrrolo[2,3-c]pyridin-7-one | 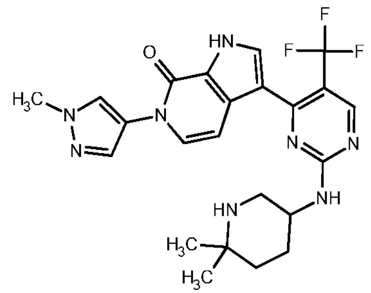 |

(continued)

| Code Name | Structure | Code Name | Structure |
|---|---|---|---|
| **CDK7_548**<br>4-(2-aminophenoxy )-N-[(3S)-piperidin-3-yl]-5-(trifluoromethy l)pyrimidin-2-amine | 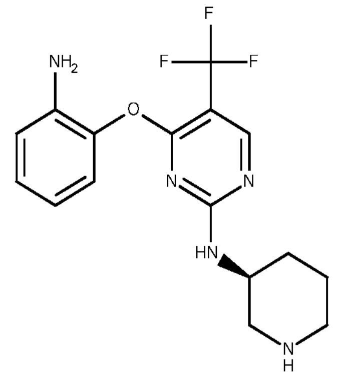 | **CDK7_1136**<br>3-(2-{[(6S)-4-azaspiro[2.5]octa n-6-yl]amino}-5-(trifluoromethyl)p yrimidin-4-yl)-7-(1-methyl-1H-pyrazol-4-yl)-1H,4H,5H,6H,7H, 8H-pyrrolo[2,3-c]azepin-8-one | 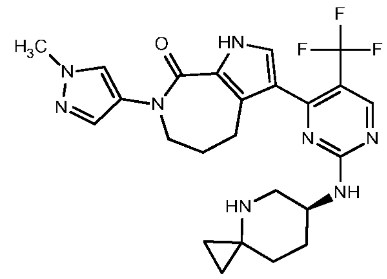 |
| **CDK7_548_a**<br>4-(2-aminophenoxy )-N-(piperidin-3-yl)-5-(trifluoromethy l)pyrimidin-2-amine | 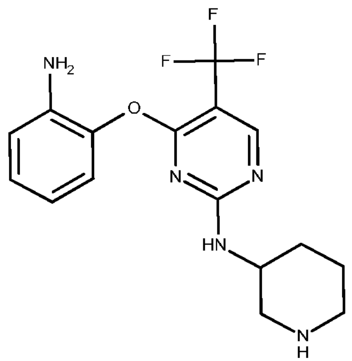 | **CDK7_1136_a**<br>3-[2-({4-azaspiro[2.5]octa n-6-yl}amino)-5-(trifluoromethyl)p yrimidin-4-yl]-7-(1-methyl-1H-pyrazol-4-yl)-1H,4H,5H,6H,7H, 8H-pyrrolo [2,3-c]azepin-8-one | 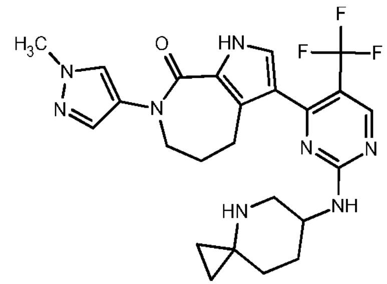 |
| **CDK7_550**<br>N4-(2-aminophenyl)-N2-[(3S)-piperidin-3-yl]-5-(trifluoromethy l)pyrimidine-2,4-diamine | 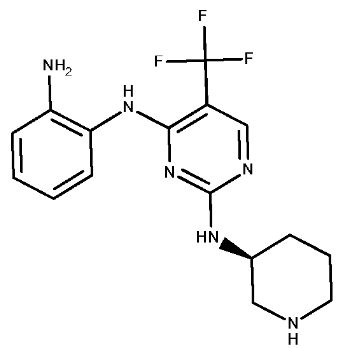 | **CDK7_1137**<br>3-(2-{[(7S)-5-azaspiro[3.5]nona n-7-yl]amino}-5-(trifluoromethyl)p yrimidin-4-yl)-7-(1-methyl-1H-pyrazol-4-yl)-1H,4H,5H,6H,7H, 8H-pyrrolo[2,3-c]azepin-8-one | 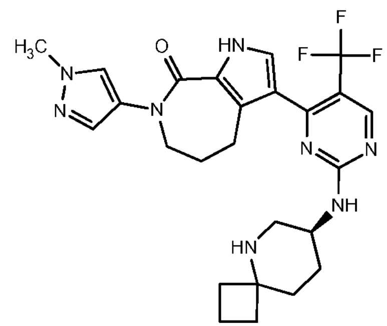 |

(continued)

| Code Name | Structure | Code Name | Structure |
|---|---|---|---|
| **CDK7_550_a** N4-(2-aminophenyl)-N2-(piperidin-3-yl)-5-(trifluoromethy l)pyrimidine-2,4-diamine | 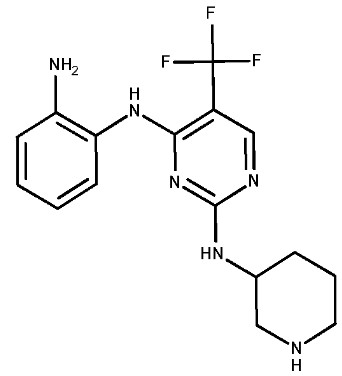 | **CDK7_1137_a** 3-[2-({5-azaspiro[3.5]nona n-7-yl}amino)-5-(trifluoromethyl)p yrimidin-4-yl]-7-(1-methyl-1H-pyrazol-4-yl)-1H,4H,5H,6H,7H, 8H-pyrrolo[2,3-c]azepin-8-one | 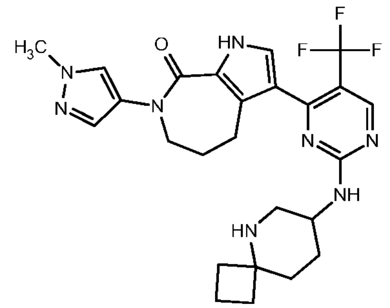 |
| **CDK7_551** N4-(2-aminophenyl)-N4-methyl-N2-[(3S)-piperidin-3-yl]-5-(trifluoromethy l)pyrimidine-2,4-diamine | 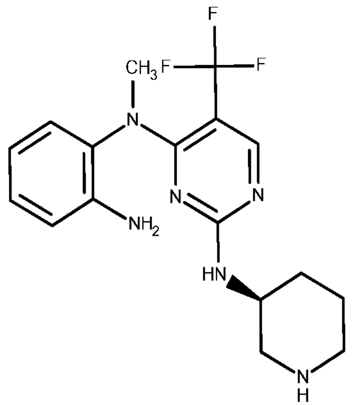 | **CDK7_1138** 3-(2-{[(3S)-6,6-dimethylpiperidin -3-yl]amino}-5-(trifluoromethyl)p yrimidin-4-yl)-7-(1,3-thiazol-2-yl)-1H,4H,5H,6H,7H, 8H-pyrrolo[2,3-c]azepin-8-one | 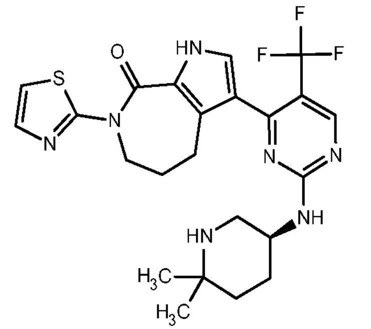 |

(continued)

| Code Name | Structure | Code Name | Structure |
| --- | --- | --- | --- |
| **CDK7_551_a** N4-(2-aminophenyl)-N4-methyl-N2-(piperidin-3-yl)-5-(trifluoromethy l) pyrimidine-2,4-diamine | 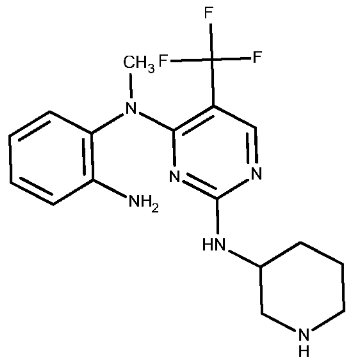 | **CDK7_1138_a** 3-{2-[(6,6-dimethylpiperidin -3-yl)amino]-5-(trifluoromethyl)p yrimidin-4-yl}-7-(1,3-thiazol-2-yl)-1H,4H,5H,6H,7H, 8H-pyrrolo[2,3-c]azepin-8-one | 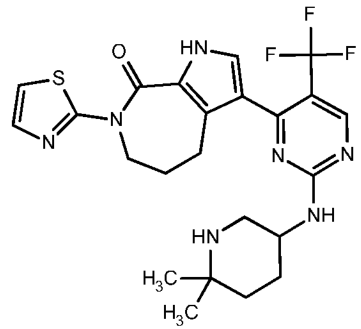 |
| **CDK7_812** N4-phenyl-N2-[(3S)-piperidin-3-yl]-5-(trifluoromethy l)pyrimidine-2,4-diamine | 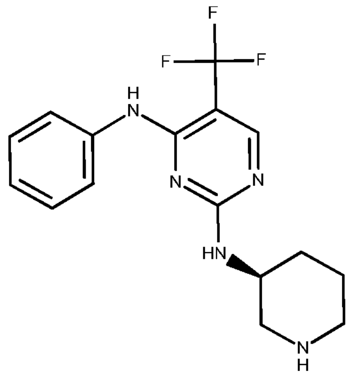 | **CDK7_1139** 7-(3,5-dimethyl-1,2-oxazol-4-yl)-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]azepin-8-one | 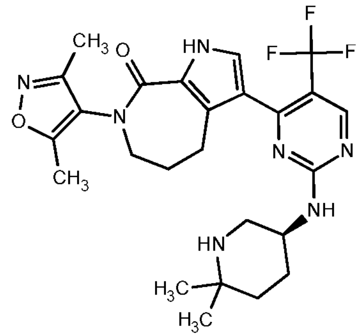 |
| **CDK7_812_a** N4-phenyl-N2-(piperidin-3-yl)-5-(trifluoromethy l)pyrimidine-2,4-diamine | 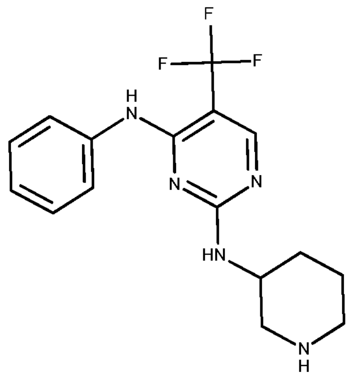 | **CDK7_1139_a** 7-(3,5-dimethyl-1,2-oxazol-4-yl)-3-{2-[(6,6-dimethylpiperidin -3-yl)amino]-5-(trifluoromethyl)p yrimidin-4-yl }-1H,4H,5H,6H,7H, 8H-pyrrolo[2,3-c]azepin-8-one | 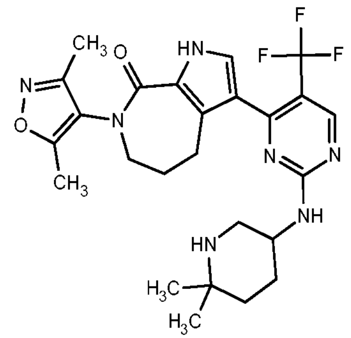 |

(continued)

| Code Name | Structure | Code Name | Structure |
|---|---|---|---|
| **CDK7_754** 4-phenoxy-N-[(3S)-piperidin-3-yl]-5-(trifluoromethy l)pyrimidin-2-amine | 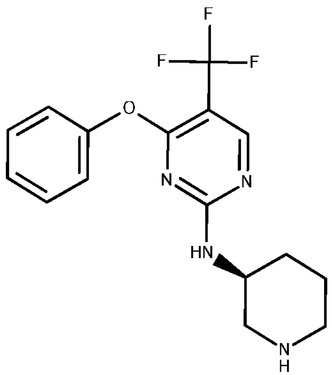 | **CDK7_1140** 5-[3-(2-{[(3S)-6,6-dimethylpiperidin -3-yl]amino}-5-(trifluoromethyl)p yrimidin-4-yl)-8-oxo-1H,4H,5H,6H,7H, 8H-pyrrolo[2,3-c]azepin-7-yl]-1-methyl-1,2-dihydropyridin-2-one | 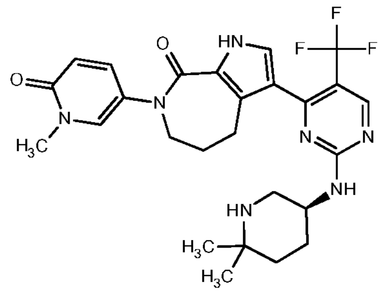 |
| **CDK7_754_a** 4-phenoxy-N-(piperidin-3-yl)-5-(trifluoromethy l)pyrimidin-2-amine | 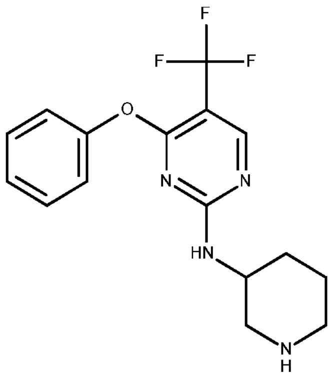 | **CDK7_1140_a** 5-(3-{2-[(6,6-dimethylpiperidin -3-yl)amino]-5-(trifluoromethyl)p yrimidin-4-yl}-8-oxo-1H,4H,5H,6H,7H, 8H-pyrrolo[2,3-c]azepin-7-yl)-1-methyl-1,2-dihydropyridin-2-one | 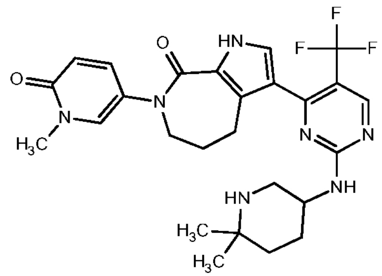 |
| **CDK7_766** 4-benzyl-N-[(3S)-piperidin-3-yl]-5-(trifluoromethy l)pyrimidin-2-amine | 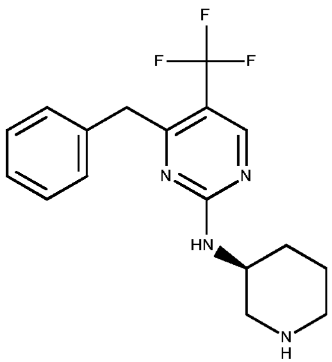 | **CDK7_1141** 3-(2-{[(3S)-6,6-dimethylpiperidin -3-yl]amino}-5-(trifluoromethyl)p yrimidin-4-yl)-7-(pyridin-4-yl)-1H,4H,5H,6H,7H, 8H-pyrrolo[2,3-c] azepin-8-one | 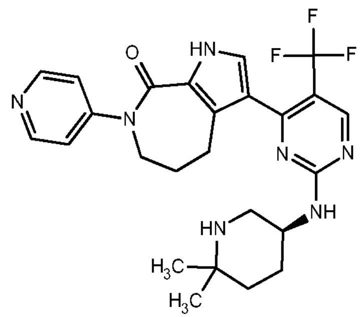 |

(continued)

| Code Name | Structure | Code Name | Structure |
|---|---|---|---|
| **CDK7_766_a** 4-benzyl-N-(piperidin-3-yl)-5-(trifluoromethy l)pyrimidin-2-amine | 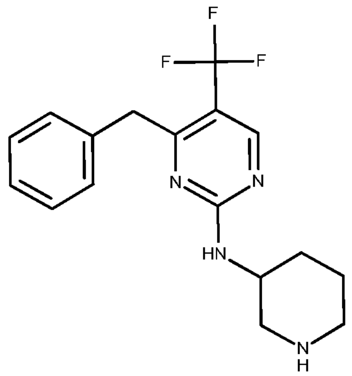 | **CDK7_1141_a** 3-{2-[(6,6-dimethylpiperidin -3-yl)amino]-5-(trifluoromethyl)p yrimidin-4-yl}-7-(pyridin-4-yl)-1H,4H,5H,6H,7H, 8H-pyrrolo[2,3-c]aze-pin-8-one | 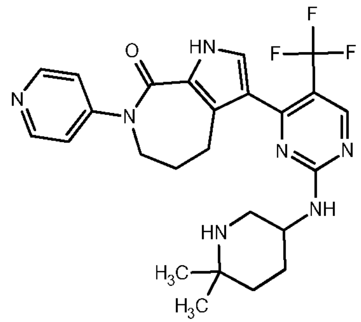 |
| **CDK7_552** 4-[(2-aminophenyl)( methyl)phosph oroso]-N-[(3S)-piperidin-3-yl]-5-(trifluoromethy l)pyrimidin-2-amine | 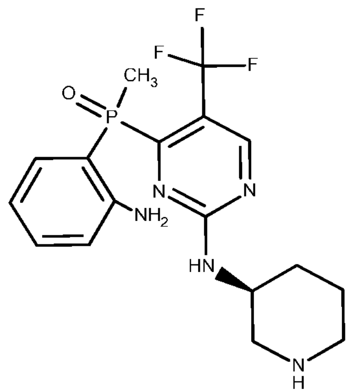 | **CDK7_1142** 3-(2-{[(3S)-6,6-dimethylpiperidin -3-yl]amino}-5-(trifluoromethyl)p yrimidin-4-yl)-7-(pyridin-3-yl)-1H,4H,5H,6H,7H, 8H-pyrrolo[2,3-c] azepin-8-one | 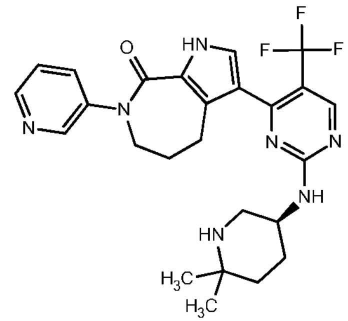 |

(continued)

| Code Name | Structure | Code Name | Structure |
|---|---|---|---|
| **CDK7_552_a** 4-[(2-aminophenyl)( methyl)phosph oroso]-N-(piperidin-3-yl)-5-(trifluoromethy l)pyrimidin-2-amine | 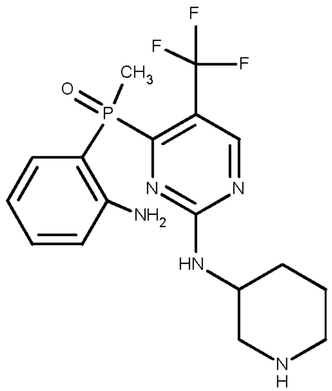 | **CDK7_1142_a** 3-{2-[(6,6-dimethylpiperidin -3-yl)amino]-5-(trifluoromethyl)p yrimidin-4-yl}-7-(pyridin-3-yl)-1H,4H,5H,6H,7H, 8H-pyrrolo[2,3-c]aze-pin-8-one | 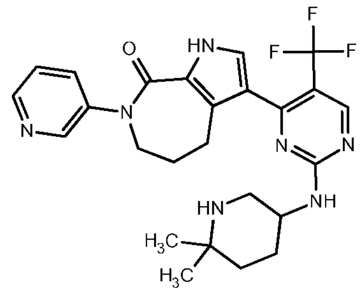 |
| **CDK7_552_b** 4-[(R)-(2-aminophenyl)( methyl) phosph oroso]-N-[(3S)-piperidin-3-yl]-5-(trifluoromethy l)pyrimidin-2-amine | 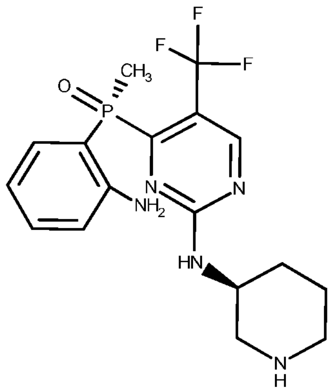 | **CDK7_1143** 3-(2-{[(3S)-6,6-dimethylpiperidin -3-yl]amino}-5-(trifluoromethyl)p yrimidin-4-yl)-7-(pyridin-2-yl)-1H,4H,5H,6H,7H, 8H-pyrrolo[2,3-c] azepin-8-one | 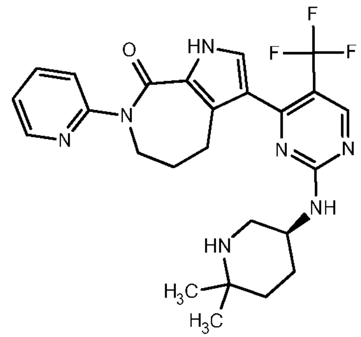 |

(continued)

| Code Name | Structure | Code Name | Structure |
|---|---|---|---|
| **CDK7_552_c** 4-[(S)-(2-aminophenyl)( methyl) phosph oroso]-N-[(3S)-piperidin-3-yl]-5-(trifluoromethy l)pyrimidin-2-amine | 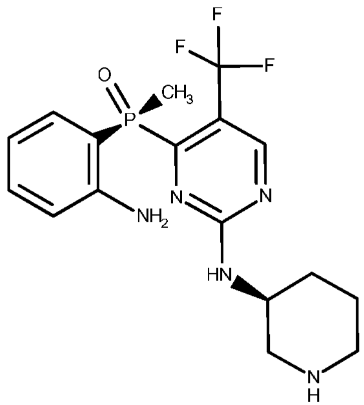 | **CDK7_1143_a** 3-{2-[(6,6-dimethylpiperidin -3-yl)amino]-5-(trifluoromethyl)p yrimidin-4-yl}-7-(pyridin-2-yl)-1H,4H,5H,6H,7H, 8H-pyrrolo[2,3-c]azepin-8-one | 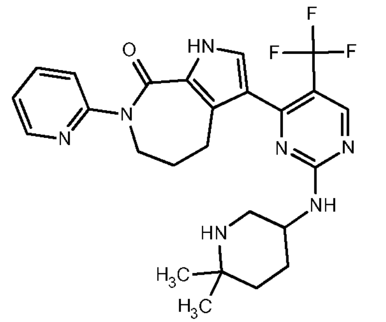 |
| **CDK7_735** N4-[2-(dimethylphos phoryl)phenyl] -N2-[(3S)-piperidin-3-yl]-5-(trifluoromethy l)pyrimidine-2,4-diamine | 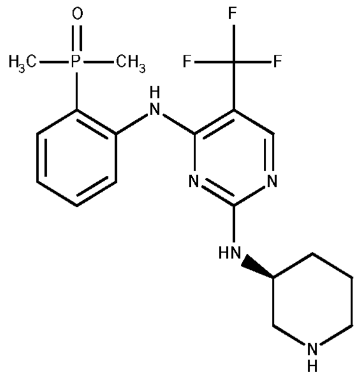 | **CDK7_1144** 3-(2-{[(3S)-6,6-dimethylpiperidin -3-yl]amino}-5-(trifluoromethyl)p yrimidin-4-yl)-7-[(1-methyl-1H-pyrazol-4-yl) methyl]-1H,4H,5H,6H,7H, 8H-pyrrolo[2,3-c] azepin-8-one | 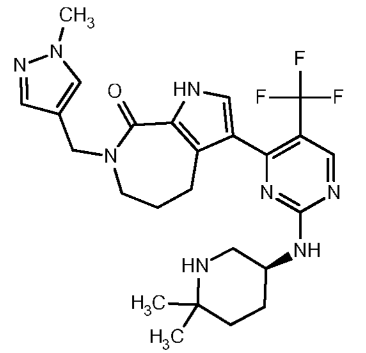 |

(continued)

| Code Name | Structure | Code Name | Structure |
|---|---|---|---|
| **CDK7_735_a** N4-[2-(dimethylphos phoryl)phenyl] -N2-(piperidin-3-yl)-5-(trifluoromethy l) pyrimidine-2,4-diamine | 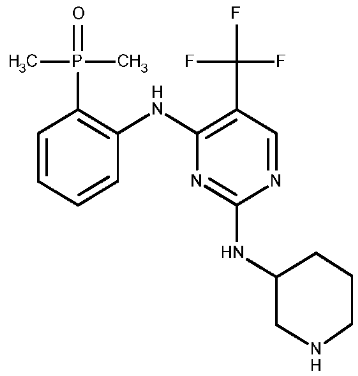 | **CDK7_1144_a** 3-{2-[(6,6-dimethylpiperidin -3-yl)amino]-5-(trifluoromethyl)p yrimidin-4-yl}-7-[(1-methyl-1H-pyrazol-4-yl)methyl]-1H,4H,5H,6H,7H, 8H-pyrrolo[2,3-c]azepin-8-one | 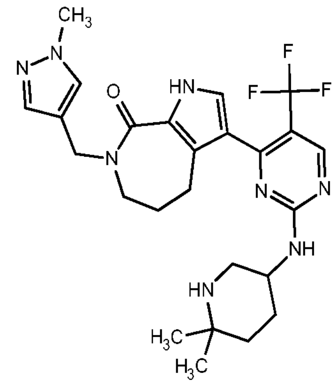 |
| **CDK7_897** 4-[2-(dimethylphos phoryl)phenox y]-N-[(3S)-piperidin-3-yl]-5-(trifluoromethy l)pyrimidin-2-amine | 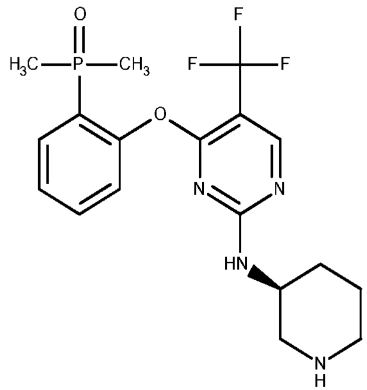 | **CDK7_1145** 3-(2-{[(3S)-6,6-dimethylpiperidin -3-yl]amino}-5-(trifluoromethyl)p yrimidin-4-yl)-7-[(1,3-thiazol-2-yl)methyl]-1H,4H,5H,6H,7H, 8H-pyrrolo[2,3-c]azepin-8-one | 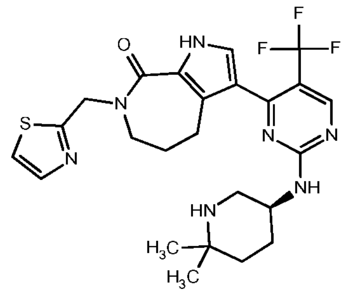 |

(continued)

| Code Name | Structure | Code Name | Structure |
|---|---|---|---|
| **CDK7_897_a** 4-[2-(dimethylphos phoryl)phenox y]-N-[piperidin-3-yl]-5-(trifluoromethy l) pyrimidin-2-amine | 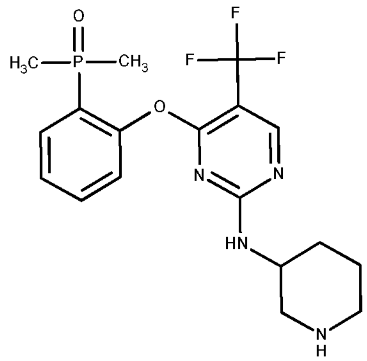 | **CDK7_1145_a** 3-{2-[(6,6-dimethylpiperidin -3-yl)amino]-5-(trifluoromethyl)p yrimidin-4-yl}-7-[(1,3-thiazol-2-yl)methyl]-1H,4H,5H,6H,7H, 8H-pyrrolo[2,3-c] azepin-8-one | 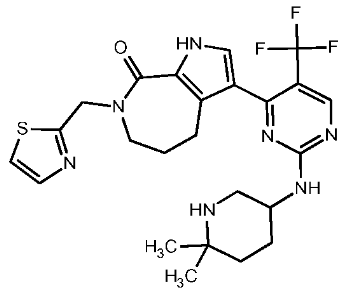 |
| **CDK7_892** 4-[3-(dimethylphos phoryl)phenox y]-N-[(3S)-piperidin-3-yl]-5-(trifluoromethy l)pyrimidin-2-amine | 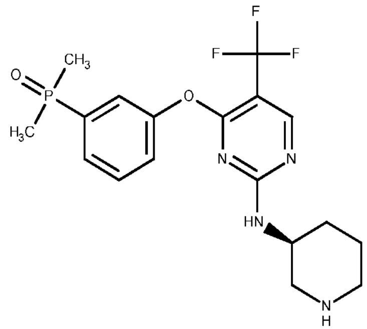 | **CDK7_1146** 7-[(3,5-dimethyl-1,2-oxazol-4-yl) methyl]-3-(2-{[(3S)-6,6-dimethylpiperidin -3-yl] amino}-5-(trifluoromethyl)p yrimidin-4-yl)-1H,4H,5H,6H,7H, 8H-pyrrolo[2,3-c]aze-pin-8-one | 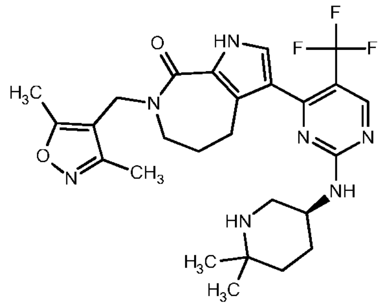 |
| **CDK7_892_a** 4-[3-(dimethylphos phoryl)phenox y]-N-(piperidin-3-yl)-5-(trifluoromethy l)pyrimidin-2-amine | 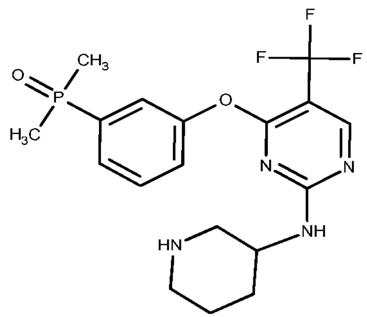 | **CDK7_1146_a** 7-[(3,5-dimethyl-1,2-oxazol-4-yl) methyl]-3-{2-[(6,6-dimethylpiperidin -3-yl)amino]-5-(trifluoromethyl)p yrimidin-4-yl}-1H,4H,5H,6H,7H, 8H-pyrrolo[2,3-c]aze-pin-8-one | 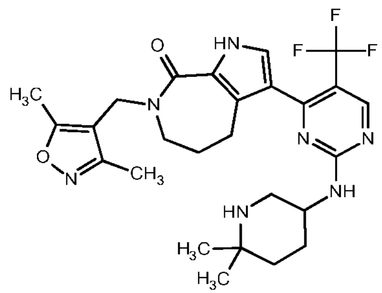 |

(continued)

| Code Name | Structure | Code Name | Structure |
|---|---|---|---|
| **CDK7_888**<br>N4-[3-(dimethylphos phoryl)phenyl] -N2-[(3S)-piperidin-3-yl]-5-(trifluoromethy l)pyrimidine-2,4-diamine | 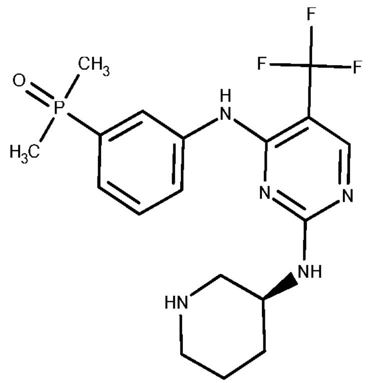 | **CDK7_1147**<br>3-(2-{[(3S)-6,6-dimethylpiperidin -3-yl]amino}-5-(trifluoromethyl)p yrimidin-4-yl)-7-[1-(1-methyl-1H-pyrazol-4-yl) ethyl]-1H,4H,5H,6H,7H, 8H-pyrrolo[2,3-c]aze-pin-8-one | 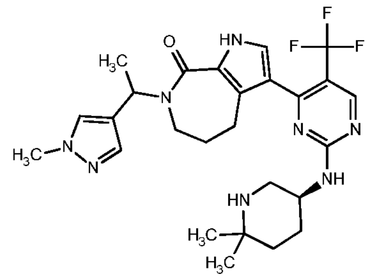 |
| **CDK7_888_a**<br>N4-[3-(dimethylphos phoryl)phenyl] -N2-(piperidin-3-yl)-5-(trifluoromethy l) pyrimidine-2,4-diamine | 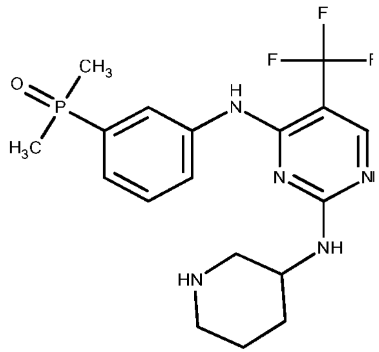 | **CDK7_1147_a**<br>3-{2-[(6,6-dimethylpiperidin -3-yl)amino]-5-(trifluoromethyl)p yrimidin-4-yl}-7-[1-(1-methyl-1H-pyrazol-4-yl) ethyl]-1H,4H,5H,6H,7H, 8H-pyrrolo[2,3-c]aze-pin-8-one | 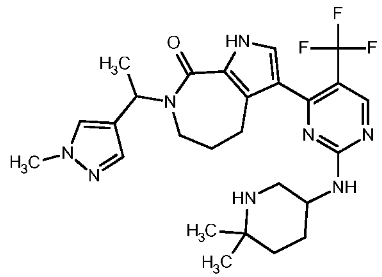 |

(continued)

| Code Name | Structure | Code Name | Structure |
|---|---|---|---|
| **CDK7_903** N4-[2-(dimethylphos phoryl)-5-methylphenyl]-N2-[(3S)-piperidin-3-yl]-5-(trifluoromethy l)pyrimidine-2,4-diamine | 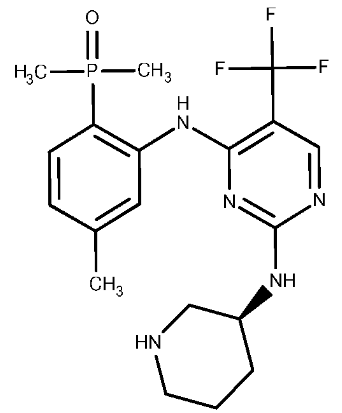 | **CDK7_1148** 3-(2-{[(3S)-6,6-dimethylpiperidin -3-yl]amino}-5-(trifluoromethyl)p yrimidin-4-yl)-7-[1-(1,3-thiazol-2-yl) ethyl]-1H,4H,5H,6H,7H, 8H-pyrrolo[2,3-c]azepin-8-one | 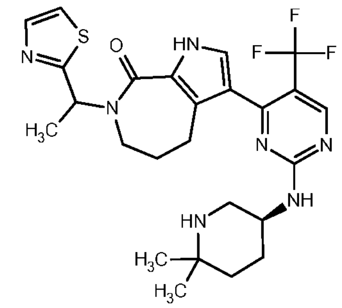 |
| **CDK7_903_a** N4-[2-(dimethylphos phoryl)-5-methylphenyl]-N2-(piperidin-3-yl)-5-(trifluoromethy l)pyrimidine-2,4-diamine | 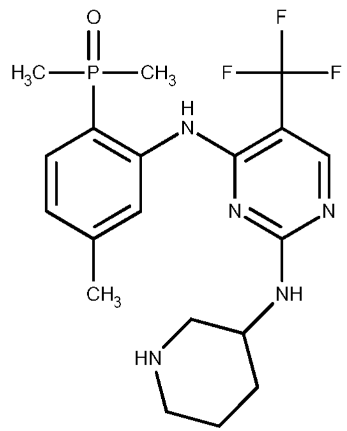 | **CDK7_1148_a** 3-{2-[(6,6-dimethylpiperidin -3-yl)amino]-5-(trifluoromethyl)p yrimidin-4-yl}-7-[1-(1,3-thiazol-2-yl)ethyl]-1H,4H,5H,6H,7H, 8H-pyrrolo [2,3-c]azepin-8-one | 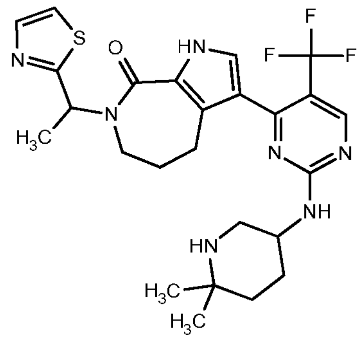 |

(continued)

| Code Name | Structure | Code Name | Structure |
|---|---|---|---|
| **CDK7_904**<br>N4-[2-(dimethylphos phoryl)-4-methylphenyl]-N2-[(3S)-piperidin-3-yl]-5-(trifluoromethy l)pyrimidine-2,4-diamine | 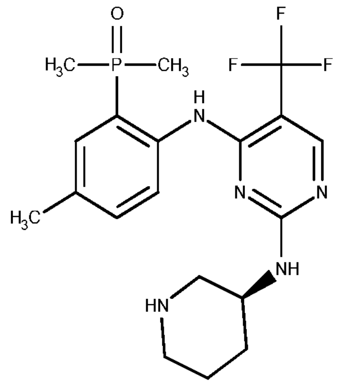 | **CDK7_1149**<br>7-[1-(3,5-dimethyl-1,2-oxazol-4-yl) ethyl]-3-(2-{[(3S)-6,6-dimethylpiperidin -3-yl] amino}-5-(trifluoromethyl)p yrimidin-4-yl)-1H,4H,5H,6H,7H, 8H-pyrrolo[2,3-c]aze-pin-8-one | 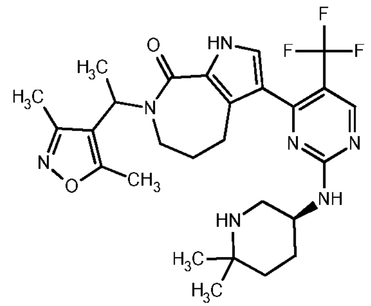 |
| **CDK7_904_a**<br>N4-[2-(dimethylphos phoryl)-4-methylphenyl]-N2-(piperidin-3-yl)-5-(trifluoromethy l)pyrimidine-2,4-diamine | 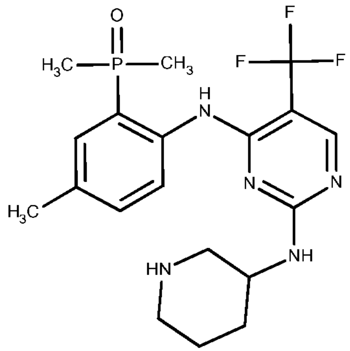 | **CDK7_1149_a**<br>7-[1-(3,5-dimethyl-1,2-oxazol-4-yl) ethyl]-3-{2-[(6,6-dimethylpiperidin -3-yl)amino]-5-(trifluoromethyl)p yrimidin-4-yl }-1H,4H,5H,6H,7H, 8H-pyrrolo[2,3-c]aze-pin-8-one | 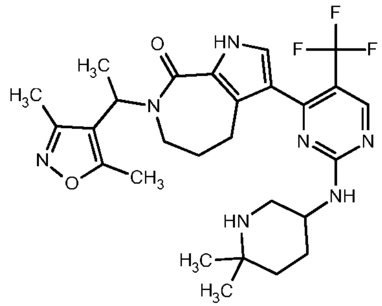 |

(continued)

| Code Name | Structure | Code Name | Structure |
|---|---|---|---|
| **CDK7_905** N4-[2-(dimethylphos phoryl)-5-(trifluoromethy l)phenyl]-N2-[(3S)-piperidin-3-yl]-5-(trifluoromethy l)pyrimi-dine-2,4-diamine | 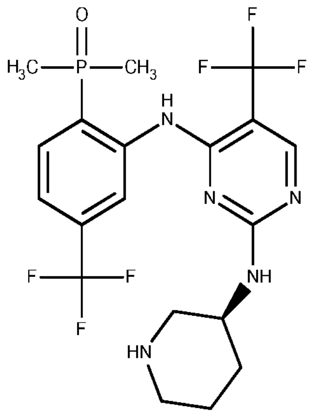 | **CDK7_1150** 3-(2-{[(3S)-6,6-dimethylpiperidin -3-yl]amino}-5-(trifluoromethyl)p yrimidin-4-yl)-7-(oxan-4-yl)-1H,4H,5H,6H,7H, 8H-pyrrolo[2,3-c] azepin-8-one | 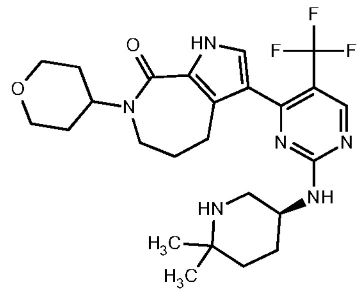 |
| **CDK7_905_a** N4-[2-(dimethylphos phoryl)-5-(trifluoromethy l)phenyl]-N2-(piperidin-3-yl)-5-(trifluoromethy l)pyrimidine-2,4-diamine | 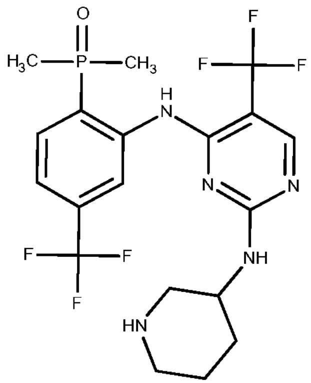 | **CDK7_1150_a** 3-{2-[(6,6-dimethylpiperidin -3-yl)amino]-5-(trifluoromethyl)p yrimidin-4-yl}-7-(oxan-4-yl)-1H,4H,5H,6H,7H, 8H-pyrrolo[2,3-c]aze-pin-8-one | 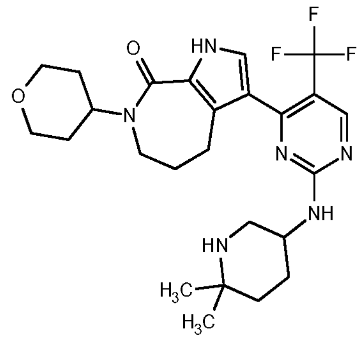 |

(continued)

| Code Name | Structure | Code Name | Structure |
|---|---|---|---|
| **CDK7_906** N4-[2-(dimethylphos phoryl)-4-(trifluoromethy l)phenyl]-N2-[(3S)-piperidin-3-yl]-5-(trifluoromethy l)pyrimi-dine-2,4-diamine | 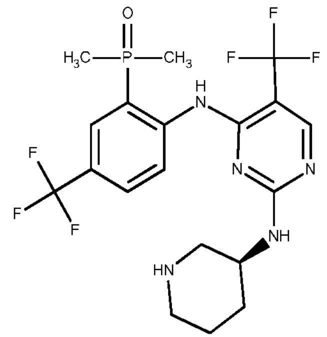 | **CDK7_1151** 3-(2-{[(3S)-6,6-dimethylpiperidin -3-yl]amino}-5-(trifluoromethyl)p yrimidin-4-yl)-7-[(oxan-4-yl)methyl]-1H,4H,5H,6H,7H, 8H-pyrrolo [2,3-c]azepin-8-one | 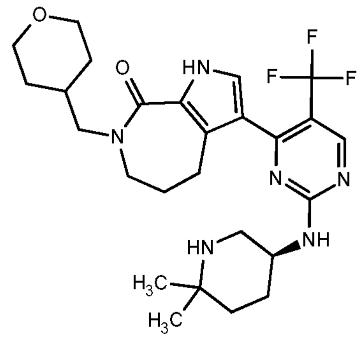 |
| **CDK7_906_a** N4-[2-(dimethylphos phoryl)-4-(trifluoromethy l)phenyl]-N2-(piperidin-3-yl)-5-(trifluoromethy l)pyrimidine-2,4-diamine | 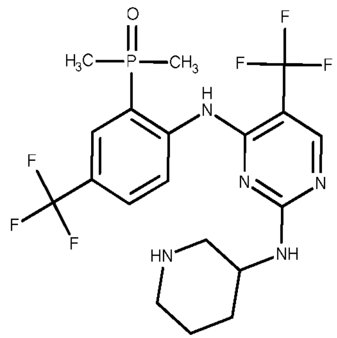 | **CDK7_1151_a** 3-{2-[(6,6-dimethylpiperidin -3-yl)amino]-5-(trifluoromethyl)p yrimidin-4-yl}-7-[(oxan-4-yl) methyl]-1H,4H,5H,6H,7H, 8H-pyrrolo[2,3-c] azepin-8-one | 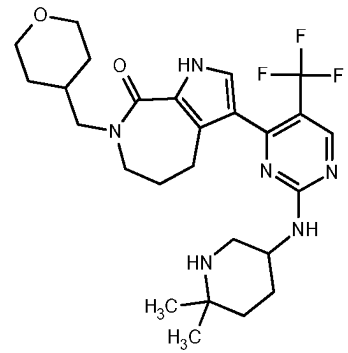 |
| **CDK7_907** N4-[5-chloro-2-(dimethylphos phoryl) phenyl] -N2-[(3S)-piperidin-3-yl]-5-(trifluoromethy l)pyrimidine-2,4-diamine | 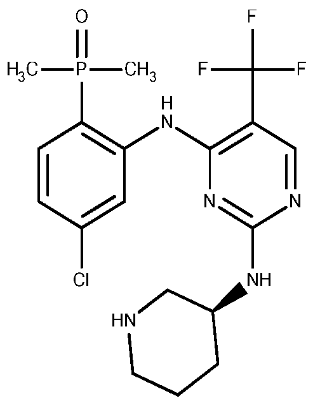 | **CDK7_1152** 3-(2-{[(3S)-6,6-dimethylpiperidin -3-yl]amino}-5-(trifluoromethyl)p yrimidin-4-yl)-7-(oxolan-3-yl)-1H,4H,5H,6H,7H, 8H-pyrrolo[2,3-c] azepin-8-one | 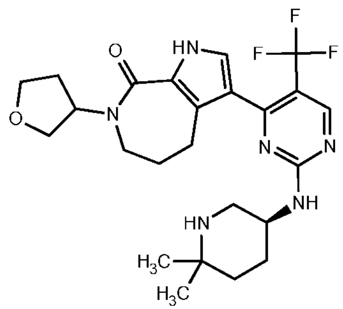 |

(continued)

| Code Name | Structure | Code Name | Structure |
|---|---|---|---|
| **CDK7_907_a** N4-[5-chloro-2-(dimethylphos phoryl) phenyl] -N2-(piperidin-3-yl)-5-(trifluoromethy l)pyrimidine-2,4-diamine | 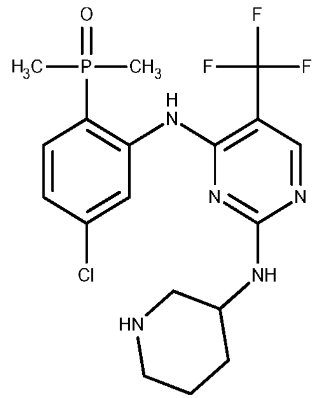 | **CDK7_1152_a** 3-{2-[(6,6-dimethylpiperidin -3-yl)amino]-5-(trifluoromethyl)p yrimidin-4-yl}-7-(oxolan-3-yl)-1H,4H,5H,6H,7H, 8H-pyrrolo[2,3-c]aze-pin-8-one | 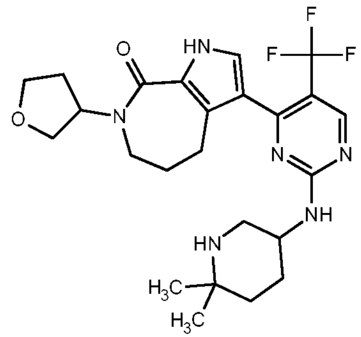 |
| **CDK7_908** N4-[4-chloro-2-(dimethylphos phoryl) phenyl] -N2-[(3S)-piperidin-3-yl]-5-(trifluoromethy l)pyrimidine-2,4-diamine | 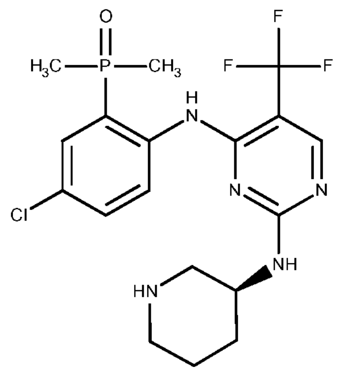 | **CDK7_1153** 3-(2-{[(3S)-6,6-dimethylpiperidin -3-yl]amino}-5-(trifluoromethyl)p yrimidin-4-yl)-7-[(oxolan-3-yl)methyl]-1H,4H,5H,6H,7H, 8H-pyrrolo [2,3-c]azepin-8-one | 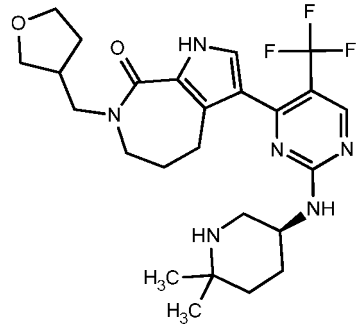 |
| **CDK7_908_a** N4-[4-chloro-2-(dimethylphos phoryl) phenyl] -N2-(piperidin-3-yl)-5-(trifluoromethy l)pyrimidine-2,4-diamine | 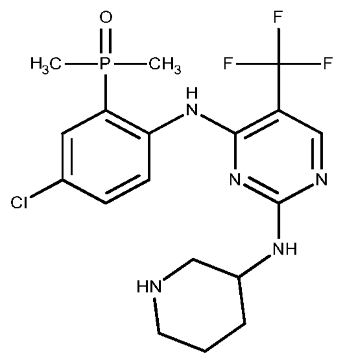 | **CDK7_1153_a** 3-{2-[(6,6-dimethylpiperidin -3-yl)amino]-5-(trifluoromethyl)p yrimidin-4-yl}-7-[(oxolan-3-yl) methyl]-1H,4H,5H,6H,7H, 8H-pyrrolo[2,3-c] azepin-8-one | 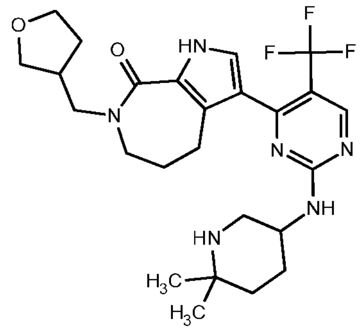 |

(continued)

| Code Name | Structure | Code Name | Structure |
|---|---|---|---|
| **CDK7_915** N4-[2-(dimethylphos phoryl)-4-(trifluorometho xy)phenyl]-N2-[(3S)-piperidin-3-yl]-5-(trifluoromethy l)pyrimi-dine-2,4-diamine | 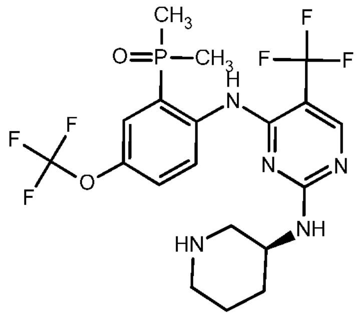 | **CDK7_1154** 7-cyclopentyl-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c] azepin-8-one | 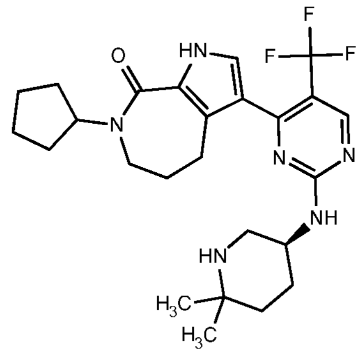 |
| **CDK7_915_a** N4-[2-(dimethylphos phoryl)-4-(tri-fluorometho xy)phenyl]-N2-(piperi-din-3-yl)-5-(trifluoromethy l)pyrimi-dine-2,4-diamine | 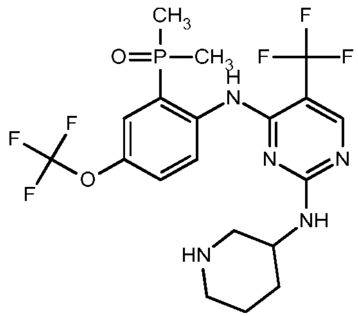 | **CDK7_1154_a** 7-cyclopentyl-3-{2-[(6,6-dimethylpiperidin -3-yl)amino]-5-(trifluoromethyl)p yrimidin-4-yl }-1H,4H,5H,6H,7H, 8H-pyrrolo[2,3-c]aze-pin-8-one | 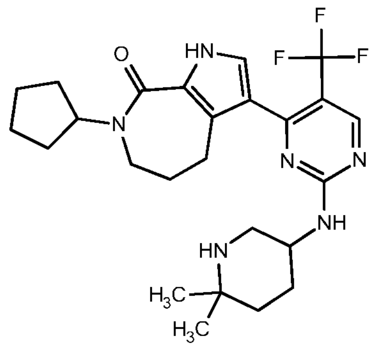 |
| **CDK7_889** 2-[(2-{[(3S)-piperidin-3-yl]amino}-5-(trifluoromethy l)pyrimidin-4-yl) amino]benz ene-1-sulfonamide | 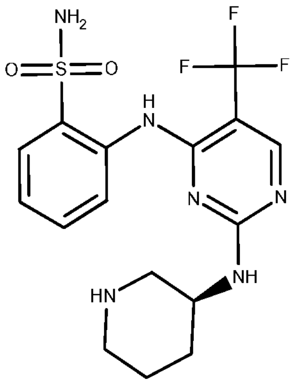 | **CDK7_1155** 7-(cyclopentylmeth yl)-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]azepin-8-one | 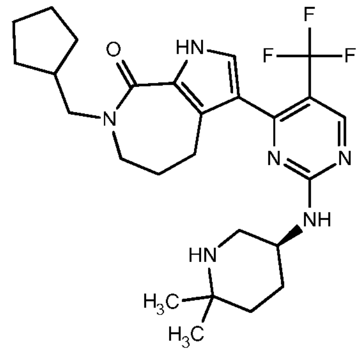 |

(continued)

| Code Name | Structure | Code Name | Structure |
|---|---|---|---|
| **CDK7_889_a** 2-({2-[(piperidin-3-yl)amino]-5-(trifluoromethy l)pyrimidin-4-yl}amino)benz ene-1-sulfonamide | 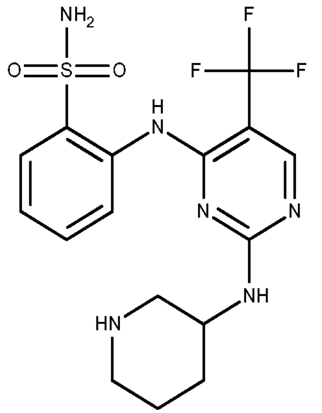 | **CDK7_1155_a** 7-(cyclopentylmeth yl)-3-{2-[(6,6-dimethylpiperidin -3-yl)amino]-5-(trifluoromethyl)p yrimidin-4-yl }-1H,4H,5H,6H,7H, 8H-pyrrolo[2,3-c] azepin-8-one | 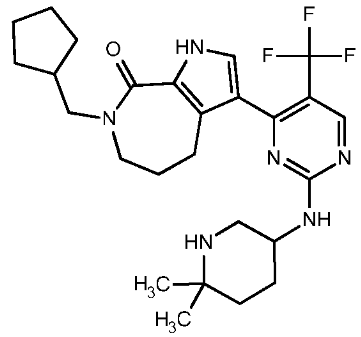 |
| **CDK7_890** N4-(3- N4-(3-methanesulfon ylphenyl)-N2-[(3S)-piperidin-3-yl]-5-(trifluoromethy l)pyrimidine-2,4-diamine | 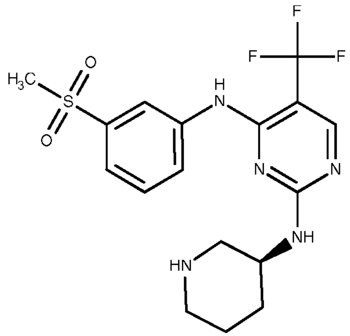 | **CDK7_1156** 3-(2-{[(3S)-6,6-dimethylpiperidin -3-yl]amino}-5-(trifluoromethyl)p yrimidin-4-yl)-7-[1-(oxan-4-yl)ethyl]-1H,4H,5H,6H,7H, 8H-pyrrolo[2,3-c]azepin-8-one | 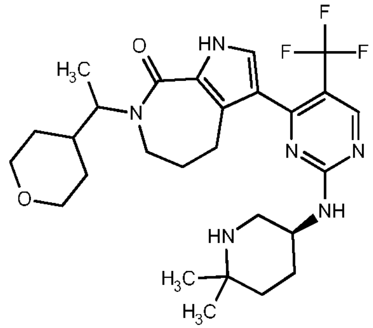 |
| **CDK7_890_a** N4-(3-methanesulfon ylphenyl)-N2-(piperidin-3-yl)-5-(trifluoromethy l)pyrimidine-2,4-diamine | 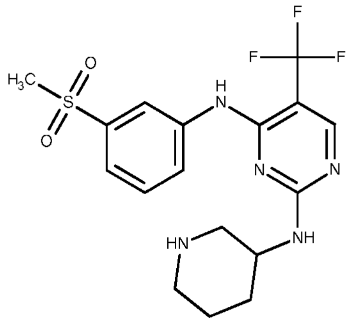 | **CDK7_1156_a** 3-{2-[(6,6-dimethylpiperidin -3-yl)amino]-5-(trifluoromethyl)p yrimidin-4-yl}-7-[1-(oxan-4-yl) ethyl]-1H,4H,5H,6H,7H, 8H-pyrrolo[2,3-c]azepin-8-one | 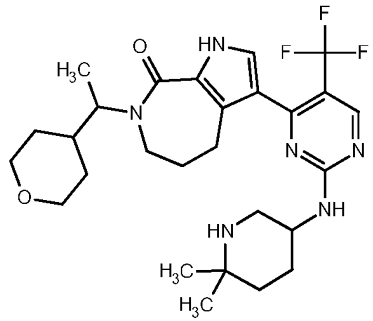 |

(continued)

| Code Name | Structure | Code Name | Structure |
|---|---|---|---|
| **CDK7_891** 3-[(2-{[(3S)-piperidin-3-yl]amino}-5-(trifluoromethy l)pyrimidin-4-yl) amino]benz ene-1-sulfonamide | 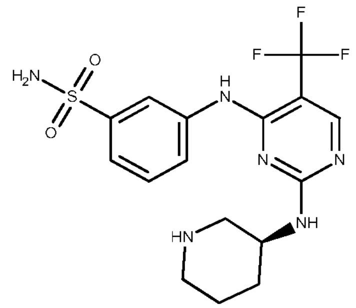 | **CDK7_1157** 3-(2-{[(3S)-6,6-dimethylpiperidin -3-yl]amino}-5-(trifluoromethyl)p yrimidin-4-yl)-7-[1-(oxolan-3-yl)ethyl]-1H,4H,5H,6H,7H, 8H-pyrrolo[2,3-c]azepin-8-one | 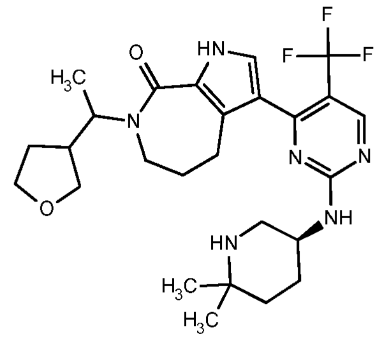 |
| **CDK7_891_a** 3-({2-[(piperidin-3-yl)amino]-5-(trifluoromethy l)pyrimidin-4-yl}amino)benz ene-1-sulfonamide | 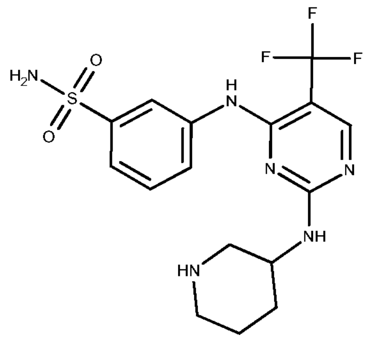 | **CDK7_1157_a** 3-{2-[(6,6-dimethylpiperidin -3-yl)amino]-5-(trifluoromethyl)p yrimidin-4-yl}-7-[1-(oxolan-3-yl) ethyl]-1H,4H,5H,6H,7H, 8H-pyrrolo[2,3-c]azepin-8-one | 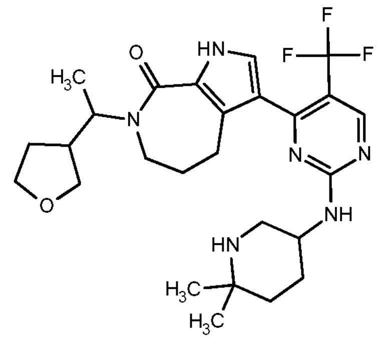 |
| **CDK7_893** 2-[(2-{[(3S)-piperidin-3-yl]amino}-5-(trifluoromethy l)pyrimidin-4-yl) oxy]benzen e-1-sulfonamide | 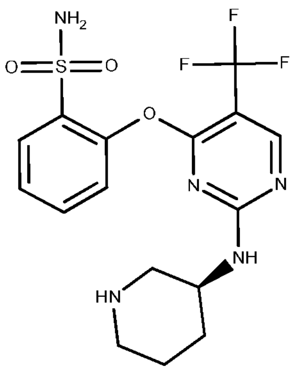 | **CDK7_1158** 7-(1-cyclopentylethyl)-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]azepin-8-one | 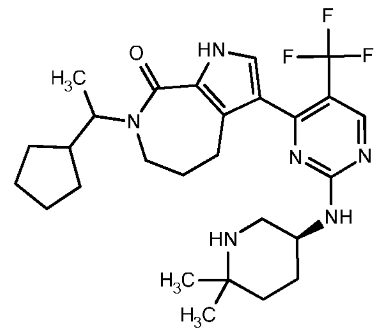 |

(continued)

| Code Name | Structure | Code Name | Structure |
|---|---|---|---|
| **CDK7_893_a** 2-({2-[(piperidin-3-yl)amino]-5-(trifluoromethy l)pyrimidin-4-yl}oxy)benzen e-1-sulfonamide | 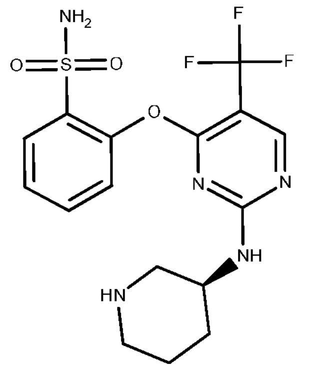 | **CDK7_1158_a** 7-(1-cyclopentylethyl)-3-{2-[(6,6-dimethylpiperidin -3-yl)amino]-5-(trifluoromethyl)p yrimidin-4-yl }-1H,4H,5H,6H,7H, 8H-pyrrolo[2,3-c] azepin-8-one | 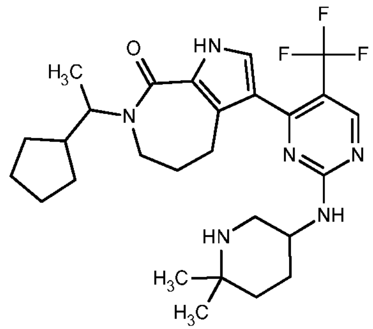 |
| **CDK7_894** 4-(3-methanesulfon ylphenox-y)-N-[(3S)-piperidin-3-yl]-5-(trifluoromethy l)pyrimidin-2-amine | 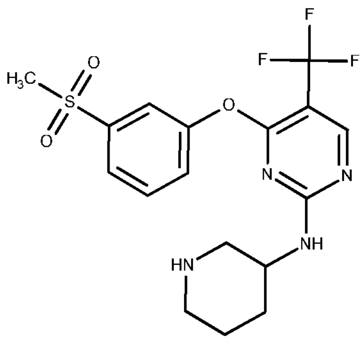 | **CDK7_1159** 7-tert-butyl-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]aze-pin-8-one | 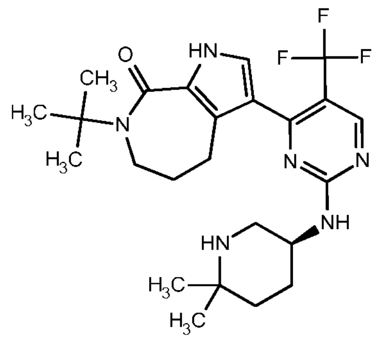 |
| **CDK7_894_a** 4-(3-methanesulfon ylphenoxy)-N-(piperidin-3-yl)-5-(trifluoromethy l)pyrimi-din-2-amine | 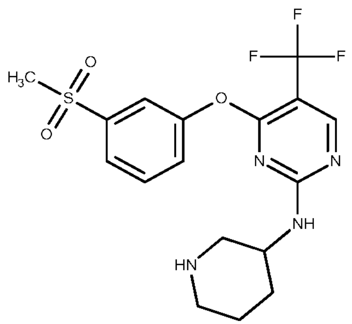 | **CDK7_1159_a** 7-tert-butyl-3-{2-[(6,6-dimethylpiperidin -3-yl) amino]-5-(trifluoromethyl)p yrimidin-4-yl}-1H,4H,5H,6H,7H, 8H-pyrrolo[2,3-c]aze-pin-8-one | 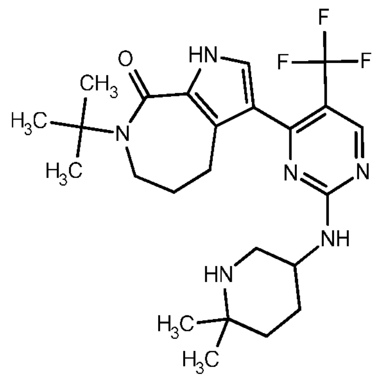 |

(continued)

| Code Name | Structure | Code Name | Structure |
|---|---|---|---|
| **CDK7_895** 3-[(2-{[(3S)-piperidin-3-yl]amino}-5-(trifluoromethy l)pyrimidin-4-yl)oxy]benzen e-1-sulfonamide | 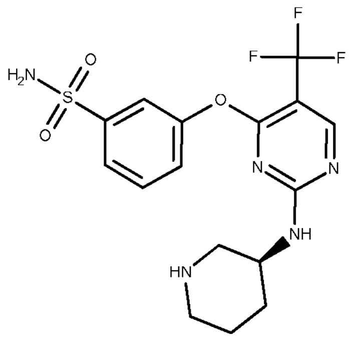 | **CDK7_1160** 3-(2-{[(3S)-6,6-dimethylpiperidin -3-yl]amino}-5-(trifluoromethyl)p yrimidin-4-yl)-7-(2,2-dimethylpropyl)-1H,4H,5H,6H,7H, 8H-pyrrolo [2,3-c]azepin-8-one | 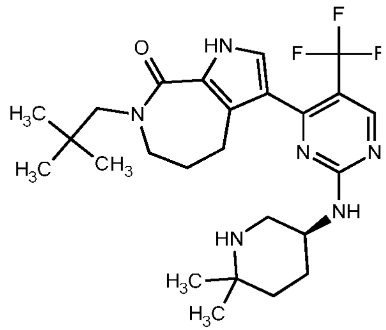 |
| **CDK7_895_a** 3-({2-[(piperidin-3-yl)amino]-5-(trifluoromethy l)pyrimidin-4-yl}oxy)benzen e-1-sulfonamide | 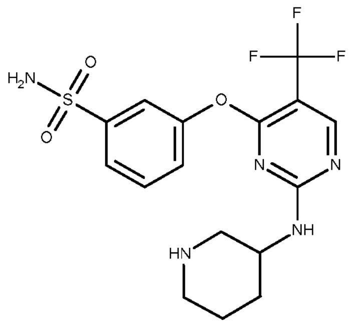 | **CDK7_1160_a** 3-{2-[(6,6-dimethylpiperidin -3-yl)amino]-5-(trifluoromethyl)p yrimidin-4-yl}-7-(2,2-dimethylpropyl)-1H,4H,5H,6H,7H, 8H-pyrrolo[2,3-c] azepin-8-one | 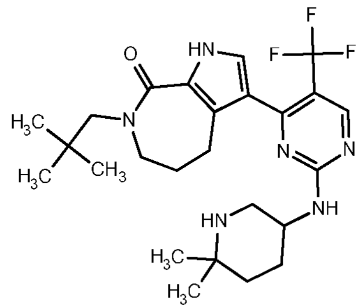 |
| **CDK7_827** 4-(2,3-dihydro-1H-indol-7-yloxy)-N-[(3S)-piperidin-3-yl]-5-(trifluoromethy l)pyrimidin-2-amine | 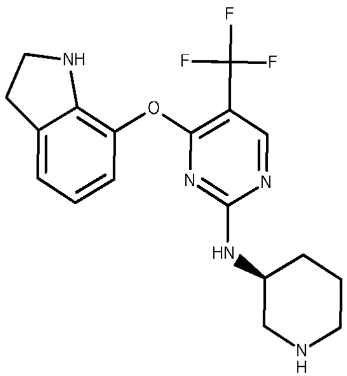 | **CDK7_1161** 7-(3,3-dimethylbutan-2-yl)-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]azepin-8-one | 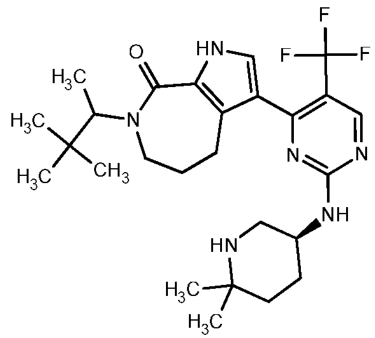 |

(continued)

| Code Name | Structure | Code Name | Structure |
|---|---|---|---|
| **CDK7_827_a** 4-(2,3-dihydro-1H-indol-7-yloxy)-N-(piperidin-3-yl)-5-(trifluoromethy l)pyrimidin-2-amine | 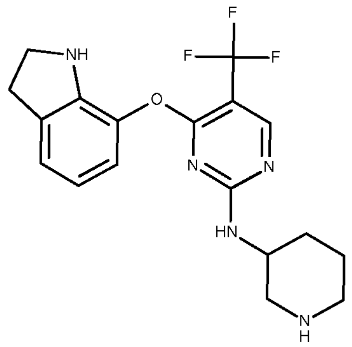 | **CDK7_1161_a** 7-(3,3-dimethylbutan-2-yl)-3-{2-[(6,6-dimethylpiperidin -3-yl)amino]-5-(trifluoromethyl)p yrimidin-4-yl}-1H,4H,5H,6H,7H, 8H-pyrrolo[2,3-c]azepin-8-one | 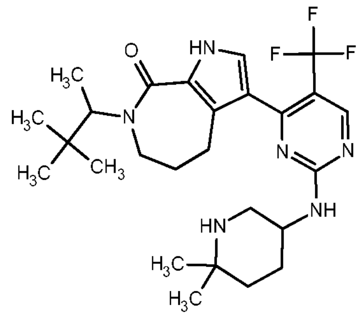 |
| **CDK7_828** 4-(1H-indol-7-yloxy)-N-[(3S)-piperidin-3-yl]-5-(trifluoromethy l)pyrimidin-2-amine | 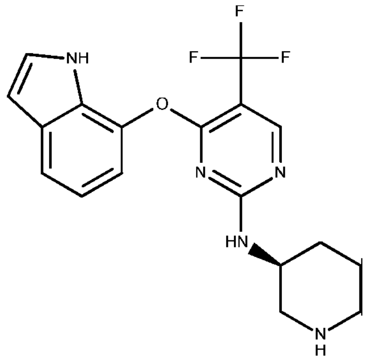 | **CDK7_1162** 8-(2-{[(3S)-6,6-dimethylpiperidin -3-yl]amino}-5-(trifluoromethyl)p yrimidin-4-yl)-4-(1-methyl-1H-pyrazol-4-yl)-2H,3H,4H,5H,6H -pyrrolo[2,3-f][1,4]oxazepin-5-one | 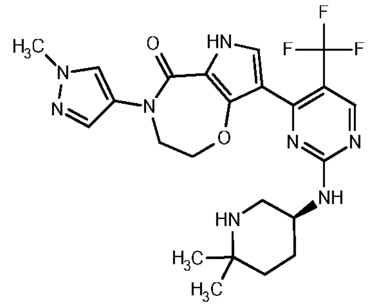 |
| **CDK7_828_a** 4-(1H-indol-7-yloxy)-N-(piperidin-3-yl)-5-(trifluoromethy l)pyrimidin-2-amine | 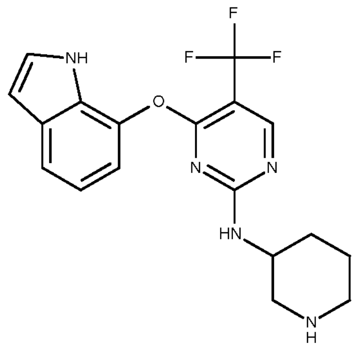 | **CDK7_1162_a** 8-{2-[(6,6-dimethylpiperidin -3-yl)amino]-5-(trifluoromethyl)p yrimidin-4-yl}-4-(1-methyl-1H-pyrazol-4-yl)-2H,3H,4H,5H,6H -pyrrolo[2,3-f][1,4]oxazepin-5-one | 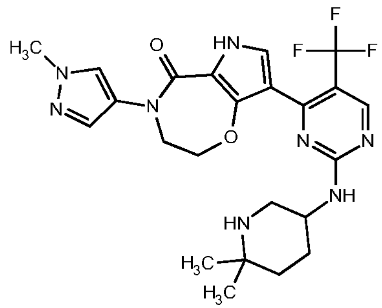 |

(continued)

| Code Name | Structure | Code Name | Structure |
|---|---|---|---|
| **CDK7_829**<br>N-[(3S)-piperidin-3-yl]-4-(1,2,3,4-tetrahydroquin olin-8-yloxy)-5-(trifluoromethy l)pyrimidin-2-amine | 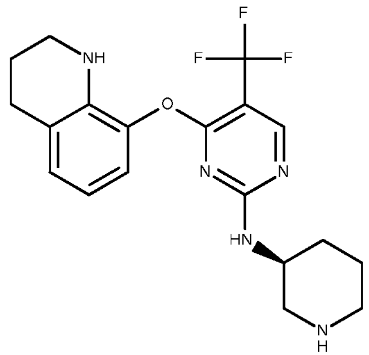 | **CDK7_1163**<br>(6S)-3-(2-{[(3S)-6,6-dimethylpiperidin -3-yl] amino}-5-(trifluoromethyl)p yrimidin-4-yl)-6-methyl-7-(1-methyl-1H-pyrazol-4-yl)-1H,4H,5H,6H,7H, 8H-pyrrolo[2,3-c]aze-pin-8-one | 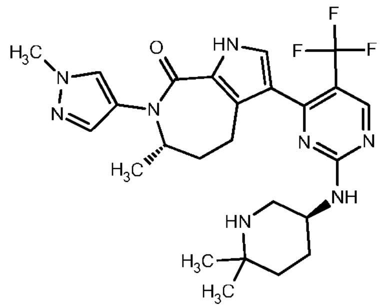 |
| **CDK7_829_a**<br>4 N-(piperidin-3-yl)-4-(1,2,3,4-tetrahydroquin olin-8-yloxy)-5-(trifluoromethy l)pyrimidin-2-amine | 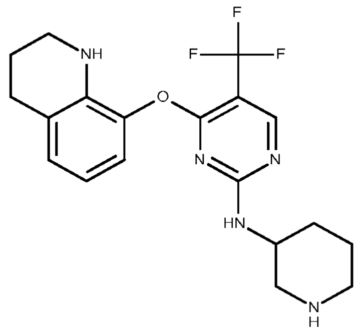 | **CDK7_1163_a**<br>3-{2-[(6,6-dimethylpiperidin -3-yl)amino]-5-(trifluoromethyl)p yrimidin-4-yl}-6-methyl-7-(1-methyl-1H-pyrazol-4-yl)-1H,4H,5H,6H,7H, 8H-pyrrolo[2,3-c]azepin-8-one | 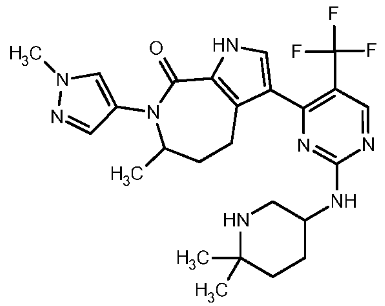 |
| **CDK7_830**<br>N-[(3S)-piperidin-3-yl]-4-(quinolin-8-yloxy)-5-(trifluoromethy l)pyrimidin-2-amine | 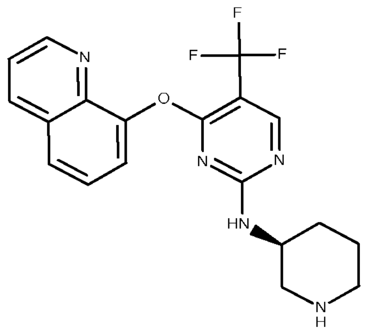 | **CDK7_1164**<br>(6R)-3-(2-{[(3S)-6,6-dimethylpiperidin -3-yl] amino}-5-(trifluoromethyl)p yrimidin-4-yl)-6-methyl-7-(1-methyl-1H-pyrazol-4-yl)-1H,4H,5H,6H,7H, 8H-pyrrolo[2,3-c]aze-pin-8-one | 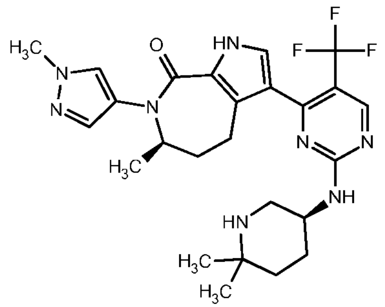 |

(continued)

| Code Name | Structure | Code Name | Structure |
|---|---|---|---|
| **CDK7_830_a**<br>N-(piperidin-3-yl)-4-(quinolin-8-yloxy)-5-(trifluoromethy l)pyrimidin-2-amine | 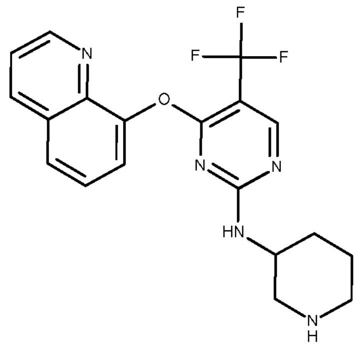 | **CDK7_1164_a**<br>3-{2-[(6,6-dimethylpiperidin -3-yl)amino]-5-(trifluoromethyl)p yrimidin-4-yl}-6-methyl-7-(1-methyl-1H-pyrazol-4-yl)-1H,4H,5H,6H,7H, 8H-pyrrolo[2,3-c]azepin-8-one | 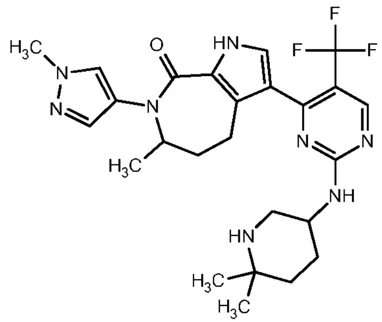 |
| **CDK7_898**<br>N-{2-[(2-{[(3S)-piperidin-3-yl]amino}-5-(trifluoromethy l)pyrimidin-4-yl) amino]phen yl } acetamide | 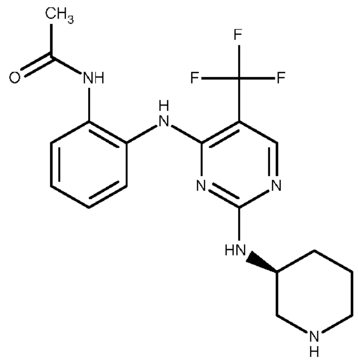 | **CDK7_1165**<br>3-(2-{[(3S)-6,6-dimethylpiperidin -3-yl]amino}-5-(trifluoromethyl)p yrimidin-4-yl)-6,6-dimethyl-7-(1-methyl-1H-pyrazol-4-yl)-1H,4H,5H,6H,7H, 8H-pyrrolo[2,3-c]aze-pin-8-one | 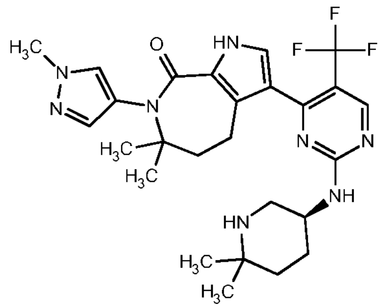 |
| **CDK7_898_a**<br>N-[2-({2-[(piperidin-3-yl)amino]-5-(trifluoromethy l)pyrimidin-4-yl}amino) phen yl]acetamide | 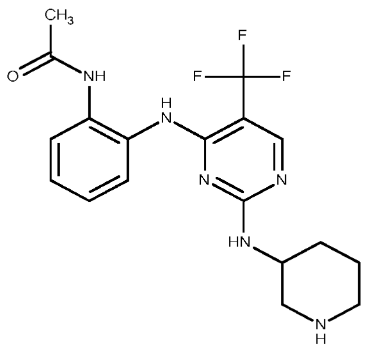 | **CDK7_1165_a**<br>3-{2-[(6,6-dimethylpiperidin -3-yl)amino]-5-(trifluoromethyl)p yrimidin-4-yl }-6,6-dimethyl-7-(1-methyl-1H-pyrazol-4-yl)-1H,4H,5H,6H,7H, 8H-pyrrolo[2,3-c]aze-pin-8-one | 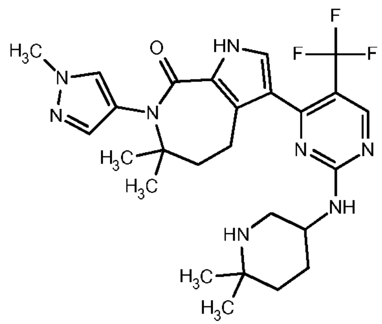 |

(continued)

| Code Name | Structure | Code Name | Structure |
|---|---|---|---|
| **CDK7_899** N-{2-[(2-{[(3S)-piperidin-3-yl]amino}-5-(trifluoromethy l)pyrimidin-4-yl) oxy]phenyl} acetamide | 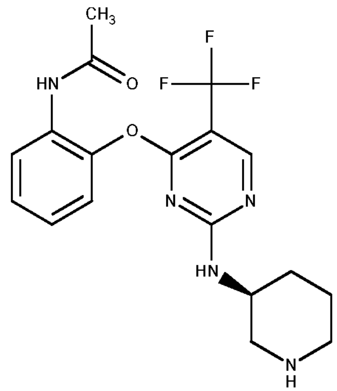 | **CDK7_1105** 6-cyclopropyl-3-(2-{[(3S)-piperidin-3-yl]amino}-5-(trifluoromethyl)p yrimidin-4-yl)-1H,6H,7H-pyrrolo[2,3-c]pyridin-7-one | 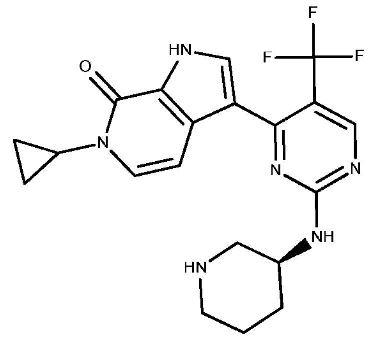 |
| **CDK7_899_a** N-[2-({2-[(piperidin-3-yl)amino]-5-(trifluoromethy l)pyrimidin-4-yl}oxy)phenyl] acetamide | 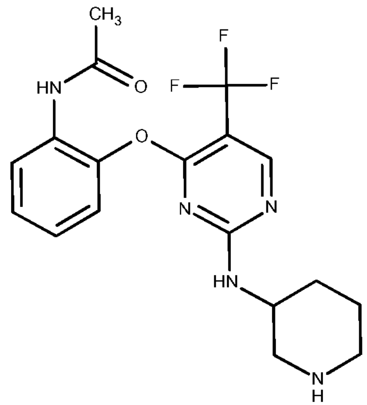 | **CDK7_1105_a** 6-cyclopropyl-3-(2-{[piperidin-3-yl]amino}-5-(trifluoromethyl)p yrimidin-4-yl)-1H,6H,7H-pyrrolo[2,3-c]pyridin-7-one | 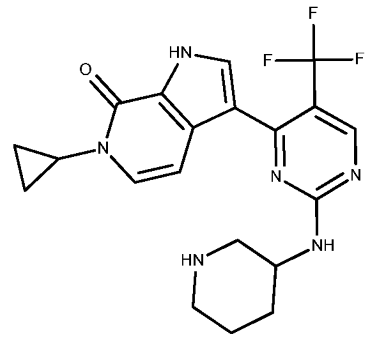 |

(continued)

| Code Name | Structure | Code Name | Structure |
|---|---|---|---|
| **CDK7_460**<br>N-[(3S)-6,6-dimethylpiperi din-3-yl]-4-(5-phenyl-1H-pyrrol-3-yl)-5-(trifluoromethy l)pyrimidin-2-amine | 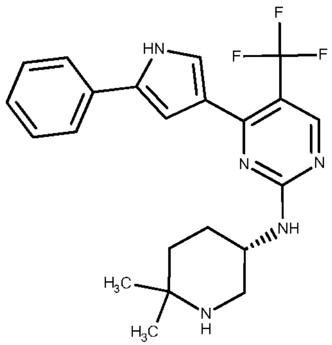 | **CDK7_1058**<br>3-(2-{[(3S)-piperidin-3-yl]amino}-5-(trifluoromethyl)p yrimidin-4-yl)-6-(1,3-thiazol-2-yl)-1H,6H,7H-pyrrolo[2,3-c]pyridin-7-one | 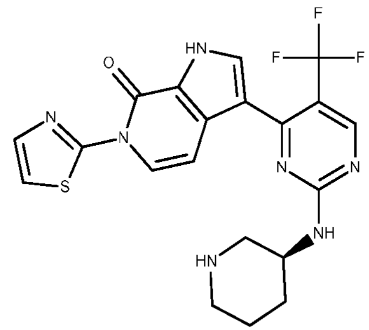 |
| **CDK7_460_a**<br>N-(6,6-dimethylpiperi din-3-yl)-4-(5-phenyl-1H-pyrrol-3-yl)-5-(trifluoromethy l)pyrimidin-2-amine | 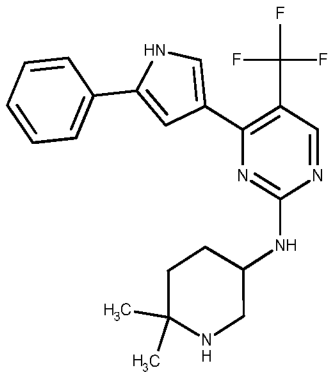 | **CDK7_1058_a**<br>3-(2-{[piperidin-3-yl]amino}-5-(trifluoromethyl) p yrimidin-4-yl)-6-(1,3-thiazol-2-yl)-1H,6H,7H-pyrrolo[2,3-c]pyridin-7-one | 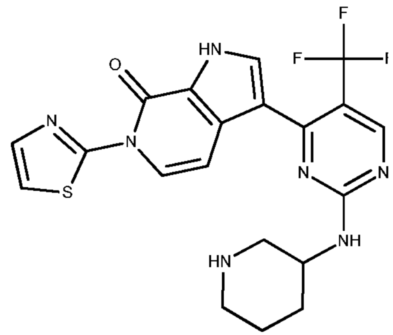 |
| **CDK7_533**<br>4-(5-phenyl-1H-pyrrol-3-yl)-N-[(3S)-piperidin-3-yl]-5-(trifluoromethy l)pyrimi-din-2-amine | 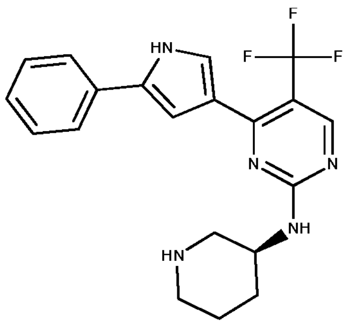 | **CDK7_1052**<br>1-methyl-5-[7-oxo-3-(2-{[(3S)-piperidin-3-yl] amino}-5-(trifluoromethyl)p yrimidin-4-yl)-1H,6H,7H-pyrrolo[2,3-c]pyridin-6-yl]-1,2-dihydropyridin-2-one | 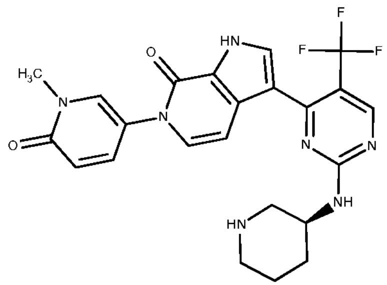 |

(continued)

| Code Name | Structure | Code Name | Structure |
|---|---|---|---|
| **CDK7_533_a** 4-(5-phenyl-1H-pyrrol-3-yl)-N-(piperidin-3-yl)-5-(trifluoromethy l)pyrimidin-2-amine | 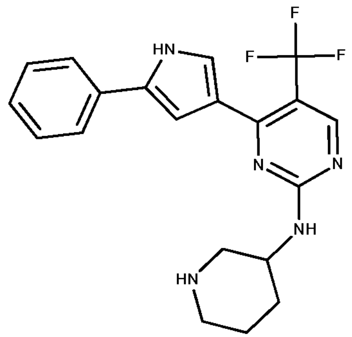 | **CDK7_1052_a** 1-methyl-5-[7-oxo-3-(2-{ [piperidin-3-yl]amino}-5-(trifluoromethyl)p yrimidin-4-yl)-1H,6H,7H-pyrrolo[2,3-c]pyridin-6-yl]-1,2-dihydropyridin-2-one | 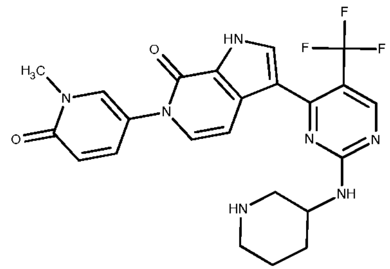 |
| **CDK7_534** 4-(4-phenyl-1H-pyrrol-3-yl)-N-[(3S)-piperidin-3-yl]-5-(trifluoromethy l)pyrimidin-2-amine | 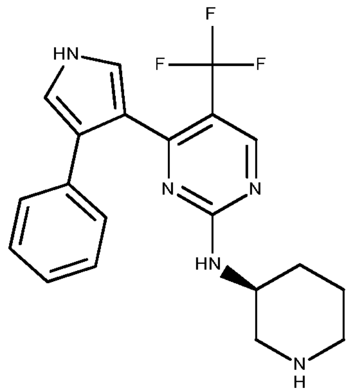 | **CDK7_972** N-[(3S)-piperidin-3-yl]-4-(1H-pyrrol-3-yl)-5-(trifluoromethyl)p yrimidin-2-amine | 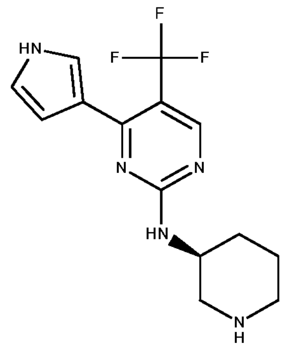 |
| **CDK7_534_a** 4-(4-phenyl-1H-pyrrol-3-yl)-N-(piperidin-3-yl)-5-(trifluoromethy l)pyrimidin-2-amine | 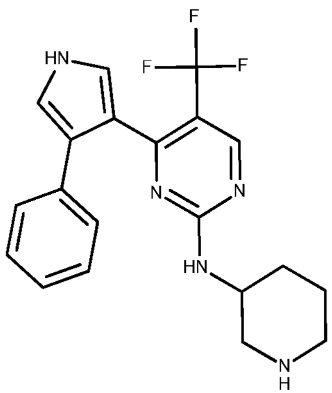 | **CDK7_972_a** N-[piperidin-3-yl]-4-(1H-pyrrol-3-yl)-5-(trifluoromethyl)p yrimidin-2-amine | 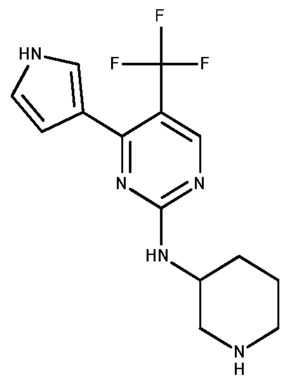 |

(continued)

| Code Name | Structure | Code Name | Structure |
|---|---|---|---|
| **CDK7_535** 4-(5-benzoyl-1H-pyrrol-3-yl)-N-[(3 S)-piperidin-3-yl]-5-(trifluoromethy l)pyrimidin-2-amine | 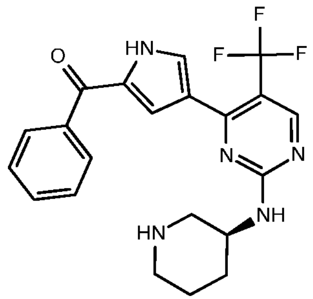 | **CDK7_1021** 7-methyl-3-(2-{ [(3S)-piperidin-3-yl]amino}-5-(trifluoromethyl)p yrimidin-4-yl)-1H,4H,5H,6H,7H, 8H-pyrrolo[2,3-c]azepine-4,8-dione | 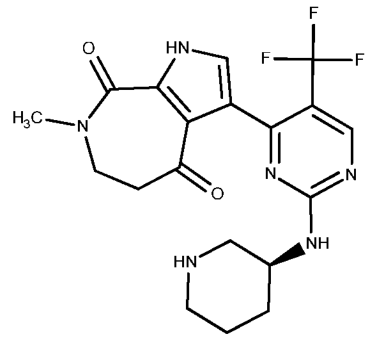 |
| **CDK7_535_a** 4-(5-benzoyl-1H-pyrrol-3-yl)-N-(piperidin-3-yl)-5-(trifluoromethy l)pyrimidin-2-amine | 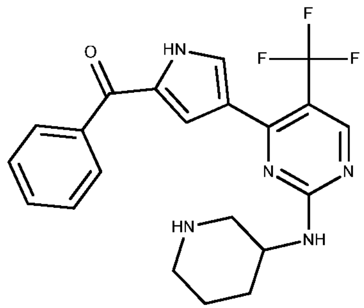 | **CDK7_1021_a** 7-methyl-3-(2-{[piperidin-3-yl]amino}-5-(trifluoromethyl)p yrimidin-4-yl)-1H,4H,5H,6H,7H, 8H-pyrrolo[2,3-c]azepine-4,8-dione | 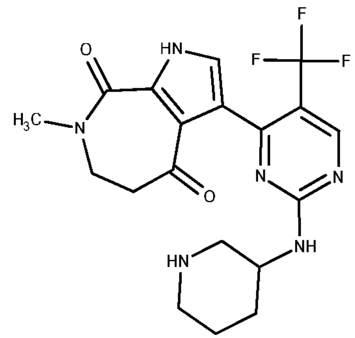 |
| **CDK7_536** N,N-dimethyl-4-(2-{[(3S)-piperidin-3-yl]amino}-5-(trifluoromethy l)pyrimidin-4-yl)-1H-pyrrole-2-carboxamide | 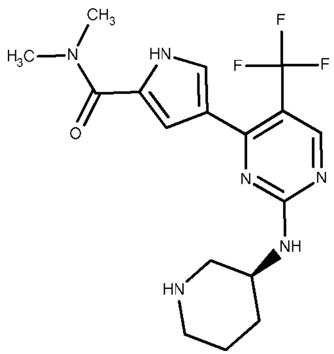 | **CDK7_1121** 6-(oxan-4-yl)-3-(2-{[(3S)-piperidin-3-yl]amino}-5-(trifluoromethyl)p yrimidin-4-yl)-1H,6H,7H-pyrrolo[2,3-c]pyridin-7-one | 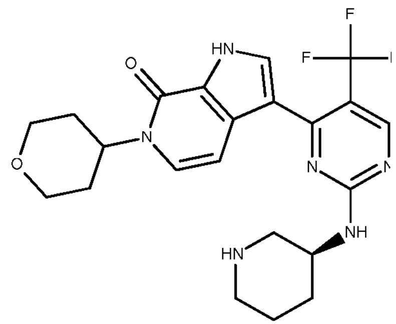 |

(continued)

| Code Name | Structure | Code Name | Structure |
|---|---|---|---|
| **CDK7_536_a** N,N-dimethyl-4-{2-[(piperidin-3-yl)amino]-5-(trifluoromethy l)pyrimidin-4-yl}-1H-pyrrole-2-carboxamide | 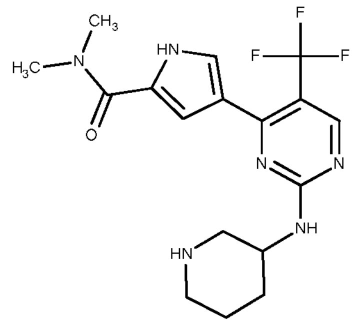 | CDK7_1121_a 6-(oxan-4-yl)-3-(2-{ [piperidin-3-yl]amino}-5-(trifluoromethyl)p yrimidin-4-yl)-1H,6H,7H-pyrrolo[2,3-c]pyridin-7-one | 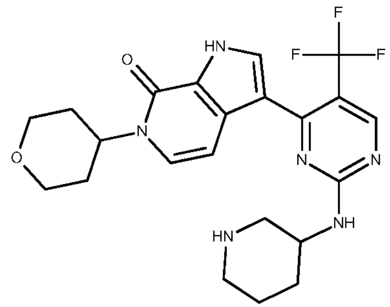 |
| **CDK7_537** 4-(2-{[(3S)-piperidin-3-yl]amino}-5-(trifluoromethy l)pyrimidin-4-yl)-1H-pyrrole-2-carboxamide | 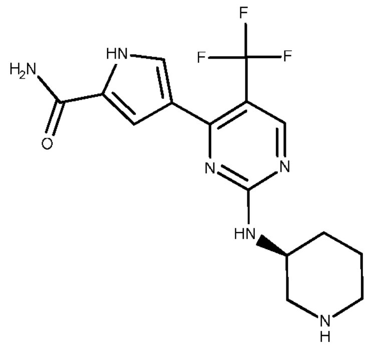 | **CDK7_809** 4-(1H-indol-1-yl)-N-[(3S)-piperidin-3-yl]-5-(trifluoromethyl)p yrimidin-2-amine | 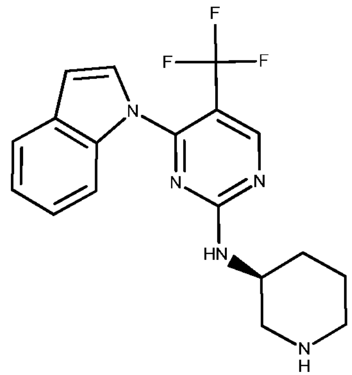 |
| **CDK7_537_a** 4-{2-[(piperidin-3-yl)amino]-5-(trifluoromethy l)pyrimidin-4-yl}-1H-pyrrole-2-carboxamide | 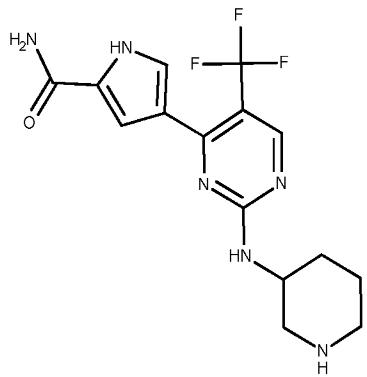 | **CDK7_809_a** 4-(1H-indol-1-yl)-N-[piperidin-3-yl]-5-(trifluoromethyl)p yrimidin-2-amine | 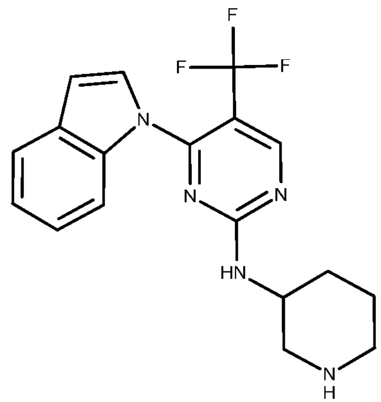 |

(continued)

| Code Name | Structure | Code Name | Structure |
|---|---|---|---|
| **CDK7_538** N-phenyl-4-(2-{[(3S)-piperidin-3-yl] amino}-5-(trifluoromethy l)pyrimidin-4-yl)-1H-pyrrole-2-carboxamide | 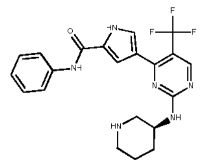 | **CDK7_810** 4-(1H-1,3-benzodiazol-1-yl)-N-[(3S)-piperidin-3-yl]-5-(trifluoromethyl)p yrimidin-2-amine | 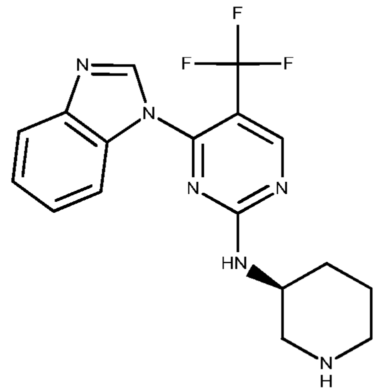 |
| **CDK7_538_a** N-phenyl-4-{2-[(piperidin-3-yl)amino]-5-(trifluoromethy l)pyrimidin-4-yl}-1H-pyrrole-2-carboxamide | 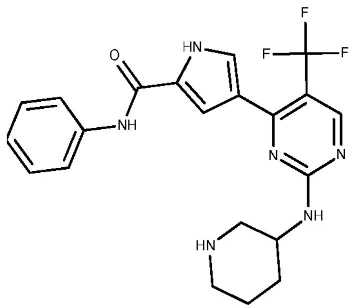 | **CDK7_810_a** 4-(1H-1,3-benzodiazol-1-yl)-N-[piperidin-3-yl]-5-(trifluoromethyl)p yrimidin-2-amine | 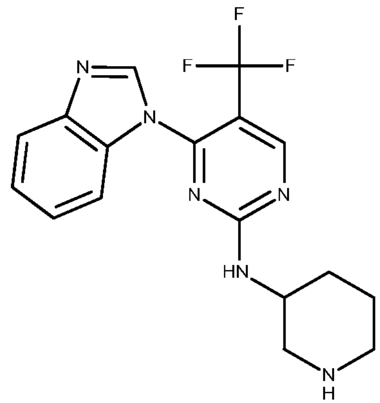 |

(continued)

| Code Name | Structure | Code Name | Structure |
|---|---|---|---|
| **CDK7_539** 4-[5-(4-methylpiperazi ne-1-carbonyl)-1H-pyrrol-3-yl]-N-[(3S)-piperidin-3-yl]-5-(trifluoromethy l)pyrimidin-2-amine | 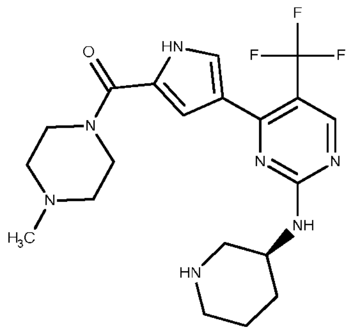 | **CDK7_811** 4-(1H-1,2,3-benzotriazol-1-yl)-N-[(3 S)-piperidin-3-yl]-5-(trifluoromethyl)p yrimidin-2-amine | 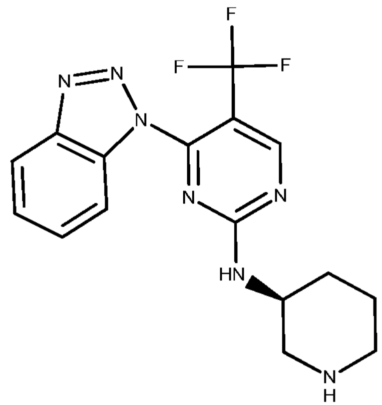 |
| **CDK7_539_a** 4-[5-(4-methylpiperazi ne-1-carbonyl)-1H-pyrrol-3-yl]-N-(piperidin-3-yl)-5-(trifluoromethy l)pyrimidin-2-amine | 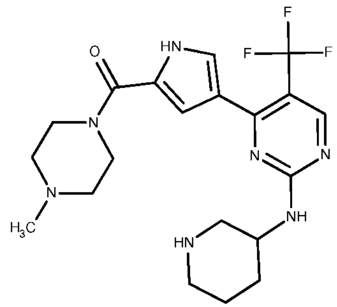 | **CDK7_811_a** 4-(1H-1,2,3-benzotriazol-1-yl)-N-[piperidin-3-yl]-5-(trifluoromethyl)p yrimidin-2-amine | 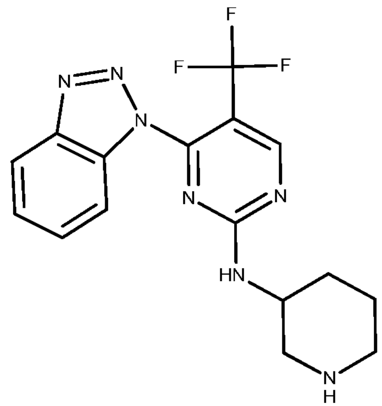 |

(continued)

| Code Name | Structure | Code Name | Structure |
|---|---|---|---|
| **CDK7_922**<br>4-(2-{[(3S)-piperidin-3-yl]amino}-5-(tri-fluorometһy l)pyrimidin-4-yl)-1H-pyr-role-2-carboxylic acid | 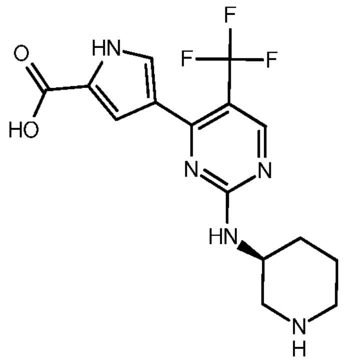 | **CDK7_953**<br>7-methyl-3-(2-{ [(3S)-piperidin-3-yl]amino}-5-(trifluoromethyl)p yrimidin-4-yl)-1H,4H,5H,6H,7H, 8H-pyrrolo[2,3-c]aze-pin-8-one | 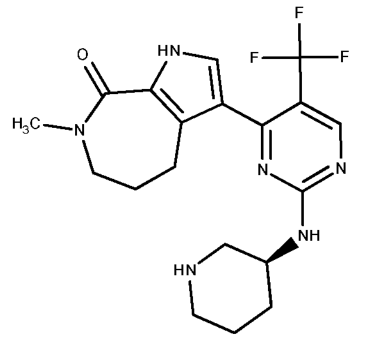 |
| **CDK7_922_a**<br>4-{2-[(piperidin-3-yl)amino]-5-(trifluoromethy l)pyrimidin-4-yl}-1H-pyrrole-2-carboxylic acid | 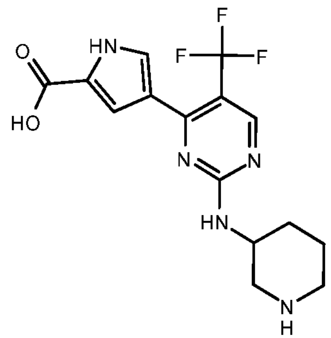 | **CDK7_953_a**<br>7-methyl-3-(2-{[piperidin-3-yl]amino}-5-(trifluoromethyl)p yrimidin-4-yl)-1H,4H,5H,6H,7H, 8H-pyrrolo[2,3-c]aze-pin-8-one | 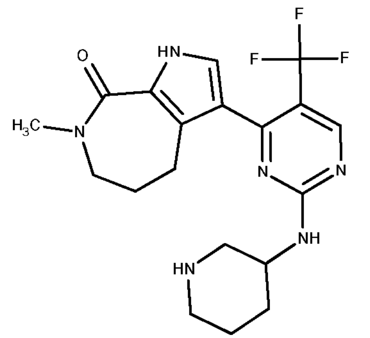 |
| **CDK7_695**<br>4-[4-(2-{[(3S)-piperidin-3-yl]amino}-5-(trifluoromethy l)pyrimidin-4-yl)-1H-pyrrol-2-yl]benzonitrile | 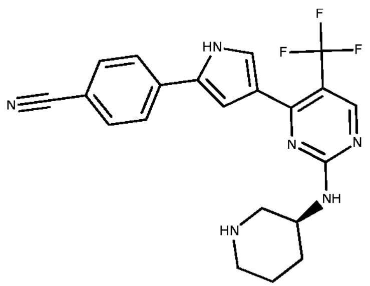 | **CDK7_959**<br>6-(1-methyl-1H-pyrazol-4-yl)-3-(2-{[(3S)-piperidin-3-yl]amino}-5-(trifluoromethyl)p yrimidin-4-yl)-1H,6H,7H-pyrrolo[2,3-c]pyridin-7-one | 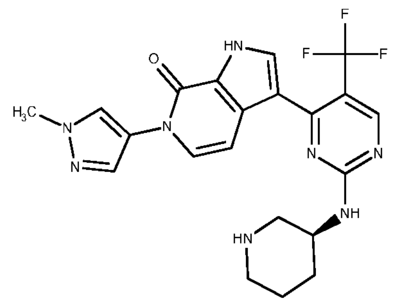 |

(continued)

| Code Name | Structure | Code Name | Structure |
|---|---|---|---|
| **CDK7_695_a** 4-(4-{2-[(piperidin-3-yl)amino]-5-(trifluoromethy l)pyrimidin-4-yl}-1H-pyrrol-2-yl)benzonitrile | 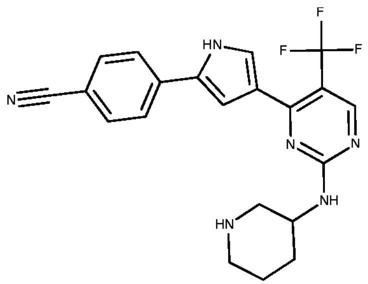 | **CDK7_959_a** 6-(1-methyl-1H-pyrazol-4-yl)-3-(2-{[piperidin-3-yl]amino}-5-(trifluoromethyl)p yrimidin-4-yl)-1H,6H,7H-pyrrolo[2,3-c]pyridin-7-one | 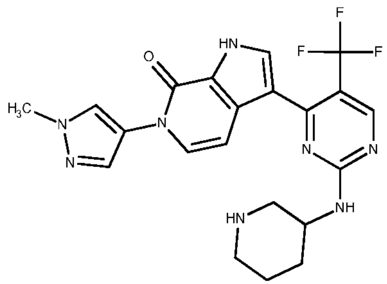 |
| **CDK7_696** 3-[4-(2-{[(3S)-piperidin-3-yl]amino}-5-(trifluoromethy l)pyrimidin-4-yl)-1H-pyrrol-2-yl]benzonitrile | 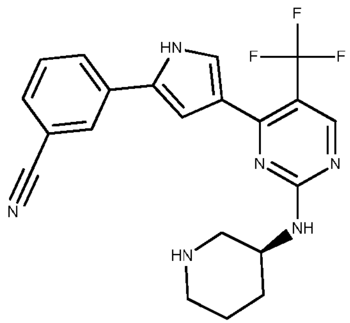 | **CDK7_961** 6-benzyl-3-(2-{ [(3S)-piperidin-3-yl]amino}-5-(trifluoromethyl)p yrimidin-4-yl)-1H,6H,7H-pyrrolo[2,3-c]pyridin-7-one | 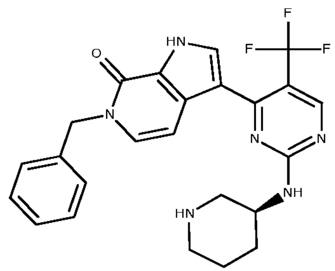 |
| **CDK7_696_a** 3-(4-{2-[(piperidin-3-yl)amino]-5-(trifluoromethy l)pyrimidin-4-yl}-1H-pyrrol-2-yl)benzonitrile | 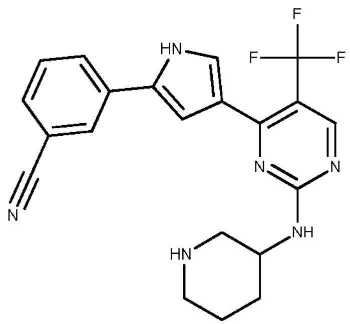 | **CDK7_961_a** 6-benzyl-3-{2-[(piperidin-3-yl)amino]-5-(trifluoromethyl)p yrimidin-4-yl}-1H,6H,7H-pyrrolo[2,3-c]pyridin-7-one | 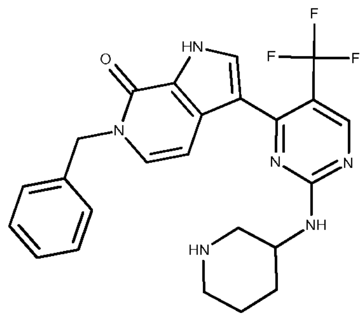 |

(continued)

| Code Name | Structure | Code Name | Structure |
|---|---|---|---|
| **CDK7_814** 3-(2-{[(3S)-piperidin-3-yl]amino}-5-(trifluoromethy l)pyrimidin-4-yl)-1H,4H,5H,6H, 7H-pyrrolo[2,3-c] pyridin-7-one | 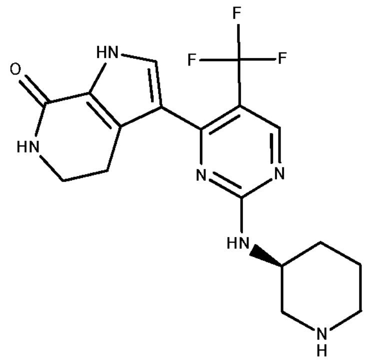 | **CDK7_966** N,N-dimethyl-4-(2-{[(3S)-piperidin-3-yl]amino}-5-(trifluoromethyl)p yrimidin-4-yl)-1H-imidazole-2-carboxamide | 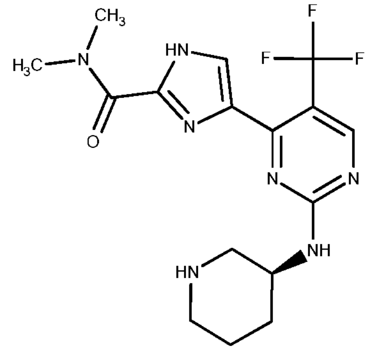 |
| **CDK7_814_a** 3-{2-[(piperidin-3-yl)amino]-5-(trifluoromethy l)pyrimidin-4-yl}-1H,4H,5H,6H, 7H-pyrrolo[2,3-c]pyridin-7-one | 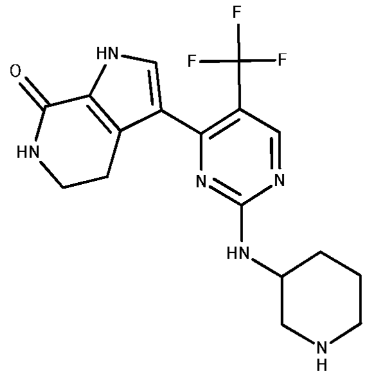 | **CDK7_966_a** N,N-dimethyl-4-(2-{[piperidin-3-yl]amino}-5-(trifluoromethyl)p yrimidin-4-yl)-1H-imidazole-2-carboxamide | 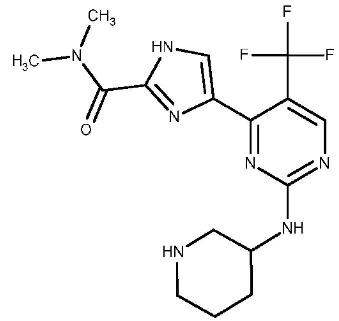 |
| **CDK7_815** 3-(2-{[(3S)-piperidin-3-yl]amino}-5-(trifluoromethy l)pyrimidin-4-yl)-1H,4H,5H,6H, 7H-pyrrolo[3,2-c] pyridin-4-one | 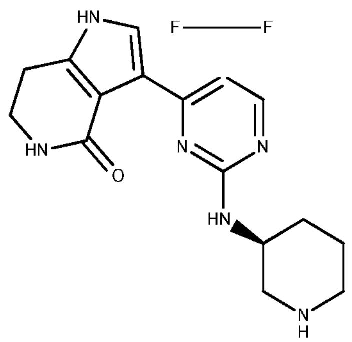 | **CDK7_991** N,N-dimethyl-4-(2-{[(3S)-piperidin-3-yl]amino}-5-(trifluoromethyl)p yrimidin-4-yl)-1H-pyrrole-3-carboxamide | 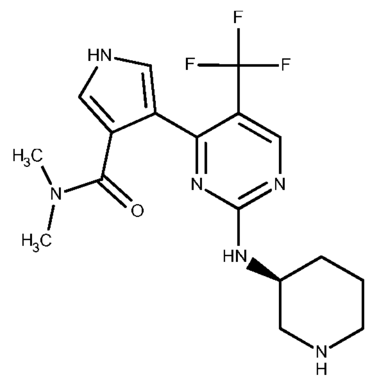 |

(continued)

| Code Name | Structure | Code Name | Structure |
|---|---|---|---|
| **CDK7_815_a** 3-{2-[(piperidin-3-yl)amino]-5-(trifluoromethy l)pyrimidin-4-yl}-1H,4H,5H,6H,7H-pyrrolo[3,2-c]pyridin-4-one | 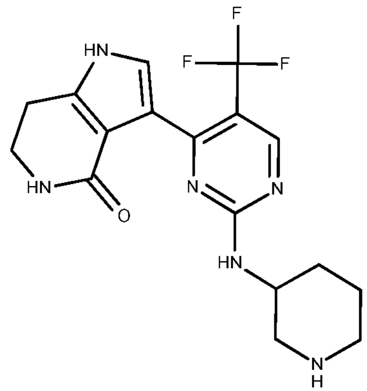 | **CDK7_991_a** N,N-dimethyl-4-(2-{ [piperidin-3-yl]amino}-5-(trifluoromethyl)p yrimidin-4-yl)-1H-pyrrole-3-carboxamide | 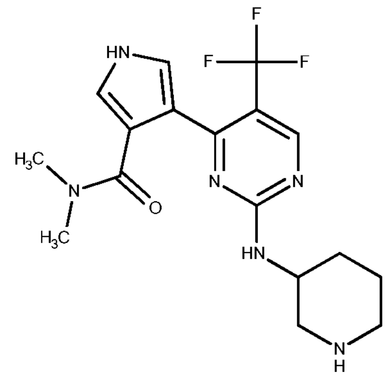 |
| **CDK7_843** 6-methyl-3-(2-{[(3S)-piperidin-3-yl] amino}-5-(trifluoromethy l)pyrimidin-4-yl)-1H,4H,5H,6H, 7H-pyrrolo[2,3-c] pyridin-7-one | 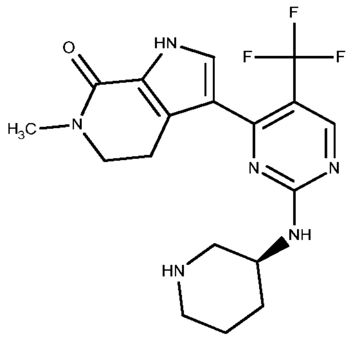 | **CDK7_1176** 3-(2-{[(3S)-piperidin-3-yl]amino}-5-(trifluoromethyl)p yrimidin-4-yl)-7-(pyridin-2-yl)-1H,4H,5H,6H,7H, 8H-pyrrolo[2,3-c]azepin-8-one | 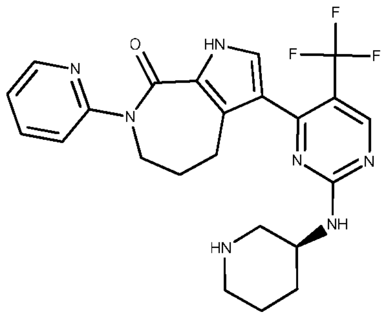 |
| **CDK7_843_a** 6-methyl-3-{2-[(piperidin-3-yl)amino]-5-(trifluoromethy l)pyrimidin-4-yl}-1H,4H,5H,6H, 7H-pyrrolo[2,3-c] pyridin-7-one | 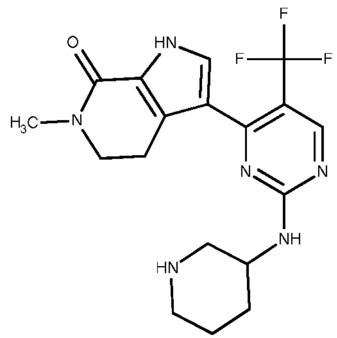 | **CDK7_1176_a** 3-(2-{[piperidin-3-yl]amino}-5-(trifluoromethyl) p yrimidin-4-yl)-7-(pyridin-2-yl)-1H,4H,5H,6H,7H, 8H-pyrrolo[2,3-c]azepin-8-one | 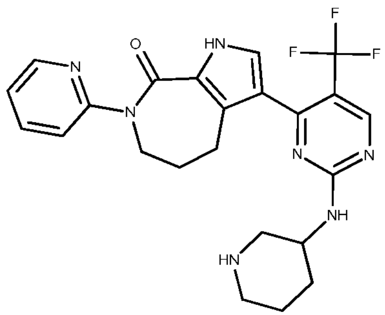 |

(continued)

| Code Name | Structure | Code Name | Structure |
|---|---|---|---|
| **CDK7_844** 3-(2-{[(3S)-piperidin-3-yl]amino}-5-(trifluoromethy l)pyrimidin-4-yl)-1H,4H,5H,6H, 7H-pyrrolo[2,3-c] pyridin-4-one | 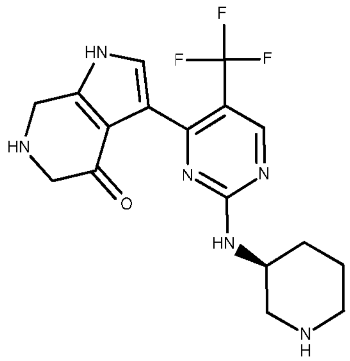 | **CDK7_1187** 3-(2-{[(3S)-6,6-dimethylpiperidin -3-yl]amino}-5-(trifluoromethyl)p yrimidin-4-yl)-7-methyl-1H,4H,5H,6H,7H, 8H-pyrrolo[2,3-c] azepin-8-one | 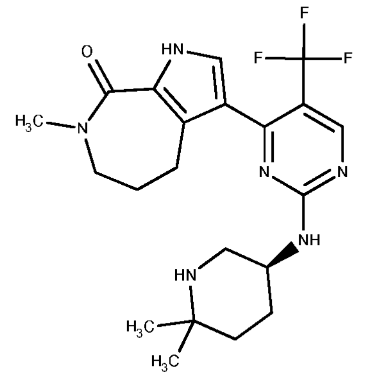 |
| **CDK7_844_a** 3-{2-[(piperidin-3-yl)amino]-5-(trifluoromethy l)pyrimidin-4-yl}-1H,4H,5H,6H, 7H-pyrrolo[2,3-c]pyridin-4-one | 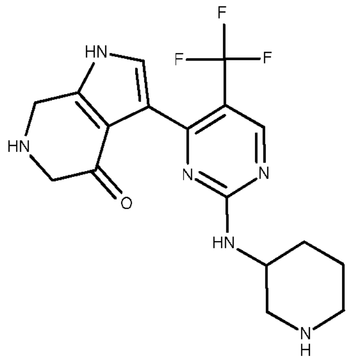 | **CDK7_1187_a** 3-(2-{[6,6-dimethylpiperidin -3-yl]amino}-5-(trifluoromethyl)p yrimidin-4-yl)-7-methyl-1H,4H,5H,6H,7H, 8H-pyrrolo[2,3-c] azepin-8-one | 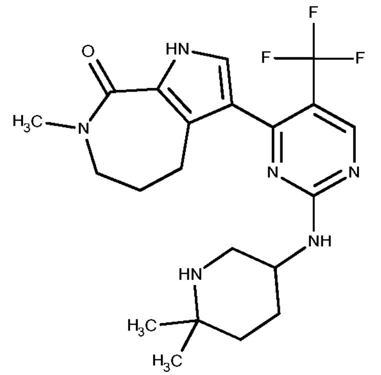 |
| **CDK7_845** 6-methyl-3-(2-{[(3S)-piperidin-3-yl] amino}-5-(trifluoromethy l)pyrimidin-4-yl)-1H,6H,7H-pyrrolo[2,3-c]pyridin-7-one | 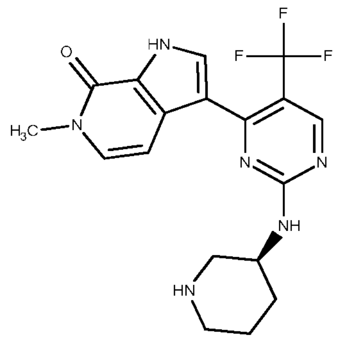 | **CDK7_1199** 3-(2-{[(6S)-4-azaspiro[2.5]octa n-6-yl]amino}-5-(trifluoromethyl)p yrimidin-4-yl)-6-(1-methyl-1H-pyrazol-4-yl)-1H,6H,7H-pyrrolo [2,3-c]pyridin-7-one | 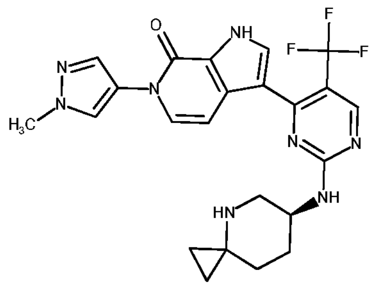 |

(continued)

| Code Name | Structure | Code Name | Structure |
|---|---|---|---|
| **CDK7_845_a**<br>6-methyl-3-{2-[(piperidin-3-yl)amino]-5-(trifluoromethy l)pyrimidin-4-yl}-1H,6H,7H-pyrrolo[2,3-c]pyridin-7-one | 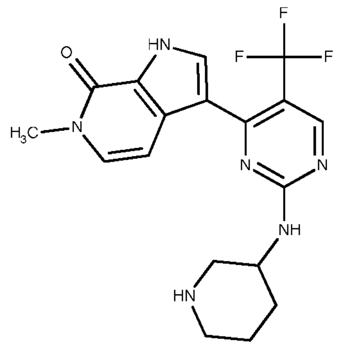 | **CDK7_1199_a**<br>3-[2-({4-azaspiro[2.5]octa n-6-yl}amino)-5-(trifluoromethyl)p yrimidin-4-yl]-6-(1-methyl-1H-pyrazol-4-yl)-1H,6H,7H-pyrrolo[2,3-c]pyridin-7-one | 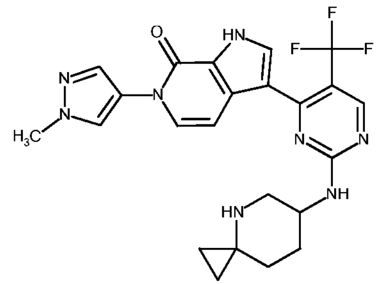 |
| **CDK7_833**<br>N-(1H-imidazol-2-yl)-4-(2-{[(3S)-piperidin-3-yl]amino}-5-(trifluoromethy l) pyrimidin-4-yl)-1H-pyrrole-2-carboxamide | 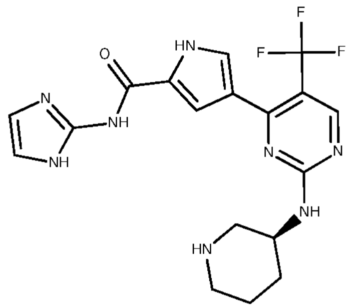 | **CDK7_1200**<br>3-(2-{[(6S)-4-azaspiro[2.5]octa n-6-yl]amino}-5-(trifluoromethyl)p yrimidin-4-yl)-6-methyl-1H,6H,7H-pyrrolo[2,3-c]pyridin-7-one | 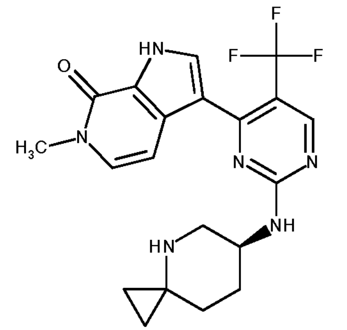 |
| **CDK7_833_a**<br>N-(1H-imidazol-2-yl)-4-{2-[(piperidin-3-yl)amino]-5-(trifluoromethy l)pyrimidin-4-yl}-1H-pyrrole-2-carboxamide | 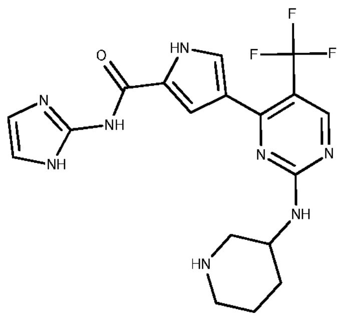 | **CDK7_1200_a**<br>3-[2-({4-azaspiro[2.5]octa n-6-yl}amino)-5-(trifluoromethyl)p yrimidin-4-yl]-6-methyl-1H,6H,7H-pyrrolo[2,3-c]pyridin-7-one | 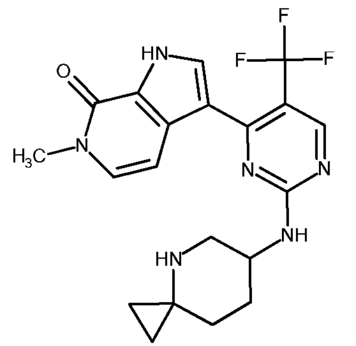 |

(continued)

| Code Name | Structure | Code Name | Structure |
|---|---|---|---|
| **CDK7_834** 4-(2-{[(3S)-piperidin-3-yl]amino}-5-(trifluoromethy l)pyrimidin-4-yl)-N-(1,3-thiazol-2-yl)-1H-pyrrole-2-carboxamide | 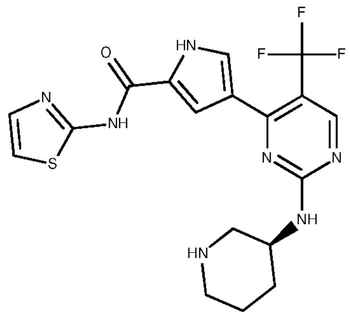 | **CDK7_1201** 3-[2-({4-azaspiro[2.5]octa n-6-yl}amino)-5-(trifluoromethyl)p yrimidin-4-yl]-7-methyl-1H,4H,5H,6H,7H, 8H-pyrrolo[2,3-c] azepin-8-one | 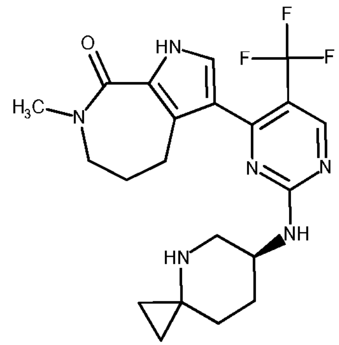 |
| **CDK7_834_a** 4-{2-[(piperidin-3-yl)amino]-5-(trifluoromethy l)pyrimidin-4-yl}-N-(1,3-thiazol-2-yl)-1H-pyrrole-2-carboxamide | 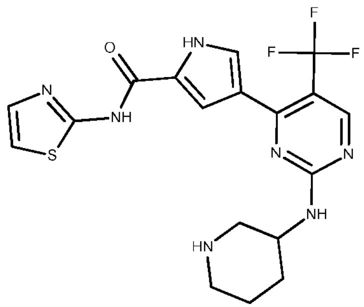 | **CDK7_1201_a** 3-(2-{[(3S)-piperidin-3-yl]amino}-5-(trifluoromethyl)p yrimidin-4-yl)-6-(1,3-thiazol-4-yl)-1H,6H,7H-pyrrolo[2,3-c]pyridin-7-one | 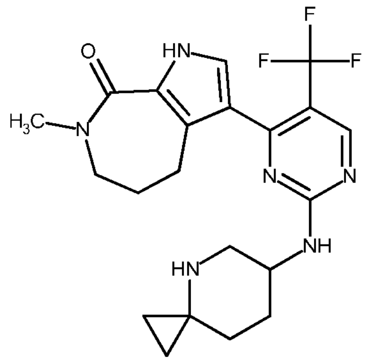 |
| **CDK7_835** N-(1-methyl-1H-pyrazol-4-yl)-4-(2-{[(3S)-piperidin-3-yl]amino}-5-(trifluoromethy l)pyrimidin-4-yl)-1H-pyrrole-2-carboxamide | 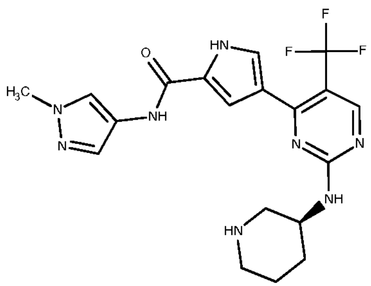 | **CDK7_1202** 3-(2-{[(3S)-piperidin-3-yl]amino}-5-(trifluoromethyl)p yrimidin-4-yl)-6-(1,3-thiazol-4-yl)-1H,6H,7H-pyrrolo[2,3-c]pyridin-7-one | 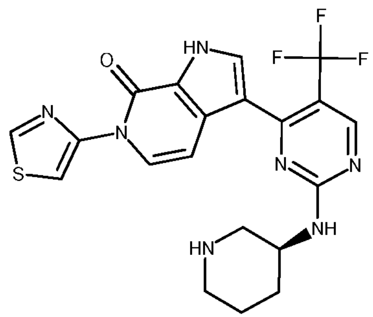 |

(continued)

| Code Name | Structure | Code Name | Structure |
|---|---|---|---|
| **CDK7_835_a** N-(1-methyl-1H-pyrazol-4-yl)-4-{2-[(piperidin-3-yl)amino]-5-(trifluoromethy l)pyrimidin-4-yl}-1H-pyrrole-2-carboxamide | 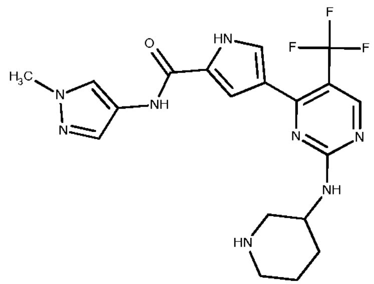 | **CDK7_1202_a** 3-{2-[(piperidin-3-yl)amino]-5-(trifluoromethyl)p yrimidin-4-yl}-6-(1,3-thiazol-4-yl)-1H,6H,7H-pyrrolo[2,3-c]pyridin-7-one | 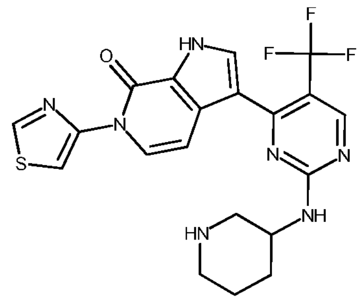 |
| **CDK7_836** 4-(2-{[(3S)-piperidin-3-yl]amino}-5-(trifluoromethy l)pyrimidin-4-yl)-N-(pyridin-4-yl)-1H-pyrrole-2-carboxamide | 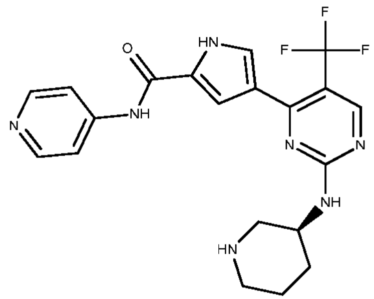 | **CDK7_1203** 3-(2-{[(3S)-piperidin-3-yl]amino}-5-(trifluoromethyl)p yrimidin-4-yl)-6-(1,3-thiazol-5-yl)-1H,6H,7H-pyrrolo[2,3-c]pyridin-7-one | 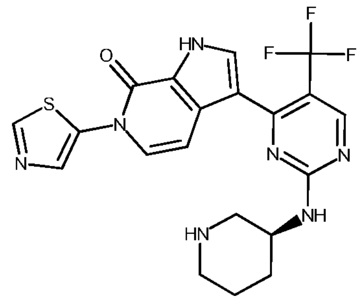 |
| **CDK7_836_a** 4-{2-[(piperidin-3-yl)amino]-5-(trifluoromethy l)pyrimidin-4-yl}-N-(pyridin-4-yl)-1H-pyrrole-2-carboxamide | 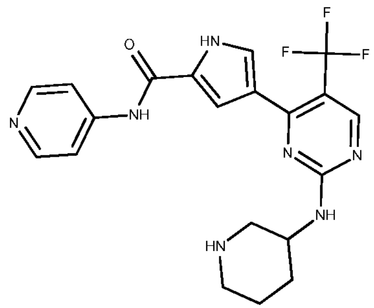 | **CDK7_1203_a** 3-{2-[(piperidin-3-yl)amino]-5-(trifluoromethyl)p yrimidin-4-yl}-6-(1,3-thiazol-5-yl)-1H,6H,7H-pyrrolo[2,3-c]pyridin-7-one | 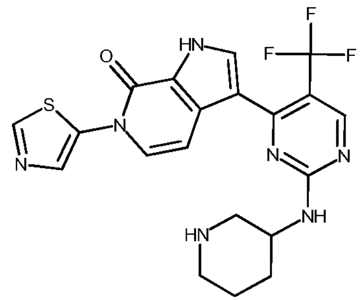 |

(continued)

| Code Name | Structure | Code Name | Structure |
|---|---|---|---|
| **CDK7_837**<br>N-(4-cyanophenyl)-4-(2-{[(3S)-piperidin-3-yl]amino}-5-(trifluoromethy l)pyrimidin-4-yl)-1H-pyrrole-2-carboxamide | 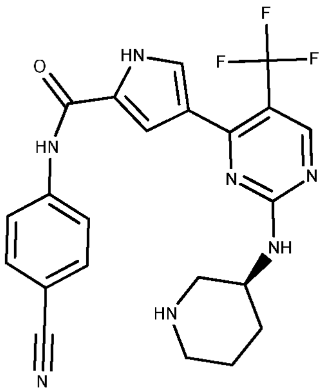 | **CDK7_1204**<br>6-(2-methyl-1,3-thiazol-4-yl)-3-(2-{[(3S)-piperidin-3-yl]amino}-5-(trifluoromethyl)p yrimidin-4-yl)-1H,6H,7H-pyrrolo[2,3-c]pyridin-7-one | 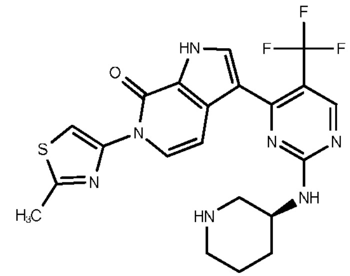 |
| **CDK7_837_a**<br>N-(4-cyanophenyl)-4-{2-[(piperidin-3-yl)amino]-5-(trifluoromethy l)pyrimidin-4-yl}-1H-pyrrole-2-carboxamide | 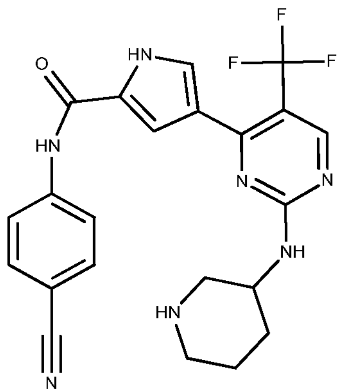 | **CDK7_1204_a**<br>6-(2-methyl-1,3-thiazol-4-yl)-3-{2-[(piperidin-3-yl)amino]-5-(trifluoromethyl)p yrimidin-4-yl }-1H,6H,7H-pyrrolo[2,3-c]pyridin-7-one | 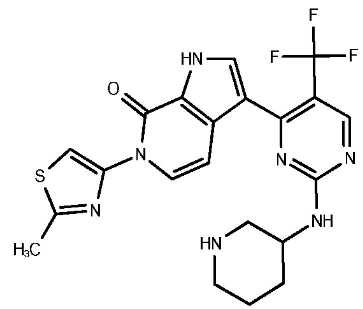 |

(continued)

| Code Name | Structure | Code Name | Structure |
|---|---|---|---|
| **CDK7_838**<br>N-[4-(morpholin-4-yl)phenyl]-4-(2-{[(3S)-piperidin-3-yl]amino}-5-(trifluoromethy l)pyrimidin-4-yl)-1H-pyrrole-2-carboxamide | 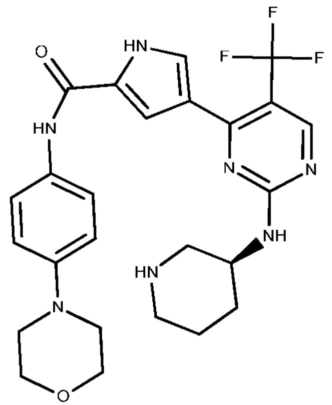 | **CDK7_1205**<br>6-(2-methyl-1,3-thiazol-5-yl)-3-(2-{[(3S)-piperidin-3-yl]amino}-5-(trifluoromethyl)p yrimidin-4-yl)-1H,6H,7H-pyrrolo[2,3-c]pyridin-7-one | 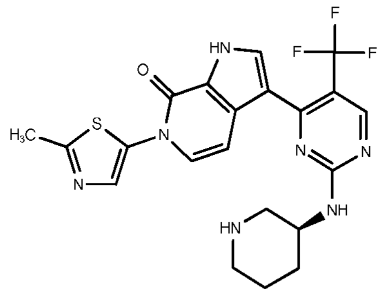 |
| **CDK7_838_a**<br>N-[4-(morpholin-4-yl)phenyl]-4-{2-[(piperidin-3-yl)amino]-5-(trifluoromethy l) pyrimidin-4-yl}-1H-pyrrole-2-carboxamide | 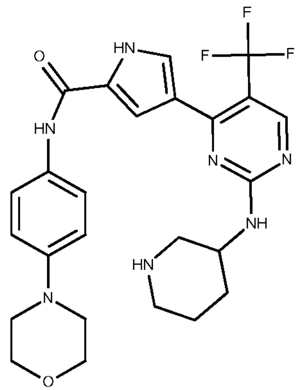 | **CDK7_1205_a**<br>6-(2-methyl-1,3-thiazol-5-yl)-3-{2-[(piperidin-3-yl)amino]-5-(trifluoromethyl)p yrimidin-4-yl }-1H,6H,7H-pyrrolo[2,3-c]pyridin-7-one | 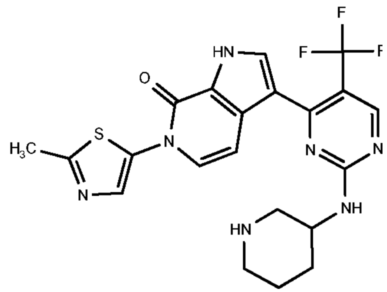 |

(continued)

| Code Name | Structure | Code Name | Structure |
|---|---|---|---|
| **CDK7_846** N-methyl-4-(2-{[(3S)-piperidin-3-yl] amino}-5-(trifluoromethy l)pyrimidin-4-yl)-1H-pyrrole-2-carboxamide | 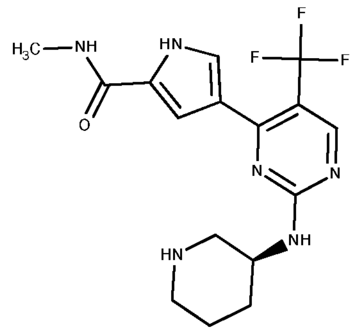 | **CDK7_1206** 6-(4-methyl-1,3-thiazol-2-yl)-3-(2-{[(3S)-piperidin-3-yl]amino}-5-(trifluoromethyl)p yrimidin-4-yl)-1H,6H,7H-pyrrolo[2,3-c]pyridin-7-one | 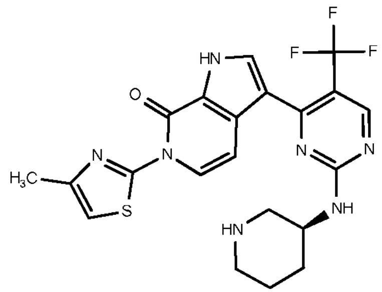 |
| **CDK7_846_a** N-methyl-4-{2-[(piperidin-3-yl)amino]-5-(trifluoromethy l)pyrimidin-4-yl}-1H-pyrrole-2-carboxamide | 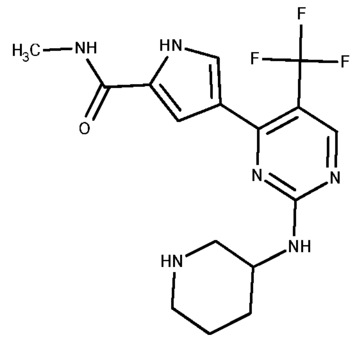 | **CDK7_1206_a** 6-(4-methyl-1,3-thiazol-2-yl)-3-{2-[(piperidin-3-yl)amino]-5-(trifluoromethyl)p yrimidin-4-yl}-1H,6H,7H-pyrrolo[2,3-c]pyridin-7-one | 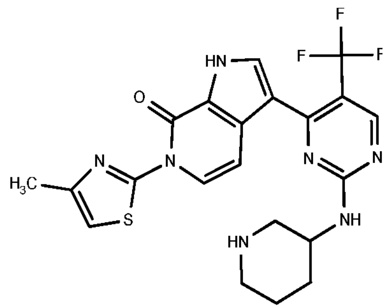 |
| **CDK7_847** 4-(2-{[(3S)-piperidin-3-yl]amino}-5-(trifluoromethy l)pyrimidin-4-yl)-N-(2,2,2-trifluoroethyl)-1H-pyrrole-2-carboxamide | 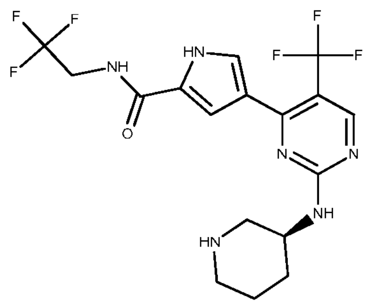 | **CDK7_1207** 6-(5-methyl-1,3-thiazol-2-yl)-3-(2-{[(3S)-piperidin-3-yl]amino}-5-(trifluoromethyl)p yrimidin-4-yl)-1H,6H,7H-pyrrolo[2,3-c]pyridin-7-one | 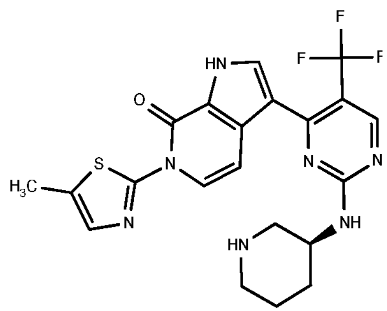 |

(continued)

| Code Name | Structure | Code Name | Structure |
|---|---|---|---|
| **CDK7_847_a** 4-{2-[(piperidin-3-yl)amino]-5-(trifluoromethy l)pyrimidin-4-yl}-N-(2,2,2-tri-fluoroethyl)-1H-pyrrole-2-carboxa-mide | 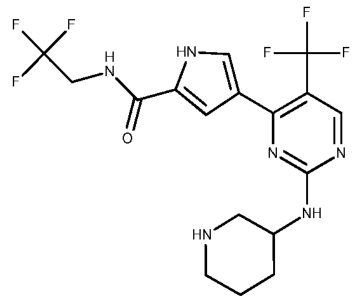 | **CDK7_1207_a** 6-(5-methyl-1,3-thiazol-2-yl)-3-{2-[(piperidin-3-yl)amino]-5-(trifluoromethyl)p yrimidin-4-yl}-1H,6H,7H-pyrrolo[2,3-c]pyridin-7-one | 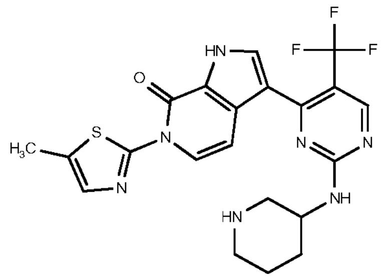 |
| **CDK7_848** N-(2-methanesulfon ylethyl)-4-(2-{[(3S)-piperidin-3-yl]amino}-5-(trifluoromethy l)pyrimidin-4-yl)-1H-pyrrole-2-carboxamide | 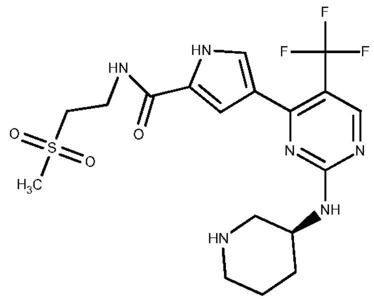 | **CDK7_1208** 6-(cyclopropylmeth yl)-3-(2-{[(3S)-piperidin-3-yl]amino}-5-(trifluoromethyl)p yrimidin-4-yl)-1H,6H,7H-pyrrolo[2,3-c]pyridin-7-one | 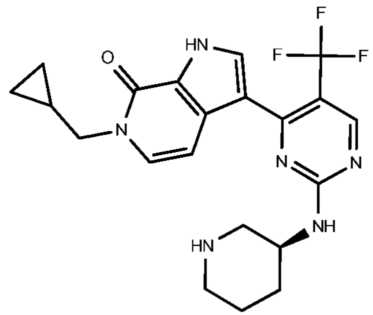 |
| **CDK7_848_a** N-(2-methanesulfon ylethyl)-4-{2-[(piperidin-3-yl)amino]-5-(trifluoromethy l) pyrimidin-4-yl}-1H-pyrrole-2-carboxa-mide | 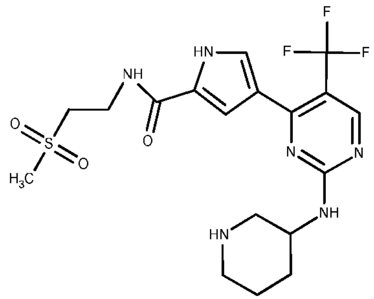 | **CDK7_1208_a** 6-(cyclopropylmeth yl)-3-{2-[(piperidin-3-yl) amino]-5-(trifluoromethyl)p yrimidin-4-yl }-1H,6H,7H-pyrrolo[2,3-c]pyridin-7-one | 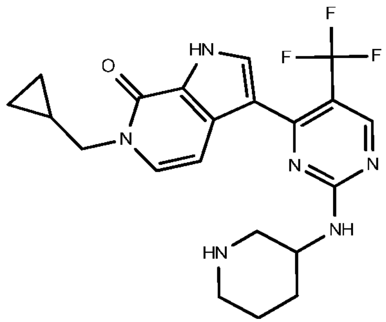 |

(continued)

| Code Name | Structure | Code Name | Structure |
|---|---|---|---|
| **CDK7_849**<br>4-[5-(morpholine-4-carbonyl)-1H-pyrrol-3-yl]-N-[(3S)-piperidin-3-yl]-5-(trifluoromethy l)pyrimidin-2-amine | 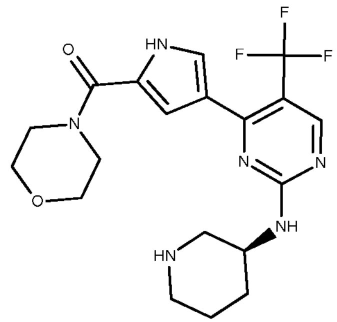 | **CDK7_1209**<br>6-cyclobutyl-3-(2-{[(3S)-piperidin-3-yl]amino}-5-(trifluoromethyl)p yrimidin-4-yl)-1H,6H,7H-pyrrolo[2,3-c]pyridin-7-one | 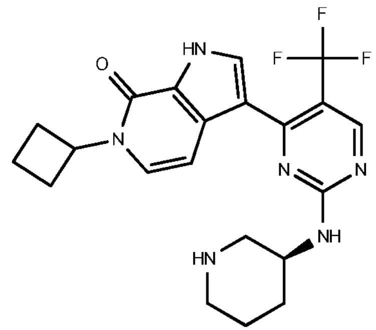 |
| **CDK7_849_a**<br>4-[5-(morpholine-4-carbonyl)-1H-pyrrol-3-yl]-N-(piperidin-3-yl)-5-(trifluoromethy l)pyrimidin-2-amine | 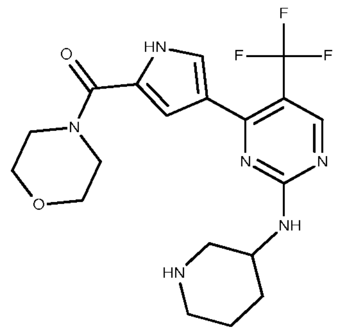 | **CDK7_1209_a**<br>6-cyclobutyl-3-{2-[(piperidin-3-yl)amino]-5-(trifluoromethyl)p yrimidin-4-yl }-1H,6H,7H-pyrrolo[2,3-c]pyridin-7-one | 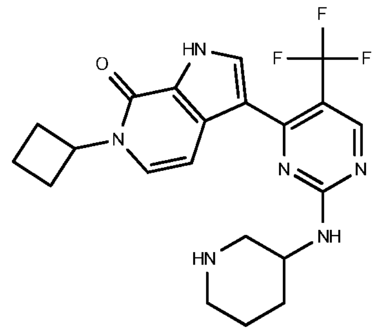 |
| **CDK7_850**<br>N-(oxan-4-yl)-4-(2-{[(3S)-piperidin-3-yl]amino}-5-(trifluoromethy l)pyrimidin-4-yl)-1H-pyrrole-2-carboxamide | 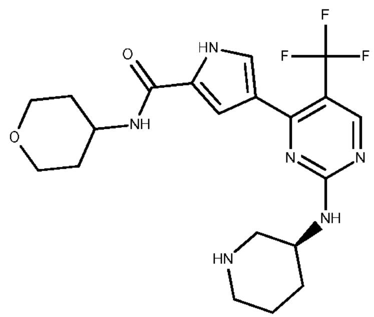 | **CDK7_1210**<br>6-cyclopentyl-3-(2-{[(3S)-piperidin-3-yl]amino}-5-(trifluoromethyl)p yrimidin-4-yl)-1H,6H,7H-pyrrolo[2,3-c]pyridin-7-one | 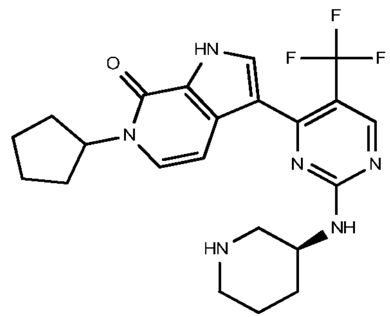 |

(continued)

| Code Name | Structure | Code Name | Structure |
|---|---|---|---|
| **CDK7_850_a** N-(oxan-4-yl)-4-{2-[(piperidin-3-yl)amino]-5-(trifluoromethy l)pyrimidin-4-yl}-1H-pyrrole-2-carboxamide | 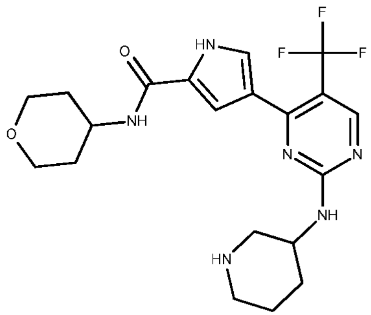 | **CDK7_1210_a** 6-cyclopentyl-3-{2-[(piperidin-3-yl)amino]-5-(trifluoromethyl)p yrimidin-4-yl}-1H,6H,7H-pyrrolo[2,3-c]pyridin-7-one | 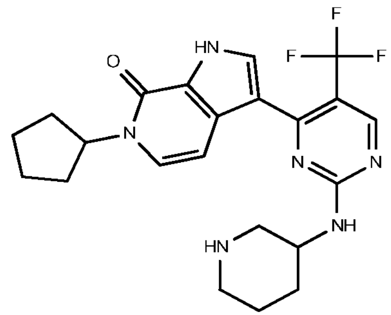 |
| **CDK7_851** N-(4-hydroxycycloh exyl)-4-(2-{[(3S)-piperidin-3-yl]amino}-5-(trifluoromethy l)pyrimidin-4-yl)-1H-pyrrole-2-carboxamide | 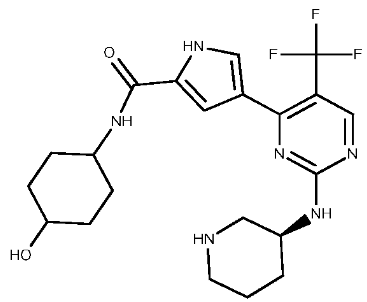 | **CDK7_1211** 6-(1-methylcyclopropy l)-3-(2-{[(3S)-piperidin-3-yl]amino}-5-(trifluoromethyl)p yrimidin-4-yl)-1H,6H,7H-pyrrolo[2,3-c]pyridin-7-one | 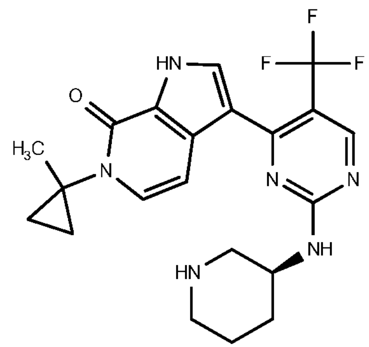 |
| **CDK7_851_a** N-(4-hydroxycycloh exyl)-4-{2-[(piperidin-3-yl)amino]-5-(trifluoromethy l)pyrimidin-4-yl}-1H-pyrrole-2-carboxamide | 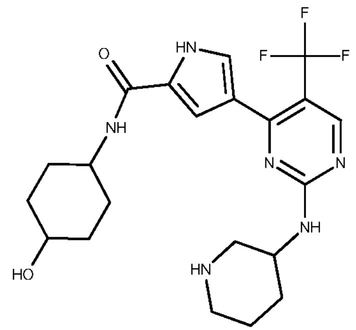 | **CDK7_1211_a** 6-(1-methylcyclopropy l)-3-{2-[(piperidin-3-yl) amino]-5-(trifluoromethyl)p yrimidin-4-yl }-1H,6H,7H-pyrrolo[2,3-c]pyridin-7-one | 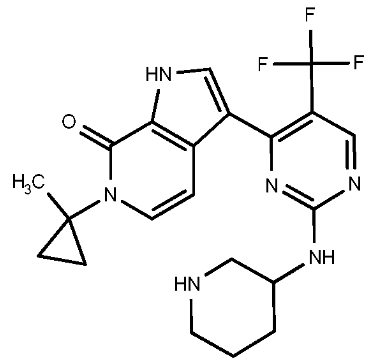 |

(continued)

| Code Name | Structure | Code Name | Structure |
|---|---|---|---|
| **CDK7_852**<br>N-(4-cyanocyclohex yl)-4-(2-{[(3S)-piperidin-3-yl]amino}-5-(trifluoromethy l)pyrimidin-4-yl)-1H-pyrrole-2-carboxamide | 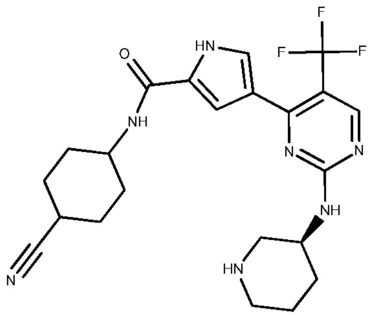 | **CDK7_1212**<br>6-cyclohexyl-3-(2-{[(3S)-piperidin-3-yl]amino}-5-(trifluoromethyl)p yrimidin-4-yl)-1H,6H,7H-pyrrolo[2,3-c]pyridin-7-one | 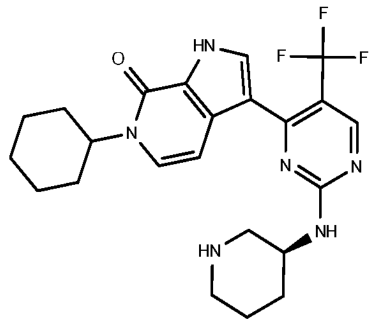 |
| **CDK7_852_a**<br>N-(4-cyanocyclohex yl)-4-{2-[(piperidin-3-yl)amino]-5-(trifluoromethy l)pyrimidin-4-yl}-1H-pyrrole-2-carboxamide | 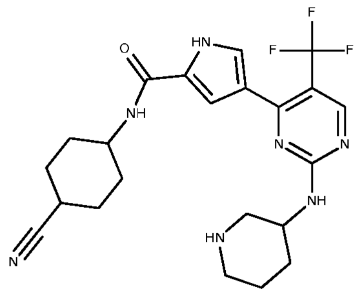 | **CDK7_1212_a**<br>6-cyclohexyl-3-{2-[(piperidin-3-yl)amino]-5-(trifluoromethyl)p yrimidin-4-yl }-1H,6H,7H-pyrrolo[2,3-c]pyridin-7-one | 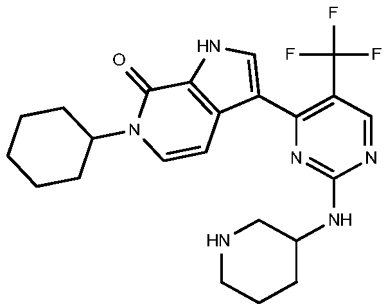 |
| **CDK7_951**<br>N-cyclopropyl-4-(2-{[(3S)-piperidin-3-yl]amino}-5-(trifluoromethy l)pyrimidin-4-yl)-1H-pyrrole-2-carboxamide | 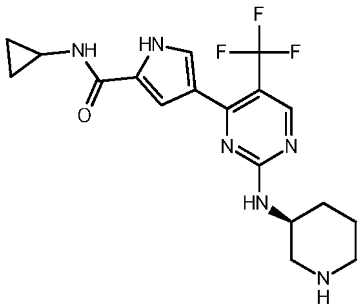 | **CDK7_1213**<br>3-(2-{[(3S)-piperidin-3-yl]amino}-5-(trifluoromethyl)p yrimidin-4-yl)-6-(pyridin-2-yl)-1H,6H,7H-pyrrolo[2,3-c]pyridin-7-one | 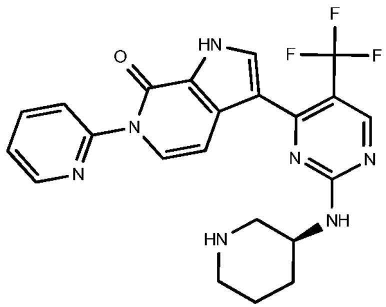 |

(continued)

| Code Name | Structure | Code Name | Structure |
|---|---|---|---|
| **CDK7_951_a**<br>N-cyclopropyl-4-{2-[(piperidin-3-yl) amino]-5-(trifluoromethy l)pyrimidin-4-yl}-1H-pyrrole-2-carboxamide | 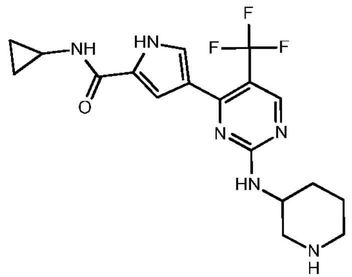 | **CDK7_1213_a**<br>3-{2-[(piperidin-3-yl)amino]-5-(trifluoromethyl) p yrimidin-4-yl}-6-(pyridin-2-yl)-1H,6H,7H-pyrrolo[2,3-c]pyridin-7-one | 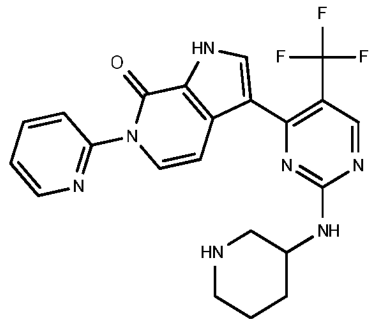 |
| **CDK7_923**<br>6-(3,5-dimethyl-1,2-oxazol-4-yl)-3-(2-{[(3S)-piperidin-3-yl]amino}-5-(trifluoromethy l)pyrimidin-4-yl)-1H,4H,5H,6H, 7H-pyrrolo[2,3-c] pyridin-7-one | 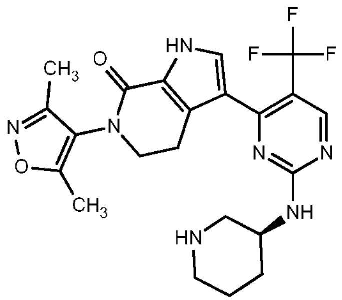 | **CDK7_1214**<br>3-(2-{[(3S)-piperidin-3-yl]amino}-5-(trifluoromethyl)p yrimidin-4-yl)-6-(pyridin-3-yl)-1H,6H,7H-pyrrolo[2,3-c]pyridin-7-one | 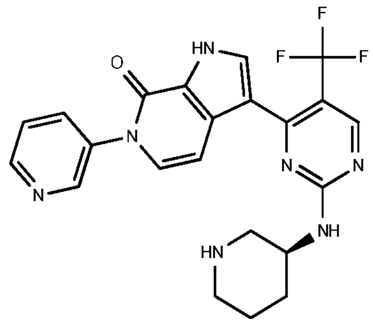 |
| **CDK7_923_a**<br>6-(3,5-dimethyl-1,2-oxazol-4-yl)-3-{2-[(piperidin-3-yl)amino]-5-(trifluoromethy l)pyrimidin-4-yl}-1H,4H,5H,6H, 7H-pyrrolo[2,3-c] pyridin-7-one | 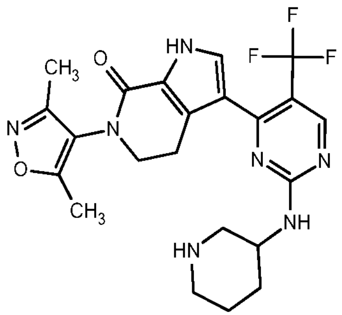 | **CDK7_1214_a**<br>3-{2-[(piperidin-3-yl)amino]-5-(trifluoromethyl) p yrimidin-4-yl}-6-(pyridin-3-yl)-1H,6H,7H-plo [2,3-c]pyridin-7-one | 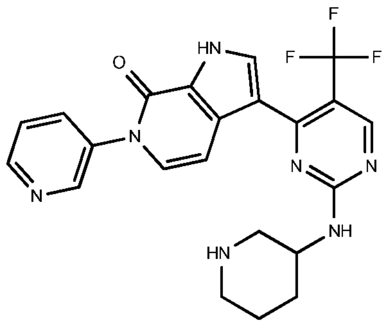 |

(continued)

| Code Name | Structure | Code Name | Structure |
|---|---|---|---|
| **CDK7_924** 6-(3,5-dimethyl-1,2-oxazol-4-yl)-3-(2-{[(3S)-piperidin-3-yl]amino}-5-(trifluoromethy l)pyrimidin-4-yl)-1H,6H,7H-pyrrolo[2,3-c]pyridin-7-one | 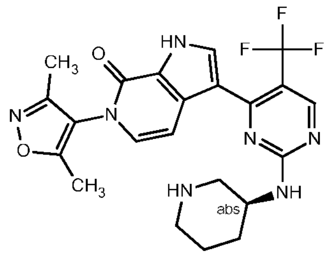 | **CDK7_1215** 3-(2-{[(3S)-piperidin-3-yl]amino}-5-(trifluoromethyl)p yrimidin-4-yl)-6-(pyridin-4-yl)-1H,6H,7H-pyrrolo[2,3-c]pyridin-7-one | 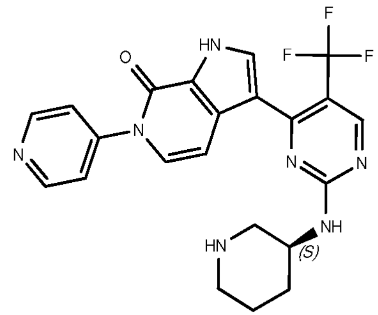 |
| **CDK7_924_a** 6-(3,5-dimethyl-1,2-oxazol-4-yl)-3-{2-[(piperidin-3-yl)amino]-5-(trifluoromethy l)pyrimidin-4-yl}-1H,6H,7H-pyrrolo[2,3-c]pyridin-7-one | 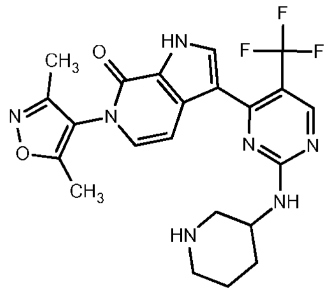 | **CDK7_1215_a** 3-{2-[(piperidin-3-yl)amino]-5-(trifluoromethyl) p yrimidin-4-yl}-6-(pyridin-4-yl)-1H,6H,7H-pyrrolo[2,3-c]pyridin-7-one | 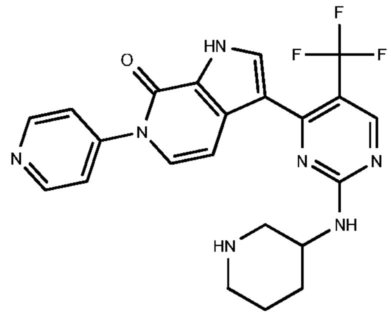 |
| **CDK7_925** 3-(2-{[(3S)-piperidin-3-yl]amino}-5-(trifluoromethy l)pyrimidin-4-yl)-1H,4H,7H,8H-pyrrolo[2,3-c]azepin-8-one | 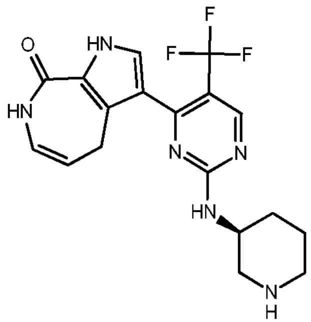 | **CDK7_1216** 6-(3-methylpyridin-4-yl)-3-(2-{ [(3 S)-piperidin-3-yl]amino}-5-(trifluoromethyl)p yrimidin-4-yl)-1H,6H,7H-pyrrolo[2,3-c]pyridin-7-one | 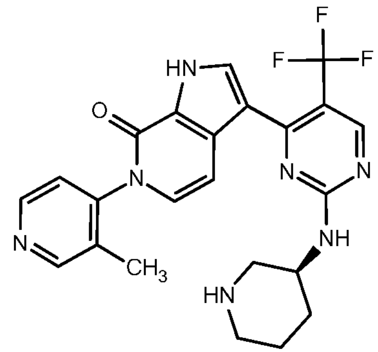 |

(continued)

| Code Name | Structure | Code Name | Structure |
|---|---|---|---|
| **CDK7_925_a** 3-{2-[(piperidin-3-yl)amino]-5-(trifluoromethy l)pyrimidin-4-yl}-1H,4H,7H,8H-pyrrolo[2,3-c]azepin-8-one | 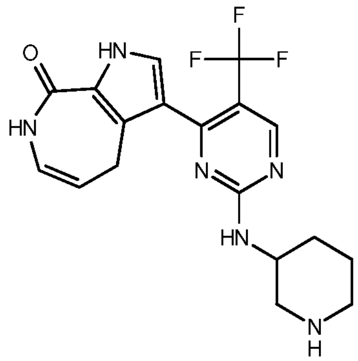 | **CDK7_1216_a** 6-(3-methylpyridin-4-yl)-3-{2-[(piperidin-3-yl)amino]-5-(trifluoromethyl)p yrimidin-4-yl}-1H,6H,7H-pyrrolo[2,3-c]pyridin-7-one | 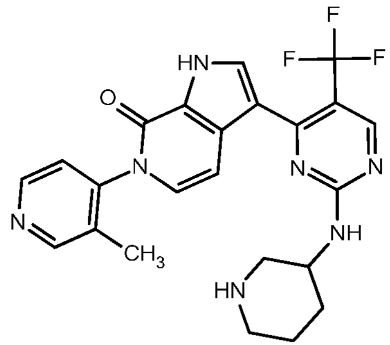 |
| **CDK7_926** 3-(2-{[(3S)-piperidin-3-yl]amino}-5-(trifluoromethy l)pyrimidin-4-yl)-1H,6H,7H,8H-pyrrolo[2,3-c]azepin-8-one | 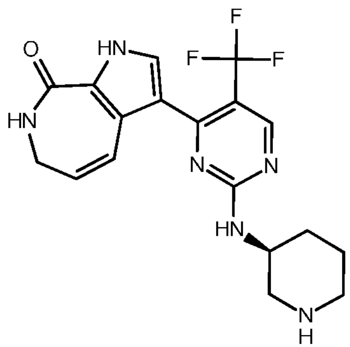 | **CDK7_1217** 6-(2-methylpyridin-3-yl)-3-(2-{ [(3 S)-piperidin-3-yl]amino}-5-(trifluoromethyl)p yrimidin-4-yl)-1H,6H,7H-pyrrolo[2,3-c]pyridin-7-one | 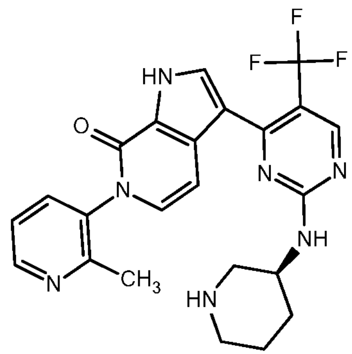 |
| **CDK7_926_a** 3-{2-[(piperidin-3-yl)amino]-5-(trifluoromethy l)pyrimidin-4-yl}-1H,6H,7H,8H-pyrrolo[2,3-c]azepin-8-one | 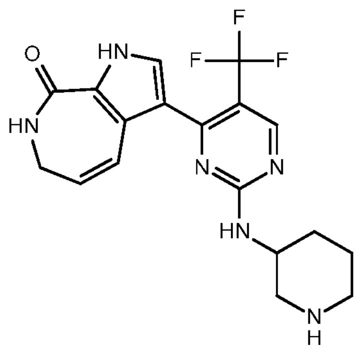 | **CDK7_1217_a** 6-(2-methylpyridin-3-yl)-3-{2-[(piperidin-3-yl)amino]-5-(trifluoromethyl)p yrimidin-4-yl }-1H,6H,7H-pyrrolo[2,3-c]pyridin-7-one | 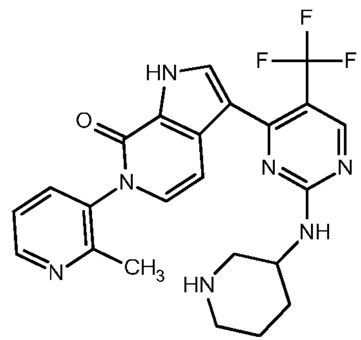 |

(continued)

| Code Name | Structure | Code Name | Structure |
|---|---|---|---|
| **CDK7_927** 3-(2-{[(3S)-piperidin-3-yl]amino}-5-(trifluoromethy l)pyrimidin-4-yl)-1H,8H-pyrrolo[2,3-c]azepin-8-one | 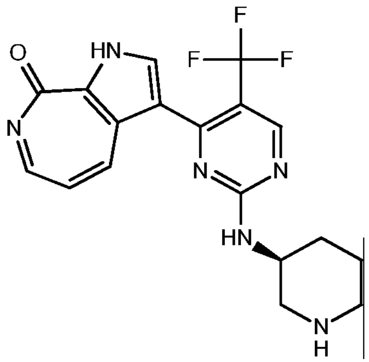 | **CDK7_1218** 6-(4-methylpyridin-3-yl)-3-(2-{ [(3 S)-piperidin-3-yl]amino}-5-(trifluoromethyl)p yrimidin-4-yl)-1H,6H,7H-pyrrolo[2,3-c]pyridin-7-one | 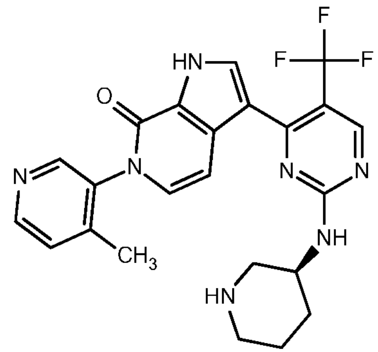 |
| **CDK7_927_a** 3-{2-[(piperidin-3-yl)amino]-5-(trifluoromethy l)pyrimidin-4-yl}-1H,8H-pyrrolo [2,3-c]azepin-8-one | 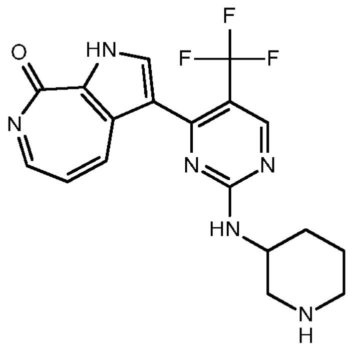 | **CDK7_1218_a** 6-(4-methylpyridin-3-yl)-3-{2-[(piperidin-3-yl) amino]-5-(trifluoromethyl)p yrimidin-4-yl }-1H,6H,7H-pyrrolo[2,3-c]pyridin-7-one | 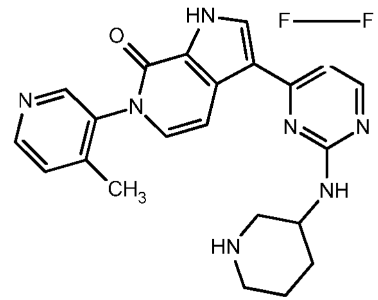 |
| **CDK7_928** 7-methyl-3-(2-{[(3S)-piperidin-3-yl] amino}-5-(trifluoromethy l)pyrimidin-4-yl)-1H,4H,7H,8H-pyrrolo[2,3-c]azepin-8-one | 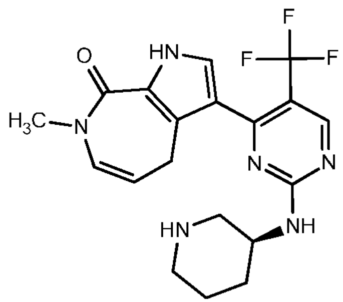 | **CDK7_1219** 6-(3-methylpyridin-2-yl)-3-(2-{ [(3 S)-piperidin-3-yl]amino}-5-(trifluoromethyl)p yrimidin-4-yl)-1H,6H,7H-pyrrolo[2,3-c]pyridin-7-one | 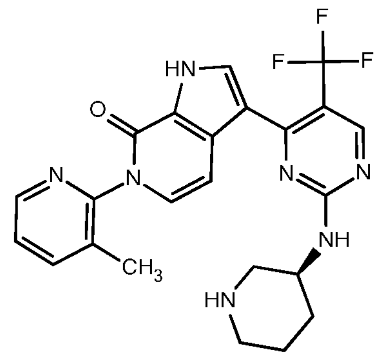 |

(continued)

| Code Name | Structure | Code Name | Structure |
|---|---|---|---|
| **CDK7_928_a** 7-methyl-3-{2-[(piperidin-3-yl)amino]-5-(trifluoromethy l)pyrimidin-4-yl}-1H,4H,7H,8H-pyrrolo[2,3-c]azepin-8-one | 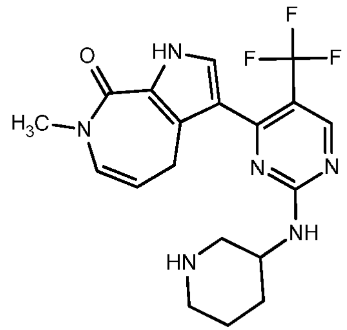 | **CDK7_1219_a** 6-(3-methylpyridin-2-yl)-3-{2-[(piperidin-3-yl) amino]-5-(trifluoromethyl)p yrimidin-4-yl }-1H,6H,7H-pyrrolo[2,3-c]pyridin-7-one | 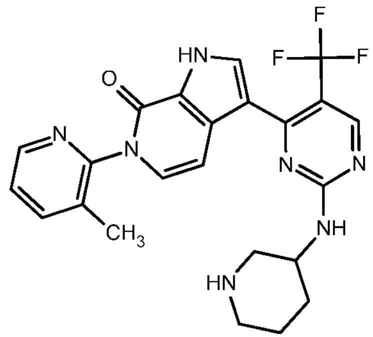 |
| **CDK7_929** 7-methyl-3-{2-[((3S)-piperidin-3-yl) amino]-5-(trifluoromethy l)pyrimidin-4-yl}-1H,4H,7H,8H-pyrrolo[2,3-c]azepin-8-one | 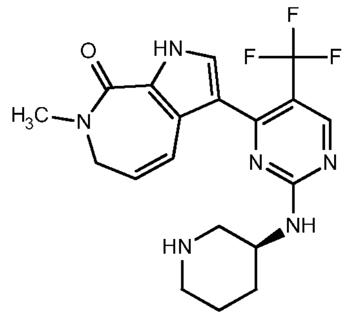 | **CDK7_1225** 7-cyclopropyl-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c] azepin-8-one | 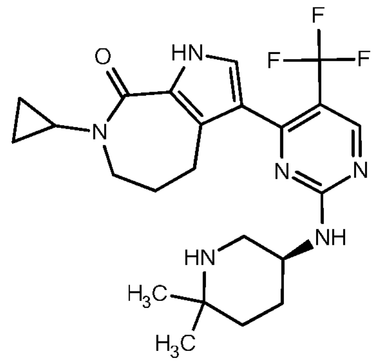 |
| **CDK7_929_a** 7-methyl-3-{2-[(piperidin-3-yl)amino]-5-(trifluoromethy l)pyrimidin-4-yl}-1H,4H,7H,8H-pyrrolo[2,3-c]azepin-8-one | 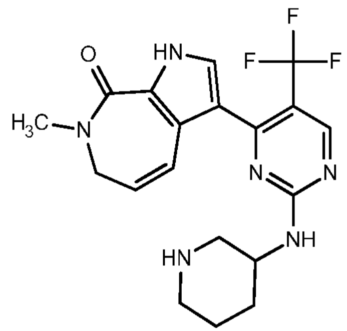 | **CDK7_1225_a** 7-cyclopropyl-3-{2-[(6,6-dimethylpiperidin -3-yl)amino]-5-(trifluoromethyl)p yrimidin-4-yl }-1H,4H,5H,6H,7H, 8H-pyrrolo[2,3-c]azepin-8-one | 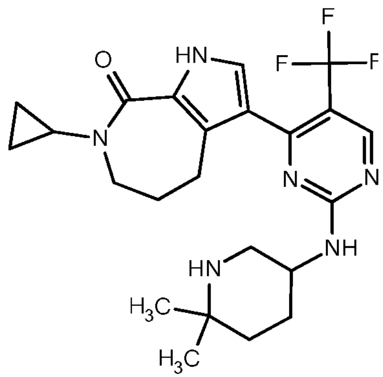 |

(continued)

| Code Name | Structure | Code Name | Structure |
|---|---|---|---|
| **CDK7_930**<br>7-methyl-3-(2-{[(3S)-piperidin-3-yl] amino}-5-(trifluoromethy l)pyrimidin-4-yl)-7H,8H-pyrrolo[2,3-c]azepin-8-one | 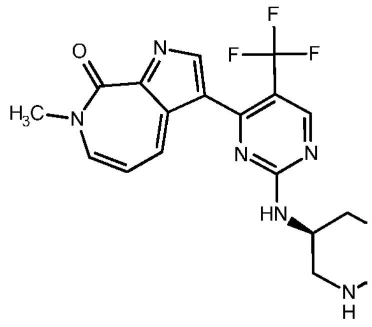 | **CDK7_1226**<br>7-cyclobutyl-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]aze-pin-8-one | 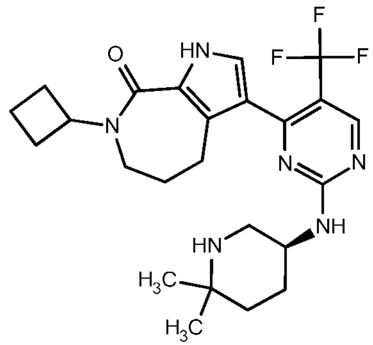 |
| **CDK7_930_a**<br>7-methyl-3-{2-[(piperidin-3-yl)amino]-5-(trifluoromethy l)pyrimidin-4-yl}-7H,8H-pyrrolo[2,3-c]azepin-8-one | 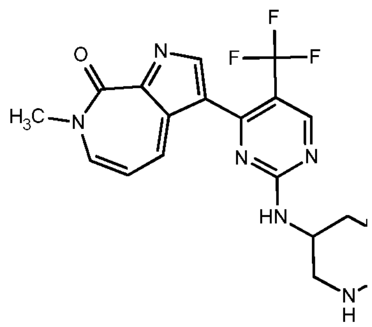 | **CDK7_1226_a**<br>7-cyclobutyl-3-(2-{[(6,6-dimethylpiperidin -3-yl]amino}-5-(trifluoromethyl)p yrimidin-4-yl)-1H,4H,5H,6H,7H, 8H-pyrrolo[2,3-c]aze-pin-8-one | 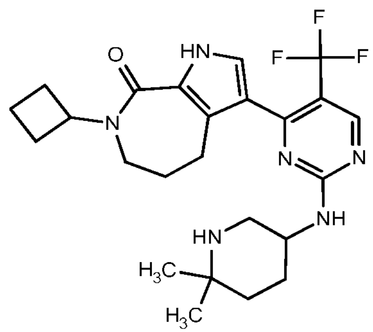 |
| **CDK7_931**<br>3-(2-{[(3S)-piperidin-3-yl]amino}-5-(trifluoromethy l)pyrimidin-4-yl)-7H,8H-pyrrolo[2,3-c]azepin-8-one | 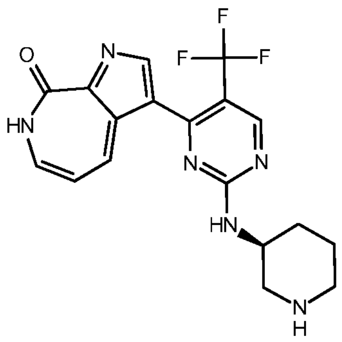 | **CDK7_1227**<br>7-(cyclopropylmeth yl)-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]azepin-8-one | 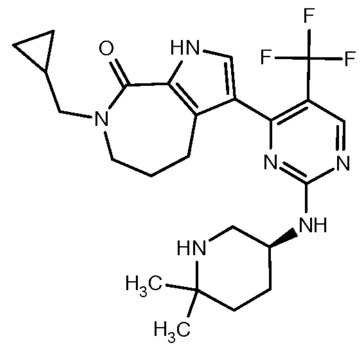 |

(continued)

| Code Name | Structure | Code Name | Structure |
|---|---|---|---|
| **CDK7_931_a** 3-{2-[(piperidin-3-yl)amino]-5-(trifluoromethy l)pyrimidin-4-yl}-7H,8H-pyrrolo [2,3-c]azepin-8-one | 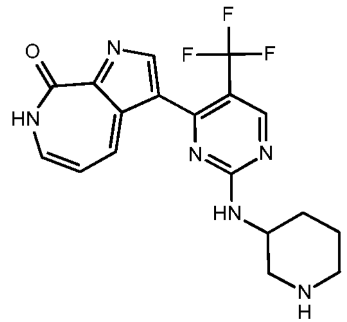 | **CDK7_1227_a** 7-(cyclopropylmeth yl)-3-{2-[(6,6-dimethylpiperidin -3-yl)amino]-5-(trifluoromethyl)p yrimidin-4-yl }-1H,4H,5H,6H,7H, 8H-pyrrolo[2,3-c] azepin-8-one | 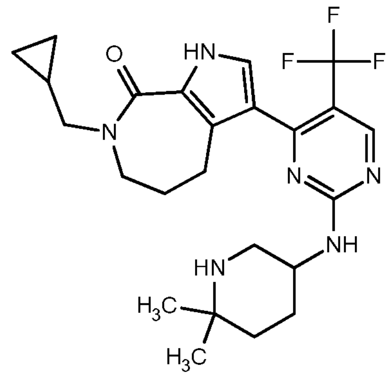 |
| **CDK7_932** 3-(2-{[(3S)-piperidin-3-yl]amino}-5-(trifluoromethy l)pyrimidin-4-yl)-1H,4H,5H,6H, 7H,8H,9H-pyrrolo [2,3-c]azocin-9-one | 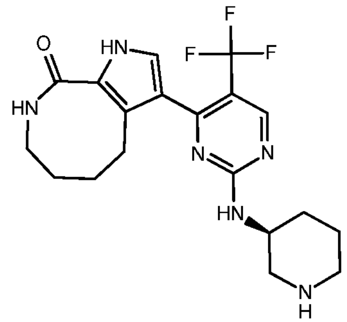 | **CDK7_1228** 7-(cyclobutylmethyl )-3-(2-{[(3S)-6,6-dimethylpiperidin -3-yl]amino}-5-(trifluoromethyl)p yrimidin-4-yl)-1H,4H,5H,6H,7H, 8H-pyrrolo[2,3-c]azepin-8-one | 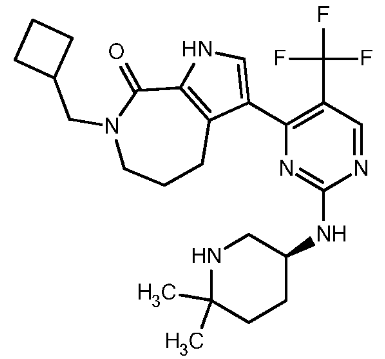 |
| **CDK7_932_a** 3-{2-[(piperidin-3-yl)amino]-5-(trifluoromethy l)pyrimidin-4-yl}-1H,4H,5H,6H, 7H,8H,9H-pyrrolo[2,3-c]azocin-9-one | 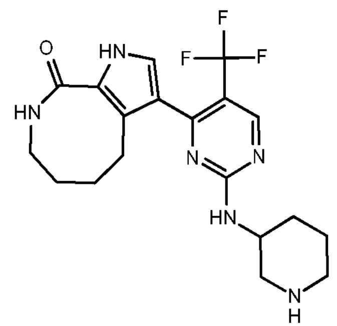 | **CDK7_1228_a** 7-(cyclobutylmethyl )-3-{2-[(6,6-dimethylpiperidin -3-yl)amino]-5-(trifluoromethyl)p yrimidin-4-yl }-1H,4H,5H,6H,7H, 8H-pyrrolo[2,3-c] azepin-8-one | 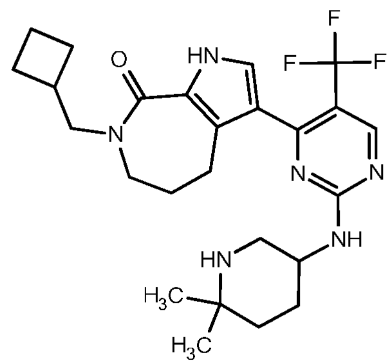 |

(continued)

| Code Name | Structure | Code Name | Structure |
|---|---|---|---|
| **CDK7_933**<br>8-methyl-3-(2-{[(3S)-piperidin-3-yl] amino}-5-(trifluoromethy l)pyrimidin-4-yl)-1H,4H,5H,6H, 7H,8H,9H-pyrrolo [2,3-c]azocin-9-one | 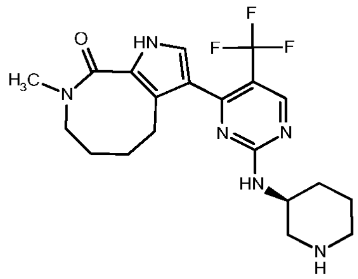 | **CDK7_1229**<br>3-(2-{[(3S)-6,6-dimethylpiperidin -3-yl]amino}-5-(trifluoromethyl)p yrimidin-4-yl)-7-(propan-2-yl)-1H,4H,5H,6H,7H, 8H-pyrrolo[2,3-c] azepin-8-one | 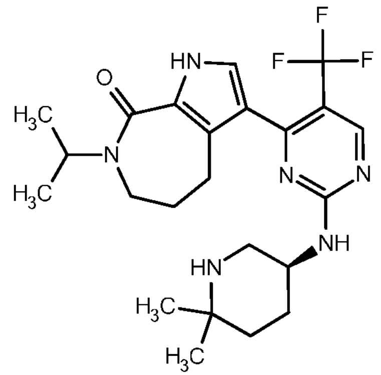 |
| **CDK7_933_a**<br>8-methyl-3-{2-[(piperidin-3-yl)amino]-5-(trifluoromethy l)pyrimidin-4-yl}-1H,4H,5H,6H, 7H,8H,9H-pyrrolo [2,3-c]azocin-9-one | 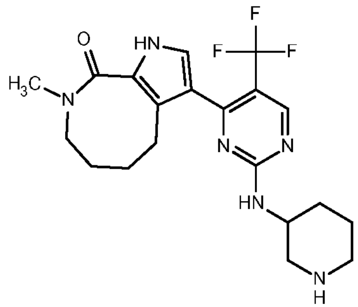 | **CDK7_1229_a**<br>3-{2-[(6,6-dimethylpiperidin -3-yl)amino]-5-(trifluoromethyl)p yrimidin-4-yl}-7-(propan-2-yl)-1H,4H,5H,6H,7H, 8H-pyrrolo[2,3-c]azepin-8-one | 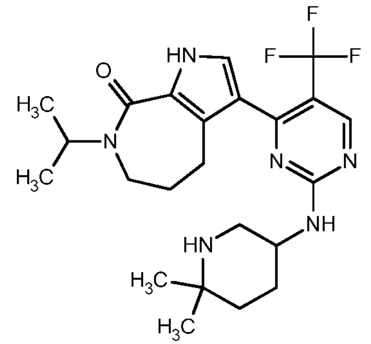 |
| **CDK7_934**<br>3-(2-{[(3S)-piperidin-3-yl]amino}-5-(trifluoromethy l)pyrimidin-4-yl)-1H,4H,5H,8H, 9H-pyrrolo[2,3-c] azocin-9-one | 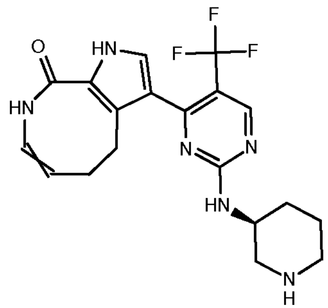 | **CDK7_1230**<br>3-(2-{[(3S)-6,6-dimethylpiperidin -3-yl]amino}-5-(trifluoromethyl)p yrimidin-4-yl)-7-(2,2,2-trifluoroethyl)-1H,4H,5H,6H,7H, 8H-pyrrolo [2,3-c]azepin-8-one | 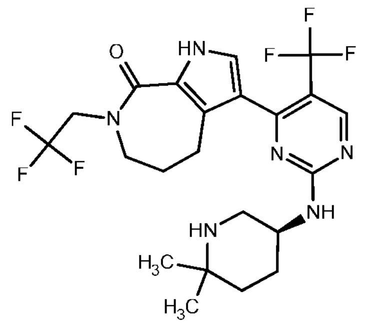 |

(continued)

| Code Name | Structure | Code Name | Structure |
|---|---|---|---|
| **CDK7_934_a**<br>3-{2-[(piperidin-3-yl)amino]-5-(trifluoromethy l)pyrimidin-4-yl}-1H,4H,5H,8H, 9H-pyrrolo[2,3-c]azocin-9-one | 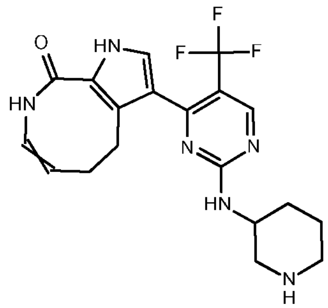 | **CDK7_1230_a**<br>3-{2-[(6,6-dimethylpiperidin -3-yl)amino]-5-(trifluoromethyl)p yrimidin-4-yl}-7-(2,2,2-trifluoroethyl)-1H,4H,5H,6H,7H, 8H-pyrrolo[2,3-c] azepin-8-one | 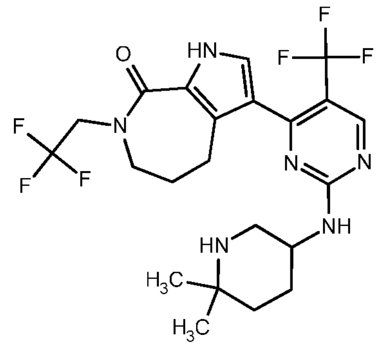 |
| **CDK7_935**<br>8-methyl-3-(2-{[(3S)-piperidin-3-yl] amino}-5-(trifluoromethy l)pyrimidin-4-yl)-1H,4H,5H,8H, 9H-pyrrolo[2,3-c] azocin-9-one | 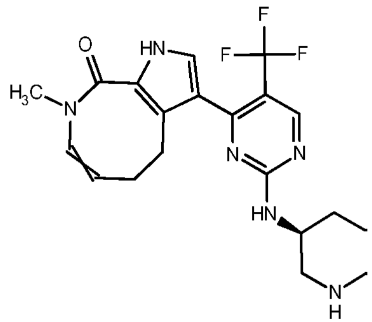 | **CDK7_1236**<br>4-{5-methyl-3,4,6,13-tetraazatricyclo[8. 3.0.0$^{2,6}$]trideca-1(10),2,4,11-tetraen-11-yl}-N-[(3S)-piperidin-3-yl]-5-(trifluoromethyl)p yrimidin-2-amine | 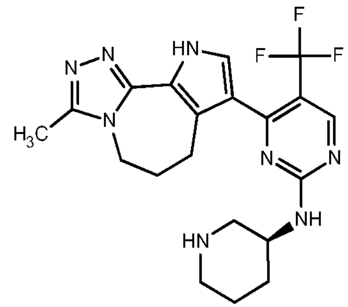 |
| **CDK7_935_a**<br>8-methyl-3-{2-[(piperidin-3-yl)amino]-5-(trifluoromethy l)pyrimidin-4-yl}-1H,4H,5H,8H, 9H-pyrrolo[2,3-c] azocin-9-one | 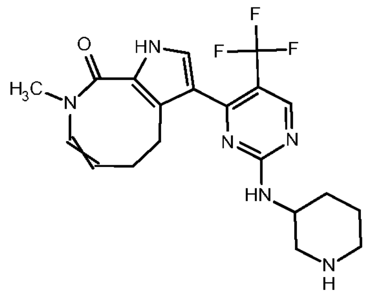 | **CDK7_1236_a**<br>4-{5-methyl-3,4,6,13-tetraazatricyclo[8. 3 .0.0$^{2,6}$]trideca-1(10),2,4,11-tetraen-11-yl}-N-(piperidin-3-yl)-5-(trifluoromethyl)p yrimidin-2-amine | 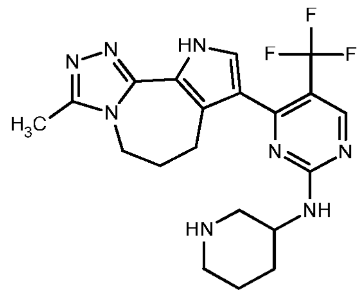 |

(continued)

| Code Name | Structure | Code Name | Structure |
|---|---|---|---|
| **CDK7_936**<br>3-(2-{[(3S)-piperidin-3-yl]amino}-5-(trifluoromethy l)pyrimidin-4-yl)-1H,4H,7H,8H, 9H-pyrrolo[2,3-c] azocin-9-one | 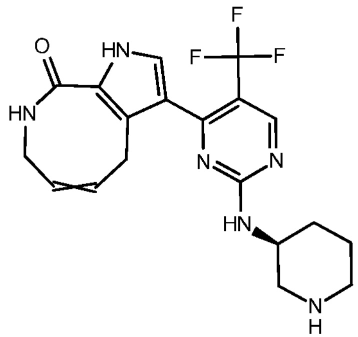 | **CDK7_1237**<br>4-{5-cyclopropyl-3,4,6,13-tetraazatricyclo[8. 3 .0.0$^{2,6}$]trideca-1(10),2,4,11-tetraen-11-yl}-N-[(3S)-piperidin-3-yl]-5-(trifluoromethyl)p yrimidin-2-amine | 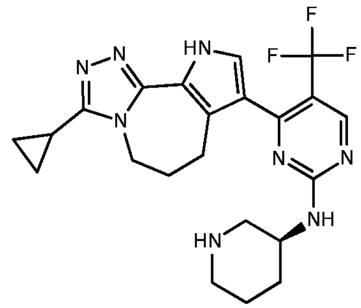 |
| **CDK7_936_a**<br>3-{2-[(piperidin-3-yl)amino]-5-(trifluoromethy l)pyrimidin-4-yl}-1H,4H,7H,8H, 9H-pyrrolo[2,3-c]azocin-9-one | 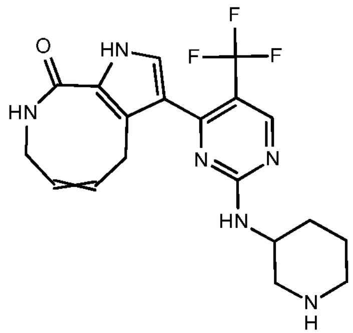 | **CDK7_1237_a**<br>4-{5-cyclopropyl-3,4,6,13-tetraazatricyclo[8. 3 .0.0$^{2,6}$]trideca-1(10),2,4,11-tetraen-11-yl}-N-(piperidin-3-yl)-5-(trifluoromethyl)p yrimidin-2-amine | 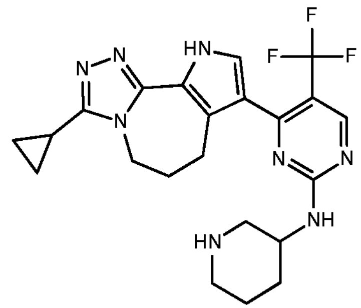 |
| **CDK7_937**<br>8-methyl-3-(2-{[(3S)-piperidin-3-yl] amino}-5-(trifluoromethy l)pyrimidin-4-yl)-1H,4H,7H,8H, 9H-pyrrolo[2,3-c] azocin-9-one | 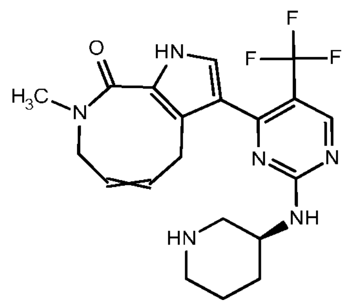 | **CDK7_1238**<br>3-(2-{[(3S)-6,6-dimethylpiperidin -3-yl]amino}-5-(trifluoromethyl)p yrimidin-4-yl)-6,6,7-trimethyl-1H,4H,5H,6H,7H, 8H-pyrrolo[2,3-c] azepin-8-one | 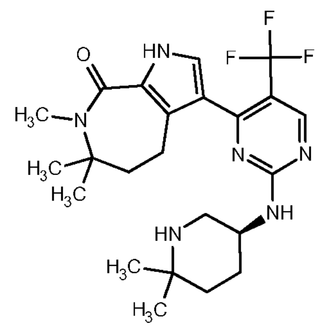 |

(continued)

| Code Name | Structure | Code Name | Structure |
|---|---|---|---|
| **CDK7_937_a**<br>8-methyl-3-{2-[(piperidin-3-yl)amino]-5-(trifluoromethy l)pyrimidin-4-yl}-1H,4H,7H,8H, 9H-pyrrolo[2,3-c]azocin-9-one | 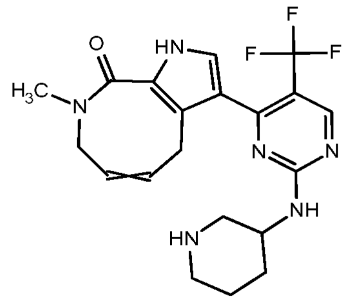 | **CDK7_1238_a**<br>3-{2-[(6,6-dimethylpiperidin -3-yl)amino]-5-(trifluoromethyl)p yrimidin-4-yl }-6,6,7-trimethyl-1H,4H,5H,6H,7H, 8H-pyrrolo[2,3-c]azepin-8-one | 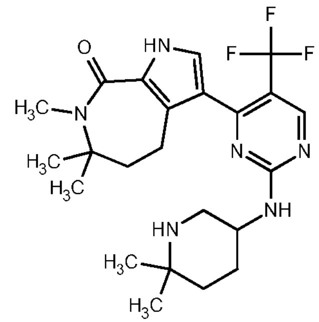 |
| **CDK7_938**<br>3-(2-{[(3S)-piperidin-3-yl]amino}-5-(trifluoromethy l)pyrimidin-4-yl)-1H,6H,7H,8H, 9H-pyrrolo[2,3-c]azocin-9-one | 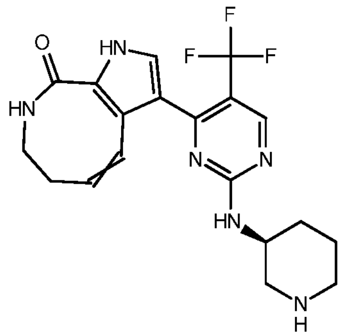 | **CDK7_1239**<br>(6S)-3-(2-{[(3S)-6,6-dimethylpiperidin -3-yl]amino}-5-(trifluoromethyl)p yrimidin-4-yl)-6,7-dimethyl-1H,4H,5H,6H,7H, 8H-pyrrolo[2,3-c]azepin-8-one | 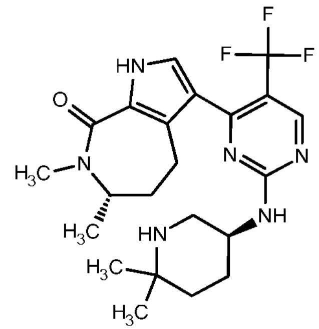 |
| **CDK7_938_a**<br>3-{2-[(piperidin-3-yl)amino]-5-(trifluoromethy l)pyrimidin-4-yl}-1H,6H,7H,8H, 9H-pyrrolo[2,3-c]azocin-9-one | 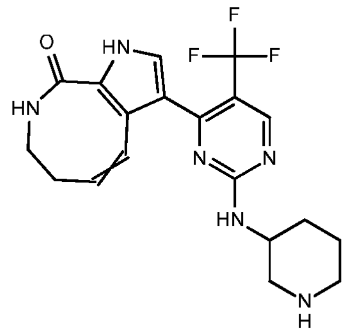 | **CDK7_1239_a**<br>3-{2-[(6,6-dimethylpiperidin -3-yl)amino]-5-(trifluoromethyl)p yrimidin-4-yl}-6,7-dimethyl-1H,4H,5H,6H,7H, 8H-pyrrolo[2,3-c]azepin-8-one | 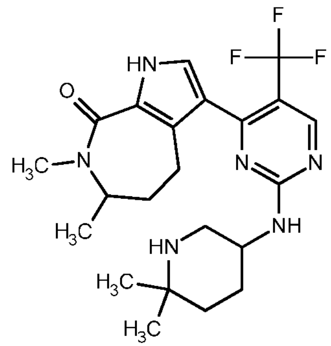 |

(continued)

| Code Name | Structure | Code Name | Structure |
|---|---|---|---|
| **CDK7_939** 8-methyl-3-(2-{[(3S)-piperidin-3-yl] amino}-5-(trifluoromethy l)pyrimidin-4-yl)-1H,6H,7H,8H, 9H-pyrrolo[2,3-c] azocin-9-one | 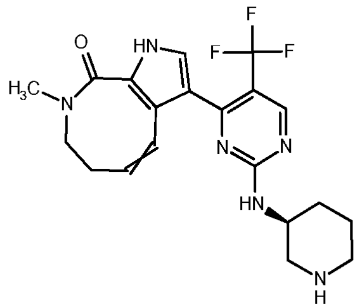 | **CDK7_1240** (6R)-3-(2-{[(3S)-6,6-dimethylpiperidin -3-yl] amino}-5-(trifluoromethyl)p yrimidin-4-yl)-6,7-dimethyl-1H,4H,5H,6H,7H, 8H-pyrrolo[2,3-c] azepin-8-one | 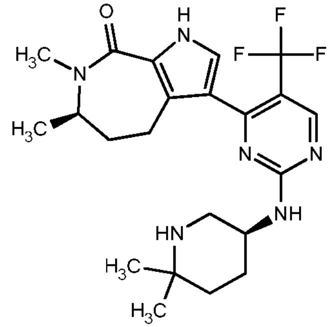 |
| **CDK7_939_a** 8-methyl-3-{2-[(piperidin-3-yl)amino]-5-(trifluoromethy l)pyrimidin-4-yl}-1H,6H,7H,8H, 9H-pyrrolo[2,3-c] azocin-9-one | 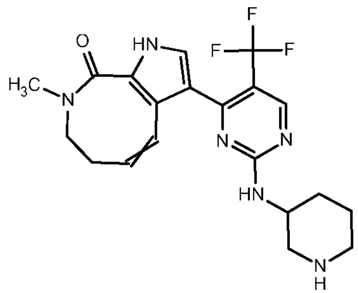 | **CDK7_1240_a** (6R)-3-(2-{[6,6-dimethylpiperidin -3-yl]amino}-5-(trifluoromethyl)p yrimidin-4-yl)-6,7-dimethyl-1H,4H,5H,6H,7H, 8H-pyrrolo[2,3-c] azepin-8-one | 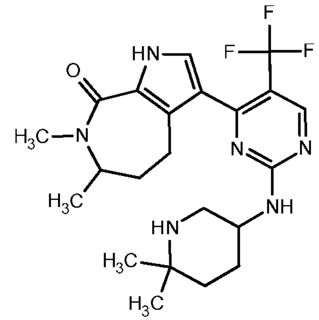 |
| **CDK7_940** 3-(2-{[(3S)-piperidin-3-yl]amino}-5-(trifluoromethy l)pyrimidin-4-yl)-1H,8H,9H-pyrrolo[2,3-c]azocin-9-one | 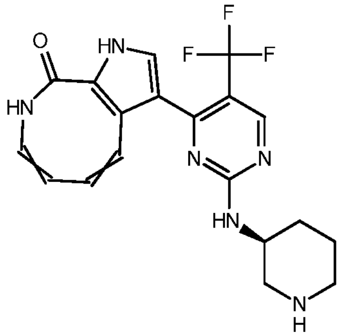 | **CDK7_1241** 7-cyclopropyl-3-(2-{[(3S)-6,6-dimethylpiperidin -3-yl]amino}-5-(trifluoromethyl)p yrimidin-4-yl)-6,6-dimethyl-1H,4H,5H,6H,7H, 8H-pyrrolo[2,3-c]azepin-8-one | 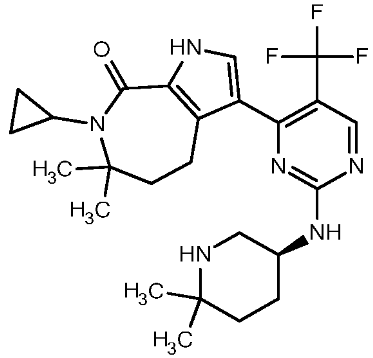 |

(continued)

| Code Name | Structure | Code Name | Structure |
|---|---|---|---|
| **CDK7_940_a** 3-(2-{[piperidin-3-yl]amino}-5-(trifluoromethy l)pyrimidin-4-yl)-1H,8H,9H-pyrrolo[2,3-c]azocin-9-one | 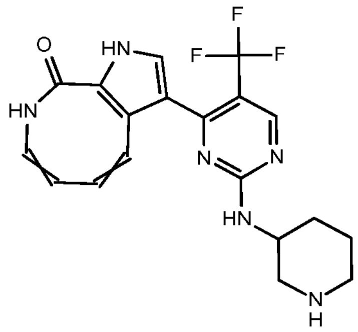 | **CDK7_1241_a** 7-cyclopropyl-3-{2-[(6,6-dimethylpiperidin -3-yl)amino]-5-(trifluoromethyl)p yrimidin-4-yl}-6,6-dimethyl-1H,4H,5H,6H,7H, 8H-pyrrolo [2,3-c]azepin-8-one | 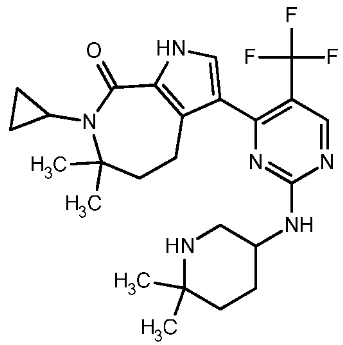 |
| **CDK7_941** 8-methyl-3-(2-{[(3S)-piperidin-3-yl] amino}-5-(trifluoromethy l)pyrimidin-4-yl)-1H,8H,9H-pyrrolo[2,3-c]azocin-9-one | 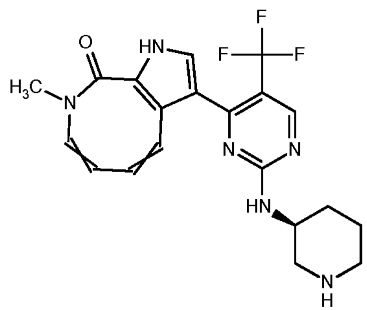 | **CDK7_1242** (6S)-7-cyclopropyl-3-(2-{[(3S)-6,6-dimethylpiperidin -3-yl]amino}-5-(trifluoromethyl)p yrimidin-4-yl)-6-methyl-1H,4H,5H,6H,7H, 8H-pyrrolo[2,3-c]azepin-8-one | 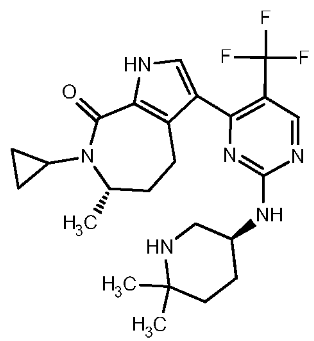 |
| **CDK7_941_a** 3-(2-{[(3S)-piperidin-3-yl]amino}-5-(trifluoromethy l)pyrimidin-4-yl)-9H-pyrrolo[2,3-c]azocin-9-one | 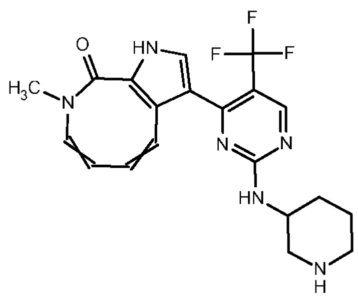 | **CDK7_1242_a** 7-cyclopropyl-3-{2-[(6,6-dimethylpiperidin -3-yl)amino]-5-(trifluoromethyl)p yrimidin-4-yl}-6-methyl-1H,4H,5H,6H,7H, 8H-pyrrolo[2,3-c] azepin-8-one | 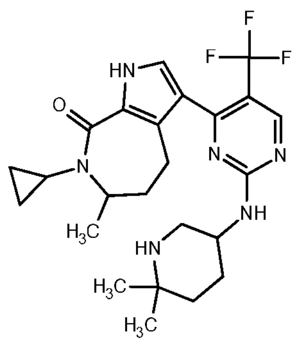 |

(continued)

| Code Name | Structure | Code Name | Structure |
|---|---|---|---|
| **CDK7_942** 8-methyl-3-{2-[(piperidin-3-yl)amino]-5-(trifluoromethy l)pyrimidin-4-yl}-1H,8H,9H-pyrrolo[2,3-c]azocin-9-one | 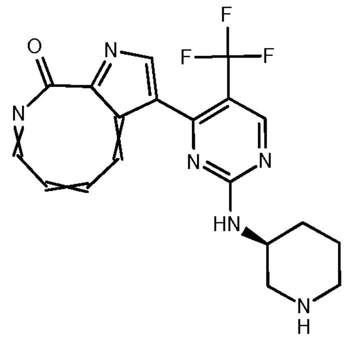 | **CDK7_1243** (6R)-7-cyclopropyl-3-(2-{[(3S)-6,6-dimethylpiperidin -3-yl]amino}-5-(trifluoromethyl)p yrimidin-4-yl)-6-methyl-1H,4H,5H,6H,7H, 8H-pyrrolo[2,3-c]azepin-8-one | 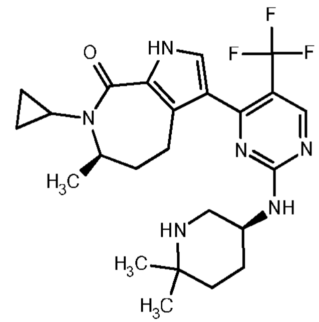 |
| **CDK7_942_a** 3-{2-[(piperidin-3-yl)amino]-5-(trifluoromethy l)pyrimidin-4-yl}-9H-pyrrolo [2,3-c]azocin-9-one | 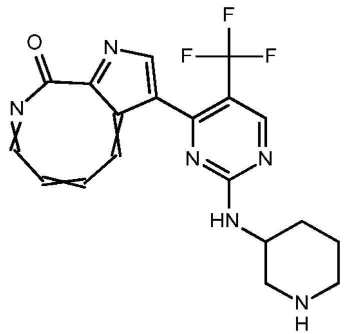 | **CDK7_1243_a** 7-cyclopropyl-3-{2-[(6,6-dimethylpiperidin -3-yl)amino]-5-(trifluoromethyl)p yrimidin-4-yl}-6-methyl-1H,4H,5H,6H,7H, 8H-pyrrolo[2,3-c] azepin-8-one | 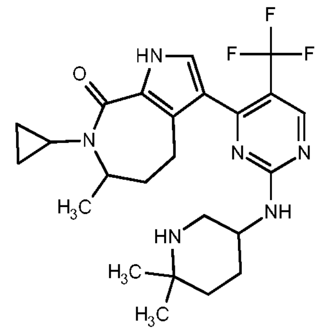 |
| **CDK7_943** 3-(2-{[(3S)-piperidin-3-yl]amino}-5-(trifluoromethy l)pyrimidin-4-yl)-1H,4H,5H,6H-pyrrolo[2,3-c]pyrrol-6-one | 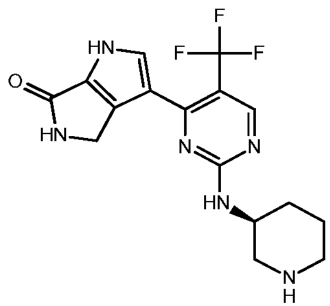 | **CDK7_1244** 3-(2-{[(3S)-6,6-dimethylpiperidin -3-yl]amino}-5-(trifluoromethyl)p yrimidin-4-yl)-5,5,6-trimethyl-1H,4H,5H,6H,7H -pyrrolo[2,3-c]pyridin-7-one | 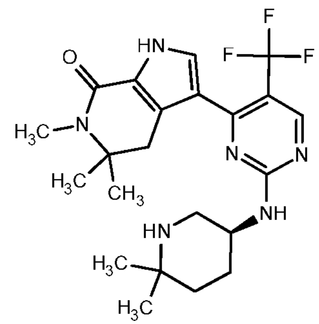 |

(continued)

| Code Name | Structure | Code Name | Structure |
|---|---|---|---|
| **CDK7_943_a** 3-{2-[(piperidin-3-yl)amino]-5-(trifluoromethy l)pyrimidin-4-yl}-1H,4H,5H,6H-pyrrolo[2,3-c]pyr-rol-6-one | 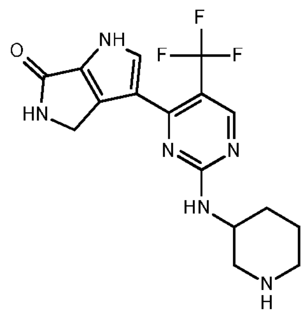 | **CDK7_1244_a** 3-{2-[(6,6-dimethylpiperidin -3-yl)amino]-5-(trifluoromethyl)p yrimidin-4-yl }-5,5,6-trimethyl-1H,4H,5H,6H,7H -pyrrolo[2,3-c]pyri-din-7-one | 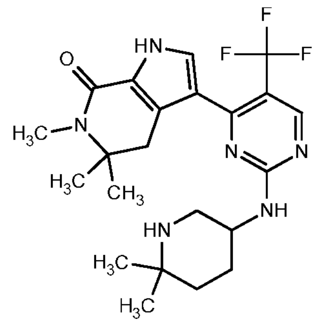 |
| **CDK7_944** 5-methyl-3-(2-{[(3S)-piperidin-3-yl] amino}-5-(trifluoromethy l)pyrimidin-4-yl)-1H,4H,5H,6H-pyrrolo[2,3-c\|pyr-rol-6-one | 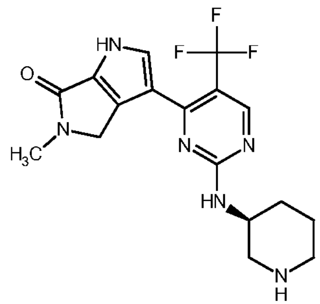 | **CDK7_1254** 6,7-dimethyl-3-(2-{[(3S)-piperidin-3-yl]amino}-5-(trifluoromethyl)p yrimidin-4-yl)-1H,4H,5H,6H,7H, 8H-pyrrolo[2,3-c]aze-pin-8-one | 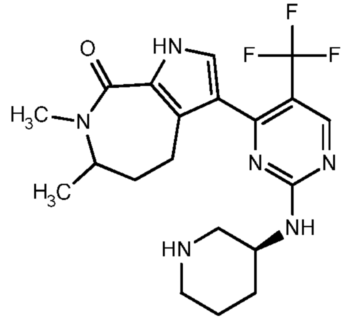 |
| **CDK7_944_a** 5-methyl-3-{2-[(piperidin-3-yl)amino]-5-(trifluoromethy l)pyrimidin-4-yl}-1H,4H,5H,6H-pyrrolo[2,3-c]pyr-rol-6-one | 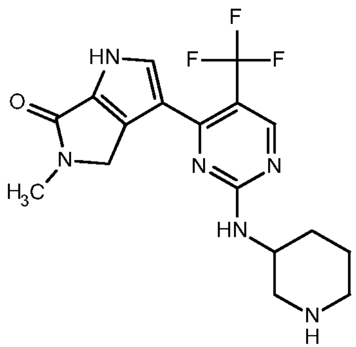 | **CDK7_1254_a** 6,7-dimethyl-3-{2-[(piperidin-3-yl)amino]-5-(trifluoromethyl)p yrimidin-4-yl }-1H,4H,5H,6H,7H, 8H-pyrrolo[2,3-c]aze-pin-8-one | 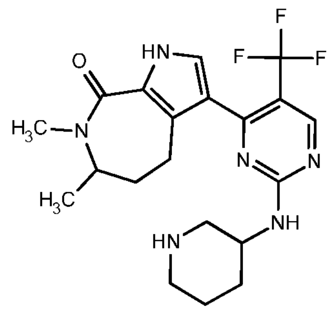 |

(continued)

| Code Name | Structure | Code Name | Structure |
|---|---|---|---|
| **CDK7_945** N-[(3S)-piperidin-3-yl]-4-(1H-pyrazol-4-yl)-5-(trifluoromethy l)pyrimidin-2-amine | 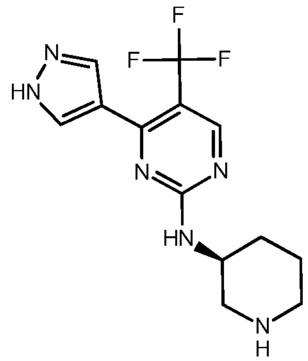 | **CDK7_1259** 3-(2-{[(3S)-6,6-dimethylpiperidin -3-yl]amino}-5-(trifluoromethyl)p yrimidin-4-yl)-7-ethyl-1H,4H,5H,6H,7H, 8H-pyrrolo[2,3-c]azepin-8-one | 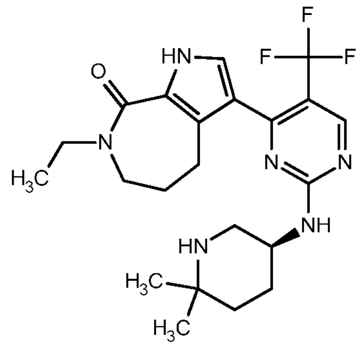 |
| **CDK7_945_a** N-(piperidin-3-yl)-4-(1H-pyrazol-4-yl)-5-(trifluoromethy l)pyrimidin-2-amine | 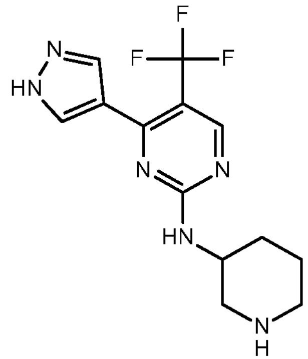 | **CDK7_1259_a** 3-{2-[(6,6-dimethylpiperidin -3-yl)amino]-5-(trifluoromethyl)p yrimidin-4-yl}-7-ethyl-1H,4H,5H,6H,7H, 8H-pyrrolo[2,3-c]azepin-8-one7 | 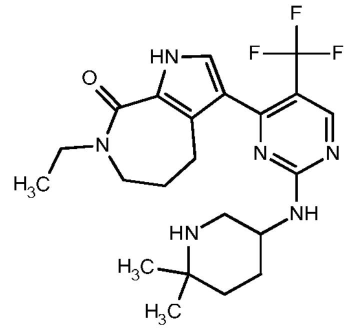 |
| **CDK7_946** 4-(1H-imidazol-4-yl)-N-[(3S)-piperidin-3-yl]-5-(trifluoromethy l)pyrimidin-2-amine | 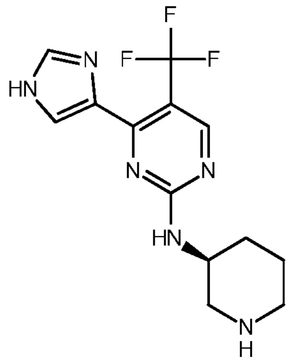 | **CDK7_1261** 6-(2-{[(3S)-6,6-dimethylpiperidin -3-yl]amino}-5-(trifluoromethyl)p yrimidin-4-yl)-1,4-diazatricyclo[7.1. 1.0$^{3,7}$]undeca-3(7),5-dien-2-one | 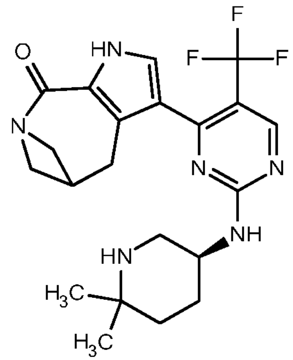 |

(continued)

| Code Name | Structure | Code Name | Structure |
|---|---|---|---|
| **CDK7_946_a**<br>4-(1H-imidazol-4-yl)-N-(piperidin-3-yl)-5-(trifluoromethy l)pyrimidin-2-amine | 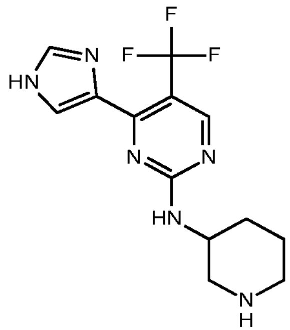 | **CDK7_1261_a**<br>6-{2-[(6,6-dimethylpiperidin -3-yl)amino]-5-(trifluoromethyl)p yrimidin-4-yl }-1,4-diazatricyclo [7.1. 1.0$^{3,7}$]undeca-3(7),5-dien-2-one | 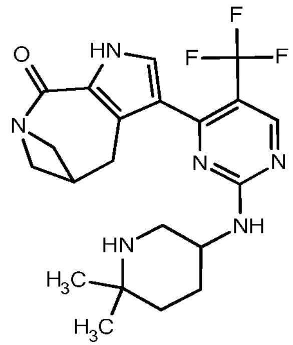 |
| **CDK7_947**<br>N-[(3S)-piperidin-3-yl]-4-(1H-pyrazol-3-yl)-5-(trifluoromethy l)pyrimidin-2-amine | 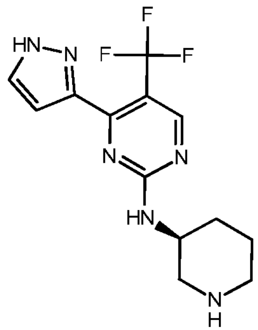 | **CDK7_1264**<br>7-methyl-3-(2-{[(3S,6S)-6-methylpiperidin-3-yl]amino}-5-(trifluoromethyl)p yrimidin-4-yl)-1H,4H,5H,6H,7H, 8H-pyrrolo[2,3-c]azepin-8-one | 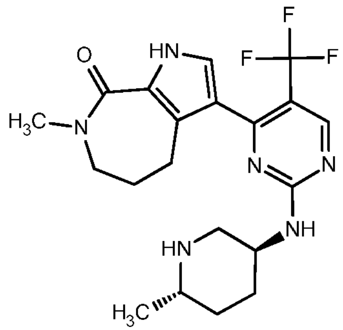 |
| **CDK7_947_a**<br>N-[piperidin-3-yl]-4-(1H-pyrazol-3-yl)-5-(trifluoromethy l)pyrimidin-2-amine | 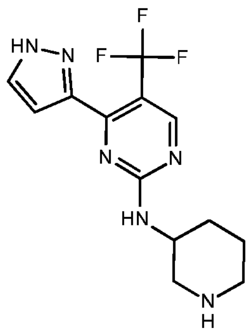 | **CDK7_1264_a**<br>7-methyl-3-{2-[(6-methylpiperidin-3-yl)amino]-5-(trifluoromethyl) pyrimidin-4-yl}-1H,4H,5H,6H,7H, 8H-pyrrolo[2,3-c]azepin-8-one | 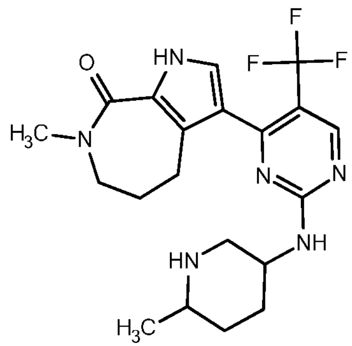 |

(continued)

| Code Name | Structure | Code Name | Structure |
|---|---|---|---|
| **CDK7948** _ N-[(3S)-piperidin-3-yl]-4-(1H-1,2,3-triazol-4-yl)-5-(trifluoromethy l)pyrimidin-2-amine | 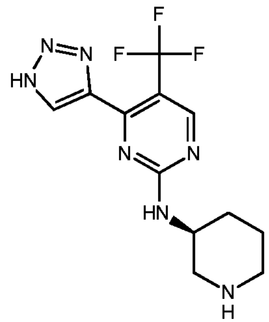 | **CDK7_1265** 3-(2-{[(3S,6S)-6-ethylpiperidin-3-yl]amino}-5-(trifluoromethyl)p yrimidin-4-yl)-7-methyl-1H,4H,5H,6H,7H, 8H-pyrrolo[2,3-c] azepin-8-one | 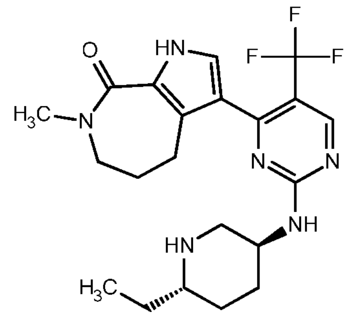 |
| **CDK7_948_a** N-(piperidin-3-yl)-4-(1H-1,2,3-triazol-4-yl)-5-(trifluoromethy l)pyrimidin-2-amine | 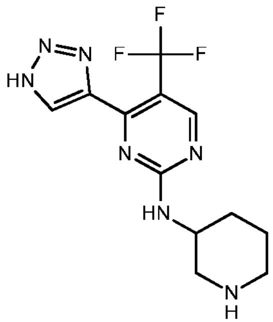 | **CDK7_1265_a** 3-{2-[(6-ethylpiperidin-3-yl)amino]-5-(trifluoromethyl)p yrimidin-4-yl}-7-methyl-1H,4H,5H,6H,7H, 8H-pyrrolo[2,3-c] azepin-8-one | 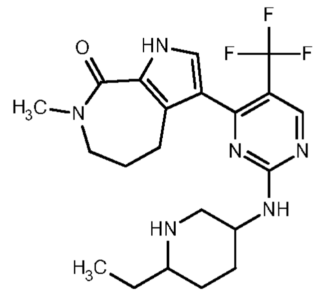 |
| **CDK7_949** N-[(3S)-piperidin-3-yl]-4-(1H-1,2,4-triazol-3-yl)-5-(trifluoromethy l)pyrimidin-2-amine | 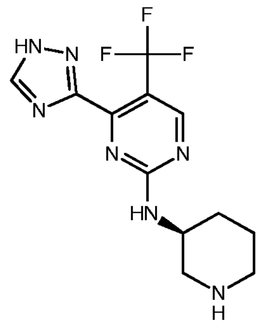 | **CDK7_1268** 3-(2-{[(3S)-6,6-dimethylpiperidin -3-yl]amino}-5-(trifluoromethyl)p yrimidin-4-yl)-7-(2-methoxyethyl)-1H,4H,5H,6H,7H, 8H-pyrrolo [2,3-c]azepin-8-one | 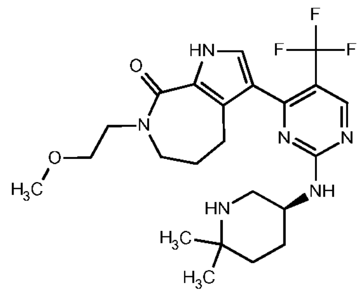 |

(continued)

| Code Name | Structure | Code Name | Structure |
|---|---|---|---|
| **CDK7_949_a** N-(piperidin-3-yl)-4-(1H-1,2,4-triazol-3-yl)-5-(trifluoromethy l)pyrimidin-2-amine | 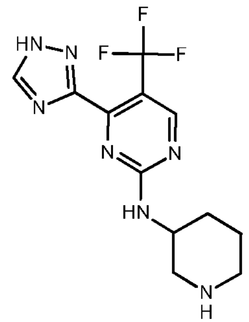 | **CDK7_1268_a** 3-{2-[(6,6-dimethylpiperidin -3-yl)amino]-5-(trifluoromethyl)p yrimidin-4-yl}-7-(2-methoxyethyl)-1H,4H,5H,6H,7H, 8H-pyrrolo[2,3-c]azepin-8-one | 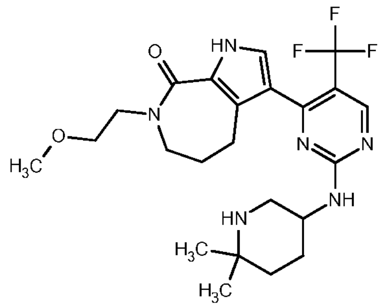 |
| **CDK7_950** N-[(3S)-piperidin-3-yl]-4-(2H-1,2,3,4-tetrazol-5-yl)-5-(trifluoromethy l)pyrimidin-2-amine | 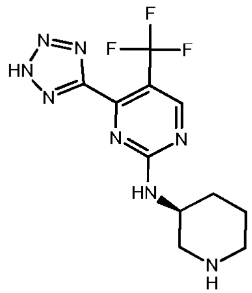 | **CDK7_1269** 2-[3-(2-{[(3S)-6,6-dimethylpiperidin -3-yl]amino}-5-(trifluoromethyl)p yrimidin-4-yl)-8-oxo-1H,4H,5H,6H,7H, 8H-pyrrolo[2,3-c]azepin-7-yl]acetonitrile | 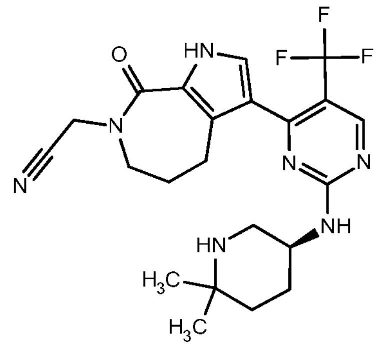 |
| **CDK7_950_a** N-(piperidin-3-yl)-4-(2H-1,2,3,4-tetrazol-5-yl)-5-(trifluoromethy l)pyrimidin-2-amine | 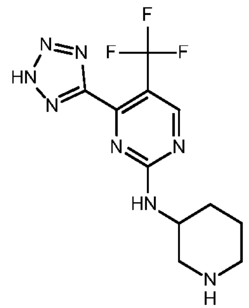 | **CDK7_1269_a** 2-(3-{2-[(6,6-dimethylpiperidin -3-yl)amino]-5-(trifluoromethyl)p yrimidin-4-yl}-8-oxo-1H,4H,5H,6H,7H, 8H-pyrrolo[2,3-c]azepin-7-yl)acetonitrile | 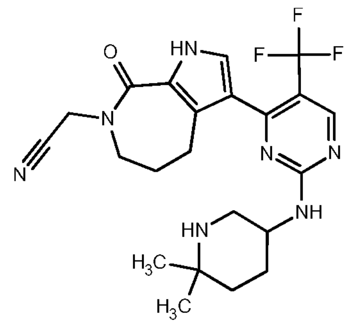 |

(continued)

| Code Name | Structure | Code Name | Structure |
|---|---|---|---|
| **CDK7_900** 4-(2-{[(3S)-piperidin-3-yl]amino}-5-(trifluoromethy l)pyrimidin-4-yl)-1,2,3,4-tetrahydroquin oxalin-2-one | 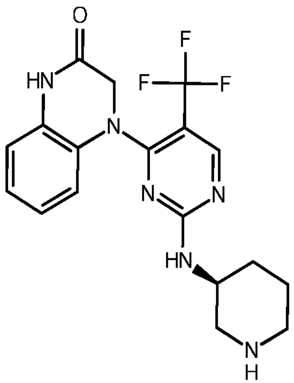 | **CDK7_1270** 3-(2-{[(3S)-6,6-dimethylpiperidin -3-yl]amino}-5-(trifluoromethyl)p yrimidin-4-yl)-7-(oxetan-3-yl)-1H,4H,5H,6H,7H, 8H-pyrrolo[2,3-c] azepin-8-one | 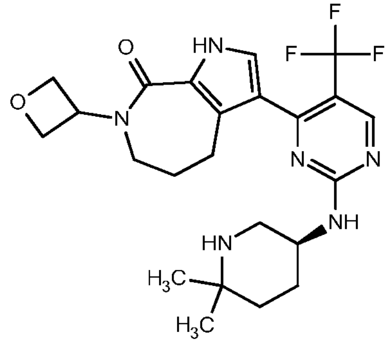 |
| **CDK7_900_a** 4-{2-[(piperidin-3-yl)amino]-5-(trifluoromethy l)pyrimidin-4-yl}-1,2,3,4-tetrahydroquin oxalin-2-one | 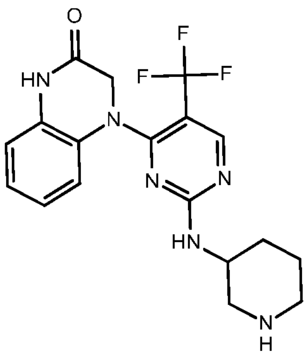 | **CDK7_1270_a** 3-{2-[(6,6-dimethylpiperidin -3-yl)amino]-5-(trifluoromethyl)p yrimidin-4-yl}-7-(oxetan-3-yl)-1H,4H,5H,6H,7H, 8H-pyrrolo[2,3-c]azepin-8-one | 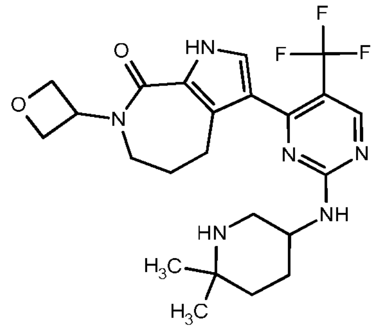 |

(continued)

| Code Name | Structure | Code Name | Structure |
|---|---|---|---|
| **CDK7_901**<br>4-(2-{[(3S)-piperidin-3-yl]amino}-5-(trifluoromethy l)pyrimidin-4-yl)-1,2-dihy-droquinoli n-2-one | 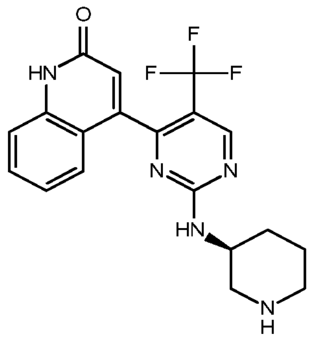 | **CDK7_1271**<br>3-(2-{[(3S)-6,6-dimethylpiperidin -3-yl]amino}-5-(trifluoromethyl)p yrimidin-4-yl)-7-[(oxetan-3-yl)methyl]-1H,4H,5H,6H,7H, 8H-pyrrolo [2,3-c]azepin-8-one | 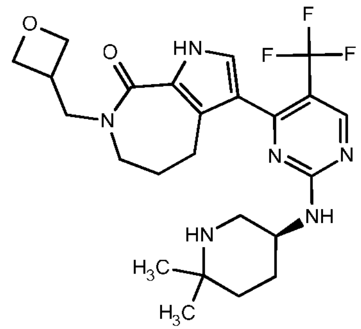 |
| **CDK7_901_a**<br>4-{2-[(piperidin-3-yl)amino]-5-(trifluoromethy l)pyrimidin-4-yl}-1,2-dihydro-quinoli n-2-one | 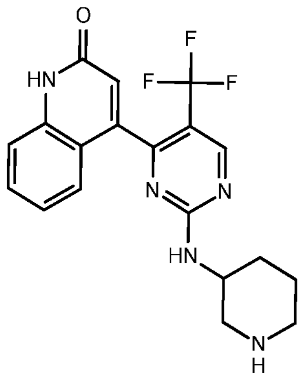 | **CDK7_1271_a**<br>3-{2-[(6,6-dimethylpiperidin -3-yl)amino]-5-(trifluoromethyl)p yrimidin-4-yl}-7-[(oxetan-3-yl) methyl]-1H,4H,5H,6H,7H, 8H-pyrrolo[2,3-c] azepin-8-one | 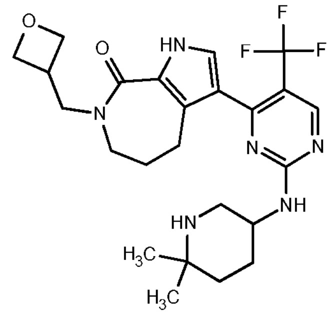 |
| **CDK7_921**<br>4-(2-{[(3S)-piperidin-3-yl]amino}-5-(trifluoromethy l)pyrimidin-4-yl)-1,2-dihy-droisoquin olin-1-one | 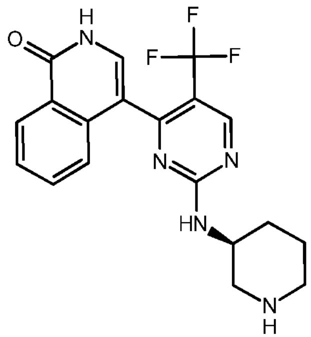 | **CDK7_1272**<br>3-(2-{[(6S)-4-azaspiro[2.5]octa n-6-yl]amino}-5-(trifluoromethyl)p yrimidin-4-yl)-7-(2-methoxyethyl)-1H,4H,5H,6H,7H, 8H-pyrrolo [2,3-c]azepin-8-one | 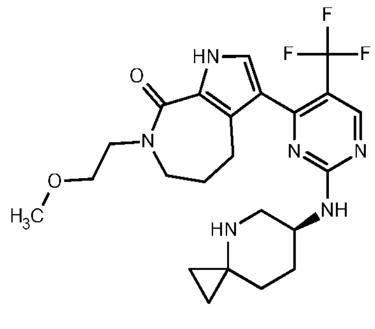 |

(continued)

| Code Name | Structure | Code Name | Structure |
|---|---|---|---|
| **CDK7_921_a** 4-{2-[(piperidin-3-yl)amino]-5-(trifluoromethy l)pyrimidin-4-yl}-1,2-dihydroisoquin olin-1-one | 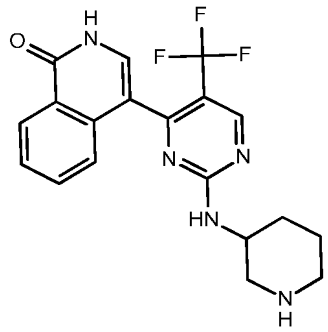 | **CDK7_1272_a** 3-[2-({4-azaspiro[2.5]octa n-6-yl}amino)-5-(trifluoromethyl)p yrimidin-4-yl]-7-(2-methoxyethyl)-1H,4H,5H,6H,7H, 8H-pyrrolo[2,3-c]azepin-8-one | 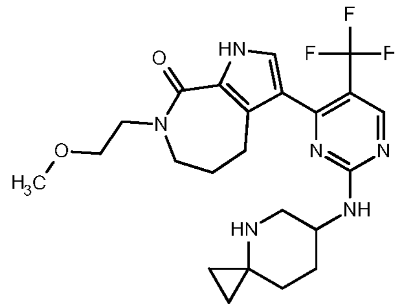 |
| **CDK7_1001** N-cyclopropyl-N-methyl-4-(2-{[(3S)-piperidin-3-yl]amino}-5-(trifluoromethy l)pyrimidin-4-yl)-1H-pyrrole-2-carboxamide | 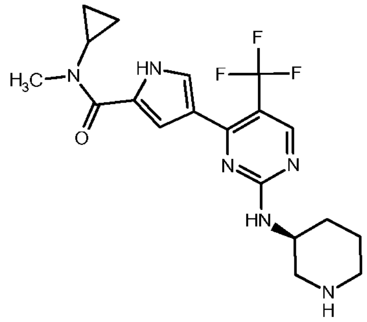 | **CDK7_1273** 2-[3-(2-{[(6S)-4-azaspiro[2.5]octa n-6-yl]amino}-5-(trifluoromethyl)p yrimidin-4-yl)-8-oxo-1H,4H,5H,6H,7H, 8H-pyrrolo[2,3-c]azepin-7-yl]acetonitrile | 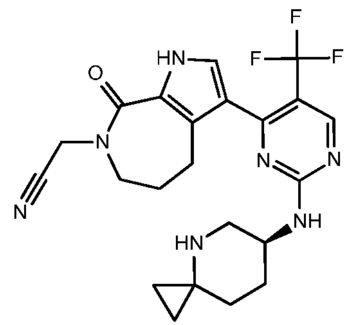 |
| **CDK7_1001_ a** N-cyclopropyl-N-methyl-4-{2-[(piperidin-3-yl)amino]-5-(trifluoromethy l)pyrimidin-4-yl}-1H-pyrrole-2-carboxamide | 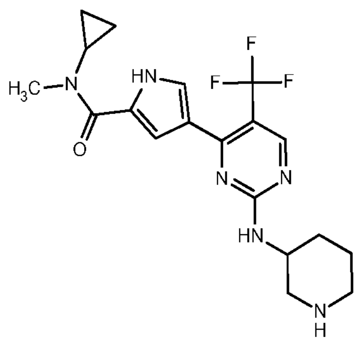 | **CDK7_1273_a** 2-{3-[2-({4-azaspiro[2.5]octa n-6-yl}amino)-5-(trifluoromethyl)p yrimidin-4-yl]-8-oxo-1H,4H,5H,6H,7H, 8H-pyrrolo[2,3-c]azepin-7-yl}acetonitrile | 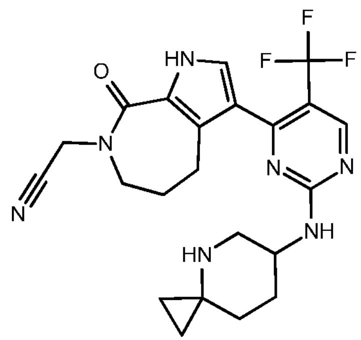 |

(continued)

| Code Name | Structure | Code Name | Structure |
|---|---|---|---|
| **CDK7_1003** N-methyl-4-(2-{[(3S)-piperidin-3-yl] amino}-5-(trifluoromethy l)pyrimidin-4-yl)-N-(1,3-thiazol-2-yl)-1H-pyrrole-2-carboxamide | 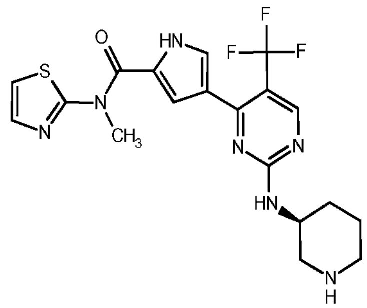 | **CDK7_1274** 3-(2-{[(6S)-4-azaspiro[2.5]octa n-6-yl]amino}-5-(trifluoromethyl)p yrimidin-4-yl)-7-(oxetan-3-yl)-1H,4H,5H,6H,7H, 8H-pyrrolo[2,3-c] azepin-8-one | 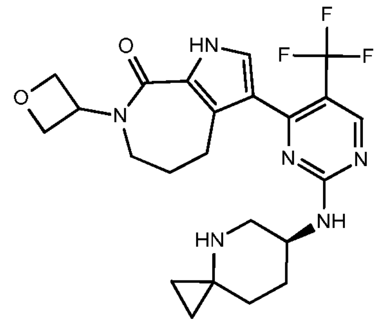 |
| **CDK7_1003_ a** N-methyl-4-{2-[(piperidin-3-yl)amino]-5-(trifluoromethy l)pyrimidin-4-yl}-N-(1,3-thiazol-2-yl)-1H-pyrrole-2-carboxamide | 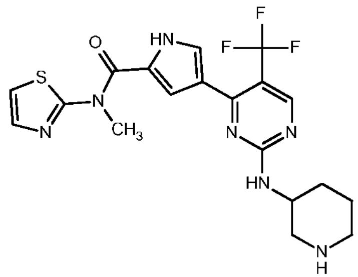 | **CDK7_1274_a** 3-[2-({4-azaspiro[2.5]octa n-6-yl}amino)-5-(trifluoromethyl)p yrimidin-4-yl]-7-(oxetan-3-yl)-1H,4H,5H,6H,7H, 8H-pyrrolo[2,3-c]azepin-8-one | 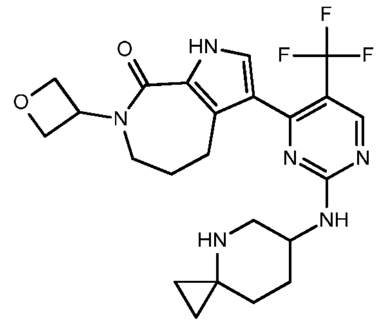 |
| **CDK7_997** N-benzyl-4-(2-{[(3S)-piperidin-3-yl] amino}-5-(trifluoromethy l)pyrimidin-4-yl)-1H-pyrrole-2-carboxamide | 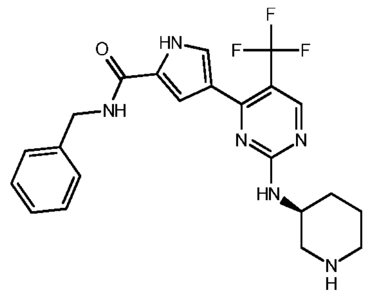 | **CDK7_1275** 3-(2-{[(6S)-4-azaspiro[2.5]octa n-6-yl]amino}-5-(trifluoromethyl)p yrimidin-4-yl)-7-[(oxetan-3-yl)methyl]-1H,4H,5H,6H,7H, 8H-pyrrolo [2,3-c]azepin-8-one | 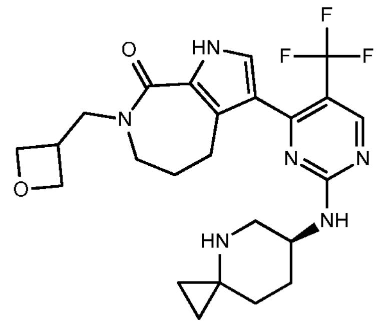 |

(continued)

| Code Name | Structure | Code Name | Structure |
|---|---|---|---|
| **CDK7_997_a** N-benzyl-4-{2-[(piperidin-3-yl)amino]-5-(trifluoromethy l)pyrimidin-4-yl}-1H-pyrrole-2-carboxamide | 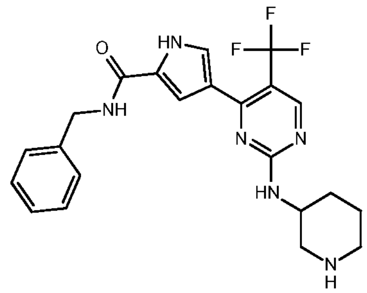 | **CDK7_1275_a** 3-[2-({4-azaspiro[2.5]octa n-6-yl}amino)-5-(trifluoromethyl)p yrimidin-4-yl]-7-[(oxetan-3-yl) methyl]-1H,4H,5H,6H,7H, 8H-pyrrolo[2,3-c] azepin-8-one | 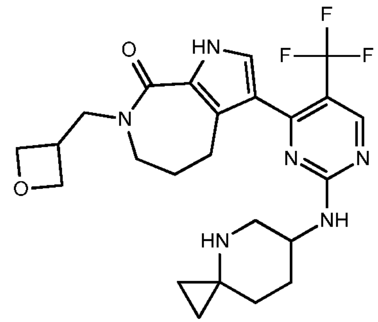 |
| **CDK7_998** N-benzyl-N-methyl-4-(2-{[(3S)-piperidin-3-yl]amino}-5-(trifluoromethy l)pyrimidin-4-yl)-1H-pyrrole-2-carboxamide | 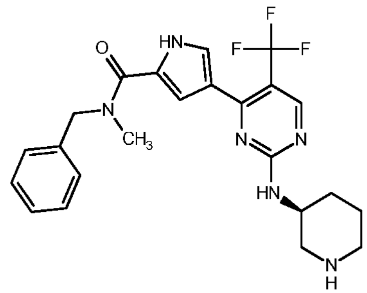 | **CDK7_1276** 3-(2-{[(6S)-4-azaspiro[2.5]octa n-6-yl]amino}-5-(trifluoromethyl)p yrimidin-4-yl)-7-cyclopropyl-1H,4H,5H,6H,7H, 8H-pyrrolo[2,3-c] azepin-8-one | 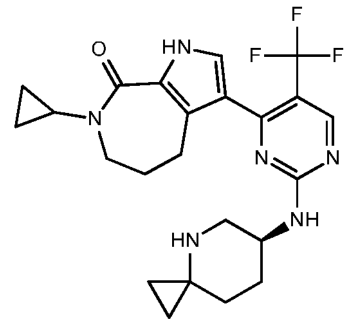 |
| **CDK7_998_a** N-benzyl-N-methyl-4-{2-[(piperidin-3-yl)amino]-5-(trifluoromethy l)pyrimidin-4-yl}-1H-pyrrole-2-carboxamide | 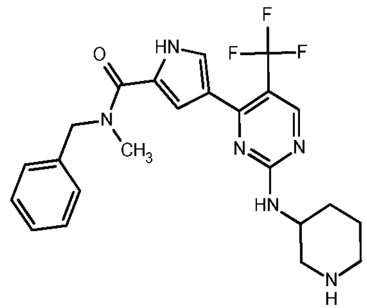 | **CDK7_1276_a** 3-[2-({4-azaspiro[2.5]octa n-6-yl}amino)-5-(trifluoromethyl)p yrimidin-4-yl]-7-cyclopropyl-1H,4H,5H,6H,7H, 8H-pyrrolo[2,3-c]azepin-8-one | 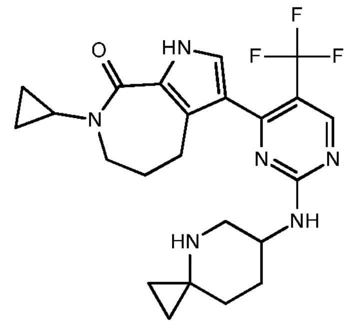 |

(continued)

| Code Name | Structure | Code Name | Structure |
|---|---|---|---|
| **CDK7_999** 4-[5-(1-methyl-1H-1,2,3,4-tetrazol-5-yl)-1H-pyrrol-3-yl]-N-[(3S)-piperidin-3-yl]-5-(trifluoromethy l)pyrimidin-2-amine | 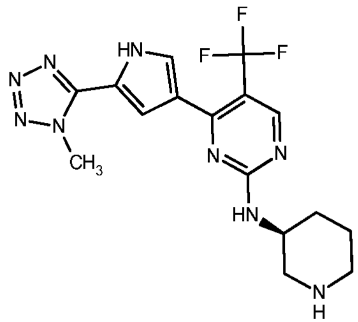 | **CDK7_1277** 3-(2-{[(6S)-4-azaspiro[2.5]octa n-6-yl]amino}-5-(trifluoromethyl)p yrimidin-4-yl)-7-(propan-2-yl)-1H,4H,5H,6H,7H, 8H-pyrrolo[2,3-c] azepin-8-one | 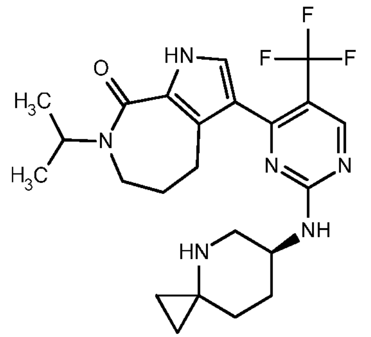 |
| **CDK7_999_a** 4-[5-(1-methyl-1H-1,2,3,4-tetrazol-5-yl)-1H-pyrrol-3-yl]-N-(piperidin-3-yl)-5-(trifluoromethy l)pyrimidin-2-amine | 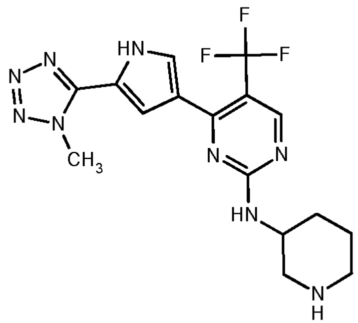 | **CDK7_1277_a** 3-[2-({4-azaspiro[2.5]octa n-6-yl}amino)-5-(trifluoromethyl)p yrimidin-4-yl]-7-(propan-2-yl)-1H,4H,5H,6H,7H, 8H-pyrrolo[2,3-c]azepin-8-one | 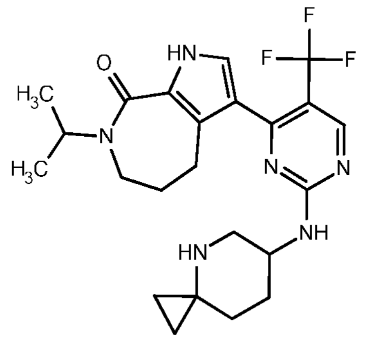 |

The present invention also relates to a method for inhibiting the biological activity of cyclin-dependent protein kinases CDK7 in a subject, consisting in contacting cyclin-dependent protein kinases CDK7 with a compound according to the present invention.

CDK7-inhibiting compounds may be used to manufacture medicinal products intended for treating any of the pathological conditions described herein, for example, compounds of formula I, pharmaceutically acceptable salts, solvates or stereoisomers will be useful in the prophylaxis or treatment of diseases or medical conditions associated, alone or partially, with CDK7 activity, for example, oncological, infectious inflammatory or non-infectious inflammatory, auto-inflammatory, autoimmune disease. Exemplary oncological disease which may be amenable to treatment using the above compounds is selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma, but not limited thereto.

Exemplary autoimmune disease is selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjogren's syndrome, systemic lupus erythematosus, sarcoidosis, ulcerative colitis, multiple sclerosis, myasthenia gravis and uveitis, but not limited thereto.

Exemplary autoinflammatory disease is selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS), but not limited thereto.

Exemplary infectious inflammatory disease or non-infectious inflammatory disease is selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis, but not limited thereto.

In one embodiment, the present invention relates to a pharmaceutical composition that comprises a therapeutically effective amount of the compound described herein, or pharmaceutically acceptable salt, solvate, or stereoisomer thereof, and one or more pharmaceutically acceptable excipients.

In one embodiment, the present invention relates to a pharmaceutical composition comprising a therapeutically effective amount of at least one of the compounds described herein, or pharmaceutically acceptable salt, solvate, stereoisomer thereof, and one or more pharmaceutically acceptable excipients.

In another one embodiment, the pharmaceutical composition of the present invention is intended to treat or

prevent a disease or disorder associated with increased activity of cyclin-dependent protein kinases CDK7. In another embodiment of the invention, the pharmaceutical composition according to the present invention is intended for the prophylaxis and treatment of a disease or disorder associated with increased activity of cyclin-dependent protein kinase CDK7, wherein the disease or disorder associated with increased activity of cyclin-dependent protein kinases CDK7 is an oncological, infectious inflammatory or non-infectious inflammatory, autoinflammatory, autoimmune disease.

In another one embodiment of the present invention, the pharmaceutical composition comprising compounds of the present invention is intended to prevent or treat

an oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjogren's syndrome, systemic lupus erythematosus, sarcoidosis, ulcerative colitis, multiple sclerosis, myasthenia gravis and uveitis;

an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);

an infectious inflammatory or non-infectious inflammatory disease selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

In another embodiment of the present invention, the pharmaceutical composition comprising the compounds according to the present invention is intended for the prophylaxis or treatment of an oncological disease being breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer.

The pharmaceutical composition of the present invention may comprise, for example, from about 5% to about 100% active ingredients by weight, from about 10% to about 100% active ingredients, from about 10% to about 60% active ingredients. It is to be understood that each dosage unit may not comprise an effective amount of an active ingredient or ingredients, because the sufficient effective amount may be achieved by administering multiple dosage unit forms.

A typical composition is prepared by mixing the compound of the present invention with a carrier, diluent or excipient. Suitable carriers, diluents and fillers are well known to those skilled in the art and include materials such as carbohydrates, waxes, water soluble and/or swellable polymers, hydrophilic or hydrophobic materials, gelatin, oils, solvents, water, and the like. The particular carrier, diluent or filler used will depend upon the means and purpose for which compound of the present invention is being applied. Solvents are generally selected based on solvents recognized by those skilled in the art as safe to be administered to a mammal. In general, safe solvents are aqueous solvents such as water and other solvents that are soluble or miscible in water. Suitable aqueous solvents include water, as the main ingredient, ethanol, propylene glycol, polyethylene glycols (e.g., PEG400, PEG300), etc. and mixtures thereof. The compositions may also include one or more buffers, stabilizing agents, surfactants, wefting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug product (i.e., compound of the present invention or pharmaceutical composition thereof) or aid in the manufacturing of the pharmaceutical product (i.e. medicinal product). The pharmaceutical compositions should preferably be manufactured in compliance with the GMP (Good Manufacturing Practice) requirements.

The pharmaceutical compositions may also include salts, solvates and hydrates of compounds of the present invention, or stabilized form of the compound (e.g., complex with a cyclodextrin derivative or other known complexation agent).

The pharmaceutical compositions of the present invention are typically suitable for oral administration. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; granules; soft or hard capsules containing multi- or nano-particulates, liquids, or powders; lozenges (including liquid-filled); chews; gels; fast dispersing dosage forms; films; ovules; sprays; and buccal/mucoadhesive patches. More preferred formulations for oral administration are tablets, granules, and capsules.

Liquid formulations include suspensions, solutions, syrups and elixirs. Such formulations may be employed as fillers in soft or hard capsules (made, for example, made of gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The pharmaceutical compositions of the invention may also be administered parenterally. As used herein, "parenteral administration" of a pharmaceutical composition includes any route of administration characterized by physical breaching of a tissue of a subject and administration of the pharmaceutical composition through the breach in the tissue, thus generally resulting in the direct administration into the blood stream, into muscle, or into an internal organ. Parenteral administration thus includes, inter alia, administration of a pharmaceutical composition by injection of the composition, by application of the composition through a surgical incision, by application of the composition through a tissue-penetrating non-surgical wound, and the like. In particular, parenteral administration is contemplated to include, inter alia, subcutaneous, intraperitoneal, intramuscular, intravenous, intraarterial, intrathecal, intraventricular, intraurethral, intracranial, intrasynovial injection or infusions; and kidney dialytic infusion techniques. Intratumoral delivery, e.g. intratumoral injection, may also be advantageous. Regional perfusion is also provided.

Formulations of pharmaceutical compositions suitable for parenteral administration typically comprise the active ingredient combined with a pharmaceutically acceptable carrier, such as sterile water or sterile isotonic saline. Such formulations may be prepared, packaged in a form suitable for bolus administration or for continuous administration. Injectable formulations may be prepared, packaged in unit dosage form, such as in ampoules or in multi-dose containers containing a preservative. Formulations for parenteral administration include, inter alia, suspensions, solutions, emulsions in oily or aqueous vehicles, pastes, and the like.

The compounds of the invention can also be administered intranasally or by inhalation, typically in the form of a dry powder (either alone, as a mixture, or as a mixed component particle, for example, mixed with a suitable pharmaceutically acceptable excipient) from a dry powder inhaler, as an aerosol pressurised container, pump, spray, atomiser (preferably an atomiser using electrohydrodynamics to produce a fine mist), or nebuliser, with or without the use of a suitable propellant, or as nasal drops.

The pressurised container, pump, spray, atomizer, or nebuliser typically contains a solution or suspension of the compound of the present invention comprising, for example, a suitable agent for dispersing, solubilising, or extending release of the active ingredient, a propellant as solvent.

Prior to use as dry powder or suspension, the medicinal product is typically micronised to a size suitable for

delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules, blisters and cartridges for use in an inhaler or insufflator may be formulated to contain a powder mix of the compound of the present invention, a suitable powder base and a performance modifier.

A suitable solution formulation for use in an atomiser using electrohydrodynamics to produce a fine mist may contain a suitable dose of the compound of the present invention per actuation and the actuation volume may, for example, vary from 1 μL to 100 μL.

Suitable flavours, such as menthol and levomenthol, or sweeteners, such as saccharin or sodium saccharin, may be added to those formulations of the present invention intended for inhaled/intranasal administration.

Formulations may be formulated to be immediate and/or modified release. Modified release formulations include delayed, sustained, pulsed, controlled, targeted and programmed release.

In one embodiment, the present invention relates to the prophylaxis or method of treatment of a disease or disorder associated with increased activity of cyclin-dependent protein kinases CDK7, which comprises administering in a therapeutically effective amount the compound of the present invention or a pharmacologically acceptable salt, solvate or stereoisomer thereof, or the pharmaceutical composition of the present invention to a subject in need of such treatment or prophylaxis.

In another embodiment, the present invention relates to the prophylaxis or method of treatment of a disease or disorder, wherein the disease or disorder associated with increased activity of cyclin-dependent protein kinases CDK7 is an oncological, infectious inflammatory or non-infectious inflammatory, autoinflammatory, autoimmune disease.

In another embodiment, the present invention relates to the prophylaxis or method of treatment of a disease or disorder, wherein the disease or disorder associated with increased activity of cyclin-dependent protein kinases CDK7 is

an oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjogren's syndrome, systemic lupus erythematosus, sarcoidosis, ulcerative colitis, multiple sclerosis, myasthenia gravis and uveitis;

an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);

an infectious inflammatory or non-infectious inflammatory disease selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis,

cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

In another embodiment, the present invention relates to the prophylaxis or method of treatment of a disease or disorder, wherein the disease or disorder associated with increased activity of cyclin-dependent protein kinases CDK7 is an oncological disease selected from a group comprising breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer.

It is meant that the compounds of the present invention may be used in the prophylaxis, methods of treatment as described above, may be used in the treatment as described above, and/or may be used in the manufacture of therapeutical products (medication) for treatment as described above.

In one embodiment, the present invention relates to the use of the compound according to the present invention or a pharmacologically acceptable salt, solvate or stereoisomer thereof, or the pharmaceutical composition described above for the treatment of a disease or disorder associated with increased activity of CDK7 in a subject in need of such treatment.

In one embodiment, the present invention relates to the use of the compound according to the present invention or a pharmaceutically acceptable salt, solvate or stereoisomer thereof, or the pharmaceutical composition described above for the treatment of a disease or disorder associated with increased activity of CDK7, wherein the disease or disorder associated with increased activity of CDK7 is an oncological, infectious inflammatory or non-infectious inflammatory, autoinflammatory, autoimmune disease.

In another one embodiment, the present invention relates to the use described above, wherein the disease or disorder associated with increased activity of CDK7 is

an oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjogren's syndrome, systemic lupus erythematosus, sarcoidosis, ulcerative colitis, multiple sclerosis, myasthenia gravis and uveitis;

an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);

an infectious inflammatory or non-infectious inflammatory disease selected from a group comprising allergies

(including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

In another embodiment, the invention relates to the above-described use, wherein the oncological disease is selected from a group comprising breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer.

The compounds of the invention may be administered alone or in combination with one or more other drug products or monoclonal antibody-based drug products (or in any combination thereof). The pharmaceutical compositions, methods and use of the invention thus also encompass embodiments of combinations (co-administration) with other active agents.

As used herein, the terms "co-administration", "co-administered" or "in combination with" referring to the compounds with one or more other therapeutic agents, are intended to mean, refer to or include the following:

- simultaneous administration to said patient of such combination of the compound of the invention and therapeutic agent to a patient in need of treatment, when such components are formulated together into a single dosage form which releases said components at substantially the same time,
- simultaneous administration of such combination of the compound of the invention and therapeutic agent to a patient in need of treatment, when such components are formulated apart from each other into separate dosage forms which are administered at substantially the same time to said patient, whereupon said components are released at substantially the same time in said patient,
- sequential administration of such combination of the compound of the invention and therapeutic agent to a patient in need of treatment, when such components are formulated apart from each other into separate dosage forms which are taken at consecutive times by said patient with a significant time interval between each administration, whereupon said components are released at different times in said patient; as well as
- sequential administration of such combination of the compound of the invention and therapeutic agent to a patient in need of treatment, when such components are formulated together into a single dosage form which releases said components in a controlled manner whereupon they are concurrently, consecutively, and/or overlappingly released at the same and/or different times to said patient, wherein each part may be administered by either the same or a different route.

As well known to those skilled in the art, therapeutically effective dosages may vary when the drug products are used in combination treatment. Methods for experimentally determining therapeutically effective dosages of drug products and other agents for use in combination treatment regimens have been described in the literature. For example, the use of metronomic dosing, i.e., providing more frequent, lower doses in order to minimize toxic side effects, has been described in the literature. Combination treatment further includes periodic treatments that start and stop at various times in accordance with the patient treatment plan. For combination therapy described in the present patent, dosages of co-administered compounds may vary depending on the type of co-drug employed, on the specific drug employed, on the condition or disorder being treated and so forth.

The antitumor treatment described above can be used either as a stand-alone therapy, or in combination with surgery, or radiotherapy, or drug therapy. Such therapy may be administered concurrently, simultaneously, sequentially or separately with the treatment of the compound according to the invention and may comprise one or more agents from the following categories of antitumor agents: antiproliferative/antitumor therapeutical products and combinations thereof used in medical oncology, such as alkylating agents, alkylated sulfonates, nitrosoureas or triazenes; antimetabolites; hormonal

agents or hormone antagonists; platinum compounds; antitumor antibiotics; topoisomerase inhibitors.

Examples of antimetabolites include, without limitation, folic acid antagonists (e.g., methotrexate, trimetrexate, pemetrexed, pralatrexate, raltitrexed, calcium levofolinate) or pyrimidine antagonists (e.g., cytarabine, tegafur, fluorouracil, capecitabine, floxouridine, azacitidine, enocitabine, karmofur, gemcitabine, sapacitabine, elacitarabine, doxifluridine), or purine antagonists (for example, mercaptopurine, thioguanine, pentostatin, fludarabine, cladribine, nelarabine, azathioprine, clofarabine), or asparaginase.

Examples of alkylating agents include, without limitation, mechlorethamine, cyclophosphamide, chlorambucil, melphalan, bendamustine, hexamethylmelamine, thiotepa, busulfan, carmustine, lomustine, laromustine, semustine, streptozocin, dacarbazine, ifosfamide, improsulfan, mitobronitol, mitolactol, nimustine, ranimustine, temozolomide, treosulfan, carboquinone, apaziquinone, fotemustine, altretamine, gluphosphamide, pipobroman, trophosfamide, uramustine, evophosphamide, VAL-083.

Examples of hormonal agents and hormone antagonists include, without limitation, hydrocortisone, dexamethasone, prednisone, prednisone, hydroxyprogesterone caproate, megestrol acetate, medroxyprogesterone acetate, diethylstilbestrol, estradiol, tamoxifen, testosterone propionate, fluoxymesterone, flutamide, leuprolide, abarelix, abiraterone, bicalutamide, buserelin, calusteron, chlorotrianizene, degarelix, dexamethasone, fluocortolone, fulvestrant, goserelin, histrelin, leuprorelin, mitotane, nafarelin, nandrolone, nilutamide, octreotide, raloxifene, thyrotropin alpha, toremifene, triptorelin, diethylstilbestrol, acolbifene, danazol, deslorelin, epitiostanol, orteronel, enzalutamide, aminoglutethimide, anastrozole, exemestane, fadrozole, letrozole, testolactone, formestane.

Examples of platinum compounds include, without limitation, cisplatin, carboplatin, oxaliplatin, eptaplatin, miriplatin hydrate, lobaplatin, nedaplatin, picoplatin, satraplatin.

Examples of antitumor antibiotics include, without limitation, doxorubicin, daunorubicin, idarubicin, carubicin, valrubicin, zorubicin, aclarubicin, pirarubicin, nemorubicin, amrubicin, epirubicin, bleomycin, dactinomycin, plicamycin, peplomycin, mitomycin-C, zinostatin, streptozotocin.

Examples of topoisomerase inhibitors include, without limitation, irinotecan, topotecan, belotecan, teniposide, etoposide, voreloxin, amonafide.

Examples of antitumor agents include, without limitation, any one of the following agents: microtubule targeting drugs, such as taxanes (e.g., paclitaxel, nab-paclitaxel, docetaxel, cabazitaxel, tesetaxel), Vinca alkaloids (e.g., vinorelbine, vinblastine, vincristine, vindesine, vinflunine); mitogen-activated protein kinases inhibitors (e.g., U0126, PD98059, PD184352, PD0325901, ARRY-142886, SB239063, SP600125, BAY 43-9006, wortmannin or LY294002); mTOR inhibitors (e.g. sirolimus, temsirolimus, everolimus, ridaforolimus); antibodies (for example, prolgolimab, rituximab, trastuzumab, alemtuzumab, besilesomab, cetuximab, denosumab, ipilimumab, bevacizumab, pertuzumab, pembrolizumab, nivolumab, cemiplimab, durvalumab, atezolizumab, avelumab, ofatumumab, panitumumab, tozitumomab, catumaxomab, elotuzumab, epratuzumab, farletuzumab, mogamulizumab, necitumumab, nimotuzumab, obinutuzumab, okaratuzumab, oregovomab, ramucirumab, rilotumumab, siltuximab, tocilizumab, zalutuzumab, zanolimumab, matuzumab, dalotuzumab, onartuzumab, rakotumomab, tabalumab, abituzumab); kinase inhibitors (fosmatanib, entospletenib, erlotinib, imatinib, lapatinib, nilotinib, pazopanib, vemurafenib, gefitinib, crizotinib, dazatinib, regorafenib, ruxolitinib, sorafenib, sunitinib, vandetanib, bozutinib, axitinib, afatinib, alisertib, dabrafenib, dacomitinib, dinaciclib, dovitinib, nintedanib, lenvatinib, linifanib, linsitinib, masitinib, motesanib, neratinib, orantinib, ponatinib, radotinib, tipifarnib, tivantinib, tivozanib, trametinib, apatinib, ibrutinib, acalabrutinib, cobimetinib, fedratinib, brivanib alaninate, cediranib, cabozantinib, icotinib, cipatinib, rigosertib, pimasertib, buparlisib, idelalisib, midostaurin, perifosine, tesevatinib); photosensitizers (e.g., talaporfin, temoporfin, porfimer sodium); cytokines (e.g., aldesleukin, interferon alfa, interferon alfa-2a, interferon alfa-2b, celmoleukin, tasonermin, recombinant interleukin-2, oprelvekin, recombinant interferon beta-1a); vaccines (e.g., picibanil, sipuleucel-T, vitespen, emepepimut-S, oncoVAX, rindopepimut, troVAX, MGN-1601, MGN-1703); bisantrene, decitabine, mitoxantrone, procarbazine, trabectedin, amsacrin, brostallicin, miltefosin, romidepsin, plitidepsin, eribulin, ixabepilone, fosbretabulin, denileukin diftitox, ibritumomab tiuxetan, prednimustine, trastuzumab emtansine, estramustin, gemtuzumab ozogamicin, aflibercept, oportuzumab monatox, cintredekin besudotox, edotreotide, inotuzumab ozogamicin, naptumomab estafenatox, vintafolide, brentuximab vedotin, bortezomib, ixazomib, carfilzomib, lenalidomide, thalidomide, pomalidomide, zoledronic acid, ibandronic acid, pamidronic acid, alitretinoin, tretinoin, peretinoin, bexarotene, tamibarotene, imiquimod, lentinan, mifamurtide, romurtide, pegaspargase, pentostatin, endostatin, sizofiran, vismodegib, vorinostat, entinostat, panobinostat, celecoxib, cilengitide, etanidazole, ganetespib, idronoxil, iniparib, lonidamine, nimorazole, procodazole, tasquinimod, telotristat, belinostat, thymalfasin, tirapazamine, tosedostat, trabedersen, ubenimex, valspodar, gendicine, reolisin, retaspimicin, trebananib, virulisin.

Thus, in another embodiment of the invention, there is described a pharmaceutical product comprising a compound of formula (I) or pharmaceutically acceptable salt, solvate or stereoisomer thereof as defined hereinbefore, in combination with an anti-tumour agent as defined hereinbefore, intended for the conjoint treatment of cancer.

Dosage regimens may be adjusted to provide the optimum desired response. For example, a single dose may be administered, several divided doses may be administered over time or the dose may be proportionally reduced or increased as indicated by the exigencies of the therapeutic situation. It is especially advantageous to formulate peroral

compositions in a unit dosage form for ease of administration and uniformity of dosage. A unit dosage form as used herein refers to physically discrete units suited as unitary dosages for patients/subjects to be treated; each unit contains a predetermined quantity of active compound calculated to produce the desired therapeutic effect in association with the desired pharmaceutical carrier. Specification for the unit dosage forms of the invention is typically dictated by and directly dependent on (a) the unique characteristics of a therapeutic agent and particular therapeutic or prophylactic effect to be achieved, and (b) the limitations inherent in the art of compounding such an active compound for the treatment of sensitivity in subjects.

Thus, a skilled artisan would appreciate, based upon the disclosure provided herein, that the doses and dosage regimen are adjusted in accordance with methods well-known in the therapeutic arts. That is, the maximum tolerable dose can be readily established, and the effective amount providing a detectable therapeutic effect to a patient may also be determined, as can the temporal requirements for administering each agent to provide a detectable therapeutic effect to a patient. Thus, while certain doses and administration regimens are exemplified herein, these examples in no way limit the doses and administration regimens that may be provided to a patient in practicing the embodiments of the invention.

It is to be noted that dosage values may vary with the type and severity of the condition to be alleviated, and may include single or multiple doses. Furthermore, it is to be understood that for any particular subject, specific dosage regimens should be adjusted over time according to the individual need and the judgment of a medical professional administering or supervising the administration of the compositions, and that dosage ranges set forth in the present description are exemplary only and are not intended to limit the scope or practice of the claimed compositions. Furthermore, the dosage regimen with the compositions of the present invention may be based on a variety of factors, including the type of disease, age, weight, sex, medical condition of the patient, the severity of the condition, the route of administration, and the particular compound of the present invention employed. Thus, the dosage regimen may vary widely, but can be determined routinely using standard methods. For example, doses may be adjusted based on pharmacokinetic and pharmacodynamic parameters, which may include clinical effects such as toxic effects or laboratory values. Thus, the present invention encompasses intra-patient dose-escalation as determined by one skilled in the art. Methods for determining appropriate dosage and regimens are well-known in the art and would be understood by a skilled artisan once provided the ideas disclosed herein.

Typically, doses used to treat an adult are usually in the range of 0.01-5000 mg per day, or from 0.1-500 mg per day or from 0.1 to 100 mg per day.

Once improvement of the patient's conditions has occurred, a maintenance dose is administered, if necessary. Subsequently, the dosage or the frequency of administration, or both, may be reduced, as a function of the symptoms, to a level at which the improved disease, disorder or condition is retained. Patients may be however required periodic treatment on a long-term basis upon any relapse of symptoms.

The foregoing ranges are merely suggestive, as the number of variables in regard to an individual treatment regime is large, and considerable excursions from these recommended values are not uncommon. These dosages may be altered depending on a number of variables, not limited to the activity of the compound used, the disorder or condition to be treated, the method of administration, the requirements of the individual subject, the severity of the disorder or condition being treated, and the judgment of the physician.

The following examples are provided for better understanding of the invention. These examples are for purposes of illustration only and are not to be construed as limiting the scope of the invention in any manner.

The compounds and processes of the present invention will be better understood in connection with the following synthetic schemes which illustrate the methods by which the compounds of the present invention can be prepared. The initial products can be obtained from commercial sources or prepared using conventional prior art methods known to one of ordinary skill in the art. One of ordinary skill in the art will also readily appreciate that the steps of selective protection and deprotection, as well as the order of these steps, may be carried out in different order, depending on the nature of the substituents, for the successful completion of the synthesis described below.

The abbreviations used in this description, including those given in the illustrative diagrams and examples that follow, are well known to one of ordinary skill in the art.

DIPEA - diisopropylethylamine;
TBAF - tetrabutylammonium fluoride;
DMF - N,N-dimethylformamide;
THF - tetrahydrofuran;
BINAP - (±)-2,2'-*bis*(diphenylphosphino)-1,1'-dinaphthalene;
MTBE - methyl *tert*-butyl ether;
TMEDA - *N,N,N',N'*-tetramethylethane-1,2-diamine;
HATU - 1-[*bis*(dimethylamino)methylene]-1*H*-1,2,3-triazolo[4,5-*b*]pyridinium 3-oxide hexafluorophosphate;
NBS - *N*-bromosuccinimide;
TEA - triethylamine;

DMAP - 4-dimethylaminopyridine;
TFA - trifluoroacetic acid;
TIPS - triisopropyl silane;
dppf - 1,1'-*bis*(diphenylphosphino)ferrocene;
ac - acetyl;
boc - *tert*-butoxycarbonyl;
pin - pinacolato;
HOBt - 1-hydroxybenzotriazole;
EDC - 1-ethyl-3-(3-dimethylaminopropyl)carbodiimide;
PPA - polyphosphoric acid;
HPLC - high performance liquid chromatography;
Ph - phenyl;
HMDS - hexamethyldisilazane.

## Examples

**Example 1.** Synthesis of compound **1.6.**

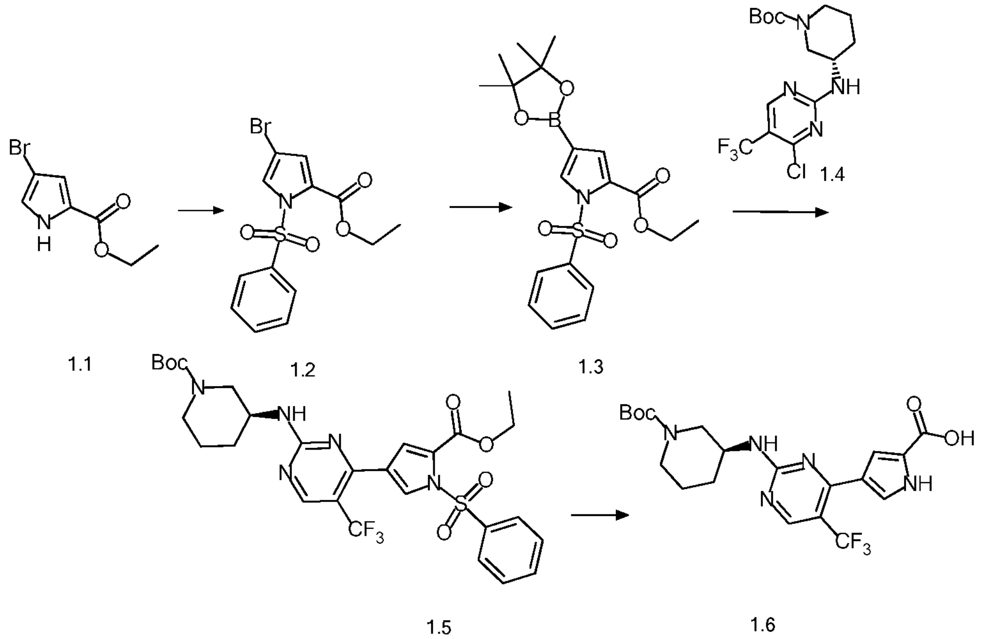

1.1 1.2 1.3 1.4 1.5 1.6

*Synthesis of compound **1.2***

TEA (4.28 g, 42.2 mmol), DMAP (0.679 g, 5.50 mmol) and benzenesulfonyl chloride (3.64 g, 21.7 mmol) were added to solution of **1.1** (4.00 g, 18.34 mmol) in 40 ml of dichloromethane. The reaction mass was stirred for 20 h at room temperature. After standard processing, the product was isolated using silica gel column chromatography. 5.63 g (86%) of compound **1.2** were yielded.

*Synthesis of compound **1.3***

Compound **1.2** (2.5 g, 6.98 mmol), $(BPin)_2$ (3.62 g, 13.96 mmol), KOAc (1.77 g, 17.45 mmol), $Pd(dppf)Cl_2$ (0.417 g, 0.56 mmol) in 50 ml of 1,4-dioxane were stirred in nitrogen atmosphere for 10 h at 100°C. After standard treatment, the reaction product was isolated using silica gel column chromatography. 2.16 g (76%) of compound **1.3** were yielded.

*Synthesis of compound **1.5***

Compound **1.3** (0.90 g, 2.0 mmol), pyrimidine **1.4** (0.692 g, 1.82 mmol), $Na_2CO_3$ (0.448 g, 4.18 mmol), Pd(dppf)

$Cl_2$ (0.136 g, 0.18 mmol) in a mixture of 18 ml of 1,4-dioxane and 6 ml of water were stirred in nitrogen atmosphere for 4 h at 100°C. After standard treatment, the product was isolated using silica gel column chromatography. 0.95 g (84%) of compound **1.5** were yielded.

*Synthesis of compound* ***1.6***

Compound **1.5** (0.90 g, 1.44 mmol) was dissolved in 9 ml of ethanol; NaOH (0.35 g, 8.64 mmol) and 9 ml of water were added. The reaction mass was stirred at 60°C for 6 h, the solvent was removed, water was added to the residue until it completely dissolved (10 ml), pH of the mixture was brought to 3 with a 10% citric acid solution. The precipitate was filtered, the residual solvents were removed in vacuo. 0.6 g (92%) of product **1.6** was yielded.

**Example 2.** Synthesis of compound **2.4.**

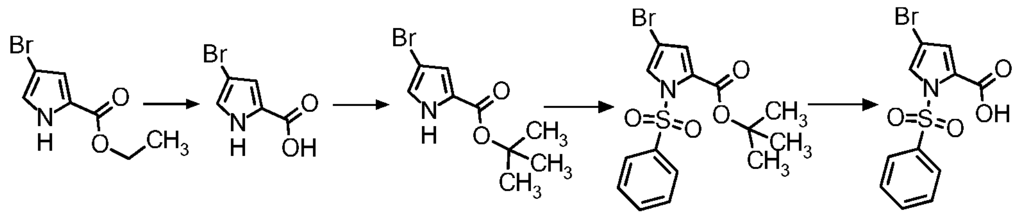

1.1 2.1 2.2 2.3 2.4

*Synthesis of compound 2.1*

Compound **1.1** (9.08 g, 41.6 mmol) was dissolved in 90 ml of ethanol; NaOH (6.73 g, 167 mmol) and 45 ml of water were added. The reaction mass was stirred at 70°C for 6 h, ethanol was removed in vacuo, water was added to the residue until it completely dissolved, pH of the mixture was brought to 3 with $HCl_{conc.}$, the precipitate was filtered off, washed with water, residual solvents were removed in vacuo. 7.1 g (90%) of compound **2.1** were yielded.

*Synthesis of compound* ***2.2***

Compound **2.1** (3.52 g, 18.5 mmol) was dissolved in 40 ml of dichloromethane; oxalyl chloride (9.41 g, 74.1 mmol) and 0.3 ml of DMF were added. The reaction mass was stirred for 2 h, volatile components were removed in vacuo, the residue was dissolved in 20 ml of dichloromethane and added while being cooled on an ice bath to a solution of tert-butanol (2.77 g, 37.1 mmol) and TEA (15.0 g, 148 mmol) in 10 ml of dichloromethane, stirred for 20 h at room temperature. After standard treatment, the product was isolated using silica gel column chromatography. 3.7 g (81%) of compound **2.2** were yielded.

*Synthesis of compound* ***2.3***

TEA (4.28 g, 42.2 mmol), DMAP (0.22 g, 1.79 mmol) and benzenesulfonyl chloride (3.24 g, 20.2 mmol) were added to a solution of **2.2** (4.5 g, 18.3 mmol) in 50 ml of dichloromethane. The reaction mass was stirred for 20 h at room temperature. After standard treatment, the product was isolated using silica gel column chromatography. 6.73 g (80%) of product **2.3** were yielded.

*Synthesis of compound* ***2.4***

TFA (1.37 g, 12.0 mmol) was added to a solution of **2.3** (0.579 g, 1.50 mmol) in 12 ml of dichloromethane, the reaction mass was stirred for 20 h at room temperature. Volatile components were removed in vacuo. 0.43 g (95%) of product **2.4** was yielded.

**Example 3.** Synthesis of compounds **CDK7_536, CDK7_537, CDK7_833, CDK7_835, CDK7_846, CDK7_847, CDK7_848, CDK7_849, CDK7_850, CDK7_851, CDK7_951, CDK7_997, CDK7_998, CDK7_1001, CDK7_1003.**

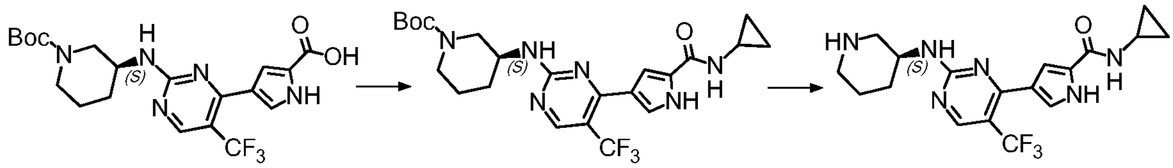

1.6 3.1 CDK7_951

*Synthesis of compound* ***3.1***

Compound **1.6** (0.065 g, 0.14 mmol), HATU (0.108 g, 0.28 mmol), cyclopropylamine (0.012 g, 0.21 mmol), DIPEA (0.073 g, 0.56 mmol) were dissolved in 0.6 ml DMF and stirred for 20 h at room temperature. After standard treatment, the product was isolated using silica gel column chromatography. 0.035 g (51%) of compound **3.1** was yielded.

*Synthesis of compound* **CDK7_951**

Compound **3.1** (0.035 g, 0.07 mmol) was dissolved in TFA (0.403 g, 3.5 mmol) and stirred at 20 °C for 20 h. TFA was removed at reduced pressure, product was isolated using preparative HPLC. 0.015 g (54%) of compound **CDK7_951** was yielded.

The following compounds were produced analogously from compound **1.6** and corresponding amines:

| Item no. | Compound | Original amine | Yield at stage 1, % | Yield at stage 2, % |
|---|---|---|---|---|
| 1 | **CDK7_536** | 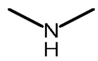 | 99 | 79 |
| 2 | **CDK7_833** | 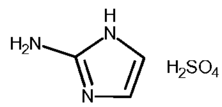 | 39 | 3 |
| 3 | **CDK7_835** | 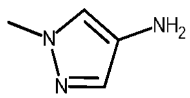 | 57 | 39 |
| 4 | **CDK7_846** | 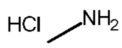 | 66 | 58 |
| 5 | **CDK7_847** | 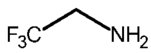 | 34 | 77 |
| 6 | **CDK7_848** | 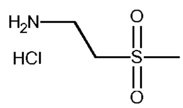 | 69 | 54 |
| 7 | **CDK7_849** | 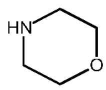 | 75 | 48 |
| 8 | **CDK7_850** | 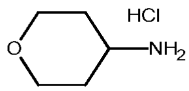 | 50 | 96 |
| 9 | **CDK7_851** | 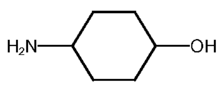 | 8 (total yield for two stages) | |
| 10 | **CDK7_537** | $NH_3$ | 69 | 36 |
| 11 | **CDK7_1001** | 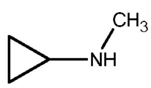 | 53 | 48 |

(continued)

| Item no. | Compound | Original amine | Yield at stage 1, % | Yield at stage 2, % |
|---|---|---|---|---|
| 12 | **CDK7_1003** | 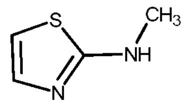 | 77 | 56 |
| 13 | **CDK7_997** | 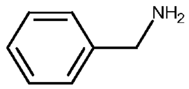 | 99 | 19 |
| 14 | **CDK7_998** | 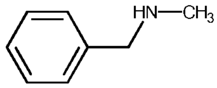 | 50 | 50 |

**Example 4.** Synthesis of compounds **CDK7_538, CDK7_539, CDK7_834, CDK7_836, CDK7_837, CDK7_838**

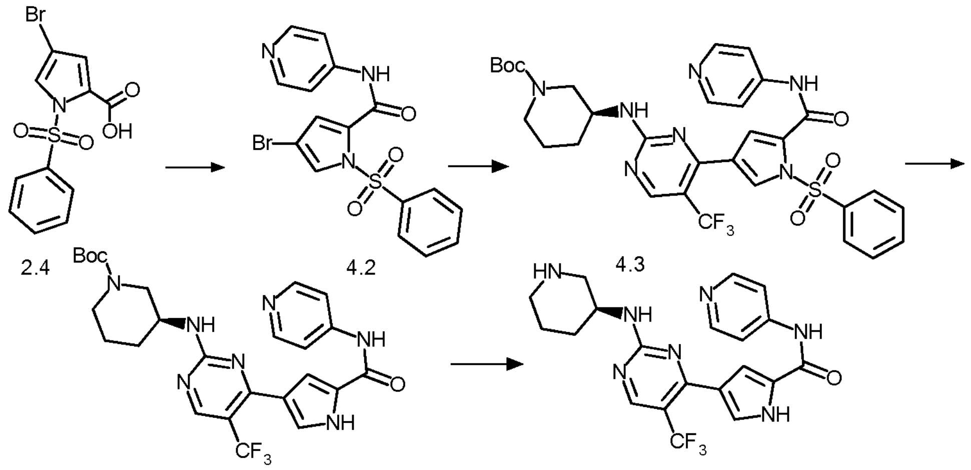

2.4 4.2 4.3

4.4 CDK7_836

*Synthesis of compound **4.2***

Compound **2.4** (0.3 g, 0.91 mmol) was dissolved in 5 ml of dichloromethane; oxalyl chloride (0.289 g, 2.28 mmol) and 0.1 ml of DMF were added. The reaction mass was stirred for 3 h. Volatile components were removed in vacuo, the residue was dissolved in 5 ml of dichloromethane and added dropwise while stirring and cooling on an ice bath to a solution of 4-aminopyridine (0.087 g, 0.91 mmol) and TEA (0.386 g, 3.64 mmol) in 5 ml of dichloromethane. The reaction mass was stirred at room temperature for 20 h. After standard treatment, the product was isolated using column chromatography on silica gel. 0.3 g (80%) of compound **4.2** was yielded.

*Synthesis of compound **4.3***

Compound **4.2** (0.10 g, 0.25 mmol), $(BPin)_2$ (0.13 g, 0.50 mmol), KOAc (0.063 g, 0.63 mmol), $Pd(dppf)Cl_2$ (0.021 g, 0.03 mmol) were dissolved in 2 ml of 1,4-dioxane and heated in a sealed vessel while stirring in nitrogen atmosphere for 6 h at 100 °C. The reaction mixture was filtered off through a Celite layer, and the solvent was removed. Compound **1.4** (0.088 g, 0.33 mmol), $Na_2CO_3$ (0.088 g, 0.82 mmol), $Pd(dppf)Cl_2$ (0.025 g, 0.03 mmol), 5 ml of 1,4-dioxane and 1.7 ml of water were added to the residue. The reaction mass was heated in a sealed vessel in nitrogen atmosphere for 3 h at 100 °C. After standard treatment, the product was isolated using column chromatography on silica gel. 0.075 g (44%) of compound **4.3** was yielded.

*Synthesis of compound* ***4.4***

An aqueous solution of NaOH (0.089 g, 2.2 mmol) was added to a solution of **4.3** (0.075 g, 0.11 mmol) in ethanol (1.5 ml) and stirred for 3 h at 50 °C. After standard treatment, the product was isolated using column chromatography on silica gel. 0.042 g (72%) of compound **4.4** was yielded.

*Synthesis of compound* ***CDK7_836***

Compound **4.4** (0.042 g, 0.08 mmol) was dissolved in 2 ml of dichloromethane; TFA (0.092 g, 0.8 mmol) was added, the reaction mass was stirred for 20 h at 20 °C, the solvent was removed. The product was isolated by HPLC. 0.012 g (34%) of compound **CDK7_836** was yielded.
The following compounds shown in the table below were produced analogously.

| N | Product | Original amine | Yield, %, stage | | | |
|---|---|---|---|---|---|---|
| | | | 1 | 2+3 | 4 | 5 |
| 1 | **CDK7_538** | 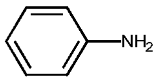 | 58 | 79 | 64 | 85 |
| 2 | **CDK7_539** | 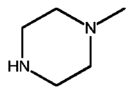 | ** | 19 | 42 | 77 |
| 3 | **CDK7_834** | 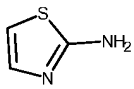 | 86 | 52 | 80 | 27 |
| 4 | **CDK7_837** | 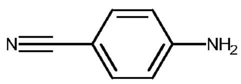 | 39 | 23 | * | 9 |
| 5 | **CDK7_838** | 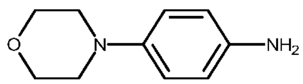 | 71 | 55 | 82 | 44 |
| *Intermediate was not isolated | | | | | | |

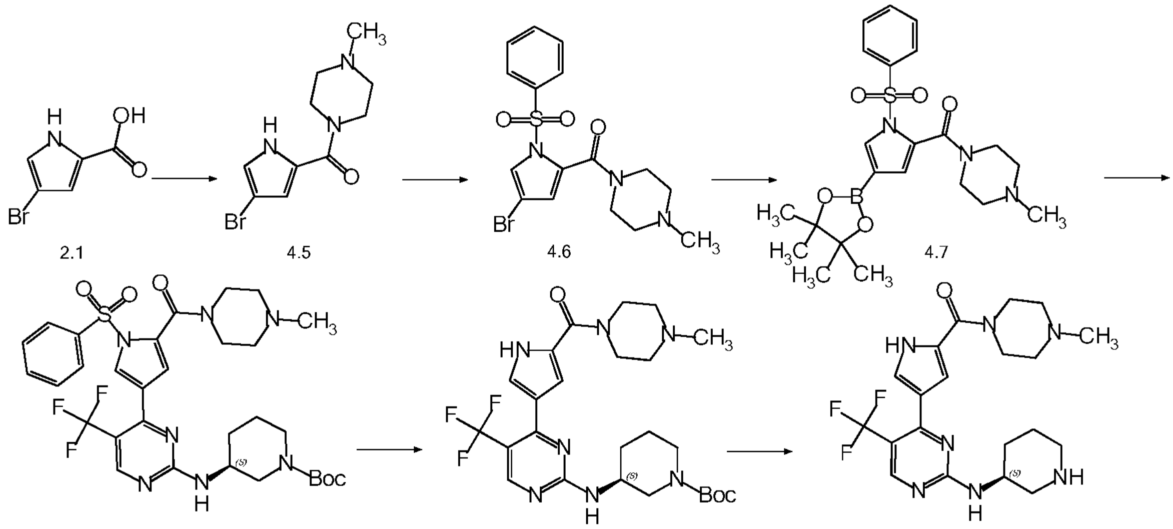

2.1 4.5 4.6 4.7

4.8 4.9 CDK7_539

*Synthesis of compound* ***4.5***

Compound **2.1** (0.6 g, 3.16 mmol), N-methylpiperazine (0.425 ml, 3.79 mmol), HATU (1.58 g, 4.11 mmol), DIPEA

(1.11 ml, 6.32 mmol) were dissolved in 6 ml of DMF and stirred at room temperature for 20 h. After standard treatment, the product was isolated using silica gel column chromatography. 0.86 g (99%) of compound **4.5** was yielded.

*Synthesis of compound **4.6***

TEA (0.769 ml, 5.52 mmol), DMAP (0.023 g, 0.18 mmol) and benzenesulfonyl chloride (0.36 g, 2.02 mmol) were added to a solution of **4.5** (0.5 g, 1.84 mmol) in 15 ml of dichloromethane. The reaction mass was stirred for 20 h at room temperature. After standard treatment, the product was isolated using silica gel column chromatography. 0.56 g (74%) of product **4.6** was yielded.

*Synthesis of compound **4.7***

Compound **4.6** (0.56 g, 1.36 mmol), $(BPin)_2$ (0.698 g, 2.72 mmol), KOAc (0.413 g, 4.08 mmol), $Pd(dppf)Cl_2$ (0.112 g, 0.14 mmol) in 12 ml of 1,4-dioxane were stirred in nitrogen atmosphere for 10 h at 100°C. After standard treatment, the reaction product was isolated using silica gel column chromatography. 0.52 g (83%) of compound **4.7** was yielded.

*Synthesis of compound **4.8***

Boronic ether **4.7** (0.149 g, 0.32 mmol), pyrimidine **1.4** (0.1 g, 0.26 mmol), $Na_2CO_3$ (0.07 g, 0.65 mmol), $Pd(dppf)Cl_2$ (0.024 g, 0.03 mmol) in 2 ml of 1,4-dioxane and 0.7 ml of water were stirred in nitrogen atmosphere for 4 h at 100°C. After standard treatment, the reaction product was isolated using column chromatography on silica gel. 0.05 g (23%) of compound **4.8** was yielded.

*Synthesis of compound **4.9***

Compound **4.8** (0.05 g, 0.07 mmol), NaOH (0.065 g, 1.61 mmol) in 0.5 ml of ethanol and 0.5 ml of $H_2O$ were stirred at 50°C for 2 h. 2 ml of 10% solution of citric acid was added, extraction was performed with ethyl acetate, and, following removal of the solvent in vacuo, 0.016 g (42%) of compound **4.9** was yielded.

*Synthesis of compound **CDK7_539***

Compound **4.9** (0.016 g, 0.03 mmol) was dissolved in 1 ml of dichloromethane, 0.2 ml of 4 H HCl solution in 1,4-dioxane was added, the reaction mass was stirred at 20 °C for 18 h, volatile components were removed in vacuo, the product was isolated by preparative HPLC. 0.010 g (77%) of compound **CDK7_539** was yielded.

**Example 5.** Synthesis of compound **CDK7_535**

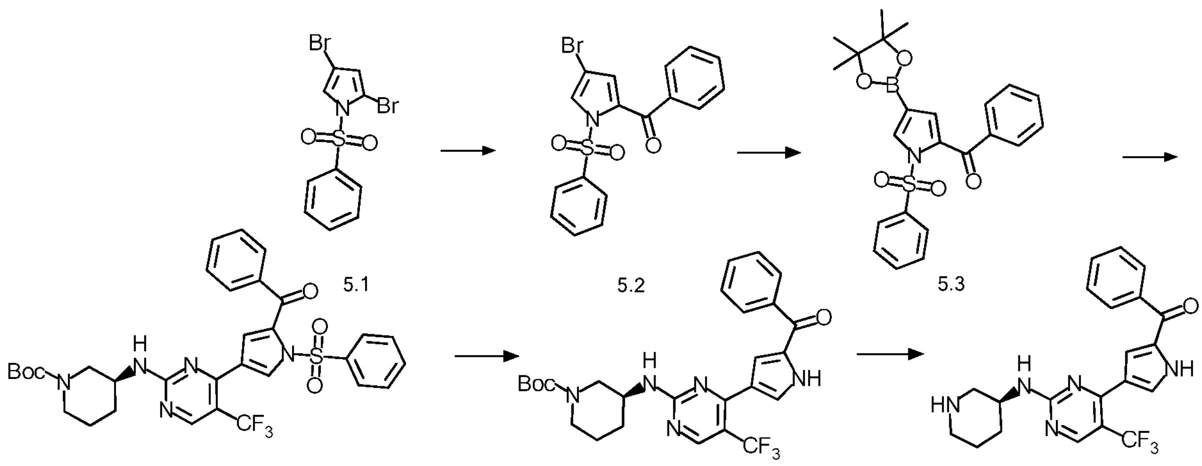

5.1 5.2 5.3

5.4 5.5 CDK7_535

*Synthesis of compound* ***5.2***

0.75 ml (1.84 mmol) 2.5 M of n-butyllithium solution in hexane was added while stirring in nitrogen atmosphere at -78 °C to a solution of compound **5.1** (0.45 g, 1.23 mmol) in 50 ml of THF, stirred for 30 min, benzoyl chloride (0.262 g, 1.84 mmol) was then added, and stirred for another 30 min. After standard treatment, the product was isolated using silica gel column chromatography. 0.3 g (62%) of compound **5.2** was yielded.

*Synthesis of compound* ***5.3***

Pyrrole **5.2** (0.126 g, 0.35 mmol), $(BPin)_2$ (0.124 g, 0.48 mmol), KOAc (0.063 g, 0.63 mmol), $Pd(dppf)Cl_2$ (0.012 g, 0.02 mmol) were dissolved in 5 ml of 1,4-dioxane, heated in a sealed vessel for 7 h at 100°C. After standard treatment, the product was isolated using column chromatography on silica gel. 0.05 g (36%) of compound **5.3** was yielded.

*Synthesis of compound* ***5.4***

Boronic ether **5.3** (0.05 g, 0.1 mmol), pyrimidine **1.4** (0.027 g, 0.07 mmol), $Na_2CO_3$ (0.021 g, 0.2 mmol), Pd(dppf) $Cl_2$ (0.007 g, 0.01 mmol) in 1.5 ml of 1,4-dioxane and 0.5 ml of water were stirred in nitrogen atmosphere for 3 h at 90 °C. After standard treatment, the reaction product was isolated using column chromatography on silica gel. 0.04 g (62%) of compound **5.4** was yielded.

*Synthesis of compound* ***5.5***

Compound **5.5** was produced analogously to compound **4.9** with a yield of 87%.

*Synthesis of compound* ***CDK7_535***

Compound **CDK7_535** was produced analogously to compound **539** with a yield of 38%.

**Example 6.** Synthesis of compounds **CDK7_845, CDK7_924, CDK7_959, CDK7_961, CDK7_1052.**

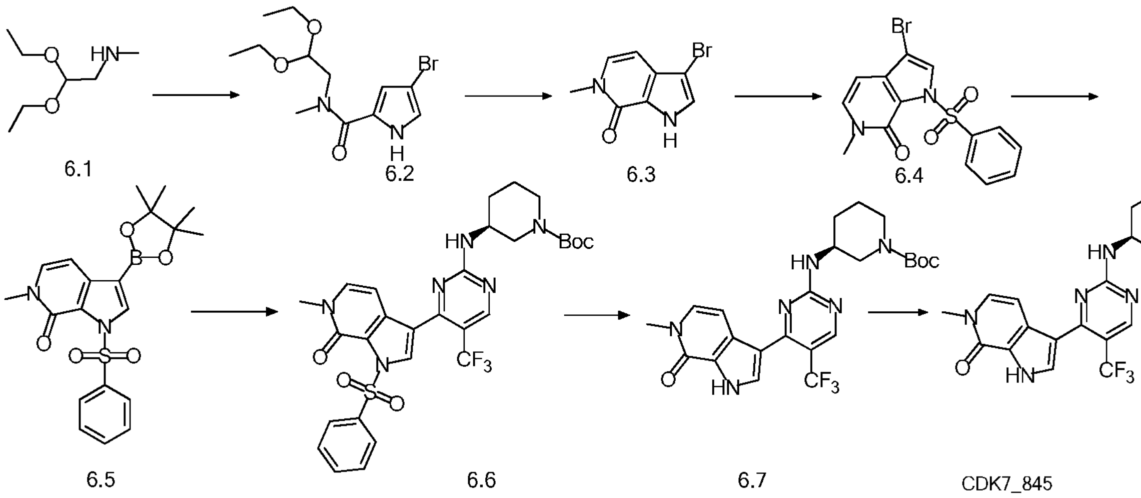

6.1 6.2 6.3 6.4

6.5 6.6 6.7 CDK7_845

*Synthesis of compound* ***6.2***

Compound was produced analogously to compound **4.2** with a yield of 93%.

*Synthesis of compound* ***6.3***

Amide **6.2** (0.29 g, 1.00 mmol) and 0.97 g of methanesulfonic acid (10 mmol) were stirred for 72 h at 20 °C, 5 ml of water was added while cooling to the reaction mass, stirred for 1 hour, the precipitate was filtered off, washed with water, residual solvents were removed in vacuo. 0.19 g (84%) of compound **6.3** was yielded.

*Synthesis of compound* ***6.4***

TEA (0.26 g, 2.60 mmol), DMAP (0.01 g, 0.08 mmol) and benzenesulfonyl chloride (0.165 g, 0.92 mmol) were added to a solution of pyrrolopyridone **6.3** (0.19 g, 0.84 mmol) in 6 ml of dichloromethane, the reaction mass was stirred for 18 h at 20 °C. After standard treatment, the product was isolated using column chromatography on silica gel. 0.26 g (73%) of compound **6.4** was yielded.

*Synthesis of compound* ***6. 5***

Compound **6.4** (0.22 g, 0.60 mmol), $(BPin)_2$ (0.31 g, 1.2 mmol), KOAc (0.153 g, 1.50 mmol), $Pd(dppf)Cl_2$ (0.045 g, 0.06 mmol) in 5 ml of 1,4-dioxane was heated while stirring in nitrogen atmosphere for 7 h at 100°C. After standard treatment, the product was isolated using column chromatography on silica gel. 0.14 g (59%) of compound **6.5** was yielded.

*Synthesis of compound* ***6. 6***

Compound **6.5** (0.54 g, 1.30 mmol), pyrimidine **1.4** (0.495 g, 1.30 mmol), $Na_2CO_3$ (0.348 g, 3.25 mmol), $Pd(dppf)Cl_2$ (0.97 g, 0.13 mmol) in a mixture of 12 ml of 1,4-dioxane and 4 ml of water were stirred in nitrogen atmosphere for 4 h at 100 °C. After standard treatment, the reaction product was isolated using column chromatography on silica gel. 0.26 g (32%) of compound **6.6** was yielded.

*Synthesis of compound* ***6.7***

The compound was produced analogously to compound **4.9** with a yield of 95%.

*Synthesis of compound* ***CDK7_845***

The compound was produced analogously to compound **CDK7_539** with a yield of 38%.

The following compounds were analogously produced:

| **N** | **Compound** | **Initial compound** | **Stage, yield, %** | | | | | |
|---|---|---|---|---|---|---|---|---|
| | | | 1 | 2 | 3 | 4+5 | 6 | 7 |
| **1** | **CDK7_959** | 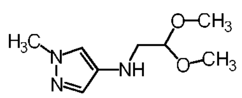 | 53 | 48 | 81 | 41 | 77 | 35 |
| **2** | **CDK7_961** | 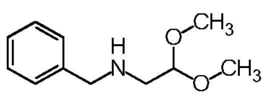 | 46 | 93 | 67 | 18 | 99 | 63 |
| **3** | **CDK7_924** | 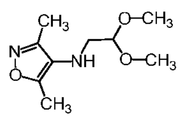 | 54 | 50 | 92 | 65 | 91 | 38 |
| **4** | **CDK7_1052** | 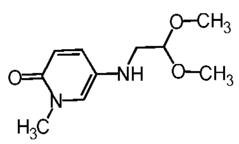 | 25 | 62 | 76 | 70 | 80 (total yield for two stages) | |

**Example 7.** Synthesis of compound **CDK7_944**

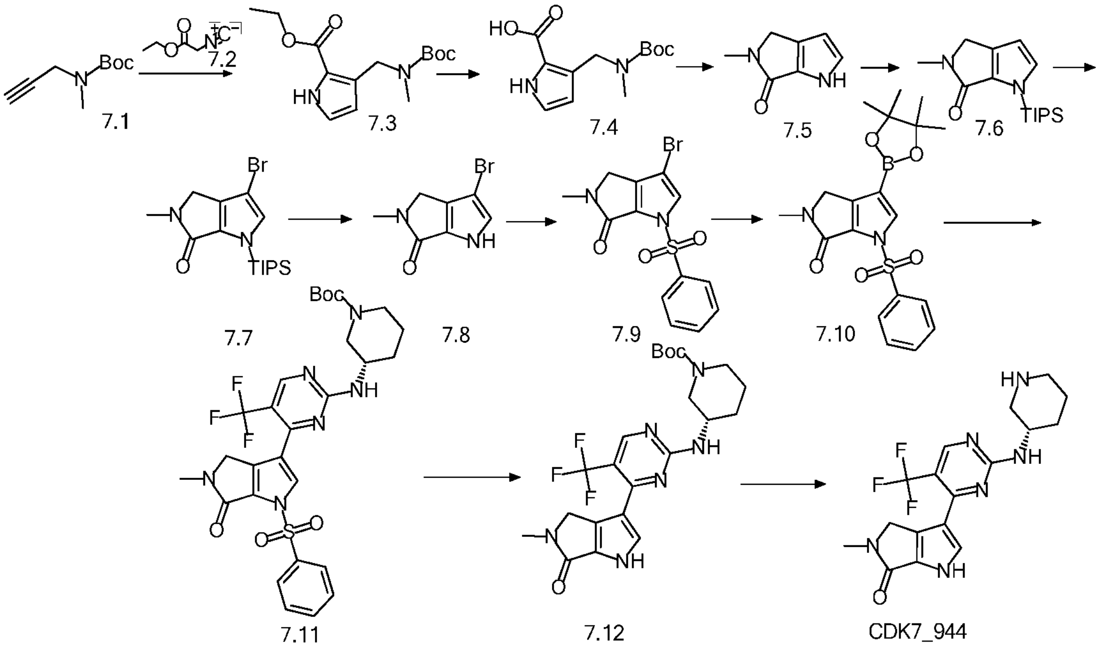

7.1 7.2 7.3 7.4 7.5 7.6 7.7 7.8 7.9 7.10 7.11 7.12 CDK7_944

*Synthesis of compound **7.3***

Compound **7.1** (2.30 g, 12.9 mmol), compound **7.2** (1.49 g, 12.9 mmol) and $Ag_2CO_3$ (1.87 g, 0.065 mmol) in 23 ml of 1,4-dioxane and stirred for 5 h at 100 °C. After standard treatment, the product was isolated using column chromatography on silica gel. 3.00 g (82%) of compound **7.3** were yielded.

*Synthesis of compound **7.4***

Compound **7.3** (3.00 g, 8.5 mmol) and LiOH (0.630 g, 25.5 mmol) in a mixture of 15 ml of water and 30 ml of methanol and stirred for 8 h at 55 °C. Methanol was removed at reduced pressure, pH of the mixture was adjusted to 2, precipitate was filtered off and residual solvents were removed in vacuo. 2.10 g (97%) of compound **7.4** were yielded.

*Synthesis of compound **7.5***

Compound **7.4** (1.070 g, 4.0 mmol), $SOCl_2$ (0.980 g, 8.0 mmol) and DMF (0.015 g, 0.2 mmol) in 10 ml of dichloromethane and stirred for 2 h at 40 °C. 5 ml of 4 N solution of HCl in 1,4-dioxane was added to the mixture and stirred for 16 h at room temperature. After standard treatment, the product was isolated using silica gel column chromatography. 0.540 g (99%) of compound **7.5** was yielded.

*Synthesis of compound **7.6***

Compound **7.5** (0.30 g, 2.09 mmol) was dissolved in 30 ml of THF, added at 0 °C to the resulting solution of NaH (0.125 g, 3.13 mmol), and the mixture was stirred for 30 minutes at 0 °C. TIPSCl (0.540 g, 2.72 mmol) was then added dropwise and stirred for 30 min at room temperature. After standard treatment, the product was isolated using silica gel column chromatography. 0.611 g (99%) of compound **7.6** was yielded.

*Synthesis of compound **7.7***

Compound **7.6** (0.611 g, 1.98 mmol) was dissolved in 20 ml of THF, a solution of NBS (0.356 g, 1.98 mmol) was added at -78 °C in 10 ml of THF, and the mixture was stirred for 30 minutes at - 78 °C and for 30 minutes at room temperature. After standard treatment, the product was isolated using silica gel column chromatography. 0.735 g (100%) of compound **7.7** was yielded.

*Synthesis of compound **7.8***

Compound **7.7** (0.625 g, 1.60 mmol) and 30% TBAF solution in tetrahydrofuran (1.59 g, 1.76 mmol) were mixed in

20 ml of THF. The mixture was stirred for 30 minutes at 25 °C. After standard treatment, the product was isolated using column chromatography on silica gel. 0.240 g (70%) of compound **7.8** was yielded.

*Synthesis of compound **7.9***

Compound **7.8** (0.240 g, 1.06 mmol) was dissolved in 30 ml of THF; NaH (0.064 g, 1.59 mmol) was added at 0 °C to the resulting solution and the mixture was stirred for 30 minutes, benzenesulfonyl chloride (0.227 g, 1.27 mmol) was added and stirred for 30 minutes at room temperature. After standard treatment, the product was isolated using silica gel column chromatography. 0.31 g (82%) of compound **7.9** was yielded.

*Synthesis of compound **7.10***

Compound **7.10** was produced analogously to compound **1.3** with a yield of 80%.

*Synthesis of compound **7.11***

Compound **7.11** was produced analogously to compound **1.5** with a yield of 65%.

*Synthesis of compound **7.12***

Compound **7.12** was produced analogously to compound **1.6** with a yield of 97%.

*Synthesis of compound **CDK7_944***

Compound **CDK7_944** was produced analogously to compound **CDK7_951** with a yield of 60%.

**Example 8.** Synthesis of compound **CDK7_929.**

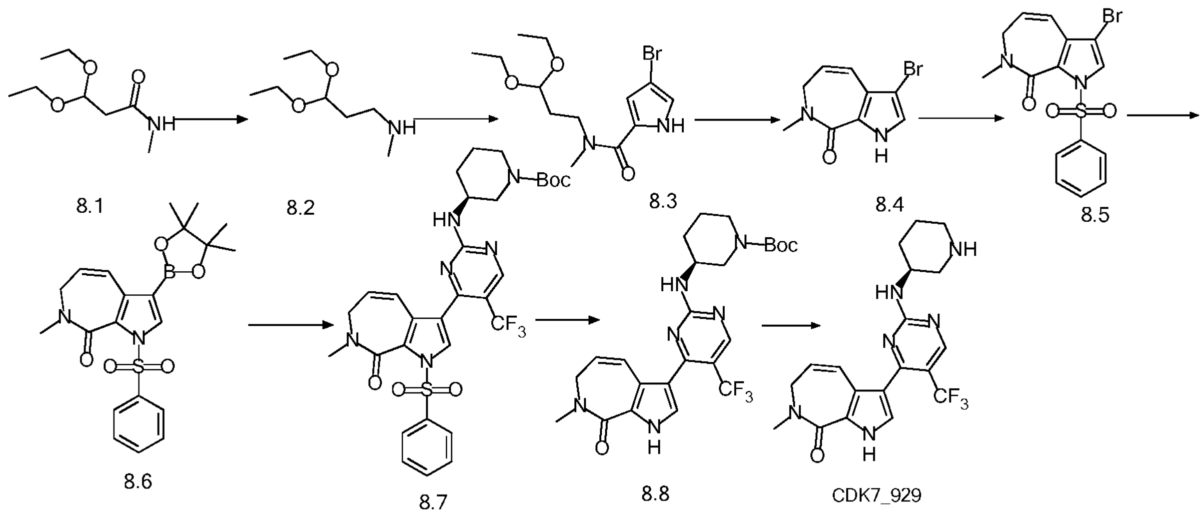

8.1 8.2 8.3 8.4 8.5

8.6 8.7 8.8 CDK7_929

*Synthesis of compound **8.2***

A solution of 4.6 g (0.026 mol) of compound 8.1 in 50 ml of THF was slowly added to a suspension of 2.00 g (0.053 mol) of LiAlH4 in 100 ml of THF while stirring and cooling. The reaction mass was stirred for 5 h at 100 °C, cooled to 0 °C, and 50 ml of water was added. After standard processing, the product was isolated using silica gel column chromatography. 0.5 g (12%) of compound **8.2** was yielded.

*Synthesis of compound **8.3***

Compound **8.3** was produced analogously to compound **6.2** with a yield of 70%.

*Synthesis of compound **8.4***

Compound **8.4** was produced analogously to compound **6.3** with a yield of 13%.

*Synthesis of compound **8.5***

Compound **8.5** was produced analogously to compound **6.4** with a yield of 56%.

*Synthesis of compound **8.6***

Compound **8.6** was produced analogously to compound **6.5** with a yield of 64%.

*Synthesis of compound **8.7***

Compound **8.7** was produced analogously to compound **6.6** with a yield of 31%.

*Synthesis of compound **8.8***

Compound **8.8** was produced analogously to compound **6.7** with a yield of 95%.

*Synthesis of compound **CDK7_929***

Compound **CDK7_929** was produced analogously to compound **CDK7_845** with a yield of 90%.

**Example 9.** Synthesis of compound **CDK7_966**

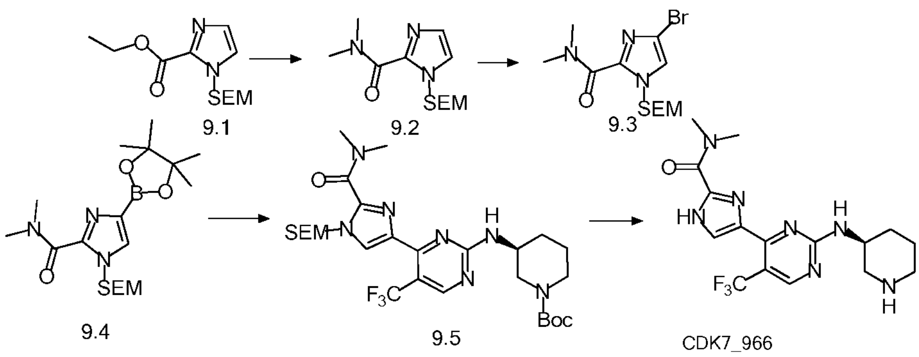
9.1
9.2
9.3
9.4
9.5
CDK7_966

*Synthesis of compound **9.2***

Compound **9.1** (2.4 g, 8.87 mmol) was dissolved in 30 ml of methanol. 50 ml (44.5 g, 0.395 mmol) of a 40% aqueous solution of dimethylamine was added. The reaction mass was stirred for 24 h, the solvent was removed, the product was isolated using column chromatography on silica gel. 0.5 g (21%) of compound **9.2** was yielded.

*Synthesis of compound **9.3***

Compound **9.2** (0.40 g, 1.48 mmol) was dissolved in 10 ml of dichloromethane, a solution of NBS (0.38 g, 2.13 mol) in 10 ml of dichloromethane was added. The reaction mass was stirred for 1 h. After standard treatment, the product was isolated using column chromatography on silica gel. 0.16 g (31%) of compound **9.3** was yielded.

*Synthesis of compound **9.4***

The compound **9.3** (0.05 g, 0.14 mmol), $(BPin)_2$ (0.047 g, 0.18 mmol), KOAc (0.042 g, 0.42 mmol), $Pd(dppf)Cl_2$ (0.01 g, 0.01 mmol) was dissolved in 1 ml of 1,4-dioxane, heated in a sealed vessel for 1 h at 100° C. The reaction mixture was filtered off through Celite and the solvent was removed in vacuo. 0.055 g (99%) of product in the form of brown oil was

yielded.

*Synthesis of compound **9.5***

Compound **9.5** was produced analogously to compound **1.5** with a yield of 93%.

*Synthesis of compound **CDK7_966***

Compound **CDK7_966** was produced analogously to compound **CDK7_951** with a yield of 69%.

**Example 10.** Synthesis of compound **CDK7_972**

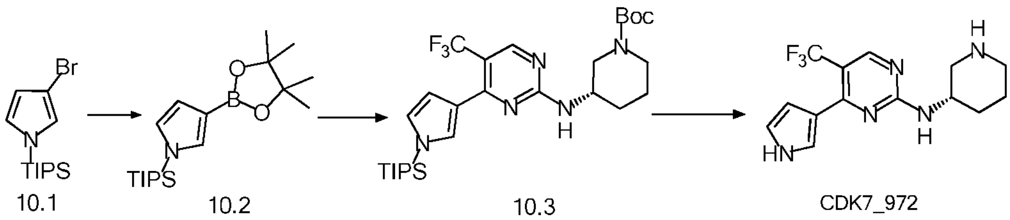

10.1 10.2 10.3 CDK7_972

*Synthesis of compound **10.2***

Compound **10.2** was derived from compound **10.1** analogously to compound **1.3** with a yield of 98%.

*Synthesis of compound **10.3***

Compound **10.3** was produced analogously to compound **1.5** with a yield of 3%.

*Synthesis of compound **CDK7_972***

Compound **CDK7_972** was produced analogously to compound **CDK7_951** with a yield of 33%.

**Example 11.** Synthesis of compound **CDK7_921**

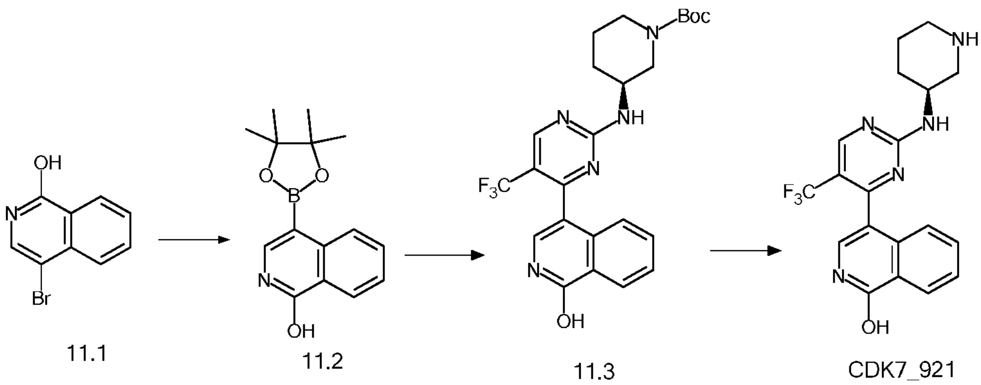

11.1 11.2 11.3 CDK7_921

*Synthesis of compound **11.2***

Compound **11.2** was produced analogously to compound **1.3** with a yield of 62%.

*Synthesis of compound **11.3***

Compound **11.3** was produced analogously to compound **1.5** with a yield of 34%.

*Synthesis of compound **CDK7_921***

Compound **CDK7_921** was produced analogously to compound **CDK7_951** with a yield of 34%.

**Example 12.** Synthesis of compound **CDK7_901**

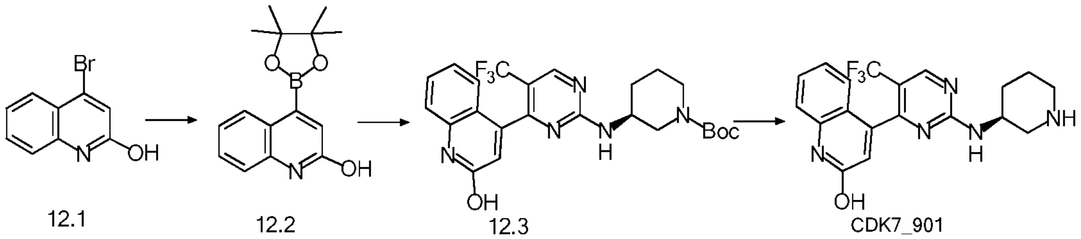

12.1 12.2 12.3 CDK7_901

*Synthesis of compound **12.2***

Compound **12.2** was produced analogously to compound **1.3** with a yield of 87%.

*Synthesis of compound **12.3***

Compound **12.3** was produced analogously to compound **1.5** with a yield of 46%.

*Synthesis of compound **CDK7_901***

Compound **CDK7_901** was produced analogously to compound **CDK7_951** with a yield of 89%.

**Example 13.** Synthesis of compound **CDK7_766.**

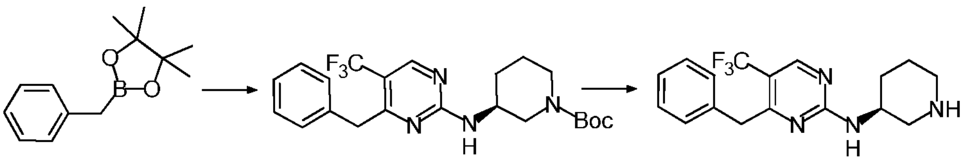

13.1 13.2 CDK7_766

*Synthesis of compound **13.2***

Boronic ether **13.1** (0.04 g, 0.17 mmol), pyrimidine **1.4** (0.059 g, 0.15 mmol), $Pd(OAc)_2$ (0.003 g, 0.015 mmol), $PPh_3$ (0.008 g, 0.03 mmol), $Na_2CO_3$ (0.05 g, 0.46 mmol) in 0.37 ml of 1.4-dioxane and 0.04 ml of water were stirred at 100 °C in nitrogen atmosphere for 8 h. After standard treatment, the product was isolated using silica gel column chromatography. 0.046 g (68%) of compound **13.2** was yielded.

*Synthesis of compound **CDK7_766***

Compound **13.2** (0.025 g, 0.05 mmol) was dissolved in 3 ml of dichloromethane, 0.2 ml of TFA was added, stirred for 18 h at 20 °C, the solvent was removed in vacuo, the product was isolated by preparative HPLC. 0.008 g (40%) of compound **CDK7_766** was yielded.

**Example 14.** Synthesis of compound Synthesis of compounds **CDK7_809, CDK7_810, CDK7_811, CDK7_829, CDK7_830, CDK7_897**

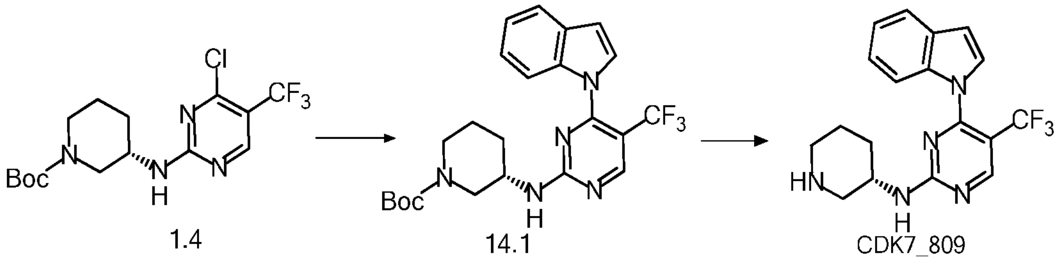

1.4 14.1 CDK7_809

*Synthesis of compound **14.1***

*Method a:*

NaH (0.014 g, 0.302 mmol) was added to a solution of indole (0.031 g, 0.276 mmol) in 2 ml of DMF, stirred for 30 min, a solution of pyrimidine **1.4** (0.100 g, 0.263 mmol) in 1 ml of DMF was added, the reaction mass was stirred for 20 h, a saturated solution of $NaHCO_3$ was added, extraction was performed with ethyl acetate. The product was isolated using column chromatography on silica gel using a mixture of hexane-ethyl acetate (92:8) as an eluent. 0.081 mg (67%) of compound **14.1** was yielded.

*Method b* is analogous to method A, $K_2CO_3$ was used instead of NaH, and MeCN was used instead of DMF; instead of stirring at room temperature, the reaction mixture was heated while boiling until the initial reagents disappeared.

*Synthesis of compound **CDK7_809***

*Method a:*

Compound **14.1** (0.063 g, 0.137 mmol) was dissolved in 1.5 ml of 7.65 M solution of HCl in 1,4-dioxane. The reaction mass was stirred for 18 h at 20 °C. The solvent was removed in vacuo, the residue was treated with ether, the precipitate was filtered off and the residual solvents were removed in vacuo. 0.014 g (24%) of compound **CDK7_809** in the form of hydrochloride was yielded.
*Method b* is analogous to method A, TFA was used instead of 7.65 M solution of HCl in 1,4-dioxane, and dichloromethane was used as a solvent.

The following compounds were produced analogously:

| Item no. | Compound | Initial compound | Reaction conditions, yield at stage 1, % | Reaction conditions, yield at stage 2, % |
|---|---|---|---|---|
| 1 | **CDK7_810** | 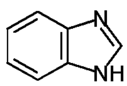 | **a,** 75% | **b,** 52**%** |
| 2 | **CDK7_811** | 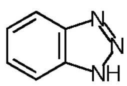 | **a,** 43% | **a,** 79% |
| 3 | **CDK7_829** | 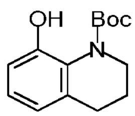 | **b,** 90% | **b,** 78% |
| 4 | **CDK7_830** | 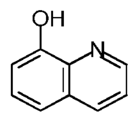 | **a,** 178% | **b,** 67% |

(continued)

| Item no. | Compound | Initial compound | Reaction conditions, yield at stage 1, % | Reaction conditions, yield at stage 2, % |
|---|---|---|---|---|
| 5 | **CDK7_897** | 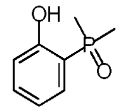 | **b,** 92% | **b**, 43% |

**Example 15.** Synthesis of compounds **CDK7_548, CDK7_550, CDK7_735, CDK7_754, CDK7_888, CDK7_891, CDK7_898, CDK7_900, CDK7_903, CDK7_904**

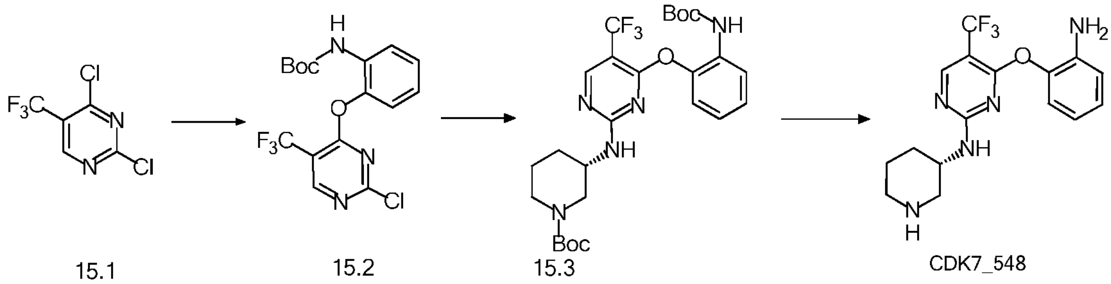

15.1 15.2 15.3 CDK7_548

*Synthesis of compound **15.2***

DIPEA (0.214 g, 1.66 mmol) was added to a solution of tert-butyl N-(2-hydroxyphenyl)carbamate of compound (0.289 g, 1.38 mmol) in 3 ml of absolute ethanol. A solution of compound **15.1** (0.300 g, 1.38 mmol) in 2 ml of absolute ethanol was added to the resulting mixture at 0°C. The mixture was stirred at 0°C for 30 minutes, then at room temperature for 16 h. After standard treatment, the product was isolated using silica gel column chromatography. Product **15.2** (0.45 g, 84%) was yielded.

*Synthesis of compound **15.3***

Compound **15.2** (0.445 g, 1.14 mmol), *tert*-butyl-3(*S*)-3-aminopiperidine carboxylate (0.229 g, 1.14 mmol) and DIPEA (0.221 mg, 1.71 mmol) in 2 ml of *i*-PrOH were stirred at room temperature for 16 h. After standard processing, the product was isolated using silica gel column chromatography, and then by preparative TLC. 0.057 g (9%) of compound **15.3 was yielded.**

*Synthesis of compound **CDK7_548***

1 ml of 7.65 M solution of HCl in 1,4-dioxane was added to a solution of compound **15.3** (0.057 g, 0.107 mmol) in 1 ml of ethanol. The mixture was stirred at room temperature for 12 h, volatile components were removed, the residue was recrystallized from a mixture of MTBE:acetone:i-PrOH (4:1:1). 0.015 g (yield 31%) of compound **CDK7_548** in the form of hydrochloride was yielded.

The following compounds were produced analogously:

| Item no. | Compound | Initial compound | Reaction conditions, yield at stage 1, % | Reaction conditions, yield at stage 2, % | Reaction conditions, yield at stage 3, % |
|---|---|---|---|---|---|
| 1 | **CDK7_550** | 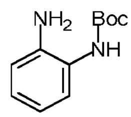 | **a**, 16% | **b**, 98% | **a,** 83% |

(continued)

| Item no. | Compound | Initial compound | Reaction conditions, yield at stage 1, % | Reaction conditions, yield at stage 2, % | Reaction conditions, yield at stage 3, % |
|---|---|---|---|---|---|
| 2 | **CDK7_735** | 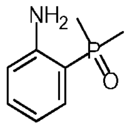 | **a**, 21% | **b**, 98% | **a,** 92% |
| 3 | **CDK7_754** | 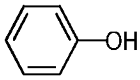 | **a**, 25% | **a**, 98% | **a,** 32% |
| 4 | **CDK7_888** | 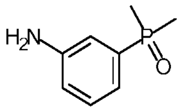 | **a**, 78% | **b**, 66% | **b**, 47% |
| 5 | **CDK7_891** | 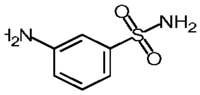 | **a**, 68% | **c,** 39% | **b**, 9% |
| 6 | **CDK7_898** | 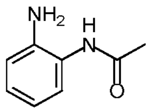 | **a**, 37% | **b,** 98% | **b,** 62% |
| 7 | **CDK7_900** | 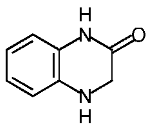 | **a**, 27% | **b**, 43% | **b**, 43% |
| 8 | **CDK7_903** | 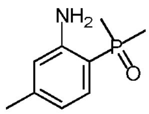 | **b**, 9% | **d,** 94% | **b**, 86% |
| 9 | **CDK7_904** | 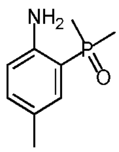 | **b**, 45% | **d**, 97% | **b**, 51% |

Reaction conditions:

Stage 1 **a:** DIPEA, EtOH, r. **t.;b:** DIPEA, MeCN, r. t. or 40 °C
Stage 2 **a: a:** DIPEA, *i*-PrOH, r.t.; **b:** DIPEA, *i*-PrOH, boiling; **c:** DIPEA, MeCN, r. t.; **d:** DIPEA, MeCN, boiling.
Stage 3: **a:** HCl, 1,4-dioxane; **b:** TFA, dichloromethane.

**Example 16.** Preparation of compound **CDK7_991**

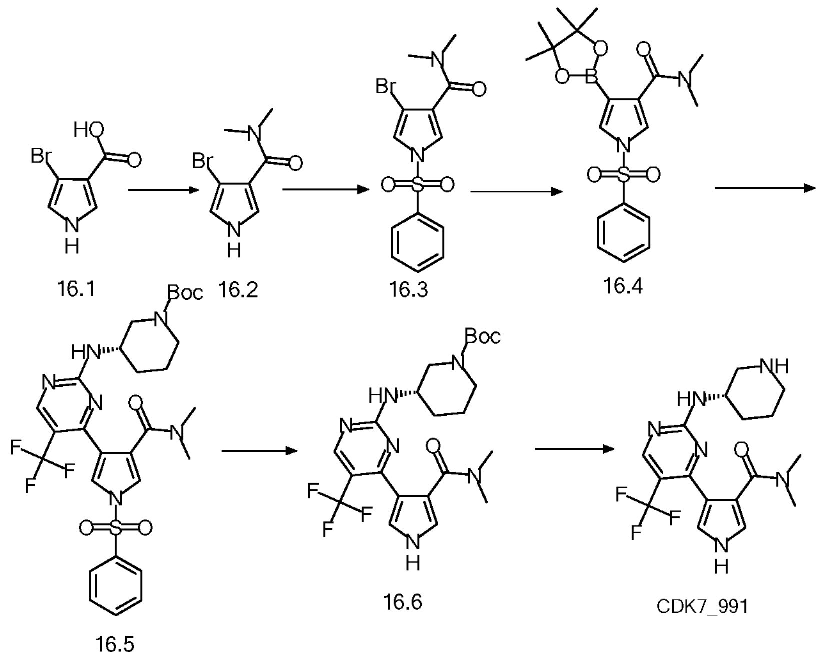

16.1 16.2 16.3 16.4

16.5 16.6 CDK7_991

*Synthesis of compound **16.2***

It was produced analogously to compound **4.2** from compound **16.1.** Yield: 64%.

*Synthesis of compound **16.3***

Compound **16.3** was produced analogously to compound **1.2** with a yield of 92%.

*Synthesis of compound **16.4***

Compound **16.4** was produced analogously to compound **1.3** with a yield of 38%.

*Synthesis of compound **16.5***

Compound **16.5** was produced analogously to compound **1.5** with a yield of 32%.

*Synthesis of compound **16.6***

Compound **16.6** was produced analogously to compound **4.5** with a yield of 99%.

*Synthesis of compound **CDK7_991***

Compound **CDK7_991** was produced analogously to compound **CDK7_951** with a yield of 26%.

**Example 17.** Preparation of compound **CDK7_1017**

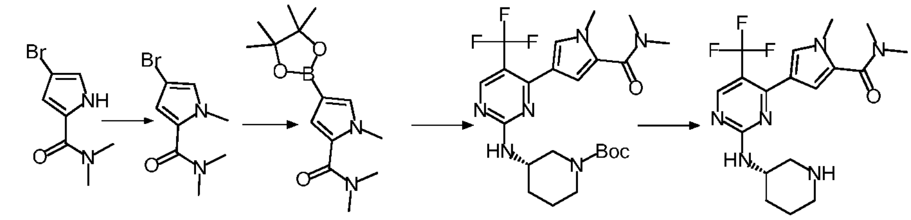

17.1 17.2 17.3 CDK7_1017

*Synthesis of compound **17.1***

NaH (60% suspension in paraffin oil) (0.087 g, 2.17 mmol) at 0 °C was added to a solution of 4-bromo-N,N-dimethyl-1*H*-pyrrol-2-carboxamide (0.332 g, 1.45 mmol) in 15 ml of THF, the reaction mass was stirred for 30 min, $CH_3I$ (0.270 g, 1.88 mmol) was added. The reaction mixture was heated to room temperature and stirred for 24 h. After standard treatment, the product was isolated using silica gel column chromatography. 0.33 g (99%) of compound **17.1** was yielded.

*Synthesis of compound **17.2***

Compound **17.2** was produced analogously to compound **1.3** with a yield of 98%.

*Synthesis of compound **17.3***

Compound **17.3** was produced analogously to compound **1.5** with a yield of 40%.

*Synthesis of compound **CDK7_1017***

Compound **CDK7_1017** was produced analogously to compound **CDK7_951** with a yield of 10%.

**Example 18.** Synthesis of compound **CDK7_953.**

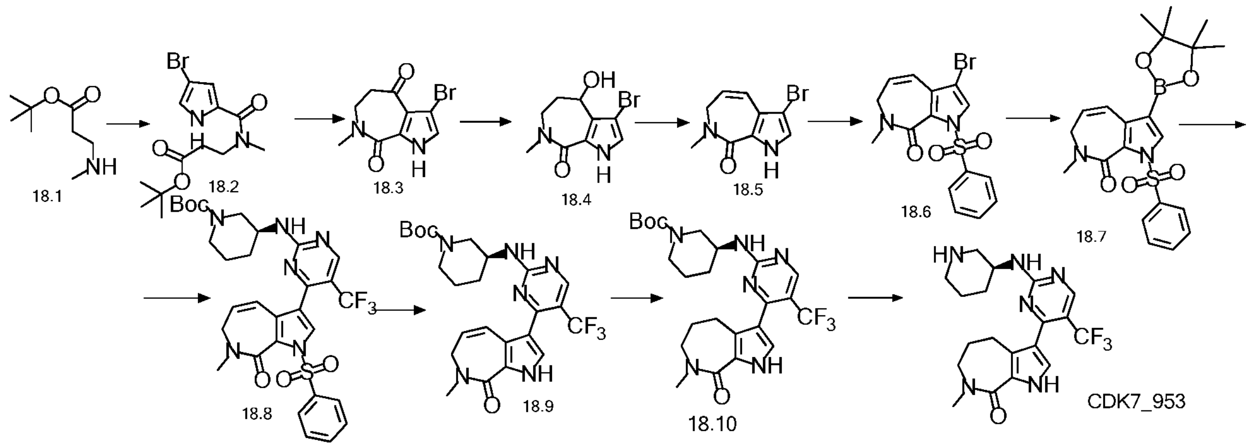

18.1 18.2 18.3 18.4 18.5 18.6 18.7 18.8 18.9 18.10 CDK7_953

*Synthesis of compound **18.2***

Compound **18.2** was produced analogously to compound **8.3** from acid **2.1** and amine **18.1** with a yield of 71%

*Synthesis of compound **18.3***

TFA (4.17 g, 36.2 mmol) was added to a solution of amide **18.2** (0.60 g, 1.81 mmol) in 5 ml of dichloromethane, the reaction mass was stirred for 18 h at 20 °C, volatile components were removed in vacuo, polyphosphoric acid (PPA) (9.34 g, 36.2 mmol) and phosphoric anhydride (1.05 g, 3.62 mmol) were added to the residue, heated while stirring at 110 °C for 3 h, 10 g of ice was added, stirred for 2 h, precipitate was filtered off, washed with water, residual solvents were removed in

vacuo. 0.32 g (69%) of compound **18.3** was yielded.

*Synthesis of compound **18.4***

$NaBH_4$ (0.053 g, 1.32 mmol) was added while cooling on an ice bath to a solution of compound **18.3** (0.100 g, 0.33 mmol) in 3 ml of ethanol, the reaction mass was stirred for 1 hour at the same temperature, then for 72 h at 20 °C. After standard treatment, the product was isolated using column chromatography on silica gel. 0.076 g (88%) of compound **18.4** was yielded.

*Synthesis of compound **18.5***

A 4 N solution of HCl in 1,4-dioxane (0.19 ml, 0.76 mmol) was added to a solution of compound **18.4** (0.22 g, 0.76 mmol) in 11 ml of chloroform, the reaction mass was stirred for 3 h. After standard treatment, the product was isolated using silica gel column chromatography. 0.14 g (77%) of product **18.5** was yielded.

*Synthesis of compound **18.6***

TEA (0.52 g, 5.00 mmol), DMAP (0.024 g, 0.2 mmol) and benzenesulfonyl chloride (0.21 g, 1.18 mmol) were added to a solution of compound **18.5** (0.14 g, 0.49 mmol) in 6 ml of dichloromethane, the reaction mass was stirred for 40 h at 20 °C. After standard treatment, the product was isolated using column chromatography on silica gel. 0.15 g (78%) of product **18.6** was yielded.

*Synthesis of compound **18.7***

Compound **18.7** was synthesized analogously to **6.5** from 0.15 g of compound **18.6.** Following borylation, the reaction mass was filtered off through a silica gel layer, the solvent was removed, yield was 0.54 g of the product in the form of a dark red oil comprising 30% of the target compound **18.7,** the product was used at the next stage without additional purification.

*Synthesis of compound **18.8***

Compound **18.7** (0.54 g, 0.38 mmol), pyrimidine **1.4** (0.111 g, 0.29 mmol), $Na_2CO_3$ (0.094 g, 0.88 mmol), Pd(dppf)$Cl_2$ (0.022 g, 0.03 mmol) in a mixture of 6 ml of 1,4-dioxane and 2 ml of water were mixed in nitrogen atmosphere for 3 h at 100 °C. After standard treatment, the product was isolated using column chromatography on silica gel. 0.081 g (43%) of compound **18.8** was yielded.

*Synthesis of compound **18.9***

Compound **18.9** was produced analogously to compound **6.7** from 0.081 g of compound **18.8** with a yield of 91%.

*Synthesis of compound **18.10***

Pd/C 5% (0.25 g, 0.12 mmol) was added to a solution of compound **18.9** (0.06 g, 0.12 mmol) in 6 ml of methanol. Hydrogenation was performed at atmospheric pressure for 3 h, the reaction mass was filtered off through a Celite layer, the solvent was removed in vacuo. 0.033 g (54%) of compound **18.10** was yielded.

*Synthesis of compound **CDK7_953***

TFA (0.346 g, 3 mmol) was added to a solution of compound **18.10** (0.033 g, 0.06 mmol) in 2 ml of dichloromethane, the reaction mass was stirred at 20 °C for 18 h, volatile components were removed in vacuo, the product was isolated using preparative HPLC. 0.009 g (36%) of compound **CDK7_953** was yielded.

**Example 19.** Synthesis of compound **19.6.**

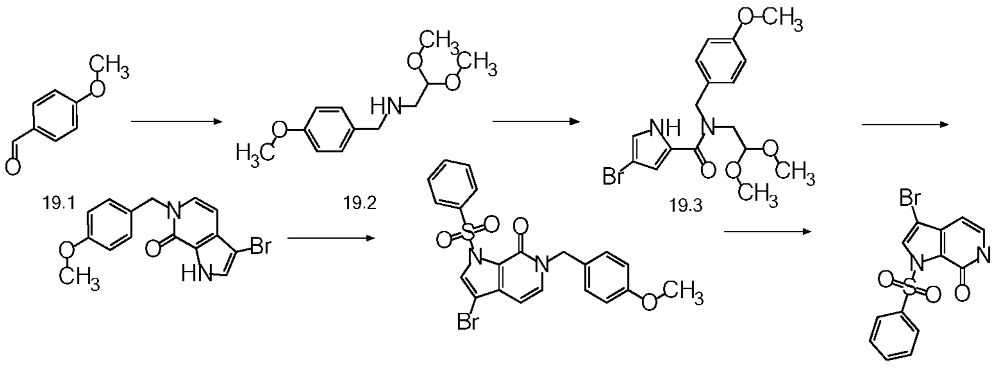

19.1 19.2 19.3 19.4 19.5 19.6

*Synthesis of compound **19.2***

Aldehyde **19.1** (5.00 g, 34.9 mmol), 2,2-dimethoxyethane-1-amine (3.87 ml, 34.9 mmol), sodium triacetoxyborohydride (23.24 g, 105 mmol) were suspended in 150 ml of dichloromethane, stirred at room temperature for 24 h. After standard treatment, the product was isolated using silica gel column chromatography. 6.31 g (80%) of compound **19.2** were yielded.

*Synthesis of compound **19.3***

Oxalyl chloride (0.451 ml, 5.26 mmol) and a drop of DMF were added to a suspension of 4-bromo-1H-pyrrol-2-carboxylic acid (0.5 g, 2.63 mmol) in 5 ml of dichloromethane, stirred for 2 h at room temperature, volatile components were removed in vacuo, added to a solution of compound **19.2** (0.592 g, 2.63 mmol) and TEA in 5 ml of dichloromethane at 0 °C, stirred for 1 h. After standard treatment, the product was isolated using silica gel column chromatography. 1.00 g (95%) of compound **19.3** was yielded.

*Synthesis of compound **19.4***

Compound **19.3** (2.68 g, 6.41 mmol) was dissolved in 15 ml of TFA, stirred for 6 days. Volatile components were removed in vacuo and the product was isolated using column chromatography. 1.78 g (83%) of compound **19.4** were yielded.

*Synthesis of compound **19.5***

Compound **19.5** was produced analogously to compound **6.4** with a yield of 83%.

*Synthesis of compound **19.6***

Compound **19.5** (0.440 g, 0.880 mmol) was dissolved in 2 ml of TFA in a sealed vessel, heated while stirring at 100 °C for 30 h. Volatile components were removed, the product was isolated using column chromatography. 0.22 g (71%) of compound **19.6** was yielded.

**Example 20.** Synthesis of compound **CDK7_1105**

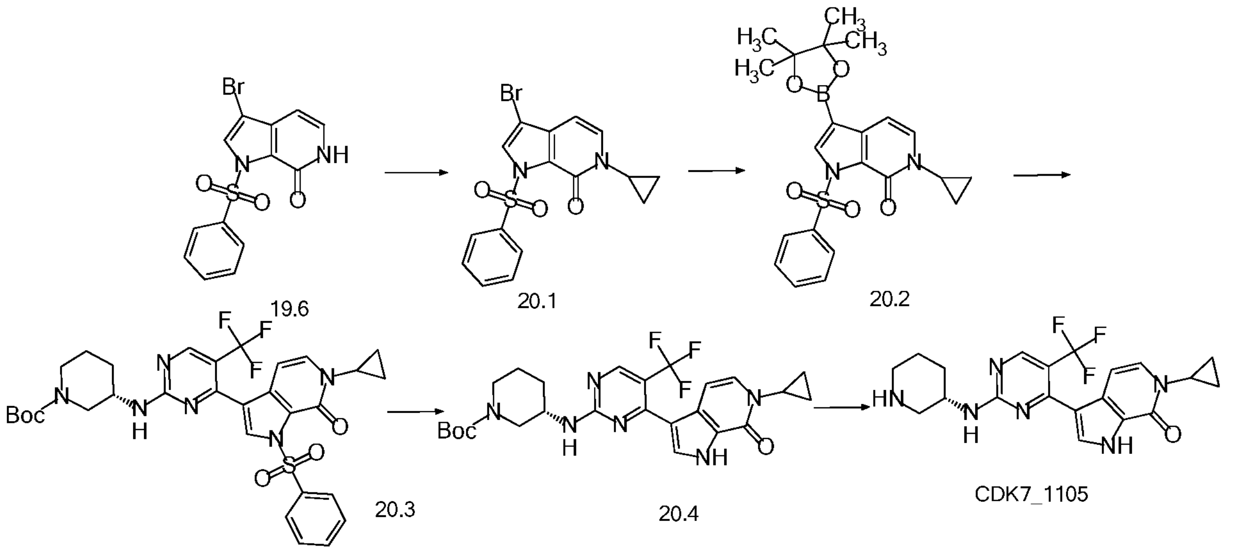

19.6 20.1 20.2

20.3 20.4 CDK7_1105

*Synthesis of compound* ***20.1***

Compound **19.6** (0.040 g, 0.110 mmol), cyclopropylboronic acid (0.029 g, 0.330 mmol), pyridine (0.018 ml, 0.220 mmol), triethylamine (0.081 ml, 0.550 mmol) in 4 ml of tetrahydrofuran was heated in a microwave reactor for 1 hour at 150 °C. After standard treatment, the product was isolated using column chromatography on silica gel. 0.042 g (98%) of compound **20.1** was yielded.

*Synthesis of compound* ***20.2***

Compound **20.1** (0.042 g, 0.100 mmol), $(BPin)_2$ (0.039 g, 0.150 mmol), KOAc (0.051 g, 0.500 mmol), $Pd(dppf)Cl_2$ (0.015 g, 0.020 mmol) was dissolved in 2 ml of 1,4-dioxane, heated in a sealed vessel for 1 h at 100° C. The reaction mixture was filtered off through Celite and the solvent was removed in vacuo. 0.04 g (91%) of product in the form of brown oil was yielded.

*Synthesis of compound* ***20.3***

Compound **20.2** (0.04 g, 0.090 mmol), pyrimidine **1.4** (0.034 g, 0.090 mmol), $Na_2CO_3$ (0.019 g, 0.180 mmol), $Pd(dppf)Cl_2$ (0.010 g, 0.010 mmol) in a mixture of 2 ml of 1,4-dioxane and 0.5 ml of water were stirred in nitrogen atmosphere for 1 hour at 100 °C. After standard treatment, the product was isolated using column chromatography on silica gel. 0.026 g (44%) of compound **20.3** was yielded.

*Synthesis of compound* ***20.4***

Compound **20.3** (0.026 g, 0.04 mmol), NaOH (0.105 g, 2.60 mmol) in 5 ml of methanol and 0.5 ml of water were stirred at room temperature for 3 h, filtered off through Celite, the solvent was removed in vacuo. 0.02 g (95%) of compound **20.4** was yielded.

*Synthesis of compound* ***CDK7_1105***

Compound **20.4** (0.020 g, 0.04 mmol) was dissolved in 2 ml of TFA, the reaction mass was stirred at 20 °C for 18 h, volatile components were removed in vacuo, re-evaporated with dichloromethane, volatile components were removed in vacuo, the product was isolated using preparative HPLC. 0.017 g (47%) of compound **CDK7_1105** was yielded.

**Example 21.** Synthesis of compound **CDK7_1062.**

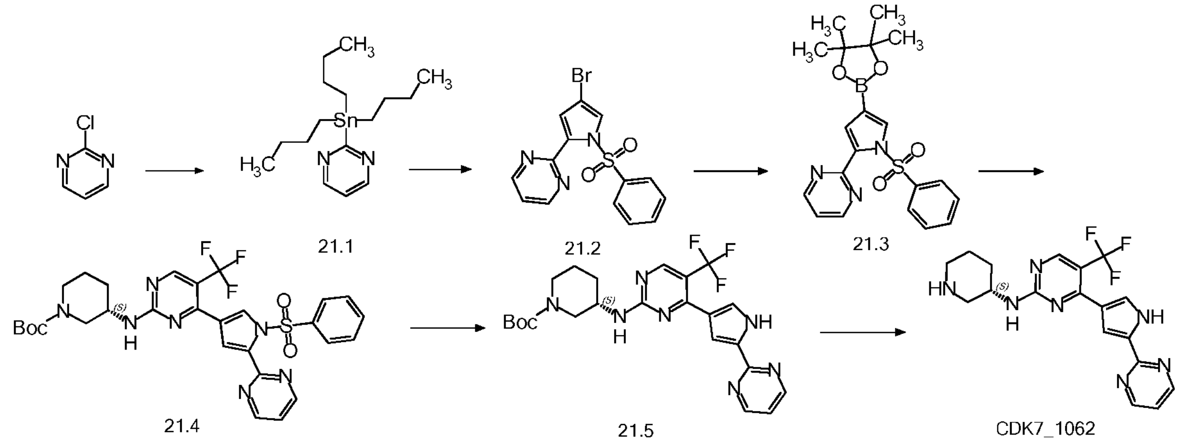

21.1

21.2

21.3

21.4

21.5

CDK7_1062

*Synthesis of compound* ***21.1***

2.23 ml (5.56 mmol) of 2.5M solution of n-butyllithium in hexane was added while stirring in nitrogen atmosphere at -78 °C to a solution of 2-chloropyrimidine (0.5 g, 4.28 mmol) in 10 ml of THF, stirred for 30 minutes, tributyltin hydride (1.42 ml, 5.14 mmol) was then added and stirred for another 30 minutes, temperature was adjusted to room temperature, stirred for 12 h at room temperature. After standard treatment, the product was isolated using silica gel column chromatography. 0.55 g (35%) of compound **21.1** was yielded.

*Synthesis of compound* ***21.2***

Compound **21.1** (0.089 g, 0.240 mmol), 1-benzenesulfonyl-4-bromo-2-iodo-1*H*-pyrrol (0.1 g, 0.240 mmol), copper(I) iodide (0.014 g, 0.070 mmol), $Pd(PPh_3)_4$ (0.028 g, 0.020 mmol) in 20.1 ml of DMF were heated in a sealed vessel in an inert atmosphere for 4 h at 120 °C. After standard treatment, the product was isolated using column chromatography on silica gel. 0.035 g (40%) of compound **21.2** was yielded.

*Synthesis of compound* ***21.3***

Compound **21.2** (0.051 g, 0.140 mmol), $(BPin)_2$ (0.073 g, 0.280 mmol), KOAc (0.035 g, 0.350 mmol), $Pd(dppf)Cl_2$ (0.010 g, 0.010 mmol) was dissolved in 2 ml of 1,4-dioxane, heated in a sealed vessel for 10 h at 110 °C, the reaction mixture was filtered off through Celite and the solvent was removed in vacuo. 0.058 g (99%) of compound **21.3** in the form of brown oil was yielded.

*Synthesis of compound* ***21.4***

Compound **21.3** (0.058 g, 0.140 mmol), pyrimidine **1.4** (0.053 g, 0.140 mmol), $Na_2CO_3$ (0.019 g, 0.018 mmol), $Pd(dppf)Cl_2$ (0.031 g, 0.04 mmol) in a mixture of 2 ml of 1,4-dioxane and 1 ml of water were stirred in nitrogen atmosphere for 2 h at 110 °C. After standard treatment, the product was isolated using column chromatography on silica gel. 0.044 g (50%) of compound **21.4** was yielded.

*Synthesis of compound* ***21.5***

Compound **21.5** was produced analogously to compound **6.7** with a yield of 99%.

*Synthesis of compound* ***CDK7_1062***

Compound **CDK7_1062** was produced analogously to compound **6.7** with a yield of 37%.

**Example 22.** Synthesis of compound **CDK7_1058**

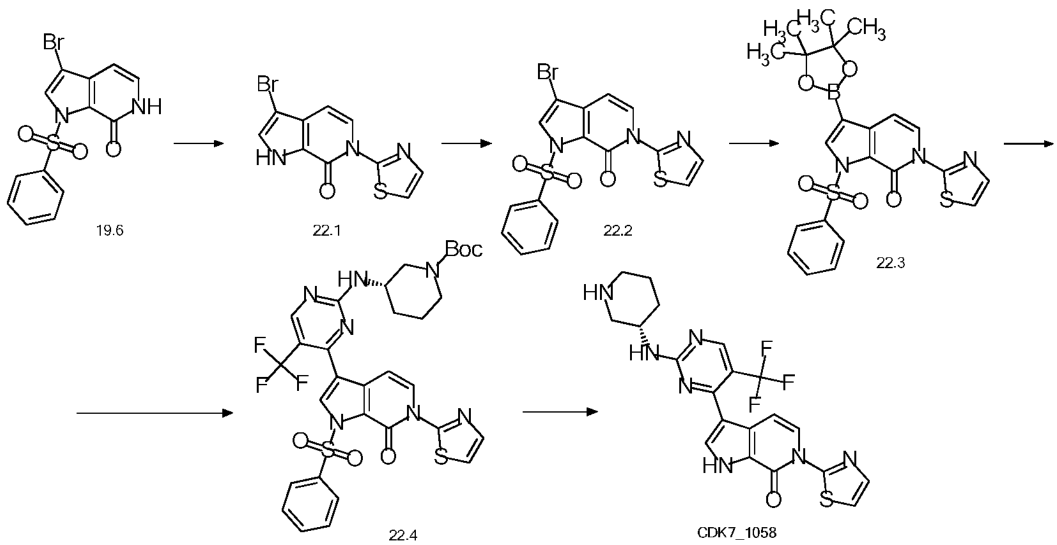

19.6 22.1 22.2 22.3 22.4 CDK7_1058

*Synthesis of compound* ***22.2***

Compound **19.6** (0.12 g, 0.34 mmol), 2-bromothiazole (0.1 g, 0.6 mmol), CuI (0.06 g, 0.3 mmol), $K_2CO_3$ (0.09 g, 0.65 mmol) in 2 ml of DMF were heated while stirring in a sealed vessel at 130 °C for 2 h, cooled to room temperature, 3M solution of NaOH was added to pH 8, stirred at room temperature for 2 h, the solvent was removed in vacuo, the product was isolated using column chromatography. 0.1 g of a mixture in the form of oil with a content of compound **22.1** of 60% according to HPLC (0.06 g, 60%) was yielded. 0.056 g (0.32 mmol) of benzenesulfonyl chloride, 0.008 g (0.06 mmol) of DMAP, 0.064 g (0.64 mmol) of triethylamine and 5 ml of dichloromethane were added to this mixture. The reaction mixture was stirred at room temperature for 18 h. After standard treatment, the product was isolated using silica gel column chromatography. 0.015 g (16%) of compound **22.2** was yielded.

*Synthesis of compound* ***CDK7_1058***

0.015 g (0.21 mmol) of compound **22.2,** $(BPin)_2$ (0.012 g, 0.05 mmol), KOAc (0.01 g, 0.1 mmol), $Pd(dppf)Cl_2$ (0.01 g, 0.01 mmol) and 1 ml of 1,4-dioxane were mixed, the mixture was heated in a sealed vessel for 1 h at 100 °C. After cooling, the reaction mixture was filtered off through Celite, and the solvent was removed in vacuo. Pyrimidine **1.4** (0.011 g, 0.03 mmol), $Na_2CO_3$ (0.006 g, 0.06 mmol), $Pd(dppf)Cl_2$ (0.01 g, 0.01 mmol) in a mixture of 1 ml of 1,4-dioxane and 0.5 ml of water were added to the residue comprising **22.3.** The resulting mixture was stirred in nitrogen atmosphere for 2 h at 110 °C, the reaction mixture was filtered off through celite, and the solvent was removed in vacuo. The residue comprising compound **22.4** was dissolved in 1.5 ml of ethanol, NaOH (0.12 g, 3.00 mmol) and 1 ml of water were added. The reaction mass was stirred at 60 °C for 6 h, solvents were removed in vacuo, water was added to the residue until completely dissolved (1 ml), acidified with 10% solution of citric acid to pH 4, extraction was performed with ethyl acetate, volatile components were removed in vacuo. The residue was dissolved in 1 ml of TFA, the reaction mass was stirred at 20 °C for 18 h, the volatile components were removed in vacuo, the product was isolated using preparative HPLC. 1.3 mg (total yield: 7 %) of compound **CDK7_1058** were yielded.

**Example 23.** Synthesis of compounds **CDK7_1060, CDK7_1103.**

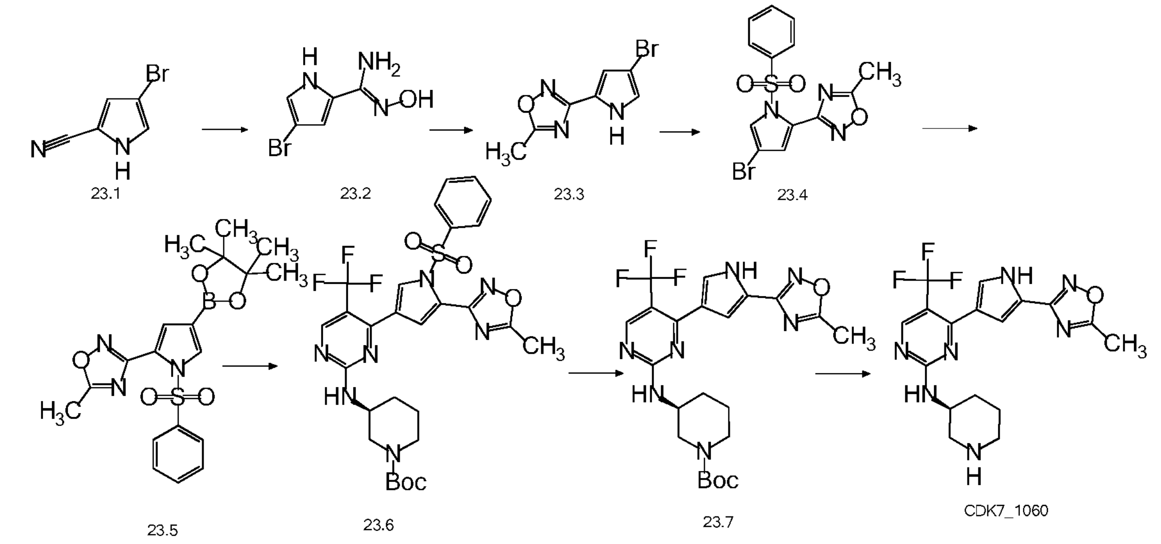

23.1 23.2 23.3 23.4

23.5 23.6 23.7 CDK7_1060

*Synthesis of compound* ***23.2***

A mixture of 4-bromo-1H-pyrrole-2-carbonitrile (0.40 g, 2.29 mmol) (**23.1**), $NH_2OH{\cdot}HCl$ (0.804 g, 11.45 mmol) and TEA (1.6 ml, 11.45 mmol) in 20 ml of ethanol was boiled while stirring for 1 hour. After standard treatment and without additional purification, 0.465 g (99%) was produced and used in the next step.

*Synthesis of compound* ***23.3***

EDC (0.137 g, 0.71 mmol), HOBt (0.073 g, 0.47 mmol) were suspended in 3 ml of 1,4-dioxane; 0.027 ml (0.47 mmol) of acetic acid was added and stirred at room temperature in nitrogen atmosphere for 1 h. Compound **23.2** (0.100 g, 0.47 mmol) was then added to 3 ml of 1,4-dioxane and stirred at room temperature for 12 h, the volatile components were removed in vacuo. The intermediate was produced by column chromatography, dissolved in 2 ml of 1,4-dioxane, heated in a microwave reactor at 130 °C for 2 h, volatile components were removed in vacuo. The product was isolated using column chromatography on silica gel. 0.025 g (23%) of compound **23.3** was yielded.

*Synthesis of compound* ***23.4***

Compound **23.4** was produced analogously to compound **1.2** with a yield of 71%.

*Synthesis of compound* ***23.5***

Compound **23.4** (0.044 g, 0.120 mmol), $(BPin)_2$ (0.04 g, 0.16 mmol), KOAc (0.036 g, 0.36 mmol), $Pd(dppf)Cl_2$ (0.009 g, 0.01 mmol) in 1 ml of 1,4-dioxane was heated while stirring in nitrogen atmosphere at 100 °C for 4 h. After standard treatment, the product was isolated using column chromatography on silica gel. 0.034 g (68%) of compound **23.5** was yielded.

*Synthesis of compound* ***23.6***

Compound **23.6** was produced analogously to compound **20.3** with a yield of 41%.

*Synthesis of compound* ***CDK7_1060***

Compound **23.6** (0.016 g, 0.02 mmol), NaOH (0.016 g, 0.40 mmol) in 0.5 ml of ethanol and 0.4 ml of water were stirred at room temperature for 4 h. After standard treatment, the residue comprising compound **23.7** was dissolved in 1 ml of dichloromethane, 0.058 ml of TFA was added, the reaction mass was stirred at 20 °C for 18 h, volatile components were removed in vacuo, the product was isolated by preparative HPLC. 0.008 g (66%) of compound **CDK7_1060** was yielded.

Compound **CDK7_1103** was produced analogously using cyclopropyl carboxylic acid with a total yield of 1%.

**Example 24.** Synthesis of compounds **CDK7_1061, CDK7_533.**

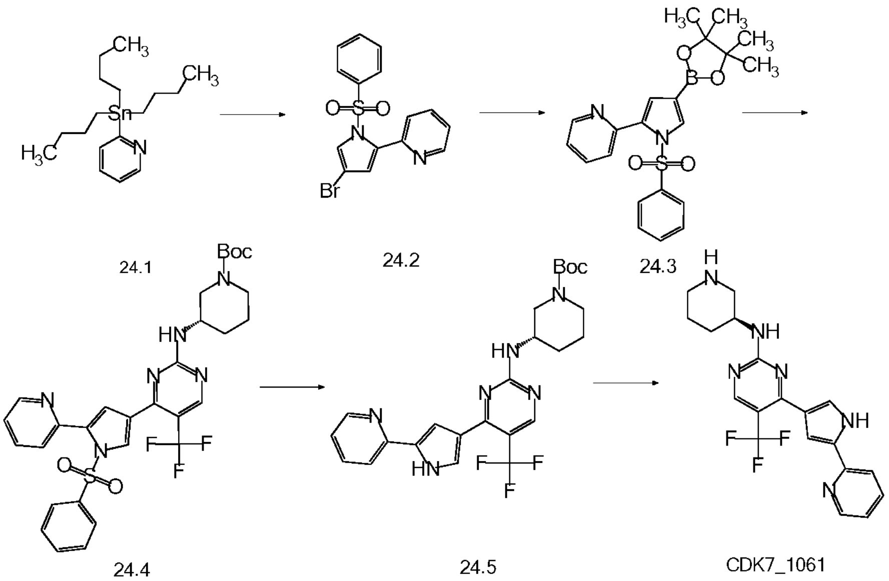

24.1 24.2 24.3

24.4 24.5 CDK7_1061

*Synthesis of compound **24.2***

A mixture of pyridine of **24.1** (0.304 g, 0.82 mmol), 1-(benzenesulfonyl)-4-bromo-2-iodine-1 *H*-pyrrole (0.228 g, 0.55 mmol), CuI (0.032 g, 0.16 mmol), $Pd(PPh_3)_4$ (0.064 g, 0.06 mmol) in 5 ml of 1,4-dioxane was heated while stirring in nitrogen atmosphere at 100 °C for 4 h. After standard treatment, the product was isolated using silica gel column chromatography. 0.054 g (32%) of compound **24.2** was yielded.

*Synthesis of compound **24.3***

Compound **24.2** (0.064 g, 0.18 mmol), $(BPin)_2$ (0.093 g, 0.36 mmol), KOAc (0.055 g, 0.54 mmol), $Pd(dppf)Cl_2$ (0.04 g, 0.05 mmol) in 2 ml of 1,4-dioxane were heated while stirring in nitrogen atmosphere at 100 °C for 4 h. After standard treatment, the product was isolated using column chromatography on silica gel. 0.074 g (99%) of compound **24.3** was yielded.

*Synthesis of compound **24.4***

Compound **3.3** (0.074 g, 0.18 mmol), pyrimidine of **1.4** (0.069 g, 0.18 mmol), $Na_2CO_3$ (0.058 g, 0.54 mmol), $Pd(dppf)Cl_2$ (0.04 g, 0.05 mmol) in a mixture of 2 ml of 1,4-dioxane and 1 ml of water were stirred in nitrogen atmosphere at 100 °C for 2 h. After standard treatment, the reaction product was isolated using column chromatography on silica gel. 0.04 g (35%) of compound **24.4** was yielded.

*Synthesis of compound **24.5***

Compound **24.4** (0.04 g, 0.06 mmol), NaOH (0.024 g, 0.6 mmol) in a mixture of 3 ml of ethanol and 3 ml of water were stirred at room temperature for 1 h. After standard treatment, 0.029 g (99%) of compound **24.5** was yielded.

*Synthesis of compound **CDK7_1061***

Compound **CDK7_1061** was produced analagously to compound **CDK7_1060** with a yield of 44%.

Compound **CDK7_533** was produced analagously using the corresponding boronic acid (conditions were analagous to the synthesis of compound **21.4**) with a total yield of 22%.

**Example 25.** Synthesis of compounds **CDK7_1000**

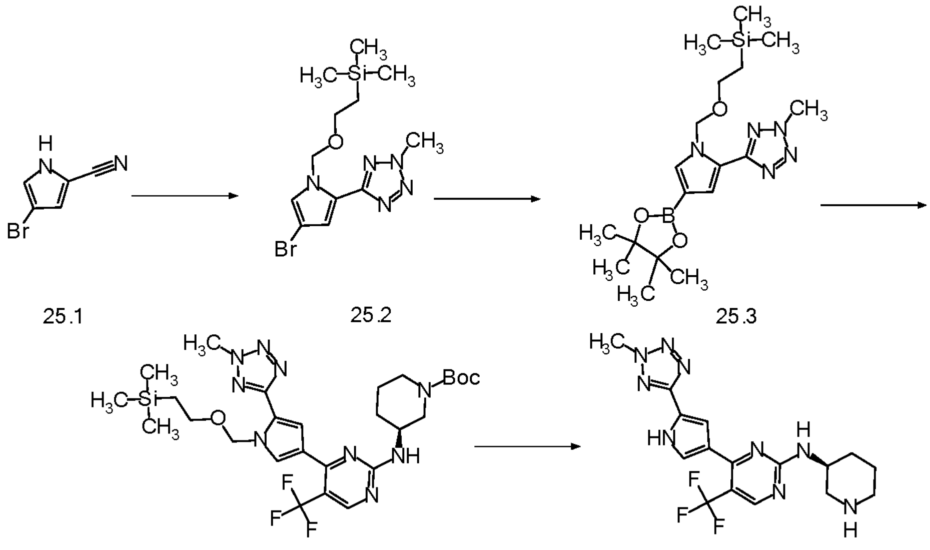

25.1 25.2 25.3

25.4 CDK7_1000

*Synthesis of compound **25.2***

NaH (0.045 g, 1.13 mmol) was added at 0 °C to a solution of 4-bromo-1*H*-pyrrol-2-carbonitrile **25.1** (0.130 g, 0.750 mmol) in 3 ml of tetrahydrofuran, stirred for 30 min, 0.217 ml (1.2 mmol) of SEMCl was added, stirred at room temperature for 1 h. After standard treatment, volatile components were removed in vacuo. The residue was dissolved in 2.5 ml of DMF, $NaN_3$ (0.058 g, 0.88 mmol), $NH_4Cl$ (0.047 g, 0.88 mmol) were added, heated while stirring in a microwave synthesizer vial at 160 °C for 2 h. 0.069 ml (1.10 mmol) of methyl iodide was added to the reaction mixture, stirred at room temperature for 12 h. Volatile components were removed and the product was isolated using column chromatography. $(BPin)_2$ (0.168 g, 0.66 mmol), KOAc (0.134 g, 1.32 mmol), Pd(dppf)$Cl_2$ (0.025 g, 0.03 mmol) in 2 ml of 1,4-dioxane were added to the residue comprising compound **25.2** and heated while stirring in nitrogen atmosphere at 100 °C for 4 h. After standard treatment, the reaction mixture was filtered off through a silica gel layer and volatile components were removed. The residue comprising compound **25.3** was dissolved in a mixture of 3 ml of 1,4-dioxane and 1.5 ml of water, pyrimidine **1.4** (0.118 g, 0.310 mmol), $Na_2CO_3$ (0.100 g, 0.930 mmol), Pd(dppf)$Cl_2$ (0.069 g, 0.090 mmol) were added and stirred in nitrogen atmosphere for 1 hour at 100 °C. After standard treatment, the reaction mixture was filtered off through a silica gel layer and volatile components were removed. 1 ml of dichloromethane, **0.07 ml of TFA were added to the residue comprising compound** 25.4, the reaction mass was stirred at 20 °C for 18 h, volatile components were removed in vacuo. The residue was dissolved in 1 ml of ethanol; NaOH (0.036 g, 0.90 mmol) and 1 ml of water were added, stirred at 20 °C for 18 h. After standard treatment, the reaction product was isolated using preparative HPLC**CDK7_1000** was yielded.

**Example 26.** Synthesis of compound **CDK7_1124**

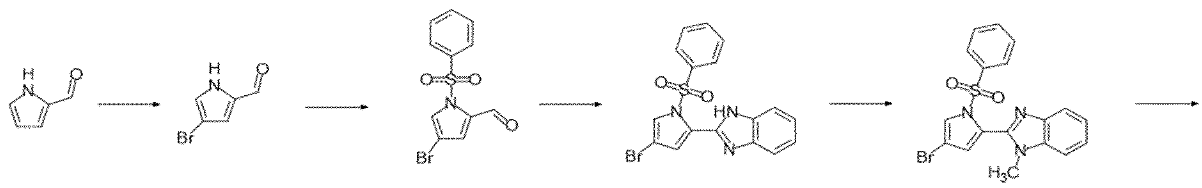

26.1 26.2 26.3 26.4 26.5

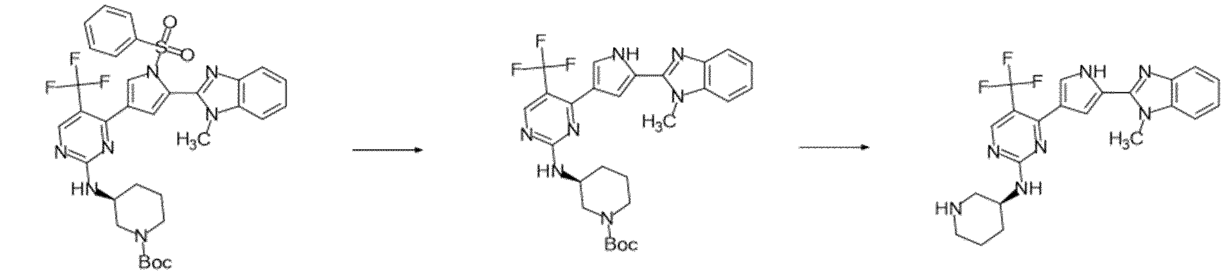

26.6 26.7 CDK7_1124

*Synthesis of compound **26.2***

NBS (0.926 g, 122.87 mmol) was added at 0 °C to a solution of pyrrol-2-carbaldehyde **26.1** (0.5 g, 5.15 mmol) in 10 ml of THF and stirred for 15 minutes at 0 °C. The solvent was removed in vacuo, the product was isolated using recrystallization. 0.446 g (50%) of compound **26.2** was yielded.

*Synthesis of compound **26.3***

NaH (0.069 g, 1.72 mmol) was added at 0 °C to a solution of compound **26.2** (0.200 g, 1.06 mmol) in 10 ml of THF and stirred for 30 minutes, benzenesulfonyl chloride (0.222 ml, 1.72 mmol) was added and stirred at room temperature for 1 h. After standard treatment, the product was isolated using silica gel column chromatography. 0.270 g (75%) of compound **26.3** was yielded.

*Synthesis of compound **26.4***

Compound **26.3** (0.246 g, 0.78 mmol), 1,2-diaminobenzene (0.089 g, 0.78 mmol), $Na_2S_2O_5$ (0.148 g, 0.78 mmol) in 6 ml DMF were stirred in a microwave reactor at 80 °C for 25 min. After standard treatment, the reaction product was isolated using recrystallization. 0.100 g (32%) of compound **26.4** was yielded.

*Synthesis of compound **26.5***

NaH (0.015 g, 0.38 mmol) was added at 0 °C to a solution of compound **26.4** (0.100 g, 0.25 mmol) in 5 ml of THF (or DMF) and stirred for 20 min, methyl iodide (0.045 g, 0.30 mmol) was added and stirred at room temperature for 18 h. After standard treatment, the product was isolated using silica gel column chromatography. 0.103 g (99%) of compound **26.5** was yielded.

*Synthesis of compound **26.6***

Compound **26.5** (0.110 g, 0.250 mmol), $(BPin)_2$ (0.130 g, 0.500 mmol), KOAc (0.076 g, 0.75 mmol), $Pd(dppf)Cl_2$ (0.056 g, 0.07 mmol) were mixed in 3 ml of 1,4-dioxane and heated in a sealed vessel at 100°C for 4 h. After cooling, the reaction mixture was filtered off through celite, the solvent was removed in vacuo. Pyrimidine **1.4** (0.095 g, 0.250 mmol), $Na_2CO_3$ (0.08 g, 0.750 mmol), $Pd(dppf)Cl_2$ (0.056 g, 0.07 mmol) in a mixture of 3 ml of 1,4-dioxane and 1.5 ml of water were added to the residue. The resulting mixture was stirred in nitrogen atmosphere for 4 h at 110 °C, the reaction mixture was filtered off through celite, and the solvent was removed in vacuo. The product was isolated using column chromatography on silica gel. 0.042 g (25%) of compound **26.6** was yielded.

*Synthesis of compound **26.7***

Compound **26.6** (0.042 g, 0.060 mmol), NaOH (0.048 g, 1.20 mmol) in 3 ml of ethanol and 3 ml of water were stirred at room temperature for 1 h. The solvent was removed in vacuo, 0.028 g (88%) of compound **26.7** was yielded.

*Synthesis of compound **CDK7_1124***

TFA (0.116 g, 1.50 mmol) was added to a solution of **26.7** (0.028 g, 0.05 mmol) in 12 ml of dichloromethane, the reaction mass was stirred for 16 h at room temperature. Volatile components were removed in vacuo. The product was isolated using preparative HPLC. 0.008 g (36%) of compound **CDK7_1124** was yielded.

**Example 27.** Synthesis of compound **CDK7_1021**

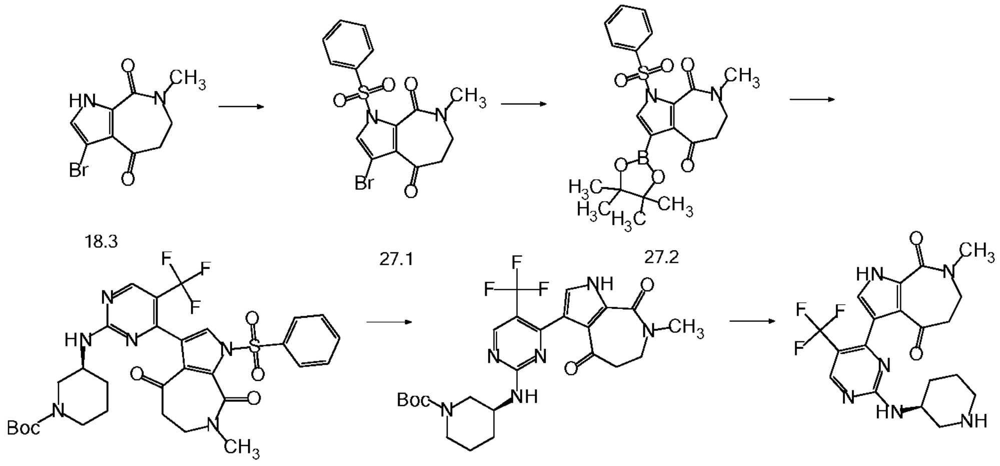

18.3 27.1 27.2

27.3 27.4 CDK7_1021

*Synthesis of compound **27.1***

Compound **18.3** (0.250 g, 0.830 mmol) was dissolved in 15 ml of THF, NaH (0.037 g, 0.910 mmol) was added at 0 °C to the resulting solution, and the mixture was stirred for 10 minutes, benzenesulfonyl chloride (0.139 ml, 1.08 mmol) was added and stirred for 1 hour at room temperature. After standard treatment, the product was isolated using silica gel column chromatography. 0.131 g (40%) of compound **27.1** was yielded.

*Synthesis of compound **27.2***

Compound **27.1** (0.131 g, 0.310 mmol), $(BPin)_2$ (0.157 g, 0.620 mmol), KOAc (0.094 0.930 mmol), $Pd(dppf)Cl_2$ (0.023 g, 0.030 mmol) in 5 ml of 1,4-dioxane were stirred in nitrogen atmosphere for 8 h at 100°C. After standard treatment, the reaction product was isolated using column chromatography on silica gel. 0.06 g (43%) of compound **27.2** was yielded.

*Synthesis of compound **27.3***

Compound **27.3** was produced analogously to compound **4.3** with a yield of 23%.

*Synthesis of compound **CDK7_1021***

Compound **27.3** (0.021 g, 0.03 mmol), NaOH (0.012 g, 0.03 mmol) in 0.5 ml of ethanol and 0.5 ml of $H_2O$ were stirred at room temperature for 2 h. After standard treatment, the residue containing compound **27.4** was dissolved in 1 ml of dichloromethane, 0.093 ml of TFA was added and stirred at room temperature for 20 h. After standard treatment, the product was isolated using preparative HPLC. 0.006 g (35%) of compound **CDK7_1021** was yielded.

**Example 28.** Synthesis of compound **CDK7_1121**

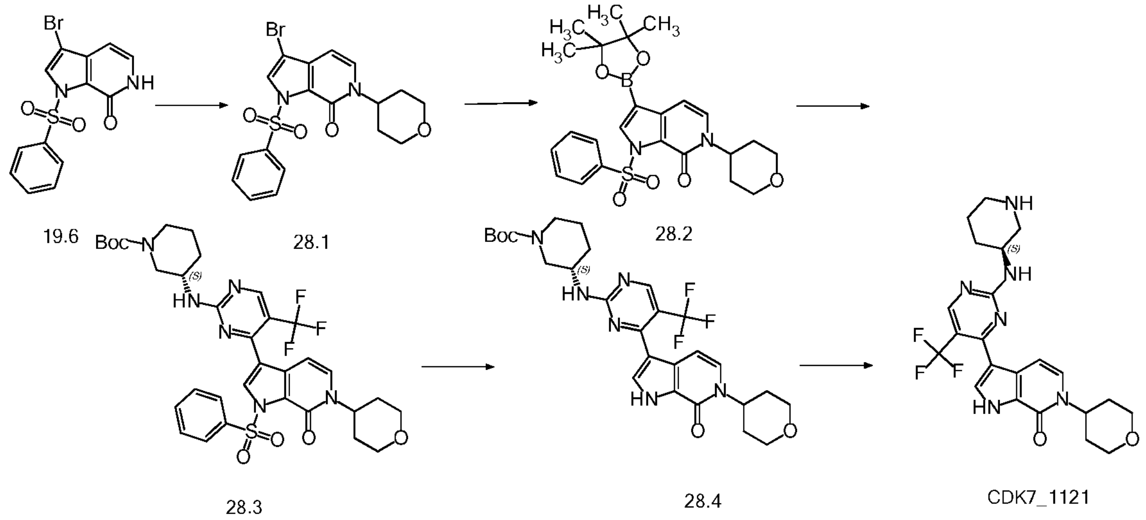

19.6 28.1 28.2 28.3 28.4 CDK7_1121

*Synthesis of compound* ***28.1***

Compound **19.6** (0.071 g, 0.190 mmol), oxane-4-yl methanesulfonate (0.070 g, 0.380 mmol), Cs $_2CO_3$ (0.125 g, 0.380 mmol) in a mixture of 1 ml of DMF and 1 ml of dimethoxyethane were heated while stirring at 90°C for 40 h. After standard treatment, the product was isolated using column chromatography on silica gel. 0.058 g (70%) of compound **28.1** was yielded.

*Synthesis of compound* ***28.2***

Compound **28.1** (0.058 g, 0.120 mmol), $(BPin)_2$ (0.050 g, 0.190 mmol), KOAc (0.039 g, 0.380 mmol), Pd(dppf)$Cl_2$ (0.009 g, 0.010 mmol) was dissolved in 1 ml of 1,4-dioxane, heated in a sealed vessel for 4 h at 110°C. After standard treatment, the product was isolated using column chromatography. 0.049 g (85%) of compound **28.2** was yielded.

*Synthesis of compound* ***28.3***

Compound **28.2** (0.049 g, 0.100 mmol), pyrimidine **1.4** (0.046 g, 0.120 mmol), $Na_2CO_3$ (0.040 g, 0.370 mmol), Pd(dppf)$Cl_2$ (0.030 g, 0.040 mmol) in a mixture of 1 ml of 1,4-dioxane and 0.5 ml of water were stirred in nitrogen atmosphere for 1 hour at 110 °C. After standard treatment, the product was isolated using column chromatography on silica gel. 0.040 g (57%) of compound **28.3** was yielded.

*Synthesis of compound* ***CDK7_1121***

Compound **28.3** (0.040 g, 0.060 mmol), NaOH (0.121 g, 3.00 mmol) in a mixture of 1 ml of ethanol and 1 ml of $H_2O$ was stirred at room temperature for 2 h. After standard treatment, the residue containing compound **28.4** was dissolved in 3 ml of dichloromethane, 0.696 ml of TFA was added and stirred at room temperature for 18 h. After standard treatment, the product was isolated using preparative HPLC. 0.003 g (11%) of compound **CDK7_1121** was yielded.

**Example 29.** Synthesis of compound **29.5.**

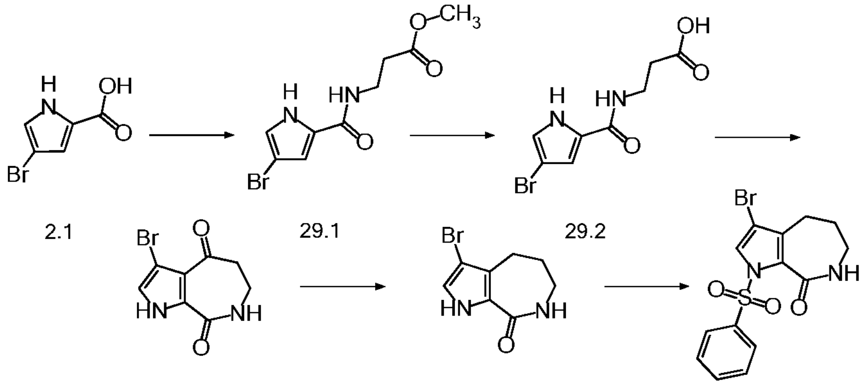

2.1 29.1 29.2

29.3 29.4 29.5

*Synthesis of compound* ***29.1***

Oxalyl chloride (5.94 ml, 67.9 mmol) and a drop of DMF were added to acid **2.1** (4.30 g, 22.6 mmol) in 60 ml of dichloromethane and stirred at room temperature for 1 hour. Volatile components were then removed, the residue was dissolved in 30 ml of dichloromethane and added dropwise to a solution of methyl 3-aminopropanoate hydrochloride (4.11 g, 29.4 mmol) and TEA (16.6 ml, 113 mmol) in 30 ml of dichloromethane at 0 °C. The mixture was stirred at room temperature for 1 h. After standard treatment, the product was isolated using column chromatography on silica gel. 5.70 g (92%) of compound **29.1** was yielded.

*Synthesis of compound* ***29.2***

Compound **29.1** (3.90 g, 12.8 mmol) was dissolved in 40 ml of ethanol, NaOH (1.08 g, 26.8 mmol) and 20 ml of water were added. The reaction mass was stirred at room temperature for 18 h. After standard treatment, 2.8 g (84%) of product **29.2** was yielded.

*Synthesis of compound* ***29.3***

A mixture of compound **29.2** (4.07 g, b 14.8 mmol), PPA (38.2 g, 148 mmol), $P_2O_5$ (4.29 g, 14.8 mmol) was stirred at 110 °C for 2 h. After standard treatment, 3.51 g (97%) of compound **29.3** were yielded.

*Synthesis of compound* ***29.4***

Compound **29.3** (2.04 g, 6.72 mmol), triethylsilane (3.16 g, 26.9 mmol) in 31 ml of TFA were stirred at room temperature for 20 h. After standard treatment, 1.22 g (79%) of compound **29.4** was obtained.

*Synthesis of compound* ***29.5***

Compound **29.5** was produced analogously to compound **2.3** with a yield of 50%.

**Example 30.** Synthesis of compound **CDK7_955.**

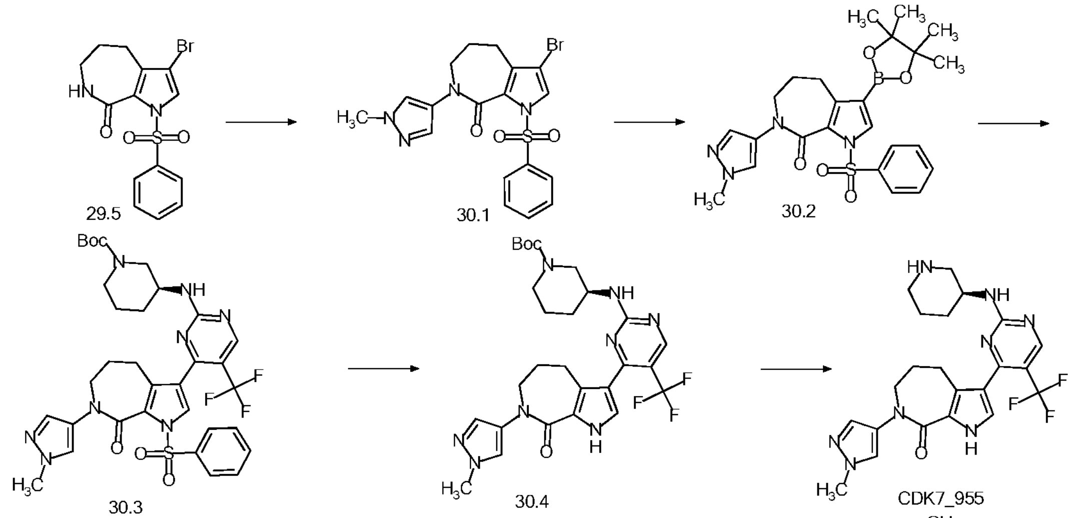

29.5 30.1 30.2

30.3 30.4 CDK7_955

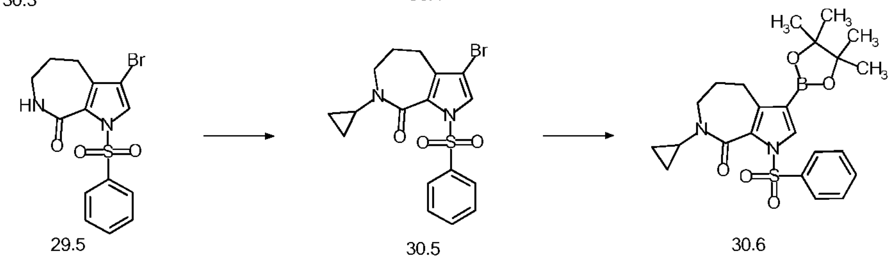

29.5 30.5 30.6

*Synthesis of compound **30.1***

Compound **29.5** (0.346 g, 0.920 mmol), 4-Iodo-1-methyl-1H-pyrazole (1.17 g, 5.52 mmol), CuI (0.088 g, 0.460 mmol), (1R, 2R)-cyclohexane-1,2-diamine (0.107 g, 0.920 mmol) in 14 ml of 1,4-dioxane were heated while stirring at 120 °C for 7 h. After standard treatment, the product was isolated using column chromatography. 0.247 g (60%) of compound **30.1** was yielded.

*Synthesis of compound **30.2***

Compound **30.1** (0.130 g, 0.290 mmol), $(BPin)_2$ (0.221 g, 0.870 mmol), KOAc (0.132 g, 1.30 mmol), $Pd(dppf)Cl_2$ (0.065 g, 0.09 mmol) was dissolved in 9 ml of 1,4-dioxane, heated in a sealed vessel for 12 h at 110° C. After standard treatment, the product was isolated by column chromatography. 0.141 g (98%) of compound **34.2** was yielded.

*Synthesis of compound **30.3***

Compound **30.2** (0.225 g, 0.12 mmol), pyrimidine **1.4** (0.059 g, 0.160 mmol), $Na_2CO_3$ (0.039 g, 0.360 mmol), $Pd(dppf)Cl_2$ (0.009 g, 0.01 mmol) in a mixture of 3 ml of 1,4-dioxane and 1 ml of water were stirred in nitrogen atmosphere for 10 h at 100 °C. After standard treatment, the product was isolated using column chromatography on silica gel. 0.060 g (70%) of compound **30.3** was yielded.

*Synthesis of compound **30.4***

Compound **30.3** (0.026 g, 0.04 mmol), NaOH (0.112 g, 2.80 mmol) in 5 ml of ethanol and 5 ml of water were stirred at room temperature for 1 hour, filtered off through celite, the solvent was removed in vacuo. 0.08 g (99%) of compound **30.4** was yielded.

*Synthesis of compound **CDK7_955***

Compound **30.4** (0.080 g, 0.14 mmol) was dissolved in 2 ml of dichloromethane, 0.217 ml of TFA was added, the reaction mass was stirred at 20 °C for 20 h. After standard treatment, the product was isolated using preparative HPLC. 0.043 g (65%) of compound **CDK7_955** was yielded.

*Synthesis of compound **30.5***

Compound **29.5** (0.201 g, 0.53 mmol), cyclopropylboronic acid (0.141 g, 1.59 mmol), copper(II) acetate (0.147 g, 0.079 mmol), TEA (0.339 g, 3.18 mmol), DMAP (0.196 g, 1.59 mmol) in 5 ml of toluene were stirred at 100 °C in for 12 h. After standard treatment, the product was isolated using silica gel column chromatography. 0.098 g (44%) of product **30.5** was yielded.

*Synthesis of compound **30.6***

Compound **30.5** (1.00 g, 2.32 mmol), $(BPin)_2$ (1.80 g, 6.96 mmol), KOAc (0.704 g, 6.96 mmol), $Pd(dppf)Cl_2$ (0.139 g, 0.190 mmol) was dissolved in 30 ml of 1,4-dioxane, heated in a sealed vessel for 24 h at 100° C. After standard treatment, the product was isolated using column chromatography. 0.700 g (66%) of compound **30.6** was yielded.

**Example 31.** Synthesis of compound **CDK7_1122, CDK7_1225.**

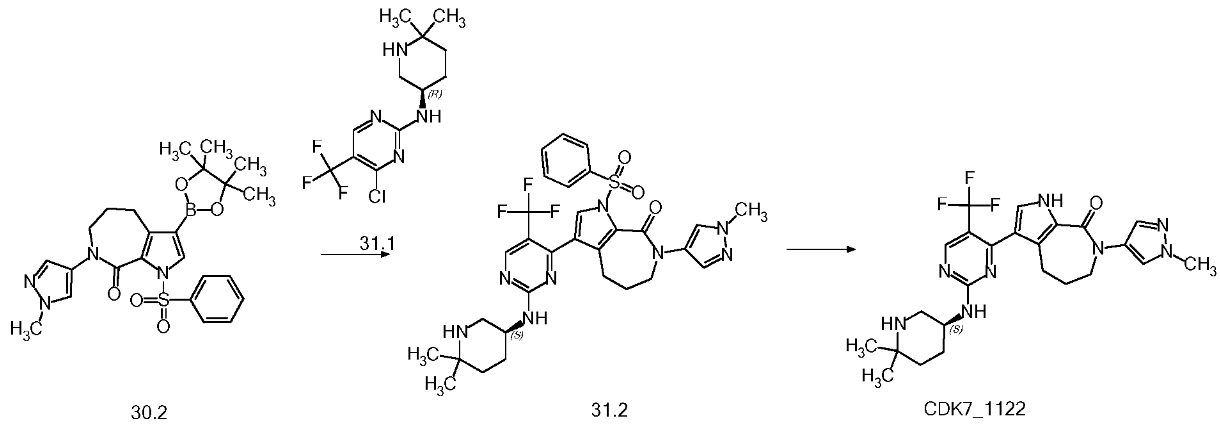


30.2 31.2 CDK7_1122

*Synthesis of compound **31.2***

Compound **30.2** (0.216 g, 0.440 mmol), pyrimidine **31.1** (0.125 g, 0.390 mmol), $Na_2CO_3$ (0.124 g, 1.16 mmol), $Pd(dppf)Cl_2$ (0.058 g, 0.08 mmol) in a mixture of 6 ml of 1,4-dioxane and 2 ml of water were stirred in nitrogen atmosphere for 4 h at 120 °C. After standard treatment, the product was isolated using column chromatography on silica gel. 0.114 g (46%) of compound **31.2** was yielded.

*Synthesis of compound **CDK7_1122***

Compound **31.2** (0.171 g, 0.270 mmol), NaOH (0.109 g, 2.70 mmol) in 2 ml of ethanol and 1 ml of water were stirred at room temperature for 4 h. After standard treatment, the product was isolated using preparative HPLC. 0.028 g (21%) of compound **CDK7_1122** was yielded.

Compound **CDK7_1225** was produced analogously with a total yield of 69%.

**Example 32.** Synthesis of compound **CDK7_1123.**

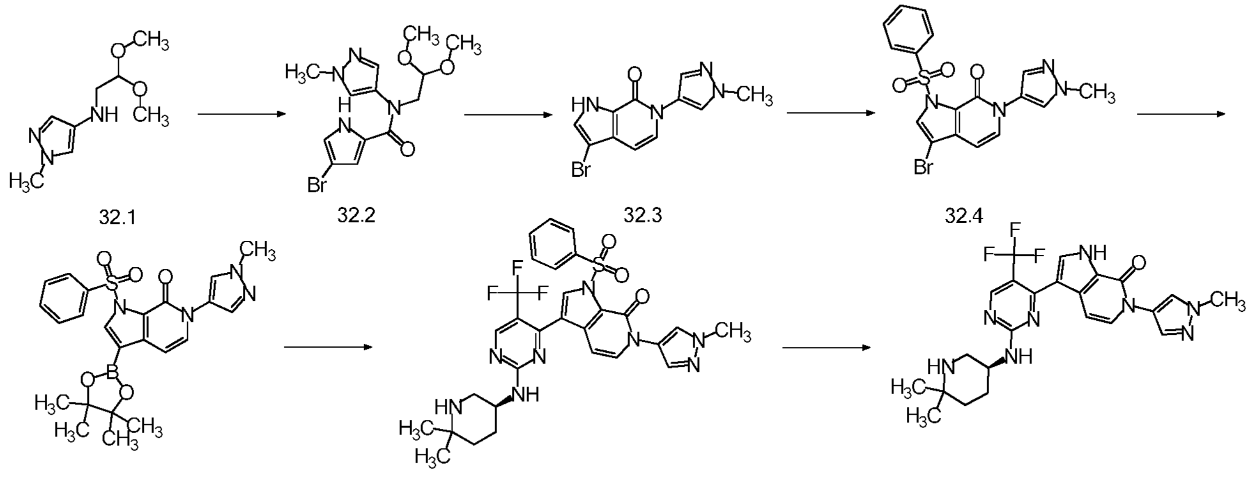

32.1 32.2 32.3 32.4

32.5 32.6 CDK7_1123

*Synthesis of compound **32.2***

Compound **32.2** was produced analogously to compound **4.2** with a yield of 79%.

*Synthesis of compound **32.3***

Amide **32.2** (7.34 g, 20.5 mmol) and 27 ml of methanesulfonic acid (410 mmol) were stirred for 16 h at 20 °C. After standard treatment, 4.98 g (83%) of compound **32.3** were yielded.

*Synthesis of compound **32.4***

DIPEA (6.68 g, 51.2 mmol), DMAP (1.05 g, 8.53 mmol) and benzenesulfonyl chloride (4.87 g, 27.3 mmol) were added to a solution of pyrrolopyridone **32.3** (5.00 g, 17.1 mmol) in 100 ml of dichloromethane, the reaction mass was stirred at room temperature for 18 h. After standard treatment, the product was isolated using silica gel column chromatography. 3.00 g (41%) of compound **32.4** was yielded.

*Synthesis of compound **32.5***

Compound **32.4** (1.50 g, 3.46 mmol), $(BPin)_2$ (1.76 g, 6.92 mmol), KOAc (1.05 g, 10.4 mmol), $Pd(dppf)Cl_2$ (0.258 g, 0.350 mmol) in 20 ml of 1,4-dioxane was heated while stirring in nitrogen atmosphere for 3 h at 100°C. After standard treatment, the product was isolated using column chromatography on silica gel. 1.60 g (96%) of compound **32.5** was yielded.

*Synthesis of compound **32.6***

Compound **32.5** (0.117 g, 0.230 mmol), pyrimidine **31.1** (0.110 g, 0.210 mmol), $Na_2CO_3$ (0.067 g, 0.630 mmol), $Pd(dppf)Cl_2$ (0.031 g, 0.040 mmol) in a mixture of 3 ml of 1,4-dioxane and 1 ml of water were stirred in nitrogen atmosphere for 18 h at 100 °C. After standard treatment, 0.130 g (98%) of compound **32.6** was yielded.

*Synthesis of compound **CDK7_1123***

Compound **32.6** (0.600 g, 0.960 mmol), NaOH (0.384 g, 9.60 mmol) in 18 ml of ethanol and 3 ml of water were stirred at 45 °C for 3 h. After standard treatment, the product was isolated using preparative HPLC. 0.106 g (23%) of compound **CDK7_1123** was yielded.

**Example 33.** Synthesis of compound **CDK7_1176, CDK7_1172, CDK7_1177, CDK7_1188.**

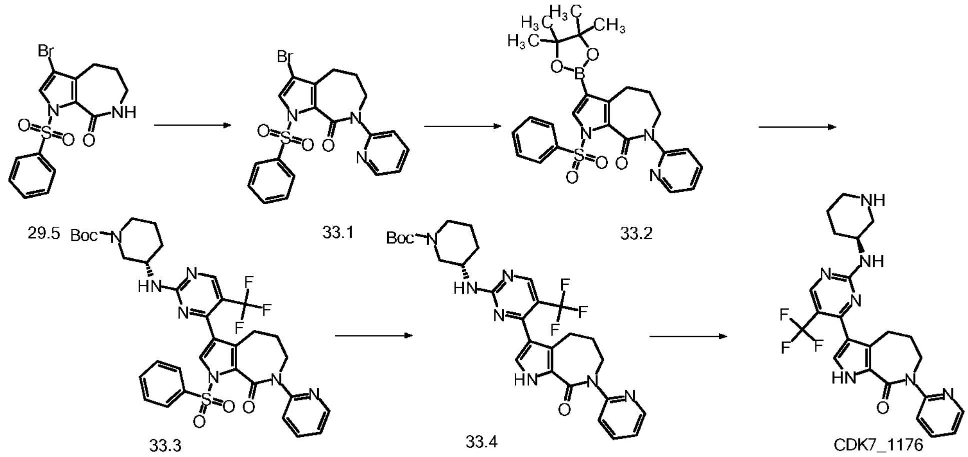

29.5 33.1 33.2 33.3 33.4 CDK7_1176

*Synthesis of compound* ***33.1***

Compound **33.1** was produced analogously to compound **30.1** with a yield of 83%.

*Synthesis of compound* ***33.2***

Compound **33.2** was produced analogously to compound **30.2** with a yield of 99%.

*Synthesis of compound* ***33.3***

Compound **33.3** was produced analogously to compound **30.3** with a yield of 53%.

*Synthesis of compound* ***33.4***

Compound **33.4** was produced analogously to compound **30.4** with a yield of 99%.

*Synthesis of compound* ***CDK7_1176***

Compound **CDK7_1176** was produced analogously to compound **CDK7_955** with a yield of 70%.

The following compounds were produced in an analogous manner:

| No . | Compound | Initial compound | Stage, yield, % | | | | |
|---|---|---|---|---|---|---|---|
| | | | 1 | 2 | 3 | 4 | 5 |
| 1 | **CDK7_1172** | 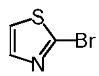 | 37 | 86 | | | 13 |
| 2 | **CDK7_1177** | 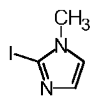 | 38 | 4 | | | |
| 3 | **CDK7_1188** | 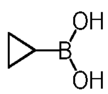 | 18* | 84 | 27 | | |
| *Conditions and methods of the stage are described in the synthesis of compound **30.5** | | | | | | | |

**Example 34.** Synthesis of compound **CDK7_1187.**

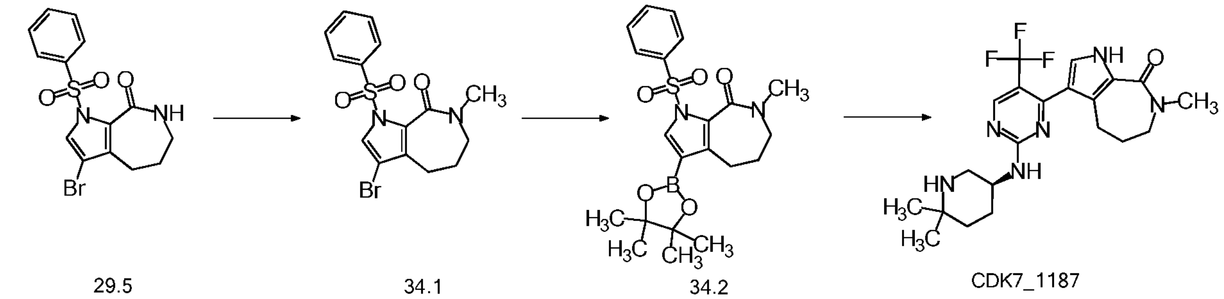

29.5 34.1 34.2 CDK7_1187

*Synthesis of compound **34.1***

*Method A:*

Compound **34.1** was produced analogously to compound **2630.51** with a yield of 79%.

*Method B:*

Compound **34.1** was produced analogously to **44.3** from compound **43.1** and MeI with a total yield of 70.0 %

*Synthesis of compound **34.2***

Compound **34.2** was produced analogously to compound **30.2** with a yield of 99%.

*Synthesis of compound **CDK7_1187***

Compound **34.2** (0.069 g, 0.120 mmol), pyrimidine **31.1** (0.062 g, 0.120 mmol), $Na_2CO_3$ (0.039 g, 0.360 mmol), $Pd(dppf)Cl_2$ (0.013 g, 0.020 mmol) in a mixture of 2 ml of 1,4-dioxane and 1 ml of water were stirred in nitrogen atmosphere for 6 h at 100 °C. After standard treatment, the product was isolated using preparative HPLC. 0.05 g (10%) of compound **CDK7_1187** was yielded.

**Example 35.** Synthesis of compounds **CDK7_999, CDK7_1102.**

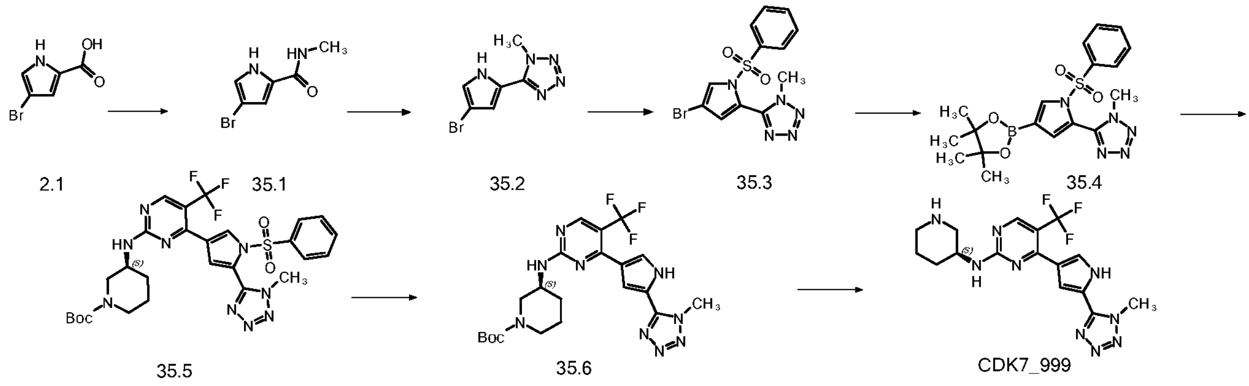

2.1 35.1 35.2 35.3 35.4

35.5 35.6 CDK7_999

*Synthesis of compound **35.1***

Compound **35.1** was produced analogously to compound **4.2** with a yield of 96%.

*Synthesis of compound **35.2***

Compound **35.1** was dissolved in 10 ml of acetonitrile; $NaN_3$ (0.136 g, 2.07 mmol) was added. A solution of trifluoromethanesulfonic anhydride (0.570 g, 1.88 mmol) in 5 ml of acetonitrile was then added and stirred at room temperature for 18 h. After standard treatment, the product was isolated using column chromatography. 0.162 g (76%) of

compound **35.2** was yielded.

*Synthesis of compound **35.3***

Compound **35.3** was produced analogously to compound **26.3** with a yield of 49%.

*Synthesis of compound **35.4***

Compound **35.4** was produced analogously to compound **20.2** with a yield of 50%.

*Synthesis of compound **35.5***

Compound **35.5** was produced analogously to compound **20.3** with a yield of 84%.

*Synthesis of compound **35.6***

Compound **35.6** was produced analogously to compound **24.5** with a yield of 74%.

*Synthesis of compound **CDK7_999***

Compound **CDK7_999** was produced analogously to compound **CDK7_951** with a yield of 58%.

Compound **CDK7_1102** was produced analogously using cyclopropylamine with a total yield of 1%.

**Example 36.** Synthesis of compound **CDK7_814.**

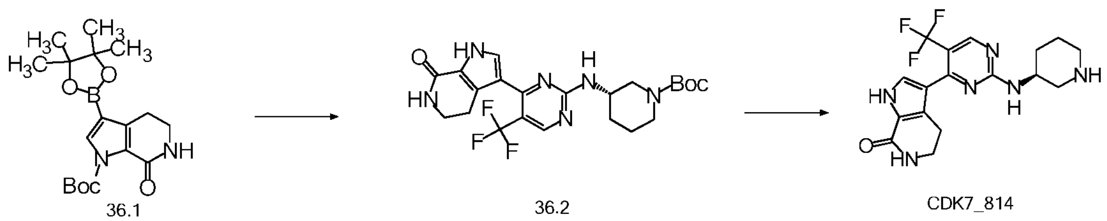

36.1 36.2 CDK7_814

*Synthesis of compound **CDK7_814***

Compound **36.1** (0.152 g, 0.210 mmol), pyrimidine **1.4** (0.080 g, 0.210 mmol), $Na_2CO_3$ (0.067 g, 0.630 mmol), Pd(dppf)$Cl_2$ (0.047 g, 0.060 mmol) in a mixture of 3 ml of 1,4-dioxane and 1.5 ml of water were stirred in nitrogen atmosphere for 1 hour at 100 °C. Volatile components were removed, and the product was isolated using column chromatography. 3 ml of TFA was added to the residue containing compound **36.2** and stirred at room temperature for 12 h. After standard treatment, the product was isolated using preparative chromatography. 0.023 g (30%) of compound **CDK7_814** was yielded.

**Example 37.** Synthesis of compound **CDK7_843.**

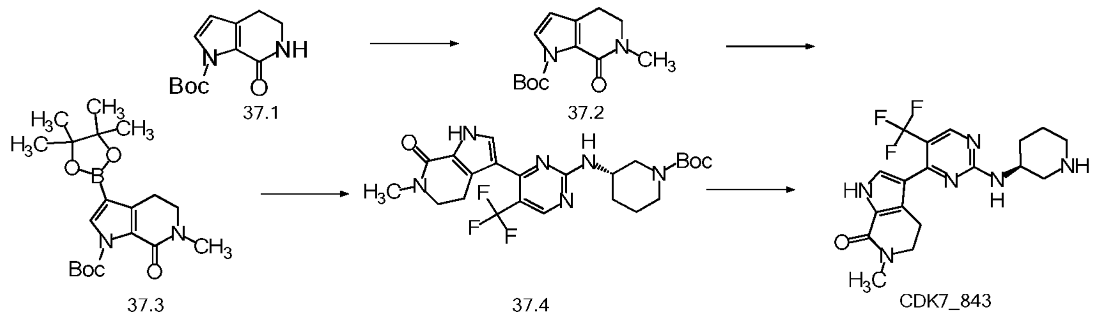
37.1 37.2 37.3 37.4 CDK7_843

*Synthesis of compound **37.2***

1M solution of LiHMDS in tetrahydrofuran (0.405 ml, 0.410 mmol) was added at -10 °C to a solution of compound **37.1** (0.082 g, 0.340 mmol) in 4 ml of THF and stirred for 1 hour; methyl iodide (0.161 g, 1.12 mmol) was added and stirred at 0 °C for 10 minutes, then stirred at room temperature for 2 h. After standard treatment, the product was isolated using silica gel column chromatography. 0.067 g (79%) of compound **37.2** was yielded.

*Synthesis of compound **CDK7_843***

A mixture of compound **37.2** (0.085 g, 0.330 mmol), $(BPin)_2$ (0.128 g, 0.490 mmol), $[Ir(OMe)cod]_2$ (0.016 g, 0.020 mmol), 4,4'-di-tert-butyl-2,2'-bipyridine (0.013 g, 0.050 mmol) was mixed in 2 ml of THF and stirred at room temperature for 10 minutes, a solution of compound **37.2** (0.085 g, 0.330 mmol in 1 ml of THF was then added and heated in a sealed vessel at 70 °C for 2 h, then at room temperature for 12 h. After cooling, the mixture was filtered off through a silica gel layer, the solvent was removed in vacuo. **Pyrimidine** 1.4 (0.122 g, 0.320 mmol), **Na2CO3** (0.103 g, 0.960 mmol), Pd(dppf)Cl2 (0.072 g, 0.100 mmol) in a mixture of 3 ml of 1,4-dioxane and 1.5 ml of water were added to the residue containing compound 37.3. The resulting mixture was stirred in nitrogen atmosphere for 4 h at 100 °C, the reaction mixture was filtered off through a silica gel layer, and the solvent was removed in vacuo. The product was isolated using column chromatography on silica gel. The residue containing compound **37.4** was then dissolved in 3 ml of TFA, stirred at room temperature for 12 h. After standard treatment, the product was isolated using preparative HPLC. 0.006 g (5%) of compound **CDK7_843** was yielded.

**Example 38.** Synthesis of compound **CDK7_1019.**

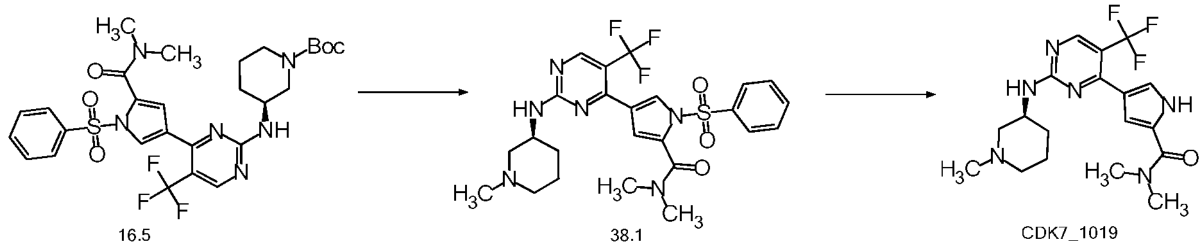
16.5 38.1 CDK7_1019

*Synthesis of compound **38.1***

Compound **16.5** (0.220 g, 0.350 mmol) was dissolved in 1 ml of TFA, stirred at room temperature for 32 h, the solvent was removed in vacuo. The dry residue was then dissolved in 15 ml of dichloromethane, methyl iodide (0.024 ml, 0.390 mmol) and DIPEA (0.311 ml, 1.75 mmol) were added, stirred at room temperature for 12 h. After standard treatment, the product was isolated using column chromatography. 0.160 g (85%) of compound **38.1** was yielded.

*Synthesis of compound **CDK7_1019***

Compound **38.1** (0.160 g, 0.280 mmol), NaOH (0.226 g, 5.60 mmol) in 6 ml of ethanol and 6 ml of water were stirred at room temperature for 1 h. The solvent was removed in vacuo, the product was isolated using preparative HPLC. 0.035 g (32%) of compound **CDK7_1019** was yielded. **Example 39.** Synthesis of compound **CDK7_1020.**

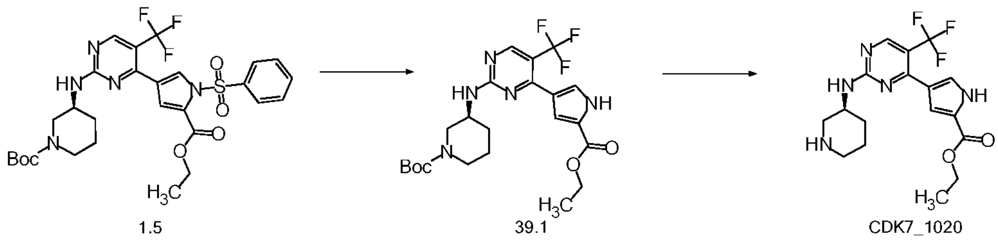

1.5 39.1 CDK7_1020

*Synthesis of compound **CDK7_1020***

Compound **1.5** (0.050 g, 0.080 mmol), NaOH (0.065 g, 1.60 mmol) in 2 ml of ethanol and 2 ml of water were stirred at room temperature for 1 h. After standard treatment, the residue was dissolved in 1.5 ml of TFA and stirred at room temperature for 16 h. After standard treatment, the product was isolated using preparative HPLC. 0.015 g (48%) of compound **CDK7_1020** was yielded.

**Example 40.** Synthesis of compound **40.2.**

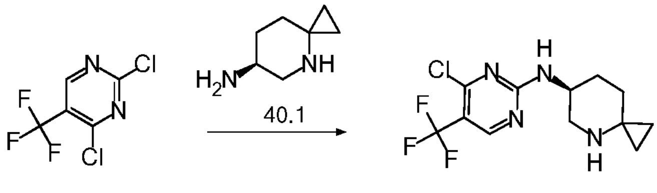


15.1 40.2

*Synthesis of compound 40.2*

Compound **40.2** was produced analogously to compound **15.3** with a yield of 48%. **Example 41.** Synthesis of compound **CDK7_1199.**

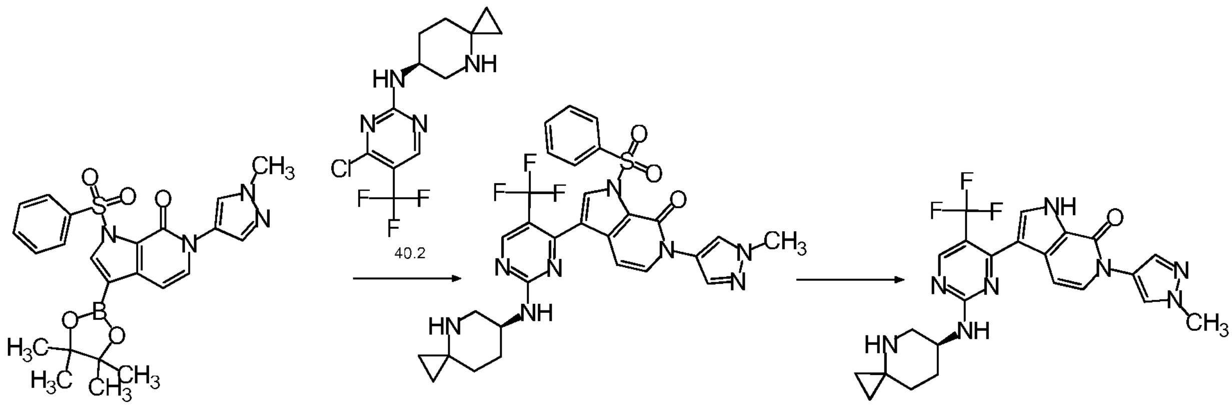


32.5 41.1 CDK7_1199

*Synthesis of compound **CDK7_1199***

Compound **32.5** (0.053 g, 0.100 mmol), pyrimidine **40.2** (0.028 g, 0.090 mmol), $Na_2CO_3$ (0.106 g, 0.270 mmol), Pd(dppf)$Cl_2$ (0.020 g, 0.027 mmol) in a mixture of 1 ml of 1,4-dioxane and 0.5 ml of water were stirred in nitrogen atmosphere at 100 °C for 2 h. After standard treatment, intermediate **41.1** was used in the next stage without additional purification. NaOH (0.032 g, 0.800 mmol), 4 ml of ethanol and 4 ml of water were added to the residue containing compound **41.1** and stirred at room temperature for 1 h. After standard treatment, the product was isolated using preparative HPLC. 0.001 g (3%) of compound **CDK7_1199** was yielded.

**Example 42.** Synthesis of compound **CDK7 1254.**

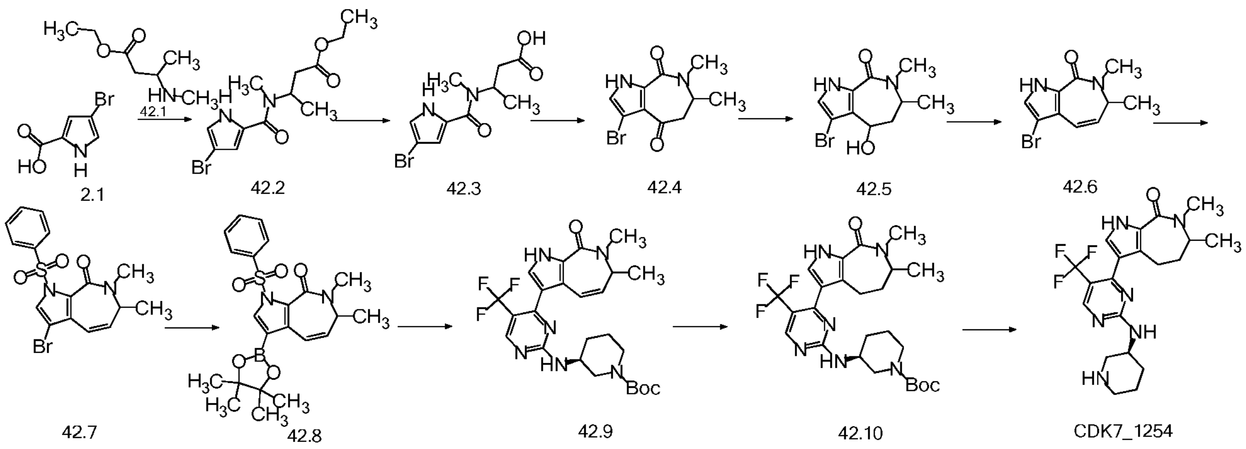


2.1, 42.2, 42.3, 42.4, 42.5, 42.6, 42.7, 42.8, 42.9, 42.10, CDK7_1254

*Synthesis of compound **42.2***

Compound **42.2** was produced analogously to compound **29.1** with a yield of 97%.

*Synthesis of compound **42.3***

Compound **42.3** was produced analogously to compound **29.2** with a yield of 62%.

*Synthesis of compound **42.4***

Compound **42.4** was produced analogously to compound **29.3** with a yield of 72%.

*Synthesis of compound **42.6***

$NaBH_4$ (0.062 g, 1.64 mmol) was added while cooling on an ice bath to a solution of compound **42.4** (0.220 g, 0.410 mmol) in 7 ml of ethanol, the reaction mass was stirred at 20 °C for 24 h. After standard processing, the product was isolated using silica gel column chromatography. The residue comprising compound **42.5** was dissolved in 4 ml of dichloromethane, and 0.57 ml of 4N solution of HCl in 1,4-dioxane was added and stirred at 20 °C for 20 h. After standard treatment, the product was isolated using column chromatography. 0.077 g (79%) of compound **42.6** was yielded.

*Synthesis of compound **42.7***

Compound **42.7** was produced analogously to compound **2.3** with a yield of 87%.

*Synthesis of compound **42.8***

Compound **42.8** was produced analogously to compound **27.2** with a yield of 83%.

*Synthesis of compound **42.9***

Compound **42.9** was produced analogously to compound **1.5** with a yield of 41%.

*Synthesis of compound **42.10***

Pd/C 5% (0.170 g, 0.080 mmol) was added to a solution of compound **42.9** (0.045 g, 0.080 mmol) in 5 ml of methanol. Hydrogenation was performed at atmospheric pressure for 20 h, the reaction mass was filtered off through a Celite layer, the solvent was removed in vacuo. 0.023 g (55%) of compound **42.10** was yielded.

*Synthesis of compound **CDK7_1254***

Compound **CDK7_1254** was produced analogously to compound **CDK7_836** with a yield of 29%. **Example 43.** Synthesis of compound **43.1.**

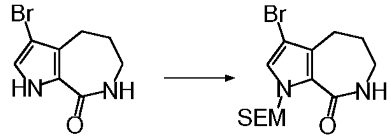

29.4 43.1

*Synthesis of compound **43.1***

NaH (0.227 g, 5.68 mmol) at 0 °C was added to a solution of compound **29.4** (1.00 g, 4.37 mmol) in 50 ml of tetrahydrofuran, stirred for 30 min, 0.789 ml (4.37 mmol) of SEMCl was added, stirred at room temperature for 3 h. After standard treatment, the reaction product was isolated by column chromatography. 0.927 g (59%) of compound **43.1** was yielded.

**Example 44.** Synthesis of compounds **CDK7_1154, CDK7_1155, CDK7_1226, CDK7_1227, CDK7_1229, CDK7_1259, CDK7_1268.**

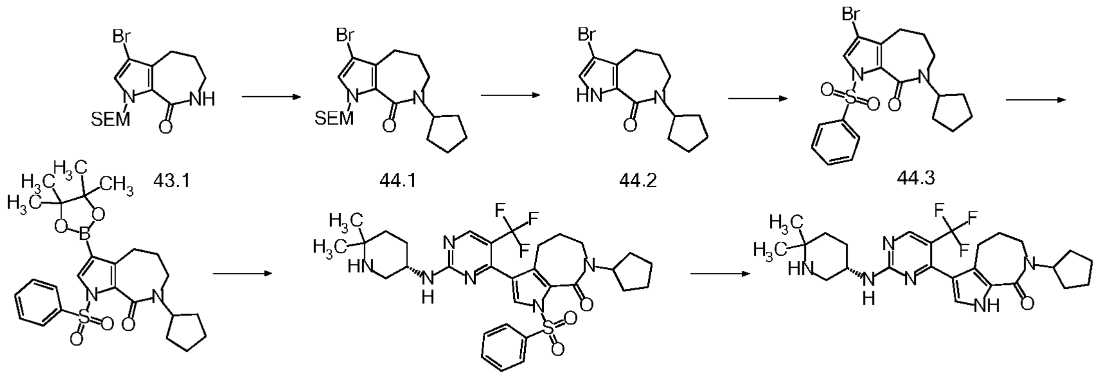

43.1 44.1 44.2 44.3

44.4 44.5 CDK7_1154

*Synthesis of compound **44.1***

NaH (0.948 g, 23.7 mmol) at 0 °C was added to a solution of compound **43.1** (0.300 g, 0.790 mmol) in 15 ml of DMF, stirred for 10 minutes, bromocyclopentane (3.72 g, 23.7 mmol) was added, stirred at room temperature for 28 h. After standard treatment, the reaction product was isolated by column chromatography. 0.154 g (46%) of compound **44.1** was yielded.

*Synthesis of compound **44.3***

Compound **44.1** was dissolved in 5 ml of TFA and stirred at room temperature for 1 h. Volatile components were then removed in vacuo and the dry residue was dissolved in 5 ml of ethanol. 3 ml of 3M solution of NaOH was added, stirred at room temperature for 1 hour, volatile components were removed. After standard treatment, the product was purified by column chromatography. The residue containing compound **44.2** was then dissolved in 5 ml of DMF; NaH (0.029 g, 0.720 mmol) was added at 0 °C, stirred for 30 minutes; benzenesulfonyl chloride (0.134 g, 0.720 mmol) was then added and stirred at room temperature for 16 h. After standard treatment, the product was isolated using column chromatography. 0.054 g (34%) of compound **44.3** was yielded.

*Synthesis of compound **44.4***

Compound **44.4** was produced analogously to compound **30.2.**

*Synthesis of compound **44.5***

Compound **44.5** was produced analogously to compound **32.6** using pyrimidine **31.1** with a yield of 54%.

*Synthesis of compound **CDK7_1154***

Compound **CDK7_1154** was produced analogously to compound **CDK7_1122** with a yield of 12%.

The following compounds were produced in an analogous manner:

| No. | Compound | Initial compound | Stage, yield, % | | | | | |
|---|---|---|---|---|---|---|---|---|
| | | | 1 | 2 | 3 | 4 | 5 | 6 |
| 1 | **CDK7_1155** | 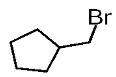 | 42 | 51 | 67 | 80 | 32 | 56 |
| 2 | **CDK7_1226** | 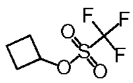 | 25 | 46 | | 4 | | |
| 3 | **CDK7_1227** | 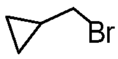 | 91 | 38 | 72 | 84 | 14 | |
| 4 | **CDK7_1229** | 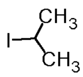 | 74 | 92 | 96 | 60 | 36 | |
| 5 | **CDK7_1259** | 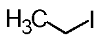 | 63 | | | 15 | | |
| 6 | **CDK7_1268** | 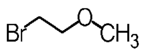 | 68 | 43 | 78 | 15 | | |

**Example 45.** Synthesis of compound **CDK7_1201, CDK7_1277.**

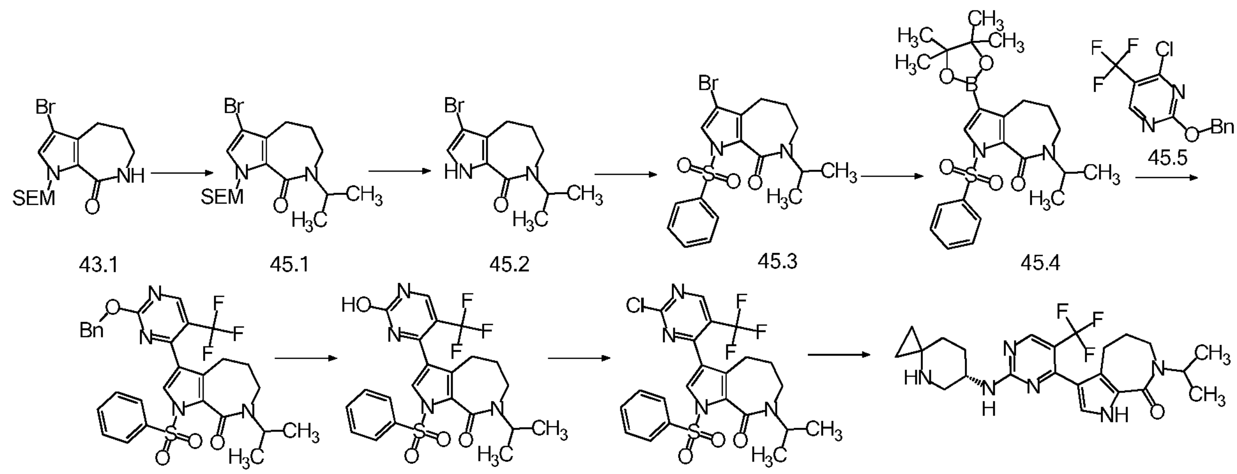

43.1 45.1 45.2 45.3 45.4 45.5

45.6 45.7 45.8 CDK7_1277

*Synthesis of compound **45.1***

Compound **45.1** was produced analogously to compound **44.1** using 2-iodopropane with a yield of 74%.

*Synthesis of compound **45.2***

Compound **45.2** was produced analogously to compound **44.2** with a yield of 92%.

*Synthesis of compound **45.3***

Compound **45.3** was produced analogously to compound **44.3** with a yield of 96%.

*Synthesis of compound **45.4***

Compound **45.4** was produced analogously to compound **44.4** with a yield of 60%.

*Synthesis of compound **45.6***

Compound **45.6** was produced analogously to compound **18.8** with a yield of 85%.

*Synthesis of compound **45.7***

Compound **45.6** (1.55 g, 2.65 mmol) was dissolved in 15 ml of 4 N solution of HCl in 1,4-dioxane, the reaction mass was stirred at 60 °C for 30 min. After standard treatment, the product was filtered off and volatile components were removed in vacuo. 1.15 g (88%) of compound **45.7** were yielded.

*Synthesis of compound **45.8***

Compound **45.7** (0.470 g, 0.900 mmol) was dissolved in 6 ml of $POCl_3$ and stirred at 90 °C for 30 min. After standard treatment, the product was isolated using column chromatography. 0.367 g (79%) of compound **45.8** was yielded.

*Synthesis of compound **CDK7_1277***

A suspension of compound **45.8** (0.534 g, 1.04 mmol), amine **40.1** (0.171 g, 1.35 mmol) and TEA (0.916 ml, 6.24 mmol) in 30 ml of absolute ethanol was stirred at room temperature for 60 h. 2 ml of 3 M solution of NaOH was added to the resulting mixture and mixed at room temperature for 2 h. After standard treatment, the product was isolated using preparative HPLC. 0.170 g (35%) of product **CDK7_1277** was yielded.

Compound **CDK7_1201** was produced analogously with a total yield of 3%.

**Example 46.** Synthesis of compound **CDK7_1276.**

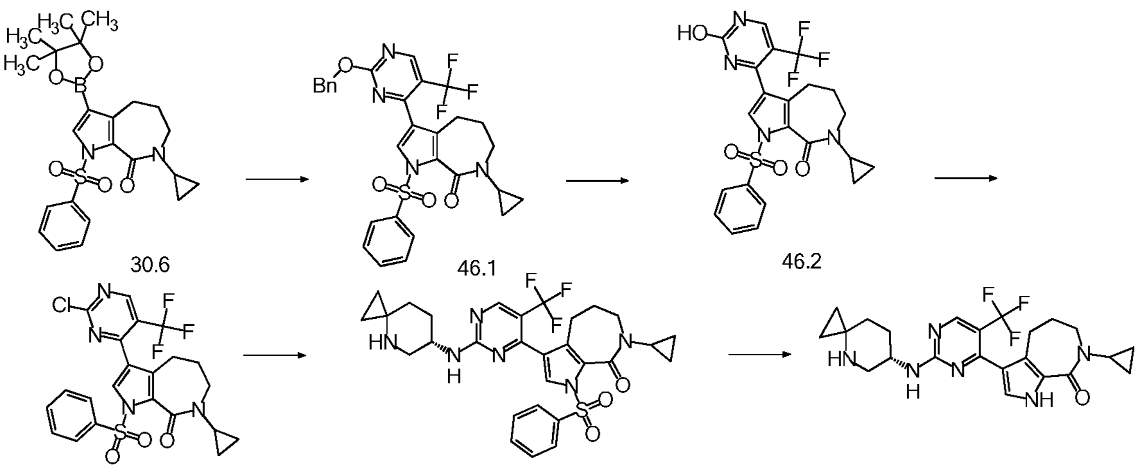

30.6

46.1

46.2

46.3

46.4

CDK7_1276

*Synthesis of compound **46.1***

Compound **46.1** was produced analogously to compound **18.8** using pyrimidine **45.5** with a yield of 75%.

*Synthesis of compound **46.2***

Compound **46.2** was produced analogously to compound **45.7** using pyrimidine **45.5** with a yield of 75%.

*Synthesis of compound **CDK7_1276***

Compound **46.2** (0.075 g, 0.140 mmol) was dissolved in 0.5 ml of $POCl_3$ and stirred at 90 °C for 30 min. After standard treatment, the product was isolated using column chromatography. The residue containing compound **46.3,** amine **40.1** (0.083 g, 1.70 mmol) and TEA (0.117 ml, 0.840 mmol) in 8 ml of absolute ethanol was mixed at room temperature for 72 h. 2 ml of 3 M solution of NaOH was added to the resulting mixture and stirred at room temperature for 4 h. After standard treatment, the product was isolated using preparative HPLC. 0.012 g of product **CDK7_1276** (15%) was yielded.

**Example 47.** Analysis of resulting compounds.

The purity and structure of the resulting compounds was confirmed by chromatography/mass spectrometry LC/MS-ESI and $^1$H NMR spectroscopy (**Tables** 3 and 4).

**Equipment data:**

**Table 1.** Chromatography/mass spectrometry

| Name | Manufacturer, country |
|---|---|
| Agilent Triple Quad LC/MS system: | Agilent, USA |
| Agilent 1200 autosampler | |
| Agilent 1200 thermostatted column | |
| Agilent 1200 degasser | |
| Agilent 1200 autosampler thermostat | |
| Agilent 6410 QQQ MS detector | |
| Agilent 1200 UV Detector | |
| Agilent 1200 pump | |

**Table 2.** NMR spectrometer

| Name | Manufacturer, country | Model, main characteristics |
|---|---|---|
| NMR spectrometer | Germany | AVANCE III, 400 MHz |

**Table 3.** Analytical data for exemplary compounds

| Code | $^1$H NMR (400 MHz, DMSO-$d_6$), δ |
|---|---|
| **CDK7_533** | $^1$H NMR (400 MHz, DMSO) δ 11.89 (s, 1H), 8.62 - 8.30 (m, 1H), 7.74 - 7.32 (m, 6H), 7.31 - 7.14 (m, 1H), 7.03 (s, 1H), 4.00 - 3.71 (m, 1H), 3.14 - 2.94 (m, 1H), 2.87 - 2.70 (m, 1H), 2.47 - 2.24 (m, 2H), 1.99 - 1.81 (m, 1H), 1.70 - 1.58 (m, 1H), 1.57 - 1.36 (m, 1H). |
| **CDK7_535** | $^1$H NMR (400 MHz, DMSO) δ 12.86 - 12.57 (m, 1H), 8.96 - 8.74 (m, 2H), 8.71 - 8.49 (m, 1H), 8.06 - 7.94 (m, 1H), 7.94 - 7.79 (m, 2H), 7.71 - 7.46 (m, 2H), 7.62 - 7.54 (m, 2H), 7.33 - 7.21 (m, 1H), 4.32 - 4.11 (m, 1H), 3.44 - 3.12 (m, 3H), 2.91 - 2.73 (m, 2H), 2.05 - 1.83 (m, 2H), 1.78 - 1.50 (m, 2H). |
| **CDK7_536** | $^1$H NMR (400 MHz, DMSO) δ 12.16 - 11.80 (m, 1H), 8.53 and 8.47 (2s, 1H, rotamers), 7.68 - 7.55 (m, 1H), 7.48 and 7.42 (2s, 1H, rotamers), 7.05 (s, 1H), 3.91 - 3.78 (m, 1H), 3.22 - 2.94 (m, 6H), 2.85 - 2.71 (m, 1H), 2.47 - 2.36 (m, 2H), 1.89 (s, 2H), 1.69 - 1.55 (m, 1H), 1.53 - 1.41 (m, 2H). |

(continued)

| Code | $^1$H NMR (400 MHz, DMSO-$d_6$), δ |
|---|---|
| **CDK7_537** | $^1$H NMR (400 MHz, DMSO) δ 12.28 - 11.80 (m, 1H), 8.64 - 8.43 (m, 1H), 8.34 (s, 1H), 8.15 - 7.58 (m, 2H), 7.54 - 7.24 (m, 2H), 7.20 - 6.94 (m, 1H), 4.10 - 3.95 (m, 1H), 3.72 - 3.52 (m, 1H), 3.30 - 2.86 (m, 2H), 2.68 - 2.58 (m, 1H), 1.97 - 1.84 (m, 1H), 1.79 - 1.45 (m, 3H). |
| **CDK7_538** | $^1$H NMR (400 MHz, DMSO) δ 12.52 - 12.09 (m, 1H), 10.47 and 10.07 (2s, 1H, rotamers), 8.61 - 8.45 (m, 1H), 8.33 (s, 1H), 7.83 - 7.56 (m, 5H), 7.38 - 7.31 (m, 2H), 7.11 - 7.03 (m, 1H), 4.20 - 4.05 (m, 1H), 3.34 - 3.07 (m, 1H), 3.09 - 2.85 (m, 1H), 2.71 - 2.58 (m, 2H), 2.00 - 1.86 (m, 1H), 1.80 - 1.45 (m, 3H). |
| **CDK7_539** | $^1$H NMR (400 MHz, DMSO) δ 12.37 - 12.12 (m, 1H), 11.24 - 10.88 (m, 1H), 9.65 - 8.85 (m, 2H), 8.66 - 8.50 (m, 1H), 8.07 - 7.89 (m, 1H), 7.65 - 7.58 and 7.51 - 7.44 (2m, 1H, rotamers), 7.15 - 7.01 (m, 1H), 4.55 - 4.40 (m, 2H), 4.40 - 4.17 (m, 1H), 3.54 - 3.31 (m, 5H), 3.25 - 3.04 (m, 3H), 2.98 - 2.69 (m, 5H), 2.04 - 1.51 (m, 4H). |
| **CDK7_548** | 1H NMR (400 MHz, DMSO) δ 10.49 - 9.94 (m, 1H), 9.33 - 8.84 (m, 2H), 8.80 - 8.44 (m, 1H), 8.43 - 8.24 (m, 1H), 8.00 - 7.73 (m, 1H), 7.22 - 6.71 (m, 3H), 3.96 - 3.81 (m, 1H), 3.36 - 2.73 (m, 4H), 2.01 - 1.43 (m, 4H). |
| **CDK7_735** | $^1$H NMR (400 MHz, DMSO) δ 11.25 and 10.73 (2s, 1H, rotamers), 9.49 - 8.94 (m, 2H), 8.58 - 8.27 (m, 2H), 7.86 - 7.48 (m, 2H), 7.34 - 7.13 (m, 1H), 4.27 - 4.01 (m, 1H), 3.74 - 3.43 (m, 1H), 3.37 - 3.22 (m, 1H), 3.19 - 3.07 (m, 1H), 3.00 - 2.73 (m, 2H) 2.05 - 1.91 (m, 1H), 1.91 - 1.82 (m, 1H), 1.82 - 1.62 (m, 7H), 1.61 - 1.43 (m, 1H). |
| **CDK7_754** | $^1$H NMR (400 MHz, DMSO) δ 9.36 - 8.71 (m, 2H), 8.63 - 8.46 (m, 1H), 8.35 - 8.23 and 8.01 - 7.82 (2m, 1H, rotamers), 7.54 - 7.39 (m, 2H), 7.35 - 7.16 (m, 3H), 4.28 - 4.13 (m, 1H), 3.33 - 2.93 (m, 2H), 2.98 - 2.61 (m, 2H), 1.97 - 1.28 (m, 4H). |
| **CDK7_766** | $^1$H NMR (400 MHz, DMSO) δ 8.56 and 8.47 (2s, 1H, rotamers), 7.88 - 7.80 (m, 1H), 7.33 - 7.18 (m, 5H), 4.03 - 3.95 (m, 2H), 3.92 - 3.79 (m, 1H), 3.06 - 2.94 (m, 1H), 2.86 - 2.77 (m, 1H), 2.46 - 2.36 (m, 2H), 1.91 - 1.76 (m, 1H), 1.68 - 1.55 (m, 1H), 1.50 - 1.32 (m, 2H). |
| **CDK7_809** | $^1$H NMR (400 MHz, DMSO) δ 9.30 - 9.09 (m, 1H), 8.93 -8.81 (m, 1H), 8.56 and 8.44 (2d, *J* = 7.7 Hz, 1H, rotamers), 7.82 - 7.75 (m, 1H), 7.69 - 7.61 (m, 1H), 7.56 - 7.45 (m, 1H), 7.32 - 7.15 (m, 2H), 6.77 (s, 1H), 4.35 - 4.06 (m, 1H), 3.42 - 3.30 (m, 1H), 3.22 - 3.07 (m, 1H), 2.92 - 2.76 (m, 2H), 2.05 - 1.97 (m, 1H), 1.92 - 1.86 (m, 1H), 1.79 - 1.54 (m, 2H). |
| **CDK7_810** | $^1$H NMR (400 MHz, DMSO) δ 9.04 - 8.93 (2s, 1H, rotamers), 8.70 - 8.60 (m, 2H), 8.49 - 8.41 (m, 1H), 7.82 - 7.73 (m, 1H), 7.73 - 7.64 (m, 1H), 7.41 - 7.30 (m, 2H), 4.34 - 4.98 (m, 1H), 3.43 - 3.35 (m, 2H), 3.15 (br s, 1H), 2.84 (br s, 2H), 2.07 - 1.84 (m, 2H), 1.74 - 1.54 (m, 2H). |
| **CDK7_811** | $^1$H NMR (400 MHz, DMSO) δ 8.96 and 8.92 (2s, 1H, rotamers), 8.56 and 8.47 (2d, *J* = 8.0 Hz, 1H, rotamers), 8.39 and 8.18 (2d, *J* = 8.4, 1H), 8.28 - 8.22 (m, 2H), 7.75 (q, *J* = 7.0 Hz, 1H), 7.64 - 7.58 (m, 1H), 4.09 - 3.96 (m, 1H), 3.19 - 3.10 (m, 2H), 2.94 - 2.82 (m, 2H), 2.03 - 1.95 (m, 1H), 1.78 - 1.64 (m, 1H), 1.60 - 1.38 (m, 2H). |
| **CDK7_814** | $^1$H NMR (400 MHz, DMSO) δ 12.12 (br.s, 1H), 8.59 - 8.44 (m, 1H), 7.80 - 7.62 (m, 1H), 7.34 - 7.27 (m, 1H), 7.24 - 7.15 (m, 1H), 3.97 - 3.86 (m, 1H), 3.42 - 3.34 (m, 2H), 3.13 - 3.06 (m, 1H), 2.99 - 2.93 (m, 2H), 2.92 - 2.85 (m, 1H), 2.57 - 2.51 (m, 2H), 1.95 - 1.86 (m, 1H), 1.73 - 1.64 (m, 1H), 1.54 - 1.38 (m, 2H). |
| **CDK7_829** | $^1$H NMR (400 MHz, DMSO) δ 8.45 and 8.38 (2s, 1H, rotamers), 7.78 and 7.64 (2d, *J* = 7.3 Hz, 1H, rotamers), 6.84 - 6.74 (m, 1H), 6.74 - 6.65 (m, 1H), 6.45 (t, *J* = 7.7 Hz, 1H), 4.92 and 4.80 (2s, 1H, rotamers), 3.18 - 3.11 (m, 2H), 2.97 - 2.88 (m, 1H), 2.85 - 2.56 (m, 4H), 2.39 - 2.23 (m, 2H), 1.83 - 1.76 (m, 2H), 1.75 - 1.51 (m, 1H), 1.49 - 0.82 (m, 4H). |
| **CDK7_830** | $^1$H NMR (400 MHz, DMSO) δ 8.88 - 8.80 (m, 1H), 8.54 and 8.47 (2s, 1H, rotamers), 8.46 - 8.42 (m, 1H), 8.27 (s, 1H), 7.98 - 7.90 (m, 1H), 7.74 and 7.43 (2s, 1H, rotamers), 7.69 - 7.60 (m, 2H), 7.60 - 7.52 (m, 1H), 3.87 - 3.79 (m, 1H), 2.99 - 2.76 (m, 1H), 2.41 - 2.25 (m, 2H), 2.19 - 2.07 (m, 1H), 1.78 and 1.51 (m, 1H), 1.40 - 1.23 (m, 1H), 1.18 - 0.88 (m, 1H), 0.83 - 0.50 (m, 1H). |

(continued)

| Code | $^1$H NMR (400 MHz, DMSO-$d_6$), δ |
|---|---|
| **CDK7_834** | $^1$H NMR (400 MHz, DMSO) δ 12.47 (br s, 1H), 8.66 - 8.50 (m, 1H), 8.38 (s, 1H), 8.05 - 7.70 (m, 2H), 7.68 - 7.57 (m, 1H), 7.52 (d, *J* = 3.6 Hz, 1H), 7.22 (d, *J* = 3.6 Hz, 1H), 4.22 and 3.95 (m, 1H), 3.45 - 2.87 (m, 2H), 2.73 - 2.55 (m, 2H), 2.04 - 1.85 (m, 1H), 1.84 - 1.64 (m, 2H), 1.64 - 1.44 (m, 1H). |
| **CDK7_835** | $^1$H NMR (400 MHz, DMSO) δ 12.46 - 12.04 (m, 1H), 10.72 - 10.27 (2s, 1H, rotamers), 8.61 - 8.49 (m, 1H), 8.36 (s, 1H), 7.96 (s, 1H), 7.84 - 7.63 (m, 1H), 7.63 -7.43 (m, 3H), 4.19 - 4.05 (m, 1H), 4.04 -3.93 (m, 1H), 3.83 (s, 3H), 3.17 - 3.09 (m, 1H), 3.08 - 3.00 (m, 1H), 2.98 - 2.85 (m, 1H), 2.03 - 1.85 (m, 1H), 1.83 - 1.45 (m, 3H). |
| **CDK7_836** | $^1$H NMR (400 MHz, DMSO) δ 12.74 - 12.07 (m, 1H), 10.82 and 10.39 (2s, 1H, rotamers), 8.62 - 8.51 (m, 1H), 8.49 - 8.44 (m, 2H), 8.33 (s, 1H), 7.89 - 7.49 (m, 5H), 4.22 - 4.02 (m, 1H), 3.39 - 3.15 (m, 1H), 3.12 - 2.96 (m, 1H), 2.75 - 2.59 (m, 2H), 2.00 - 1.88 (m, 1H), 1.86 - 1.47 (m, 3H). |
| **CDK7_838** | $^1$H NMR (400 MHz, DMSO) δ 12.23 (m, 1H), 10.31 and 9.91 (2s, 1H, rotamers), 8.65 - 8.46 (m, 1H), 7.81 - 7.44 (m, 5H), 7.02 - 6.88 (m, 2H), 4.16 - 3.90 (m, 1H), 3.77 - 3.71 (m, 4H), 3.37 - 3.26 (m, 1H), 3.10 - 3.02 (m, 4H), 3.02 and 2.90 (2br s, 1H, rotamers), 2.70 - 2.53 (m, 2H), 2.00 - 1.87 (m, 1H), 1.81 - 1.44 (m, 3H). |
| **CDK7_843** | $^1$H NMR (400 MHz, DMSO) δ 8.60 - 8.47 (m, 1H), 7.86 - 7.62 (m, 1H), 7.39 - 7.32 (m, 1H), 7.30 - 7.22 (m, 1H), 3.99 - 3.87 (m, 4H), 3.40 - 3.31 (m, 2H), 3.13 - 3.05 (m, 1H), 2.97 - 2.84 (m, 3H), 2.57 - 2.51 (m, 2H), 1.95 - 1.86 (m, 1H), 1.75 - 1.64 (m, 1H), 1.54 - 1.40 (m, 2H). |
| **CDK7_845** | $^1$H NMR (400 MHz, DMSO) δ 8.68 - 8.46 (m, 1H), 7.96 - 7.75 (m, 1H), 7.62 (s, 1H), 7.37 - 7.25 (m, 1H), 7.22 - 6.94 (m, 1H), 4.03 - 3.93 (m, 1H), 3.55 (s, 3H), 3.17 - 3.08 (m, 1H), 2.95 - 2.83 (m, 1H), 2.58 - 2.52 (m, 4H), 2.08 - 1.87 (m, 1H), 1.81 - 1.62 (m, 1H), 1.60 - 1.40 (m, 2H). |
| **CDK7_846** | $^1$H NMR (400 MHz, DMSO) δ 12.25 - 11.89 (m, 1H), 8.54 (s, 1H), 8.49 and 8.22 (2s, 1H, rotamers), 7.81 - 7.60 (m, 1H), 7.48 and 7.24 (2s, 1H, rotamers), 7.40 (s, 1H), 4.16 - 3.93 (m, 1H), 3.32 - 3.25 and 3.17 - 3.10 (2m, 1H, rotamers), 3.08 - 2.89 (m, 1H), 2.84 - 2.69 (m, 4H), 2.69 - 2.56 (m, 2H), 2.03 - 1.83 (m, 1H), 1.81 - 1.43 (m, 3H). |
| **CDK7_847** | $^1$H NMR (400 MHz, DMSO) δ 12.27 (s, 1H), 9.37 and 8.92 (2s, 1H, rotamers), 8,64 - 8.45 (m, 1H), 7.87 - 7.65 (m, 1H), 7.62 - 7.36 (m, 2H), 4.06 (m, 3H), 3.35 - 3.27 and 3.18 - 3.09 (2m, 1H, rotamers), 3.08 - 2.99 and 2.96 - 2.89 (2m, 1H, rotamers), 2.71 - 2.53 (m, 3H), 1.97 - 1.88 (m, 1H), 1.81 - 1.46 (m, 3H). |
| **CDK7_848** | $^1$H NMR (400 MHz, DMSO) δ 12.96 - 12.21 (m, 2H), 8.67 - 8.49 (m, 1H), 8.08 - 7.48 (m, 3H), 7.37 - 7.22 (m, 1H), 3.93 - 3.73 (m, 1H), 3.07 - 2.82 (m, 1H), 2.71 - 2.54 (m, 1H), 2.02 - 1.71 (m, 2H), 1.68 - 1.52 (m, 1H), 1.50 - 1.10 (m, 10H). |
| **CDK7_849** | $^1$H NMR (400 MHz, DMSO) δ 12.26 - 11.93 (m, 1H), 8.63 - 8.45 (m, 1H), 7.85 - 7.67 (m, 1H), 7.57 and 7.43 (2s, 1H, rotamers), 7.02 (s, 1H), 4.07 - 3.93 (m, 2H), 3.75 - 3.69 (m, 4H), 3.67 - 3.62 (m, 4H), 3.25 - 3.09 (m, 2H), 3.03 - 2.87 (m, 1H), 2.01 - 1.86 (m, 1H), 1.82 - 1.66 (m, 1H), 1.63 - 1.42 (m, 2H). |
| **CDK7_850** | $^1$H NMR (400 MHz, DMSO) δ 12.21 - 11.97 (m, 1H), 8.68 - 8.52 (m, 2H), 8.20 - 8.10 (m, 1H), 7.94 - 7.84 (m, 1H), 7.62 and 7.43 (2s, 1H, rotamers), 7.39 - 7.28 (m, 1H), 4.28 - 4.15 (m, 2H), 4.06 - 3.96 (m, 2H), 3.45 - 3.33 (m, 3H), 3.28 - 3.17 (m, 1H), 2.95 - 2.76 (m, 2H), 2.03 - 1.86 (m, 2H), 1.81 - 1.41 (m, 6H). |
| **CDK7_851** | $^1$H NMR (400 MHz, DMSO) δ 12.13 - 11.85 (m, 1H), 8.58 - 8.45 (m, 1H), 8.31 (s, 1H), 8.30 - 8.25 and 8.03 - 7.96 (2m, 1H, rotamers), 7.75 - 7.68 and 7.68 - 7.58 (2m, 1H, rotamers), 7.51 - 7.27 (m, 2H), 4.09 - 3.91 (m, 1H), 3.74 - 3.65 (m, 1H), 3.32 - 3.05 (m, 1H), 3.04 - 2.84 (m, 1H), 1.95 - 1.61 (m, 8H), 1.60 - 1.44 (m, 2H), 1.43 - 1.15 (m, 5H). |
| **CDK7_888** | $^1$H NMR (400 MHz, DMSO) δ 8.51 (d, J = 8 Hz 1H), 8.47 - 8.41 (m, 1H), 8.27 - 8.23 (m, 1H), 7.71 (m, 1H), 7.62 (d, J = 8 Hz, 1H), 7.51 - 7.43 (2H, m), 4.02 - 3.90 (m, 1H), 3.22 - 3.14 (m, 1H), 3.01 - 2.93 (m, 1H), 2.88 - 2,77 (m, 1H), 2.61 - 2.55 (m, 1H), 1.88 - 1.80 (m, 1H), 1.79 - 1.70 (m, 6H), 1.68 - 1.62 (m, 1H), 1.57 - 1.39 (m, 2H). |

(continued)

| Code | $^1$H NMR (400 MHz, DMSO-$d_6$), δ |
|---|---|
| **CDK7_891** | $^1$H NMR (400 MHz, DMSO) δ 8.64 (s, 1H), 8.39 (s, 1H), 8.29 and 8.24 (2s, 1H, rotamers), 8.26 (s, 1H), 7.99 - 7.83 and 7.41 - 7.24 (2m, 1H, rotamers), 7.74 - 7.54 (m, 2H), 7.54 - 7.44 (m, 2H), 4.03 - 3.92 and 3.85 - 3.70 (2m, 1H, rotamers), 3.14 - 3.00 (m, 1H), 2.96 - 2.78 (m, 1H), 2.48 - 2.31 (m, 2H), 1.92 - 1.76 (m, 1H), 1.75 - 1.56 (m, 1H), 1.55 - 1.34 (m, 2H). |
| **CDK7_897** | $^1$H NMR (400 MHz, DMSO) δ 8.57 and 8.51 (2s, 1H, rotamers), 8.04 and 7.80 (2d, *J* = 7.4 Hz, 1H, rotamers), 7.94 - 7.84 (m, 1H), 7.64 (q, 9.0 Hz, 1H), 7.49 - 7.39 (m, 1H), 7.37 - 7.28 (m, 1H), 3.84 - 3.74 and 3.13 - 3.04 (2m, 1H, rotamers), 2.97 - 2.89 and 2.68 - 2.61 (m, 1H, rotamers), 2.78 - 2.70 (m, 1H), 2.36 - 2.25 (m, 2H), 1.86 - 1.63 (m, 1H), 1.63 - 1.50 (m, 7H), 1.50 - 1.27 (m, 2H), 1.27 - 0.99 (m, 1H). |
| **CDK7_898** | $^1$H NMR (400 MHz, DMSO) δ 10.13 - 9.96 (m, 1H), 8.21 and 8.15 (2s, 1H, rotamers), 8.09 and 7.94 (2s, 1H, rotamers), 7.82 - 7.67 (m, 1H), 7.35 and 7.01 (2d, *J* = 7.6 Hz, 1H, rotamers), 7.28 - 7.21 (m, 1H), 7.21 - 7.13 (m, 2H), 3.90 - 3.68 and 3.44 - 3.40 (2m, 1H, rotamers), 3.00 - 2.82 (m, 1H), 2.80 - 2.68 (m, 1H), 2.42 - 2.27 (m, 2H), 2.09 - 2.01 (m, 3H), 1.83 - 1.73 (m, 1H), 1.62 - 1.51 (m, 1H), 1.44 - 1.31 (m, 1H), 1.44 - 1.31 (m, 1H), 1.31 - 1.25 (m, 1H), 1.25 - 1.17 and 1.06 - 1.00 (2m, 1H, rotamers). |
| **CDK7_901** | $^1$H NMR (400 MHz, DMSO) δ 12.01 (br s, 1H), 8.90 - 8.72 (m, 1H), 8.42 - 8.27 (m, 1H), 7.62 - 7.48 (m, 1H), 7.45 - 7.32 (m, 1H), 7.21 - 7.08 (m, 2H), 6.49 (s, 1H), 4.17 - 4.04 (m, 1H), 3.98 - 3.88 (m, 1H), 3.34 - 3.22 (m, 1H), 3.09 - 2.93 (m, 1H), 2.80 - 2.57 (m, 2H), 2.04 - 1.71 (m, 2H), 1.67 - 1.42 (m, 2H). |
| **CDK7_903** | $^1$H NMR (400 MHz, DMSO) δ 10.65 and 10.39 (2s, 1H, rotamers), 8.32 - 8.11 (m, 2H), 7.54 and 7.32 (2d, *J* = 8.0 Hz, 1H, rotamers), 7.49 - 7.38 (m, 1H), 6.99 (d, *J* = 7.9 Hz, 1H), 4.00 - 3.66 (m, 1H), 3.13 - 2.99 (m, 1H), 2.93 - 2.77 (m, 1H), 2.45 (s, 2H), 2.38 (s, 3H), 1.96 - 1.86 (m, 1H), 1.79 - 1.60 (m, 7H), 1.48 - 1.38 (m, 2H). |
| **CDK7_904** | $^1$H NMR (400 MHz, DMSO) δ 10.58 and 10.34 (2s, 1H, rotamers), 8.45 - 8.11 (m, 2H), 7.48 and 7.22 (2d, *J* = 8.0 Hz, 1H, rotamers), 7.41 - 7.29 (m, 2H), 3.86 - 3.54 (m, 1H), 3.02 - 2.93 (m, 1H), 2.80 - 2.73 (m, 1H), 2.44 - 2.34 (m, 2H), 2.31 (s, 3H), 1.93 - 1.80 (m, 1H), 1.78 - 1.68 (m, 6H), 1.63 - 1.56 (m, 1H), 1.42 - 1.31 (m, 2H). |
| **CDK7_921** | $^1$H NMR (400 MHz, DMSO) δ 12.01 (br s, 1H), 8.79 - 8.64 (m, 1H), 8.33 - 8.20 (m, 1H), 8.10 - 7.94 (m, 1H), 7.73 - 7.64 (m, 1H), 7.58 - 7.47 (m, 1H), 7.33 - 7.17 (m, 2H), 3.92 - 3.64 (m, 1H), 3.11 - 2.94 (m, 1H), 2.83 - 2.66 (m, 1H), 2.45 - 2.32 (m, 2H), 1.92 - 1.89 (m, 2H), 1.69 - 1.52 (m, 1H), 1.52 - 1.21 (m, 2H). |
| **CDK7_929** | $^1$H NMR (400 MHz, DMSO) δ 12.15 (br s, 1H), 8.63 - 8.48 (m, 1H), 7.88 - 7.72 (m, 1H), 7.29 - 7.16 (m, 1H), 7.10 - 6.92 (m, 1H), 6.08 - 5.92 (m, 1H), 3.95 - 3.78 (m, 2H), 3.77 - 3.62 (m, 2H), 3.12 - 2.95 (m, 4H), 2.90 - 2.75 (m, 1H), 2.49 - 2.41 (m, 2H), 1.96 - 1.80 (m, 1H), 1.74 - 1.58 (m, 1H), 1.52 - 1.31 (m, 2H). |
| **CDK7_944** | $^1$H NMR (400 MHz, DMSO) δ 12.31 (br s, 1H), 8.58 - 8.46 (m, 1H), 7.80 and 7.60 (2d, 8.0 Hz, 1H, rotamers), 7.54 - 7.42 (m, 1H), 4.61 - 4.35 (m, 2H), 4.03 - 3.85 (m, 1H), 3.18 - 3.06 (m, 1H), 3.04 (s, 3H), 2.96 - 2.83 (m, 1H), 2.62 - 2.52 (m, 2H), 2.07 - 1.86 (m, 1H), 1.80 - 1.63 (m, 1H), 1.62 - 1.42 (m, 2H). |
| **CDK7_951** | $^1$H NMR (400 MHz, DMSO) δ 12.20 - 11.90 (m, 1H), 8.68 and 8.53 (2s, 1H, rotamers), 8.48 and 8.28 (2s, 1H, rotamers), 7.75 and 7.63 (2d, *J* = 7.0 Hz, 1H, rotamers), 7.47 and 7.27 (2s, 1H, rotamers), 7.41 (s, 1H), 4.14 - 3.91 (m, 1H), 3.36 - 3.24 and 3.18 - 3.06 (2m, 1H, rotamers), 3.06 - 2.86 (m, 1H), 2.85 - 2.72 (m, 1H), 2.69 - 2.51 (m, 2H), 1.99 - 1.82 (m, 1H), 1.81 - 1.44 (m, 3H), 0.74 - 0.62 (m, 2H), 0.55 (s, 2H). |
| **CDK7_953** | $^1$H NMR (400 MHz, DMSO) δ 11.64 - 11.41 (m, 1H), 8.62 - 8.46 (m, 1H), 7.87 - 7.70 (m, 1H), 7.12 - 6.99 (m, 1H), 3.93 - 3.83 (m, 1H), 3.48 - 3.40 (m, 2H), 3.12 - 2.99 (m, 4H), 2.96 - 2.80 (m, 4H), 2.49 - 2.40 (m, 2H), 2.00 - 1.85 (m, 3H), 1.70 - 1.61 (m, 1H), 1.50 - 1.38 (m, 2H). |
| **CDK7_959** | $^1$H NMR (400 MHz, DMSO) δ 8.68 - 8.46 (m, 1H), 8.32 - 8.18 (m, 2H), 7.92 - 7.83 (m, 1H), 7.83 - 7.78 (m, 1H), 7.65 (s, 1H), 7.50 - 7.34 (m, 1H), 7.26 and 7.11 (2d, *J* = 7.4 Hz, 1H, rotamers), 4.03 - 3.93 (m, 1H), 3.90 (s, 3H), 3.16 - 3.06 (m, 1H), 2.93 - 2.82 (m, 1H), 2.63 - 2.56 (m, 2H), 2.06 - 1.85 (m, 1H), 1.77 - 1.64 (m, 1H), 1.61 - 1.41 (m, 2H). |

(continued)

| Code | $^1$H NMR (400 MHz, DMSO-$d_6$), δ |
|---|---|
| **CDK7_966** | $^1$H NMR (400 MHz, DMSO) δ 8.63 and 8.56 (2s, 1H, rotamers), 8.32 (s, 1H), 8.06 (s, 1H), 7.89 - 7.70 (m, 2H), 4.15 - 3.92 (m, 1H), 3.62 (s, 3H), 3.05 (s, 3H), 2.68 - 2.54 (m, 2H), 2.01 - 1.87 (m, 2H), 1.83 - 1.67 (m, 1H), 1.63 - 1.45 (m, 3H). |
| **CDK7_1017** | $^1$H NMR (400 MHz, DMSO) δ 8.52 and 8.45 (m, 1H, rotamers), 7.67 - 7.20 (m, 2H), 6.84 (d, *J* = 7.6 Hz, 1H), 3.93 - 3.79 (m, 1H), 3.79 - 3.69 (m, 3H), 3.12 - 2.96 (m, 5H), 2.85 - 2.72 (m, 1H), 2.45 - 2.35 (m, 2H), 1.89 (s, 4H), 1.68 - 1.58 (m, 1H), 1.51 - 1.36 (m, 2H). |
| **CDK7_550** | $^1$H NMR (400 MHz, DMSO) δ 9.17 - 9.07, and 8.50 - 8.34 (2m, 1H, rotamers), 9.06 - 8.05 (m, 1H), 8.41 - 8.23 (m, 1H), 7.76 - 7.56 and 7.14 - 6.99 (2m, 1H, rotamers), 7.42 - 7.16 (m, 3H), 4.22 - 4.05 (m, 1H), 3.73 - 3.66 (m, 1H), 3.52 - 3.46 (m, 1H), 3.29 - 2.99 (m, 2H), 2.95 - 2.62 (m, 2H), 1.94 - 1.33 (m, 4H) |
| **CDK7_837** | 1H NMR (400 MHz, DMSO) δ 12.44 - 12.27 (m, 1H), 10.60 - 10.36 (m, 1H), 8.60 - 8.50 (m, 1H), 8.00 - 7.95 (m, 2H), 7.85 - 7.80 (m. 2H), 7.78 - 7.50 (m, 3H), 4.10 - 3.80 (m, 1H), 3.20 - 2.80 (m, 2H), 2.00 - 1.80 (m, 1H), 1.79 - 1.60 (m, 1H), 1.59 - 1.42 (m, 2H), 1.35 - 1.16 (m, 1H), 0.97 - 0.76 (m, 1H). |
| **CDK7_900** | $^1$H NMR (400 MHz, DMSO) δ 10.65 (s, 1H), 8.25 (s, 1H), 7.89 - 7.64 (m, 1H), 7.15 - 6.92 (m, 3H), 6.66 - 6.43 (m, 1H), 4.77 - 4.53 (m, 2H), 4.16 - 3.85 (m, 1H), 2.92 - 2.52 (m, 4H), 1.69 - 1.16 (m, 5H) |
| **CDK7_997** | 1H NMR (400 MHz, DMSO) δ 12.23 - 11.94 (m, 1H), 9.22 - 9.12 and 8.89 - 8.79 (2m, 1H, rotamers), 8.58 - 8.44 (m, 1H), 7.76 - 7.59 (m, 1H), 7.53 - 7.46 (m, 1H), 7.46 - 7.34 (m, 1H), 7.33 - 7.28 (m, 3H), 7.28 - 7.19 (m, 1H), 4.53 - 4.39 (m, 2H), 4.11 - 3.89 (m, 1H), 3.30 - 3.04 (m, 1H), 3.01 - 2.84 (m, 1H), 2.65 - 2.52 (m, 2H), 2.13 - 2.02 (m, 2H), 1.96 - 1.85 (m, 1H), 1.78 - 1.66 (m, 1H), 1.66 - 1.42 (m, 2H). |
| **CDK7_998** | 1H NMR (400 MHz, DMSO) δ 12.31 - 11.99 (m, 1H), 8.58 - 8.40 (m, 1H), 7.78 - 7.63 (m, 1H), 7.58 - 7.44 (m, 1H), 7.42 - 7.33 (m, 2H), 7.32 -7.24 (m, 3H), 7.15 - 6.90 (m, 1H), 4.89 - 4.63 (m, 2H), 4.01 - 3.83 (m, 2H), 3.99 - 2.99 (m, 4H), 2.96 - 2.81 (m, 2H), 2.64 - 2.52 (m, 1H), 2.00 - 1.58 (m, 2H), 1.56 - 1.39 (m, 2H). |
| **CDK7_999** | 1H NMR (400 MHz, DMSO) δ 8.64 - 8.51 (m, 1H), 7.89 - 7.80 (m, 1H), 7.72 and 7.59 (2s, 1H, rotamers), 7.37 - 7.29 (m, 1H), 4.27 - 4.19 (m, 3H), 4.16 - 3.94 (m, 1H), 3.32 - 3.11 (m, 1H), 3.07 - 2.88 (m, 1H), 2.69 - 2.53 (m, 2H), 2.00 - 1.88 (m, 2H), 1.81 - 1.69 (m, 1H), 1.64 - 1.47 (m, 2H). |
| **CDK7_1000** | 1H NMR (400 MHz, DMSO) δ 8.86 - 8.53 (m, 1H), 7.88 - 7.76 (m, 1H), 7.71 and 7.60 (2s, 1H, rotamers), 7.33 (s, 1H). 4.27 - 4.20 (m, 3H), 4.09 - 3.94 (m, 2H), 3.26 - 3.18 (m, 1H), 3.16 - 3.09 (m, 1H), 3.00 - 2.05 (m, 1H), 2.64 - 2.53 (m, 2H), 2.01 - 1.87 (m, 1H), 1.79 - 1.76 (m,1H), 1.59 - 1.44 (m, 2H). |
| **CDK7_1001** | 1H NMR (400 MHz, DMSO) δ 12.07 - 11.86 (m, 1H), 8.59 - 8.47 (m, 1H), 7.78 - 7.63 (m, 1H), 7.57 - 7.44 (m, 1H), 7.43 - 7.30 (m, 1H), 4.03 - 3.69 (m, 2H), 3.23 - 3.12 (m, 1H), 3.02 (s, 3H), 2.96 - 2.86 (m, 1H), 2.58 - 2.51 (m, 2H), 1.99 - 1.87 (m, 1H), 1.79 - 1.63 (m, 1H), 1.57 - 1.42 (m, 2H), 0.94 - 0.84 (m, 2H), 0.77 - 0.68 (m, 2H). |
| **CDK7_1003** | 1H NMR (400 MHz, DMSO) δ 12.53 - 12.44 (m, 1H), 8.58 - 8.49 (m, 1H), 7.76 - 7.69 (m, 1H), 7.67 - 7.54 (m, 3H), 7.39 - 7.30 (m, 2H), 3.99 - 3.94 (m, 3H), 3.92-3.82 (m, 1H), 3.14 - 3.00 (m, 1H), 2.86 - 2.76 (m, 1H), 2.46 - 2.39 (m, 2H), 1.97 - 1.92 (m, 1H), 1.69 - 1.59 (m, 1H), 1.54 - 1.35 (m, 2H). |
| **CDK7_991** | $^1$H NMR (400 MHz, DMSO) δ 11.42 (s, 1H), 8.55 - 8.44 (m, 1H), 8.28 (s, 1H), 7.73 - 7.55 (m, 1H), 7.10 - 6.94 (m, 2H), 2.91 - 2.78 (m, 8H), 1.88 - 1.74 (m, 1H), 1.71 - 1.60 (m, 1H), 1.52 - 1.36 (m, 1H). |
| **CDK7_924** | $^1$H NMR (400 MHz, DMSO) δ 8.76 - 8.51 (m, 1H), 7.95 - 7.78 (m, 1H), 7.69 (s, 1H), 7.35 - 7.00 (m, 2H), 4.01 - 3.83 (m, 1H), 3.15 - 3.05 (m, 1H), 2.97 - 2.76 (m, 1H), 2.51 - 2.55 (m, 2H), 2.32 (s, 3H), 2.13 (s, 3H), 2.02 - 1.85 (m, 1H), 1.81 - 1.60 (m, 1H), 1.57 - 1.33 (m, 2H). |

(continued)

| Code | $^1$H NMR (400 MHz, DMSO-$d_6$), δ |
|---|---|
| CDK7_955 | $^1$H NMR (400 MHz, DMSO) δ 11.92 - 11.46 (m, 1H), 8.66 - 8.47 (m, 1H), 7.99 (s, 1H), 7.90 - 7.76 (m, 1H), 7.63 (s, 1H), 7.24 - 7.15 (m, 1H), 4.01 - 3.89 (m, 1H), 3.85 - 3.72 (m, 5H), 3.19 - 3.07 (m, 1H), 3.04 - 2.86 (m, 3H), 2.62 - 2.52 (m, 2H), 2.15 - 2.00 (m, 2H), 1.97 - 1.84 (m, 1H), 1.77 - 1.64 (m, 1H), 1.57 - 1.39 (m, 2H). |
| CDK7_1019 | $^1$H NMR (400 MHz, DMSO) δ 8.59 - 8.41 (m, 1H), 7.69 - 7.50 (m, 1H), 7.49 - 7.34 (m, 1H), 7.05 (s, 1H), 4.07 - 3.86 (m, 1H), 2.91 - 2.74 (m, 1H), 2.65 - 2.51 (m, 2H), 2.16 (s, 3H), 1.96 - 1.74 (m, 8H), 1.73 - 1.62 (m, 1H), 1.60 - 1.44 (m, 1H), 1.37 - 1.21 (m, 1H). |
| CDK7_1020 | $^1$H NMR (400 MHz, DMSO) δ 12.46 (br.s, 1H), 8.61 - 8.48 (m, 1H), 7.90 - 7.75 (m, 1H), 7.66 and 7.53 (2s, 1H, rotamers), 7.32 - 7.23 (m, 1H), 4.31 - 4.24 (m, 2H), 4.08 - 3.96 (m, 1H), 3.22 - 3.12 (m, 1H), 3.01 - 2.91 (m, 1H), 2.68 - 2.55 (m, 2H), 1.97 - 1.87 (m, 1H), 1.80 - 1.68 (m, 1H), 1.62 - 1.46 (m, 2H), 1.30 (t, *J* = 7.0 Hz, 3H). |
| CDK7_1105 | $^1$H NMR (400 MHz, DMSO) δ 8.64 - 8.50 (m, 1H), 7.83 (d, J=7.4, 1H), 7.61 (s, 1H), 7.25 - 7.16 and 7.03 - 6.94 (2m, 1H, rotamers), 7.12 (s, 1H), 4.00 - 3.86 (m, 1H), 3.42 - 3.32 (m, 1H), 3.15 - 3.05 (m, 1H), 2.95 - 2.79 (m, 2H), 2.58 - 2.52 (m, 1H), 1.99 - 1.88 (m, 1H), 1.76 - 1.62 (m, 1H), 1.57 - 1.41 (m, 2H), 1.10 - 0.97 (m, 2H), 0.93 - 0.81 (m, 2H). |
| CDK7_1058 | 1H NMR (400 MHz, DMSO) δ 8.67 - 8.57 (m, 1H), 8.56 - 8.47 (m, 1H), 7.91 - 7.87 (m, 1H), 7.77 (s,2H), 7.64 - 7.57 (m, 1H), 7.51 and 7.37 (2d, J=7.7 Hz, rotamers, 1H), 4.00 - 3.84 (m, 1H), 3.17 - 3.01 (m, 2H), 2.89 - 2.81 (m, 1H), 2.03 - 1.86 (m, 1H), 1.74 - 1.63 (m, 1H), 2.00 - 1.37 (m, 2H), 1.24 (s, 1H), 0.90 - 0.74 (m, 1H). |
| CDK7_1176 | $^1$H NMR (400 MHz, DMSO) δ 12.00 - 11.67 (m, 1H), 8.65 - 8.52 (m, 1H), 8.49 - 8.42 (m, 1H), 7.90 - 7.84 (m, 1H), 7.83 - 7.78 (m, 1H), 7.76 - 7.72 (m, 1H), 7.24 - 7.15 (m, 2H), 4.18 - 4.04 (m, 2H), 4.01 - 3.89 (m, 1H), 3.19 - 3.07 (m, 1H), 3.04 - 2.86 (m, 4H), 2.63 - 2.52 (m, 2H), 2.13 - 2.02 (m, 2H), 1.96 - 1.83 (m, 1H), 1.77 - 1.65 (m, 1H), 1.56 - 1.42 (m, 2H). |
| CDK7_1187 | $^1$H NMR (400 MHz, DMSO) δ 11.60 - 11.40 (m, 1H), 8.61 - 8.45 (m, 1H), 8.31 (s, 1H, formate), 7.82 - 7.66 (m, 1H), 7.13 - 6.99 (m, 1H), 3.94 - 3.82 (m, 1H), 3.50 - 3.43 (s, 2H), 3.05 (s, 3H), 2.97 - 2.79 (m, 2H), 2.74 - 2.64 (m, 1H), 2.03 - 1.09 (m, 2H), 1.82 - 1.50 (m, 3H), 1.44 - 1.31 (m, 1H), 1.18 - 1.02 (m, 6H). |
| CDK7_1188 | $^1$H NMR (400 MHz, DMSO) δ 11.66 - 11.37 (m, 1H), 8.63 - 8.45 (m, 1H), 7.82 - 7.66 (m, 1H), 7.15 - 6.98 (m, 1H), 3.97 - 3.75 (m, 1H), 3.45 - 3.38 (m, 2H), 3.12 - 2.97 (m, 1H), 2.89 - 2.78 (m, 4H), 2.48 - 2.41 (m, 2H), 1.96 - 1.82 (m, 3H), 1.72 - 1.59 (m, 1H), 1.52 - 1.34 (m, 2H), 0.80 - 0.74 (m, 2H), 0.65 - 0.59 (m, 2H). |
| CDK7_1199 | $^1$H NMR (400 MHz, DMSO) δ 12.92 - 12.60 (m, 1H), 8.66 - 8.54 (m, 1H), 7.84 - 7.76 (m, 1H), 7.70 - 7.61 (m, 1H), 7.44 - 7.32 (m, 1H), 3.90 (s, 3H), 2.21 - 2.09 (m, 3H), 2.04 - 1.81 (m, 2H), 1.72 - 1.55 (m, 1H), 1.32 - 1.15 (m, 4H), 1.03 - 0.78 (m, 4H). |
| CDK7_1122 | $^1$H NMR (400 MHz, DMSO) δ 11.80 - 11.54 (m, 1H), 8.62 - 8.46 (m, 1H), 7.99 (s, 1H), 7.80 - 7.68 (m, 1H), 7.63 (s, 1H), 7.19 - 7.06 (m, 1H), 3.87 - 3.70 (m, 5H), 3.00 - 2.81 (m, 3H), 2.70 - 2.53 (m, 2H), 2.11 - 2.01 (m, 2H), 1.89 (s, 1H), 1.81 - 1.71 (m, 1H), 1.69 - 1.57 (m, 1H), 1.53 - 1.43 (m, 1H), 1.36 - 1.22 (m, 1H), 1.09 - 0.99 (m, 6H). |
| CDK7_1123 | $^1$H NMR (400 MHz, DMSO) δ 8.61 (m, 1H), 8.24 (s, 1H), 7.96 - 7.87 (m, 1H), 7.81 (s, 1H), 7.66 (s, 1H), 7.47 - 7.42 and 7.12 - 6.99 (m, 1H, rotamers), 7.41 - 7.35 and 7.30 - 7.23 (m, 1H, rotamers), 4.09 - 3.99 (m, 1H), 3.91 (s, 3H), 3.09 - 3.01 (m, 1H), 2.87 - 2.75 (m, 1H), 1.93 - 1.41 (m, 4H), 1.26 - 1.10 (m, 6H). |
| CDK7_1021 | $^1$H NMR (400 MHz, DMSO) δ 12.36 (bs, 1H), 8.62 - 8.43 (m, 1H), 7.91 - 7.69 (m, 1H), 7.08 (s, 1H), 3.95 - 3.59 (m, 3H), 3.12 (s, 3H), 3.08 - 2.95 (m, 1H), 2.88 - 2.64 (m, 3H), 2.44 - 2.31 (m, 2H), 1.96 - 1.78 (m, 1H), 1.73 - 1.58 (m, 1H), 1.54 - 1.32 (m, 2H). |
| CDK7_1052 | 1H NMR (400 MHz, DMSO) δ 8.65 - 8.55 (m, 1H), 8.08 - 7.97 (m, 1H), 7.86 - 7.76 (m, 1H), 7.71 - 7.62 (m, 1H), 7.61 - 7.52 (m, 1H), 7.33 - 7.26 and 7.16 - 7.08 (2m, 1 H, rotamers), 7.26 - 7.19 (m, 1H), 6.51 - 6.45 (m, 1H), 3.96 - 3.81 (m, 2H), 3.49 (s, 3H), 3.10 - 3.00 (m, 1H), 2.87 - 2.74 (m, 1H), 2.69 - 2.66 and 2.35 - 2.31 (2m, 1H, rotamers), 2.49 - 2.37 (m, 2H), 2.00 - 1.90 (m, 1H), 1.70 - 1.59 (m, 1H), 1.56 - 1.35 (m, 1H). |

(continued)

| Code | $^1$H NMR (400 MHz, DMSO-$d_6$), δ |
|---|---|
| **CDK7_1060** | $^1$H NMR (400 MHz, DMSO) δ 12.74 - 12.38 (m, 1H), 8.60 - 8.48 (m, 1H), 7.80 - 7.69 (m, 1H), 7.66 and 7.56 (s, 1H, rotamers), 7.29 (s, 1H), 4.08 - 3.85 (m, 1H), 3.20 - 3.06 (m, 1H), 2.97 - 2.82 (m, 2H), 2.65 (s, 3H), 2.58 - 2.51 (m, 2H), 2.08 - 1.83 (m, 1H), 1.81 - 1.60 (m, 1H), 1.60 - 1.41 (m, 1H). |
| **CDK7_1061** | 1H NMR (400 MHz, DMSO) δ 12.31 - 11.86 (m, 1H), 8.63 - 8.40 (m, 2H), 7.89 - 7.62 (m, 3H), 7.60 - 7.43 (m, 1H), 7.35 - 7.16 (m, 2H), 4.14 - 3.89 (m, 1H), 3.26 - 3.07 (m, 1H), 3.01 - 2.85 (m, 1H), 2.65 - 2.53 (m, 2H), 2.00 - 1.84 (m, 1H), 1.79 - 1.66 (m, 1H), 1.65 - 1.45 (m, 2H). |
| **CDK7_1062** | 1H NMR (400 MHz, DMSO) δ 12.56 - 12.03 (m, 1H), 8.85 - 8.71 (m, 2H), 8.61 - 8.44 (m, 1H), 8.31 (s, 1H), 7.76 - 7.66 (m, 1H), 7.65 - 7.53 (m, 1H), 7.50 (s, 1H) 7.35 - 7.20 (m, 1H), 4.10 - 3.83 (m, 1H), 3.22 - 3.03 (m, 1H), 2.98 - 2.82 (m, 1H), 2.61 - 2.51 (m, 2H), 2.01 - 1.82 (m, 1H), 1.79 - 1.61 (m, 1H), 1.59 - 1.39 (m, 2H). |
| **CDK7_1121** | 1H NMR (400 MHz, DMSO) δ 11.17 (m, 1H), 8.66 - 8.53 (m, 1H), 8.27 (s, 1H), 7.89 - 7.80 (m, 1H), 7.76 (s, 1H), 7.04 - 6.82 (m, 2H), 5.66 - 5.48 (m, 1H), 4.05 - 3.97 (m, 2H), 3.96 - 3.88 (m, 1H), 3.53 - 3.44 (m, 3H), 3.14 - 3.05 (m, 1H), 2.92 - 2.80 (m, 1H), 2.05 - 1.85 (m, 6H), 1.73 - 1.62 (m, 1H), 1.55 - 1.40 (m, 2H). |
| **CDK7_1102** | $^1$H NMR (400 MHz, DMSO) δ 8.62 - 8.51 (m, 1H), 7.84 - 7.43 (m, 3H), 4.07 - 3.99 (m, 1H), 3.98 - 3.89 (m, 1H), 3.26 - 3.09 (m, 2H), 2.99 - 2.87 (m, 1H), 2.62 - 2.52 (m, 2H), 1.99 - 1.88 (m, 1H), 1.79 - 1.66 (m, 1H), 1.59 - 1.46 (m, 2H), 1.37 - 1.18 (m, 4H). |
| **CDK7_1103** | 1H NMR (400 MHz, DMSO) δ 12.69 - 12.27 (m, 1H), 8.63 - 8.43 (m, 1H), 8.34 - 8.24 (s, 1H), 7.79 - 7.69 (m, 1H), 7.63 and 7.53 (2s, 1H, rotamers), 7.30 - 7.20 (m, 1H), 4.07 - 3.82 (m, 1H), 3.19 - 3.02 (m, 2H), 2.97 - 2.80 (m, 2H), 2.43 - 2.35 (m, 1H), 1.98 - 1.86 (m, 1H), 1,77 - 1.61 (m, 1H), 1.59 - 1.39 (m, 2H), 1.32 - 1.12 (m, 4H). |
| **CDK7_1124** | 1H NMR (400 MHz, DMSO) δ 12.97 - 11.91 (m, 1H), 8.63 - 8.46 (m, 1H), 8.28 (s, 1H), 7.80 - 7.70 (m, 1H), 7.67 - 7.52 (m, 3H), 7.32 (s, 1H), 7.30 - 7.18 (m, 2H), 4.09 - 3.90 (m, 4H), 3.26 - 3.08 (m, 1H), 2.99 - 2.84 (m, 1H), 2.61 - 2.53 (m, 2H), 2.03 - 1.86 (m, 1H), 1.80 - 1.64 (m, 1H), 1.63 - 1.41 (m, 2H). |
| **CDK7_1154** | $^1$H NMR (400 MHz, DMSO) δ 11.57 - 11.39 (m, 1H), 7.73 - 7.63 (m, 1H), 7.14 - 7.00 (m, 1H), 5.06 - 4.87 (m, 1H), 3.76 (br.s, 1H), 2.94 - 2.78 (m, 3H), 2.65 - 2.56 (m, 1H), 1.95 - 1.88 (m, 3H), 1.80 - 1.43 (m, 12H), 1.36 - 1.18 (m, 2H), 1.07 - 0.97 (m, 6H). |
| **CDK7_1155** | $^1$H NMR (400 MHz, DMSO) δ 11.56 - 11.36 (m, 1H), 8.59 - 8.44 (m, 1H), 7.74 - 7.61 (m, 1H), 7.13 - 6.98 (m, 1H), 3.82 - 3.68 (m, 1H), 3.48 - 3.39 (m, 3H), 2.95 - 2.80 (m, 3H), 2.66 - 2.56 (m, 1H), 2.26 - 2.15 (m, 1H), 1.99 - 1.92 (m, 2H), 1.81 - 1.42 (m, 10H), 1.35 - 1.16 (m, 3H), 1.08 - 0.98 (m, 6H). |
| **CDK7_1172** | $^1$H NMR (400 MHz, DMSO) δ 12.14 - 11.98 (m, 1H), 8.66 - 8.57 (m, 1H), 7.86 - 7.79 (m, 1H), 7.59 - 7.53 (m, 1H), 7.33 - 7.21 (m, 2H), 4.64 - 4.44 (m, 2H), 3.90 - 3.78 (m, 1H), 3.08 - 2.93(m, 3H), 2.87 -2.75 (m, 1H), 2.46 - 2.36 (m, 2H), 2.20 - 1.96 (m, 2H), 1.91 - 1.81 (m, 1H), 1.71 - 1.57 (m, 1H), 1.54 - 1.34 (m, 2H). |
| **CDK7_1177** | $^1$H NMR (400 MHz, DMSO) δ 11.89 - 11.68 (m, 1H), 8.66 - 8.51 (m, 1H), 7.87 - 7.76 (m, 1H), 7.23 - 7.13 (m, 1H), 7.09 (s, 1H), 6.81 (s, 1H), 3.98 - 3.82 (m, 1H), 3.81 - 3.66 (m, 2H), 3.13 - 2.93 (m, 5H), 2.90 - 2.79 (m, 2H), 2.17 - 2.03 (m, 2H), 1.97 - 1.83 (m, 1H), 1.77 - 1.57 (m, 1H), 1.56 - 1.37 (m, 2H), 1.19 - 1.03 (m, 2H). |
| **CDK7_1201** | $^1$H NMR (400 MHz, DMSO) δ 11.58 - 11.38 (m, 1H), 8.66 - 8.44 (m, 1H), 7.86 - 7.69 (m, 1H), 7.17 - 6.96 (m, 1H), 3.98 - 3.75 (m, 1H), 3.53 - 3.41 (m, 2H), 3.09 - 3.00 (m, 3H), 2.99 - 2.83 (m, 3H), 2.57 - 2.52 (m, 1H), 2.02 - 1.82 (m, 3H), 1.72 - 1.56 (m, 2H), 1.31 - 1.17 (m, 1H), 0.52 - 0.42 (m, 1H), 0.41 - 0.32 (m, 2H), 0.31 - 0.23 (m, 1H). |
| **CDK7_1225** | $^1$H NMR (400 MHz, DMSO) δ 11.60 - 11.40 (m, 1H), 8.61 - 8.43 (m, 1H), 7.76 - 7.62 (m, 1H), 7.16 - 6.94 (m, 1H), 3.75 (br.s, 1H), 3.47 - 3.38 (m, 2H), 2.89 - 2.73 (m, 4H), 2.65 - 2.56 (m, 1H), 1.94 - 1.83 (m, 1H), 1.81 - 1.70 (m, 1H), 1.68 - 1.55 (m, 1H), 1.53 - 1.42 (m, 1H), 1.37 - 1.19 (m, 2H), 1.06 - 0.99 (m, 6H), 0.81 - 0.72 (m, 2H), 0.69 - 0.57 (m, 2H). |

(continued)

| Code | $^1H$ NMR (400 MHz, DMSO-$d_6$), δ |
|---|---|
| **CDK7_1226** | $^1H$ NMR (400 MHz, DMSO) δ 11.59 - 11.40 (m, 1H), 8.58 - 8.47 (m, 1H), 7.74 - 7.62 (m, 1H), 7.12 - 7.01 (m, 1H), 5.91 - 5.75 (m, 1H), 5.15 - 5.06 (m, 1H), 5.05 - 4.98 (m, 1H), 3.76 (br.s, 1H), 3.59 - 3.51 (m, 2H), 3.49 - 3.41 (m, 2H), 2.94 - 2.78 (m, 3H), 2.66 - 2.56 (m, 1H), 2.36 - 2.26 (m, 2H), 2.00 - 1.91 (m, 2H), 1.81 - 1.71 (m, 1H), 1.70 - 1.56 (m, 1H), 1.54 - 1.45 (m, 1H), 1.37 - 1.20 (m, 1H), 1.09 - 0.99 (m, 6H). |
| **CDK7_1227** | $^1H$ NMR (400 MHz, DMSO) δ 11.60 - 11.41 (m, 1H), 8.61 - 8.41 (m, 1H), 7.79 - 7.64 (m, 1H), 7.16 - 7.00 (m, 1H), 3.90 - 3.81 (m, 1H), 3.55 - 3.47 (m, 3H), 2.97 - 2.63 (m, 5H), 2.05 - 1.90 (m, 2H), 1.81 - 1.45 (m, 3H), 1.42 - 1.27 (m, 1H), 1.12 - 0.94 (m, 7H), 0.51 - 0.37 (m, 2H), 0.32 - 0.17 (m, 2H). |
| **CDK7_1229** | $^1H$ NMR (400 MHz, DMSO) δ 11.59 - 11.40 (m, 1H), 8.58 - 8.46 (m, 1H), 7.74 - 7.62 (m, 1H), 7.12 - 7.00 (m, 1H), 4.91 - 4.82 (m, 1H), 3.82 - 3.71 (m, 1H), 2.97 - 2.79 (m, 4H), 2.66 - 2.53 (m, 2H), 1.93 - 1.86 (m, 1H), 1.81 - 1.71 (m, 1H), 1.69 - 1.58 (m, 1H), 1.53 - 1.45 (m, 1H), 1.38 - 1.20 (m, 2H), 1.16 - 1.08 (m, 6H), 1.07 - 0.99 (m, 6H). |
| **CDK7_1254** | $^1H$ NMR (400 MHz, DMSO) δ 11.54 - 11.53 (m, 1H), 8.61 - 8.47 (m, 1H), 7.82 - 7.70 (m, 1H), 7.14 - 6.98 (m, 1H), 3.97 - 3.84 (m, 1H), 3.81 - 3.72 (m, 1H), 3.12 - 3.04 (s, 4H), 2.98 - 2.80 (m, 3H), 2.49 - 2.43 (m, 2H), 2.16 - 2.05 (m, 1H), 1.97 - 1.82 (m, 2H), 1.75 - 1.62 (m, 1H), 1.53 - 1.36 (m, 2H), 1.19 - 1.06 (m, 3H). |
| **CDK7_1259** | $^1H$ NMR (400 MHz, DMSO) δ 11.60 - 11.36 (m, 1H), 8.57 - 8.48 (m, 1H), 7.73 - 7.65 (m, 1H), 7.10 - 7.02 (m, 1H), 3.76 (br.s, 1H), 3.54 - 3.47 (m, 2H), 3.46 - 3.39 (m, 2H), 2.93 - 2.80 (m, 3H), 2.67 - 2.52 (m, 2H), 1.99 - 1.91 (m, 1H), 1.80 - 1.70 (m, 1H), 1.68 - 1.57 (m, 1H), 1.53 - 1.44 (m, 1H), 1.36 - 1.20 (m, 1H), 1.13 - 0.99 (m, 9H). |
| **CDK7_1268** | $^1H$ NMR (400 MHz, DMSO) δ 11.58 - 11.42 (m, 1H), 8.59 - 8.49 (m, 1H), 7.79 - 7.66 (m, 1H), 7.14 - 6.98 (m, 1H), 3.96 - 3.84 (m, 1H), 3.67 - 3.61 (m, 2H), 3.50 - 3.46 (m, 3H), 3.27 (s, 3H), 3.02 - 2.91 and 2.76 - 2.63 (2m, 3H, rotamers), 2.90 - 2.81 (m, 2H), 1.99 - 1.88 (m, 2H), 1.84 - 1.74 (m, 1H), 1.74 - 1.51 (m, 2H), 1.48 - 1.33 (m, 1H), 1.15 - 1.06 (m, 6H). |
| **CDK7_1276** | $^1H$ NMR (400 MHz, DMSO) δ 11.71 - 11.32 (m, 1H), 8.66 - 8.45 (m, 1H), 7.92 - 7.67 (m, 1H), 7.14 - 7.00 (m, 1H), 3.93 - 3.72 (m, 1H), 3.49 - 3.38 (m, 2H), 2.99 - 2.77 (m, 4H), 2.61 - 2.52 (m, 1H), 1.98 - 1.82 (m, 3H), 1.72 - 1.56 (m, 2H), 1.33 - 1.17 (m, 1H), 0.82 - 0.72 (m, 2H), 0.66 - 0.58 (m, 2H), 0.51 - 0.42 (m, 1H), 0.40 - 0.31 (m, 2H), 0.31 - 0.23 (m, 1H). |
| **CDK7_1277** | $^1H$ NMR (400 MHz, DMSO) δ 11.59 - 11.40 (m, 1H), 8.60 - 8.45 (m, 1H), 7.86 - 7.68 (m, 1H), 7.13 - 7.00 (m, 1H), 4.95 - 4.78 (m, 1H), 3.96 - 3.77 (m, 1H), 3.02 - 2.80 (m, 3H), 2.57 - 2.51 (m, 2H), 1.99 - 1.81 (m, 3H), 1.74 - 1.56 (m, 2H), 1.33 - 1.19 (m, 2H), 1.15 - 1.06 (m, 6H), 0.51 - 0.42 (m, 1H), 0.40 - 0.32 (m, 2H), 0.31 - 0.23 (m, 1H). |

**Table 4.** Analytical data for exemplary compounds

| Code | ESI-MS $[M+H]^+$ | Code | ESI-MS $[M+H]^+$ |
|---|---|---|---|
| **CDK7_535** | 416.2 | **CDK7_997** | 445.3 |
| **CDK7_536** | 383.1 | **CDK7_998** | 459.3 |
| **CDK7_537** | 355.2 | **CDK7_999** | 394.3 |
| **CDK7_538** | 431.2 | **CDK7_1000** | 394.1 |
| **CDK7_539** | 438.2 | **CDK7_1001** | 409.3 |
| **CDK7_548** | 354.1 | **CDK7_1003** | 452.2 |
| **CDK7_735** | 414.2 | **CDK7_991** | 383.2 |
| **CDK7_754** | 339.2 | **CDK7_924** | 474.2 |
| **CDK7_766** | 337.2 | **CDK7_955** | 475.2 |
| **CDK7_809** | 362.2 | **CDK7_1105** | 419.2 |
| **CDK7_810** | 363.2 | **CDK7_1058** | 462.1 |

(continued)

| Code | ESI-MS $[M+H]^+$ | Code | ESI-MS $[M+H]^+$ |
|---|---|---|---|
| **CDK7_811** | 364.1 | **CDK7_1176** | 472.2 |
| **CDK7_829** | 394.2 | **CDK7_1187** | 437.2 |
| **CDK7_830** | 390.2 | **CDK7_1122** | 503.3 |
| **CDK7_834** | 438.2 | **CDK7_1123** | 487.3 |
| **CDK7_835** | 435.2 | **CDK7_1021** | 423.1 |
| **CDK7_836** | 432.2 | **CDK7_1052** | 486.1 |
| **CDK7_838** | 516.2 | **CDK7_1060** | 394.2 |
| **CDK7_845** | 393.2 | **CDK7_1061** | 389.1 |
| **CDK7_846** | 369.2 | **CDK7_1062** | 390.2 |
| **CDK7_847** | 437.2 | **CDK7_1121** | 463.2 |
| **CDK7_848** | 461.2 | **CDK7_1103** | 420.1 |
| **CDK7_849** | 425.2 | **CDK7_1124** | 442.1 |
| **CDK7_850** | 439.2 | **CDK7_533** | 387.9 |
| **CDK7_851** | 453.3 | **CDK7_814** | 381.1 |
| **CDK7_888** | 414.2 | **CDK7_843** | 395.2 |
| **CDK7_891** | 417.2 | **CDK7_1019** | 397.2 |
| **CDK7_897** | 415.2 | **CDK7_1020** | 384.2 |
| **CDK7_898** | 353.2 (-Ac) | **CDK7_1188** | 435.2 |
| **CDK7_901** | 390.2 | **CDK7_1199** | 485.2 |
| **CDK7_903** | 428.3 | **CDK7_1102** | 420.1 |
| **CDK7_904** | 428.3 | **CDK7_1154** | 491.3 |
| **CDK7_921** | 390.3 | **CDK7_1155** | 505.2 |
| **CDK7_929** | 407.2 | **CDK7_1172** | 478.1 |
| **CDK7_944** | 381.2 | **CDK7_1177** | 475.2 |
| **CDK7_951** | 395.2 | **CDK7_1201** | 435.3 |
| **CDK7_953** | 409.2 | **CDK7_1225** | 463.2 |
| **CDK7_959** | 459.2 | **CDK7_1226** | 477.2 |
| **CDK7_966** | 384.2 | **CDK7_1227** | 477.2 |
| **CDK7_1017** | 397.2 | **CDK7_1229** | 465.3 |
| **CDK7_550** | 353.2 | **CDK7_1254** | 423.2 |
| **CDK7_833** | 421.4 | **CDK7_1259** | 451.3 |
| **CDK7_837** | 456.2 | **CDK7_1268** | 481.3 |
| **CDK7_972** | 312.2 | **CDK7_1276** | 461.2 |
| **CDK7_900** | 393.2 | **CDK7_1277** | 463.2 |

**Example 48.** Inhibiting enzyme activity of CDK7-CycH, CDK2-CycA, CDK9-CycK, CDK13-CycK.

Measurements were carried out in a 5 μl reaction volume using a 384-well plate (Corning Inc., order no. 4513). The analyzed substances were titrated from 50 μM in increments of 4 at 11 concentration points in a normal strength kinase buffer A (Thermo Fisher Scientific Inc, cat. no. PV3189) supplemented with DTT (Sigma, orde. no. 646563-10X.5ML) and prepared with water (Panreac, order no. 701074). Kinase was diluted in an analogous buffer, mixed with test compounds in

plate wells and preincubated for 10 minutes at 25 °C. Following incubation, a mixture of reaction substrate (CDKtide (SignalChem, order no. C06-58) or Histone H1 (SignalChem, order no. H10-54N) or PDKtide (SignalChem, order no. P10-58) and ATP (SignalChem, order no.V915B) prepared on a normal strength kinase buffer A was added. 1% solution of dimethyl sulfoxide (DMSO) (Sigma-Aldrich, order no. D2438) in the reaction volume was used as a negative control. The final concentrations of all reaction components are shown in **Table 5.** The plate was centrifuged at 400 rcf (Eppendorf, 5804 R) for 1 minute and incubated for 40-60 minutes at 25 °C. The amount of ATP consumed in the kinase reaction was then measured using the ADP-Glo™ detection system. Luminescence signal was measured using Spark 20M tablet multifunction reader (Tecan Group Ltd, Switzerland). $IC_{50}$ value was calculated using Magellan 7.2 (Tecan Group Ltd, Switzerland) by approximating experimental points by four-parameter model with the optimization by Levenberg-Marquardt:

$$Y = D + \frac{A - D}{1 + \left(\frac{X}{C}\right)^{B}},$$

where A is the upper asymptote; D is the lower asymptote; C is $IC_{50}$, half-maximal inhibitory concentration of kinase, nM; B is the curvature (slope).

**Table 5.** Final concentrations of kinase reaction components.

| **Active kinase** | **CDK7/Cyclin H/MNAT1** | **CDK2/Cyclin A** | **CDK9/Cyclin K** | **CDK13/Cyclin K** |
|---|---|---|---|---|
| | 2.5 ng/μl | 0.225 ng/μl | 2.5 ng/μl | 5 ng/μl |
| **Substrate** | CDKtide | Histone H1 | PDKtide | CDKtide |
| | 0.05 mg/ml | 0.1 mg/ml | 0.2 mg/ml | 0.2 mg/ml |
| **ATP** | 10 μM | 50 μM | 50 μM | 10 μM |
| **DMSO** | - | 1% | 1% | 1% |
| **DTT** | 2000 μM | 50 μM | 50 μM | 50 μM |

**Table 6.** Results of biochemical test of inhibition of CDK7/Cyclin H/MNAT1, CDK2/Cyclin A, CDK9/Cyclin K kinase activities. $IC_{50}$ is shown as a mean value from several measurements, "A" for $IC_{50}$<5nM, "B" for 5nM<$IC_{50}$<50nM, "C" for 50nM<$IC_{50}$<200nM, and "D" for $IC_{50}$>200nM, "N/A" means that a given compound was not analyzed in the present test.

| **Compound number** | **CDK7/Cyclin H/ MNAT** | **CDK2/Cyclin A** | **CDK9/Cyclin K** | **CDK13/Cyclin K** |
|---|---|---|---|---|
| **CDK7_533** | C | D | D | N/A |
| **CDK7_536** | A | D | B | N/A |
| **CDK7_548** | B | D | B | N/A |
| **CDK7_538** | B | D | C | N/A |
| **CDK7_537** | B | D | B | N/A |
| **CDK7_539** | B | D | C | N/A |
| **CDK7_754** | B | D | N/A | N/A |
| **CDK7_550** | C | D | N/A | N/A |
| **CDK7_735** | A | D | B | N/A |
| **CDK7_535** | B | D | C | N/A |
| **CDK7_809** | B | D | N/A | N/A |
| **CDK7_810** | B | D | N/A | N/A |
| **CDK7_811** | B | D | N/A | N/A |
| **CDK7_845** | A | D | B | N/A |
| **CDK7_837** | C | D | D | N/A |

(continued)

| Compound number | CDK7/Cyclin H/ MNAT | CDK2/Cyclin A | CDK9/Cyclin K | CDK13/Cyclin K |
|---|---|---|---|---|
| CDK7_766 | C | D | N/A | N/A |
| CDK7_835 | B | D | B | N/A |
| CDK7_846 | B | D | B | N/A |
| CDK7_847 | B | D | B | N/A |
| CDK7_848 | B | D | B | N/A |
| CDK7_849 | A | D | C | N/A |
| CDK7_850 | B | D | C | N/A |
| CDK7_851 | B | D | C | N/A |
| CDK7_897 | N/A | D | N/A | N/A |
| CDK7_898 | B | D | N/A | N/A |
| CDK7_951 | A | D | B | N/A |
| CDK7_834 | B | D | B | N/A |
| CDK7_838 | B | D | C | N/A |
| CDK7_972 | B | D | C | N/A |
| CDK7_961 | A | D | C | N/A |
| CDK7_833 | B | D | C | N/A |
| CDK7_836 | B | D | C | N/A |
| CDK7_888 | B | D | N/A | N/A |
| CDK7_929 | A | D | B | N/A |
| CDK7_944 | A | D | B | N/A |
| CDK7_991 | D | N/A | N/A | N/A |
| CDK7_891 | B | D | N/A | N/A |
| CDK7_921 | B | N/A | N/A | N/A |
| CDK7_966 | B | D | D | N/A |
| CDK7_903 | B | D | D | N/A |
| CDK7_904 | A | D | B | N/A |
| CDK7_1017 | B | D | C | N/A |
| CDK7_959 | A | D | C | N/A |
| CDK7_997 | B | D | C | N/A |
| CDK7_998 | B | D | D | N/A |
| CDK7_999 | A | D | B | N/A |
| CDK7_953 | A | D | C | B |
| CDK71000 | A | D | B | B |
| CDK7_1001 | A | D | B | N/A |
| CDK7_1003 | B | D | C | N/A |
| CDK7_1021 | B | B | D | N/A |
| CDK7_1052 | A | D | D | N/A |
| CDK7_1060 | A | D | B | N/A |
| CDK7_1061 | B | N/A | B | N/A |

(continued)

| Compound number | CDK7/Cyclin H/ MNAT | CDK2/Cyclin A | CDK9/Cyclin K | CDK13/Cyclin K |
|---|---|---|---|---|
| CDK7_1062 | A | D | B | N/A |
| CDK7_1105 | A | D | B | N/A |
| CDK7_924 | A | D | D | N/A |
| CDK7_955 | B | D | D | N/A |
| CDK7_1122 | B | N/A | D | N/A |
| CDK7_1124 | B | D | D | N/A |
| CDK7_1123 | A | D | D | B |
| CDK7_1176 | B | N/A | N/A | N/A |
| CDK7_1187 | A | D | D | C |
| CDK7_1020 | A | D | B | N/A |
| CDK7_1034 | A | D | B | N/A |
| CDK7_1019 | B | D | D | N/A |
| CDK7_814 | A | D | C | N/A |
| CDK7_843 | B | D | D | N/A |
| CDK7_960 | A | D | B | N/A |
| CDK7_1058 | A | D | B | N/A |
| CDK7_1102 | B | D | B | N/A |
| CDK7_1103 | A | D | C | N/A |
| CDK7_524 | A | D | D | N/A |
| CDK7_1154 | A | D | D | N/A |
| CDK7_1155 | B | D | D | N/A |
| CDK7_1172 | A | D | D | N/A |
| CDK7_1177 | B | D | D | N/A |
| CDK7_1188 | A | D | D | N/A |
| CDK7_1199 | A | D | D | N/A |
| CDK7_1201 | A | D | C | C |
| CDK7_1225 | A | D | D | D |
| CDK7_1226 | A | D | D | N/A |
| CDK7_1227 | A | D | D | N/A |
| CDK7_1229 | A | D | D | D |
| CDK7_1236 | A | D | C | N/A |
| CDK7_1237 | N/A | N/A | N/A | N/A |
| CDK7_1254 | A | D | D | N/A |
| CDK7_1259 | A | D | D | N/A |
| CDK7_1268 | N/A | D | D | N/A |
| CDK7_1276 | A | D | D | C |
| CDK7_1277 | A | D | D | D |

**Table 7.** Results of biochemical test of inhibition of CDK7/Cyclin H/MNAT1, CDK2/Cyclin A, CDK9/Cyclin K, CDK13/Cyclin K kinase activities. $IC_{50}$ is shown as a mean value from the data of multiple measurement series:

| Compound number | CDK7/Cyclin H/ MNAT1 | CDK2/Cyclin A | CDK9/Cyclin K | CDK13/Cyclin K |
|---|---|---|---|---|
| | $IC_{50}$, nM | | | |
| **CDK7_924** | <5 | 7796 | 431 | N/A |
| **CDK7_953** | <5 | 5828 | 60 | 20 |
| **CDK7_961** | <5 | 3698 | 69 | N/A |
| **CDK7_959** | <5 | 5258 | 106 | N/A |
| **CDK7_1052** | <5 | 12396 | 479 | N/A |
| **CDK7_1123** | <5 | 35219 | 265 | 25 |
| **CDK7_1187** | <5 | 15651 | 182 | 81 |
| **CDK7_1199** | <5 | 3394 | 843 | N/A |
| **CDK7_1201** | <5 | 4496 | 112 | 65 |
| **CDK7_1225** | <5 | 13883 | 441 | 203 |
| **CDK7_1226** | 5.6 | 18646 | 760 | N/A |
| **CDK7_1229** | <5 | 22938 | 485 | 242 |
| **CDK7_1276** | <5 | 6224 | 296 | 152 |
| **CDK7_1277** | <5 | 14316 | 442 | 425 |

The results of these experiments confirm production of high-affinity CDK7 inhibitors selective for CDK9, CDK13 and CDK2.

**Example 49.** Antiproliferative activity against sensitive cell lines *in vitro.*

The antiproliferative activity of CDK7 inhibitors according to the present invention was evaluated in a cell test using continuous cell cultures: MDA-MB-468 (*triple negative breast cancer,* ATCC® HTB-132™) and NCI-H82 (small cell *lung cancer,* ATCC® HTB-175™) using a viable AlamarBlue dye (Thermo Fisher Scientific Inc, order no. DAL1100). The cells were grown in DMEM (PanEco, order no. C330π) and RPMI-1640 (PanEco, order no. C330π), respectively. The basal growth medium was enriched with L-glutamine at a concentration of 2 mM (PanEco, order no. Φ032) and 10% fetal bovine serum (Gibco, order no. 16140-071). The night before measurement, MDA-MB-468 adherent cells were detached from plastic with a solution of trypsin-EDTA (PanEco, order no. П039π), the solution was then inactivated by a DMEM complete medium, centrifuged at 1000 RPM (Eppendorf, 5804 R), a suspension of cells was prepared in a DMEM complete medium, introduced into 96-well culture plates (Corning Inc, order no. 3599) at 8×10^3 cells in 100 μl of medium per well. The next day, test compounds were dissolved in DMSO and diluted with a DMEM complete medium to a final concentration ranging from 50 μM to 10 pM. Diluted compounds in a volume of 50 μl were introduced into the plate wells with cells in triplicates (the final concentration of DMSO was no more than 1%) and incubated at 37 °C in an incubator with 5% $CO_2$ for 96 hours. NCI-H82 suspension cell line was prepared on the day of the analysis. 10×10^3 cells in 100 μl of an RPMI-1640 complete medium were added to each well of a 96-well plate. The procedure for sample dilution is as described above. Cells with test compounds were incubated at 37 °C in an incubator with 5% $CO_2$ for 120 hours. Following incubation, 15 ml of AlamarBlue reagent (Thermo Fisher Scientific Inc., order no. DAL1 100) was added to the wells, the contents of the plates were stirred in an orbital shaker (Biosan, Latvia), then further incubated for 5.5-6 hours at 37 °C in an incubator with 5% $CO_2$. The number of viable cells was detected using the Infinite M200Pro microplate spectrophotometer (Tecan Group Ltd, Switzerland) by way of measuring a fluorescent signal at an excitation wavelength (λEx) of 540 nm and an emission wavelength (λEm) of 590 nm.

$IC_{50}$ value was calculated using the Magellan software (Tecan Group Ltd, Switzerland) by approximating experimental points by four-parameter model with the optimization by Levenberg-Marquardt:

**Table 8.** Results of antiproliferative assay on MDA-MB-468 and NCI-H82 cell lines. $IC_{50}$ is shown as a mean value from several measurements, "A" for $IC_{50}$<10nM, "B" for 10nM<$IC_{50}$<100nM, "C" for 100nM<$IC_{50}$<1000nM, and "D" for $IC_{50}$>1000nM, "N/A" means that a given compound was not analyzed in the present test.

| Compound number | MDA-MB-468 | NCI-H82 | Compound number | MDA-MB-468 | NCI-H82 |
|---|---|---|---|---|---|
| **CDK7_533** | C | N/A | **CDK7_891** | C | N/A |
| **CDK7_536** | B | N/A | **CDK7_966** | C | N/A |
| **CDK7_548** | B | N/A | **CDK7_904** | C | N/A |
| **CDK7_538** | C | N/A | **CDK7_1017** | C | D |
| **CDK7_537** | C | N/A | **CDK7_959** | A | A |
| **CDK7_539** | C | N/A | **CDK7_924** | A | B |
| **CDK7_754** | D | N/A | **CDK7_953** | A | A |
| **CDK7_550** | D | N/A | **CDK7_1052** | C | C |
| **CDK7_735** | C | N/A | **CDK7_1060** | B | B |
| **CDK7_535** | C | N/A | **CDK7_1061** | B | B |
| **CDK7_809** | D | N/A | **CDK7 1062** | B | B |
| **CDK7_810** | D | N/A | **CDK7_1105** | A | B |
| **CDK7_811** | C | N/A | **CDK7 955** | C | D |
| **CDK7_845** | A | A | **CDK7 1122** | C | N/A |
| **CDK7_829** | D | N/A | **CDK7_1123** | A | B |
| **CDK7_830** | D | N/A | **CDK7 1176** | C | N/A |
| **CDK7_837** | C | N/A | **CDK7_1187** | A | A |
| **CDK7_766** | D | N/A | **CDK7_1020** | B | B |
| **CDK7_835** | C | N/A | **CDK7_1034** | A | A |
| **CDK7_846** | B | N/A | **CDK7_814** | B | B |
| **CDK7_847** | B | N/A | **CDK7_960** | B | B |
| **CDK7_848** | D | N/A | **CDK7_1058** | A | A |
| **CDK7_849** | B | N/A | **CDK7_1102** | B | B |
| **CDK7_850** | C | N/A | **CDK7_1103** | B | B |
| **CDK7_851** | D | N/A | **CDK7_524** | A | A |
| **CDK7_898** | C | N/A | **CDK7_1154** | B | B |
| **CDK7_951** | B | N/A | **CDK7_1155** | B | B |
| **CDK7_834** | B | N/A | **CDK7_1172** | B | B |
| **CDK7_838** | C | C | **CDK7_1188** | A | A |
| **CDK7_972** | C | N/A | **CDK7_1199** | A | A |
| **CDK7_997** | C | N/A | **CDK7_1201** | A | A |
| **CDK7_998** | D | N/A | **CDK7_1225** | B | B |
| **CDK7_999** | B | C | **CDK7_1226** | B | B |
| **CDK7_1000** | B | B | **CDK7_1227** | B | B |
| **CDK7_1001** | C | N/A | **CDK7_1229** | B | B |
| **CDK7_1003** | C | N/A | **CDK7_1236** | B | B |
| **CDK7_961** | A | A | **CDK7_1254** | B | B |
| **CDK7_833** | C | N/A | **CDK7_1259** | B | B |

(continued)

| Compound number | MDA-MB-468 | NCI-H82 | Compound number | MDA-MB-468 | NCI-H82 |
|---|---|---|---|---|---|
| **CDK7_836** | C | N/A | **CDK7_1268** | B | B |
| **CDK7_888** | D | N/A | **CDK7_1276** | A | A |
| **CDK7_929** | A | A | **CDK7_1277** | A | A |
| **CDK7_944** | A | N/A | | | |

Results of these experiments confirm production of inhibitors capable of stopping the growth of triple-negative breast cancer cells and small cell lung cancer cells in nM concentrations.

**Example 50.** General cytotoxicity against primary HepaRG liver cells *in vitro.*

General cytotoxicity of CDK7 inhibitors according to the present invention was investigated using primary differentiated HepaRG cells (Biopredic Int, order no. HPR101). A week before the measurement, the cells were thawed and maintained in Basal hepatic cell medium (Biopredic Int, order no. MII,700079) supplemented with HepaRG maintenance/metabolism medium supplement with antibiotics (Biopredic Int, order no. ADD620C). The medium in vials with cells was changed every 2-3 days. The night before the test, the cells were plated in wells of 96-well plates (Corning Inc., order no. 3599) at 25×10^3 cells in 100 μl of medium per well. The next day, test compounds were introduced into plate wells with cells, untitrated at a concentration ranging from 200 μM to 0.2 nM, and incubated for 72 hours. Cell viability was assessed as described above.

**Table 9.** Results of general toxicity investigation on primary HepaRG cells. $CC_{50}$ is shown as a mean value from several measurements, "A" for $CC_{50}$>2000nM, "B" for 2000nM>$CC_{50}$>500nM, "C" for 500nM>$CC_{50}$>100nM, and "D" for $CC_{50}$<100nM, "N/A" means that a given compound was not analyzed in the present test.

| Compound number | HepaRG | Compound number | HepaRG | Compound number | HepaRG |
|---|---|---|---|---|---|
| **CDK7_533** | B | **CDK7_997** | B | **CDK7_1187** | A |
| **CDK7_536** | B | **CDK7_998** | A | **CDK7_829** | A |
| **CDK7_548** | B | **CDK7_999** | B | **CDK7_830** | A |
| **CDK7_538** | B | **CDK7_1000** | C | **CDK7_900** | A |
| **CDK7_537** | C | **CDK7_1001** | B | **CDK7_1020** | A |
| **CDK7_539** | A | **CDK7_1003** | B | **CDK7_1034** | C |
| **CDK7_754** | A | **CDK7_961** | C | **CDK7_1019** | A |
| **CDK7_550** | A | **CDK7_833** | B | **CDK7_814** | B |
| **CDK7_735** | A | **CDK7_836** | B | **CDK7_960** | C |
| **CDK7_535** | A | **CDK7_888** | A | **CDK7_1058** | B |
| **CDK7_809** | A | **CDK7_900** | A | **CDK7_1102** | B |
| **CDK7_810** | A | **CDK7_929** | C | **CDK7_1103** | C |
| **CDK7_811** | B | **CDK7_944** | D | **CDK7_1121** | A |
| **CDK7_845** | C | **CDK7_966** | A | **CDK7_524** | A |
| **CDK7_829** | A | **CDK7_904** | A | **CDK7_1154** | A |
| **CDK7_830** | A | **CDK71017** | A | **CDK7_1155** | A |
| **CDK7_837** | B | **CDK7_959** | A | **CDK7 1172** | A |
| **CDK7_766** | A | **CDK7_924** | A | **CDK7_1177** | A |
| **CDK7 835** | A | **CDK7_1021** | A | **CDK7_1188** | A |
| **CDK7_846** | B | **CDK7_1052** | A | **CDK7_1201** | B |
| **CDK7_847** | C | **CDK7_1060** | C | **CDK7_1225** | A |

(continued)

| Compound number | HepaRG | Compound number | HepaRG | Compound number | HepaRG |
|---|---|---|---|---|---|
| **CDK7_848** | A | **CDK7_1061** | B | **CDK7_1226** | A |
| **CDK7_849** | B | **CDK7_1062** | C | **CDK7_1227** | A |
| **CDK7_850** | A | **CDK7_953** | B | **CDK7_1229** | A |
| **CDK7_851** | A | **CDK7_1105** | C | **CDK7_1236** | A |
| **CDK7_897** | A | **CDK7_955** | A | **CDK7_1254** | A |
| **CDK7_898** | A | **CDK7_1121** | A | **CDK7_1259** | A |
| **CDK7_951** | B | **CDK7_1122** | A | **CDK7_1268** | A |
| **CDK7_834** | C | **CDK7_1123** | A | **CDK7_1276** | A |
| **CDK7_838** | B | **CDK7_1124** | A | **CDK7_1277** | A |
| **CDK7_972** | B | **CDK7_1176** | A | | |

Results of these experiments confirm production of CDK7 inhibitors that are non-toxic in the human hepatocyte model.

**Example 51.** Determination of incorporation of bromodeoxyuridine into DNA

Upon adding bromodeoxyuridine (BrdU) to dividing cells, Thymidine is replaced by its analogue, BrdU, and BrdU is incorporated into DNA. Thus, BrdU incorporated into DNA characterizes the intensity of DNA replication and ongoing S phase of the cell cycle. Reduced incorporation of bromodeoxyuridine into DNA under the action of the compounds characterizes a slowdown in the cell cycle and DNA replication.

The ability of the CDK7 inhibitors according to the present invention to inhibit the incorporation of BrdU into DNA was evaluated in a cell test on continuous cell cultures: MDA-MB-468 (*triple negative breast cancer,* ATCC® HTB-132™) using a BrdU detection kit (Roche, order no. 11 669 915 001)). The cells were grown in DMEM medium (PanEco, order no. C330π). The basal growth medium was enriched with L-glutamine at a concentration of 2 mM (PanEco, order no. Ф032) and 10% fetal bovine serum (Gibco, order no. 16140-071). MDA-MB-468 adherent cells were detached from plastic with a solution of trypsin-EDTA (PanEco, order no. П039π), the solution was then inactivated by a DMEM complete medium, centrifuged at 1000 RPM (Eppendorf, 5804 R), a suspension of cells was prepared in a DMEM complete medium, introduced into 96-well culture plates (Corning Inc, order no. 3599) at 4×10^3 cells in 75 μl of medium per well. Test compounds were dissolved in DMSO and diluted with a DMEM complete medium to a final concentration ranging from 0.5 μM to 2 pM. Serum-free growth medium was removed from plate wells, and solutions comprising diluted compounds in the volume of 100 μl in basal growth medium were added. Candidates were introduced into the plate wells with cells in triplicates (the final concentration of DMSO was no more than 1%) and incubated at 37 °C in an incubator with 5% $CO_2$ for 24 hours. Following incubation, 10 μl of solution of BrdU at a concentration of 10 μM was added to wells, the contents of the plates were agitated on an orbital shaker (Biosan, Latvia), and incubated for additional 72 hours at 37 °C in an incubator with 5% $CO_2$. Incorporated BrdU was then detected according to the guidelines of the kit manufacturer. BrdU-containing medium was removed from plate wells and the plate with cells was dried at 60 °C for 1 hour. To fix the cells, 200 ml of FixDenat solution was introduced into each well and incubated for 90 minutes at room temperature (+15-25 °C). The fixing solution was removed and 100 ml of anti-BrdU-POD working solution was introduced, followed by 90 minute incubation at room temperature (+15-25 °C). The anti-BrdU-POD antibody solution was removed from plate wells and the wells were washed 3 times with 200 ml of washing solution (10 minutes at room temperature (+15-25 °C)). 100 μl of substrate solution was introduced into each analyzed well and incubated for 3 minutes at room temperature (+15-25 °C) on an orbital shaker (Biosan, Latvia) and for another 5-7 minutes without stirring.

The amount of incorporated BrdU was detected on the Infinite M200Pro microplate spectrophotometer (Tecan Group Ltd, Switzerland) by measuring the luminescent signal. $IC_{50}$ value was calculated using the Magellan software (Tecan Group Ltd, Switzerland) by approximating experimental points by four-parameter model with the optimization by Levenberg-Marquardt:

**Table 10.** Results of BrdU incorporation into DNA on MDA-MB-468 cell lines. $IC_{50}$ is shown as a mean value from the data of multiple measurements, $IC_{50}$ , nM.

| Compound number | MDA-MB-468, IC50, nM |
|---|---|
| **CDK7_1187** | 14 |
| **CDK7_1225** | 24 |
| **CDK7_1229** | 28 |
| **CDK7_1276** | 2 |

The results of these experiments confirm production of CDK7 inhibitors, the antiproliferative activity of which on the MDA-MB-468 model **(Example 49)** is caused by a stopped cell cycle and slowed-down DNA replication.

**Example 52.** Determination of passive membrane permeability through PAMPA artificial membrane.

Passive membrane permeability of the compounds described in the present invention was determined using the PAMPA (parallel artificial membrane permeability assay) model at pH 7.4. We used a 96-well plate (Corning® BioCoat® Pre-coated PAMPA Plate System, order no. 353015), composed of acceptor and donor parts with cell membrane-modeling PVDF filter preimpregnated with a lecithin-phospholipid bilayer. The substance (at a concentration of 10 μmol) in a 0.01 M phosphate buffer with pH 7.4 was introduced into a donor well of the plate, and pure buffer was introduced into an acceptor well; afterwards, the both parts were combined and incubated for 20 hours at room temperature so that the compound was redistributed between the two wells and the membrane. Verapamil (USP, USA, order no. 1711202) and propranalol (MCE LLC, USA, cat. no. HY-B0573/CS-2680) were used as control compounds with high and medium permeability. Samples were analyzed by reversed-phase HPLC with UV detection (Agilent 1260 chromatograph with UV detector) by determining their peak areas in the donor and acceptor wells. Permeability (Pe, cm/s) and retention factor (R, %) in the lipid bilayer were calculated using the formula:

$$P_e=(-\ln[1-C_A\ (t)/C_{equilibrium}])/(A \times (1/V_D+1/V_A) \times t)$$

$$R=1-[C_D(t) \times V_D+ C_A(t) \times V_A]/(C_o+V_D),$$

where

$C_o$ is donor-well concentration of the substance at the initial moment of time t (μM);
$C_A$ (t) is acceptor-well concentration of the substance at time t (μM);
$V_d$ is donor-well volume (0.3 ml);
$V_a$ is acceptor-well volume (0.15 ml).

$$C_{equilibrium}=[C_D(t) \times V_D+ C_A(t) \times V_A]/(V_D+V_A)$$

A is filter area (0.3 $cm^2$);
t is incubation time (72000 s);

**Table 11.** Results of investigation of passive permeability through PAMPA lipid membrane model. $P_e$ is shown as a mean value from multiple measurements, "A" for $P_e > 1\cdot10^{-5}$ cm/s, "B" for $10^{-5}$ cm/s > $P_e > 5\cdot10^{-6}$ cm/s, "C" for $5\cdot10^{-6}$ cm/s > $P_e > 1\cdot10^{-6}$ cm/s, and "D" for $P_e < 1\cdot 10^{-6}$ cm/s, and "N/A" means that a given compound was not analyzed in the present test.

| Compound number | PAMPA | Compound number | PAMPA | Compound number | PAMPA |
|---|---|---|---|---|---|
| **CDK7_597** | C | **CDK7_904** | D | **CDK7_1176** | C |
| **CDK7_624** | D | **CDK7_1017** | C | **CDK7 524** | C |
| **CDK7_478** | C | **CDK7_1034** | C | **CDK7_1154** | B |
| **CDK7_807** | B | **CDK7_959** | D | **CDK7 1155** | B |

(continued)

| Compound number | PAMPA | Compound number | PAMPA | Compound number | PAMPA |
|---|---|---|---|---|---|
| **CDK7_524** | C | **CDK7_1000** | D | **CDK7_1172** | C |
| **CDK7_533** | D | **CDK7_1060** | B | **CDK7_1187** | C |
| **CDK7_536** | C | **CDK7_814** | D | **CDK7_1188** | C |
| **CDK7_548** | C | **CDK7_960** | D | **CDK7_1201** | B |
| **CDK7_735** | D | **CDK7_953** | C | **CDK7 1225** | C |
| **CDK7_811** | B | **CDK7_1021** | D | **CDK7_1226** | C |
| **CDK7_845** | D | **CDK7_1105** | D | **CDK7 1227** | B |
| **CDK7_835** | D | **CDK7_1058** | C | **CDK7_1229** | B |
| **CDK7_951** | D | **CDK7_1102** | C | **CDK7_1254** | C |
| **CDK7_961** | C | **CDK7_1121** | D | **CDK7 1259** | C |
| **CDK7_929** | D | **CDK7_1061** | C | **CDK7_1276** | B |
| **CDK7_944** | D | **CDK7_955** | D | **CDK7_1277** | B |
| **CDK7_999** | D | **CDK7_1122** | C | | |
| **CDK7_903** | D | **CDK7_1123** | D | | |

Results of these experiments confirm production of CDK7 inhibitors capable of passively penetrating a lipid membrane.

**Example 53.** *In vitro* microsomal stability study of compounds in human, rat and mouse microsomes.

The procedure for investigating the metabolism of selected compounds was carried out in microsomal fractions of the liver (of rats, mice, humans) in the presence of a phase I metabolism cofactor (NADPH).

The rate of enzymatic decomposition of the compound in liver microsomes in *in vitro* analysis was determined by keeping a reaction mixture comprising 0.5 mg/ml of pooled human, rat or mouse liver microsomes, 1 μM test compound, 1.5 mM β-nicotinamide adenine dinucleotide (Carbosynth, UK, cat. no. NN108711101) and 1.5 mM magnesium chloride in 0.1 M sodium phosphate buffer (pH 7.4) in a solid-state thermostat at 37 °C (XenoTech, USA, order no. H2610, cat. no. R1000, cat. no. M1000). Incubation was carried out in three replicates.

To determine the stability of compounds under sample preparation conditions, an aliquot in two replicates with the test compound and a microsomal fraction devoid of the NADPH cofactor, which was incubated for 40 minutes at 37 °C.

Reactions were stopped at 0, 10, 20, 30 and 40 min time points by way of adding an ice mixture of acetonitrile: methanol (3:1) comprising an internal standard.

Samples were then centrifuged to remove the precipitated protein, supernatants were analyzed using HPLC-MS/MS to determine *in vitro* clearance ($CL_{int}$) of the compounds. The Agilent 6410 mass spectrometer (Agilent, USA) combined with the Agilent 1260 Infinity II HPLC liquid chromatograph (Agilent, USA) and a cooling autosampler controlled by MassHunter software (Agilent, USA) was used as an HPLC-MS/MS system. Chromatographic analysis was performed on a C18 reversed-phase HPLC column (Agilent, Waters or equivalent) in a gradient elution mode in a system of mobile phases: 0.1% formic acid in water / 0.1% formic acid in acetonitrile, flow rate: 0.5 ml/min, column T =35°C. Mass spectrometric detection was performed with electrospray ionization in multiple reaction monitoring mode. Precursor ions, fragment ions and collision energies for each substance were selected experimentally.

A graph of dependence of the logarithmic ratio between substance's peak area and internal standard's peak area vs time was built. The dependent coefficient of this line corresponded to the elimination rate constant k, on the basis of which the in vitro clearance CLint was calculated:

$$\text{Elimination rate constant (k)} = (-\text{gradient}) \qquad \text{Half life } (t_{1/2})\ (\text{min}) = \frac{0.693}{k}$$

$$V\ (\mu L/mg) = \frac{\text{volume of incubation } (\mu L)}{\text{protein in the incubation (mg)}} \qquad \text{Intrinsic Clearance } (CL_{int})\ (\mu L/min/mg\ \text{protein}) = \frac{V \times 0.693}{t_{1/2}}$$

Based on resulting *in vitro* clearance CLint values, the microsomal stability of the selected compounds for humans, rats and mice was concluded.

Results are shown in Tables 12 and 13.

**Table 12.** Results of microsomal stability of selected compounds *in vitro* in humans (HLM). Clint is shown as a mean value from multiple measurements, "A" for Clint < 20 µl/(min·mg of protein), "B" for 50 µl/(min·mg of protein)> Clint > 20 µl/(min·mg of protein), "C" for 100 µl/(min·mg of protein)> Clint > 50 µl/(min·mg of protein), and "D" for Clint > 100 µl/(min·mg of protein).

| Compound number | *In vitro* clearance CLint (HLM) | Compound number | *In vitro* clearance CLint (HLM) |
|---|---|---|---|
| **CDK7_961** | A | **CDK7 524** | A |
| **CDK7_959** | A | **CDK7_1172** | B |
| **CDK7_924** | A | **CDK7 1177** | A |
| **CDK7_953** | A | **CDK7_1188** | A |
| **CDK7_955** | A | **CDK7_1225** | A |
| **CDK7_1122** | A | **CDK7_1229** | B |
| **CDK7_1123** | A | **CDK7_1254** | A |
| **CDK7_1176** | A | **CDK7_1276** | B |
| **CDK7_1187** | B | **CDK7_1277** | B |
| **CDK7 1103** | A | | |

**Table 13.** Stability results of selected compounds *in vitro* in rat (RLM) and mouse (MLM) microsomes. Clint is shown as a mean value from multiple measurements, "A" for Clint < 20 µl/(min·mg of protein), "B" for 50 µl/(min·mg of protein)> Clint > 20 µl/(min·mg of protein), "C" for 100 µl/(min·mg of protein)> Clint > 50 µl/(min·mg of protein), and "D" for Clint > 100 µl/(min·mg of protein), and "N/A" means that a given compound was not analyzed in the present test.

| Compound number | RLM | MLM |
|---|---|---|
| **CDK7_953** | A | A |
| **CDK7_1187** | A | A |
| **CDK7_1188** | A | N/A |
| **CDK7_1225** | A | A |
| **CDK7_1227** | A | N/A |
| **CDK7_1229** | A | A |
| **CDK7_1259** | A | N/A |
| **CDK7_1276** | B | B |
| **CDK7_1277** | B | B |

Results of these experiments confirm production of CDK7 inhibitors resistant (B) and highly resistant (A) to the activity of human, mouse and rat liver microsomal enzymes.

**Example 54.** Pharmacokinetics (PK) study.

**Dosing and administration**

Pharmacokinetic studies of the compounds were carried out on CD-1 mice, males, aged about 8 weeks, weighing 16-29 g. The animals were given a single oral (PO) administration (intragastrically) at a dose of 10 mg/kg in a formulation in the form of a 0.2% solution of hydroxypropyl methylcellulose (HPMC) in a 5% aqueous solution of glucose and a single IV-bolus administration (intravenously) at a dose of 1.0 mg/kg for 1277 and at a dose of 2.0 mg/kg for substances **CDK7_1187, CDK7_1225, CDK7_1229** and **CDK7_1276** in a formulation comprising 0.2% DMSO in 5% aqueous

solution of glucose.

For each substance, 21-30 animals were used for each dose of PO and IV administration. All mice were weighed and randomized before dosing.

3-5 animals were used for each time point. Each animal was sampled at one time point. At least 12 h before the start of the experiment, the animals were deprived of food while maintaining free access to water.

Animals from the PO and IV groups were subjected to blood sampling before administering the substance and after 0.5, 2, 4, 8, 12 and 24 h.

**Blood samples**

Blood was collected by cardiac puncture using the closed method. Blood was collected in a volume of 0.2 ml into polypropylene tubes comprising 20 μl of 5% EDTA. Blood plasma was separated by centrifugation at 10000 rpm for 10 minutes at 4±2 °C and transferred into clean labeled tubes.

**Blood plasma analysis**

Test blood plasma samples of mice from the experimental groups were obtained in a volume of 50 μl. Afterwards, 200 ml of a solution of internal standard (tolbutamide (Sigma, T0891)) prepared in acetonitrile, at a concentration of 50 ng/ml, pre-cooled in the refrigerator at 2-8 °C, was added to the samples, stirred in a vortex mixer (G560E, Scientific Industries) for 10 seconds and incubated at 4 °C for 15 minutes for protein precipitation. Samples were then centrifuged (5417R centrifuge, Eppendorf) at 1500 g for 10 minutes while cooling to 4 °C. 150 μl of the supernatant were transferred to a 96-well plate for HPLC-MS/MS analysis. Quantitative determination of test compounds in blood plasma was carried out by absolute calibration method. The calibration curve was generated by analyzing plasma samples with supplementations of known amounts of the standard of the compound being determined. The lower limit of the quantitative determination of test compounds in blood plasma was 1 ng/ml, the analytical range of the technique was 1 ng/ml to 2000 ng/ml. The technique was partially validated in terms of selectivity, accuracy, precision, linearity, matrix effects, stability.

The Qtrap 5500 mass spectrometer (AB Sciex) in combination with the Agilent 1290 Infinity II HPLC liquid chromatograph (Agilent) and a cooling multisampler (Agilent 1290 Infinity II Multisampler G7167B) was used as an HPLC-MS/MS system. Chromatographic analysis was performed on a C18 reversed-phase HPLC column (YMC-Triart C18, 50*2 mm, 1.9 μm, YMC CO. Ltd). The mobile phase used was used a mixture of deionized water and acetonitrile supplemented with 0.1% formic acid in gradient mode, flow rate: 0.5 ml/min, column T =40$^0$C, autosampler T = 8°C, analysis time: 2.8 min. Mass spectrometric detection was performed in multiple reaction monitoring (MRM) mode. Precursor ions, fragment ions and collision energies for each substance were selected experimentally. Two Q1/Q3 transitions were used to record MRM chromatograms of the analyte and internal standard; one of transitions was used for quantitative calculations, the other one was used to confirm that the extraction time corresponds to a given substance. Based on the results of HPLC-MS/MS analysis of calibration samples using the Analyst 1.6.3 software, the calibration dependence of areas of chromatographic analyte peaks normalized to the signal of the internal standard on the concentration of the analyte was built.

**Pharmacokinetic analysis**

Pharmacokinetic analysis was performed by non-compartmental method using the Phoenix WinNonlin 6.3 software package. Main averaged PK parameters were determined: time to maximum concentration (Tmax), elimination half-life (T1/2), maximum concentration (Cmax), area under pharmacokinetic curve (AUClast) from administration time to the last measured concentration, clearance (CL), steady-state volume of distribution (Vss), and also absolute systemic bioavailability value (F). The pharmacokinetic parameters of the compounds according to the present invention are shown in **Tables 14-15.**

**Table 14.** Pharmacokinetic parameters of compounds in mice plasma following intravenous administration.

| **Intravenous administration** | | | | | | |
|---|---|---|---|---|---|---|
| **PK parameter** | **Unit** | **CDK7_ 1187** | **CDK7_ 1225** | **CDK7_ 1229** | **CDK7_ 1276** | **CDK7_ 1277** |
| **dose** | **mg/kg** | **2** | **2** | **2** | **2** | **1** |
| **Animals in group** | **pc** | **5** | **5** | **5** | **3** | **5** |
| T1/2 | h | 1.7 | 1.8 | 2.0 | 4.15 | 2.1 |
| >AUClast | h×ng/ml | 314 | 691 | 1135 | 1376 | 363 |

(continued)

| Intravenous administration | | | | | | |
|---|---|---|---|---|---|---|
| **PK parameter** | **Unit** | **CDK7_ 1187** | **CDK7_ 1225** | **CDK7_ 1229** | **CDK7_ 1276** | **CDK7_ 1277** |
| Vss | ml/kg | 10101 | 6298 | 4166 | 6520 | 6889 |
| Cl | ml/h/kg | 6194 | 2878 | 1740 | 1288 | 2701 |

**Table 15.** Pharmacokinetic parameters of compounds in mice plasma following oral administration.

| Oral administration | | | | | | |
|---|---|---|---|---|---|---|
| **Parameter** | **Unit** | **CDK7_ 1187** | **CDK7_ 1225** | **CDK7_ 1229** | **CDK7_ 1276** | **CDK7_ 1277** |
| **dose** | **mg/kg** | **10** | **10** | **10** | **10** | **10** |
| **Animals in group** | **pc** | **5** | **5** | **5** | **3** | **5** |
| T1/2 | h | 1.7 | 1.9 | 2.5 | 2.0 | 3.8 |
| Tmax | h | 0.5 | 2.0 | 0.5 | 0.5 | 0.5 |
| Cmax | ng/ml | 309 | 712 | 1066 | 1513 | 893 |
| AUClast | h×ng/ml | 1164 | 3536 | 5787 | 7979 | 3113 |
| F | % | 72.7 | 102 | 102 | 116 | 85.8 |

The compounds according to the present invention correspond to target pharmacokinetic characteristics: the compounds are well absorbed and have a sufficient oral absorption effect. According to mean value of area under curve (AUClast), absolute bioavailability of the compounds was calculated to be more than 72.7%. In turn, this demonstrates the possibility of oral administration of these compounds to achieve pharmacodynamic effects.

**Example 55.** Permeability of blood-brain barrier (BBB)

The ability of substances to penetrate the blood-brain barrier and enter the central nervous system (mouse brain) was evaluated by the ratio of their areas under pharmacokinetic curves in rat brain and blood plasma following oral administration, characterizing tissue bioavailability to the rat brain, and expressed as the AUCbrain / AUCplasma coefficient.

**Dosing and administration**

Pharmacokinetic studies of the compounds were carried out on CD-1 mice, males, aged about 8 weeks, weighing 16-29 g. Animals were subjected a single oral (PO) administration (intragastrically) at a dose of 10 mg/kg in a formulation in the form of a 0.2% solution of hydroxypropyl methylcellulose (HPMC) in a 5% aqueous solution of glucose.
21 animals were used for each substance. All mice were weighed and randomized before dosing.
3 animals were used for each time point. Biomaterials were collected from each animal at one time point. At least 12 h before the start of the experiment, the animals were deprived of food while maintaining free access to water. Blood and brain samples were collected 0.5, 2, 4, 6, 8, 12 and 16 h after administration of the substance.

**Blood samples**

All manipulations were carried out analogously to **Example 54.**

**Brain samples**

Perfusion of mice brains was carried out with saline solution via the vascular system. To this end, the chest was opened, the right atrium was incised with microsurgical scissors, and saline cooled to 4 °C was administered using a 10-ml syringe into the left ventricle until the liver turned white. After washing, the skull was opened and the brain removed, it was weighed and transferred to labeled 5 ml tubes.

## Analysis of brain samples

A 4-fold volume of water was added to the brain suspension and homogenized using a homogenizer (Omni Bead Ruptor 24) 2 times for 45 seconds. Analogously, the intact mice brain homogenate was prepared, which was then used to prepare calibration samples and QC samples. The calibration curve was generated by analyzing brain homogenate samples with supplementations of known amounts of the standard of the compound being determined.

Samples were precipitated with cooled acetonitrile at 1:4 ratio, containing the internal standard tolbutamide (Sigma, T0891) at a concentration of 50 ng/ml. Samples were stirred on a vortex mixer (G560E, Scientific Industries) for 10 seconds and kept at 4 °C for 15 minutes to precipitate proteins, then centrifuged (5417R centrifuge, Eppendorf) for 10 minutes at 1500 g. 150 ml of supernatants were collected and transferred to corresponding wells of a 96-well plate for HPLC-MS/MS analysis. Quantitative determination of test compounds in brain homogenates was carried out analogously to the procedure described in **Example 54.** Lower limit of quantitative determination of test compounds in brain homogenate was 0.3 ng/ml.

Pharmacokinetic analysis of data was performed analogously to the procedure described in **Example 54.**

In addition to main PK parameters, the ratio of concentration in the brain and plasma at the studied time points (AUCbrain / AUCplasma) was calculated to assess bioavailability in the brain (permeability through the blood-brain barrier).

**Table 16.** Pharmacokinetic parameters of compounds in mice brain homogenate following oral administration.

| Parameter | Unit | CDK7_1225 | CDK7_1229 | CDK7_1276 | CDK7_1277 |
|---|---|---|---|---|---|
| **dose** | **mg/kg** | **10** | **10** | **10** | **10** |
| **Animals in group** | **pc** | **3** | **3** | **3** | **3** |
| t1/2 | h | 2.33 | 2.25 | 1.99 | 2.76 |
| Tmax | h | 6.0 | 0.5 | 0.5 | 2.0 |
| Cmax | ng/ml | 93.8 | 136 | 335 | 226 |
| AUClast | h*ng/ml | 855 | 909 | 1939 | 1750 |
| AUCbrain / AUCplasma | - | 0.165 | 0.182 | 0.243 | 0.302 |

Resulting data demonstrate the ability of compounds according to the present invention to penetrate through the BBB

**Example 56.** Study of antitumor activity *in vivo.*

The antitumor activity of **CDK7_1187** *in vivo* was evaluated on a subcutaneous xenograft model of the NCI-H69 line (*small cell lung cancer,* ATCC® HTB-119™) transplanted to BALB/c Nude mice.

NCI-H69 cell line was cultured in RPMI-1640 medium (PanEco, Russia) with enrichment of the basal growth medium with L-glutamine at a concentration of 2 mM (PanEco, Russia) and 10% fetal bovine serum (Gibco, Thermo Fisher Scientific, USA).

The study used 20 BALB/c Nude mice (females) obtained from the Federal State Budgetary Scientific Institution "Federal Research Center Institute of Cytology and Genetics, Siberian Branch of Russian Academy of Sciences" (ICG SB RAS).

BALB/c Nude mice were subcutaneously administered into the right lateral side of the body with 200 ml of suspension containing $5\times10^6$ NCI-H69 tumor cells and Matrigel (Corning® Matrigel® Basement Membrane Matrix) at 1:1 ratio.

Linear dimensions of the tumor were determined using an electronic caliper. Volume of the tumor node was determined by the formula (1):

$$V = L\times W^2/2, \qquad (1)$$

where L and W are large and small diameters of the tumor node, respectively.

The efficacy of test product was measured by the index of tumor growth inhibition (TGI, %) calculated according to the formula (2):

$$TGI(V)= \frac{Vc-V}{Vc} \times 100\%, \qquad (2)$$

where Vc and Vp are mean volumes of the tumor node in the control group and the group of animals that received the test substance, respectively.

After achieving an average tumor volume of 130 $mm^3$, animals were divided into 2 groups of 10 animals, volume of the tumor node was used as a randomization criterion. The day of the first administration of the product was designated as day 0.

Test substance **CDK7_1187** was administered intragastrically at a dose of 6 mg/kg daily, for 21 days. Before each administration, **CDK7_1187** was diluted in 5% solution of glucose (Solopharm, Russia) and 0.1% solution of DMSO (Sigma, USA). The control group received a 5% solution of glucose and a 0.1% solution of DMSO in an analogous volume.

Statistical analysis was carried out using the GraphPad Prism 9 software package. Mann-Whitney U test was employed to assess the significance of differences when comparing the volumes of tumor nodes. Differences were identified as significant if $p < 0.05$.

Dynamics of tumor growth is shown in **Fig 1.** TGI indexes, %, are shown in **Table 17.**

**Table 17.** TGI index, %

| Group | Day 9 | Day 13 | Day 17 | Day 21 |
|---|---|---|---|---|
| **CDK7_1187** | 10 | 36 | 40 | 48 |

Results of the study showed that following administering **CDK7_1187** to BALB/c Nude mice the tumor volume was statistically significantly less than that in the control group of animals on days 17 and 21 of the experiment. The index of tumor growth inhibition at the end of the observation period was 48%.

## Claims

1. Compound of formula **I:**

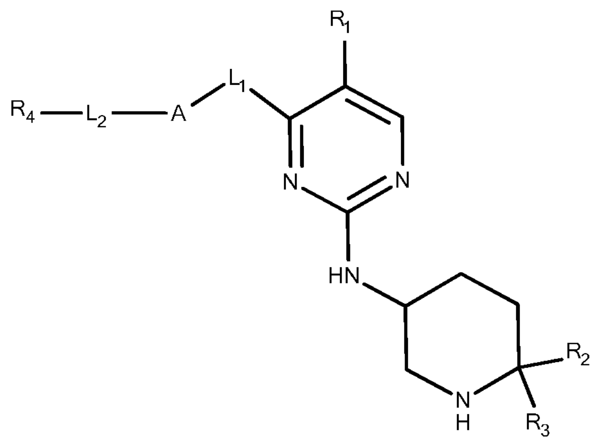

**I**

or a pharmaceutically acceptable salt, solvate or stereoisomer thereof,
$R_1$ is $-C(Hal)_3$, $-CH(Hal)_2$, $-CH_2Hal$, -Hal, -H, $-NO_2$, $-(C_1-C_6)$alkyl unsubstituted or substituted by one or several atoms of Hal, $-O-(C_1-C_6)$alkyl unsubstituted or substituted by one or several atoms of Hal, $-S-(C_1-C_6)$alkyl unsubstituted or substituted by one or several atoms of Hal;
$R_2$, $R_3$ are each independently H, $-(C_1-C_6)$alkyl;
or $R_2$, $R_3$ together form $-(C_3-C_6)$cycloalkyl unsubstituted or substituted by one or several - $(C_1-C_6)$alkyls;
$L_1$, $L_2$ are each independently a chemical bond, $-NR_5-$, -O-, -S-, -S(O)-, $-S(O)_2-$, -C(O)-, - C(O)O-, -C(S)-, -C(=NH)-, $-(CR_{6a}R_{6b})_{1-3}-$, $-P(O)(CH_3)-$, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;
A is 6-10 membered aryl unsubstituted or substituted by one or several $R_7$; 6-10 membered aryl unsubstituted or substituted by one or several $R_{7a}$, fused with $-(C_3-C_6)$cycloalkyl unsubstituted or substituted by one or several $R_{8a}$; 6-10 membered aryl unsubstituted or substituted by one or several $R_{7b}$, fused with 4-10 membered

heterocyclyl with 1,2,3 or 4 heteroatoms selected from N, S or O, unsubstituted or substituted by one or several $R_{8b}$; 6-10 membered aryl unsubstituted or substituted by one or several $R_{7c}$, fused with 5-6 membered heteroaryl with 1,2,3 or 4 heteroatoms selected from N,O or S, unsubstituted or substituted by one or several $R_{8c}$; 5-6 membered heteroaryl with 1,2,3 or 4 atoms N, unsubstituted or substituted by one or several $R_{7d}$; 5-6 membered heteroaryl with 1,2,3 or 4 heteroatoms selected from N,O or S, unsubstituted or substituted by one or several $R_{7e}$, fused with $(C_3-C_6)$cycloalkyl unsubstituted or substituted by one or several $R_{8e}$; 5-6 membered heteroaryl with 1,2,3 or 4 heteroatoms selected from N,O or S, unsubstituted or substituted by one or several $R_{7f}$, fused with 4-10 membered heterocyclyl with 1,2,3 or 4 heteroatoms selected from N, S or O, unsubstituted or substituted by one or several $R_{8f}$; 5-6 membered heterocyclyl with 1,2,3 or 4 heteroatoms selected from N, S or O, unsubstituted or substituted by one or several $R_{7g}$; 5-6 membered heterocyclyl with 1,2,3 or 4 heteroatoms selected from N, S or O, unsubstituted or substituted by one or several $R_{7h}$, fused with 4-10 membered heterocyclyl with 1,2,3 or 4 heteroatoms selected from N, S or O, unsubstituted or substituted by one or several $R_{8h}$;

$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$; 5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH $-NH_2$, -Hal, -CN, $-C_1-C_6$ alkyl unsubstituted or substituted by one or several radicals selected from -Hal, -OH, $-NH_2$, -CN, phenyl; $-O-C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl, $-C_1-C_6$ alkyl; - $S(O)_2C_1-C_6$ alkyl, $-S(O)_2NH_2$, $-NH(C_1-C_6\ alkyl)$, $-N(C_1-C_6\ alkyl)_2$, $-P(O)(C_1-C_6\ alkyl)_2$, morpholinyl, thiazolyl, phenyl,

$R_5$, $R_{5a}$ are each independently -H, $C_1-C_6$ alkyl,

$R_{6a}$, $R_{6b}$ are each independently -H, $C_1-C_6$ alkyl, -Hal,

or $R_{6a}$ and $R_{6b}$ together with the C atom they are attached to form $C_3-C_6$ cycloalkyl unsubstituted or unsubstituted by several $-(C_1-C_6)$alkyls;

$R_7$, $R_{7a}$, $R_{7b}$, $R_{7c}$, $R_{7d}$, $R_{7e}$ $R_{7f}$ $R_{7g}$ $R_{7h}$ are each independently -Hal, -OH, $-NH_2$, -CN, (=O), - $(C_1-C_6)$alkyl unsubstituted or substituted by one or several -Hal; -O $(C_1-C_6)$alkyl unsubstituted or substituted by one or several -Hal;

$R_{8a}$, $R_{8b}$, $R_{8c}$, $R_{8d}$, $R_{8e}$ $R_{8f}$ $R_{8h}$ are each independently -Hal, -OH, $-NH_2$, -CN, (=O), $-(C_1-C_6)$alkyl unsubstituted or substituted by one or several -Hal;

Hal is an atom of F, Cl, Br, I.

**2.** The compound according to claim 1, wherein $-A-L_2-R_4$ is:

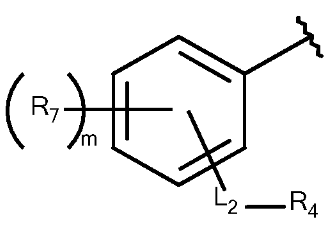

,

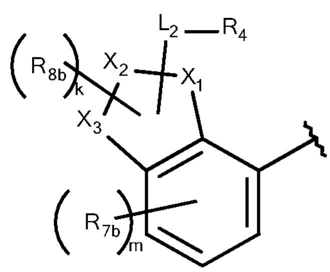

,

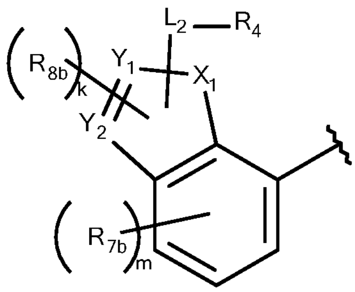

,

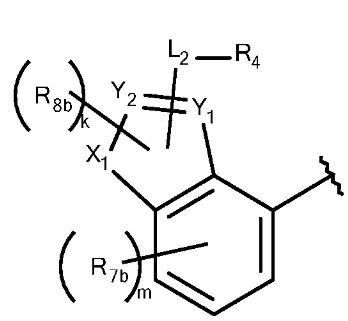,
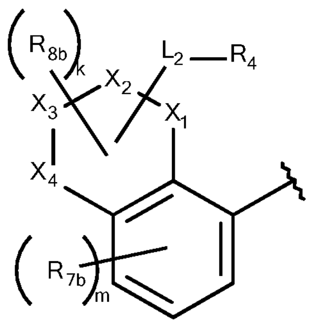,
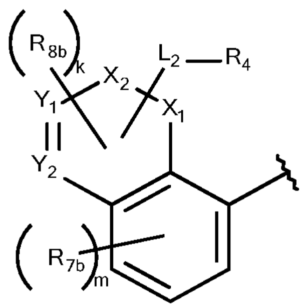,

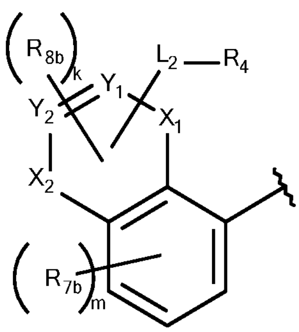,
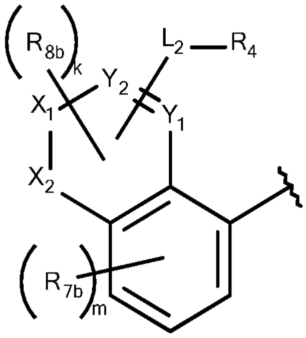,
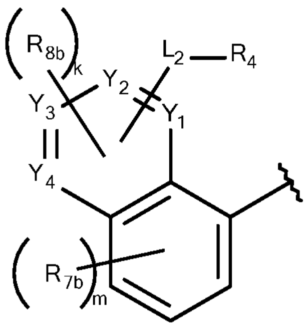,

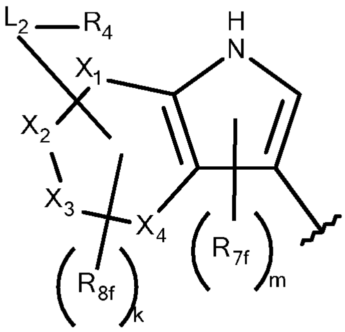,
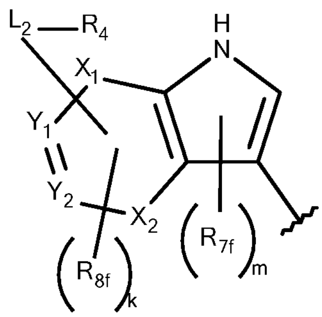,
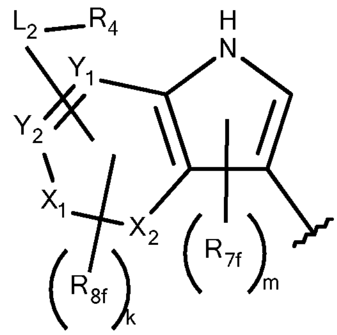,

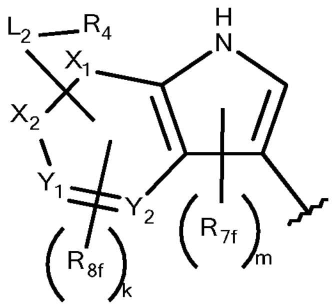,
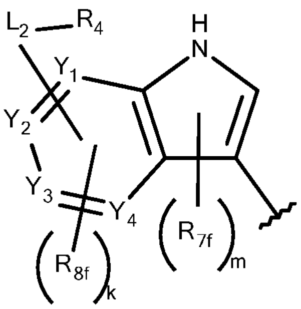,
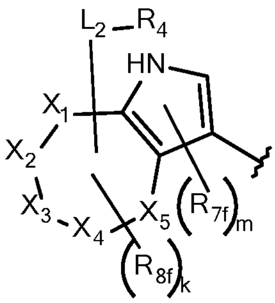,

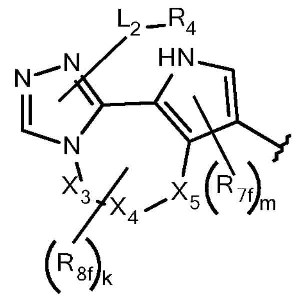,

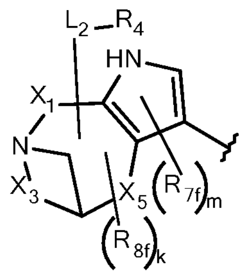,

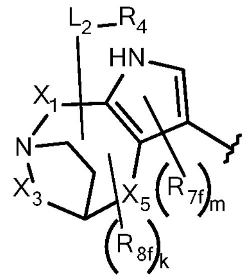,

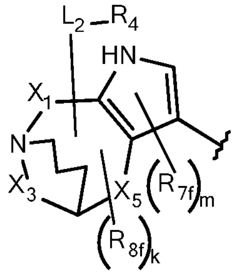,

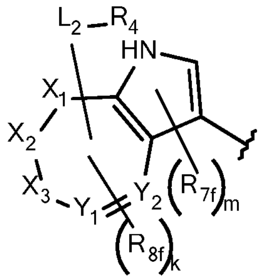,

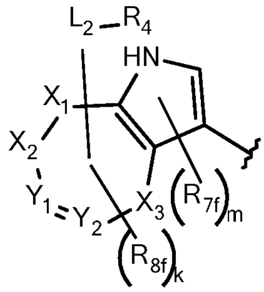,

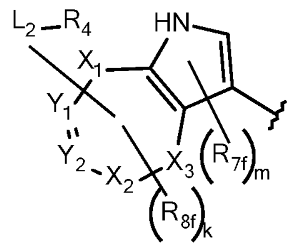,

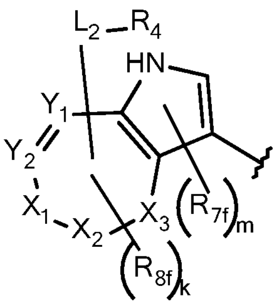,

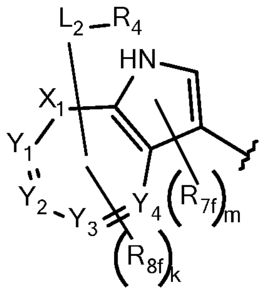,

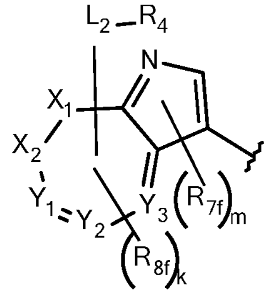,

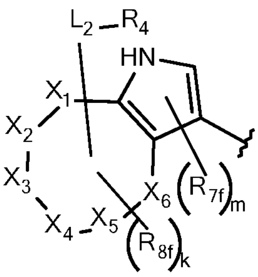

,

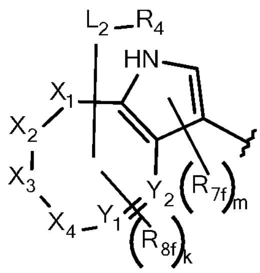,

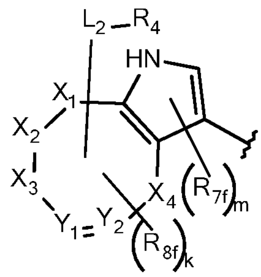,

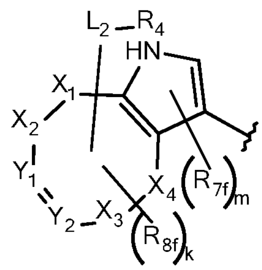,

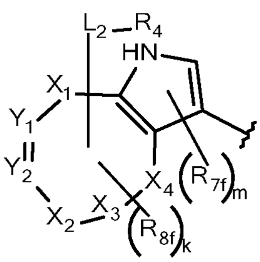,

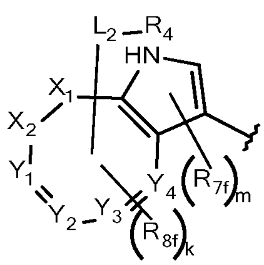,

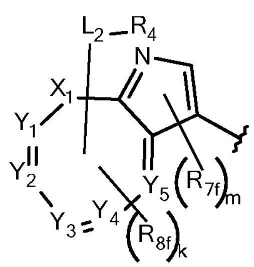,

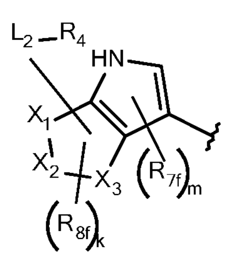,

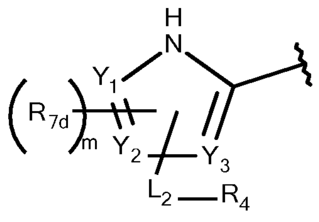
,
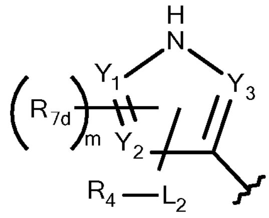
,
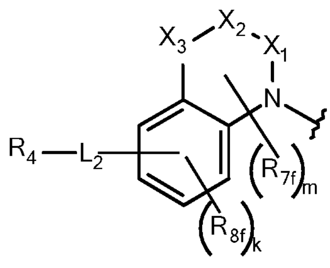
,

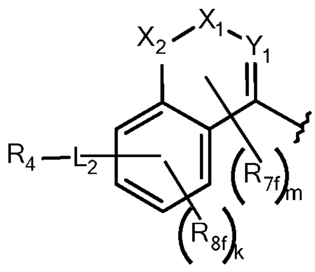
,

wherein k,m are each independently 0, 1,2 or 3,
$X_1$, $X_2$, $X_3$, $X_4$, $X_5$, $X_6$ are each independently C, N, CH, $CH_2$, NH, S or O;
$Y_1$, $Y_2$, $Y_3$, $Y_4$, $Y_5$ are each independently C, N or CH;
$L_2$ is a chemical bond, $-NR_5-$, -O-, -S-, -S(O)-, $-S(O)_2-$, -C(O)-, -C(O)O-, -C(S)-, -C(=NH)-, $-(CR_{6a}R_{6b})_{1-3}-$, -P(O)($CH_3$)-, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;
$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;
$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH $-NH_2$, -Hal, -CN, $-C_1-C_6$ alkyl unsubstituted or substituted by one or several radicals selected from -Hal, -OH, $-NH_2$, -CN, phenyl; $-O-C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl, $-C_1-C_6$ alkyl; - $S(O)_2C_1-C_6$ alkyl, $-S(O)_2NH_2$, $-NH(C_1-C_6$ alkyl), $-N(C_1-C_6\ alkyl)_2$, $-P(O)(C_1-C_6\ alkyl)_2$, morpholinyl, thiazolyl, phenyl,
$R_5$, $R_{5a}$ are each independently -H, $C_1-C_6$ alkyl,
$R_{6a}$, $R_{6b}$ are each independently -H, $C_1-C_6$ alkyl, -Hal,
or $R_{6a}$ and $R_{6b}$ together with the C atom they are attached to form $C_3-C_6$ cycloalkyl unsubstituted or unsubstituted by several $-(C_1-C_6)$alkyls;
$R_7$, $R_{7b}$, $R_{7d}$, $R_{7f}$, are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;
$R_{8b}$, $R_{8d}$, $R_{8f}$ are each independently -Hal, -OH, $-NH_2$, -CN, (=O), $-(C_1-C_6)$alkyl unsubstituted or substituted by one or several -Hal;
Hal is an atom of F, Cl, Br, I.

**3.** The connection according to claims 1-2, wherein $-A-L_2-R_4$ is

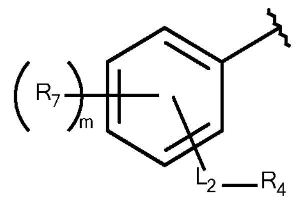
,
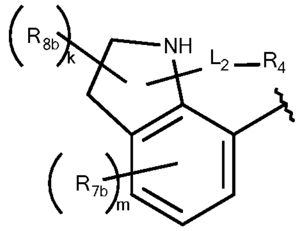
,
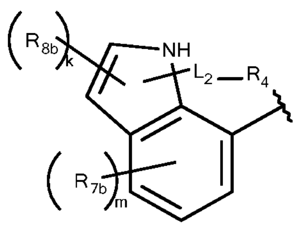
,

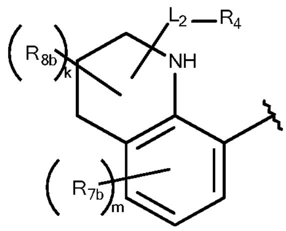
,
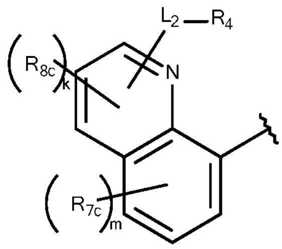
,
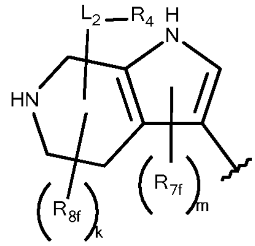
,

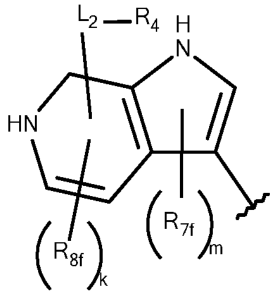
,
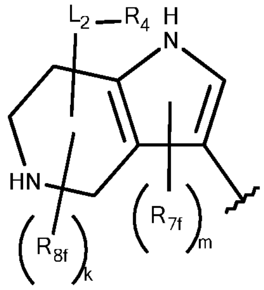
,
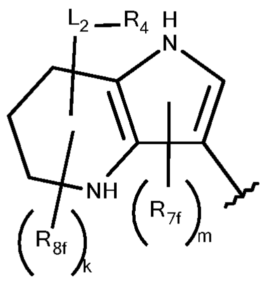
,
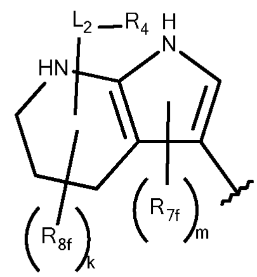

,
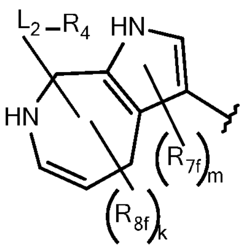
,
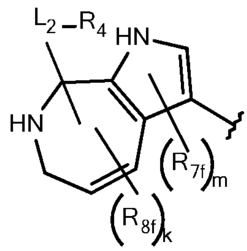
,
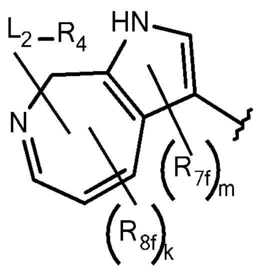
,

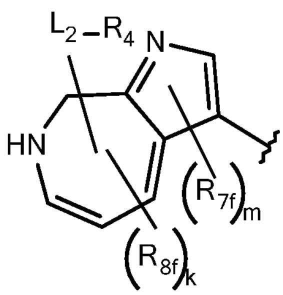
,
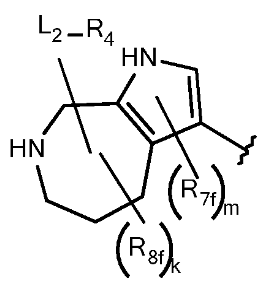
,
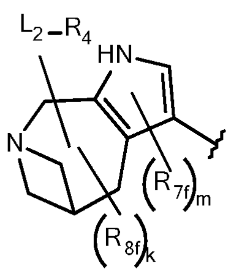
,

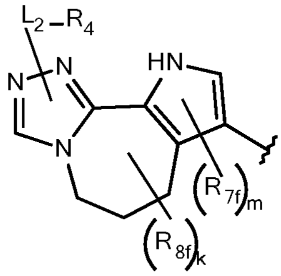,
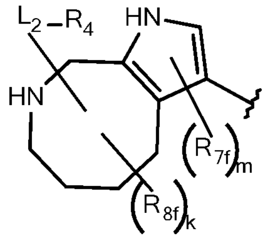,
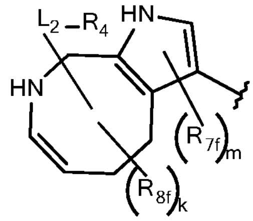,

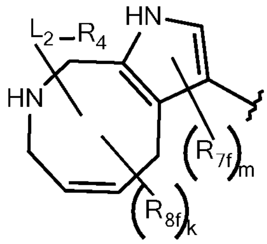,
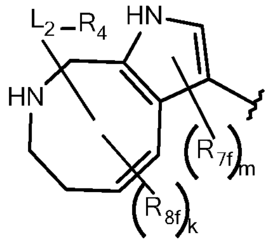,
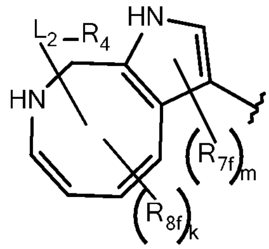,
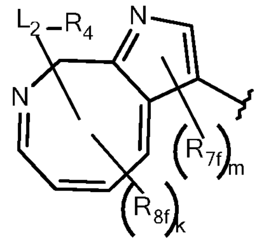,

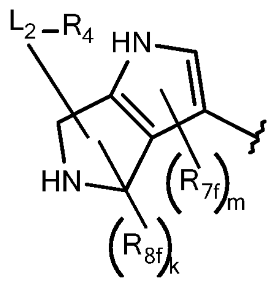,
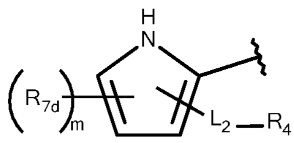,
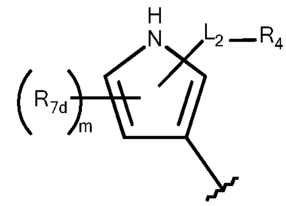,

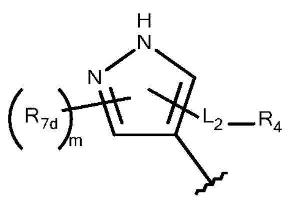,
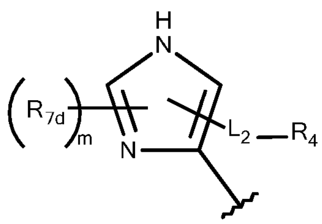,
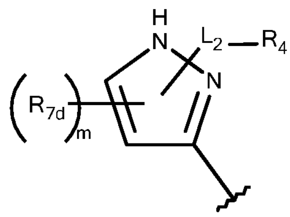,

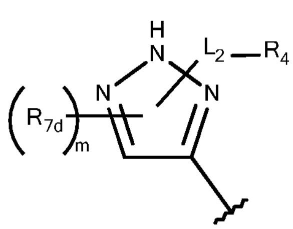,
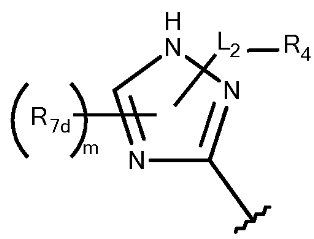,
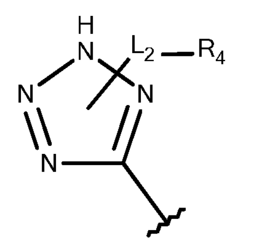,

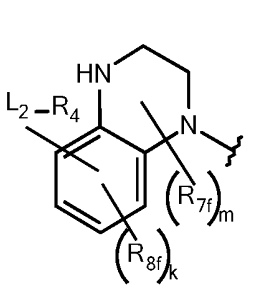, 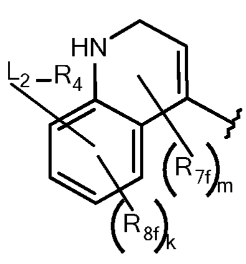, 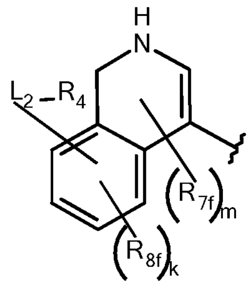, 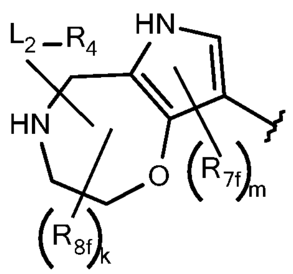,

wherein k,m are each independently 0, 1,2 or 3,

$L_2$ is a chemical bond, $-NR_5-$, -O-, -S-, -S(O)-, $-S(O)_2-$, -C(O)-, -C(O)O-, -C(S)-, -C(=NH)-, $-(CR_{6a}R_{6b})_{1-3}-$, -P(O)$(CH_3)$-, $-O-(CH_2)_{1-3}-$, $-(CH_2)_{1-3}-O-$, $-NR_{5a}-(CH_2)_{1-3}-$, $-(CH_2)_{1-3}-NR_{5a}-$, $-C(O)-(CH_2)_{1-3}-$, $-(CH_2)_{1-3}-C(O)-$, -C(O)-NH- or -NH-C(O)-;

$R_4$ is -H -Hal, (=O), -OH, -C(O)OH, $-NR_{4a}R_{4b}$, $-P(O)(C_1-C_6\ alkyl)_2$, $-S(O)_2NH_2$, $-S(O)_2C_1-C_6$ alkyl, $-(C_1-C_6)$alkyl unsubstituted or substituted by one or several $R_{4c}$; $-O-C_1-C_6$ alkyl unsubstituted or substituted by one or several $R_{4d}$; phenyl unsubstituted or substituted by one or several $R_{4e}$; 4-7 membered heterocyclyl with 1,2,3 or 4 heteroatoms selected from N, S or O, unsubstituted or substituted by one or several $R_{4f}$; $-(C_3-C_6)$cycloalkyl unsubstituted or substituted by one or several $R_{4g}$; $-C_2-C_6$ alkenyl unsubstituted or substituted by one or several $R_{4h}$; 5-6 membered heteroaryl with 1,2,3 or 4 atoms N, unsubstituted or substituted by one or several $R_{4i}$; 4-7 membered heterocyclyl with 1 or 2 heteroatoms selected from N, S or O, unsubstituted or substituted by one or several $R_{4j}$, fused with phenyl unsubstituted or substituted by one or several $R_{4k}$; 5-6 membered heteroaryl with 1,2,3 or 4 heteroatoms selected from N,O or S, unsubstituted or substituted by one or several $R_{4l}$, fused with phenyl unsubstituted or substituted by one or several $R_{4m}$;

$R_{4a}$, $R_{4b}$, $R_{4c}$, $R_{4d}$, $R_{4e}$, $R_{4f}$, $R_{4g}$, $R_{4h}$, $R_{4i}$, $R_{4j}$, $R_{4k}$, $R_{4l}$, $R_{4m}$ are each independently -H, (=O), -OH, $-NH_2$, -Hal, -CN, $-C_1-C_6$ alkyl unsubstituted or substituted by one or several radicals selected from -Hal, -OH, $-NH_2$, -CN, phenyl, $-C_1-C_6$ alkyl; $-O-C_1-C_6$ alkyl unsubstituted or substituted by one or several radicals selected from -Hal, -OH, $-NH_2$, -CN, phenyl; $-C_3-C_6$ cycloalkyl unsubstituted or substituted by one or several radicals selected from -Hal, -OH, $-NH_2$, -CN, phenyl; $-S(O)_2C_1-C_6$ alkyl, $-S(O)_2NH_2$, $-NH(C_1-C_6\ alkyl)$, $-N(C_1-C_6\ alkyl)_2$, $-P(O)(C_1-C_6\ alkyl)_2$, morpholinyl, thiazolyl, phenyl,

$R_5$, $R_{5a}$ are each independently -H, $C_1-C_6$ alkyl,

$R_{6a}$, $R_{6b}$ are each independently -H, $C_1-C_6$ alkyl, -Hal,

or $R_{6a}$ and $R_{6b}$ together with the C atom they are attached to form $C_3-C_6$ cycloalkyl unsubstituted or unsubstituted by several $-(C_1-C_6)$alkyls;

$R_7$, $R_{7b}$, $R_{7d}$, $R_{7f}$, are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;

$R_{8b}$, $R_{8d}$, $R_{8f}$ are each independently -Hal, -OH, $-NH_2$, -CN, (=O), $-(C_1-C_6)$alkyl unsubstituted or substituted by one or several -Hal; -O $(C_1-C_6)$alkyl unsubstituted or substituted by one or several -Hal;

Hal is an atom of F, Cl, Br, I.

**4.** The compounds according to claims 1-3, wherein $L_1$, $L_2$ are each independently a chemical bond, -C(O)-, $-CH_2-$, $-CH(CH_3)-$, $-C(CH_3)_2-$, $-C(CH_2)_2-$, $-CF_2-$, -S-, -S(O)-, $-S(O)_2-$, -O-, - NH-, $-NCH_3-$, $-P(O)(CH_3)-$, -C(O)O-, -C(O)NH- or -NHC(O)-.

**5.** The compounds according to claims 1-3, wherein $R_1$ is -H, $-CF_3$, $-CCl_3$, $-CHF_2$, $-CH_2F$, - $CHCl_2$, $-CH_2Cl$, $-NO_2$.

**6.** The compounds according to claims 1-3, wherein $R_2$, $R_3$ are each independently -H, methyl, ethyl, propyl, or $R_2$ and $R_3$ together with the C atom they are attached to form cyclopropyl, cyclobutyl or cyclopentyl.

**7.** The compounds according to claims 1-3, wherein $R_4$ is -H, (=O), -OH, -F, -Cl, -Br, - $P(O)(C_1-C_6\ alkyl)_2$; $-S(O)_2NH_2$; $-S(O)_2-(C_1-C_6)$alkyl; $-(C_1-C_6)$alkyl unsubstituted or substituted by one or several (=O), -Hal, -CN, $-S(O)_2-(C_1-C_6)$alkyl; $-O-(C_1-C_6)$alkyl unsubstituted or substituted by one or several (=O), -CN, -Hal; $-(C_3-C_6)$cycloalkyl unsubstituted or substituted by one or several (=O), -OH, -Hal, -CN; phenyl unsubstituted or substituted by one or several -Hal, -CN, morpholine, $-P(O)(CH_3)_2$; 5-6 membered heterocyclyl with 1,2 or 3 heteroatoms selected from N or O unsubstituted or substituted by $-(C_1-C_6)$alkyl, (=O), -Hal, -CN; 5-6 membered heteroaryl with 1,2,3 or 4 heteroatoms selected from N,O or S unsubstituted or substituted by (=O), $-(C_1-C_6)$alkyl, $-(C_3-C_6)$cycloalkyl, -Hal, -CN; 5-6 membered heteroaryl with 1

or 2 atoms N, unsubstituted or substituted by one or several (=O), -($C_1$-$C_6$)alkyl, fused with phenyl; -$NR_{4a}R_{4b}$, $R_{4a}$, $R_{4b}$ are each independently -H, (=O), morpholinyl, -F, -$S(O)_2CH_3$, -CN, -one, cyclopropyl, thiazolyl, -$C_1$-$C_3$ alkyl unsubstituted or substituted with phenyl, -$P(O)(CH_3)_2$.

**8.** The compounds according to claim 7, wherein $R_4$ is -H, -one, (=O), -$P(O)(CH_3)_2$, -$CH_3$, - $CH_2CH_3$, tert-butyl, -$CH_2CF_3$, - $CH_2CH_2S(O)_2CH_3$, -$CF_3$, -Cl, -$OCF_3$, -$S(O)_2NH_2$, - $S(O)_2CH_3$, phenyl unsubstituted or substituted by one or several -Hal, -CN; methylpiperazinyl, piperazinyl, imidazolyl, thiazolyl, methylpyrazolyl, pyrazolyl, pyridinyl, pyrimidinyl, morpholine, oxanyl, oxolanyl, cyclohexyl, cyclopentyl, cyclopropyl, oxazolyl, methyloxazolyl, dimethyloxazolyl, dihydropyridinyl, methyl dihydropyridinyl, -$NR_{4a}R_{4b}$,
$R_{4a}$, $R_{4b}$ are each independently -H, (=O), morpholinyl, -F, -$S(O)_2CH_3$, -CN, -one, cyclopropyl, thiazolyl, -$C_1$-$C_3$ alkyl unsubstituted or substituted by phenyl, -$P(O)(CH_3)_2$.

**9.** The compounds according to claims 1-3,7, wherein $R_{4a}$, $R_{4b}$, $R_{4c}$, $R_{4d}$, $R_{4e}$, $R_{4f}$, $R_{4g}$, $R_{4l}$, $R_{4i}$, $R_{4j}$, $R_{4k}$, $R_{4l}$, $R_{4m}$ are each independently -H, (=O) -$CH_3$, morpholinyl, -F, -$S(O)_2CH_3$, -CN, -one, cyclopropyl, thiazolyl, -$C_1$-$C_3$ alkyl unsubstituted or substituted by phenyl, - $P(O)(CH_3)_2$.

**10.** The compounds according to claims 1-3, wherein $R_5$ is -H, -$CH_3$.

**11.** The compounds according to claims 1-3, wherein $R_{6a}$ and $R_{6b}$ are each independently -H, -F, -$CH_3$, or $R_{6a}$ and $R_{6b}$ together with the C atom they are attached to are cyclopropyl.

**12.** The compounds according to claims 1-3, wherein $R_7$, $R_{7a}$, $R_{7b}$, $R_{7c}$, $R_{7d}$, $R_{7e}$ $R_{7f}$ $R_{7g}$ $R_{7h}$ are each independently (=O), -Hal, -$CH_3$; -$OCH_3$; -$CHal_3$; -$OCHal_3$.

**13.** The compounds according to claims 1-3, wherein $R_{8a}$, $R_{8b}$, $R_{8c}$, $R_{8d}$, $R_{8e}$ $R_{8f}$ $R_{8h}$ are each independently (=O), -Hal, -$CH_3$; -$OCH_3$; -$CHal_3$; -$OCHal_3$.

**14.** The compound according to claim 1, being:

4-(2-aminobenzoyl)-N-[(3S)-6,6-dimethylpiperidin-3-yl]-5-(trifluoromethyl)pyrimidin-2-amine (**CDK7_461**);
4-(2-aminobenzoyl)-N-(6,6-dimethylpiperidin-3-yl)-5-(trifluoromethyl)pyrimidin-2-amine (**CDK7_461_a**);
4-(2-aminobenzoyl)-N-[(3S)-piperidin-3-yl]-5-(trifluoromethyl)pyrimidin-2-amine (**CDK7_549**);
4-(2-aminobenzoyl)-N-(piperidin-3-yl)-5-(trifluoromethyl)pyrimidin-2-amine (**CDK7_549_a**);
4-benzoyl-N-[(3S)-piperidin-3-yl]-5-(trifluoromethyl)pyrimidin-2-amine (**CDK7_813**);
4-benzoyl-N-(piperidin-3-yl)-5-(trifluoromethyl)pyrimidin-2-amine (**CDK7_ 813_a);**
4-[(2-aminophenyl)methyl]-N-[(3S)-piperidin-3-yl]-5-(trifluoromethyl)pyrimidin-2-amine (**CDK7_ 540**);
4-[(2-aminophenyl)methyl]-N-(piperidin-3-yl)-5-(trifluoromethyl)pyrimidin-2-amine (**CDK7_540_a**);
4-[1-(2-aminophenyl)ethyl]-N-[(3S)-piperidin-3-yl]-5-(trifluoromethyl)pyrimidin-2-amine (**CDK7_541**);
4-[1-(2-aminophenyl)ethyl]-N-(piperidin-3-yl)-5-(trifluoromethyl)pyrimidin-2-amine (**CDK7_541_a**);
4-[(1S)-1-(2-aminophenyl)ethyl]-N-[(3S)-piperidin-3-yl]-5-(trifluoromethyl)pyrimidin-2-amine (**CDK7_541_b**);
4-[(1R)-1-(2-aminophenyl)ethyl]-N-[(3S)-piperidin-3-yl]-5-(trifluoromethyl)pyrimidin-2-amine (**CDK7_541_c**);
4-[2-(2-aminophenyl)propan-2-yl]-N-[(3S)-piperidin-3-yl]-5-(trifluoromethyl)pyrimidin-2-amine (**CDK7_542**);
4-[2-(2-aminophenyl)propan-2-yl]-N-(piperidin-3-yl)-5-(trifluoromethyl)pyrimidin-2-amine (**CDK7_542_a**);
4-[1-(2-aminophenyl)cyclopropyl]-N-[(3S)-piperidin-3-yl]-5-(trifluoromethyl)pyrimidin-2-amine (**CDK7_543**);
4-[1-(2-aminophenyl)cyclopropyl]-N-(piperidin-3-yl)-5-(trifluoromethyl)pyrimidin-2-amine (**CDK7_543_a**);
4-[(2-aminophenyl)difluoromethyl]-N-[(3S)-piperidin-3-yl]-5-(trifluoromethyl)pyrimidin-2-amine (**CDK7_544**);
4-[(2-aminophenyl)difluoromethyl]-N-(piperidin-3-yl)-5-(trifluoromethyl)pyrimidin-2-amine (**CDK7_544_a**);
4-[(2-aminophenyl)sulfanyl]-N-[(3S)-piperidin-3-yl]-5-(trifluoromethyl)pyrimidin-2-amine (**CDK7_545**);
4-[(2-aminophenyl)sulfanyl]-N-(piperidin-3-yl)-5-(trifluoromethyl)pyrimidin-2-amine (**CDK7_545_a**);
4-(2-aminobenzenesulfinyl)-N-[(3S)-piperidin-3-yl]-5-(trifluoromethyl)pyrimidin-2-amine (**CDK7_546**);
4-(2-aminobenzenesulfinyl)-N-(piperidin-3-yl)-5-(trifluoromethyl)pyrimidin-2-amine (**CDK7_546_a**);
4-[(R)-2-aminobenzenesulfinyl]-N-[(3S)-piperidin-3-yl]-5-(trifluoromethyl)pyrimidin-2-amine (**CDK7_546_b**);
4-[(S)-2-aminobenzenesulfinyl]-N-[(3S)-piperidin-3-yl]-5-(trifluoromethyl)pyrimidin-2-amine (**CDK7_546_c**);
4-(2-aminobenzenesulfonyl)-N-[(3S)-piperidin-3-yl]-5-trifluoromethyll)pyrimidin-2-amine (**CDK7_547**);
4-(2-aminobenzenesulfonyl)-N-(piperidin-3-yl)-5-(trifluoromethyl)pyrimidin-2-amine (**CDK7_547_a**);
4-(2-aminophenoxy)-N-[(3S)-piperidin-3-yl]-5-(trifluoromethyl)pyrimidin-2-amine (**CDK7_548**);
4-(2-aminophenoxy)-N-(piperidin-3-yl)-5-(trifluoromethyl)pyrimidin-2-amine (**CDK7_548_a**);
N4-(2-aminophenyl)-N2-[(3S)-piperidin-3-yl]-5-(trifluoromethyl)pyrimidin-2,4-diamine (**CDK7_550**);

N4-(2-aminophenyl)-N2-(piperidin-3-yl)-5-(trifluoromethyl)pyrimidin-2,4-diamine (**CDK7_550_a**);
N4-(2-aminophenyl)-N4-methyl-N2-[(3S)-piperidin-3-yl]-5-(trifluoromethyl)pyrimidin-2,4-diamine (**CDK7_551**);
N4-(2-aminophenyl)-N4-methyl-N2-(piperidin-3-yl)-5-(trifluoromethyl)pyrimidin-2,4-diamine (**CDK7_551_a**);
N4-phenyl-N2-[(3S)-piperidin-3-yl]-5-(trifluoromethyl)pyrimidin-2,4-diamine (**CDK7_812**);
N4-phenyl-N2-(piperidin-3-yl)-5-(trifluoromethyl)pyrimidin-2,4-diamine (**CDK7_812_a**);
4-phenoxy-N-[(3S)-piperidin-3-yl]-5-(trifluoromethyl)pyrimidin-2-amine (**CDK7_754**);
4-phenoxy-N-(piperidin-3-yl)-5-(trifluoromethyl)pyrimidin-2-amine (**CDK7_754_a**);
4-benzyl-N-[(3S)-piperidin-3-yl]-5-(trifluoromethyl)pyrimidin-2-amine (**CDK7_766**);
4-benzyl-N-(piperidin-3-yl)-5-(trifluoromethyl)pyrimidin-2-amine (**CDK7_766_a**);
4-[(2-aminophenyl)(methyl)phosphoryl]-N-[(3S)-piperidin-3-yl]-5-(trifluoromethyl)pyrimidin-2-amine (**CDK7_552**);
4-[(2-aminophenyl)(methyl)phosphoryl]-N-(piperidin-3-yl)-5-(trifluoromethyl)pyrimidin-2-amine (**CDK7_552_a**);
4-[(R)-(2-aminophenyl)(methyl)phosphoryl]-N-[(3S)-piperidin-3-yl]-5-(trifluoromethyl)pyrimidin-2-amine (**CDK7_552_b**);
4-[(S)-(2-aminophenyl)(methyl)phosphoryl]-N-[(3S)-piperidin-3-yl]-5-(trifluoromethyl)pyrimidin-2-amine (**CDK7_552_c**);
N4-[2-(dimethylphosphoryl)phenyl]-N2-[(3S)-piperidin-3-yl]-5-(trifluoromethyl)pyrimidin-2,4-diamine (**CDK7_735**);
N4-[2-(dimethylphosphoryl)phenyl]-N2-(piperidin-3-yl)-5-(trifluoromethyl)pyrimidin-2,4-diamine (**CDK7_735_a**);
4-[2-(dimethylphosphoryl)phenoxy]-N-[(3S)-piperidin-3-yl]-5-(trifluoromethyl)pyrimidin-2-amine (**CDK7_897**);
4-[2-(dimethylphosphoryl)phenoxy]-N-(piperidin-3-yl)-5-(trifluoromethyl)pyrimidin-2-amine (**CDK7_897_a**);
4-[3-(dimethylphosphoryl)phenoxy]-N-[(3S)-piperidin-3-yl]-5-(trifluoromethyl)pyrimidin-2-amine (**CDK7_892**);
4-[3-(dimethylphosphoryl)phenoxy]-N-(piperidin-3-yl)-5-(trifluoromethyl)pyrimidin-2-amine (**CDK7_892_a**);
N4-[3-(dimethylphosphoryl)phenyl]-N2-[(3S)-piperidin-3-yl]-5-(trifluoromethyl)pyrimidin-2,4-diamine (**CDK7_888**);
N4-[3-(dimethylphosphoryl)phenyl]-N2-(piperidin-3-yl)-5-(trifluoromethyl)pyrimidin-2,4-diamine (**CDK7_888_a**);
N4-[2-(dimethylphosphoryl)-5-methylphenyl]-N2-[(3S)-piperidin-3-yl]-5-(trifluoromethyl)pyrimidin-2,4-diamine (**CDK7_903**);
N4-[2-(dimethylphosphoryl)-5-methylphenyl]-N2-(piperidin-3-yl)-5-(trifluoromethyl)pyrimidin-2,4-diamine (**CDK7_903_a**);
N4-[2-(dimethylphosphoryl)-4-methylphenyl]-N2-[(3S)-piperidin-3-yl]-5-(trifluoromethyl)pyrimidin-2,4-diamine (**CDK7_904**);
N4-[2-(dimethylphosphoryl)-4-methylphenyl]-N2-(piperidin-3-yl)-5-(trifluoromethyl)pyrimidin-2,4-diamine (**CDK7_904_a**);
N4-[2-(dimethylphosphoryl)-5-(trifluoromethyl)phenyl]-N2-[(3S)-piperidin-3-yl]-5-(trifluoromethyl)pyrimidin-2,4-diamine (**CDK7_905**);
N4-[2-(dimethylphosphoryl)-5-(trifluoromethyl)phenyl]-N2-(piperidin-3-yl)-5-(trifluoromethyl)pyrimidin-2,4-diamine (**CDK7_905_a**);
N4-[2-(dimethylphosphoryl)-4-(trifluoromethyl)phenyl]-N2-[(3S)-piperidin-3-yl]-5-(trifluoromethyl)pyrimidin-2,4-diamine (**CDK7_906**);
N4-[2-(dimethylphosphoryl)-4-(trifluoromethyl)phenyl]-N2-(piperidin-3-yl)-5-(trifluoromethyl)pyrimidin-2,4-diamine (**CDK7_906_a**);
N4-[5-chloro-2-(dimethylphosphoryl)phenyl]-N2-[(3S)-piperidin-3-yl]-5-(trifluoromethyl)pyrimidin-2,4-diamine (**CDK7_907**);
N4-[5-chloro-2-(dimethylphosphoryl)phenyl]-N2-(piperidin-3-yl)-5-(trifluoromethyl)pyrimidin-2,4-diamine (**CDK7_907_a**);
N4-[4-chloro-2-(dimethylphosphoryl)phenyl]-N2-[(3S)-piperidin-3-yl]-5-(trifluoromethyl)pyrimidin-2,4-diamine (**CDK7_908**);
N4-[4-chloro-2-(dimethylphosphoryl)phenyl]-N2-(piperidin-3-yl)-5-(trifluoromethyl)pyrimidin-2,4-diamine (**CDK7_908_a**);
N4-[2-(dimethylphosphoryl)-4-(trifluoromethoxy)phenyl]-N2-[(3S)-piperidin-3-yl]-5-(trifluoromethyl)pyrimidin-2,4-diamine (**CDK7_915**);
N4-[2-(dimethylphosphoryl)-4-(trifluoromethoxy)phenyl]-N2-(piperidin-3-yl)-5-(trifluoromethyl)pyrimidin-2,4-diamine (**CDK7_915_a**);
2-[(2-{[(3S)-piperidin-3-yl]amino}-5-(trifluoromethyl)pyrimidin-4-yl)amino]benzene-1-sulfonamide

(**CDK7_889**);
2-({2-[(piperidin-3-yl)amino]-5-(trifluoromethyl)pyrimidin-4-yl}amino)benzene-1-sulfonamide (**CDK7_889_a**);
N4-(3-methylsulfonylphenyl)-N2-[(3S)-piperidin-3-yl]-5-(trifluoromethyl)pyrimidin-2,4-diamine (**CDK7_890**);
N4-(3-methylsulfonylphenyl)-N2-(piperidin-3-yl)-5-(trifluoromethyl)pyrimidin-2,4-diamine (**CDK7_890_a**);
3-[(2-{[(3S)-piperidin-3-yl]amino}-5-(trifluoromethyl)pyrimidin-4-yl)amino]benzene-1-sulfonamide (**CDK7_891**);
3-({2-[(piperidin-3-yl)amino]-5-(trifluoromethyl)pyrimidin-4-yl } amino]benzene-1-sulfonamide (**CDK7_891_a**);
2-[(2-{[(3S)-piperidin-3-yl]amino}-5-(trifluoromethyl)pyrimidin-4-yl)oxy]benzene-1-sulfonamide (**CDK7_893**);
2-({2-[(piperidin-3-yl)amino]-5-(trifluoromethyl)pyrimidin-4-yl}oxy)benzene-1-sulfonamide (**CDK7_893_a**);
4-(3-methylsulfonylphenoxy)-N-[(3S)-piperidin-3-yl]-5-(trifluoromethyl)pyrimidin-2-amine (**CDK7_894**);
4-(3-methylsulfonylphenoxy)-N-(piperidin-3-yl)-5-(trifluoromethyl)pyrimidin-2-amine (**CDK7_894_a**);
3-[(2-{[(3S)-piperidin-3-yl]amino}-5-(trifluoromethyl)pyrimidin-4-yl)oxy]benzene-1-sulfonamide (**CDK7_895**);
3-({2-[(piperidin-3-yl)amino]-5-(trifluoromethyl)pyrimidin-4-yl}oxy)benzene-1-sulfonamide (**CDK7_895_a**);
4-(2,3-dihydro-1H-indol-7-yloxy)-N-[(3S)-piperidin-3-yl]-5-(trifluoromethyl)pyrimidin-2-amine (**CDK7_827**);
4-(2,3-dihydro-1H-indol-7-yloxy)-N-(piperidin-3-yl)-5-(trifluoromethyl)pyrimidin-2-amine (**CDK7_827_a**);
4-(1H-indol-7-yloxy)-N-[(3S)-piperidin-3-yl]-5-(trifluoromethyl)pyrimidin-2-amine (**CDK7_828**);
4-(1H-indol-7-yloxy)-N-(piperidin-3-yl)-5-(trifluoromethyl)pyrimidin-2-amine (**CDK7_828_a**);
4-(1,2,3,4-tetrahydroquinolin-8-yloxy)-N-[(3S)-piperidin-3-yl]-5-(trifluoromethyl)pyrimidin-2-amine (**CDK7_829**);
4-(1,2,3,4-tetrahydroquinolin-8-yloxy)-N-(piperidin-3-yl)-5-(trifluoromethyl)pyrimidin-2-amine (**CDK7_829_a**);
N-[(3S)-piperidin-3-yl]-5-(trifluoromethyl)-4-(quinolin-8-yloxy)pyrimidin-2-amine (**CDK7_830**);
N-(piperidin-3-yl)-5-(trifluoromethyl)-4-(quinolin-8-yloxy)pyrimidin-2-amine (**CDK7_830_a**);
N-{2-[(2-{[(3S)-piperidin-3-yl]amino}-5-(trifluoromethyl)pyrimidin-4-yl)amino]phenyl}acetamide (**CDK7_898**);
N-[2-({2-[(piperidin-3-yl)amino]-5-(trifluoromethyl)pyrimidin-4-yl}amino)phenyl]acetamide (**CDK7_898_a**);
N-{2-[(2-{[(3S)-piperidin-3-yl]amino}-5-(trifluoromethyl)pyrimidin-4-yl)oxy]phenyl}acetamide (**CDK7_899**);
N-[2-({2-[(piperidin-3-yl)amino]-5-(trifluoromethyl)pyrimidin-4-yl}oxy)phenyl]acetamide (**CDK7_899_a**);
N-[(3S)-6,6-dimethylpiperidin-3-yl]-4-(5-phenyl-1H-pyrrol-3-yl)-5-(trifluoromethyl)pyrimidin-2-amine (**CDK7_460**);
N-(6,6-dimethylpiperidin-3-yl)-4-(5-phenyl-1H-pyrrol-3-yl)-5-(trifluoromethyl)pyrimidin-2-amine (**CDK7_460_a**);
N-[(3S)-piperidin-3-yl]- 4-(5-phenyl-1H-pyrrol-3-yl)-5-(trifluoromethyl)pyrimidin-2-amine (**CDK7_533**);
N-(piperidin-3-yl)-4-(5-phenyl-1H-pyrrol-3-yl)-5-(trifluoromethyl)pyrimidin-2-amine (**CDK7_533_a**);
4-(4-phenyl-1H-pyrrol-3-yl)-N-[(3S)-piperidin-3-yl]-4-(4-phenyl-1H-pyrrol-3-yl)-5-(trifluoromethyl)pyrimidin-2-amine (**CDK7_534**);
4-(4-phenyl-1H-pyrrol-3-yl)-N-[piperidin-3-yl]-4-(4-phenyl-1H-pyrrol-3-yl)-5-(trifluoromethyl)pyrimidin-2-amine **(CDK7_534_a);**
4-(5-benzoyl-1H-pyrrol-3-yl)-N-[(3S)-piperidin-3-yl]-5-(trifluoromethyl)pyrimidin-2-amine (**CDK7_535**);
4-(5-benzoyl-1H-pyrrol-3-yl)-N-(piperidin-3-yl)-5-(trifluoromethyl)pyrimidin-2-amine (**CDK7_535_a**);
N,N-dimethyl-4-(2-{[(3S)-piperidin-3-yl]amino}-5-(trifluoromethyl)pyrimidin-4-yl)-1H-pyrrol-2-carboxamide (**CDK7_536**);
N,N-dimethyl-4-{2-[(piperidin-3-yl)amino]-5-(trifluoromethyl)pyrimidin-4-yl}-1H-pyrrol-2-carboxamide (**CDK7_536_a**);
4-(2-{[(3S)-piperidin-3-yl]amino}-5-(trifluoromethyl)pyrimidin-4-yl)-1H-pyrrol-2-carboxamide (**CDK7_537**);
4-{2-[(piperidin-3-yl)amino]-5-(trifluoromethyl)pyrimidin-4-yl}-1H-pyrrol-2-carboxamide (**CDK7_537_a**);
N-phenyl-4-(2-{[(3S)-piperidin-3-yl]amino}-5-(trifluoromethyl)pyrimidin-4-yl)-1H-pyrrol-2-carboxamide (**CDK7_538**);
N-phenyl-4-{2-[(piperidin-3-yl)amino]-5-(trifluoromethyl)pyrimidin-4-yl}-1H-pyrrol-2-carboxamide (**CDK7_538_a**);
4-[5-(4-methylpiperazine-1-carbonyl)-1H-pyrrol-3-yl]-N-[(3S)-piperidin-3-yl]-5-(trifluoromethyl)pyrimidin-2-amine (**CDK7_539**);
4-[5-(4-methylpiperazine-1-carbonyl)-1H-pyrrol-3-yl]-N-(piperidin-3-yl)-5-(trifluoromethyl)pyrimidin-2-amine (**CDK7_539_a**);
4-(2-{[(3S)-piperidin-3-yl]amino}-5-(trifluoromethyl)pyrimidin-4-yl)-1H-pyrrol-2-carboxylic acid (**CDK7_922**);
4-{2-[(piperidin-3-yl)amino]-5-(trifluoromethyl)pyrimidin-4-yl}-1H-pyrrol-2-carboxylic acid (**CDK7_922_a**);
4-[4-(2-{[(3S)-piperidin-3-yl]amino}-5-(trifluoromethyl)pyrimidin-4-yl)-1H-pyrrol-2-yl]benzonitrile (**CDK7_695**);
4-(4-{2-[(piperidin-3-yl)amino]-5-(trifluoromethyl)pyrimidin-4-yl}-1H-pyrrol-2-yl)benzonitrile (**CDK7_695_a**);
3-[4-(2-{[(3S)-piperidin-3-yl]amino}-5-(trifluoromethyl)pyrimidin-4-yl)-1H-pyrrol-2-yl]benzonitrile (**CDK7_696**);
3-(4-{2-[(piperidin-3-yl)amino]-5-(trifluoromethyl)pyrimidin-4-yl}-1H-pyrrol-2-yl)benzonitrile (**CDK7_696_a**);

3-(2-{[(3S)-piperidin-3-yl]amino}-5-(trifluoromethyl)pyrimidin-4-yl)-1H,4H,5H,6H,7H-pyrrolo[2,3-c]pyridin-7-one (**CDK7_814**);
3-{2-[(piperidin-3-yl)amino]-5-(trifluoromethyl)pyrimidin-4-yl }-1H,4H, SH, 6H,7H-pyrrolo[2,3-c]pyridin-7-one (**CDK7_814_a**);
3-(2-{[(3S)-piperidin-3-yl]amino}-5-(trifluoromethyl)pyrimidin-4-yl)-1H,4H,5H,6H,7H-pyrrolo[3,2-c]pyridin-4-one (**CDK7_815**);
3-{2-[(piperidin-3-yl)amino]-5-(trifluoromethyl)pyrimidin-4-yl }-1H,4H, SH, 6H,7H-pyrrolo[3,2-c]pyridin-4-one (**CDK7_815_a**);
6-methyl-3-(2-{[(3S)-piperidin-3-yl]amino}-5-(trifluoromethyl)pyrimidin-4-yl)-1H,4H,5H,6H,7H-pyrrolo[2,3-c]pyridin-7-one (**CDK7_843**);
6-methyl-3-{2-[(piperidin-3-yl)amino]-5-(trifluoromethyl)pyrimidin-4-yl}-1H,4H,5H,6H,7H-pyrrolo[2,3-c]pyridin-7-one (**CDK7_843_a**);
3-(2-{[(3S)-piperidin-3-yl]amino}-5-(trifluoromethyl)pyrimidin-4-yl)-1H,4H,5H,6H,7H-pyrrolo[2,3-c]pyridin-4-one (**CDK7_844**);
3-{2-[(piperidin-3-yl)amino]-5-(trifluoromethyl)pyrimidin-4-yl }-1H,4H, SH, 6H,7H-pyrrolo[2,3-c]pyridin-4-one (**CDK7_844_a**);
6-methyl-3-(2-{[(3S)-piperidin-3-yl]amino}-5-(trifluoromethyl)pyrimidin-4-yl)-1H,6H,7H-pyrrolo[2,3-c]pyridin-7-one (**CDK7_845**);
6-methyl-3-{2-[(piperidin-3-yl)amino]-5-(trifluoromethyl)pyrimidin-4-yl }-1H,6H,7H-pyrrolo[2,3-c]pyridin-7-one (**CDK7_845_a**);
N-(1H-imidazol-2-yl)-4-(2-{[(3S)-piperidin-3-yl]amino}-5-(trifluoromethyl)pyrimidin-4-yl)-1H-pyrrol-2-carboxamide (**CDK7_833**);
N-(1H-imidazol-2-yl)-4-{2-[(piperidin-3-yl)amino]-5-(trifluoromethyl)pyrimidin-4-yl}-1H-pyrrol-2-carboxamide (**CDK7_833_a**);
4-(2-{[(3S)-piperidin-3-yl]amino}-5-(trifluoromethyl)pyrimidin-4-yl)-N-(1,3-thiazol-2-yl)-1H-pyrrol-2-carboxamide (**CDK7_834**);
4-{2-[(piperidin-3-yl)amino]-5-(trifluoromethyl)pyrimidin-4-yl}-N-(1,3-thiazol-2-yl)-1H-pyrrol-2-carboxamide (**CDK7_834_a**);
N-(1-methyl-1H-pyrazol-4-yl)-4-(2-{[(3S)-piperidin-3-yl]amino}-5-(trifluoromethyl)pyrimidin-4-yl)-1H-pyrrol-2-carboxamide (**CDK7_835**);
N-(1-methyl-1H-pyrazol-4-yl)-4-{2-[(piperidin-3-yl)amino]-5-(trifluoromethyl)pyrimidin-4-yl}-1H-pyrrol-2-carboxamide (**CDK7_835_a**);
4-(2-{[(3S)-piperidin-3-yl]amino}-5-(trifluoromethyl)pyrimidin-4-yl)-N-(pyridin-4-yl)-1H-pyrrol-2-carboxamide (**CDK7_836**);
4-{2-[(piperidin-3-yl)amino]-5-(trifluoromethyl)pyrimidin-4-yl}-N-(pyridin-4-yl)-1H-pyrrol-2-carboxamide (**CDK7_836_a**);
N-(4-cyanophenyl)-4-(2-{[(3S)-piperidin-3-yl]amino}-5-(trifluoromethyl)pyrimidin-4-yl)-1H-pyrrol-2-carboxamide (**CDK7_837**);
N-(4-cyanophenyl)-4-{2-[(piperidin-3-yl)amino]-5-(trifluoromethyl)pyrimidin-4-yl}-1H-pyrrol-2-carboxamide (**CDK7_837_a**);
N-[4-(morpholin-4-yl)phenyl]-4-(2-{[(3S)-piperidin-3-yl]amino}-5-(trifluoromethyl)pyrimidin-4-yl)-1H-pyrrol-2-carboxamide (**CDK7_838**);
N-[4-(morpholin-4-yl)phenyl]-4-{2-[(piperidin-3-yl)amino]-5-(trifluoromethyl)pyrimidin-4-yl}-1H-pyrrol-2-carboxamide (**CDK7_838_a**);
N-methyl-4-(2-{[(3S)-piperidin-3-yl]amino}-5-(trifluoromethyl)pyrimidin-4-yl)-1H-pyrrol-2-carboxamide (**CDK7_846**);
N-methyl-4-{2-[(piperidin-3-yl)amino]-5-(trifluoromethyl)pyrimidin-4-yl}-1H-pyrrol-2-carboxamide (**CDK7_846_a**);
4-(2-{[(3S)-piperidin-3-yl]amino}-5-(trifluoromethyl)pyrimidin-4-yl)-N-(2,2,2-trifluoroethyl)-1H-pyrrol-2-carboxamide (**CDK7_847**);
4-{2-[(piperidin-3-yl)amino]-5-(trifluoromethyl)pyrimidin-4-yl}-N-(2,2,2-trifluoroethyl)-1H-pyrrol-2-carboxamide (**CDK7_847_a**);
N-(2-methanesulfonylethyl)-4-(2-{[(3S)-piperidin-3-yl]amino}-5-(trifluoromethyl)pyrimidin-4-yl)-1H-pyrrol-2-carboxamide (**CDK7_848**);
N-(2-methanesulfonylethyl)-4-{2-[(piperidin-3-yl)amino]-5-(trifluoromethyl)pyrimidin-4-yl}-1H-pyrrol-2-carboxamide (**CDK7_848_a**);
4-[5-(morpholin-4-carbonyl)-1H-pyrrol-3-yl]-N-[(3 S)-piperidin-3-yl]-5-(trifluoromethyll)pyrimidin-2-amine (**CDK7_849**);

4-[5-(morpholin-4-carbonyl)-1H-pyrrol-3-yl]-N-(piperidin-3-yl)-5-(trifluoromethyl)pyrimidin-2-amine (**CDK7_849_a**);
N-(oxan-4-yl)-4-(2-{[(3S)-piperidin-3-yl]amino}-5-(trifluoromethyl)pyrimidin-4-yl)-1H-pyrrol-2-carboxamide (**CDK7_850**);
N-(oxan-4-yl)-4-{2-[(piperidin-3-yl)amino]-5-(trifluoromethyl)pyrimidin-4-yl}-1H-pyrrol-2-carboxamide (**CDK7_850_a**);
N-(4-hydroxycyclohexyl)-4-(2-{[(3S)-piperidin-3-yl]amino}-5-(trifluoromethyl)pyrimidin-4-yl)-1H-pyrrol-2-carboxamide (**CDK7_851**);
N-(4-hydroxycyclohexyl)-4-{2-[(piperidin-3-yl)amino]-5-(trifluoromethyl)pyrimidin-4-yl}-1H-pyrrol-2-carboxamide (**CDK7_851_a**);
N-(4-cyanocyclohexyl)-4-(2-{[(3S)-piperidin-3-yl]amino}-5-(trifluoromethyl)pyrimidin-4-yl)-1H-pyrrol-2-carboxamide (**CDK7_852**);
N-(4-cyanocyclohexyl)-4-{2-[(piperidin-3-yl)amino]-5-(trifluoromethyl)pyrimidin-4-yl}-1H-pyrrol-2-carboxamide (**CDK7_852_a**);
N-cyclopropyl-4-(2-{[(3S)-piperidin-3-yl]amino}-5-(trifluoromethyl)pyrimidin-4-yl)-1H-pyrrol-2-carboxamide (**CDK7_951**);
N-cyclopropyl-4-{2-[(piperidin-3-yl)amino]-5-(trifluoromethyl)pyrimidin-4-yl}-1H-pyrrol-2-carboxamide (**CDK7_951_a**);
6-(3,5-dimethyl-1,2-oxazol-4-yl)-3-(2-{[(3S)-piperidin-3-yl]amino}-5-(trifluoromethyl)pyrimidin-4-yl)-1H,4H,5H,6H,7H-pyrrolo[2,3-c]pyridin-7-one (**CDK7_923**);
6-(3,5-dimethyl-1,2-oxazol-4-yl)-3-{2-[(piperidin-3-yl)amino]-5-(trifluoromethyl) pyrimidin-4-yl }-1H,4H,5H,6H,7H-pyrrolo[2,3-c]pyridin-7-one (**CDK7_923_a**);
6-(3,5-dimethyl-1,2-oxazol-4-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_924**);
6-(3,5-dimethyl-1,2-oxazol-4-yl)-3-{2-[(piperidin-3-yl)amino]-5-(trifluoromethyl)pyrimidin-4-yl}-1H,6H,7H-pyrrolo[2,3-c]pyridin-7-one (**CDK7_924_a**);
3-(2-{[(3S)-piperidin-3-yl]amino}-5-(trifluoromethyl)pyrimidin-4-yl)-1H,4H,7H,8H-pyrrolo[2,3-c]azepin-8-one (**CDK7_925**);
3-{2-[(piperidin-3-yl)amino]-5-(trifluoromethyl)pyrimidin-4-yl }-1H,4H,7H, 8H-pyrrolo[2,3-c]azepin-8-one **(CDK7_925_a);**
3-(2-{[(3S)-piperidin-3-yl]amino}-5-(trifluoromethyl)pyrimidin-4-yl)-1H,6H,7H,8H-pyrrolo[2,3-c]azepin-8-one (**CDK7_926**);
3-{2-[(piperidin-3-yl)amino]-5-(trifluoromethyl)pyrimidin-4-yl }-1H, 6H,7H, 8H-pyrrolo[2,3-c]azepin-8-one **(CDK7_926_a);**
3-(2-{[(3S)-piperidin-3-yl]amino}-5-(trifluoromethyl)pyrimidin-4-yl)-1H,8H-pyrrolo[2,3-c]azepin-8-one (**CDK7_927**);
3-{2-[(piperidin-3-yl)amino]-5-(trifluoromethyl)pyrimidin-4-yl }-1H, 8H-pyrrolo[2,3-c]azepin-8-one (**CDK7_927_a**);
7-methyl-3-(2-{[(3S)-piperidin-3-yl]amino}-5-(trifluoromethyl)pyrimidin-4-yl)-1H,4H,7H,8H-pyrrolo[2,3-c]azepin-8-one (**CDK7_928**);
7-methyl-3-{2-[(piperidin-3-yl)amino]-5-(trifluoromethyl)pyrimidin-4-yl}-1H,4H,7H,8H-pyrrolo[2,3-c]azepin-8-one **(CDK7_928_a);**
7-methyl-3-(2-{[(3S)-piperidin-3-yl]amino}-5-(trifluoromethyl)pyrimidin-4-yl)-1H,6H,7H,8H-pyrrolo[2,3-c]azepin-8-one (**CDK7_929**);
7-methyl-3-{2-[(piperidin-3-yl)amino]-5-(trifluoromethyl)pyrimidin-4-yl }-1H,6H,7H, 8H-pyrrolo[2,3-c]azepin-8-one (**CDK7_929_a**);
7-methyl-3-(2-{[(3S)-piperidin-3-yl]amino}-5-(trifluoromethyl)pyrimidin-4-yl)-7H,8H-pyrrolo[2,3-c]azepin-8-one (**CDK7_930**);
7-methyl-3-{2-[(piperidin-3-yl)amino]-5-(trifluoromethyl)pyrimidin-4-yl}-7H,8H-pyrrolo[2,3-c]azepin-8-one **(CDK7_930_a);**
3-(2-{ [(3 S)-piperidin-3-yl] amino}-5-(trifluoromethyl)pyrimidin-4-yl)-7H, 8H-pyrrolo[2,3-c]azepin-8-one (**CDK7_931**);
3-{2-[(piperidin-3-yl)amino]-5-(trifluoromethyl)pyrimidin-4-yl }-7H, 8H-pyrrolo[2,3-c]azepin-8-one (**CDK7_931_a**);
3-(2-{[(3S)-piperidin-3-yl]amino}-5-(trifluoromethyl)pyrimidin-4-yl)-1H,4H,5H,6H,7H,8H,9H-pyrrolo[2,3-c]azocin-9-one (**CDK7_932**);
3-{2-[(piperidin-3-yl)amino]-5-(trifluoromethyl)pyrimidin-4-yl}-1H,4H,5H,6H,7H,8H,9H-pyrrolo[2,3-c]azocin-9-one (**CDK7_932_a**);

8-methyl-3-(2-{[(3S)-piperidin-3-yl]amino}-5-(trifluoromethyl)pyrimidin-4-yl)-1H,4H,5H,6H,7H,8H,9H-pyrrolo[2,3-c]azocin-9-one (**CDK7_933**);
8-methyl-3-{2-[(piperidin-3-yl)amino]-5-(trifluoromethyl)pyrimidin-4-yl}-1H,4H,5H,6H,7H,8H,9H-pyrrolo[2,3-c]azocin-9-one (**CDK7_933_a**);
3-(2-{[(3S)-piperidin-3-yl]amino}-5-(trifluoromethyl)pyrimidin-4-yl)-1H,4H,5H,8H,9H-pyrrolo[2,3-c]azocin-9-one (**CDK7_934**);
3-{2-[(piperidin-3-yl)amino]-5-(trifluoromethyl)pyrimidin-4-yl }-1H,4H, SH, 8H,9H-pyrrolo[2,3-c]azocin-9-one (**CDK7_934_a**);
8-methyl-3-(2-{[(3S)-piperidin-3-yl]amino}-5-(trifluoromethyl)pyrimidin-4-yl)-1H,4H,5H,8H,9H-pyrrolo[2,3-c]azocin-9-one (**CDK7_935**);
8-methyl-3-{2-[(piperidin-3-yl)amino]-5-(trifluoromethyl)pyrimidin-4-yl}-1H,4H,5H,8H,9H-pyrrolo[2,3-c]azocin-9-one (**CDK7_935_a**);
3-(2-{[(3S)-piperidin-3-yl]amino}-5-(trifluoromethyl)pyrimidin-4-yl)-1H,4H,7H,8H,9H-pyrrolo[2,3-c]azocin-9-one (**CDK7_936**);
3-{2-[(piperidin-3-yl)amino]-5-(trifluoromethyl)pyrimidin-4-yl }-1H,4H,7H, 8H,9H-pyrrolo[2,3-c]azocin-9-one (**CDK7_936_a**);
8-methyl-3-(2-{[(3S)-piperidin-3-yl]amino}-5-(trifluoromethyl)pyrimidin-4-yl)-1H,4H,7H,8H,9H-pyrrolo[2,3-c]azocin-9-one (**CDK7_937**);
8-methyl-3-{2-[(piperidin-3-yl)amino]-5-(trifluoromethyl)pyrimidin-4-yl}-1H,4H,7H,8H,9H-pyrrolo[2,3-c]azocin-9-one (**CDK7_937_a**);
3-(2-{[(3S)-piperidin-3-yl]amino}-5-(trifluoromethyl)pyrimidin-4-yl)-1H,6H,7H,8H,9H-pyrrolo[2,3-c]azocin-9-one (**CDK7_938**);
3-{2-[(piperidin-3-yl)amino]-5-(trifluoromethyl)pyrimidin-4-yl }-1H, 6H,7H, 8H,9H-pyrrolo[2,3-c]azocin-9-one (**CDK7_938_a**);
8-methyl-3-(2-{[(3S)-piperidin-3-yl]amino}-5-(trifluoromethyl)pyrimidin-4-yl)-1H,6H,7H,8H,9H-pyrrolo[2,3-c]azocin-9-one (**CDK7_939**);
8-methyl-3-{2-[(piperidin-3-yl)amino]-5-(trifluoromethyl)pyrimidin-4-yl}-1H,6H,7H,8H,9H-pyrrolo[2,3-c]azocin-9-one (**CDK7_939_a**);
3-(2-{[(3S)-piperidin-3-yl]amino}-5-(trifluoromethyl)pyrimidin-4-yl)-1H,8H,9H-pyrrolo[2,3-c]azocin-9-one (**CDK7_940**);
3-{2-[(piperidin-3-yl)amino]-5-(trifluoromethyl)pyrimidin-4-yl }-1H, 8H, 9H-pyrrolo[2,3-c]azocin-9-one (**CDK7_940_a**);
8-methyl-3-(2-{[(3S)-piperidin-3-yl]amino}-5-(trifluoromethyl)pyrimidin-4-yl)-1H,8H,9H-pyrrolo[2,3-c]azocin-9-one (**CDK7_941**);
8-methyl-3-{2-[(piperidin-3-yl)amino]-5-(trifluoromethyl)pyrimidin-4-yl}-1H,8H,9H-pyrrolo[2,3-c]azocin-9-one (**CDK7_941_a**);
3-(2-{[(3S)-piperidin-3-yl]amino}-5-(trifluoromethyl)pyrimidin-4-yl)-9H-pyrrolo[2,3-c]azocin-9-one (**CDK7_942**);
3-{2-[(piperidin-3-yl)amino]-5-(trifluoromethyl)pyrimidin-4-yl}-9H-pyrrolo[2,3-c]azocin-9-one (**CDK7_942_a**);
3-(2-{[(3S)-piperidin-3-yl]amino}-5-(trifluoromethyl)pyrimidin-4-yl)-1H,4H,5H,6H-pyrrolo[2,3-c]pyrrol-6-one (**CDK7_943**);
3-{2-[(piperidin-3-yl)amino]-5-(trifluoromethyl)pyrimidin-4-yl }-1H,4H, SH, 6H-pyrrolo[2,3-c]pyrrol-6-one (**CDK7_943_a**);
5-methyl-3-(2-{[(3S)-piperidin-3-yl]amino}-5-(trifluoromethyl)pyrimidin-4-yl)-1H,4H,5H,6H-pyrrolo[2,3-c]pyrrol-6-one (**CDK7_944**);
5-methyl-3-{2-[(piperidin-3-yl)amino]-5-(trifluoromethyl)pyrimidin-4-yl }-1H,4H,5H,6H-pyrrolo[2,3-c]pyrrol-6-one (**CDK7_944_a**);
N-[(3 S)-piperidin-3-yl]-4-(1H-pyrazol-4-yl)-5-(trifluoromethyl)pyrimidin-2-amine (**CDK7_945**);
N-(piperidin-3-yl)-4-(1H-pyrazol-4-yl)-5-(trifluoromethyl)pyrimidin-2-amine (**CDK7_945_a**);
4-(1H-imidazol-4-yl)-N-[(3 S)-piperidin-3-yl]-5-(trifluoromethyl)pyrimidin-2-amine (**CDK7_946**);
4-(1H-imidazol-4-yl)-N-(piperidin-3-yl)-5-(trifluoromethyl)pyrimidin-2-amine (**CDK7_946_a**);
N-[(3 S)-piperidin-3-yl]-4-(1H-pyrazol-3-yl)-5-(trifluoromethyl)pyrimidin-2-amine (**CDK7_947**);
N-(piperidin-3-yl)-4-(1H-pyrazol-3-yl)-5-(trifluoromethyl)pyrimidin-2-amine (**CDK7_947_a**);
N-[(3 S)-piperidin-3-yl]-4-(1H-1,2,3-triazol-4-yl)-5-(trifluoromethyl)pyrimidin-2-amine (**CDK7_948**);
N-(piperidin-3-yl)-4-(1H-1,2,3-triazol-4-yl)-5-(trifluoromethyl)pyrimidin-2-amine (**CDK7_948_a**);
N-[(3 S)-piperidin-3-yl]-4-(1H-1,2,4-triazol-3-yl)-5-(trifluoromethyl)pyrimidin-2-amine (**CDK7_949**);
N-(piperidin-3-yl)-4-(1H-1,2,4-triazol-3-yl)-5-(trifluoromethyl)pyrimidin-2-amine (**CDK7_949_a**);
N-[(3 S)-piperidin-3-yl]-4-(2H-1,2,3,4-tetrazol-5-yl)-5-(trifluoromethyl)pyrimidin-2-amine (**CDK7_950**);
N-(piperidin-3-yl)-4-(2H-1,2,3,4-tetrazol-5-yl)-5-(trifluoromethyl)pyrimidin-2-amine (**CDK7_950_a**);

4-(2-{ [(3 S)-piperidin-3-yl]amino}-5-(trifluoromethyl)pyrimidin-4-yl)-1,2,3,4-tetrahydroquinoxalin-2-one (**CDK7_900**);
4-{2-[(piperidin-3-yl)amino]-5-(trifluoromethyl)pyrimidin-4-yl}-1,2,3,4-tetrahydroquinoxalin-2-one (**CDK7_900_a**);
4-(2-{[(3S)-piperidin-3-yl]amino}-5-(trifluoromethyl)pyrimidin-4-yl)-1,2-dihydroquinolin-2-one (**CDK7_901**);
4-{2-[(piperidin-3-yl)amino]-5-(trifluoromethyl)pyrimidin-4-yl}-1,2-dihydroquinolin-2-one (**CDK7_901_a**);
4-(2-{[(3S)-piperidin-3-yl]amino}-5-(trifluoromethyl)pyrimidin-4-yl)-1,2-dihydroisoquinolin-1-one (**CDK7_921**);
4-{2-[(piperidin-3-yl)amino]-5-(trifluoromethyl)pyrimidin-4-yl}-1,2-dihydroisoquinolin-1-one (**CDK7_921_a**);
N-cyclopropyl-N-methyl-4-(2-{[(3S)-piperidin-3-yl]amino}-5-(trifluoromethyl)pyrimidin-4-yl)-1H-pyrrol-2-carboxamide (**CDK7_1001**);
N-cyclopropyl-N-methyl-4-{2-[(piperidin-3-yl)amino]-5-(trifluoromethyl)pyrimidin-4-yl}-1H-pyrrol-2-carboxamide (**CDK7_1001_a**);
N-methyl-4-(2-{[(3S)-piperidin-3-yl]amino}-5-(trifluoromethyl)pyrimidin-4-yl)-N-(1,3-thiazol-2-yl)-1H-pyrrol-2-carboxamide (**CDK7_1003**);
N-methyl-4-{2-[(piperidin-3-yl)amino]-5-(trifluoromethyl)pyrimidin-4-yl}-N-(1,3-thiazol-2-yl)-1H-pyrrol-2-carboxamide (**CDK7_1003_a**);
N-benzyl-4-(2-{[(3S)-piperidin-3-yl]amino}-5-(trifluoromethyl)pyrimidin-4-yl)-1H-pyrrol-2-carboxamide (**CDK7_997**);
N-benzyl-4-{2-[(piperidin-3-yl)amino]-5-(trifluoromethyl)pyrimidin-4-yl}-1H-pyrrol-2-carboxamide (**CDK7_997_a)**;
N-benzyl-N-methyl-4-(2-{[(3S)-piperidin-3-yl]amino}-5-(trifluoromethyl)pyrimidin-4-yl)-1H-pyrrol-2-carboxamide (**CDK7_998**);
N-benzyl-N-methyl-4-{2-[(piperidin-3-yl)amino]-5-(trifluoromethyl)pyrimidin-4-yl}-1H-pyrrol-2-carboxamide (**CDK7_998_a**);
4-[5-(1-methyl-1H-1,2,3,4-tetrazol-5-yl)-1H-pyrrol-3-yl]-N-[(3S)-piperidin-3-yl]-5-(trifluoromethyl)pyrimidin-2-amine (**CDK7_999**);
4-[5-(1-methyl-1H-1,2,3,4-tetrazol-5-yl)-1H-pyrrol-3-yl]-N-(piperidin-3-yl)-5-(trifluoromethyl)pyrimidin-2-amine (**CDK7_999_a**);
4-[5-(2-methyl-2H-1,2,3,4-tetrazol-5-yl)-1H-pyrrol-3-yl]-N-[(3S)-piperidin-3-yl]-5-(trifluoromethyl)pyrimidin-2-amine (**CDK7_1000**);
4-[5-(2-methyl-2H-1,2,3,4-tetrazol-5-yl)-1H-pyrrol-3-yl]-N-(piperidin-3-yl)-5-(trifluoromethyl)pyrimidin-2-amine (**CDK7_1000_a**);
4-[5-(5-methyl-1,2,4-oxadiazol-3-yl)-1H-pyrrol-3-yl]-N-[(3 S)-piperidin-3-yl]-5-(trifluoromethyl)pyrimidin-2-amine (**CDK7_1060**);
4-[5-(5-methyl-1,2,4-oxadiazol-3-yl)-1H-pyrrol-3-yl]-N-(piperidin-3-yl)-5-(trifluoromethyl)pyrimidin-2-amine (**CDK7_1060_a**);
4-[5-(1-cyclopropyl-1H-1,2,3,4-tetrazol-5-yl)-1H-pyrrol-3-yl]-N-[(3 S)-piperidin-3-yl]-5-(trifluoromethyl)pyrimidin-2-amine (**CDK7_1102**);
4-[5-(1-cyclopropyl-1H-1,2,3,4-tetrazol-5-yl)-1H-pyrrol-3-yl]-N-(piperidin-3-yl)-5-(trifluoromethyl)pyrimidin-2-amine (**CDK7_1102_a**);
4-[5-(5-cyclopropyl-1,2,4-oxadiazol-3-yl)-1H-pyrrol-3-yl]-N-[(3S)-piperidin-3-yl]-5-(trifluoromethyl)pyrimidin-2-amine (**CDK7_1103**);
4-[5-(5-cyclopropyl-1,2,4-oxadiazol-3-yl)-1H-pyrrol-3-yl]-N-(piperidin-3-yl)-5-(trifluoromethyl)pyrimidin-2-amine (**CDK7_1103_a**);
4-[5-(1-methyl-1H-1,3-benzodiazol-2-yl)-1H-pyrrol-3-yl]-N-[(3S)piperidin-3-yl]-5-(trifluoromethyl)pyrimidin-2-amine (**CDK7_1124**);
4-[5-(1-methyl-1H-1,3-benzodiazol-2-yl)-1H-pyrrol-3-yl]-N-(piperidin-3-yl)-5-(trifluoromethyl)pyrimidin-2-amine (**CDK7_1124_a**);
ethyl 4-(2-{[(3S)-piperidin-3-yl]amino}-5-(trifluoromethyl)pyrimidin-4-yl)-1H-pyrrol-2-carboxylate (**CDK7_1020**);
ethyl 4-{2-[(piperidin-3-yl)amino]-5-(trifluoromethyl)pyrimidin-4-yl}-1H-pyrrol-2-carboxylate (**CDK7_1020_a**);
N,N,1-trimethyl-4-(2-{[(3S)-piperidin-3-yl]amino}-5-(trifluoromethyl)pyrimidin-4-yl)-1H-pyrrol-2-carboxamide (**CDK7_1017**);
N,N,1-trimethyl-4-{2-[(piperidin-3-yl)amino]-5-(trifluoromethyl)pyrimidin-4-yl}-1H-pyrrol-2-carboxamide (**CDK7_1017_a**);
N-[(3S)-piperidin-3-yl]-4-[5-(pyridin-2-yl)-1H-pyrrol-3-yl]-5-(trifluoromethyl)pyrimidin-2-amine (**CDK7_1061**);
N-(piperidin-3-yl)-4-[5-(pyridin-2-yl)-1H-pyrrol-3-yl]-5-(trifluoromethyl)pyrimidin-2-amine (**CDK7_1061_a**);
N-[(3S)-piperidin-3-yl]-4-[5-(pyrimidin-2-yl)-1H-pyrrol-3-yl]-5-(trifluoromethyl)pyrimidin-2-amine (**CDK7_1062**);
N-(piperidin-3-yl)-4-[5-(pyrimidin-2-yl)-1H-pyrrol-3-yl]-5-(trifluoromethyl)pyrimidin-2-amine (**CDK7_1062_a**);

4-{5-[2-(dimethylphosphoryl)phenyl]-1H-pyrrol-3-yl}-N-[(3S)-piperidin-3-yl]-5-(trifluoromethyl)pyrimidin-2-amine (**CDK7_1057**);

4-{5-[2-(dimethylphosphoryl)phenyl]-1H-pyrrol-3-yl}-N-(piperidin-3-yl)-5-(trifluoromethyl)pyrimidin-2-amine (**CDK7_1057_a**);

7-(1-methyl-1H-pyrazol-4-yl)-3-(2-{[(3S)-piperidin-3-yl]amino}-5-(trifluoromethyl)pyrimidin-4-yl)-1H,4H,5H,6H,7H,8H-pyrrolo[2,3-c]azepin-8-one (**CDK7_955**);

7-(1-methyl-1H-pyrazol-4-yl)-3-{2-[(piperidin-3-yl)amino]-5-(trifluoromethyl)pyrimidin-4-yl}-1H,4H,5H,6H,7H,8H-pyrrolo[2,3-c]azepin-8-one (**CDK7_955_a**);

3-(2-{[(3S)-6,6-dimethylpiperidin-3-yl]amino}-5-(trifluoromethyl)pyrimidin-4-yl)-7-(1-methyl-1H-pyrazol-4-yl)-1H,4H,5H,6H,7H,8H-pyrrolo[2,3-c]azepin-8-one (**CDK7_1122**);

3-{2-[(6,6-dimethylpiperidin-3-yl)amino]-5-(trifluoromethyl)pyrimidin-4-yl}-7-(1-methyl-1H-pyrazol-4-yl)-1H,4H,5H,6H,7H,8H-pyrrolo[2,3-c]azepin-8-one (CDK7_1122_a);

3-(2-{[(3S)-6,6-dimethylpiperidin-3-yl]amino}-5-(trifluoromethyl)pyrimidin-4-yl)-6-(1-methyl-1H-pyrazol-4-yl)-1H,6H, 7H-pyrrolo[2,3-c]pyridin-7-one (CDK7_1123);

3-{2-[(6,6-dimethylpiperidin-3-yl)amino]-5-(trifluoromethyl)pyrimidin-4-yl}-6-(1-methyl-1H-pyrazol-4-yl)-1H,6H,7H-pyrrolo[2,3-c]pyridin-7-one (CDK7_1123_a);

3-(2-{[(6S)-4-azaspiro[2.5]octan-6-yl]amino}-5-(trifluoromethyl)pyrimidin-4-yl)-7-(1-methyl-1H-pyrazol-4-yl)-1H,4H,5H,6H,7H,8H-pyrrolo[2,3-c]azepin-8-one (CDK7_1136);

3-[2-({4-azaspiro[2.5]octan-6-yl}amino)-5-(trifluoromethyl)pyrimidin-4-yl]-7-(1-methyl-1H-pyrazol-4-yl)-1H,4H,5H,6H,7H,8H-pyrrolo[2,3-c]azepin-8-one (CDK7_1136_a);

3-(2-{[(7S)-5-azaspiro[3.5]nonan-7-yl]amino}-5-(trifluoromethyl)pyrimidin-4-yl)-7-(1-methyl-1H-pyrazol-4-yl)-1H,4H,5H,6H,7H,8H-pyrrolo[2,3-c]azepin-8-one (**CDK7_1137**);

3-[2-({5-azaspiro[3.5]nonan-7-yl}amino)-5-(trifluoromethyl)pyrimidin-4-yl]-7-(1-methyl-1H-pyrazol-4-yl)-1H,4H,5H,6H,7H,8H-pyrrolo[2,3-c]azepin-8-one (**CDK7_1137_a**);

3-(2-{[(3S)-6,6-dimethylpiperidin-3-yl]amino}-5-(trifluoromethyl)pyrimidin-4-yl)-7-(1,3-thiazol-2-yl)-1H,4H,5H,6H,7H,8H-pyrrolo[2,3-c]azepin-8-one (**CDK7_1138**);

3-{2-[(6,6-dimethylpiperidin-3-yl)amino]-5-(trifluoromethyl)pyrimidin-4-yl}-7-(1,3-thiazol-2-yl)-1H,4H,5H,6H,7H,8H-pyrrolo[2,3-c]azepin-8-one (**CDK7_1138_a**);

7-(3,5-dimethyl-1,2-oxazol-4-yl)-3-(2-{[(3S)-6,6-dimethylpiperidin-3-yl]amino}-5-(trifluoromethyl)pyrimidin-4-yl)-1H,4H,5H,6H,7H,8H-pyrrolo[2,3-c]azepin-8-one (**CDK7_1139**);

7-(3,5-dimethyl-1,2-oxazol-4-yl)-3-{2-[(6,6-dimethylpiperidin-3-yl)amino]-5-(trifluoromethyl)pyrimidin-4-yl}-1H,4H,5H,6H,7H,8H-pyrrolo[2,3-c]azepin-8-one (**CDK7_1139_a**);

5-[3-(2-{[(3S)-6,6-dimethylpiperidin-3-yl]amino}-5-(trifluoromethyl)pyrimidin-4-yl)-8-oxo-1H,4H,5H,6H,7H,8H-pyrrolo[2,3-c]azepin-7-yl]-1-methyl-1,2-dihydropyridin-2-one (**CDK7_1140**);

5-(3-{2-[(6,6-dimethylpiperidin-3-yl)amino]-5-(trifluoromethyl)pyrimidin-4-yl}-8-oxo-1H,4H,5H,6H,7H,8H-pyrrolo[2,3-c]azepin-7-yl)-1-methyl-1,2-dihydropyridin-2-one (**CDK7_1140_a**);

3-(2-{[(3S)-6,6-dimethylpiperidin-3-yl]amino}-5-(trifluoromethyl)pyrimidin-4-yl)-7-(pyridin-4-yl)-1H,4H,5H,6H,7H,8H-pyrrolo[2,3-c]azepin-8-one (**CDK7_1141**);

3-{2-[(6,6-dimethylpiperidin-3-yl)amino]-5-(trifluoromethyl)pyrimidin-4-yl } -7-(pyridin-4-yl)-1H,4H,5H,6H,7H,8H-pyrrolo[2,3-c]azepin-8-one (**CDK7_1141_a**);

3-(2-{[(3S)-6,6-dimethylpiperidin-3-yl]amino}-5-(trifluoromethyl)pyrimidin-4-yl)-7-(pyridin-3-yl)-1H,4H,5H,6H,7H,8H-pyrrolo[2,3-c]azepin-8-one (**CDK7_1142**);

3-{2-[(6,6-dimethylpiperidin-3-yl)amino]-5-(trifluoromethyl)pyrimidin-4-yl }-7-(pyridin-3-yl)-1H,4H,5H,6H,7H,8H-pyrrolo[2,3-c]azepin-8-one (**CDK7_1142_a**);

3-(2-{[(3S)-6,6-dimethylpiperidin-3-yl]amino}-5-(trifluoromethyl)pyrimidin-4-yl)-7-(pyridin-2-yl)-1H,4H,5H,6H,7H,8H-pyrrolo[2,3-c]azepin-8-one (**CDK7_1143**);

3-{2-[(6,6-dimethylpiperidin-3-yl)amino]-5-(trifluoromethyl)pyrimidin-4-yl }-7-(pyridin-2-yl)-1H,4H,5H,6H,7H,8H-pyrrolo[2,3-c]azepin-8-one (**CDK7_1143_a**);

3-(2-{[(3S)-6,6-dimethylpiperidin-3-yl]amino}-5-(trifluoromethyl)pyrimidin-4-yl)-7-[(1-methyl-1H-pyrazol-4-yl)methyl]-1H,4H,5H,6H,7H,8H-pyrrolo[2,3-c]azepin-8-one (**CDK7_1144**);

3-{2-[(6,6-dimethylpiperidin-3-yl)amino]-5-(trifluoromethyl)pyrimidin-4-yl}-7-[(1-methyl-1H-pyrazol-4-yl)methyl]-1H,4H,5H,6H,7H,8H-pyrrolo[2,3-c]azepin-8-one (**CDK7_1144a**);

3-(2-{[(3S)-6,6-dimethylpiperidin-3-yl]amino}-5-(trifluoromethyl)pyrimidin-4-yl)-7-[(1,3-thiazol-2-yl)methyl]-1H,4H,5H,6H,7H,8H-pyrrolo[2,3-c]azepin-8-one (**CDK7_1145**);

3-{2-[(6,6-dimethylpiperidin-3-yl)amino]-5-(trifluoromethyl)pyrimidin-4-yl}-7-[(1,3-thiazol-2-yl)methyl]-1H,4H,5H,6H,7H,8H-pyrrolo[2,3-c]azepin-8-one (**CDK7_1145_a**);

7-[(3,5-dimethyl-1,2-oxazol-4-yl)methyl]-3-(2-{[(3S)-6,6-dimethylpiperidin-3-yl]amino}-5-(trifluoromethyl)pyrimidin-4-yl)-1H,4H,5H,6H,7H,8H-pyrrolo[2,3-c]azepin-8-one (**CDK7_1146**);

7-[(3,5-dimethyl-1,2-oxazol-4-yl)methyl]-3-{2-[(6,6-dimethylpiperidin-3-yl)amino]-5-(trifluoromethyl)pyrimidin-4-yl}-1H,4H,5H,6H,7H,8H-pyrrolo[2,3-c]azepin-8-one (**CDK7_1146_a**);
3-(2-{[(3S)-6,6-dimethylpiperidin-3-yl]amino}-5-(trifluoromethyl)pyrimidin-4-yl)-7-[1-(1-methyl-1H-pyrazol-4-yl)ethyl]-1H,4H,5H,6H,7H,8H-pyrrolo[2,3-c]azepin-8-one (**CDK7_1147**);
3-{2-[(6,6-dimethylpiperidin-3-yl)amino]-5-(trifluoromethyl)pyrimidin-4-yl}-7-[1-(1-methyl-1H-pyrazol-4-yl)ethyl]-1H,4H,5H,6H,7H,8H-pyrrolo[2,3-c]azepin-8-one (**CDK7_1147_a**);
3-(2-{[(3S)-6,6-dimethylpiperidin-3-yl]amino}-5-(trifluoromethyl)pyrimidin-4-yl)-7-[1-(1,3-thiazol-2-yl)ethyl]-1H,4H,5H,6H,7H,8H-pyrrolo[2,3-c]azepin-8-one (**CDK7_1148**);
3-{2-[(6,6-dimethylpiperidin-3-yl)amino]-5-(trifluoromethyl)pyrimidin-4-yl}-7-[1-(1,3-thiazol-2-yl)ethyl]-1H,4H,5H,6H,7H,8H-pyrrolo[2,3-c]azepin-8-one (**CDK7_1148_a**);
7-[1-(3,5-dimethyl-1,2-oxazol-4-yl)ethyl]-3-(2-{[(3S)-6,6-dimethylpiperidin-3-yl]amino}-5-(trifluoromethyl)pyrimidin-4-yl)-1H,4H,5H,6H,7H,8H-pyrrolo[2,3-c]azepin-8-one (**CDK7_1149**);
7-[1-(3,5-dimethyl-1,2-oxazol-4-yl)ethyl]-3-{2-[(6,6-dimethylpiperidin-3-yl)amino]-5-(trifluoromethyl)pyrimidin-4-yl}-1H,4H,5H,6H,7H,8H-pyrrolo[2,3-c]azepin-8-one (**CDK7_1149_a**);
3-(2-{ [(3 S)-6,6-dimethylpiperidin-3-yl]amino}-5-(trifluoromethyl)pyrimidin-4-yl)-7-(oxan-4-yl)-1H,4H,5H,6H,7H,8H-pyrrolo[2,3-c]azepin-8-one (**CDK7_1150**);
3-{2-[(6,6-dimethylpiperidin-3-yl)amino]-5-(trifluoromethyl)pyrimidin-4-yl}-7-(oxan-4-yl)-1H,4H,5H,6H,7H,8H-pyrrolo[2,3-c]azepin-8-one (**CDK7_1150_a**);
3-(2-{ [(3 S)-6,6-dimethylpiperidin-3-yl]amino}-5-(trifluoromethyl)pyrimidin-4-yl)-7-[(oxan-4-yl)methyl]-1H,4H,5H,6H,7H,8H-pyrrolo[2,3-c]azepin-8-one (**CDK7_1151**);
3-{2-[(6,6-dimethylpiperidin-3-yl)amino]-5-(trifluoromethyl)pyrimidin-4-yl}-7-[(oxan-4-yl)methyl]-1H,4H,5H,6H,7H,8H-pyrrolo[2,3-c]azepin-8-one (**CDK7_1151_a**);
3-(2-{ [(3 S)-6,6-dimethylpiperidin-3-yl]amino}-5-(trifluoromethyl)pyrimidin-4-yl)-7-(oxolan-3-yl)-1H,4H,5H,6H,7H,8H-pyrrolo[2,3-c]azepin-8-one (**CDK7_1152**);
3-{2-[(6,6-dimethylpiperidin-3-yl)amino]-5-(trifluoromethyl)pyrimidin-4-yl}-7-(oxolan-3-yl)-1H,4H,5H,6H,7H,8H-pyrrolo[2,3-c]azepin-8-one (**CDK7_1152_a**);
3-{2-[(6,6-dimethylpiperidin-3-yl)amino]-5-(trifluoromethyl)pyrimidin-4-yl}-7-[(oxolan-3-yl)methyl]-1H,4H,5H,6H,7H,8H-pyrrolo[2,3-c]azepin-8-one (**CDK7_1153**);
3-{2-[(6,6-dimethylpiperidin-3-yl)amino]-5-(trifluoromethyl)pyrimidin-4-yl}-7-[(oxolan-3-yl)methyl]-1H,4H,5H,6H,7H,8H-pyrrolo[2,3-c]azepin-8-one (**CDK7_1153_a**);
7-cyclopentyl-3-(2-{[(3S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1154**);
7-cyclopentyl-3-{2-[(6,6-dimethylpiperidin-3-yl)amino]-5-(trifluoromethyl)pyrimidin-4-yl}-1H,4H,5H,6H,7H,8H-pyrrolo[2,3-c]azepin-8-one (**CDK7_1154_a**);
7-(cyclopentylmethyl)-3-(2- f [(3 S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1155**);
7-(cyclopentylmethyl)-3-{2-[(6,6-dimethylpiperidin-3-yl)amino]-5-(trifluoromethyl) pyrimidin-4-yl}-1H,4H,5H,6H,7H,8H-pyrrolo[2,3-c]azepin-8-one (**CDK7_1155_a**);
3-(2-{[(3S)-6,6-dimethylpiperidin-3-yl]amino}-5-(trifluoromethyl)pyrimidin-4-yl)-7-[1-(oxan-4-yl)ethyl]-1H,4H,5H,6H,7H,8H-pyrrolo[2,3-c]azepin-8-one (**CDK7_1156**);
3-{2-[(6,6-dimethylpiperidin-3-yl)amino]-5-(trifluoromethyl)pyrimidin-4-yl}-7-[1-(oxanyl-4-yl)ethyl]-1H,4H,5H,6H,7H,8H-pyrrolo[2,3-c]azepin-8-one (**CDK7_1156_a**),
3-(2-{[(3S)-6,6-dimethylpiperidin-3-yl]amino}-5-(trifluoromethyl)pyrimidin-4-yl)-7-[1-(oxolan-3-yl)ethyl]-1H,4H,5H,6H, 7H,8H-pyrrolo[2,3-c]azepin-8-one (**CDK7_1157**);
3-{2-[(6,6-dimethylpiperidin-3-yl)amino]-5-(trifluoromethyl)pyrimidin-4-yl}-7-[1-(oxolan-3-yl)ethyl]-1H,4H,5H,6H,7H,8H-pyrrolo[2,3-c]azepin-8-one (**CDK7_1157_a**);
7-(1-cyclopentylethyl)-3-(2-{[(3S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1158**);
7-(1-cyclopentylethyl)-3-{2-[(6,6-dimethylpiperidin-3-yl)amino]-5-(trifluoromethyl) pyrimidin-4-yl}-1H,4H,5H,6H,7H,8H-pyrrolo[2,3-c]azepin-8-one (**CDK7_1158_a**);
7-tert-butyl-3-(2-{[(3S)-6,6-dimethylpiperidin-3-yl]amino}-5-(trifluoromethyl)pyrimidin-4-yl)-1H,4H,5H,6H,7H,8H-pyrrolo[2,3-c]azepin-8-one (**CDK7_1159**);
7-tert-butyl-3-{2-[(6,6-dimethylpiperidin-3-yl)amino]-5-(ttrifluoromethyl)pyrimidin-4-yl}-1H,4H,5H,6H,7H,8H-pyrrolo[2,3-c]azepin-8-one (**CDK7_1159_a**);
3-(2-{ [(3 S)-6,6-dimethylpiperidin-3-yl]amino}-5-(trifluoromethyl)pyrimidin-4-yl)-7-(2,2-dimethylpropyl)-1H,4H,5H,6H,7H,8H-pyrrolo[2,3-c]azepin-8-one (**CDK7_1160**);
3-{2-[(6,6-dimethylpiperidin-3-yl)amino]-5-(trifluoromethyl)pyrimidin-4-yl}-7-(2,2-dimethylpropyl)-1H,4H,5H,6H,7H,8H-pyrrolo[2,3-c]azepin-8-one (**CDK7_1160_a**);

7-(3,3-dimethylbutan-2-yl)-3-(2-{[(3S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1161**);
7-(3,3-dimethylbutan-2-yl)-3-{2-[(6,6-dimethylpiperidin-3-yl)amino]-5-(trifluoromethyl) pyrimidin-4-yl}-1H,4H,5H,6H,7H,8H-pyrrolo[2,3-c]azepin-8-one (**CDK7_1161_a**);
8-(2-{ [(3 S)-6,6-dimethylpiperidin-3-yl]amino}-5-(trifluoromethyl)pyrimidin-4-yl)-4-(1-methyl-1H-pyrazol-4-yl)-2H,3H,4H,5H,6H-pyrrolo[2,3-f][1,4]oxazepin-5-one (**CDK7_1162**);
8-{2-[(6,6-dimethylpiperidin-3-yl)amino]-5-(trifluoromethyl)pyrimidin-4-yl}-4-(1-methyl-1H-pyrazol-4-yl)-2H,3H,4H,5H,6H-pyrrolo[2,3-f][1,4]oxazepin-5-one (**CDK7_1162_a**);
(6S)-3-(2-{[(3S)-6,6-dimethylpiperidin-3-yl]amino}-5-(trifluoromethyl)pyrimidin-4-yl)-6-methyl-7-(1-methyl-1H-pyrazol-4-yl)-1H,4H,5H,6H,7H,8H-pyrrolo[2,3-c]azepin-8-one (**CDK7_1163**);
3-{2-[(6,6-dimethylpiperidin-3-yl)amino]-5-(trifluoromethyl)pyrimidin-4-yl}-6-methyl-7-(1-methyl-1H-pyrazol-4-yl)-1H,4H,5H,6H,7H,8H-pyrrolo[2,3-c]azepin-8-one (**CDK7_1163_a**);
(6R)-3-(2-{[(3S)-6,6-dimethylpiperidin-3-yl]amino}-5-(trifluoromethyl)pyrimidin-4-yl)-6-methyl-7-(1-methyl-1H-pyrazol-4-yl)-1H,4H,5H,6H,7H,8H-pyrrolo[2,3-c]azepin-8-one (**CDK7_1164**);
3-{2-[(6,6-dimethylpiperidin-3-yl)amino]-5-(trifluoromethyl)pyrimidin-4-yl}-6-methyl-7-(1-methyl-1H-pyrazol-4-yl)-1H,4H,5H,6H,7H,8H-pyrrolo[2,3-c]azepin-8-one (**CDK7_1164_a**);
3-(2-{[(3S)-6,6-dimethylpiperidin-3-yl]amino}-5-(trifluoromethyl)pyrimidin-4-yl)-6,6-dimethyl-7-(1-methyl-1H-pyrazol-4-yl)-1H,4H,5H,6H,7H,8H-pyrrolo[2,3-c]azepin-8-one (**CDK7_1165**);
3-{2-[(6,6-dimethylpiperidin-3-yl)amino]-5-(trifluoromethyl)pyrimidin-4-yl}-6,6-dimethyl-7-(1-methyl-1H-pyrazol-4-yl)-1H,4H,5H,6H,7H,8H-pyrrolo[2,3-c]azepin-8-one (**CDK7_1165_a**);
6-cyclopropyl-3-(2-{[(3S)-piperidin-3-yl]amino}-5-(trifluoromethyl) pyrimidin-4-yl)-1H,6H, 7H-pyrrolo[2,3-c]pyridin-7-one (**CDK7_1105**);
6-cyclopropyl-3-{2-[(piperidin-3-yl)amino]-5-(trifluoromethyl)pyrimidin-4-yl}-1H,6H,7H-pyrrolo[2,3-c]pyridin-7-one (**CDK7_1105_a**);
3-(2-{[(3S)-piperidin-3-yl]amino}-5-(trifluoromethyl)pyrimidin-4-yl)-6-(1,3-thiazol-2-yl)-1H,6H, 7H-pyrrolo[2,3-c]pyridin-7-one (**CDK7_1158**);
3-{2-[(piperidin-3-yl)amino]-5-(trifluoromethyl)pyrimidin-4-yl }-6-(1,3-thiazol-2-yl)-1H,6H,7H-pyrrolo[2,3-c]pyridin-7-one (**CDK7_1058_a**);
1-methyl-5-[7-oxo-3-(2-{[(3S)-piperidin-3-yl]amino}-5-(trifluoromethyl)pyrimidin-4-yl)-1H,6H,7H-pyrrolo[2,3-c]pyridin-6-yl]-1,2-dihydropyridin-2-one (**CDK7_1052**);
1-methyl-5-(7-oxo-3-{2-[(piperidin-3-yl)amino]-5-(trifluoromethyl)pyrimidin-4-yl}-1H,6H, 7H-pyrrolo[2,3-c]pyridin-6-yl)-1,2-dihydropyridin-2-one (**CDK7_1052_a**);
N-[(3S)-piperidin-3-yl]-4-(1H-pyrrol-3-yl)-5-(trifluoromethyl)pyrimidin-2-amine (**CDK7_972**);
N-(piperidin-3-yl)-4-(1H-pyrrol-3-yl)-5-(trifluoromethyl)pyrimidin-2-amine (**CDK7_972_a**);
7-methyl-3-(2-{[(3S)-piperidin-3-yl]amino}-5-(trifluoromethyl)pyrimidin-4-yl)-1H,4H,5H,6H,7H,8H-pyrrolo[2,3-c]azepin-4,8-dione (**CDK7_1021**);
7-methyl-3-{2-[(piperidin-3-yl)amino]-5-(trifluoromethyl)pyrimidin-4-yl}-1H,4H,5H,6H,7H,8H-pyrrolo[2,3-c]azepin-4,8-dione (**CDK7_1021_a**);
6-(oxan-4-yl)-3-(2-{[(3S)-piperidin-3-yl]amino}-5-(trifluoromethyl) pyrimidin-4-yl)-1H,6H,7H-pyrrolo[2,3-c]pyridin-7-one (**CDK7_1121**);
6-(oxan-4-yl)-3-{2-[(piperidin-3-yl)amino]-5-(trifluoromethyl)pyrimidin-4-yl}-1H,6H,7H-pyrrolo[2,3-c]pyridin-7-one (**CDK7_1121_a**);
4-(1H-indol-1-yl)-N-[(3 S)-piperidin-3-yl]-5-(trifluoromethyl)pyrimidin-2-amine (**CDK7_809**);
4-(1H-indol-1-yl)-N-(piperidin-3-yl)-5-(trifluoromethyl)pyrimidin-2-amine (**CDK7_809_a**);
4-(1H-1,3-benzodiazol-1-yl)-N-[(3S)-piperidin-3-yl]-5-(trifluoromethyl)pyrimidin-2-amine (**CDK7_810**);
4-(1H-1,3-benzodiazol-1-yl)-N-(piperidin-3-yl)-5-(trifluoromethyl)pyrimidin-2-amine (**CDK7_810_a**);
4-(1H-1,2,3-benzotriazol-1-yl)-N-[(3S)-piperidin-3-yl]-5-(trifluoromethyl)pyrimidin-2-amine (**CDK7_811**);
4-(1H-1,2,3-benzotriazol-1-yl)-N-(piperidin-3-yl)-5-(trifluoromethyl)pyrimidin-2-amine (**CDK7_811_a**);
7-methyl-3-(2-{[(3S)-piperidin-3-yl]amino}-5-(trifluoromethyl)pyrimidin-4-yl)-1H,4H,5H,6H,7H,8H-pyrrolo[2,3-c]azepin-8-one (**CDK7_953**);
7-methyl-3-{2-[(piperidin-3-yl)amino]-5-(trifluoromethyl)pyrimidin-4-yl}-1H,4H,5H,6H,7H,8H-pyrrolo[2,3-c]azepin-8-one (**CDK7_953_a**);
6-(1-methyl-1H-pyrazol-4-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_959**);
6-(1-methyl-1H-pyrazol-4-yl)-3-{2-[(piperidin-3-yl)amino]-5-(trifluoromethyl) pyrimidin-4-yl}-1H,6H,7H-pyrrolo[2,3-c]pyridin-7-one (**CDK7_959_a**);
6-benzyl-3-(2-{[(3S)-piperidin-3-yl]amino}-5-(trifluoromethyl)pyrimidin-4-yl)-1H,6H,7H-pyrrolo[2,3-c]pyridin-7-one (**CDK7_961**);

6-benzyl-3-{2-[(piperidin-3-yl)amino]-5-(trifluoromethyl)pyrimidin-4-yl}-1H,6H,7H-pyrrolo[2,3-c]pyridin-7-one (**CDK7_961_a**);
N,N-dimethyl-4-(2-{[(3S)-piperidin-3-yl]amino}-5-(trifluoromethyl)pyrimidin-4-yl)-1H-imidazol-2-carboxamide (**CDK7_966**);
N,N-dimethyl-4-{2-[(piperidin-3-yl)amino]-5-(trifluoromethyl)pyrimidin-4-yl}-1H-imidazol-2-carboxamide (**CDK7_966_a**);
N,N-dimethyl-4-(2-{[(3S)-piperidin-3-yl]amino}-5-(trifluoromethyl)pyrimidin-4-yl)-1H-pyrrol-3-carboxamide (**CDK7_991**);
N,N-dimethyl-4-{2-[(piperidin-3-yl)amino]-5-(trifluoromethyl)pyrimidin-4-yl}-1H-pyrrol-3-carboxamide (**CDK7_991_a**);
3-(2-{[(3S)-piperidin-3-yl]amino}-5-(trifluoromethyl)pyrimidin-4-yl)-7-(pyridin-2-yl)-1H,4H,5H,6H,7H,8H-pyrrolo[2,3-c]azepin-8-one (**CDK7_1176**);
3-{2-[(piperidin-3-yl)amino]-5-(trifluoromethyl)pyrimidin-4-yl}-7-(pyridin-2-yl)-1H,4H,5H,6H,7H,8H-pyrrolo[2,3-c]azepin-8-one (**CDK7_1176_a**);
3-(2-{[(3S)-6,6-dimethylpiperidin-3-yl]amino}-5-(trifluoromethyl)pyrimidin-4-yl)-7-methyl-1H,4H,5H,6H,7H,8H-pyrrolo[2,3-c]azepin-8-one (**CDK7_1187**);
3-{2-[(6,6-dimethylpiperidin-3-yl)amino]-5-(trifluoromethyl)pyrimidin-4-yl}-7-methyl-1H,4H,5H,6H,7H,8H-pyrrolo[2,3-c]azepin-8-one (**CDK7_1187_a**);
3-(2-{[(6S)-4-azaspiro[2.5]octan-6-yl]amino}-5-(trifluoromethyl)pyrimidin-4-yl)-6-(1-methyl-1H-pyrazol-4-yl)-1H,6H,7H-pyrrolo[2,3-c]pyridin-7-one (**CDK7_1199**);
3-[2-({4-azaspiro[2.5]octan-6-yl}amino)-5-(trifluoromethyl)pyrimidin-4-yl]-6-(1-methyl-1H-pyrazol-4-yl)-1H,6H,7H-pyrrolo[2,3-c]pyridin-7-one (**CDK7_1199_a**);
3-(2-{[(6S)-4-azaspiro[2.5]octan-6-yl]amino}-5-(trifluoromethyl)pyrimidin-4-yl)-6-methyl-1H,6H,7H-pyrrolo[2,3-c]pyridin-7-one (**CDK7_1200**);
3-[2-({4-azaspiro[2.5]octan -6-yl}amino)-5-(trifluoromethyl)pyrimidin-4-yl]-6-methyl-1H,6H,7H-pyrrolo[2,3-c]pyridin-7-one (**CDK7_1200_a**);
3-(2-{[(6S)-4-azaspiro[2.5]octan-6-yl]amino}-5-(trifluoromethyl)pyrimidin-4-yl)-7-methyl-1H,4H,5H,6H,7H,8H-pyrrolo[2,3-c]azepin-8-one (**CDK7_1201**);
3-[2-({4-azaspiro[2.5]octan-6-yl}amino)-5-(trifluoromethyl)pyrimidin-4-yl]-7-methyl-1H,4H,5H,6H,7H,8H-pyrrolo[2,3-c]azepin-8-one (**CDK7_1201_a**);
3-(2-{[(3S)-piperidin-3-yl]amino}-5-(trifluoromethyl)pyrimidin-4-yl)-6-(1,3-thiazol-4-yl)-1H,6H, 7H-pyrrolo[2,3-c]pyridin-7-one (**CDK7_1202**);
3-{2-[(piperidin-3-yl)amino]-5-(trifluoromethyl)pyrimidin-4-yl }-6-(1,3-thiazol-4-yl)-1H,6H,7H-pyrrolo[2,3-c]pyridin-7-one (**CDK7_1202_a**);
3-(2-{[(3S)-piperidin-3-yl]amino}-5-(trifluoromethyl)pyrimidin-4-yl)-6-(1,3-thiazol-5-yl)-1H,6H, 7H-pyrrolo[2,3-c]pyridin-7-one (**CDK7_1203**);
3-{2-[(piperidin-3-yl)amino]-5-(trifluoromethyl)pyrimidin-4-yl }-6-(1,3-thiazol-5-yl)-1H,6H,7H-pyrrolo[2,3-c]pyridin-7-one (**CDK7_1203_a**);
6-(2-methyl-1,3-thiazol-4-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1204**);
6-(2-methyl-1,3-thiazol-4-yl)-3-{2-[(piperidin-3-yl)amino]-5-(trifluoromethyl)pyrimidin-4-yl}-1H,6H,7H-pyrrolo[2,3-c]pyridin-7-one (**CDK7_1204_a**);
6-(2-methyl-1,3-thiazol-5-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1205**);
-(2-methyl-1,3-thiazol-5-yl)-3-{2-[(piperidin-3-yl)amino]-5-(trifluoromethyl)pyrimidin-4-yl}-1H,6H,7H-pyrrolo[2,3-c]pyridin-7-one (**CDK7_1205_a**);
6-(4-methyl-1,3-thiazol-2-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1206**);
6-(4-methyl-1,3-thiazol-2-yl)-3-{2-[(piperidin-3-yl)amino]-5-(trifluoromethyl)pyrimidin-4-yl}-1H,6H,7H-pyrrolo[2,3-c]pyridin-7-one (**CDK7_1206_a**);
6-(5-methyl-1,3-thiazol-2-yl)-3-(2-{[(3S)-piperidin-3-yl]amino}-5-(trifluoromethyl)pyrimidin-4-yl)-1H,6H,7H-pyrrolo[2,3-c]pyridin-7-one (CDK7_1207);
6-(5-methyl-1,3-thiazol-2-yl)-3-{2-[(piperidin-3-yl)amino]-5-(trifluoromethyl)pyrimidin-4-yl}-1H,6H,7H-pyrrolo[2,3-c]pyridin-7-one (**CDK7_1207_a**);
6-(cyclopropylmethyl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1208**);
6-(cyclopropylmethyl)-3-{2-[(piperidin-3-yl)amino]-5-(trifluoromethyl)pyrimidin-4-yl}-1H,6H,7H-pyrrolo[2,3-c]pyridin-7-one (**CDK7_1208_a**);

6-cyclobutyl-3-(2-{[(3S)-piperidin-3-yl]amino}-5-(trifluoromethyl)pyrimidin-4-yl)-1H,6H, 7H-pyrrolo[2,3-c]pyridin-7-one (**CDK7_1209**);
6-cyclobutyl-3-{2-[(piperidin-3-yl)amino]-5-(trifluoromethyl)pyrimidin-4-yl}-1H,6H,7H-pyrrolo[2,3-c]pyridin-7-one (**CDK7_1209_a**);
6-cyclopentyl-3-(2-{[(3S)-piperidin-3-yl]amino}-5-(trifluoromethyl)pyrimidin-4-yl)-1H,6H, 7H-pyrrolo[2,3-c]pyridin-7-one (**CDK7_1210**);
6-cyclopentyl-3-{2-[(piperidin-3-yl)amino]-5-(trifluoromethyl)pyrimidin-4-yl}-1H,6H,7H-pyrrolo[2,3-c]pyridin-7-one (**CDK7_1210_a**);
6-(1-methylcyclopropyl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1211**);
6-(1-methylcyclopropyl)-3-{2-[(piperidin-3-yl)amino]-5-(trifluoromethyl)pyrimidin-4-yl}-1H,6H,7H-pyrrolo[2,3-c]pyridin-7-one (**CDK7_1211_a**);
6-cyclohexyl-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1212**);
6-cyclohexyl-3-{2-[(piperidin-3-yl)amino]-5-(trifluoromethyl)pyrimidin-4-yl}-1H,6H,7H-pyrrolo[2,3-c]pyridin-7-one (**CDK7_1212_a**);
3-(2-{[(3S)-piperidin-3-yl]amino}-5-(trifluoromethyl)pyrimidin-4-yl)-6-(pyridin-2-yl)-1H,6H,7H-pyrrolo[2,3-c]pyridin-7-one (**CDK7_1213**);
3-{2-[(piperidin-3-yl)amino]-5-(trifluoromethyl)pyrimidin-4-yl}-6-(pyridin-2-yl)-1H,6H,7H-pyrrolo[2,3-c]pyridin-7-one (**CDK7_1213_a**);
3-(2-{[(3S)-piperidin-3-yl]amino}-5-(trifluoromethyl)pyrimidin-4-yl)-6-(pyridin-3-yl)-1H,6H,7H-pyrrolo[2,3-c]pyridin-7-one (**CDK7_1214**);
3-{2-[(piperidin-3-yl)amino]-5-(trifluoromethyl)pyrimidin-4-yl}-6-(pyridin-3-yl)-1H,6H,7H-pyrrolo[2,3-c]pyridin-7-one (**CDK7_1214_a**);
3-(2-{[(3S)-piperidin-3-yl]amino}-5-(trifluoromethyl)pyrimidin-4-yl)-6-(pyridin-4-yl)-1H,6H,7H-pyrrolo[2,3-c]pyridin-7-one (**CDK7_1215**);
3-{2-[(piperidin-3-yl)amino]-5-(trifluoromethyl)pyrimidin-4-yl}-6-(pyridin-4-yl)-1H,6H,7H-pyrrolo[2,3-c]pyridin-7-one (**CDK7_1215_a**);
6-(3-methylpyridin-4-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1216**);
6-(3-methylpyridin-4-yl)-3-{2-[(piperidin-3-yl)amino]-5-(trifluoromethyl)pyrimidin-4-yl}-1H,6H,7H-pyrrolo[2,3-c]pyridin-7-one (**CDK7_1216_a**);
6-(2-methylpyridin-3-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1217**);
6-(2-methylpyridin-3-yl)-3-{2-[(piperidin-3-yl)amino]-5-(trifluoromethyl)pyrimidin-4-yl}-1H,6H,7H-pyrrolo[2,3-c]pyridin-7-one (**CDK7_1217_a**);
6-(4-methylpyridin-3-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1218**);
6-(4-methylpyridin-3-yl)-3-{2-[(piperidin-3-yl)amino]-5-(trifluoromethyl)pyrimidin-4-yl}-1H,6H,7H-pyrrolo[2,3-c]pyridin-7-one (**CDK7_1218_a**);
6-(3-methylpyridin-2-yl)-3-(2-{[(3S)-piperidin-3-yl]amino}-5-(trifluoromethyl)pyrimidin-4-yl)-1H,6H,7H-pyrrolo[2,3-c]pyridin-7-one (**CDK7_1219**);
6-(3-methylpyridin-2-yl)-3-{2-[(piperidin-3-yl)amino]-5-(trifluoromethyl)pyrimidin-4-yl}-1H,6H,7H-pyrrolo[2,3-c]pyridin-7-one (**CDK7_1219_a**);
7-cyclopropyl-3-(2-{[(3S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1225**);
7-cyclopropyl-3-{2-[(6,6-dimethylpiperidin-3-yl)amino]-5-(trifluoromethyl)pyrimidin-4-yl}-1H,4H,5H,6H,7H,8H-pyrrolo[2,3-c]azepin-8-one (**CDK7_1225_a**);
7-cyclobutyl-3-(2-{[(3S)-6,6-dimethylpiperidin-3-yl]amino}-5-(trifluoromethyl)pyrimidin-4-yl)-1H,4H,5H,6H,7H,8H-pyrrolo[2,3-c]azepin-8-one (**CDK7_1226**);
7-cyclobutyl-3-{2-[(6,6-dimethylpiperidin-3-yl)amino]-5-(trifluoromethyl)pyrimidin-4-yl}-1H,4H,5H,6H,7H,8H-pyrrolo[2,3-c]azepin-8-one (**CDK7_1226_a**);
7-(cyclopropylmethyl)-3-(2-{[(3S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1227**);
7-(cyclopropylmethyl)-3-{2-[(6,6-dimethylpiperidin-3-yl)amino]-5-(trifluoromethyl) pyrimidin-4-yl}-1H,4H,5H,6H,7H,8H-pyrrolo[2,3-c]azepin-8-one (**CDK7_1227_a**);
7-(cyclobutylmethyl)-3-(2-{[(3S)-6,6-dimethylpiperidin-3-yl]amino}-5-(trifluoromethyl) pyrimidin-4-yl)-1H,4H,5H,6H,7H,8H-pyrrolo[2,3-c]azepin-8-one (**CDK7_1228**);

7-(cyclobutylmethyl)-3-{2-[(6,6-dimethylpiperidin-3-yl)amino]-5-(trifluoromethyl) pyrimidin-4-yl}-1H,4H,5H,6H,7H,8H-pyrrolo[2,3-c]azepin-8-one (**CDK7_1228_a**);
3-(2-{[(3S)-6,6-dimethylpiperidin-3-yl]amino}-5-(trifluoromethyl)pyrimidin-4-yl)-7-(propan-2-yl)-1H,4H,5H,6H,7H,8H-pyrrolo[2,3-c]azepin-8-one (**CDK7_1229**);
3-{2-[(6,6-dimethylpiperidin-3-yl)amino]-5-(trifluoromethyl)pyrimidin-4-yl}-7-(propan-2-yl)-1H,4H,5H,6H,7H,8H-pyrrolo[2,3-c]azepin-8-one (**CDK7_1229_a**);
3-(2-{[(3S)-6,6-dimethylpiperidin-3-yl]amino}-5-(trifluoromethyl)pyrimidin-4-yl)-7-(2,2,2-trifluoroethyl)-1H,4H,5H,6H,7H,8H-pyrrolo[2,3-c]azepin-8-one (**CDK7_1230**);
3-{2-[(6,6-dimethylpiperidin-3-yl)amino]-5-(trifluoromethyl)pyrimidin-4-yl}-7-(2,2,2-trifluoroethyl)-1H,4H,5H,6H,7H,8H-pyrrolo[2,3-c]azepin-8-one (**CDK7_1230_a**);
4-{5-methyl-3,4,6,13-tetraazatricyclo[8.3.0.0$^{2}$,$^{6}$]trideca-1(10),2,4,11-tetraen-11-yl}-N-[(3S)-piperidin-3-yl]-5-(trifluoromethyl)pyrimidin-2-amine (**CDK7_1236**);
4-{5-methyl-3,4,6,13-tetraazatricyclo[8.3.0.0$^{2}$,$^{6}$]trideca-1(10),2,4,11-tetraen-11-yl}-N-(piperidin-3-yl)-5-(trifluoromethyl)pyrimidin-2-amine (**CDK7_1236_a**);
4-{5-cyclopropyl-3,4,6,13-tetraazatricyclo[8.3.0.0$^{2}$,$^{6}$]trideca-1(10),2,4,11-tetraen-11-yl}-N-[(3S)-piperidin-3-yl]-5-(trifluoromethyl)pyrimidin-2-amine (**CDK7_1237**);
4-{5-cyclopropyl-3,4,6,13-tetraazatricyclo[8.3.0.0$^{2}$,$^{6}$]trideca-1(10),2,4,11-tetraen-11-yl}-N-(piperidin-3-yl)-5-(trifluoromethyl)pyrimidin-2-amine (**CDK7_1237_a**);
3-(2-{[(3S)-6,6-dimethylpiperidin-3-yl]amino}-5-(trifluoromethyl)pyrimidin-4-yl)-6,6,7-trimethyl-1H,4H,5H,6H,7H,8H-pyrrolo[2,3-c]azepin-8-one (**CDK7_1238**);
3-{2-[(6,6-dimethylpiperidin-3-yl)amino]-5-(trifluoromethyl)pyrimidin-4-yl}-6,6,7-trimethyl-1H,4H,5H,6H,7H,8H-pyrrolo[2,3-c]azepin-8-one (**CDK7_1238_a**);
(6S)-3-(2-{[(3S)-6,6-dimethylpiperidin-3-yl]amino}-5-(trifluoromethyl)pyrimidin-4-yl)-6,7-dimethyl-1H,4H,5H,6H,7H,8H-pyrrolo[2,3-c]azepin-8-one (**CDK7_1239**);
3-{2-[(6,6-dimethylpiperidin-3-yl)amino]-5-(trifluoromethyl)pyrimidin-4-yl}-6,7-dimethyl-1H,4H,5H,6H,7H,8H-pyrrolo[2,3-c]azepin-8-one (**CDK7_1239_a**);
(6R)-3-(2-{[(3S)-6,6-dimethylpiperidin-3-yl]amino}-5-(trifluoromethyl)pyrimidin-4-yl)-6,7-dimethyl-1H,4H,5H,6H,7H,8H-pyrrolo[2,3-c]azepin-8-one (**CDK7_1240**);
3-{2-[(6,6-dimethylpiperidin-3-yl)amino]-5-(trifluoromethyl)pyrimidin-4-yl}-6,7-dimethyl-1H,4H,5H,6H,7H,8H-pyrrolo[2,3-c]azepin-8-one (**CDK7_1240_a**);
7-cyclopropyl-3-(2-{[(3S)-6,6-dimethylpiperidin-3-yl]amino}-5-(trifluoromethyl) pyrimidin-4-yl)-6,6-dimethyl-1H,4H,5H,6H,7H,8H-pyrrolo[2,3-c]azepin-8-one (**CDK7_1241**);
7-cyclopropyl-3-{2-[(6,6-dimethylpiperidin-3-yl)amino]-5-(trifluoromethyl)pyrimidin-4-yl}-6,6-dimethyl-1H,4H,5H,6H,7H,8H-pyrrolo[2,3-c]azepin-8-one (**CDK7_1241_a**);
(6S)-7 -cyclopropyl-3-(2-{ [(3 S)-6,6-dimethylpiperidin-3-yl]amino }-5-(trifluoromethyl) pyrimidin-4-yl)-6-methyl-1H,4H,5H,6H,7H,8H-pyrrolo[2,3-c]azepin-8-one (**CDK7_1242**);
7-cyclopropyl-3-{2-[(6,6-dimethylpiperidin-3-yl)amino]-5-(trifluoromethyl)pyrimidin-4-yl}-6-methyl-1H,4H,5H,6H,7H,8H-pyrrolo[2,3-c]azepin-8-one (**CDK7_1242_a**);
(6R)-7-cyclopropyl-3-(2-{[(3 S)-6,6-dimethylpiperidin-3-yl]amino}-5-(trifluoromethyl) pyrimidin-4-yl)-6-methyl-1H,4H,5H,6H,7H,8H-pyrrolo[2,3-c]azepin-8-one (**CDK7_1243**);
7-cyclopropyl-3-{2-[(6,6-dimethylpiperidin-3-yl)amino]-5-(trifluoromethyl)pyrimidin-4-yl}-6-methyl-1H,4H,5H,6H,7H,8H-pyrrolo[2,3-c]azepin-8-one (**CDK7_1243_a**);
3-(2-{[(3S)-6,6-dimethylpiperidin-3-yl]amino}-5-(trifluoromethyl)pyrimidin-4-yl)-5,5,6-trimethyl-1H,4H,5H,6H,7H-pyrrolo[2,3-c]pyridin-7-one (**CDK7_1244**);
3-{2-[(6,6-dimethylpiperidin-3-yl)amino]-5-(trifluoromethyl)pyrimidin-4-yl}-5,5,6-trimethyl-1H,4H,5H,6H,7H-pyrrolo[2,3-c]pyridin-7-one (**CDK7_1244_a**);
6,7-dimethyl-3-(2-{ [(3 S)-piperidin-3-yl]amino}-5-(trifluoromethyl)pyrimidin-4-yl)-1H,4H,5H,6H,7H,8H-pyrrolo [2,3-c]azepin-8-one (**CDK7_1254**);
6,7-dimethyl-3-{2-[(piperidin-3-yl)amino]-5-(trifluoromethyl)pyrimidin-4-yl}-1H,4H,5H,6H,7H,8H-pyrrolo[2,3-c] azepin-8-one (**CDK7_1254_a**);
3-(2-{ [(3 S)-6,6-dimethylpiperidin-3-yl]amino}-5-(trifluoromethyl)pyrimidin-4-yl)-7-ethyl-1H,4H,5H,6H,7H,8H-pyrrolo[2,3-c]azepin-8-one (**CDK7_1259**);
3-{2-[(6,6-dimethylpiperidin-3-yl)amino]-5-(trifluoromethyl)pyrimidin-4-yl}-7-ethyl-1H,4H,5H,6H,7H,8H-pyrrolo [2,3-c]azepin-8-one (**CDK7_1259_a**);
6-(2-{ [(3 S)-6,6-dimethylpiperidin-3-yl]amino}-5-(trifluoromethyl)pyrimidin-4-yl)-1,4-diazatricyclo[7.1.1.0$^{3}$,$^{7}$]undeca-3(7),5-dien-2-one (**CDK7_1261**);
6-{2-[(6,6-dimethylpiperidin-3-yl)amino]-5-(trifluoromethyl)pyrimidin-4-yl}-1,4-diazatricyclo[7.1.1.0$^{3}$,$^{7}$]undeca-3(7),5-dien-2-one (**CDK7_1261_a**);

7-methyl-3-(2-{[(3S,6S)-6-methylpiperidin-3-yl]amino}-5-(trifluoromethyl)pyrimidin-4-yl)-1H,4H,5H,6H,7H,8H-pyrrolo[2,3-c]azepin-8-one (**CDK7_1264**);
7-methyl-3-{2-[(6-methylpiperidin-3-yl)amino]-5-(trifluoromethyl)pyrimidin-4-yl}-1H,4H,5H,6H,7H,8H-pyrrolo[2,3-c]azepin-8-one (**CDK7_1264_a**);
3-(2-{[(3S,6S)-6-ethylpiperidin-3-yl]amino}-5-(trifluoromethyl)pyrimidin-4-yl)-7-methyl-1H,4H,5H,6H,7H,8H-pyrrolo[2,3-c]azepin-8-one (**CDK7_1265**);
3-{2-[(6-ethylpiperidin-3-yl)amino]-5-(trifluoromethyl)pyrimidin-4-yl}-7-methyl-1H,4H,5H,6H,7H,8H-pyrrolo[2,3-c]azepin-8-one (**CDK7_1265_a**);
3-(2-{ [(3 S)-6,6-dimethylpiperidin-3-yl]amino}-5-(trifluoromethyl)pyrimidin-4-yl)-7-(2-methoxyethyl)-1H,4H,5H,6H,7H,8H-pyrrolo[2,3-c]azepin-8-one (**CDK7_1268**);
3-{2-[(6,6-dimethylpiperidin-3-yl)amino]-5-(trifluoromethyl)pyrimidin-4-yl}-7-(2-methoxyethyl)-1H,4H,5H,6H,7H,8H-pyrrolo[2,3-c]azepin-8-one (**CDK7_1268_a**);
2-[3-(2-{[(3S)-6,6-dimethylpiperidin-3-yl]amino}-5-(trifluoromethyl)pyrimidin-4-yl)-8-oxo-1H,4H,5H,6H,7H,8H-pyrrolo[2,3-c]azepin-7-yl]acetonitrile (**CDK7_1269**);
2-(3-{2-[(6,6-dimethylpiperidin-3-yl)amino]-5-(trifluoromethyl)pyrimidin-4-yl}-8-oxo-1H,4H,5H,6H,7H,8H-pyrrolo[2,3-c]azepin-7-yl)acetonitrile (**CDK7_1269_a**);
3-(2-{[(3S)-6,6-dimethylpiperidin-3-yl]amino}-5-(trifluoromethyl)pyrimidin-4-yl)-7-(oxetan-3-yl)-1H,4H,5H,6H,7H,8H-pyrrolo[2,3-c]azepin-8-one (**CDK7_1270**);
3-{2-[(6,6-dimethylpiperidin-3-yl)amino]-5-(trifluoromethyl)pyrimidin-4-yl}-7-(oxetan-3-yl)-1H,4H,5H,6H,7H,8H-pyrrolo[2,3-c]azepin-8-one (**CDK7_1270_a**);
3-(2-{[(3S)-6,6-dimethylpiperidin-3-yl]amino}-5-(trifluoromethyl)pyrimidin-4-yl)-7-[(oxetan-3-yl)methyl]-1H,4H,5H,6H,7H,8H-pyrrolo[2,3-c]azepin-8-one (**CDK7_1271**);
3-{2-[(6,6-dimethylpiperidin-3-yl)amino]-5-(trifluoromethyl)pyrimidin-4-yl}-7-[(oxetan-3-yl)methyl]-1H,4H,5H,6H,7H,8H-pyrrolo[2,3-c]azepin-8-one (**CDK7_1271_a**);
3-(2-{[(6S)-4-azaspiro[2.5]octan-6-yl]amino}-5-(trifluoromethyl)pyrimidin-4-yl)-7-(2-methoxyethyl)-1H,4H,5H,6H,7H,8Hpyrrolo[2,3-c]azepin-8-one (**CDK7_1272**);
3-[2-({4-azaspiro[2.5]octan-6-yl}amino)-5-(trifluoromethyl)pyrimidin-4-yl]-7-(2-methoxyethyl)-1H,4H,5H,6H,7H,8H-pyrrolo[2,3-c]azepin-8-one (**CDK7_1272_a**);
2-[3-(2-{[(6S)-4-azaspiro[2.5]octan-6-yl]amino}-5-(trifluoromethyl)pyrimidin-4-yl)-8-oxo-1H,4H,5H,6H,7H,8H-pyrrolo[2,3-c]azepin-7-yl]acetonitrile (**CDK7_1273**);
2-{3-[2-({4-azaspiro[2.5]octan-6-yl}amino)-5-(trifluoromethyl)pyrimidin-4-yl]-8-oxo-1H,4H,5H,6H,7H,8H-pyrrolo[2,3-c]azepin-7-yl}acetonitrile (**CDK7_1273_a**);
3-(2-{[(6S)-4-azaspiro[2.5]octan-6-yl]amino}-5-(trifluoromethyl)pyrimidin-4-yl)-7-(oxetan-3-yl)-1H,4H,5H,6H,7H,8H-pyrrolo[2,3-c]azepin-8-one (**CDK7_1274**);
3-[2-({4-azaspiro[2.5]octan-6-yl}amino)-5-(trifluoromethyl)pyrimidin-4-yl]-7-(oxetan-3-yl)-1H,4H,5H,6H,7H,8H-pyrrolo[2,3-c]azepin-8-one (**CDK7_1274_a**);
3-(2-{[(6S)-4-azaspiro[2.5]octan-6-yl]amino}-5-(trifluoromethyl)pyrimidin-4-yl)-7-[(oxetan-3-yl)methyl]-1H,4H,5H,6H,7H,8H-pyrrolo[2,3-c]azepin-8-one (**CDK7_1275**);
3-[2-({4-azaspiro[2.5]octan-6-yl}amino)-5-(trifluoromethyl)pyrimidin-4-yl]-7-[(oxetan-3-yl)methyl]-1H,4H,5H,6H, 7H,8H-pyrrolo[2,3-c]azepin-8-one (**CDK7_1275_a**);
3-(2-{[(6S)-4-azaspiro[2.5]octan-6-yl]amino}-5-(trifluoromethyl)pyrimidin-4-yl)-7-cyclopropyl-1H,4H,5H,6H,7H,8H-pyrrolo[2,3-c]azepin-8-one (**CDK7_1276**);
3-[2-({4-azaspiro[2.5]octan-6-yl}amino)-5-(trifluoromethyl)pyrimidin-4-yl]-7-cyclopropyl-1H,4H,5H,6H,7H,8H-pyrrolo[2,3-c]azepin-8-one (**CDK7_1276_a**);
3-(2-{[(6S)-4-azaspiro[2.5]octan-6-yl]amino}-5-(trifluoromethyl)pyrimidin-4-yl)-7-(propan-2-yl)-1H,4H,5H,6H,7H,8H-pyrrolo[2,3-c]azepin-8-one (**CDK7_1277**);
3-[2-({4-azaspiro[2.5]octan-6-yl}amino)-5-(trifluoromethyl)pyrimidin-4-yl]-7-(propan-2-yl)-1H,4H,5H,6H,7H,8H-pyrrolo[2,3-c]azepin-8-one (**CDK7_1277_a**).

**15.** A method for inhibiting the biological activity of cyclin-dependent protein kinases CDK7 in a subject, consisting in contacting cyclin-dependent protein kinases CDK7 with a compound according to any one of claims 1-13.

**16.** A pharmaceutical composition comprising a therapeutically effective amount of the compound according to any one of claims 1-13, or pharmaceutically acceptable salt, solvate, stereoisomer thereof, and one or more pharmaceutically acceptable excipients.

**17.** The pharmaceutical composition according to claim 15 intended for the prophylaxis or treatment of a disease or disorder associated with increased activity of cyclin-dependent protein kinase CDK7.

**18.** The pharmaceutical composition according to claim 16 intended for the prophylaxis or treatment of a disease or disorder associated with increased activity of cyclin-dependent protein kinase CDK7, wherein the disease or disorder associated with increased activity of cyclin-dependent protein kinase CDK7 is an oncological, infectious inflammatory or non-infectious inflammatory, auto-inflammatory, autoimmune disease.

**19.** The pharmaceutical composition according to claim 17, wherein the oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjögren's syndrome, systemic lupus erythematosus, sarcoidosis, ulcerative colitis, multiple sclerosis, myasthenia gravis and uveitis;
an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);
an infectious inflammatory or non-infectious inflammatory disease selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

**20.** A method of treatment of a disease or disorder associated with increased activity of cyclin-dependent protein kinase CDK7, comprising administering a therapeutically effective amount of the compound according to any one of claims 1-13 or a pharmacologically acceptable salt, solvate or stereoisomer thereof or the pharmaceutical composition according to claim 15 to a subject in need of such treatment.

**21.** The method of treatment of a disease or disorder according to claim 19, wherein the disease or disorder associated with increased activity of cyclin-dependent protein kinase CDK7 is an oncological, infectious inflammatory or non-infectious inflammatory, autoinflammatory, autoimmune disease.

**22.** The method of treatment of a disease or disorder according to claim 20, wherein the oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjögren's syndrome, systemic lupus erythematosus, sarcoidosis, ulcerative colitis, multiple sclerosis, myasthenia gravis and uveitis;
an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);
an infectious inflammatory or non-infectious inflammatory disease selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

**23.** The use of the compound according to any one of claims 1-13 or a pharmacologically acceptable salt, solvate or stereoisomer thereof or the pharmaceutical composition according to claim 15 for treatment of a disease or disorder associated with increased activity of cyclin-dependent protein kinase CDK7 in a subject in need of such treatment.

**24.** The use according to claim 22, wherein the disease or disorder associated with increased activity of cyclin-dependent protein kinase CDK7 is an oncological, infectious inflammatory or non-infectious inflammatory, auto-inflammatory,

autoimmune disease.

**25.** The use according to claim 23, wherein the oncological disease selected from a group comprising breast cancer, metastatic breast cancer, HER2-positive breast cancer, breast cancer expressing estrogen receptors, breast cancer expressing progesterone receptors, triple negative breast cancer (TNBC), HER2-negative breast cancer expressing estrogen and/or progesterone receptors, HER2-negative breast cancer, small cell lung cancer, metastatic small cell lung cancer, non-small cell lung cancer, ovarian cancer, metastatic ovarian cancer, glioma, astrocytoma, glioblastoma, pancreatic cancer, stomach cancer, colorectal cancer, head and neck cancer, head and neck squamous cell carcinoma, oral cancer, squamous cell carcinoma of oral cavity, hepatobiliary cancer, hepatocellular carcinoma, urothelial cancer, prostate cancer, castration-resistant prostate cancer, thyroid cancer, leukemia, acute myeloid leukemia, acute lymphoblastic leukemia, lymphoma, T-cell lymphoma, B-cell lymphoma, mantle cell lymphoma, Burkitt lymphoma, diffuse large B-cell lymphoma, neuroblastoma, skin cancer, melanoma, cervical cancer, uterine corpus cancer, fallopian tube cancer, sarcoma, Ewing sarcoma, soft tissue sarcoma, primary peritoneal cancer, retinoblastoma, multiple myeloma;

an autoimmune disease selected from a group comprising axial spondyloarthritis, antiphospholipid syndrome, vasculitides (ANCA-associated vasculitides including Wegener's granulomatosis, and related forms of angiitis, temporal arteritis and polyarteritis nodosa, necrotizing vasculitis), Goodpasture syndrome, autoimmune thyroiditis, Graves' disease, bronchial asthma, cardiomyopathy, Crohn's disease, dermatomyositis, polymyositis, mixed connective tissue disease, glomerulonephritis, Guillain-Barre syndrome, Lyme arthritis, psoriasis, pemphigus, psoriatic arthritis, Reiter's syndrome, rheumatoid arthritis, scleroderma, Sjögren's syndrome, systemic lupus erythematosus, sarcoidosis, ulcerative colitis, multiple sclerosis, myasthenia gravis and uveitis;

an autoinflammatory disease selected from a group comprising Behcet's disease, Blau syndrome, chronic recurrent multi-focal osteomyelitis (CRMO), synovitis, acne, pustulosis, hyperostosis, osteitis (SAPHO) syndrome, cryopyrin-associated periodic syndromes (CAPS), deficiency of interleukin-1 receptor antagonist (DIRA), familial Mediterranean fever (FMF), NLRP12-associated autoinflammatory diseases, neonatal onset multisystem inflammatory disease (NOMID), chronic infantile neurological cutaneous and articular (CINCA) syndrome, Majeed syndrome, mevalonate kinase deficiency (hyperimmunoglobulin-D syndrome), periodic fever, aphthous stomatitis, pharyngitis, and cervical adenitis (PFAPA) syndrome, pyogenic arthritis, pyoderma gangrenosum, and acne (PAPA) syndrome, Schnitzler syndrome, Sweet syndrome, systemic juvenile idiopathic arthritis, Still's disease, adult onset Still's disease (AOSD), tumor necrosis factor (TNF) receptor-associated periodic syndrome (TRAPS);

an infectious inflammatory or non-infectious inflammatory disease selected from a group comprising allergies (including, but not limited to: delayed-type hypersensitivity reactions, immediate-type hypersensitivity reactions, anaphylactic shock), allograft rejection, graft-versus-host disease, atherosclerosis, arteritis, arthritis, arthrostitis, adult respiratory distress syndrome, respiratory tract inflammation (including, but not limited to: bronchiolitis, bronchitis, bronchiectasis, cellular interstitial pneumonia, giant cell interstitial pneumonia, lymphoid interstitial pneumonia, desquamative interstitial pneumonia, hypersensitivity pneumonitis, laryngitis, pneumonitis, pharyngitis, pleurisy, pneumonia, pneumoconiosis, asbestosis, berylliosis, silicosis, talcosis, usual interstitial pneumonitis), tonsillitis, bursitis, phlegmon, chorioamnionitis, blepharitis, iritis, conjunctivitis, dacryoadenitis, chronic cholecystitis, cystic fibrosis, diabetes mellitus, encephalitis, endocarditis, epicondylitis, fasciitis, fibromyalgia, gastritis, gastroenteritis, gingivitis, stomatitis, lymphadenitis, hay fever, inflammatory bowel disease (including, but not limited to: appendicitis, enteritis, enterocolitis, ileitis, necrotizing enterocolitis, proctitis, inflammatory dermatoses), myelitis, myocarditis, nephritis, pyelonephritis, ostitis, osteomyelitis, optic neuritis, otitis, pancreatitis, mumps, pemphigoid, pericarditis, pernicious anemia, phlebitis, rheumatic polymyalgia, hepatitis, cholangitis, progressive systemic sclerosing cholangitis, reperfusion injury, acute rheumatic fever, rhinitis, sinusitis, synovitis, tendinitis, necrotizing fasciitis, prostatitis, cystitis, epididymitis, omphalitis, oophoritis, orchitis, testitis, transverse myelitis, urethritis, urocystitis, uveitis, cervicitis, salpingitis, endometritis, vaginitis, vulvitis, vulvovaginitis.

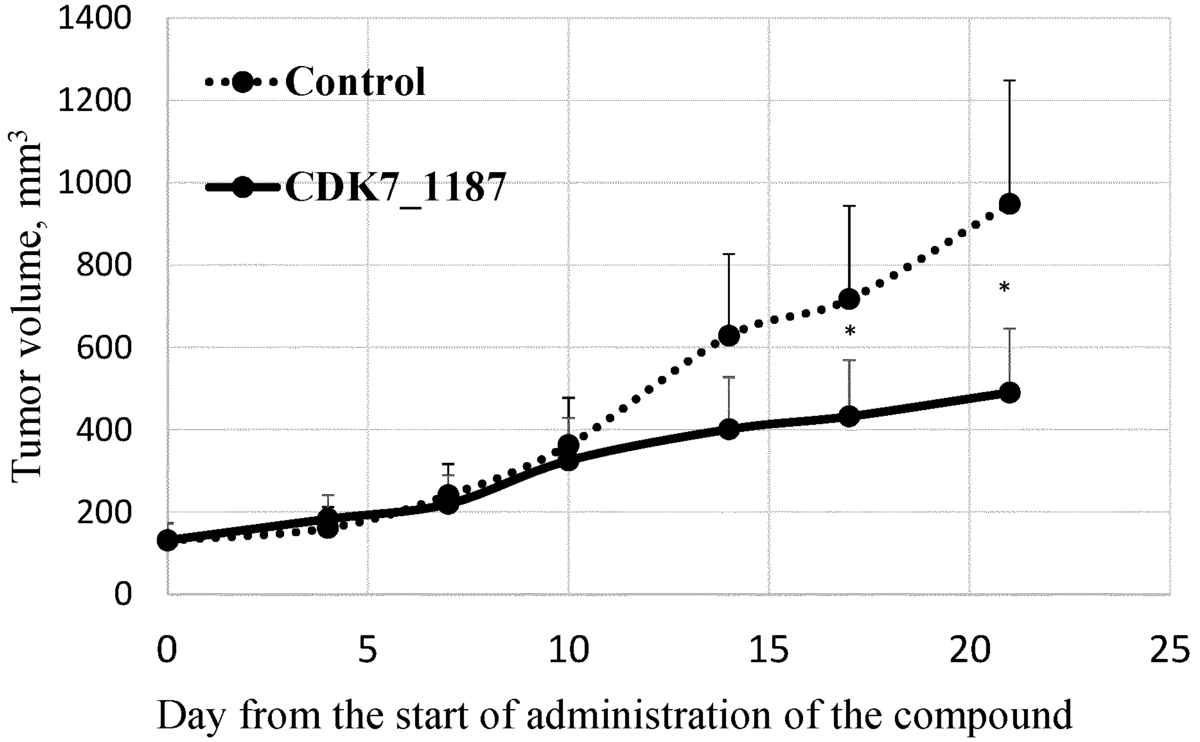
1400
1200
1000
800
600
400
200
0
Tumor volume, mm3
Control
CDK7_1187
*
*
0
5
10
15
20
25
Day from the start of administration of the compound

Fig.1

## INTERNATIONAL SEARCH REPORT

International application No.
PCT/RU2023/050208

A. CLASSIFICATION OF SUBJECT MATTER
(see the supplemental sheet)

According to International Patent Classification (IPC) or to both national classification and IPC

B. FIELDS SEARCHED

Minimum documentation searched (classification system followed by classification symbols)
C07D, A61K, A61P

Documentation searched other than minimum documentation to the extent that such documents are included in the fields searched

Electronic data base consulted during the international search (name of data base and, where practicable, search terms used)
Espacenet, PATENTSCOPE, PatSearch, STN

C. DOCUMENTS CONSIDERED TO BE RELEVANT

| Category* | Citation of document, with indication, where appropriate, of the relevant passages | Relevant to claim No. |
|---|---|---|
| X | WO 2019/143719 A1 (SYROS PHARMACEUTICALS, INC.), 25.07.2019, claims 1-24 | 1-2, 14-25 |
| X | JIANG BAISHAN et al. Discovery and resistance mechanism of a selective CDK12 degrader. Nature chemical biology 2021, vol. 17(6), pp.675-683. doi: 10.1038/s41589-021-00765-y, section Extended Data, a, compound 5 | 1-3, 16 |
| A | WO 2016/058544 A1 (SYROS PHARMACEUTICALS, INC. et al.), 21.04.2016, the claims | 1-3, 14-25 |
| A | US 2004/0162303 A1 (BARTKOVITZ DAVID JOSEPH et al.), 19.08.2004, the claims, example 11 | 1-3, 14-25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 December 2023 (12.12.2023) | 19 January 2024 (19.01.2024) |
| Name and mailing address of the ISA/<br>RU<br><br>Facsimile No. | Authorized officer<br><br>Telephone No. |

Form PCT/ISA/210 (second sheet) (April 2005)

**INTERNATIONAL SEARCH REPORT**

International application No.
PCT/RU2023/050208

C (Continuation). DOCUMENTS CONSIDERED TO BE RELEVANT

| Category* | Citation of document, with indication, where appropriate, of the relevant passages | Relevant to claim No. |
|---|---|---|
| X, P | WO 2023/040998 A1 (TAIZHOU EOC PHARMA), 23.03.2023, the claims, examples | 1-3, 14-25 |
| X, P | CN 116082312 A (ZHEJIANG TONGYUANKANG MEDICINE CO LTD) 09.05.2023, the claims, examples | 1-3, 14-25 |

Form PCT/ISA/210 (continuation of second sheet) (April 2005)

**INTERNATIONAL SEARCH REPORT**

International application No.
PCT/RU2023/050208

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☒ Claims Nos.: 4-13
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/RU2023/050208

A. CLASSIFICATION OF SUBJECT MATTER

***C07D 401/12*** (2006.01)
***C07D 401/14*** (2006.01)
***C07D 417/04*** (2006.01)
***C07D 417/12*** (2006.01)
***C07D 277/44*** (2006.01)
***C07D 413/14*** (2006.01)
***C07D 405/14*** (2006.01)
***A61K 31/506*** (2006.01)
***A61P 35/00*** (2006.01)
***A61P 37/00*** (2006.01)
***A61P 29/00*** (2006.01)
***A61P 31/00*** (2006.01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GANUZA et al.** Genetic inactivation of Cdk7 leads to cell cycle arrest and induces premature aging due to adult stem cell exhaustion. *EMBO J.*, 2012, vol. 31 (11), 2498-510
- **LAROCHELLE et al.** Cyclin-dependent kinase control of the initiation-to-elongation switch of RNA polymerase II. *Nat Struct Mol Biol.*, 2012, vol. 19 (11), 1108-15
- **LIU et al.** Cyclin-dependent kinase 7 (CDK7) expression in human hepatocellular carcinoma: association with HCC progression, prognosis and cell proliferative capacity. *Transl Cancer Res.*, 2018, vol. 7 (3), 472-479
- **WANG et al.** Upregulation of CDK7 in gastric cancer cell promotes tumor cell proliferation and predicts poor prognosis. *Exp Mol Pathol.*, 2016, vol. 100 (3), 514-21
- **PATEL et al.** Expression of CDK7, Cyclin H, and MAT1 Is Elevated in Breast Cancer and Is Prognostic in Estrogen Receptor-Positive Breast Cancer. *Clin Cancer Res.*, 2016, vol. 22 (23), 5929-5938
- **LI et al.** Therapeutic Rationale to Target Highly Expressed CDK7 Conferring Poor Outcomes in Triple-Negative Breast Cancer. *Cancer Res.*, 2017, vol. 77 (14), 3834-3845
- **ZHANG et al.** Low expression of cyclinH and cyclin-dependent kinase 7 can decrease the proliferation of human esophageal squamous cell carcinoma. *Dig Dis Sci.*, 2013, vol. 58 (7), 2028-37
- **CHIPUMURO et al.** CDK7 inhibition suppresses super-enhancer-linked oncogenic transcription in MYCN-driven cancer. *Cell*, 2014, vol. 159 (5), 1126-1139
- **KWIATKOWSKI et al.** Targeting transcription regulation in cancer with a covalent CDK7 inhibitor. *Nature*, 2014, vol. 511 (7511), 616-20
- **GREENALL**. Cyclin-dependent kinase 7 is a therapeutic target in high-grade glioma. *Oncogenesis*, 2017, vol. 6 (5), e336
- **CHRISTENSEN et al.** Targeting transcriptional addictions in small cell lung cancer with a covalent CDK7 inhibitor. *Cancer Cell.*, 08 December 2014, vol. 26 (6), 909-922
- **ZHANG et al.** CDK7 Inhibition Potentiates Genome Instability Triggering Anti-tumor Immunity in Small Cell Lung Cancer. *Cancer Cell*, 2020, vol. 37 (1), 37-54.e9
- **WANG et al.** CDK7 inhibitor THZ1 enhances antiPD-1 therapy efficacy via the p38$\alpha$/MYC/PD-L1 signaling in non-small cell lung cancer. *J Hematol Oncol.*, 2020, vol. 13 (1), 99
- **BERGE S.M. et al.** Pharmaceutical Salts. *J. Pharm. Sci.*, 1977, vol. 66, 1-19